# EUROPEAN PATENT APPLICATION

(11) **EP 1 247 860 A1**
(43) Date of publication of application: **09.10.2002**
(21) Application number: 02252180.1
(22) Date of filing: 26.03.2002
(51) Int. Cl.: C12N 9/12, G06F 19/00

(54) **Crystal structure of pyruvate dehydrogenase kinase 2 (PDHK-2) and use thereof in methods for identifying and designing new ligands**

(30) Priority: 06.04.2001 GB 0108736; 09.04.2001 GB 0108867
(71) Applicant: Pfizer Limited, Sandwich, Kent CT13 9NJ (GB); PFIZER INC., New York, N.Y. 10017 (US)
(72) Inventor: Knoechel, Thorsten Reginald, Sandwich, Kent CT13 9NJ (GB); Robinson, Colin Mark, Sandwich, Kent CT13 9NJ (GB); Taylor, Wendy Elaine, Sandwich, Kent CT13 9NJ (GB); Tucker, Alexander Dunbar, Sandwich, Kent CT13 9NJ (GB)
(74) Representative: Hayles, James Richard

(57) **Abstract**

The present invention relates to crystals of pyruvate dehydrogenase kinase (PDHK) wherein the PDHK protein includes PDHK-2 amino acid sequence as shown in SEQ ID NO: 2 but starting at amino acid position 16, or a homologue, fragment, variant or derivative thereof.). The invention also relates to high resolution three dimensional structures of PDHK in the presence and absence of physiological and synthetic ligands obtained by X-ray diffraction and use of the structures to identify, design or select compounds that bind to PDHK. The invention further relates to compounds identified, designed or selected using high resolution structures of PDHK. Also provided are nucleotide sequences used to obtain crystallisable PDHK protein.

SEQUENCE LISTING (Part of the Description)

<110> Pfizer Ltd. (EP(GB) only), Pfizer Inc. (US, JP, CA, EP except GB)

<120> Crystal structure

<130> PCS10938_BXP

<160> 10

<170> FastSEQ for Windows Version 4.0

<210> 1
<211> 1224
<212> DNA
<213> Homo sapiens

<400> 1
atgcgctggg tgtgggcgct gctgaagaat gcgtccctgg caggggcgcc caagtacata 60
gagcacttca gcaagttctc cccgtccccg ctgtccatga agcagtttct ggacttcgga 120
tccagcaatg cctgtgagaa aacctccttc accttcctca ggcaggagct gcctgtgcgc 180
ctggccaaca tcatgaaaga gatcaacctg cttcccgacc gagtgctgag cacaccctct 240
gtgcagctgg tgcagagctg gtatgtccag agcctcctgg acatcatgga gttcctggac 300
aaggatcccg aggaccatcg caccctgagc cagttcactg acgccctggt caccatccgg 360
aaccggcaca acgacgtggt gcccaccatg gcacaaggcg tgcttgagta caaggacacc 420
tacggcgatg accccgtctc caaccagaac atccagtact tcctggaccg cttctacctc 480
agccgcatct ccatccgcat gctcatcaac cagcacaccc tcatctttga tggcagcacc 540
aacccagccc atcccaaaca catcggcagc atcgacccca actgcaacgt ctctgaggtg 600
gtcaaagatg cctacgacat ggctaagctc ctgtgtgaca agtattacat ggcctcacct 660
gacctggaga tccaggagat caatgcagcc aactccaaac agccgattca catggtctac 720
gtcccctccc acctctacca catgctcttt gagctcttca agaatgccat gagggcgact 780
gtggaaagcc atgagtccag cctcattctc ccacccatca aggtcatggt ggccttgggt 840
gaggaagatc tgtccatcaa gatgagtgac cgaggtgggg gtgttccctt gaggaagatt 900
gagcgactct tcagctacat gtactccaca gcacccaccc cccagcctgg caccggggga 960
acgccgctgg ctggctttgg ttatgggctc cccatttccc gcctctacgc caagtacttc 1020
cagggagacc tgcagctctt ctccatggaa ggctttggga ccgatgctgt catctatctc 1080
aaggccctgt ccacggactc ggtggagcgc ctgcctgtct acaacaagtc agcctggcgc 1140
cactaccaga ccatccagga ggccggcgac tggtgtgtgc ccagcacgga gcccaagaac 1200
acgtccacgt accgcgtcac gtaa 1224

<210> 2
<211> 407
<212> PRT
<213> Homo sapiens

<400> 2
Met Arg Trp Val Trp Ala Leu Leu Lys Asn Ala Ser Leu Ala Gly Ala
1               5                   10                  15
Pro Lys Tyr Ile Glu His Phe Ser Lys Phe Ser Pro Ser Pro Leu Ser
            20                  25                  30
Met Lys Gln Phe Leu Asp Phe Gly Ser Ser Asn Ala Cys Glu Lys Thr
        35                  40                  45
Ser Phe Thr Phe Leu Arg Gln Glu Leu Pro Val Arg Leu Ala Asn Ile
    50                  55                  60
Met Lys Glu Ile Asn Leu Leu Pro Asp Arg Val Leu Ser Thr Pro Ser
65                  70                  75                  80
Val Gln Leu Val Gln Ser Trp Tyr Val Gln Ser Leu Leu Asp Ile Met
                85                  90                  95
Glu Phe Leu Asp Lys Asp Pro Glu Asp His Arg Thr Leu Ser Gln Phe
            100                 105                 110
Thr Asp Ala Leu Val Thr Ile Arg Asn Arg His Asn Asp Val Val Pro
        115                 120                 125
Thr Met Ala Gln Gly Val Leu Glu Tyr Lys Asp Thr Tyr Gly Asp Asp
    130                 135                 140
Pro Val Ser Asn Gln Asn Ile Gln Tyr Phe Leu Asp Arg Phe Tyr Leu
145                 150                 155                 160
Ser Arg Ile Ser Ile Arg Met Leu Ile Asn Gln His Thr Leu Ile Phe
                165                 170                 175
Asp Gly Ser Thr Asn Pro Ala His Pro Lys His Ile Gly Ser Ile Asp
            180                 185                 190

Pro Asn Cys Asn Val Ser Glu Val Val Lys Asp Ala Tyr Asp Met Ala
        195                 200                 205
Lys Leu Leu Cys Asp Lys Tyr Tyr Met Ala Ser Pro Asp Leu Glu Ile
    210                 215                 220
Gln Glu Ile Asn Ala Ala Asn Ser Lys Gln Pro Ile His Met Val Tyr
225                 230                 235                 240
Val Pro Ser His Leu Tyr His Met Leu Phe Glu Leu Phe Lys Asn Ala
                245                 250                 255
Met Arg Ala Thr Val Glu Ser His Glu Ser Ser Leu Ile Leu Pro Pro
            260                 265                 270
Ile Lys Val Met Val Ala Leu Gly Glu Glu Asp Leu Ser Ile Lys Met
        275                 280                 285
Ser Asp Arg Gly Gly Gly Val Pro Leu Arg Lys Ile Glu Arg Leu Phe
    290                 295                 300
Ser Tyr Met Tyr Ser Thr Ala Pro Thr Pro Gln Pro Gly Thr Gly Gly
305                 310                 315                 320
Thr Pro Leu Ala Gly Phe Gly Tyr Gly Leu Pro Ile Ser Arg Leu Tyr
                325                 330                 335
Ala Lys Tyr Phe Gln Gly Asp Leu Gln Leu Phe Ser Met Glu Gly Phe
            340                 345                 350
Gly Thr Asp Ala Val Ile Tyr Leu Lys Ala Leu Ser Thr Asp Ser Val
        355                 360                 365
Glu Arg Leu Pro Val Tyr Asn Lys Ser Ala Trp Arg His Tyr Gln Thr
    370                 375                 380
Ile Gln Glu Ala Gly Asp Trp Cys Val Pro Ser Thr Glu Pro Lys Asn
385                 390                 395                 400
Thr Ser Thr Tyr Arg Val Thr
                405

<210> 3
<211> 1224
<212> DNA
<213> Homo sapiens

<400> 3
atgcatcatc atcatcatca ccttctggtt ccgcgtggat ctgcgcccaa gtacatagag 60
cacttcagca agttctcccc gtccccgctg tccatgaagc agtttctgga cttcggatcc 120
agcaatgcct gtgagaaaac ctccttcacc ttcctcaggc aggagctgcc tgtgcgcctg 180
gccaacatca tgaaagagat caacctgctt cccgaccgag tgctgagcac accctctgtg 240
cagctggtgc agagctggta tgtccagagc ctcctggaca tcatggagtt cctggacaag 300
gatcccgagg accatcgcac cctgagccag ttcactgacg ccctggtcac catccggaac 360
cggcacaacg acgtggtgcc caccatggca caaggcgtgc ttgagtacaa ggacacctac 420
ggcgatgacc ccgtctccaa ccagaacatc cagtacttcc tggaccgctt ctacctcagc 480
cgcatctcca tccgcatgct catcaaccag cacaccctca tctttgatgg cagcaccaac 540
ccagcccatc ccaaacacat cggcagcatc gaccccaact gcaacgtctc tgaggtggtc 600
aaagatgcct acgacatggc taagctcctg tgtgacaagt attacatggc ctcacctgac 660
ctggagatcc aggagatcaa tgcagccaac tccaaacagc cgattcacat ggtctacgtc 720
ccctcccacc tctaccacat gctctttgag ctcttcaaga atgccatgag ggcgactgtg 780
gaaagccatg agtccagcct cattctccca cccatcaagg tcatggtggc cttgggtgag 840
gaagatctgt ccatcaagat gagtgaccga ggtgggggtg ttcccttgag gaagattgag 900
cgactcttca gctacatgta ctccacagca cccacccccc agcctggcac cgggggaacg 960
ccgctggctg gctttggtta tgggctcccc atttcccgcc tctacgccaa gtacttccag 1020
ggagacctgc agctcttctc catggaaggc tttgggaccg atgctgtcat ctatctcaag 1080
gccctgtcca cggactcggt ggagcgcctg cctgtctaca acaagtcagc ctggcgccac 1140
taccagacca tccaggaggc cggcgactgg tgtgtgccca gcacggagcc caagaacacg 1200
tccacgtacc gcgtcacgtg atag 1224

<210> 4
<211> 406
<212> PRT
<213> Homo sapiens

<220>
<223> Hexa-histidine tag

<223> Thrombin cleavage site

<400> 4
Met His His His His His His Leu Leu Val Pro Arg Gly Ser Ala Pro
1               5                   10                  15
Lys Tyr Ile Glu His Phe Ser Lys Phe Ser Pro Ser Pro Leu Ser Met

                20                  25                  30
Lys Gln Phe Leu Asp Phe Gly Ser Ser Asn Ala Cys Glu Lys Thr Ser
            35                  40                  45
Phe Thr Phe Leu Arg Gln Glu Leu Pro Val Arg Leu Ala Asn Ile Met
    50                  55                  60
Lys Glu Ile Asn Leu Leu Pro Asp Arg Val Leu Ser Thr Pro Ser Val
65                  70                  75                  80
Gln Leu Val Gln Ser Trp Tyr Val Gln Ser Leu Leu Asp Ile Met Glu
                85                  90                  95
Phe Leu Asp Lys Asp Pro Glu Asp His Arg Thr Leu Ser Gln Phe Thr
            100                 105                 110
Asp Ala Leu Val Thr Ile Arg Asn Arg His Asn Asp Val Val Pro Thr
        115                 120                 125
Met Ala Gln Gly Val Leu Glu Tyr Lys Asp Thr Tyr Gly Asp Asp Pro
    130                 135                 140
Val Ser Asn Gln Asn Ile Gln Tyr Phe Leu Asp Arg Phe Tyr Leu Ser
145                 150                 155                 160
Arg Ile Ser Ile Arg Met Leu Ile Asn Gln His Thr Leu Ile Phe Asp
                165                 170                 175
Gly Ser Thr Asn Pro Ala His Pro Lys His Ile Gly Ser Ile Asp Pro
            180                 185                 190
Asn Cys Asn Val Ser Glu Val Val Lys Asp Ala Tyr Asp Met Ala Lys
        195                 200                 205
Leu Leu Cys Asp Lys Tyr Tyr Met Ala Ser Pro Asp Leu Glu Ile Gln
    210                 215                 220
Glu Ile Asn Ala Ala Asn Ser Lys Gln Pro Ile His Met Val Tyr Val
225                 230                 235                 240
Pro Ser His Leu Tyr His Met Leu Phe Glu Leu Phe Lys Asn Ala Met
                245                 250                 255
Arg Ala Thr Val Glu Ser His Glu Ser Ser Leu Ile Leu Pro Pro Ile
            260                 265                 270
Lys Val Met Val Ala Leu Gly Glu Glu Asp Leu Ser Ile Lys Met Ser
        275                 280                 285
Asp Arg Gly Gly Gly Val Pro Leu Arg Lys Ile Glu Arg Leu Phe Ser
    290                 295                 300
Tyr Met Tyr Ser Thr Ala Pro Thr Pro Gln Pro Gly Thr Gly Gly Thr
305                 310                 315                 320
Pro Leu Ala Gly Phe Gly Tyr Gly Leu Pro Ile Ser Arg Leu Tyr Ala
                325                 330                 335
Lys Tyr Phe Gln Gly Asp Leu Gln Leu Phe Ser Met Glu Gly Phe Gly
            340                 345                 350
Thr Asp Ala Val Ile Tyr Leu Lys Ala Leu Ser Thr Asp Ser Val Glu
        355                 360                 365
Arg Leu Pro Val Tyr Asn Lys Ser Ala Trp Arg His Tyr Gln Thr Ile
    370                 375                 380
Gln Glu Ala Gly Asp Trp Cys Val Pro Ser Thr Glu Pro Lys Asn Thr
385                 390                 395                 400
Ser Thr Tyr Arg Val Thr
                405

<210> 5
<211> 394
<212> PRT
<213> Homo sapiens

<400> 5
Gly Ser Ala Pro Lys Tyr Ile Glu His Phe Ser Lys Phe Ser Pro Ser
 1               5                  10                  15

Pro Leu Ser Met Lys Gln Phe Leu Asp Phe Gly Ser Ser Asn Ala Cys
            20                  25                  30
Glu Lys Thr Ser Phe Thr Phe Leu Arg Gln Glu Leu Pro Val Arg Leu
        35                  40                  45
Ala Asn Ile Met Lys Glu Ile Asn Leu Leu Pro Asp Arg Val Leu Ser
    50                  55                  60
Thr Pro Ser Val Gln Leu Val Gln Ser Trp Tyr Val Gln Ser Leu Leu
65                  70                  75                  80
Asp Ile Met Glu Phe Leu Asp Lys Asp Pro Glu Asp His Arg Thr Leu
                85                  90                  95
Ser Gln Phe Thr Asp Ala Leu Val Thr Ile Arg Asn Arg His Asn Asp
            100                 105                 110

Val Val Pro Thr Met Ala Gln Gly Val Leu Glu Tyr Lys Asp Thr Tyr
        115                 120                 125
Gly Asp Asp Pro Val Ser Asn Gln Asn Ile Gln Tyr Phe Leu Asp Arg
    130                 135                 140
Phe Tyr Leu Ser Arg Ile Ser Ile Arg Met Leu Ile Asn Gln His Thr
145                 150                 155                 160
Leu Ile Phe Asp Gly Ser Thr Asn Pro Ala His Pro Lys His Ile Gly
                165                 170                 175
Ser Ile Asp Pro Asn Cys Asn Val Ser Glu Val Val Lys Asp Ala Tyr
            180                 185                 190
Asp Met Ala Lys Leu Leu Cys Asp Lys Tyr Tyr Met Ala Ser Pro Asp
        195                 200                 205
Leu Glu Ile Gln Glu Ile Asn Ala Ala Asn Ser Lys Gln Pro Ile His
    210                 215                 220
Met Val Tyr Val Pro Ser His Leu Tyr His Met Leu Phe Glu Leu Phe
225                 230                 235                 240
Lys Asn Ala Met Arg Ala Thr Val Glu Ser His Glu Ser Ser Leu Ile
                245                 250                 255
Leu Pro Pro Ile Lys Val Met Val Ala Leu Gly Glu Glu Asp Leu Ser
            260                 265                 270
Ile Lys Met Ser Asp Arg Gly Gly Gly Val Pro Leu Arg Lys Ile Glu
        275                 280                 285
Arg Leu Phe Ser Tyr Met Tyr Ser Thr Ala Pro Thr Pro Gln Pro Gly
    290                 295                 300
Thr Gly Gly Thr Pro Leu Ala Gly Phe Gly Tyr Gly Leu Pro Ile Ser
305                 310                 315                 320
Arg Leu Tyr Ala Lys Tyr Phe Gln Gly Asp Leu Gln Leu Phe Ser Met
                325                 330                 335
Glu Gly Phe Gly Thr Asp Ala Val Ile Tyr Leu Lys Ala Leu Ser Thr
            340                 345                 350
Asp Ser Val Glu Arg Leu Pro Val Tyr Asn Lys Ser Ala Trp Arg His
        355                 360                 365
Tyr Gln Thr Ile Gln Glu Ala Gly Asp Trp Cys Val Pro Ser Thr Glu
    370                 375                 380
Pro Lys Asn Thr Ser Thr Tyr Arg Val Thr
385                 390

<210> 6
<211> 75
<212> DNA
<213> Homo sapiens

<400> 6
cgtgaattca tgcatcatca tcatcatcac cttctggttc cgcgtggatc tgcgcccaag 60
tacatagagc acttc                                                   75

<210> 7
<211> 35
<212> DNA
<213> Homo sapiens

<400> 7
cgttctagac tatcacgtga cgcggtacgt cgacg

<210> 8
<211> 1188
<212> DNA
<213> Homo sapiens

<400> 8
ggatctgcgc ccaagtacat agagcacttc agcaagttct ccccgtcccc gctgtccatg 60
aagcagtttc tggacttcgg atccagcaat gcctgtgaga aaacctcctt caccttcctc 120
aggcaggagc tgcctgtgcg cctggccaac atcatgaaag agatcaacct gcttcccgac 180
cgagtgctga gcacaccctc tgtgcagctg gtgcagagct ggtatgtcca gagcctcctg 240
gacatcatgg agttcctgga caaggatccc gaggaccatc gcaccctgag ccagttcact 300
gacgccctgg tcaccatccg gaaccggcac aacgacgtgg tgcccaccat ggcacaaggc 360
gtgcttgagt acaaggacac ctacggcgat gaccccgtct ccaaccagaa catccagtac 420
ttcctggacc gcttctacct cagccgcatc tccatccgca tgctcatcaa ccagcacacc 480
ctcatctttg atggcagcac caacccagcc catcccaaac acatcggcag catcgacccc 540
aactgcaacg tctctgaggt ggtcaaagat gcctacgaca tggctaagct cctgtgtgac 600

aagtattaca tggcctcacc tgacctggag atccaggaga tcaatgcagc caactccaaa 660
cagccgattc acatggtcta cgtcccctcc cacctctacc acatgctctt tgagctcttc 720
aagaatgcca tgagggcgac tgtggaaagc catgagtcca gcctcattct cccacccatc 780
aaggtcatgg tggccttggg tgaggaagat ctgtccatca agatgagtga ccgaggtggg 840
ggtgttccct tgaggaagat tgagcgactc ttcagctaca tgtactccac agcacccacc 900
ccccagcctg gcaccggggg aacgccgctg gctggctttg gttatgggct ccccatttcc 960
cgcctctacg ccaagtactt ccagggagac ctgcagctct tctccatgga aggctttggg 1020
accgatgctg tcatctatct caaggccctg tccacggact cggtggagcg cctgcctgtc 1080
tacaacaagt cagcctggcg ccactaccag accatccagg aggccggcga ctggtgtgtg 1140
cccagcacgg agcccaagaa cacgtccacg taccgcgtca cgtgatag 1188

<210> 9
<211> 1182
<212> DNA
<213> Homo sapiens

<400> 9
gcgcccaagt acatagagca cttcagcaag ttctccccgt ccccgctgtc catgaagcag 60
tttctggact tcggatccag caatgcctgt gagaaaacct ccttcacctt cctcaggcag 120
gagctgcctg tgcgcctggc caacatcatg aaagagatca acctgcttcc cgaccgagtg 180
ctgagcacac cctctgtgca gctggtgcag agctggtatg tccagagcct cctggacatc 240
atggagttcc tggacaagga tcccgaggac catcgcaccc tgagccagtt cactgacgcc 300
ctggtcacca tccggaaccg gcacaacgac gtggtgccca ccatggcaca aggcgtgctt 360
gagtacaagg acacctacgg cgatgacccc gtctccaacc agaacatcca gtacttcctg 420
gaccgcttct acctcagccg catctccatc cgcatgctca tcaaccagca caccctcatc 480
tttgatggca gcaccaaccc agcccatccc aaacacatcg gcagcatcga ccccaactgc 540
aacgtctctg aggtggtcaa agatgcctac gacatggcta agctcctgtg tgacaagtat 600
tacatggcct cacctgacct ggagatccag gagatcaatg cagccaactc caaacagccg 660
attcacatgg tctacgtccc ctcccacctc taccacatgc tctttgagct cttcaagaat 720
gccatgaggg cgactgtgga aagccatgag tccagcctca ttctcccacc catcaaggtc 780
atggtggcct tgggtgagga agatctgtcc atcaagatga gtgaccgagg tgggggtgtt 840
cccttgagga agattgagcg actcttcagc tacatgtact ccacagcacc caccccccag 900
cctggcaccg ggggaacgcc gctggctggc tttggttatg ggctccccat ttcccgcctc 960
tacgccaagt acttccaggg agacctgcag ctcttctcca tggaaggctt tgggaccgat 1020
gctgtcatct atctcaaggc cctgtccacg gactcggtgg agcgcctgcc tgtctacaac 1080
aagtcagcct ggcgccacta ccagaccatc caggaggccg gcgactggtg tgtgcccagc 1140
acggagccca agaacacgtc cacgtaccgc gtcacgtgat ag 1182

<210> 10
<211> 392
<212> PRT
<213> Homo sapiens

<400> 10
Ala Pro Lys Tyr Ile Glu His Phe Ser Lys Phe Ser Pro Ser Pro Leu
1               5                   10                  15
Ser Met Lys Gln Phe Leu Asp Phe Gly Ser Ser Asn Ala Cys Glu Lys
            20                  25                  30
Thr Ser Phe Thr Phe Leu Arg Gln Glu Leu Pro Val Arg Leu Ala Asn
        35                  40                  45
Ile Met Lys Glu Ile Asn Leu Leu Pro Asp Arg Val Leu Ser Thr Pro
    50                  55                  60
Ser Val Gln Leu Val Gln Ser Trp Tyr Val Gln Ser Leu Leu Asp Ile
65                  70                  75                  80
Met Glu Phe Leu Asp Lys Asp Pro Glu Asp His Arg Thr Leu Ser Gln
                85                  90                  95
Phe Thr Asp Ala Leu Val Thr Ile Arg Asn Arg His Asn Asp Val Val
            100                 105                 110
Pro Thr Met Ala Gln Gly Val Leu Glu Tyr Lys Asp Thr Tyr Gly Asp
        115                 120                 125
Asp Pro Val Ser Asn Gln Asn Ile Gln Tyr Phe Leu Asp Arg Phe Tyr
    130                 135                 140
Leu Ser Arg Ile Ser Ile Arg Met Leu Ile Asn Gln His Thr Leu Ile
145                 150                 155                 160
Phe Asp Gly Ser Thr Asn Pro Ala His Pro Lys His Ile Gly Ser Ile
                165                 170                 175
Asp Pro Asn Cys Asn Val Ser Glu Val Val Lys Asp Ala Tyr Asp Met
            180                 185                 190
Ala Lys Leu Leu Cys Asp Lys Tyr Tyr Met Ala Ser Pro Asp Leu Glu
        195                 200                 205
Ile Gln Glu Ile Asn Ala Ala Asn Ser Lys Gln Pro Ile His Met Val
    210                 215                 220

Tyr Val Pro Ser His Leu Tyr His Met Leu Phe Glu Leu Phe Lys Asn
225                 230                 235                 240
Ala Met Arg Ala Thr Val Glu Ser His Glu Ser Ser Leu Ile Leu Pro
                245                 250                 255
Pro Ile Lys Val Met Val Ala Leu Gly Glu Glu Asp Leu Ser Ile Lys
            260                 265                 270
Met Ser Asp Arg Gly Gly Gly Val Pro Leu Arg Lys Ile Glu Arg Leu
        275                 280                 285
Phe Ser Tyr Met Tyr Ser Thr Ala Pro Thr Pro Gln Pro Gly Thr Gly
    290                 295                 300
Gly Thr Pro Leu Ala Gly Phe Gly Tyr Gly Leu Pro Ile Ser Arg Leu
305                 310                 315                 320
Tyr Ala Lys Tyr Phe Gln Gly Asp Leu Gln Leu Phe Ser Met Glu Gly
                325                 330                 335
Phe Gly Thr Asp Ala Val Ile Tyr Leu Lys Ala Leu Ser Thr Asp Ser
            340                 345                 350
Val Glu Arg Leu Pro Val Tyr Asn Lys Ser Ala Trp Arg His Tyr Gln
        355                 360                 365
Thr Ile Gln Glu Ala Gly Asp Trp Cys Val Pro Ser Thr Glu Pro Lys
    370                 375                 380
Asn Thr Ser Thr Tyr Arg Val Thr
385                 390

35

SEQUENCE LISTING

<110> Pfizer Ltd. (EP (GB) only), Pfizer Inc (US, JP, CA, EP exce
pt GB)

<120> Crystal structure

<130> PCS10938_RFB

<160> 10

<170> FastSEQ for Windows Version 4.0

<210> 1
<211> 1224
<212> DNA
<213> Homo sapiens

<400> 1
atgcgctggg tgtgggcgct gctgaagaat gcgtccctgg caggggcgcc caagtacata
 60
gagcacttca gcaagttctc cccgtccccg ctgtccatga agcagtttct ggacttcgga
 120
tccagcaatg cctgtgagaa aacctccttc accttcctca ggcaggagct gcctgtgcgc
 180
ctggccaaca tcatgaaaga gatcaacctg cttcccgacc gagtgctgag cacaccctct
 240
gtgcagctgg tgcagagctg gtatgtccag agcctcctgg acatcatgga gttcctggac
 300
aaggatcccg aggaccatcg caccctgagc cagttcactg acgccctggt caccatccgg
 360
aaccggcaca acgacgtggt gcccaccatg gcacaaggcg tgcttgagta caaggacacc
 420
tacggcgatg accccgtctc caaccagaac atccagtact tcctggaccg cttctacctc
 480
agccgcatct ccatccgcat gctcatcaac cagcacaccc tcatctttga tggcagcacc
 540
aacccagccc atcccaaaca catcggcagc atcgacccca actgcaacgt ctctgaggtg
 600
gtcaaagatg cctacgacat ggctaagctc ctgtgtgaca agtattacat ggcctcacct
 660
gacctggaga tccaggagat caatgcagcc aactccaaac agccgattca catggtctac
 720
gtcccctccc acctctacca catgctcttt gagctcttca agaatgccat gagggcgact
 780
gtggaaagcc atgagtccag cctcattctc ccacccatca aggtcatggt ggccttgggt
 840
gaggaagatc tgtccatcaa gatgagtgac cgaggtgggg gtgttccctt gaggaagatt
 900
gagcgactct tcagctacat gtactccaca gcacccaccc cccagcctgg caccggggga

960
acgccgctgg ctggctttgg ttatgggctc cccatttccc gcctctacgc caagtacttc
1020
cagggagacc tgcagctctt ctccatggaa ggctttggga ccgatgctgt catctatctc
1080
aaggccctgt ccacggactc ggtggagcgc ctgcctgtct acaacaagtc agcctggcgc
1140
cactaccaga ccatccagga ggccggcgac tggtgtgtgc ccagcacgga gcccaagaac
1200
acgtccacgt accgcgtcac gtaa
1224

<210> 2
<211> 407
<212> PRT
<213> Homo sapiens

<400> 2
Met Arg Trp Val Trp Ala Leu Leu Lys Asn Ala Ser Leu Ala Gly Ala
1               5                   10                  15
Pro Lys Tyr Ile Glu His Phe Ser Lys Phe Ser Pro Ser Pro Leu Ser
            20                  25                  30
Met Lys Gln Phe Leu Asp Phe Gly Ser Ser Asn Ala Cys Glu Lys Thr
        35                  40                  45
Ser Phe Thr Phe Leu Arg Gln Glu Leu Pro Val Arg Leu Ala Asn Ile
    50                  55                  60
Met Lys Glu Ile Asn Leu Leu Pro Asp Arg Val Leu Ser Thr Pro Ser
65                  70                  75                  80
Val Gln Leu Val Gln Ser Trp Tyr Val Gln Ser Leu Leu Asp Ile Met
                85                  90                  95
Glu Phe Leu Asp Lys Asp Pro Glu Asp His Arg Thr Leu Ser Gln Phe
            100                 105                 110
Thr Asp Ala Leu Val Thr Ile Arg Asn Arg His Asn Asp Val Val Pro
        115                 120                 125
Thr Met Ala Gln Gly Val Leu Glu Tyr Lys Asp Thr Tyr Gly Asp Asp
    130                 135                 140
Pro Val Ser Asn Gln Asn Ile Gln Tyr Phe Leu Asp Arg Phe Tyr Leu
145                 150                 155                 160
Ser Arg Ile Ser Ile Arg Met Leu Ile Asn Gln His Thr Leu Ile Phe
                165                 170                 175
Asp Gly Ser Thr Asn Pro Ala His Pro Lys His Ile Gly Ser Ile Asp
            180                 185                 190
Pro Asn Cys Asn Val Ser Glu Val Val Lys Asp Ala Tyr Asp Met Ala
        195                 200                 205
Lys Leu Leu Cys Asp Lys Tyr Tyr Met Ala Ser Pro Asp Leu Glu Ile
    210                 215                 220
Gln Glu Ile Asn Ala Ala Asn Ser Lys Gln Pro Ile His Met Val Tyr
225                 230                 235                 240
Val Pro Ser His Leu Tyr His Met Leu Phe Glu Leu Phe Lys Asn Ala
                245                 250                 255
Met Arg Ala Thr Val Glu Ser His Glu Ser Ser Leu Ile Leu Pro Pro
            260                 265                 270

Ile Lys Val Met Val Ala Leu Gly Glu Glu Asp Leu Ser Ile Lys Met
        275                 280                 285
Ser Asp Arg Gly Gly Gly Val Pro Leu Arg Lys Ile Glu Arg Leu Phe
    290                 295                 300
Ser Tyr Met Tyr Ser Thr Ala Pro Thr Pro Gln Pro Gly Thr Gly Gly
305                 310                 315                 320
Thr Pro Leu Ala Gly Phe Gly Tyr Gly Leu Pro Ile Ser Arg Leu Tyr
                325                 330                 335
Ala Lys Tyr Phe Gln Gly Asp Leu Gln Leu Phe Ser Met Glu Gly Phe
            340                 345                 350
Gly Thr Asp Ala Val Ile Tyr Leu Lys Ala Leu Ser Thr Asp Ser Val
        355                 360                 365
Glu Arg Leu Pro Val Tyr Asn Lys Ser Ala Trp Arg His Tyr Gln Thr
    370                 375                 380
Ile Gln Glu Ala Gly Asp Trp Cys Val Pro Ser Thr Glu Pro Lys Asn
385                 390                 395                 400
Thr Ser Thr Tyr Arg Val Thr
                405

<210> 3
<211> 1224
<212> DNA
<213> Homo sapiens

<400> 3
atgcatcatc atcatcatca ccttctggtt ccgcgtggat ctgcgcccaa gtacatagag
 60
cacttcagca agttctcccc gtccccgctg tccatgaagc agtttctgga cttcggatcc
 120
agcaatgcct gtgagaaaac ctccttcacc ttcctcaggc aggagctgcc tgtgcgcctg
 180
gccaacatca tgaaagagat caacctgctt cccgaccgag tgctgagcac accctctgtg
 240
cagctggtgc agagctggta tgtccagagc ctcctggaca tcatggagtt cctggacaag
 300
gatcccgagg accatcgcac cctgagccag ttcactgacg ccctggtcac catccggaac
 360
cggcacaacg acgtggtgcc caccatggca caaggcgtgc ttgagtacaa ggacacctac
 420
ggcgatgacc ccgtctccaa ccagaacatc cagtacttcc tggaccgctt ctacctcagc
 480
cgcatctcca tccgcatgct catcaaccag cacaccctca tctttgatgg cagcaccaac
 540
ccagcccatc ccaaacacat cggcagcatc gaccccaact gcaacgtctc tgaggtggtc
 600
aaagatgcct acgacatggc taagctcctg tgtgacaagt attacatggc ctcacctgac
 660
ctggagatcc aggagatcaa tgcagccaac tccaaacagc cgattcacat ggtctacgtc
 720
ccctcccacc tctaccacat gctctttgag ctcttcaaga atgccatgag ggcgactgtg
 780

gaaagccatg agtccagcct cattctccca cccatcaagg tcatggtggc cttgggtgag
 840
gaagatctgt ccatcaagat gagtgaccga ggtgggggtg ttcccttgag gaagattgag
 900
cgactcttca gctacatgta ctccacagca cccacccccc agcctggcac cgggggaacg
 960
ccgctggctg gctttggtta tgggctcccc atttcccgcc tctacgccaa gtacttccag
 1020
ggagacctgc agctcttctc catggaaggc tttgggaccg atgctgtcat ctatctcaag
 1080
gccctgtcca cggactcggt ggagcgcctg cctgtctaca acaagtcagc ctggcgccac
 1140
taccagacca tccaggaggc cggcgactgg tgtgtgccca gcacggagcc caagaacacg
 1200
tccacgtacc gcgtcacgtg atag
 1224

<210> 4
<211> 406
<212> PRT
<213> Homo sapiens

<220>
<223> Hexa-histidine tag

<223> Thrombin cleavage site

<400> 4
Met His His His His His His Leu Leu Val Pro Arg Gly Ser Ala Pro
 1               5                   10                  15
Lys Tyr Ile Glu His Phe Ser Lys Phe Ser Pro Ser Pro Leu Ser Met
            20                  25                  30
Lys Gln Phe Leu Asp Phe Gly Ser Ser Asn Ala Cys Glu Lys Thr Ser
        35                  40                  45
Phe Thr Phe Leu Arg Gln Glu Leu Pro Val Arg Leu Ala Asn Ile Met
    50                  55                  60
Lys Glu Ile Asn Leu Leu Pro Asp Arg Val Leu Ser Thr Pro Ser Val
65                  70                  75                  80
Gln Leu Val Gln Ser Trp Tyr Val Gln Ser Leu Leu Asp Ile Met Glu
                85                  90                  95
Phe Leu Asp Lys Asp Pro Glu Asp His Arg Thr Leu Ser Gln Phe Thr
            100                 105                 110
Asp Ala Leu Val Thr Ile Arg Asn Arg His Asn Asp Val Val Pro Thr
        115                 120                 125
Met Ala Gln Gly Val Leu Glu Tyr Lys Asp Thr Tyr Gly Asp Asp Pro
    130                 135                 140
Val Ser Asn Gln Asn Ile Gln Tyr Phe Leu Asp Arg Phe Tyr Leu Ser
145                 150                 155                 160
Arg Ile Ser Ile Arg Met Leu Ile Asn Gln His Thr Leu Ile Phe Asp
                165                 170                 175
Gly Ser Thr Asn Pro Ala His Pro Lys His Ile Gly Ser Ile Asp Pro
            180                 185                 190

Asn Cys Asn Val Ser Glu Val Val Lys Asp Ala Tyr Asp Met Ala Lys
        195                 200                 205
Leu Leu Cys Asp Lys Tyr Tyr Met Ala Ser Pro Asp Leu Glu Ile Gln
    210                 215                 220
Glu Ile Asn Ala Ala Asn Ser Lys Gln Pro Ile His Met Val Tyr Val
225                 230                 235                 240
Pro Ser His Leu Tyr His Met Leu Phe Glu Leu Phe Lys Asn Ala Met
                245                 250                 255
Arg Ala Thr Val Glu Ser His Glu Ser Ser Leu Ile Leu Pro Pro Ile
            260                 265                 270
Lys Val Met Val Ala Leu Gly Glu Glu Asp Leu Ser Ile Lys Met Ser
        275                 280                 285
Asp Arg Gly Gly Gly Val Pro Leu Arg Lys Ile Glu Arg Leu Phe Ser
    290                 295                 300
Tyr Met Tyr Ser Thr Ala Pro Thr Pro Gln Pro Gly Thr Gly Gly Thr
305                 310                 315                 320
Pro Leu Ala Gly Phe Gly Tyr Gly Leu Pro Ile Ser Arg Leu Tyr Ala
                325                 330                 335
Lys Tyr Phe Gln Gly Asp Leu Gln Leu Phe Ser Met Glu Gly Phe Gly
            340                 345                 350
Thr Asp Ala Val Ile Tyr Leu Lys Ala Leu Ser Thr Asp Ser Val Glu
        355                 360                 365
Arg Leu Pro Val Tyr Asn Lys Ser Ala Trp Arg His Tyr Gln Thr Ile
    370                 375                 380
Gln Glu Ala Gly Asp Trp Cys Val Pro Ser Thr Glu Pro Lys Asn Thr
385                 390                 395                 400
Ser Thr Tyr Arg Val Thr
                405

<210> 5
<211> 394
<212> PRT
<213> Homo sapiens

<400> 5
Gly Ser Ala Pro Lys Tyr Ile Glu His Phe Ser Lys Phe Ser Pro Ser
 1               5                  10                  15
Pro Leu Ser Met Lys Gln Phe Leu Asp Phe Gly Ser Ser Asn Ala Cys
            20                  25                  30
Glu Lys Thr Ser Phe Thr Phe Leu Arg Gln Glu Leu Pro Val Arg Leu
        35                  40                  45
Ala Asn Ile Met Lys Glu Ile Asn Leu Leu Pro Asp Arg Val Leu Ser
    50                  55                  60
Thr Pro Ser Val Gln Leu Val Gln Ser Trp Tyr Val Gln Ser Leu Leu
65                  70                  75                  80
Asp Ile Met Glu Phe Leu Asp Lys Asp Pro Glu Asp His Arg Thr Leu
                85                  90                  95
Ser Gln Phe Thr Asp Ala Leu Val Thr Ile Arg Asn Arg His Asn Asp
            100                 105                 110
Val Val Pro Thr Met Ala Gln Gly Val Leu Glu Tyr Lys Asp Thr Tyr
        115                 120                 125

Gly Asp Asp Pro Val Ser Asn Gln Asn Ile Gln Tyr Phe Leu Asp Arg
    130                 135                 140
Phe Tyr Leu Ser Arg Ile Ser Ile Arg Met Leu Ile Asn Gln His Thr
145                 150                 155                 160
Leu Ile Phe Asp Gly Ser Thr Asn Pro Ala His Pro Lys His Ile Gly
                165                 170                 175
Ser Ile Asp Pro Asn Cys Asn Val Ser Glu Val Val Lys Asp Ala Tyr
            180                 185                 190
Asp Met Ala Lys Leu Leu Cys Asp Lys Tyr Tyr Met Ala Ser Pro Asp
        195                 200                 205
Leu Glu Ile Gln Glu Ile Asn Ala Ala Asn Ser Lys Gln Pro Ile His
    210                 215                 220
Met Val Tyr Val Pro Ser His Leu Tyr His Met Leu Phe Glu Leu Phe
225                 230                 235                 240
Lys Asn Ala Met Arg Ala Thr Val Glu Ser His Glu Ser Ser Leu Ile
                245                 250                 255
Leu Pro Pro Ile Lys Val Met Val Ala Leu Gly Glu Glu Asp Leu Ser
            260                 265                 270
Ile Lys Met Ser Asp Arg Gly Gly Gly Val Pro Leu Arg Lys Ile Glu
        275                 280                 285
Arg Leu Phe Ser Tyr Met Tyr Ser Thr Ala Pro Thr Pro Gln Pro Gly
    290                 295                 300
Thr Gly Gly Thr Pro Leu Ala Gly Phe Gly Tyr Gly Leu Pro Ile Ser
305                 310                 315                 320
Arg Leu Tyr Ala Lys Tyr Phe Gln Gly Asp Leu Gln Leu Phe Ser Met
                325                 330                 335
Glu Gly Phe Gly Thr Asp Ala Val Ile Tyr Leu Lys Ala Leu Ser Thr
            340                 345                 350
Asp Ser Val Glu Arg Leu Pro Val Tyr Asn Lys Ser Ala Trp Arg His
        355                 360                 365
Tyr Gln Thr Ile Gln Glu Ala Gly Asp Trp Cys Val Pro Ser Thr Glu
    370                 375                 380
Pro Lys Asn Thr Ser Thr Tyr Arg Val Thr
385                 390

<210> 6
<211> 75
<212> DNA
<213> Homo sapiens

<400> 6
cgtgaattca tgcatcatca tcatcatcac cttctggttc cgcgtggatc tgcgcccaag
 60
tacatagagc acttc
 75

<210> 7
<211> 35
<212> DNA
<213> Homo sapiens

<400> 7
cgttctagac tatcacgtga cgcggtacgt cgacg
 35

<210> 8
<211> 1188
<212> DNA
<213> Homo sapiens

<400> 8
ggatctgcgc ccaagtacat agagcacttc agcaagttct ccccgtcccc gctgtccatg
 60
aagcagtttc tggacttcgg atccagcaat gcctgtgaga aaacctcctt caccttcctc
 120
aggcaggagc tgcctgtgcg cctggccaac atcatgaaag agatcaacct gcttcccgac
 180
cgagtgctga gcacaccctc tgtgcagctg gtgcagagct ggtatgtcca gagcctcctg
 240
gacatcatgg agttcctgga caaggatccc gaggaccatc gcaccctgag ccagttcact
 300
gacgccctgg tcaccatccg gaaccggcac aacgacgtgg tgcccaccat ggcacaaggc
 360
gtgcttgagt acaaggacac ctacggcgat gaccccgtct ccaaccagaa catccagtac
 420
ttcctggacc gcttctacct cagccgcatc tccatccgca tgctcatcaa ccagcacacc
 480
ctcatctttg atggcagcac caacccagcc catcccaaac acatcggcag catcgacccc
 540
aactgcaacg tctctgaggt ggtcaaagat gcctacgaca tggctaagct cctgtgtgac
 600
aagtattaca tggcctcacc tgacctggag atccaggaga tcaatgcagc caactccaaa
 660
cagccgattc acatggtcta cgtcccctcc cacctctacc acatgctctt tgagctcttc
 720
aagaatgcca tgagggcgac tgtggaaagc catgagtcca gcctcattct cccacccatc
 780
aaggtcatgg tggccttggg tgaggaagat ctgtccatca agatgagtga ccgaggtggg
 840
ggtgttccct tgaggaagat tgagcgactc ttcagctaca tgtactccac agcacccacc
 900
ccccagcctg gcaccggggg aacgccgctg gctggctttg gttatgggct ccccatttcc
 960
cgcctctacg ccaagtactt ccagggagac ctgcagctct tctccatgga aggctttggg
 1020
accgatgctg tcatctatct caaggccctg tccacggact cggtggagcg cctgcctgtc
 1080
tacaacaagt cagcctggcg ccactaccag accatccagg aggccggcga ctggtgtgtg
 1140
cccagcacgg agcccaagaa cacgtccacg taccgcgtca cgtgatag
 1188

<210> 9

<211> 1182
<212> DNA
<213> Homo sapiens

<400> 9
   gcgcccaagt acatagagca cttcagcaag ttctccccgt ccccgctgtc catgaagcag
    60
   tttctggact tcggatccag caatgcctgt gagaaaacct ccttcacctt cctcaggcag
    120
   gagctgcctg tgcgcctggc caacatcatg aaagagatca acctgcttcc cgaccgagtg
    180
   ctgagcacac cctctgtgca gctggtgcag agctggtatg tccagagcct cctggacatc
    240
   atggagttcc tggacaagga tcccgaggac catcgcaccc tgagccagtt cactgacgcc
    300
   ctggtcacca tccggaaccg gcacaacgac gtggtgccca ccatggcaca aggcgtgctt
    360
   gagtacaagg acacctacgg cgatgacccc gtctccaacc agaacatcca gtacttcctg
    420
   gaccgcttct acctcagccg catctccatc cgcatgctca tcaaccagca caccctcatc
    480
   tttgatggca gcaccaaccc agcccatccc aaacacatcg gcagcatcga ccccaactgc
    540
   aacgtctctg aggtggtcaa agatgcctac gacatggcta agctcctgtg tgacaagtat
    600
   tacatggcct cacctgacct ggagatccag gagatcaatg cagccaactc caaacagccg
    660
   attcacatgg tctacgtccc ctcccacctc taccacatgc tctttgagct cttcaagaat
    720
   gccatgaggg cgactgtgga aagccatgag tccagcctca ttctcccacc catcaaggtc
    780
   atggtggcct tgggtgagga agatctgtcc atcaagatga gtgaccgagg tgggggtgtt
    840
   cccttgagga agattgagcg actcttcagc tacatgtact ccacagcacc caccccccag
    900
   cctggcaccg ggggaacgcc gctggctggc tttggttatg ggctccccat ttcccgcctc
    960
   tacgccaagt acttccaggg agacctgcag ctcttctcca tggaaggctt tgggaccgat
    1020
   gctgtcatct atctcaaggc cctgtccacg gactcggtgg agcgcctgcc tgtctacaac
    1080
   aagtcagcct ggcgccacta ccagaccatc caggaggccg gcgactggtg tgtgcccagc
    1140
   acggagccca agaacacgtc cacgtaccgc gtcacgtgat ag
    1182

<210> 10
<211> 392
<212> PRT
<213> Homo sapiens

<400> 10

Ala Pro Lys Tyr Ile Glu His Phe Ser Lys Phe Ser Pro Ser Pro Leu
1               5                   10                  15
Ser Met Lys Gln Phe Leu Asp Phe Gly Ser Ser Asn Ala Cys Glu Lys
            20                  25                  30
Thr Ser Phe Thr Phe Leu Arg Gln Glu Leu Pro Val Arg Leu Ala Asn
        35                  40                  45
Ile Met Lys Glu Ile Asn Leu Leu Pro Asp Arg Val Leu Ser Thr Pro
    50                  55                  60
Ser Val Gln Leu Val Gln Ser Trp Tyr Val Gln Ser Leu Leu Asp Ile
65                  70                  75                  80
Met Glu Phe Leu Asp Lys Asp Pro Glu Asp His Arg Thr Leu Ser Gln
                85                  90                  95
Phe Thr Asp Ala Leu Val Thr Ile Arg Asn Arg His Asn Asp Val Val
            100                 105                 110
Pro Thr Met Ala Gln Gly Val Leu Glu Tyr Lys Asp Thr Tyr Gly Asp
        115                 120                 125
Asp Pro Val Ser Asn Gln Asn Ile Gln Tyr Phe Leu Asp Arg Phe Tyr
    130                 135                 140
Leu Ser Arg Ile Ser Ile Arg Met Leu Ile Asn Gln His Thr Leu Ile
145                 150                 155                 160
Phe Asp Gly Ser Thr Asn Pro Ala His Pro Lys His Ile Gly Ser Ile
                165                 170                 175
Asp Pro Asn Cys Asn Val Ser Glu Val Val Lys Asp Ala Tyr Asp Met
            180                 185                 190
Ala Lys Leu Leu Cys Asp Lys Tyr Tyr Met Ala Ser Pro Asp Leu Glu
        195                 200                 205
Ile Gln Glu Ile Asn Ala Ala Asn Ser Lys Gln Pro Ile His Met Val
    210                 215                 220
Tyr Val Pro Ser His Leu Tyr His Met Leu Phe Glu Leu Phe Lys Asn
225                 230                 235                 240
Ala Met Arg Ala Thr Val Glu Ser His Glu Ser Ser Leu Ile Leu Pro
                245                 250                 255
Pro Ile Lys Val Met Val Ala Leu Gly Glu Glu Asp Leu Ser Ile Lys
            260                 265                 270
Met Ser Asp Arg Gly Gly Gly Val Pro Leu Arg Lys Ile Glu Arg Leu
        275                 280                 285
Phe Ser Tyr Met Tyr Ser Thr Ala Pro Thr Pro Gln Pro Gly Thr Gly
    290                 295                 300
Gly Thr Pro Leu Ala Gly Phe Gly Tyr Gly Leu Pro Ile Ser Arg Leu
305                 310                 315                 320
Tyr Ala Lys Tyr Phe Gln Gly Asp Leu Gln Leu Phe Ser Met Glu Gly
                325                 330                 335
Phe Gly Thr Asp Ala Val Ile Tyr Leu Lys Ala Leu Ser Thr Asp Ser
            340                 345                 350
Val Glu Arg Leu Pro Val Tyr Asn Lys Ser Ala Trp Arg His Tyr Gln
        355                 360                 365
Thr Ile Gln Glu Ala Gly Asp Trp Cys Val Pro Ser Thr Glu Pro Lys
    370                 375                 380
Asn Thr Ser Thr Tyr Arg Val Thr
385                 390

## Description

The present invention relates to crystals of pyruvate dehydrogenase kinase (PDHK), and more particularly to high resolution structures of PDHK in the presence and absence of physiological and synthetic ligands obtained by X-ray diffraction. The invention further relates to the use of the three-dimensional structure so determined to identify, design or select compounds that bind to PDHK. The invention further relates to the DNA construct used to obtain a crystallisable PDHK protein.

Intermittent claudication and skeletal muscle fatigue are symptoms of peripheral vascular disease and other ischaemia-related diseases, e.g. chronic obstructive pulmonary disease, congestive heart failure and angina. In some cases, symptomatic relief can be achieved by oral administration of dichloroacetate (DCA) (Stacpoole, P.W. et al. (1989) *Metabolism* **38,** 1124-1144; Bersin, R.M. et al. (1997) *Am. Heart J.* **134,** 841-855; Henderson, G.N. et al. (1997) *J. Clin. Pharmacol*. **37,** 416-425), a well established (Whitehouse, S. et al. (1974) *Biochem. J*. **141,** 761-774), but toxic (Stacpoole, P.W. et al., (1998) *Drug Metab. Rev.* **30,** 499-539; Stacpoole, P.W. et al. (1998) *Environ. Health Perspect*. **106** (Suppl. 4), 989-994) activator of pyruvate dehydrogenase (PDH).

PDH is a multi-enzyme complex (for a review see Mattevi, A. et al. (1992) *Curr. Opin. Struct*. *Biol*. **2**, 877-887) that catalyses the decarboxylation of pyruvate with the formation of acetyl-CoA and NADH. The PDH complex consists of a pyruvate decarboxylase (E₁), a dihydrolipoyl transacetylase (E₂) and a dihydrolipoyl dehydrogenase (E₃) component. PDH is regulated by product inhibition and reversible phosphorylation through PDH kinase (PDHK) and PDH phosphatase (Linn, T.C. et al. (1969) *Proc*. *Natl. Acad. Sci. USA* **62,** 234-241). DCA inhibits ATP-dependent phosphorylation by PDHK, which explains its effect as PDH activator (Pratt, M.L. & Roche, T.E. (1979) *J. Biol. Chem.* **254,** 7191-7196). Phosphorylation of PDH is mediated by non-covalent binding of PDHK to the lipoyl domain of the dihydrolipoyl transacetylase (E₂L₂) component of the pyruvate dehydrogenase complex (Ravindran, S. et al. (1996) *J. Biol. Chem.* **271,** 653-662; Jackson, J.C. et al. (1998) *Biochem. J.* 334, 703-711).

Activation of PDH under conditions of reduced oxygen availability would promote the rapid formation of acetyl-CoA from pyruvate for entry in the citric acid cycle. In addition it would reduce the rate of accumulation of lactic acid and inorganic phosphate, which are thought to be a major factor in causing muscle fatigue (Fitts, R.H. (1994) *Physiol. Rev.* **74**, 49-94). Furthermore, it would increase the yield of ATP per molecule of oxygen consumed by shifting metabolism from fatty acid oxidation to carbohydrates as primary energy source.

Crystallisation of PDHK enzymes would enable determination of the three dimensional structure of the native enzyme. Furthermore, analysis of crystalline forms in the presence and absence of physiological and/or synthetic ligands would allow for identification of ligand binding and/or protein interaction sites of PDHK enzymes. Such information is useful for design of compounds to modulate PDHK activity and intervene with phosphorylation of PDH by PDH kinase.

Several approaches may be used to intervene with phosphorylation of PDH by PDH kinase, e.g.
- Inhibition of the kinase activity by (a) competitive ligand binding to the ATP binding site or (b) via allosteric regulatory sites.
- Blocking of the PDHK:E₂L₂ interaction by binding to the E₂L₂ binding site, or blocking other protein-protein interactions associated with and facilitating ATP dependent phosphorylation of the PDH complex by PDHK.
- Interference with binding of the PDHK substrate, i.e. the E₁ component of the PDH complex.

Development of these approaches has been hindered because efforts to produce a crystalline form of PDHK, such as PDHK isozyme 2 (PDHK-2), in particular human PDHK-2 (hPDHK-2) have been unsuccessful.

The technical problem to be addressed by the invention is the provision of a crystalline form of PDHK, such as PDHK-2 , in particular hPDHK-2.

This invention provides a PDHK crystal wherein the PDHK protein includes or consists of PDHK-2 amino acid sequence as shown in SEQ ID NO: 2 but starting at amino acid position 16 (i.e. the PDHK protein includes, or consists of the PDHK-2 amino acid sequence as shown in SEQ ID NO: 10), or a homologue, fragment, variant or derivative thereof.

The invention alleviates the problems in the art in by provision of crystalline forms of PDHK, such as PDHK-2, in particular hPDHK-2 in which a portion of the N-terminal amino acid sequence of the native sequence is absent.

The X-ray structure of engineered human PDHK isozyme 2 (PDHK-2) has been determined in the presence and absence of physiological and synthetic ligands. The structure is the first detailed three-dimensional description of this important regulatory enzyme. The protein consists of an N-terminal regulatory domain with a four-helix bundle topology, and a C-terminal catalytic domain, belonging to the GHKL superfamily (Dutta, R. & Inouye, M. (2000) *Trends Biochem. Sci.* **25,** 24-28).

At least four distinct ligand binding sites of PDHK have been identified, comprising the ATP binding site on the catalytic domain, as well as the putative binding site of the lipoyl domain of the E₂ component (E₂L₂) of the PDH complex, an allosteric binding site and the DCA binding site, located on the regulatory domain. The structures of complexes of PDHK with ligands bound at the various sites are useful for structure based ligand design.

Recently the structure of PDHK-2 from *Rattus novegicus* (rPDHK-2) has been determined in the presence of ADP at 2.5Å resolution (Steussy et al (2001), J. Biol. Chem., **276** (40), 37433-37450).

One embodiment of the invention is a crystal of pyruvate dehydrogenase kinase (PDHK), preferably a crystal of PDHK-2, preferably mammalian PDHK-2, even more preferably human PDHK-2, or homologues, fragments, variants, or derivatives thereof.

In an embodiment of the invention the PDHK-2 protein will have amino acid sequence as shown in SEQ ID NO: 2, preferably the PDHK protein includes or consists of PDHK-2 amino acid sequence as shown in SEQ ID NO: 2 but starting at amino acid position 16 (i.e. the PDHK protein includes, or consists of the PDHK-2 amino acid sequence as shown in SEQ ID NO: 10), or a homologue, fragment, variant or derivative thereof; preferably the PDHK-2 amino acid sequence is preceded by an amino acid sequence that does not correspond to the amino acid sequence at positions 1 to 15 of SEQ ID NO: 2; more preferably the PDHK-2 amino acid sequence is preceded by amino acid sequence that is an affinity purification tag and, or amino acid sequence that is a specific protein cleavage site; yet more preferably the PDHK-2 sequence is preceded by an amino acid sequence that is an affinity purification tag followed by a specific protein cleavage site. Preferably the affinity purification tag is a multiple histidine tag, most preferably a six Histidine (Hexa-His) tag. Preferably the specific protein cleavage site is a thrombin cleavage site. In an especially preferred embodiment the PDHK protein consists of PDHK amino acid sequence preceded by a Hexa-His tag followed by a thrombin cleavage site, more preferably the PDHK protein additionally has an N-terminal methionine residue. Even more preferred is a crystal where the PDHK-2 has a sequence as shown in SEQ ID NO: 4, herein referred to as hexa-His-PDHK-2*. In a preferred embodiment of the PDHK crystal, the PDHK protein consists of a PDHK -2 amino acid sequence preceded by one or two amino acid residues. A further preferred embodiment is a PDHK crystal wherein the PDHK protein includes or consists of a PDHK-2 amino acid sequence as shown in SEQ ID NO: 5, or a homologue, fragment, variant or derivative thereof; in a particularly preferred embodiment the PDHK-2 amino acid sequence consists of a PDHK-2 amino acid sequence as shown in SEQ ID NO: 5, this last embodiment is hereinafter referred to as PDHK-2*. In an embodiment of the invention PDHK-2* is provided by thrombin cleavage of the PDHK-2 amino acid sequence as shown in SEQ NO: 4.

The above mentioned crystal of PDHK-2* grown in 100mM sodium acetate pH 5.2-5.8, 5-10% isopropanol, and 25-150mM MgCl₂, preferably in 100mM sodium acetate pH 5.6-5.8, 6-9% isopropanol, and 75-125mM MgCl₂ is also an embodiment of this invention, as well as said crystal having a space group P6₄, or said crystal having unit cell dimensions of a = b = 109 Å +/- 3 Å, c = 85 Å +/- 3 Å.

Another embodiment of the invention is a crystal of hexa-His-PDHK-2*, wherein the crystal is monoclinic, or wherein said crystal is grown in 100mM citrate pH 5.5-5.7, 15% PEG 4K, and between 200-400mM ammonium acetate, or wherein said crystal has a space group C2, or said crystal which has unit cell dimensions of a = 90 Å +/- 3 Å, b = 54 Å +/- 3 Å, c = 83 Å +/- 3 Å, and β = 106° +/- 2°.

Further embodiments are crystals according to the invention with heavy atoms soaked in, preferably the heavy atoms being selected from a group consisting of mercury, iridium, and osmium.

A further embodiment of the invention is a crystal of PDHK-2, PDHK-2*, or hexa-His-PDHK-2* with a PDHK substrate, preferably ATP, or ADP, and/or a PDHK ligand soaked in. Preferably, the ligand is 4-{(2,5)-dimethyl-4-[3,3,3-trifluoro-2-hydroxy-2-methylpropanoyl]piperazinyl}carbonyl)benzonitrile, herein referred to as Compound 1, or N-{4-[(ethylanilino)sulfonyl]-2-methylphenyl}-3,3,3-trifluoro-2-hydroxy-2-methylpropanamide, herein referred to as Compound 2, or N-(2-aminoethyl)-2-{3-chloro-4-[(4-isopropylbenzyl)oxy]phenyl)acetamide, herein referred to as Compound 3, or DCA (dichloroacetate).

Another embodiment of the invention is a crystal of liganded PDHK, preferably liganded PDHK-2*; preferably said crystal is grown in 100mM MES pH6, 10% isopropanol and 200mM calcium acetate in the presence of a PDHK ligand. Preferably, said PDHK ligand is Compound 1.

Another embodiment of the invention is any crystal as mentioned above, wherein said crystal diffracts X-rays to 3.8 Å or better resolution, preferably to 3.2 Å or better resolution, preferably to 2.8 Å or better resolution, more preferably to 2.5 Å or better resolution, yet more preferably to 2.4 Å or better resolution, most preferably to 2.2 Å or better resolution.

A further embodiment of the invention is a crystal of PDHK-2*, having the atomic coordinates set out in Table 3, or a derivative set as expressed in any reference frame. The invention also includes:
(a) co-crystals of PDHK-2* with Compound 1, having the atomic coordinates set out in Table 4, or a derivative set as expressed in any reference frame,
(b) co-crystals of PDHK-2* with Compound 1, having ATP soaked in, having the atomic coordinates set out in Table 5, or a derivative set as expressed in any reference frame,
(c) crystals of PDHK-2* with Compound 2 soaked in, having the atomic coordinates set out in Table 6, or a derivative set as expressed in any reference frame,
(d) crystals of PDHK-2* with Compound 3 soaked in, having the atomic coordinates set out in Table 7, or a derivative set as expressed in any reference frame, or
(e) crystals of PDHK-2* with DCA and ADP soaked in, having the atomic coordinates set out in Table 8, or a derivative set as expressed in any reference frame.

Another aspect of the invention is the use of the atomic coordinates of a PDHK crystal according to the invention for deriving the three-dimensional structure of PDHK-2, and the use of said three-dimensional structure of PDHK-2 to computationally or otherwise evaluate the binding interactions of a chemical compound with PDHK-2, preferably with a binding site on PDHK-2, even more preferably with the ATP binding site on PDHK-2, in particular the ATP binding site which includes, but is not limited to, residues selected from Glu251, Leu252, Lys254, Asn255, Ala256, Arg258, Ala259, Met288, Ser289, Asp290, Gly292, Gly293, Gly294, Val295, Ile300, Leu303, Leu323, Ala324, Gly325, Phe326, Gly327, Tyr328, Gly329, Leu330, Pro331, Leu346, Ser348, Thr354, Asp355, Ala356. The invention also includes the use of said three-dimensional structure of PDHK-2 to computationally of otherwise evaluate the binding interactions of a compound with the putative E₂L₂ binding site on PDHK-2, in particular with the putative E₂L₂ binding site on PDHK-2 which includes, but is not limited to, residues selected from Leu31, Gln35, Phe36, Asp38, Phe39, Thr48, Ser49, Phe52, Leu53, Met167, Leu168, Gln171, His172, Ile175, Phe176. Furthermore the invention includes the use of the three-dimensional structure of PDHK-2 as mentioned above to computationally or otherwise evaluate the binding interactions of a compound with a binding site on PDHK-2 which includes, but is not limited to, residues selected from Leu71, Pro72, Arg74, Val75, Thr78, Ser80, Va181, Met130, Gly133, Val134, Glu136, Tyrl37, Asp144, Val146, Ser147, Asn150, Ile151, Phe154, Leu155, Tyr382. The invention also includes the use of a three dimensional structure of PDHK-2 to computationally or otherwise evaluate the binding interactions of a compound with a binding site on PDHK-2 which includes, but is not limited to, residues selected from Leu57, Leu61, Tyr88, Ser91, Ile95, Ile119, Arg120, His123, Ser161, Arg162, Ile165, Arg166, Ile169.

Another aspect of the invention is the use of the three-dimensional structure of PDHK-2 according to the invention to design a compound, in particular a PDHK ligand, preferably a PDHK inhibitor, capable of associating with a PDHK enzyme, preferably to design a compound capable of associating with a binding site on the enzyme, even more preferred to design a compound capable of associating with any one of the binding sites on the enzyme mentioned above; even more preferred to design a compound capable of associating with an ATP binding site of the enzyme, or with the putative E₂L₂ binding site on the enzyme, or with the third binding site described, or with the DCA binding site.

Another embodiment of the invention is the use of the three-dimensional structure of PDHK-2 as mentioned above to computationally or otherwise evaluate the binding interactions of a compound, preferably a PDHK ligand, even more preferably a PDHK inhibitor, to the dimerisation interface, as well as the use of said structure to design compounds that can interact with said dimerisation interface.

The invention also relates to compounds, preferably PDHK ligands, even more preferably PDHK inhibitors, thus evaluated or designed.

Another aspect of the invention is a method of selecting a PDHK-2 inhibitor compound from a group of potential PDHK-2 inhibitor compounds, comprising the following steps:
a) creating a three-dimensional representation of the structure of PDHK-2 as mentioned above, or preferably the structure of any of the binding sites on the enzyme;
b) displaying and superimposing a model of the potential PDHK-2 inhibitor compound on the model of the PDHK-2 structure or on the model of the binding site on the enzyme; and
c) assessing whether the model of the potential PDHK-2 inhibitor compound fits the model of the PDHK-2 structure or the model of the binding site on the enzyme.

Optionally, said method can further comprise the following steps:
d) incorporating the potential PDHK-2 inhibitor compound in a biological PDHK-2
activity assay; and
e) determining whether the potential PDHK-2 inhibitor compound inhibits PDHK-2 activity in this assay.

The invention also includes compounds, in particular PDHK-2 ligands, preferably PDHK-2 inhibitors, selected by the method described above.

Another aspect of the invention is a pharmaceutical composition comprising a compound, preferably a PDHK ligand, even more preferably a PDHK inhibitor, evaluated, designed or selected as described above.

A further embodiment of the invention is the use of a compound, preferably a PDHK ligand, even more preferably a PDHK inhibitor, evaluated, designed or selected as described above, in the manufacture of a medicament for the treatment of diseases, wherein the inhibition of PDHK is beneficial, preferably ischemia-related diseases such as congestive heart failure, peripheral vascular disease, chronic obstructive pulmonary disease, coronary artery disease, diabetes, ischemia, endotoxic shock, hemorrhagic shock, lactic acidosis, or cardiac insufficiency.

Another aspect of the invention is the use of the atomic coordinates of PDHK-2 as mentioned above, or portions thereof, to solve a crystal form of a mutant, homologue or co-complex of PDHK-2, for example by Molecular Replacement or Difference Fourier analysis.

A further aspect of the invention is the use of the structure of PDHK-2 to design mimics of other PDHK isozymes or variants thereof, e.g. single nucleotide polymorphisms (SNPs), particularly in the binding site regions identified; the structure can be used to design site-directed mutants, but also to computationally (or otherwise) assess the binding of PDHK ligands to such closely related enzymes.

Furthermore, the invention includes the use of the atomic coordinates of PDHK-2 as mentioned above, or portions thereof, to produce a model of the three-dimensional structure of related proteins or variants or portions thereof. Such related proteins include, but are not limited to, other PDHK isozymes, PDHK isozymes from different animals, from plants, bacteria, or fungi, branched-chain α-keto acid dehydrogenase kinase, and others.

A further embodiment of the invention is a nucleotide sequence comprising the sequence shown in SEQ ID NO: 3, allelic variants, or mutants thereof or a nucleotide sequence comprising a sequence encoding the peptide sequence shown in SEQ ID NO: 4, allelic variants or mutants thereof; or the sequence as shown in SEQ ID NO: 9, or allelic variants, or mutants thereof; or a nucleotide sequence encoding PDHK-2 amino acid sequence as shown in SEQ ID NO: 2, but starting at amino acid position 16, or allelic variants or mutants thereof; or the sequence as shown in SEQ ID NO: 8 or allelic variants or mutants thereof; or a sequence encoding the amino acid sequence shown in SEQ ID No: 5, or allelic variants or mutants thereof. (An allelic variant is an alternative form of the enzyme, with a difference in the nucleic acid sequence, and generally produce altered mRNAs and potentially altered proteins, whose structure or function may or may not be altered. The difference can be a single nucleotide, often referred to as single nucleotide polymorphism (SNP), but can also involve larger changes. By mutants we mean alternative forms of the enzyme deliberately produced by methods well known to a person skilled in the art, such as site-directed mutagenesis.) Preferably this nucleotide sequence is linked to a regulatory sequence, in particular a promoter sequence, suitable for driving expression in the desired host. Preferably, said nucleotide sequence is incorporated into a recombinant baculovirus, and said host is an insect cell line.

The terms "homologue", "fragment", "variant" or "derivative" in relation to the crystallized PDHK sequence include any substitution of, variation of, modification of, replacement of, deletion of or addition of one or more amino acid residues from or to the sequence, providing the resultant polypeptide is capable of folding to a structure similar to that of PDHK, in particular similar to that of PDHK-2. The term "homologue" covers homology with respect to sequence and/or structure and/or function. With respect to sequence homology, in proteins according to the invention, there is at least 65%, preferably 80%, more preferably 90%, even more preferably 95%, most preferably 98%, identity to the protein sequence of human PDHK-2 (SEQ ID NO: 2), or a significant part thereof for example to hPDHK-2 amino acid sequence as shown in SEQ ID NO: 2, but starting at amino acid position 16 . A person skilled in the art will be well aware of suitable computer programmes to use to assess such sequence homology, which include, but are not limited to, the BLAST package (Altschul, S.F. et al (1997) *Nucl*. *Acids Res.* **25,** 3389-3402), FASTA or FASTP (Pearson, W.R. (1990) *Methods Enzymol*. **183,** 63-98), or programmes included in the GCG package (University of Wisconsin; Devereux et al (1984) *Nucl*. *Acids Res*. **12,** 387) such as GAP or BESTFIT.

### List of Abbreviations used:

- ADP: adenosine diphosphate
- ATP: adenosine triphosphate
- CoA: coenzyme A
- DCA: dichloro acetate
- DNA: deoxyribonucleic acid
- EDTA: ethylenediamine tetraacetic acid
- Hsp: heat shock protein
- MES: 2-(N-morpholino) ethanesulphonic acid
- MOI: multiplicity of infection
- NAD: nicotinamide adenine dinucleotide
- NADH: nicotinamide adenine dinucleotide, reduced form
- NIH: National Institute of Health, USA
- PCR: polymerase chain reaction
- PDB: Protein Data Bank
- PDH: pyruvate dehydrogenase
- PDHK: PDH kinase
- PDHK-1: PDHK isozyme 1
- PDHK-2: PDHK isozyme 2
- hPDHK-2: PDHK isozyme 2 from *Homo sapiens* (Human)rPDHK-2 PDHK isozyme 2 from *Rattus norvegicus (Rat)*
- hexa-His-PDHK-2*: Engineered version of human PDHK-2, suitable for crystallisation; the sequence is shown in SEQ ID NO: 4
- PDHK-2*: Engineered version of human PDHK-2, suitable for crystallisation; the sequence is shown in SEQ ID NO: 5; the protein can be prepared by thrombin cleavage of hexa-His-PDHK-2*
- PDHK-3: PDHK isozyme 3
- PDHK-4: PDHK isozyme 4
- PEG: polyethylene glycol
- rmsd: root mean square deviation
- rpm: rotations per minute
- SDS-PAGE: sodium dodecyl sulfate polyacrylamide gel electrophoresis
- SNP: single nucleotide polymorphism
- Compound 1: 4-{(2,5)-dimethyl-4-[3,3,3-trifluoro-2-hydroxy-2methylpropanoyl]piperazinyl}carbonyl)benzonitrile
- Compound 2: N-{4-[(ethylanilino)sulfonyl]-2-methylphenyl}-3,3,3-trifluoro-2hydroxy-2-methylpropanamide
- Compound 3: N-(2-aminoethyl)-2-{3-chloro-4-[(4isopropylbenzyl)oxy]phenyl}acetamide

### Examples

The invention will now be illustrated by the following non-limiting examples and accompanying figures, in which:
SEQ ID NO: 1 illustrates the human PDHK-2 cDNA coding sequence;
SEQ ID NO: 2 illustrates the human PDHK-2 protein sequence;
SEQ ID NO: 3 illustrates the DNA sequence engineered to encode hexa-His-PDHK-2*.
SEQ ID NO: 4 illustrates the protein sequence of hexa-His-PDHK-2*.
SEQ ID NO: 5 illustrates the protein sequence of PDHK-2*.
SEQ ID NO: 6 shows the sequence of the 5' primer used to amplify the DNA sequence of SEQ ID NO: 3
SEQ ID NO: 7 shows the sequence of the 3' primer sequence used to amplify the DNA sequence of SEQ ID NO: 3;
SEQ ID NO: 8 illustrates the DNA sequence of PDHK-2*
SEQ ID NO: 9 illustrates human PDHK-2 DNA sequence starting at a position corresponding to amino acid residue Ala16 in the full length hPDHK-2 sequence.
SEQ ID NO: 10 illustrates human PDHK-2 protein sequence starting at a position corresponding to amino acid residue Ala16 in the full length hPDHK sequence.

Figure 1 shows the protein sequence of PDHK-2*, annotated to indicate the secondary structure features, residues interacting with ligands or located at the dimerisation interface, and conserved residues in all 4 human PDHK isozymes.
Figure 2 shows a ribbon diagram of the PDHK-2 structure.
Figure 3 shows two views of the PDHK-2 dimer.
Figure 4 shows ATP and Mg²⁺ together with the coordinating water molecules at the ATP binding site of PDHK-2.
Figure 5 shows views of (a) Compound 1 and (b) Compound 2 bound to PDHK-2, as well as their chemical structures depicted underneath.
Figure 6 shows a view of Compound 3 bound to PDHK-2, as well as its chemical structure.
Figure 7 shows the f_{obs} (*ADP, DCA*)-f_{obs} (*native*) electron density map with a model of DCA superimposed.

### Example 1: Design of a construct to enable crystallisation of human PDHK-2

Attempts to crystallise full length human PDHK-2 were unsuccessful, thus steps were taken to engineer a construct to express a crystallisable form of human PDHK-2.

Subjecting human PDHK-2 to partial chymotrypsin digestion (1 hour at 30°C) generated a predominant protein species with truncations on both ends. The N-terminal residue of the truncation product was determined to be Lys-14 by N-terminal sequencing; from the molecular weight it was deduced that 16 residues had been removed from the C-terminus as well. This truncated form of the enzyme retained enzymatic activity, therefore it was unlikely that the removed parts of the enzyme were essential for activity or the structural integrity of the enzyme.

A N-terminally truncated form of PDHK-2 was engineered, the expression construct comprising the DNA sequence encoding a methionine, a tag consisting of 6 histidine residues, followed by a Tobacco Etch Virus (TEV) protease cleavage site, followed by the human PDHK-2 sequence starting with residue Lys14. Following expression and purification of the resulting protein, crystallisation trials yielded only very small crystals; when subjected to MALDI-TOF mass spectrometry, two prominent protein species were detected, one of full length, and one truncated within the TEV protease cleavage site.

From secondary structure predictions, it appeared that the truncation at the N-terminus occurred near the start of a predicted helical region. The starting residue of this helix was predicted to be A1a16.

The implication of all the data mentioned above was that the N-terminal region of the protein may be rather flexible and potentially adversely affecting the packing of the crystal lattice, and that a more constrained part of the structure may start with the predicted helix starting at Ala16. It was therefore decided to concentrate on designing constructs starting with position Ala16 of the human PDHK-2 sequence. A hexa-histidine tag was included for ease of purification, and a thrombin cleavage site was engineered in to enable the tag to be removed during the purification and prior to crystallisation.

### Example 2: Cloning and expression of crystallisable PDHK-2

Oligonucleotide primers were designed for use in polymerase chain reaction (PCR) amplification of the desired sequence, based on the sequence of human PDHK-2 (Gudi, R. et al (1995) *J. Biol. Chem.* **270**, 28989-28994, GenBank Accession number L42451). The 5' primer was designed to comprise DNA sequence encoding, following the ATG start codon, six histidine residues, followed by a thrombin cleavage site, and then linking inframe with human PDHK-2 starting at residue Ala16. Both primers contained a restriction endonuclease site for ease of inserting the PCR product into the desired vector:

5' primer: PDHK-2 Fast5'HisThr (SEQ ID NO: 6):
5'-CGTGAATTC ATG CAT CAT CAT CAT CAT CAC CTT CTG GTT CCG CGT
GGA TCT GCG CCC AAG TAC ATA GAG CAC TTC-3'

3' primer: PDHK-2 Fast3' (SEQ ID NO: 7):
CGTTCTAGA CTA TCA CGT GAC GCG GTA CGT CGA CG-3'

DNA fragments were generated by PCR amplification from a clone containing full length human PDHK-2. In discrepancy to the published human PDHK-2 sequence as shown in GenBank accession L42451, apart from containing some silent single nucleotide differences, codon 80 encodes a serine instead of the threonine in the published sequence (SEQ ID NO: 1).

The PCR reaction was carried out for 30 cycles in a total volume of 50 µl in a solution containing 1.5 mM MgCl₂, 0.2mM dNTPs, 50pmol of each primer, 5 µl 10x Reaction Buffer (Roche Diagnostics), 0.2 µl template (1ng) (a plasmid containing full length human PDHK-2 cDNA), and 2.5 units Expand DNA polymerase (Roche Diagnostics). Each cycle consisted of 1 minute at 94°C, 1 minute at 50°C, and 2 minutes at 72°C. Amplified DNA fragments were separated by agarose gel electrophoresis, and the desired PCR product purified using a QIA quick gel extraction kit (Qiagen). The purified PCR product was then digested with EcoRl and Xba1, using Multicore buffer (Promega) at 37°C for 2 hours. The digested fragment was then ligated into pFastbac1, also digested with EcoR1/Xba1 (Life Technologies), using DNA ligase (Promega) according to the manufacturer's instructions, for 12 hours at 12°C. The ligation mix was electroporated into *E.coli* (TOP 10) (Invitrogen), using a BioRad Gene Pulser (capacitance 25 µFD, voltage 2.5 V, resistance 200Ω).

Clones containing the desired insert were selected by using 2YT plates containing 100 µg/ml ampicillin and checked using endonuclease digestion, followed by agarose gel electrophoresis, for the presence of an insert of the correct size. DNA sequence analysis was carried out by Lark (Saffron Waldon, UK). The sequence of the DNA insert is shown as SEQ ID NO: 3.

Recombinant bacmid DNA was produced by transforming *E.coli* DH10BAC™ with the recombinant pFastbac1 plasmid DNA as described above. This was carried out according to the method in the Bac to Bac baculovirus expression manual (Life Technologies). PCR analysis was used to verify successful transposition to the bacmid using pUC/M13 amplification primers (Life Technologies).

Generation of the primary baculovirus stock was carried out by transfection using Sf-9 insect cells. Bacmid DNA containing the correct insert was mixed with CELLFECTIN™ transfection reagent (Life Technologies) and added to a monolayer of Sf-9 insect cells using SF-900II serum free medium (Life Technologies). Following 72 hours incubation at 27°C, the supernatant was harvested as the initial baculovirus stock. This stock was amplified by adding the initial baculovirus stock into a suspension of Sf-9 insect cells in Erlenmeyer flasks (Corning) with agitation of 125rpm at 27°C. After 6 days post infection, the supernatant was harvested by centrifugation and stored at 4°C as the working virus stock. The titre of this stock was determined by conventional plaque assay analysis as in the Bac to Bac baculovirus expression manual (Life Technologies).

Protein expression was optimised in Erlenmeyer flask cultures using Sf-9 and *T.ni* Hi-5 insect cell cultures looking at different multiplicities of infection (MOI) and harvest times, the optimal conditions found were then scaled up into fermenters.

The fermenters used were autoclavable Applikon 3 litre stirred vessels controlled using Applikon 1030 biocontrollers. Inoculum of *T.ni* Hi-5 cells was initially prepared as shake flask cultures. The fermenter was inoculated with 5 x 10⁵ cells/ml made up in Excel 405 serum free medium (JRH Biosciences). Temperature was controlled at 27°C, dissolved oxygen concentration controlled at 60%, and pH was measured but not controlled. Oxygen concentration was controlled by initially enriching the air stream to the headspace with oxygen, then sparging pure oxygen to the culture. Agitation was set at 150rpm with a double impeller system of marine impeller within the culture and rushton impeller at the liquid/headspace interface. Aeration was continuous to the headspace at 0.5 l/min.

When the viable cell density reached 2 x 10⁶ cells/ml, the culture was infected using an MOI of 2 from the titred baculovirus working stock. Harvest time for the culture was 72 hours post infection. This was achieved by centrifugation at 2000g for 15 mins; the insect cell pellet was then stored at -80°C awaiting purification.

### Example 3: Purification of hexa-His-PDHK-2* and removal of the hexa-histidine tag

PDHK-2*, having the sequence as shown in SEQ ID NO: 5, was purified in five steps: (1) Pellet from the fermentation was resuspended into 10ml lysis buffer per gram wet cell weight and mechanically broken using a Constant Systems continuous cell disrupter at a pressure of 20kpsi. The lysis buffer consisted of 50mM Tris HCl (pH 8), 150mM sodium chloride, 10mM Imidazole, 5mM β-mercaptoethanol, containing EDTA free protease inhibitor cocktail tablets (Roche). The lysate was chilled and centrifuged at 14,000g for 45 mins to remove cell debris. (2) The supernatant was collected and applied to Nickel-NTA Superflow chelate (Qiagen), using 20ml drip columns and 3ml resin bed volume. The resin was initially washed with 10 column volumes lysis buffer and eluted with lysis buffer containing 100mM Imidazole. (3) The eluted fraction was then buffer exchanged into Thrombin cleavage buffer (20mM TrisHCl (pH8), 150mM sodium chloride and 2.5mM CaCl₂) using a 75ml G-25 fine desalting column (Pharmacia) at a flow-rate of 7.5ml/min. The desalted protein fractions were then pooled, and thrombin (Sigma T-1063) added to a final concentration of 5 NIH units/mg protein in solution. An incubation of 3 hours at room temperature then followed in order to allow for the removal of the hexa-histidine tag. (4) After thrombin cleavage the sample was applied to a 3ml Nickel-NTA Suparflow column (Qiagen) and the flow-through collected. The remainder of the cleaved PDHK-2* was removed from the column by elution with thrombin cleavage buffer containing 10mM Imidazole. (5) The flow-through and the 10mM imidazole fractions were pooled and concentrated to 2 mg/ml using 10kDa centrifugal concentrators (Vivascience), and loaded at 0.75 ml/min onto a Sephacyl -200 prep grade 16/60 column pre-equilibrated with 20mM TrisHCl (pH8), 150mM sodium chloride and 1mM DL-dithiothreitol on an Akta-Explorer purification system (Amersham Pharmacia Biotech). The eluted fractions were run on Tris-glycine SDS-PAGE gels and fractions containing PDHK-2* pooled. These were then stored at -80°C prior to crystallisation.

### Example 4: Purification of hexa-His-PDHK-2*

Hexa-His-PDHK-2*, i.e. the expression product of the construct produced in Example 2 having the sequence as shown in SEQ ID NO: 4, was purified by steps (1) and (2) as described in Example 3 above for the thrombin-cleaved material (PDHK-2*). Then the 100mM imidazole fraction was pooled and concentrated to 2 mg/ml using 10kDa centrifugal concentrators (Vivascience), and loaded at 0.75 ml/min onto a Sephacyl -200 prep grade 16/60 column pre-equilibrated with 20mM TrisHCl (pH8), 150mM sodium chloride and 1mM DL-dithiothreitol on an Akta-Explorer purification system (Amersham Pharmacia Biotech). The eluted fractions were run on Tris-glycine SDS PAGE gels and fractions containing hexa-His-PDHK-2* were pooled. These were then stored at -80°C prior to crystallisation.

### Example 5: Crystallisation of PDHK-2*, liganded PDHK-2*, and hexa-His-PDHK-2*

### (a) Crystallisation of PDHK-2*

The PDHK-2* fractions from the final gel filtration column, obtained as described in Example 3, were concentrated to 10mg/ml using 10kDa centrifugal concentrators (Vivascience) at 3,000rpm, 4°C. The crystallisation trials were set up using hanging drop vapour diffusion at 4°C. PDHK-2* crystallised in a range of conditions using 100mM sodium acetate pH 5.2-5.8, 5-10% iso-propanol and 25 -150mM magnesium chloride. Prior to crystallisation the PDHK-2* at 10mg/ml was centrifuged for 2 min at 14,000rpm in an Eppendorf centrifuge. A reservoir volume of 950µl was used, and drops of 2µl reservoir reagent and 2µl PDHK-2* suspended over the reservoir on cover slips. Larger crystals of PDHK-2* were obtained at pH 5.6-5.8 with 6-9% isopropanol and 75-125mM magnesium chloride. These crystals belonged to space group P6₄ with cell dimensions a = b = 109.3 Å, c = 85.1 Å. They contained one molecule per asymmetric unit (M_{w} = 44.6 kDa), and had a solvent content of 62 % (V_{M} = 3.29; Matthews, B.W. (1968) *J*. *Mol*. *Biol*. **33,** 491-497). Crystals appeared overnight and continued to grow for up to 2 weeks.

### (b) Co-crystallisation of PDHK-2* with Compound 1

For co-crystallisation studies PDHK-2* was mixed with Compound 1 (4-{(2,5)-dimethyl-4-[3,3,3-trifluoro-2-hydroxy-2-methylpropanoyl]piperazinyl}carbonyl)benzonitrile; Aicher. T. et al. (1999) *J*. *Med. Chem.* **42,** 2741-2746) at 3-fold molar excess before getting concentrated to 10mg/ml, and being used in crystallisation trials. This liganded PDHK-2* crystallised under similar conditions to unliganded PDHK-2*. The liganded PDHK-2* also yielded good crystals in 100mM MES pH6, 10% isopropanol and 200mM calcium acetate.

### (c) Crystallisation of hexa-His-PDHK-2*

The stored hexa-His-PDHK-2* solution was concentrated to 10mg/ml using 10kDa centrifugal concentrators (Vivascience) at 3,000rpm, 4°C. Monoclinic crystals of hexa-His-PDHK-2* were grown by hanging drop vapour diffusion at 4°C, from 100mM Citrate pH 5.6, 15% PEG 4K and between 200-400mM ammonium acetate. The crystals grew as thin plates from a precipitate over a period of 2-3 weeks and belonged to space group C2 with unit cell dimensions a = 90.8 Å, b = 53.8 Å, c = 83.3 Å, β = 105.64°.

### Example 6: Data collection, structure determination and refinement of PDHK-2*

The structure of recombinant human PDHK-2* was solved by multiple isomorphous replacement (MIR) using three heavy atom derivatives (Drenth, J. (1994) Principles of protein X-ray crystallography. Springer-Verlag, New York). Mercury, iridium and osmium heavy atom derivatives were prepared by soaking crystals for 16-18 hours in a stabilising solution (9 % isopropanol in 100 mM sodium acetate buffer at pH 5.8) containing 1 mM ethylmercury thiosalicylate, 5 mM K₂IrCl₆, or 10 mM K₂OsCl₆, respectively.

For data collection protein crystals were flash-frozen at 100 K using a cryo-protection buffer consisting of stabilising solution and 30-35 % glycerol. Native X-ray diffraction data, i.e. for PDHK-2* crystals without heavy atoms, were collected at 100 K with an ADSC Quantum 4 CCD-detector at station 9.6 (λ = 0.8700 Å) of the S.R.S. Daresbury, UK. The diffraction data for the heavy atom derivatives were collected at 100 K with Rigaku RAXIS-2 or RAXIS-4 image plate detectors on laboratory rotating anode generators using CuKα radiation. All data were processed using the HKL package (Otwinowski, Z. & Minor,W. (1997) *Methods Enzymol*. **276,** 307-326). Data collection statistics are summarised in Table 1 (see end of Example section).

Heavy atom derivative data were scaled to native data with SCALEIT (Collaborative Computational Project Number 4 (1994) *Acta Crystallogr. D* **50,** 760-763: CCP4 suite). Difference Patterson and difference Fourier analysis identified initial heavy atom positions. Refinement of the heavy atom parameters and phase calculation was performed with SHARP (de La Fortelle, E. & Bricogne, G. (1997) *Methods Enzymol.* **276,** 472-494). Phases were improved by 120 cycles of solvent flattening with SOLOMON (Abrahams, J.P. & Leslie, A. (1996) *Acta Crystallogr. D* ***52,*** 30-42). The resulting map was of good quality and used to trace about 75 % of the structure using QUANTA (Quanta98 (1998), version 98.1111; Molecular Simulations Inc., San Diego, CA 92121-3752).

The atomic coordinates are shown in Table 3 (see end of Example section).

The model was refined against a set of native structure factors (F_{P}-calc) derived with SHARP from a combination of experimental native (F_{P}) and derivative (F_{PH}) structure factors, with the respective heavy atom contributions (F_{H}) substracted. Refinement was carried out in the resolution range 30-2.2 Å using CNX (Brünger, A. et al. (1998), *Acta Crystallogr. D* **54,** 905-921) with the *"mlhl"* maximum likelihood target function. During refinement model phases were combined with the experimental phase probability distributions provided by SHARP. Partial structure factors from a flat bulk-solvent model and anisotropic B-factor correction were supplied throughout the refinement. The R-factor for the current model is 0.267 (free R-factor, 5 % of data, 0.297) for all data in the resolution range 30-2.2 Å. The refinement statistics are summarised in Table 2 (see end of Example section).

The current model contains 355 out of 394 residues present in the construct and is well defined in most regions of the polypeptide chain. The electron density is weak for residues 40-43, 73-79, 105-109, 310-312 and 375-392. No interpretable electron density was observed for residues 178-185, 313-326 and 393-407. The latter are not included in the model, and residues 386-387 are modelled as alanine.

### Example 7: Preparation, data collection, structure determination and refinement of PDHK-2* protein/ligand complexes

Crystals of PDHK-2* in complex with Compound 1 were obtained by co-crystallisation as described in Example 5(b) above. Crystals of PDHK-2* in complex with Compound 2 (N-{4-[(ethylanilino)sulfonyl]-2-methylphenyl}-3,3,3-trifluoro-2-hydroxy-2-methylpropanamide; Example 12 in patent application WO 99/62873) and Compound 3 (N-(2-aminoethyl)-2-{3-chloro-4-[(4-isopropylbenzyl)oxy]phenyl} acetamide; see Example 10) were prepared by soaking apo-crystals, i.e. crystals prepared with unliganded PDHK-2* as described in Example 5(a) above, in stabilising solution containing the inhibitor at 1mM concentration. Crystals of the PDHK-2* in complex with ATP were obtained by soaking crystals, grown in the presence of Compound 1, in stabilising solution containing 10mM ATP. Crystals of the PDHK-2* in complex with ADP and DCA were obtained by soaking apo-crystals in stabilising solution containing both ADP and DCA at 10mM and 100mM concentration, respectively.

For data collection protein crystals were flash-frozen at 100 K using a cryo-protection buffer consisting of stabilising solution and 30-35 % glycerol. Diffraction data of PDHK-2* in complex with Compound 1, Compound 2, and both ATP and compound 1 were collected at 100 K on an ADSC Quantum 4 CCD-detector at beamline ID 14-2 (λ = 0.9326 Å) of the European Synchiotron Radiation Facility (ESRF), Grenoble, France. Diffraction data of PDHK-2* in complex with Compound 3 were collected at 100 K on a Rigaku RAXIS-2 image plate detector with a laboratory rotating anode generator using CuKα radiation. Diffraction data of PDHK-2* in complex with both ADP and DCA were collected at 100K on an ADSC Quantum 4 CCD-detector at station 9.6 (λ = 0.87 Å) of the Synchiotron Radiation Source (SRS), Daresbury, UK. Data processing was carried out with the HKL program package (Otwinowski, Z. & Minor, W. (1997) *Methods Enzymol*. **276,** 307-326). Data collection statistics are summarised in Table 1 (see end of Example section).

The structures were determined by difference Fourier methods using the unliganded PDHK-2* structure to calculate model phases and subsequently refined using CNX (Brünger, A. et al. (1998), *Acta Crystallogr. D* **54,** 905-921). Interactive graphical model building was carried out with QUANTA (Quanta98 (1998) version 98.1111; Molecular Simulations Inc., San Diego, CA 92121-3752). In all structures the respective ligands were clearly defined by the initial electron density maps. For a summary of refinement statistics see Table 2 (see end of Example section). A binding site of DCA on PDHK-2 has been identified by calculating a difference fourier map with [f_{obs} (ADP/DCA) - f_{obs} (native)] expiα_{calc} (native) as coefficients, where f_{obs} (ADP/DCA) are the observed structure factor amplitudes of PDHK-2* soaked with both ADP and DCA, f_{obs} (native) are the observed structure factor amplitudes of unliganded PDHK-2*, and α_{calc} (native) are the phases derived from the refined model of unliganded PDHK-2* (Example 6).

The atomic coordinates are given in Tables 4, 5, 6, 7 and 8. (see end of Example section).

### Example 8: The structure of PDHK-2

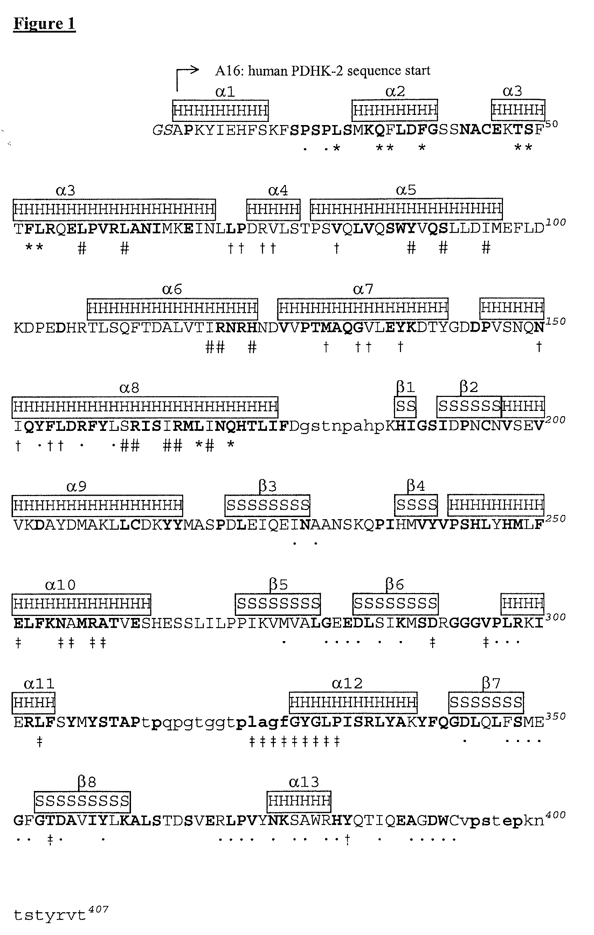

The structure of PDHK-2* is summarised in Figure 1, which shows the peptide sequence of PDHK-2*, annotated with the observed secondary structure features (boxes with H indicating residues involved in an α-helix, boxes with S indicating residues involved in a β-strand), labelled as referred to in the text below. Residues in bold are those conserved in all four human PDHK isozymes (49% in total, 45% in the regulatory domain, 52% in the catalytic domain). Residues labelled with (*) are in direct contact (i.e. within a distance of 4 Å) with Compound 1; residues in direct contact with ATP are labelled with (‡); residues in direct contact with a compound bound to the third site identified (Compound 3) are labelled with (†); residues involved in the dimer interface are labelled with (·); residues forming the DCA binding pocket are labelled with (#). Residues shown in lower case are disordered in the structure of the native enzyme (Example 6). The sequence of the human PDHK-2 starts with residue Ala16. The two preceding residues (Gly and Ser) arise from the engineered thrombin cleavage site, linking the PDHK-2 sequence to the N-terminal hexa-histidine tag

### (a) Description of the native structure and dimer assembly

The structure of PDHK-2*, prepared as described in Example 3, crystallised as described in Example 5(a), solved as described in Example 6 above, can be described as follows:

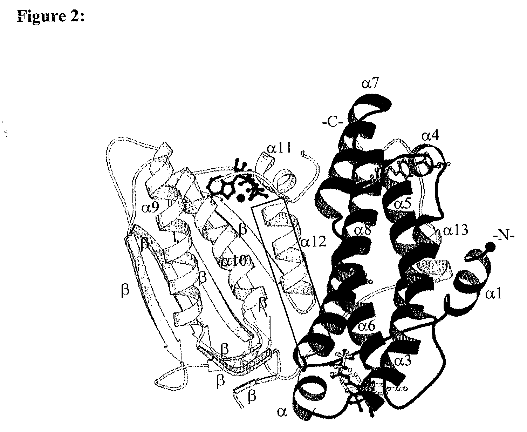

PDHK-2 is composed of two domains (see Figure 2). The Figure shows a ribbon diagram of the PDHK-2 structure. The N-terminal regulatory domain is shown in dark grey, the C-terminal catalytic domain in light grey. Ligands binding at various sites are shown as ball-and-stick models: ATP-Mg²⁺ in dark grey, Compound 1 in white, Compound 2 in black; Compound 3 in medium grey; DCA (hidden behind helix α6) in light grey. Termini and secondary structural elements are labelled. Helix α13 is hidden behind helices α3 and α5. The location of a groove between the regulatory and catalytic domain, comprising the potential binding site for a helix of the substrate protein, is indicated by a rectangle.

The structure of the N-terminal domain (residues 14-177) consists of eight helices with a distorted, antiparallel four-helix bundle core. The core is formed by helices α3, α5, α6 + α7, and α8. The helices α1 and α2 precede the central four-helix bundle, whereas the short helix α4 connects helices α3 and α5. A topological comparison of the PDHK-2 N-terminal domain with the PDB does not reveal significant additional homology with other known protein structures, apart from the four-helix bundle.

The C-terminal domain of PDHK-2 (residues 186-407) consists of five helices and eight strands. It is connected to the regulatory domain by eight residues, which are disordered in the crystal structure. The central motif is an open, five-stranded, mixed β-sheet with a right handed twist of approximately 40°. It is formed by strands β3, β5, β6, β8 and β7 (directions ++-+-) and flanked on one side by a layer of helices (α9, α10, α11 and α12). On the same side but oriented perpendicular to the central β-sheet, strands β1, β2 and β4 form an additional, antiparallel β-sheet.

The C-terminal domain of PDHK-2 belongs to the GHKL superfamily (for a review see Dutta, R & Inouye, M. (2000) *Trends Biochem. Sci.* **25,** 24-28), named after the prototypical ATP binding domains of Gyrase B (Wigley, D.B. et al. (1991) *Nature* **351,** 624-629; Brino, L. et al. (2000) *J. Biol. Chem.* **275,** 9468-9475), *H*sp90 (Obermann, W.M.J. et al., (1998) *J. Cell Biol.* **143,** 901-910), bacterial histidine kinases (Tanaka, T. et al. (1998) *Nature* **396,** 88-92; Bilwes, A.M. et al. (1999) *Cell* **96,** 131-141), and Mut*L* (Ban, C. et al. (1999) *Cell* **97**, 85-97). Superposition of the homologous domains identifies a 5-stranded mixed β-sheet (corresponding to strands β4, β5, β6, β8 and β7 of PDHK-2) and 4 helices (corresponding to α9, α10, α11 and α12 of PDHK-2) as the conserved structural motif. While PDHK and histidine kinases have identical topologies, the first strand of the central β-sheet and the preceding helix (β4 and α9 in PDHK-2) are transposed to the C-terminus of GyrB, Hsp90, and MutL. The latter also contain an insertion of two antiparallel β-strands between the fifth and the fourth strand of the conserved β-sheet.

The 40 C-terminal residues of PDHK-2, including helix α13, are an extension of the globular ATP binding domain that interact with the regulatory domain and a symmetry related molecule (see below). The interactions between the N-terminal and ATP binding domains of PDHK-2 can be summarised as follows: Loop α1-α2 interacts with loop β8-α13. Helix α2 interacts with strands β1 and β2. The C-terminal portion of helix α3 interacts with loop β8-α13 and helix α13. Helix α7 interacts with loop α11-α12. Helix α8 interacts with loop α11-α12, helix α12 and helix α13.

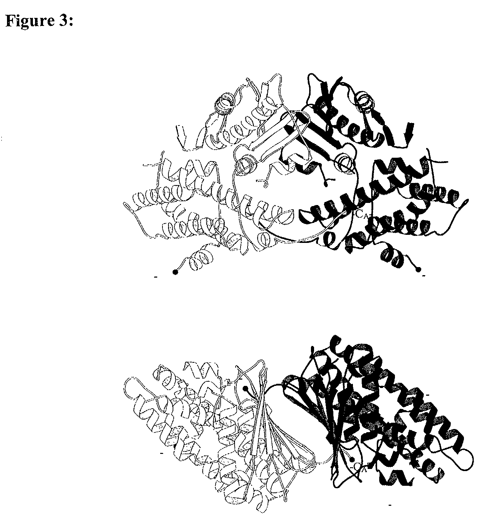

Between the regulatory and catalytic domain is a large, straight groove, with approximate dimensions of 25 Å in length and 10 Å in diameter (see Figure 2). The proportion of the groove as well as its vicinity to the ATP binding site suggests that it could be the binding site for a helix of the substrate protein. However, based on the x-ray structure from rat and a model of the lipoyl domain (L2) from rat, it has been proposed that the groove between the regulatory and catalytic domain is the binding site for the lipoyl domain (L2) of the PDH complex (Steussy et al (2001), J. Biol Chem. **276** (40), 37433 - 37450. Dimer assembly: In the crystal structure, two molecules of PDHK-2 are related by a crystallographic two-fold rotation axis (see Figure 3, showing monomer A in white, monomer B in black, viewed with the crystallographic two-fold rotation axis vertical in the plane of the paper (top) and along the two-fold rotation axis (bottom). The termini, as far as visible in the electron density maps, of both monomers are labelled.) The corresponding interface buries 2040 Å² of surface area on each molecule, suggesting that it is a biologically relevant molecular dimer interface (Janin, J. (1997) *Nat. Struct. Biol.* **4,** 973-974). Independent evidence for the possible dimeric assembly of PDHK is provided by the finding that the protein, when purified from bovine kidney or rat heart, is a heterodimer of isozymes 1 and 2 (Stepp, L.R. et al. (1983) *J. Biol. Chem.* **258,** 9454-9458; Popov, K.M. et al. (1991) *Protein Expression Purif*. **2,** 278-286; Popov, K.M. et al. (1994) *J. Biol. Chem.* **269,** 29720-29724; Liu, S., Baker, J.C. & Roche, T.E. (1995) *J. Biol. Chem.* **270,** 793-800).

The main contact is formed by the central β-sheets of the catalytic domains. In addition, the 40 C-terminal residues (including helix α13) of PDHK form a highly flexible arm (as indicated by the weak electron density for this region), extending from the catalytic domain of one monomer to the symmetry-related molecule. The following residues are involved in the dimer interaction (i.e. are within 4 Å distance of the symmetry related monomer): residues 28 and 30 of loop α1-α2; residues 153, 157, and 160 of helix α8; residues 227 and 229 of strand β3 and loop β3-β4; residue 276 of strand β5; residues 280-282 of loop β5-β6; residues 283, 285 and 287 of strand β6; residues 296-298 of loop β6-α11 and helix α11; residues 343, 347 and 348 of strand β7; residues 349-352 of loop β7-β8; residues 355 and 359 of strand β8; residues 370-373 of loop β8-α13; residues 375, 377, 378 and 380 of helix α13; residues 381, 385 and 388-392 of the flexible C-terminal dimerisation arm.

The crystal structure reported here could be used to design ligands binding at the proposed dimerisation interface. If dimer formation of PDHK is required for effective PDH complex inactivation, it could be possible to modulate PDH complex activity by regulating the amount of PDHK dimerisation.

### (b) Description of the ATP binding site: Target for competitive inhibition

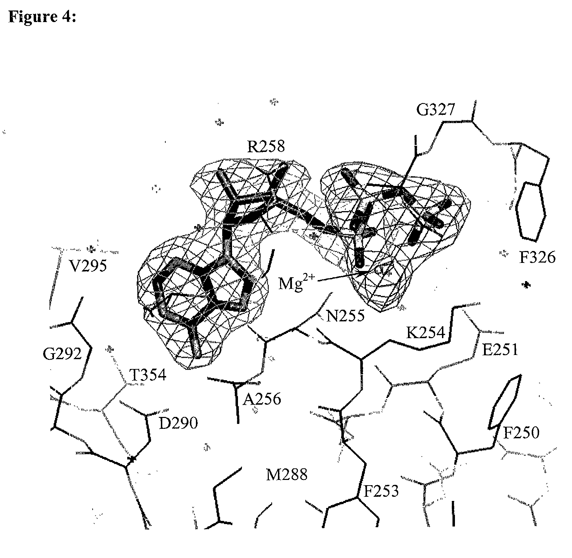

The structure of PDHK-2* was determined in the presence of ATP and synthetic ligands bound at the ATP binding site of the catalytic domain (see Figure 4, showing ATP (thick) and a Mg²⁺ ion together with coordinating water molecules at the ATP binding site of PDHK-2. The model is superimposed onto the f_{obs}-f_{calc} difference density map at 2.4 Å (contour level: 2.5σ) calculated prior to incorporation of the ATP: Mg²⁺ complex in the model.). The binding pocket is located between the central β-sheet and the adjacent layer of helices. It is formed by helix α10 (Glu251, Leu252, Lys254, Asn255, Ala256, Arg258, Ala259), strand β7 (Met288, Ser289, Asp290), loop β7α11 (Gly292, Gly293, Gly294, Va1295), helix α11 (Ile300, Leu303), loop α11α12 (Leu323, Ala324, Gly325, Phe326), helix α12 (Gly327, Tyr328, Gly329, Leu330, Pro331), strand β7 (Leu346, Ser348) and strand β9 (Thr354, Asp355, Ala356). Residues 313 to 327 (located between helices α11 and α12), constitute the major portion of the so-called ATP-lid. In the absence of ATP they are structurally disordered. Upon binding of ATP residues 323 to 327 become ordered, forming backbone interactions with the β- and γ-phosphate groups of ATP.

The mode by which ATP binds to PDHK-2 closely resembles that of ATP or ADP binding to Gyrase B (Wigley, D.B. et al. (1991) *Nature* **351,** 624-629; Brino, L. et al. (2000) *J. Biol. Chem.* **275,** 9468-9475), Hsp90 (Obermann, W.M.J. et al., (1998) *J. Cell Biol.* **143,** 901-910) and MutL (Ban, C. et al. (1999) *Cell* **97**, 85-97). The binding site can be subdivided into two parts: a polar groove in the surface, binding the phosphoribose moiety of the nucleotide, and a spacious hydrophobic pocket with a polar floor, which accommodates the adenine base. Residues Val295, Leu303, Leu346 and Thr354 line one face of the adenine ring and Asn255, Ala256 and Ala259 flank the opposite face. Asp290 is a conserved, key recognition feature for ATP within the GHKL superfamily. Its carboxylate group acts as hydrogen bond acceptor for the 6-amino group of the adenine ring. Further water-mediated hydrogen bonds complete the interactions between the adenine ring and the protein (Figure 4).

The ribose and the triphosphate bind to a groove in the surface of the catalytic domain and form direct and indirect interactions with the protein. The 4'-oxygen atom of the ribose ring is in Van der Waals contact with Leu303, whilst the 2'- and 3'-hydroxyl groups form direct or water mediated hydrogen bonds to the side chain of Arg258. The 3'-hydroxyl group stabilises the conformation of the nucleotide by forming an intramolecular hydrogen bond to the β-phosphate group. Binding of ATP is accompanied by binding of a Mg²⁺ ion. Each phosphate group contributes one oxygen atom to the octahedral coordination of the Mg²⁺ ion, which is completed by the Oδ1 atom of Asn255 and two water molecules (Figure 4). Due to the proximity to the N-terminus of helix α12 the negative charges of the tri-phosphate are further compensated by the positive dipole moment of this helix.

It is worth noting that ATP soaked into crystals of PDHK-2* is not hydrolysed. In contrast, GyrB, Hsp90 and MutL show at least moderate ATPase activity (Wang, J.C. (1996) *Annu. Rev. Biochem.* **65,** 635-692; Obermann, W.M.J. et al., (1998) *J. Cell Biol.* **143,** 901-910; Ban, C. & Yang, W. (1998) *Cell* **95,** 541-552). The differences in their catalytical properties and topology allow the distinction between ATPases (GyrB, Hsp90 and MutL, also classified as GHL subgroup) and kinases (histidine kinases and PDHK) within the GHKL superfamily (Ban, C. et al. (1999) *Cell* **97**, 85-97; Dutta, R & Inouye, M. (2000) *Trends Biochem*. *Sci.* **25,** 24-28).

### (c) Description of the putative E₂L₂ binding site

We have determined the structure of PDHK-2* in complex with synthetic PDHK inhibitors, Compound 1 (4-{(2,5)-dimethyl-4-[3,3,3-trifluoro-2-hydroxy-2-methylpropanoyl]piperazinyl}carbonyl)benzonitrile; Aicher. T. et al. (1999) *J*. *Med. Chem.* **42,** 2741-2746) and Compound 2 (N-{4-[(ethylanilino)sulfonyl]-2-methylphenyl}-3,3,3-trifluoro-2-hydroxy-2-methylpropanamide; Example 12 in patent application WO 99/62873), and identified their binding site (see Figures 1, 2 and 5). It resides on the regulatory domain of PDHK and is formed by residues Leu31, Gln35, Phe36, Asp38, and Phe39 of helix α2 and the preceding loop α1α2, residues Thr48, Ser49, Phe52 and Leu53 of helix α3 as well as residues Met167, Leu168, Cln171, His172, Ile175, and Phe176 of helix α8. Loop α1α2, helix α2 and helix α3 form a triangle, the bottom of which is lined by the C-terminal portion of helix α8.

Although both compounds prevent ATP dependent inhibition of the PDH complex, as does exogenous E₂L₂ (Jackson, J.C. (1998) *Biochem. J.* **334,** 703-711), they do not inhibit the kinase activity of PDHK when using a PDHK specific substrate peptide (data not shown). The structure of PDHK-2 shows that the binding pocket of Compounds 1 and 2 is predominantly hydrophobic, which has also been proposed for the site of the lipoyl domain of the E₂ component (E₂L₂) of the PDH complex (Jackson, J.C. (1998) *Biochem. J.* **334**, 703-711). We therefore believe, that the binding site of Compounds 1 and 2 could be identical with, or part of the E₂L₂ binding site of PDHK. Accordingly, this class of compounds could inhibit ATP dependent phosphorylation of the PDH complex by blocking the association between PDHK and the E₂L₂ component of the PDH complex.

The comparison of PDHK native and complex crystal structures reveals conformational differences. In the presence of ligands helix α2 is shifted by a hinge motion around the Cα position of Met33. This opens the binding pocket and allows accommodation of ligands at the site. The conformational differences are most pronounced for residue Phe39. In the absence of ligands the benzene ring of Phe39 occupies the space that otherwise is filled by the central portion of the ligand. Ligand binding induces a 2-2.5 Å shift of its Cα position and flipping of the side chain. The loop α2-α3 (residues 42-45) is found to be very flexible in all structures determined to date. This may be necessary to facilitate the hinge movement of helix α2.

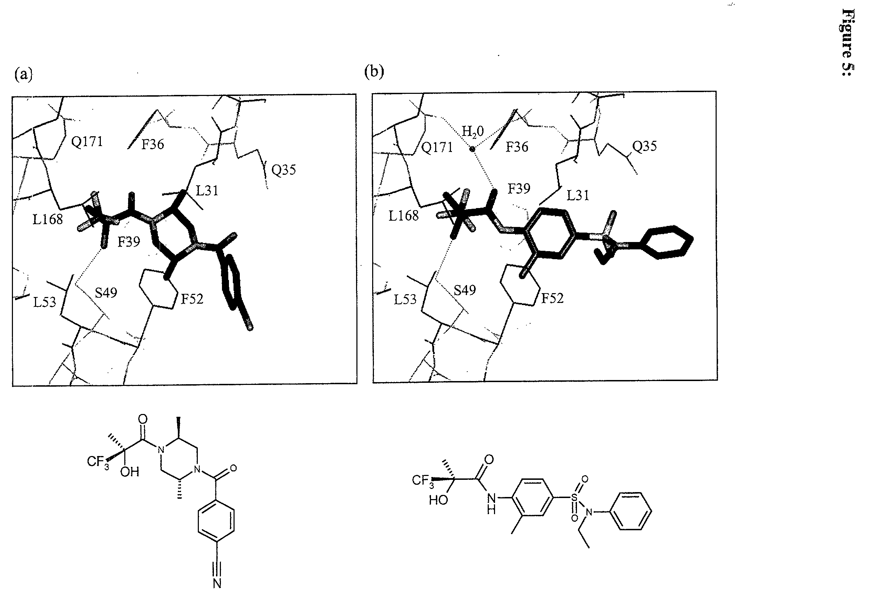

In both crystal structures the *(R)*-3,3,3-trifluoro-2-hydroxy-2-methylpropionyl moiety of the ligands binds in a similar manner (see Figures 5a and 5b). A key recognition feature is a hydrogen bond between the side chain hydroxyl of Ser49 and the 2-hydroxyl group of the ligand. The 2-methyl group extends into a small hydrophobic sub-pocket enclosed by residues Phe39, Ser49, Gln171, His172, Ile175 and Phe176. The trifluoromethyl group forms van der Waals interactions with the side chains of residues Phe52, Leu53, Phe36, Leu168 and Gln171. In the structure with Compound 1, but not with Compound 2, the side chain of Leu31 is also in contact with the trifluoromethyl group, due to steric clashes with its 2,6-dimethylpiperazine group. The amide oxygen of Compound 2 forms a hydrogen bond to a buried water molecule, which bridges the backbone carbonyl of Phe36 and atom Nε2 of Gln171. However, this water molecule could not be detected in the presence of Compound 1 and other inhibitors of this class (data not shown), indicating that it is not essential for ligand binding. The central portion of the ligands is located in a short hydrophobic channel, consisting of residues Leu31, Gln35, Phe39, Ser49 and Phe52, that connects the (*R*)-3,3,3-trifluoro-2-hydroxy-2-methylpropionyl binding pocket with the protein surface. The 2,6-dimethylpiperazine moiety of Compound 1 and correspondingly the methylbenzyl group of Compound 2 form hydrophobic interactions with the protein. The 4-cyanophenylketone of Compound 1 and the N-benzyl-N-ethylsulfonamide of Compound 2 point towards solvent and are partially disordered in the crystal structures, indicating their flexibility.

Analysis of the aligned sequences of the four PDHK isozymes reveals a residue difference between PDHK isozymes 1-3 with respect to isozyme 4 (Rowles, J. et al (1996) *J. Biol. Chem.* **271**, 22376-82; GenBank accession U54617) at the putative lipoyl binding site.
PDHK-2 displays a phenylalanine in position 36, as do PDHK-1 and PDHK-3 at the equivalent positions, but PDHK-4 has a leucine (Leu40) in the equivalent position. This residue exchange has been shown to contribute to the observed potency difference of an analogue of Compound 1 against PDHK-2 and PDHK-4 (data not shown). Furthermore, a single nucleotide polymorphism (SNP) has been identified in some individuals resulting in a Phe43Leu exchange in PDHK-4. The data we present here show that the equivalent residue in PDHK-2 (Phe39) is directly involved in binding of compounds 1 and 2. We therefore conclude, that this SNP could significantly influence the binding affinity of natural or synthetic ligands to the putative E₂L₂ binding site of effected PDHK-4 variants. This analysis highlights the potential of using the described structure of PDHK-2 to identify residues that are critical for ligand binding, and to use this information to genetically engineer site-directed mutants of PDHK-2, designed to mimic any other PDHK isozyme.

### (d) Description of a novel ligand binding site

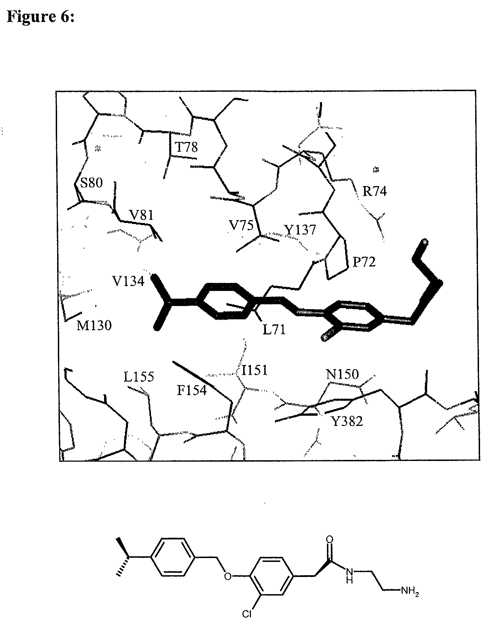

Soaking of PDHK crystals with another ligand (Compound 3) has led to the identification of a novel, additional ligand binding site (also termed third binding site) on the regulatory domain of PDHK-2 (see Figure 6 which shows a view of Compound 3 bound to the enzyme, and the structure of Compound 3). The site, which is pre-formed in the native protein, is constituted by Leu71, Pro72, Arg74, Val75 of loop α3-α4 and helix α4, Thr78, Ser80, Val81 of loop α4-α5 and helix α5, Met130, Gly133, Val134, Glu136, Tyr137 of helix α7, Asp144, Val146, Ser147, Asn150, Ile151, Phe154, Leu155 of loop α7-α8 and helix α8 as well as Tyr382 at the C-terminus of helix α13.

### (e) Identification and description of a DCA binding site

A binding site of DCA on PDHK-2 has been identified by calculating a difference fourier map with [f_{obs} (ADP/DCA) - f_{obs} (native)] expiα_{calc} (native) as coefficients, where f_{obs} (ADP/DCA) are the observed structure factor amplitudes of PDHK-2* soaked with both ADP and DCA, f_{obs} (native) are the observed structure factor amplitudes of unliganded PDHK-2*, and α_{calc} (native) are the phases derived from the refined model of unliganded PDHK-2* (Example 6).

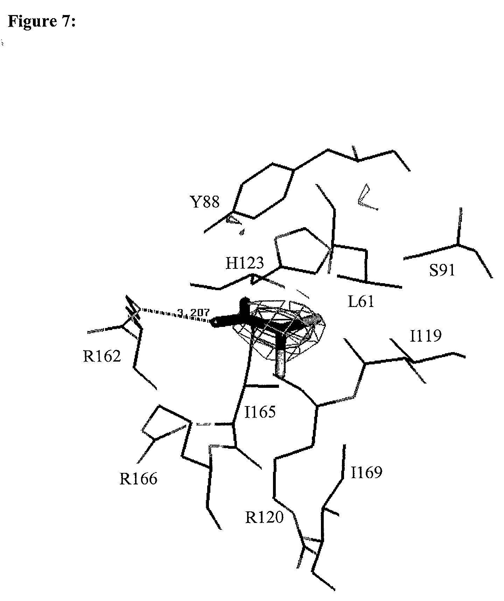

The resulting f_{obs}(*ADP, DCA*)-f_{obs}(*native*) electron density map contains a peak greater than 5.5 σ in a small pocket of the regulatory domain of PDHK-2 (Figure 7). Figure 7 shows a refined model of DCA bound to the newly identified binding site of PDHK-2. The model is superimposed onto the 2.5 Å f_{obs}(*ADP, DCA*)-f_{obs}(*native*) difference density map contoured at 4σ (light grey) and 5σ (dark grey). A hydrogen bond between DCA and Arg162 is indicated. The pocket is formed by residues Leu57, Leu61, Tyr88, Ser91, Ile95, Ile119, Arg120, His123, Ser161, Arg162, Ile165, Arg166, and Ile169. The carboxylate group of DCA forms a salt-bridge with Arg162, while the two chlorine substituents are deeply buried in the hydrophobic portion of the pocket. The binding pocket is close to the surface of the groove between the catalytic and the regulatory domain of PDHK-2 (see Figure 2), in the vicinity of a cluster of conserved arginine residues (Arg120, Arg162, Arg166). The f_{obs}(*ADP, DCA*)-f_{obs}(*native*) map does contain a further peak greater than 4 σ at the position of the β-phosphate of ADP as well as less dominant peaks for other parts of the bound ADP molecule. A peak of more than 4 σ is also observed for the side chain of Glu251, which is involved in the coordination of the ADP associated Mg²⁺ ion and is flipped with respect to its conformation in the absence of ADP/Mg²⁺. Since the f_{obs}(*ADP, DCA*)-f_{obs}(*native*) electron density map does not contain further significant peaks, we conclude that the described DCA binding pocket is the only, or at least the major binding site of DCA on PDHK-2.

The shape (see Example 8) and charge distribution of the groove as well as the high occurence of conserved residues on its surface suggest that it is of functional importance, e.g. for binding of PDHK to a subunit or domain of the PDH complex. We therefore hypothesize that DCA could inhibit the phosphorylation of the PDH complex, by interfering with the binding of one of its components to PDHK.

The DCA binding pocket of PDHK-2 identified herein could be utilised for the design of synthetic ligands that inhibit phosphorylation of the PDH complex.

### Example 9: Assay to measure whether compounds inhibit PDHK activity

Compounds were assessed for inhibition of PDHK in an assay adapted from Espinal, J. et al (1995) *Drug Development Res.* **35,** 130-136. In the first part of the assay, compounds were incubated with porcine PDH (Sigma P-7032) and MgATP, and the reaction was stopped after defined intervals. In the second part of the assay, the residual PDH activity was measured by monitoring the rate of reduction of NAD at 340nm.

The assay was performed in flat-bottom 96-well microtitre plates, using a final volume of 50µl for the first part of the assay (kinase reaction), and 250µl for the PDH reaction. At first, 10µl test compound in a suitable diluent, 20µl kinase buffer (20mM potassium phosphate, pH7.0, 4mM MgCl₂, 4mM dithiothreitol) and 10µl PDH (Sigma, P-7032, diluted with water to a final concentration of 1.83mg/ml) were mixed and incubated for 10 min; then 10µl ATP solution (125µM in kinase buffer) was added to start the reaction.

The incubations were done at 30°C. The kinase reaction was terminated after defined intervals (usually 0, 10, 20, 30, 60, and 90 min) by initiating the PDH reaction. This was done by addition of 200µl of PDH substrate solution (1mM thiamine pyrophosphate, 0.5mM NAD, 0.5mM coenzyme A, 12mM pyruvate, 5mM dithiothreitol). Kinetic readings were then taken at 340nm conducted at 12 sec intervals for 20 min with shaking for 1 sec before each reading was taken, in a SpectraMax plate reader. The data were processed using the Spectramax software.

### Example 10: Synthesis of Compound 3

### (a) Methyl {3-chloro-4-[(4-isopropylbenzyl)oxy]phenyl}acetate

A stirred solution of 4-isopropylbenzyl alcohol (4.5ml, 29.1mmoles), 3-chloro-4-hydroxybenzeneacetic acid, methyl ester (CAS registry number 57017-95-5) and triphenylphosphine (8.8g, 33.6mmoles) in dry tetrahydrofuran (100ml) was cooled to 0°C. Diisopropyl azodicarboxylate (5.7ml, 29.1mmoles) was added dropwise over 10 minutes and the reaction was stirred for a further 3 hours at room temperature. The solvent was removed under reduced pressure and the product purified on silica, eluting with pentane: ethyl acetate (4:1) to yield the title compound as a colourless oil (2.2g, 30%).
¹H NMR (400MHz, CDCl₃) δ 1.25(6H, d), 2.90(1H, m), 3.55(2H, s), 3.70(3H, s),
5.10(2H, s), 6.95(1H, d), 7.10(1H, d), 7.22(2H, d), 7.30(1H, s), 7.38(2H, d). ms 350, 352 (MNH₄⁺ for both Cl isotopes).
Found: C, 67.95; H, 6.47. C₁₉H₂₁ClO₃.0.2 ethyl acetate requires C, 67.86; H, 6.50.

### (b) 3-Chloro-4-[(4-isopropylbenzyl)oxy]phenyl}acetic acid

A mixture of methyl {3-chloro-4-[(4-isopropylbenzyl)oxy]phenyl}acetate (2.2g, 6.6mmoles) in aqueous sodium hydroxide (1M, 20ml) and dioxan (20ml) was stirred at room temperature for 1 hour. The dioxan was then removed under reduced pressure, the remaining aqueous solution acidified with aqueous hydrochloric acid (1M, 25ml) and the resultant white solid isolated by filtration. The solid was then azeotroped with ethanol to afford the title compound as a white solid (1.9g, 90%).
¹H NMR (300MHz, CDCl₃) δ 2.15(6H, d), 2.95(1H, m), 3.57(2H, s), 5.10(2H, s), 6.95(1H, d), 7.10(1H, d), 7.22(2H, d), 7.30(1H, s), 7.38(2H, d).
ms 336 (MNH₄⁺)
Found: C, 66.87; H, 6.23. C₁₈H₁₉ClO₃.0.4 ethanol requires C, 66.96; H, 6.40.

### (c) tert-butyl 2-[({3-chloro-4-[(4-isopropylbenzyl)oxy]phenyl}acetyl)amino] ethylcarbamate

3-Chloro-4-[(4-isopropylbenzyl)oxy]phenyl}acetic acid (0.240g, 0.75mmoles), N-methylmorpholine (0.240ml, 2.5mmoles), O-(7-azabenzotriazol-1-yl)-N,N,N',N'-tetramethyluronium hexafluorophosphate (0.32g, 0.85mmoles) and N-BOCethylenediamine (0.12ml, 0.75mmole) were stirred in acetonitrile at room temperature for 72 hours. The solvent was removed under reduced pressure. Purification using flash chromatography on silica eluting with pentane: ethyl acetate (1:2) yielded the title compound (0.185g, 54%), which was used without further purification.
¹H NMR (400MHz, CDCl₃) δ 1.21(6H, d), 1.37(9H,s), 2.87(1H, m), 3.18(2H, m), 3.27(2H, m), 3.41(2H, s), 4.80(1H, s), 5.04(2H, s), 6.08(1H, s), 6.90(1H, d), 7.04(1H, m), 7.24(3H, m), 7.35(2H, d).
ms 483 (MNa⁺)

### (d) N-(2-aminoethyl)-2-{3-chloro-4-[(4-isopropylbenzyl)oxy]phenyl}acetamide

*tert*-Butyl 2-[({3-chloro-4-[(4-isopropylbenzyl)oxy]phenyl} acetyl)amino]ethylcarbamate (0.185g, 0.41mmoles) was dissolved in dichloromethane. The solution was saturated with gaseous hydrogen chloride and the reaction stirred at room temperature for 1 hour. The solvent was removed under reduced pressure. The residue was dissolved in dichloromethane, washed with saturated sodium hydrogen carbonate, dried (magnesium sulphate) and the solvent removed under reduced pressure. Purification using flash chromatography on silica eluting with dichloromethane: methanol: 0.880ammonia (90:10:1) yielded the title compound (0.035g, 24%) as a white solid.
¹H NMR (400MHz, CDCl₃) δ 1.24(6H, d), 2.78(2H, t), 2.92(1H, m), 3.24(2H, m), 3.46(2H, s), 5.10(2H, s), 5.88(1H, s), 6.94(1H, d), 7.07(1H, m), 7.23(2H, m), 7.30(lH,s), 7.37(2H, d).
ms ES- 359 (M-1)
Found: C, 63.46; H, 7.10; N, 7.30: C₂₀H₂₅ClN₂O₂.0.24 CH₂Cl₂ requires C, 63.76; H, 6.74; N, 7.35.

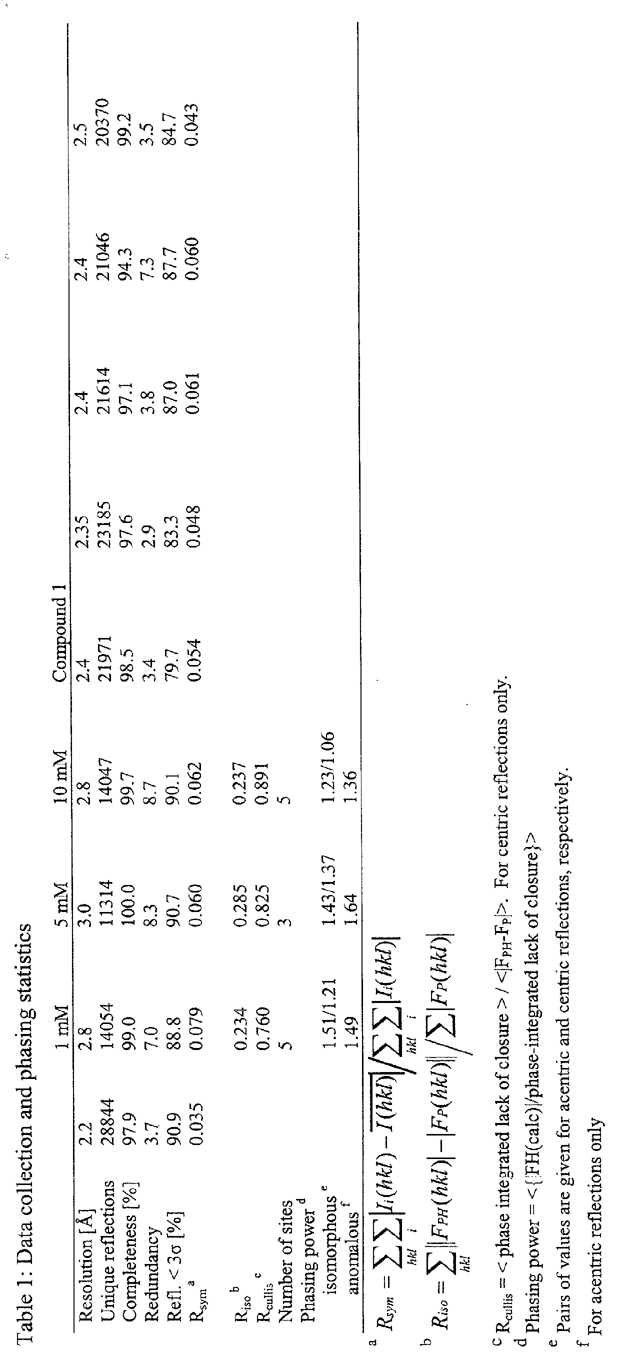

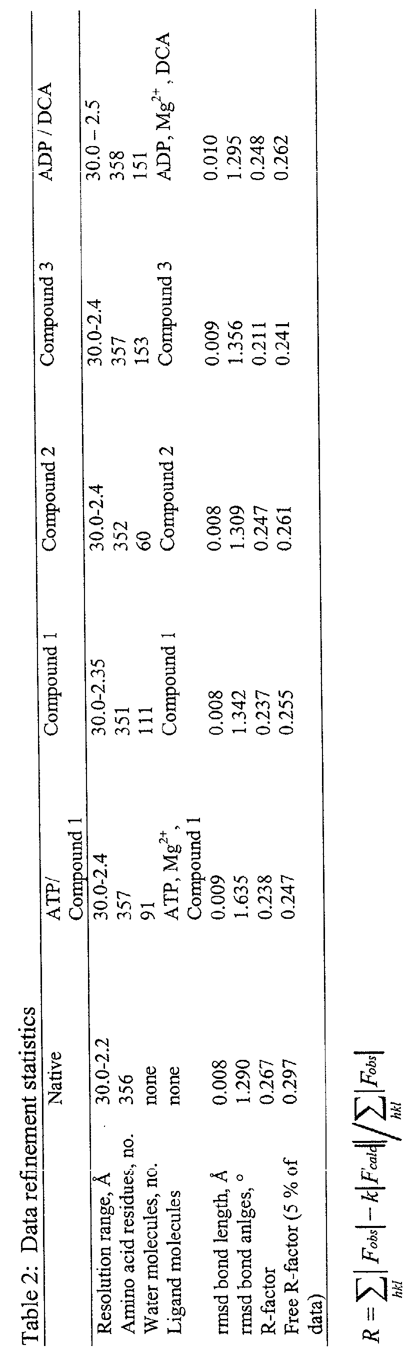

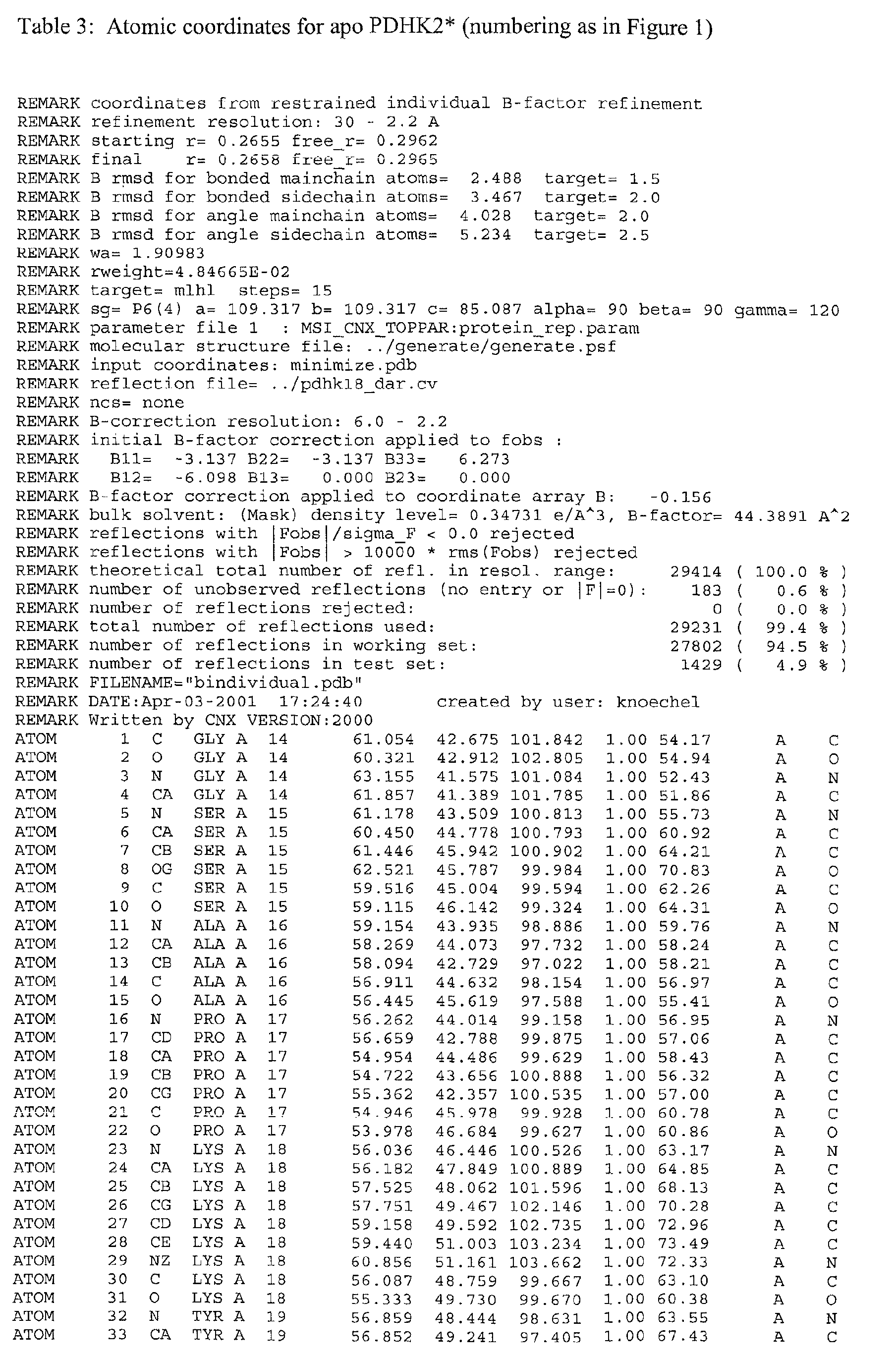

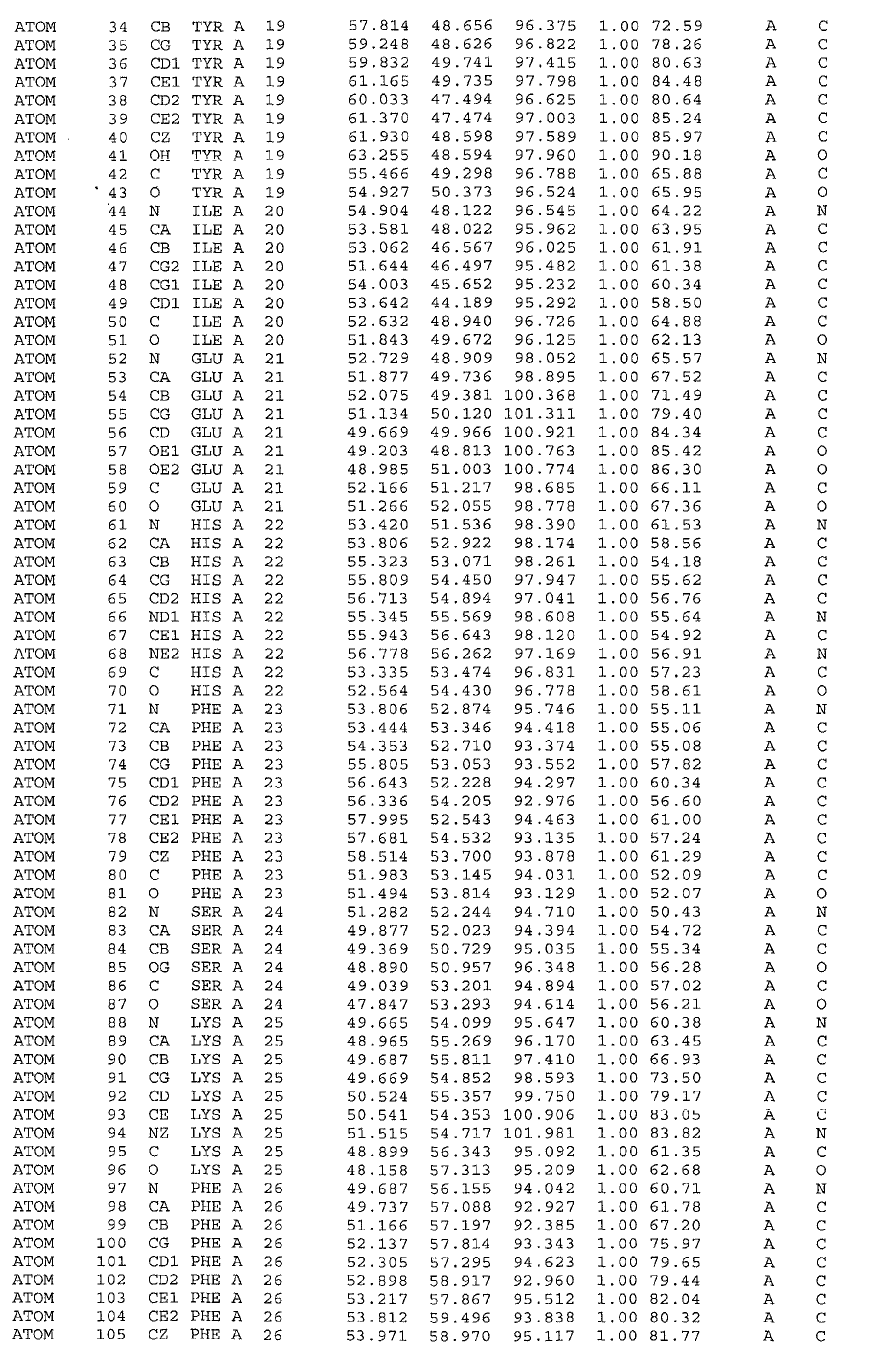

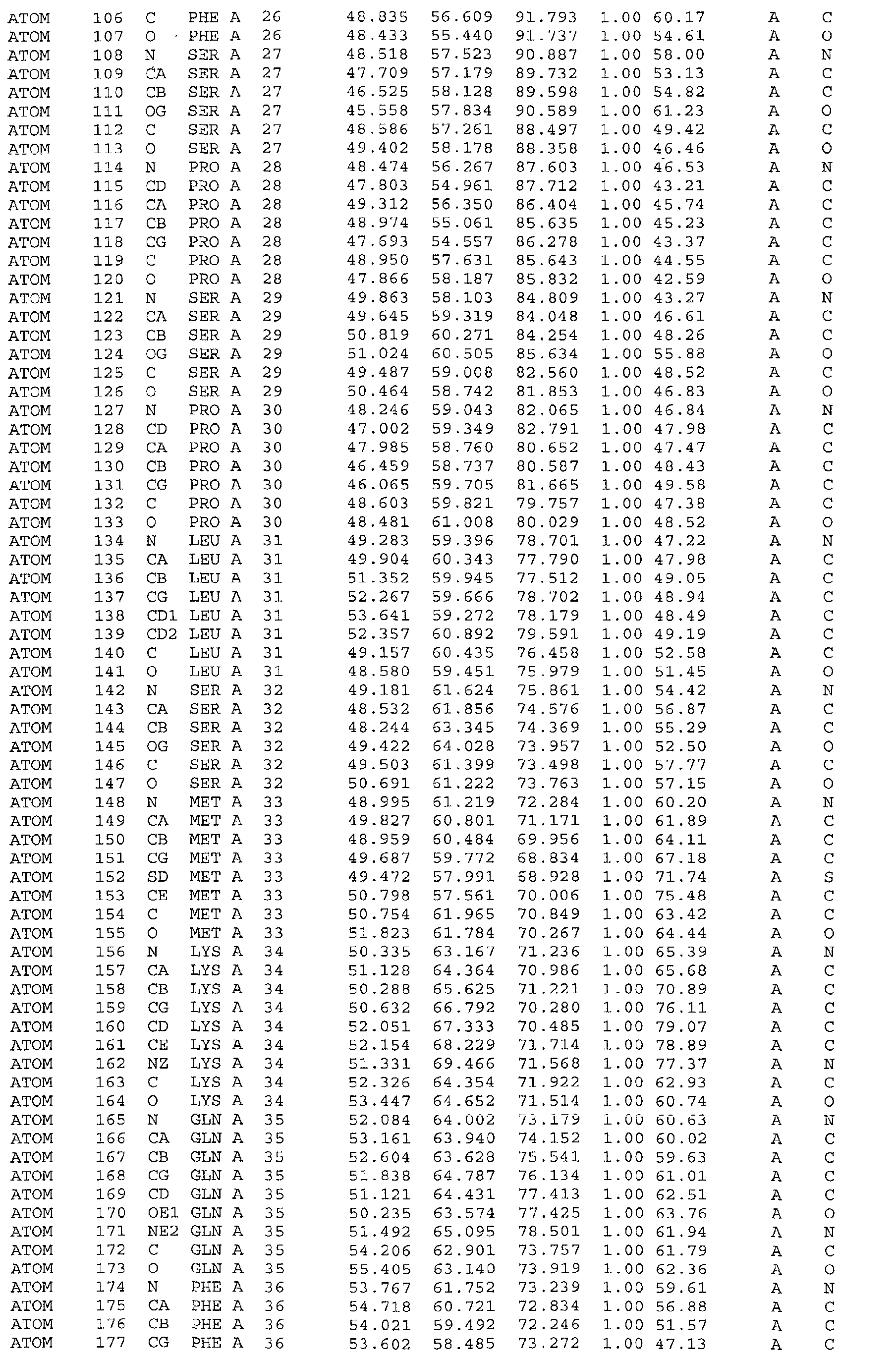

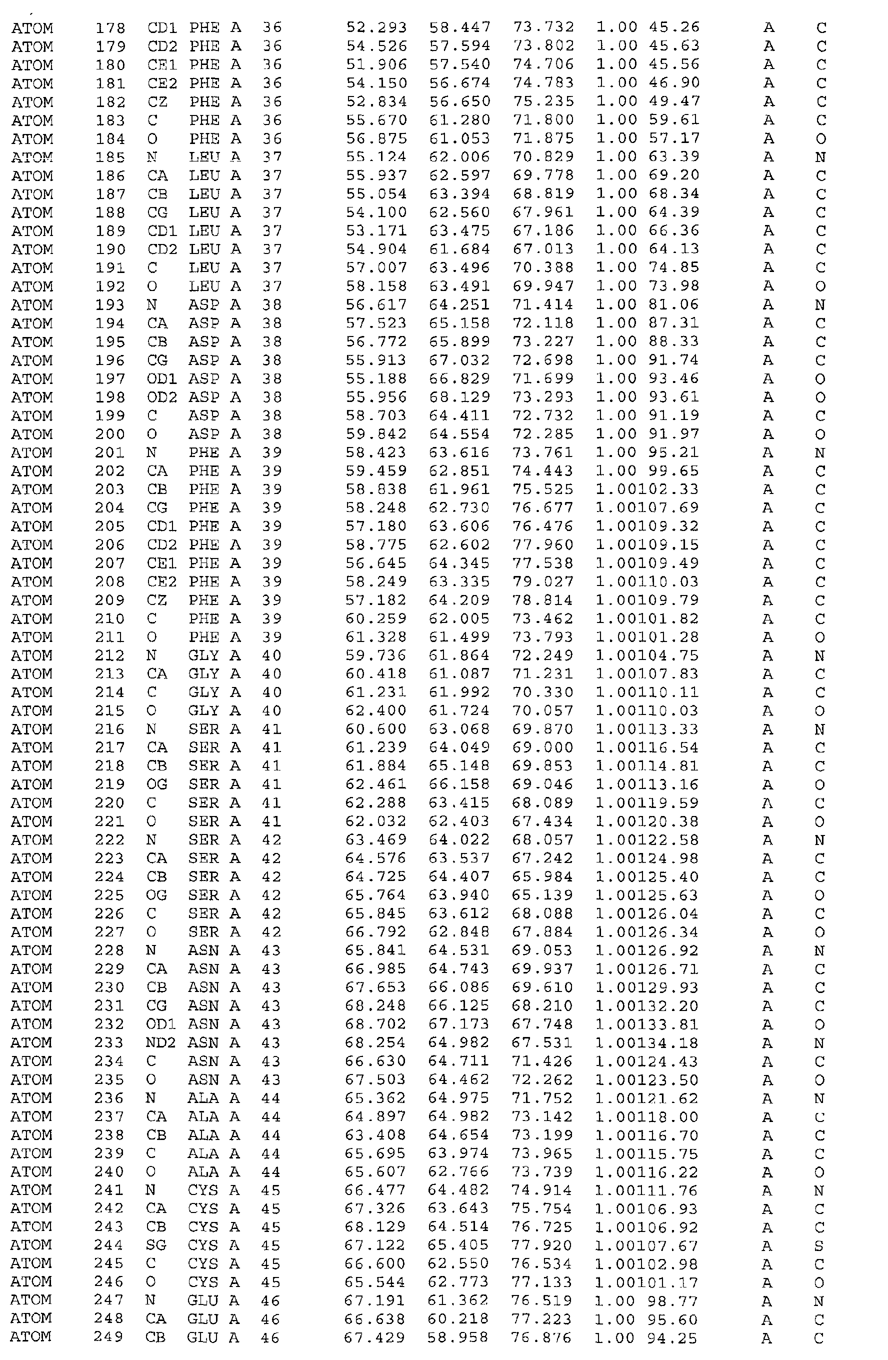

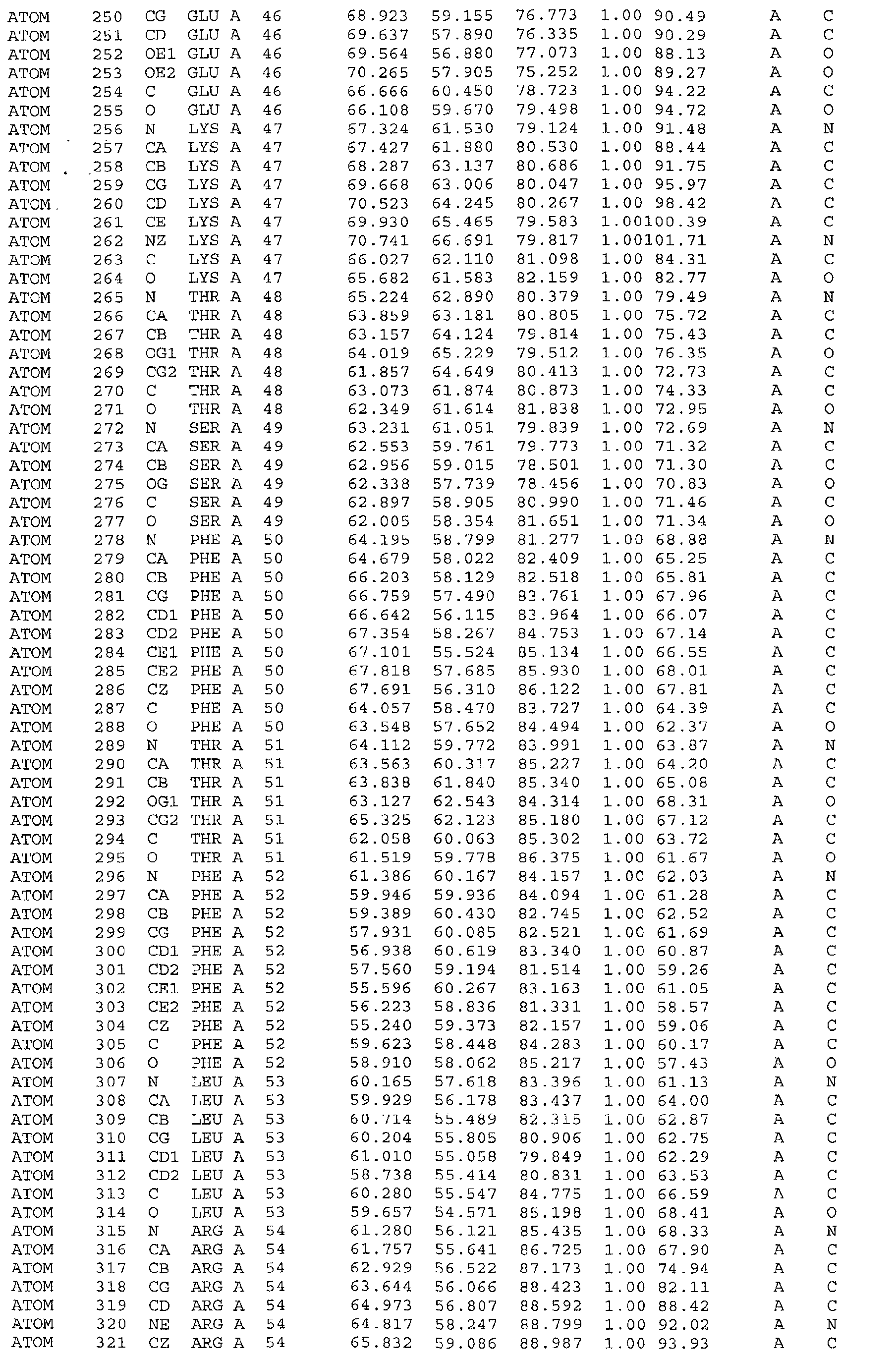

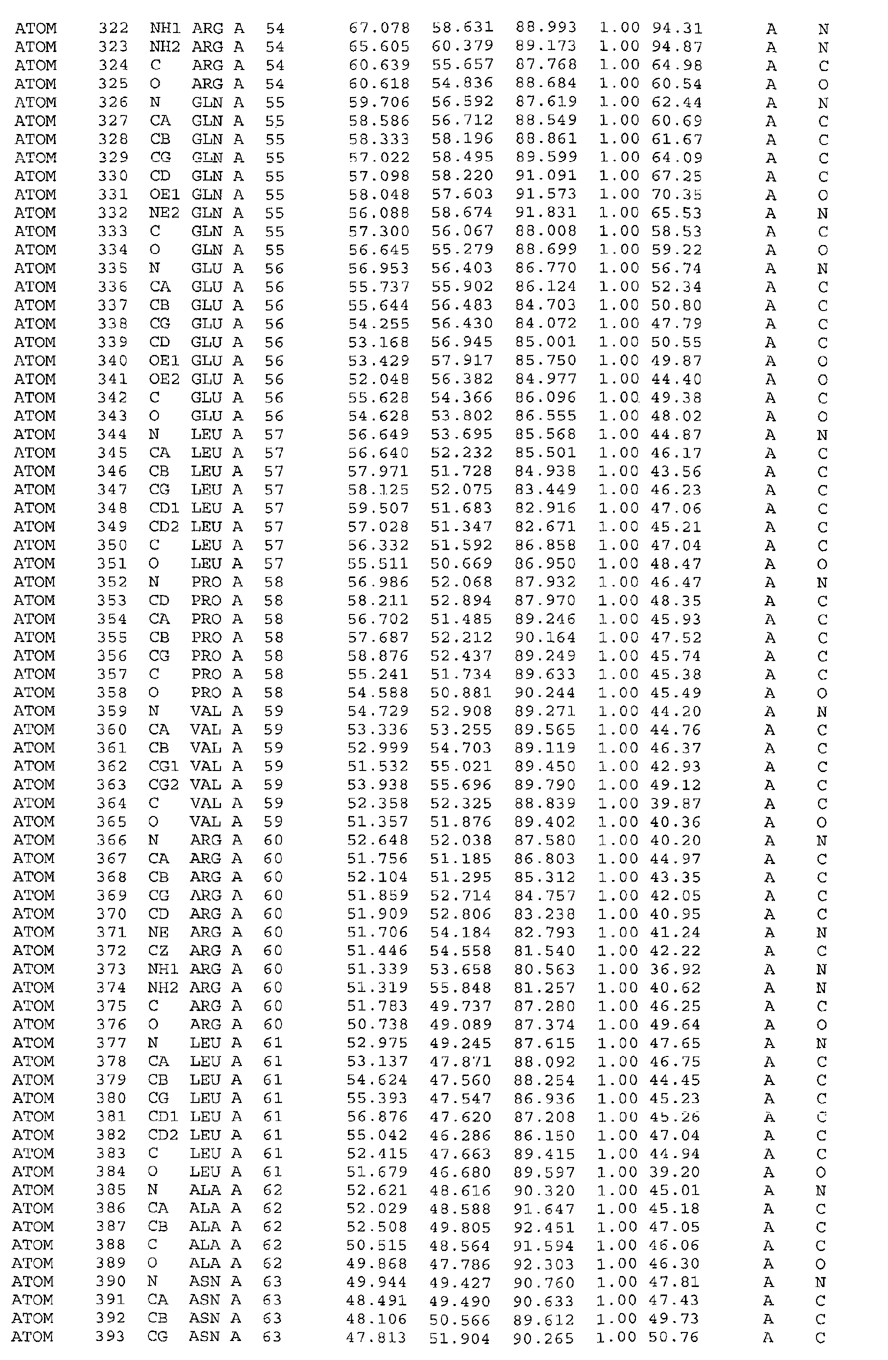

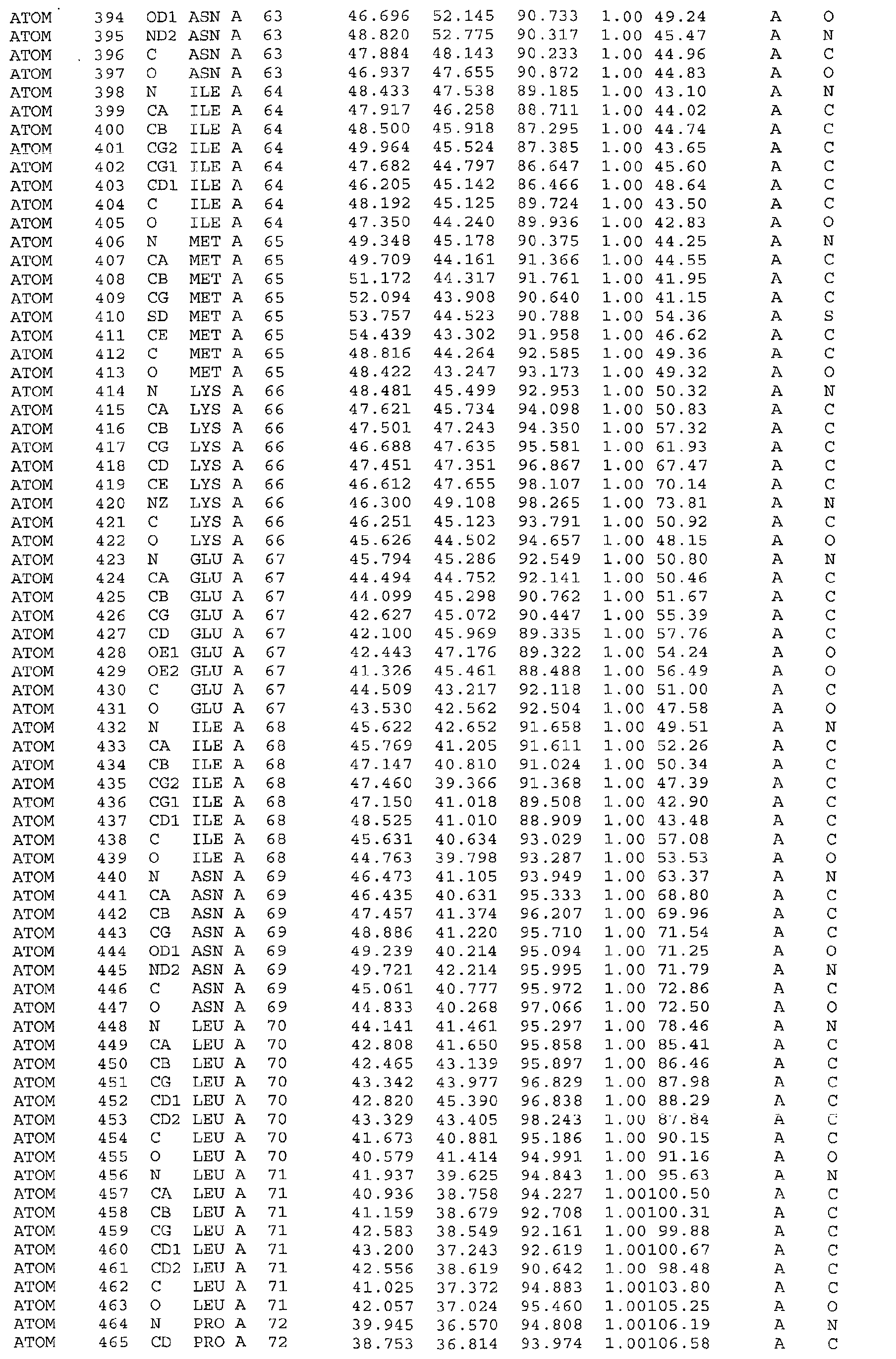

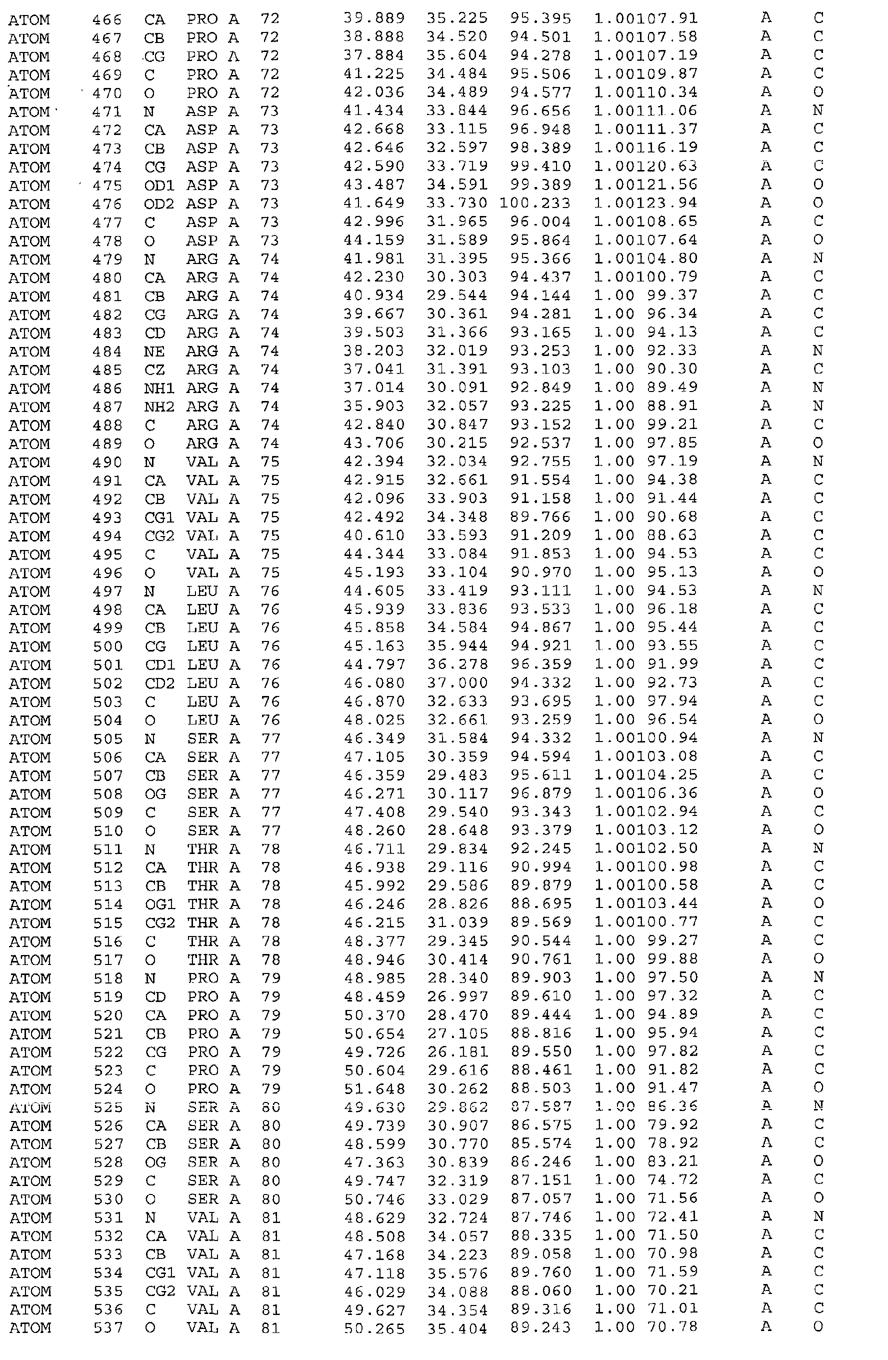

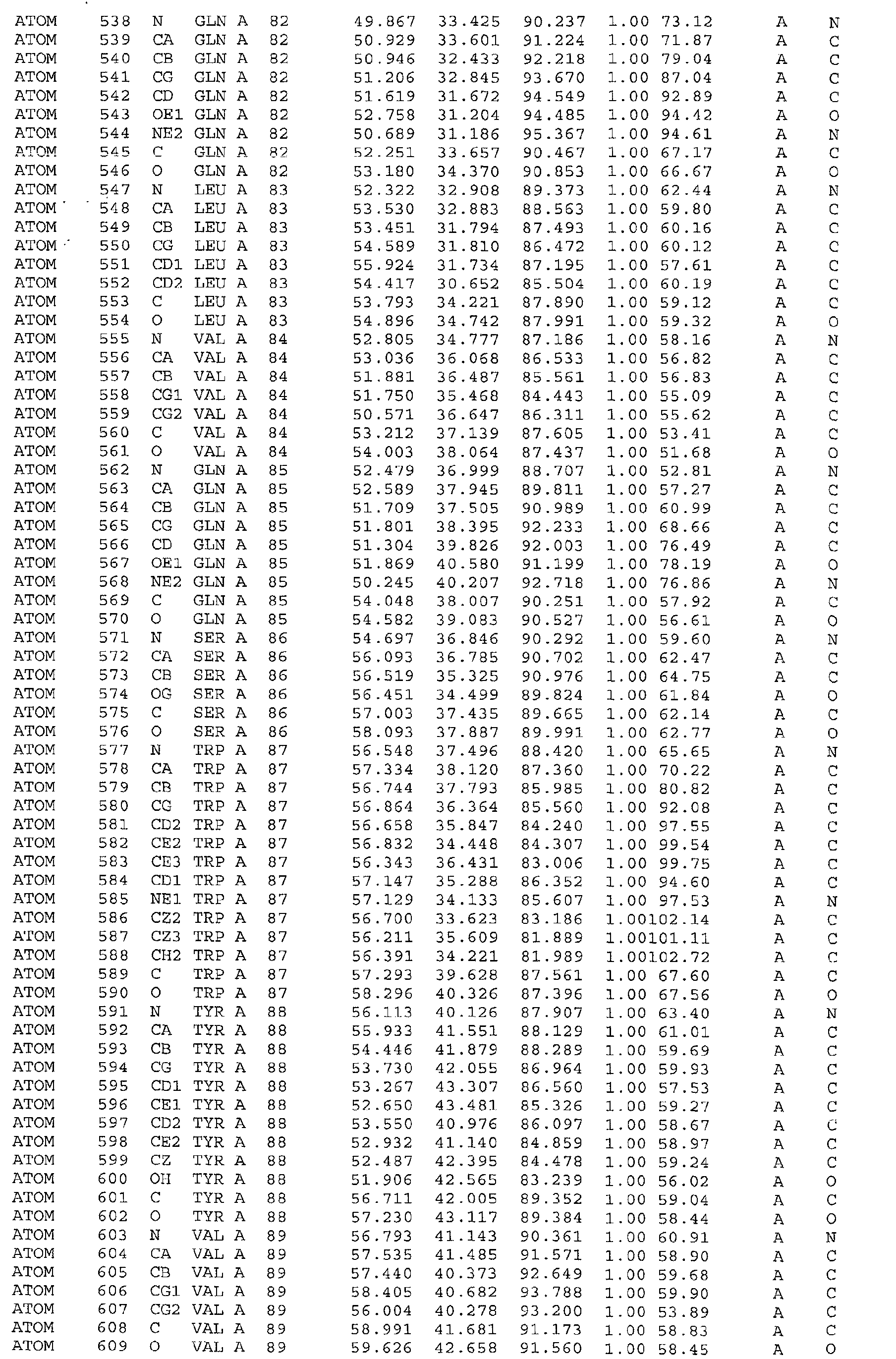

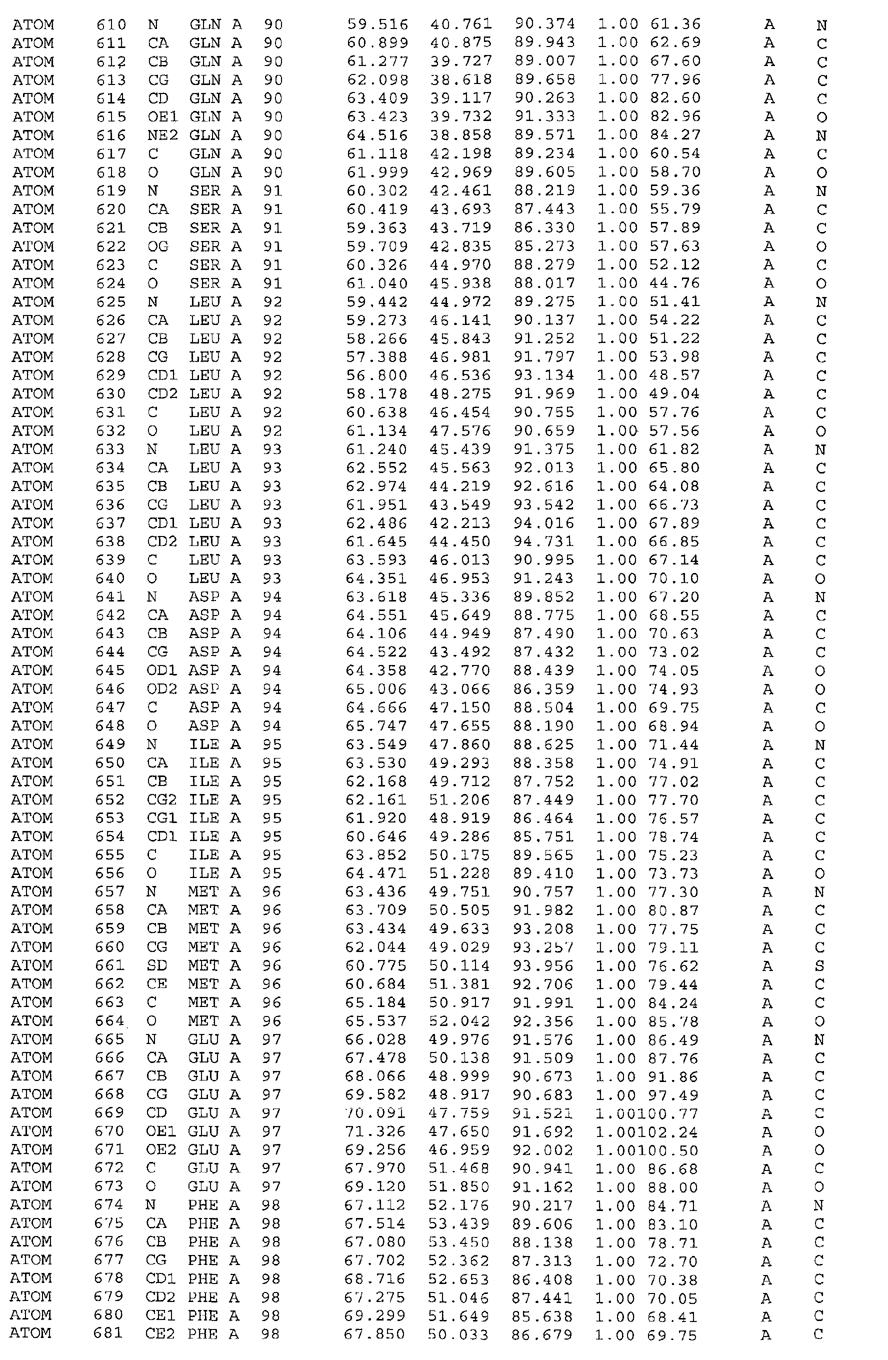

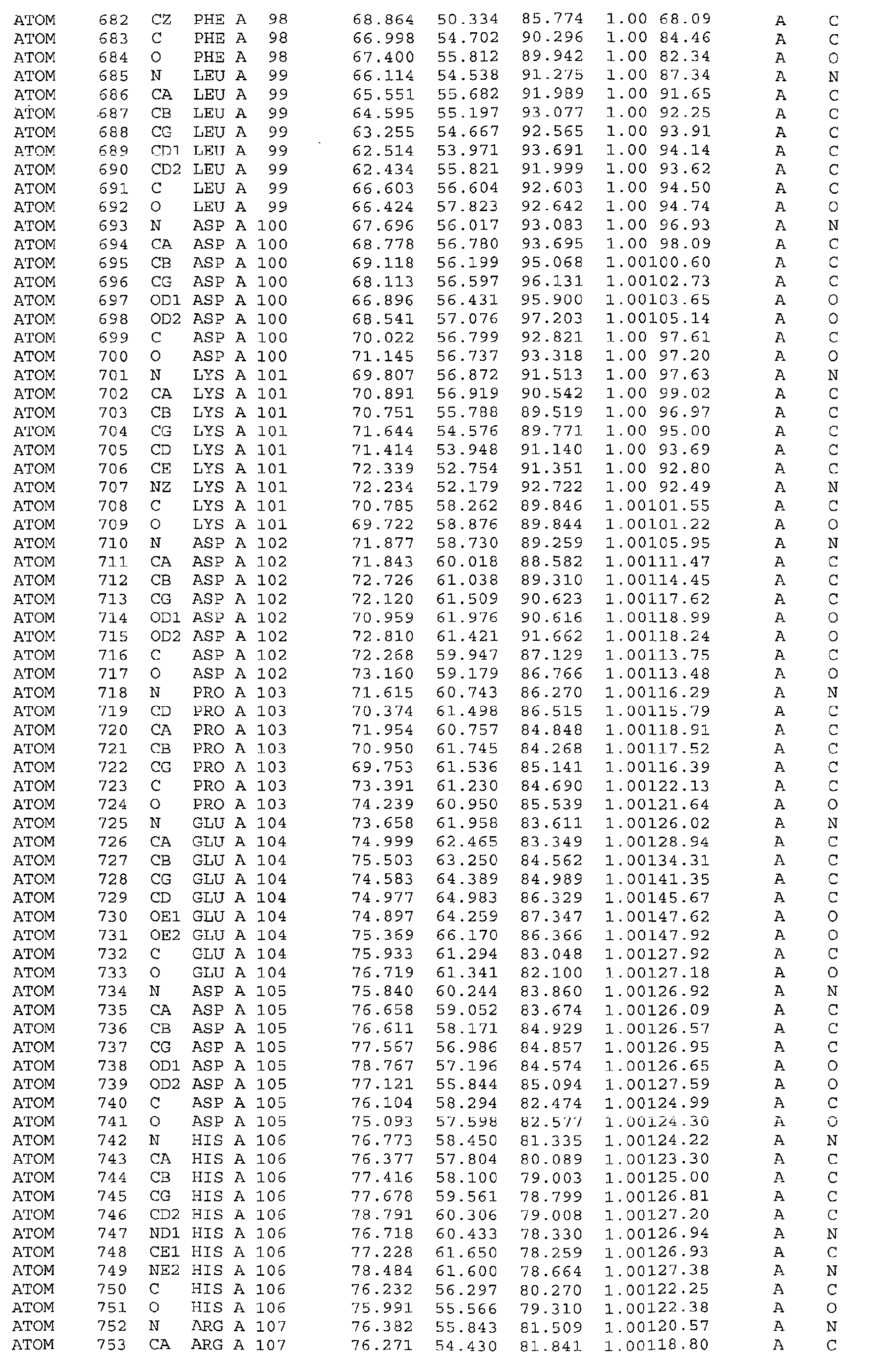

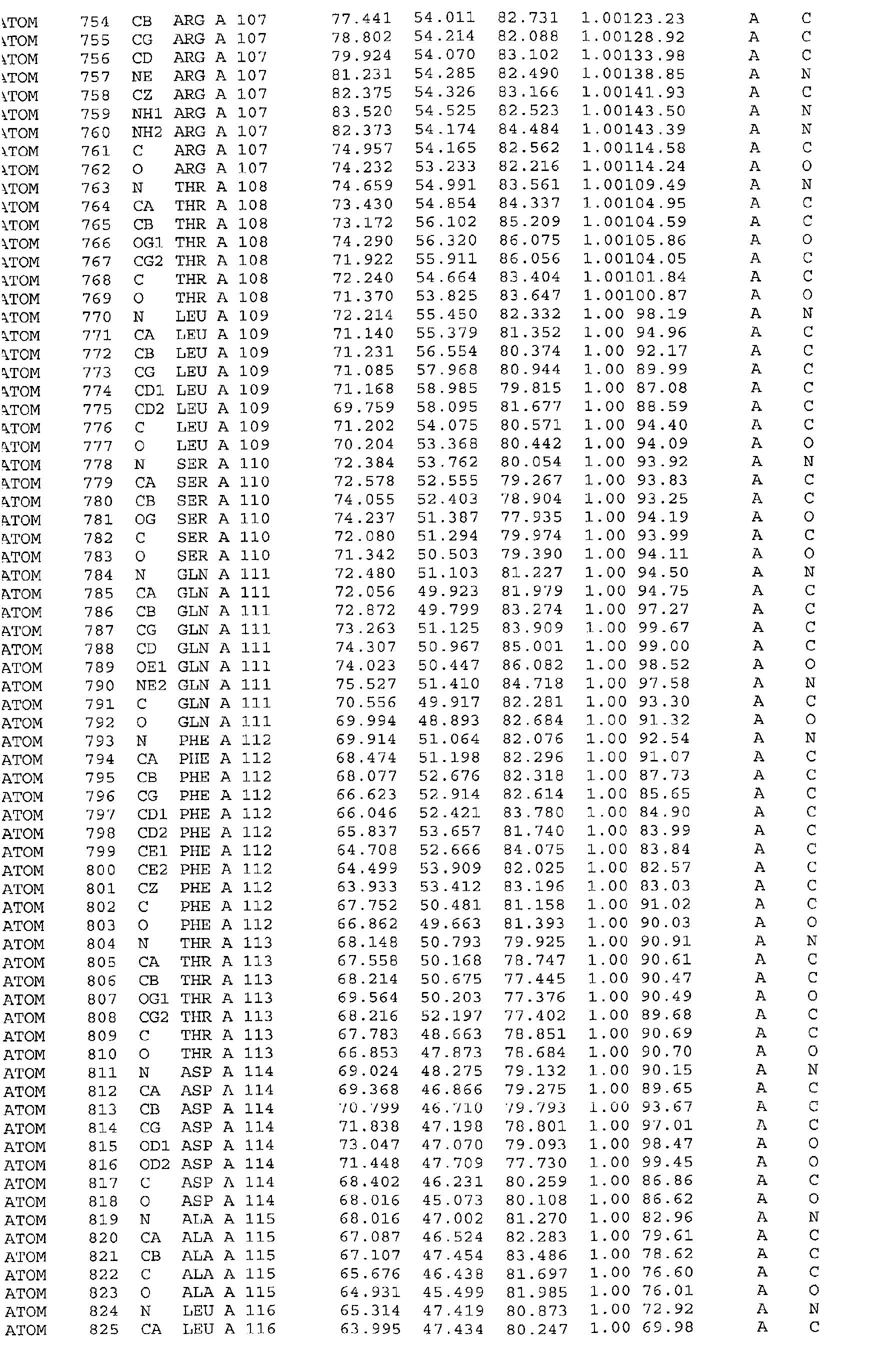

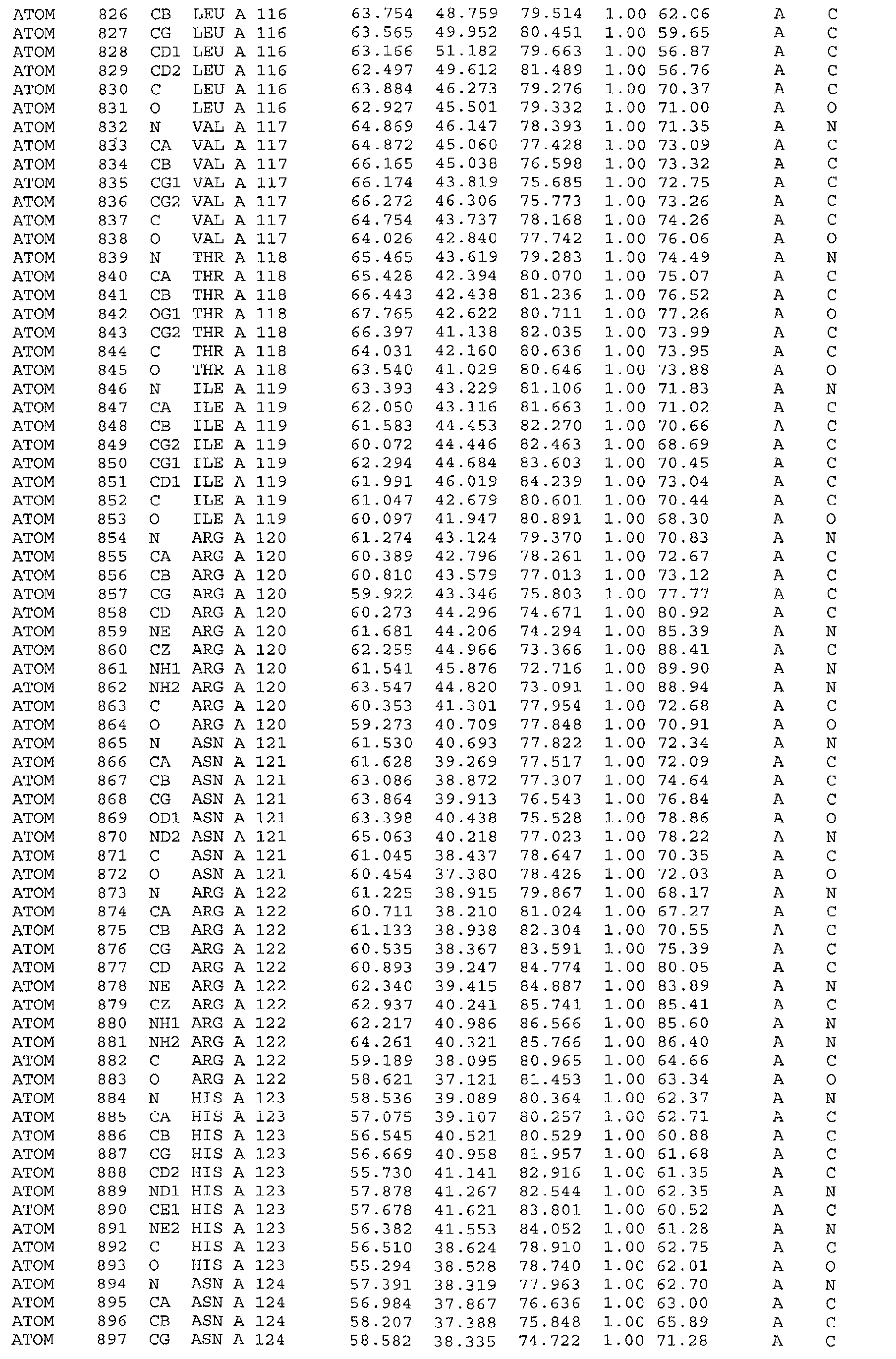

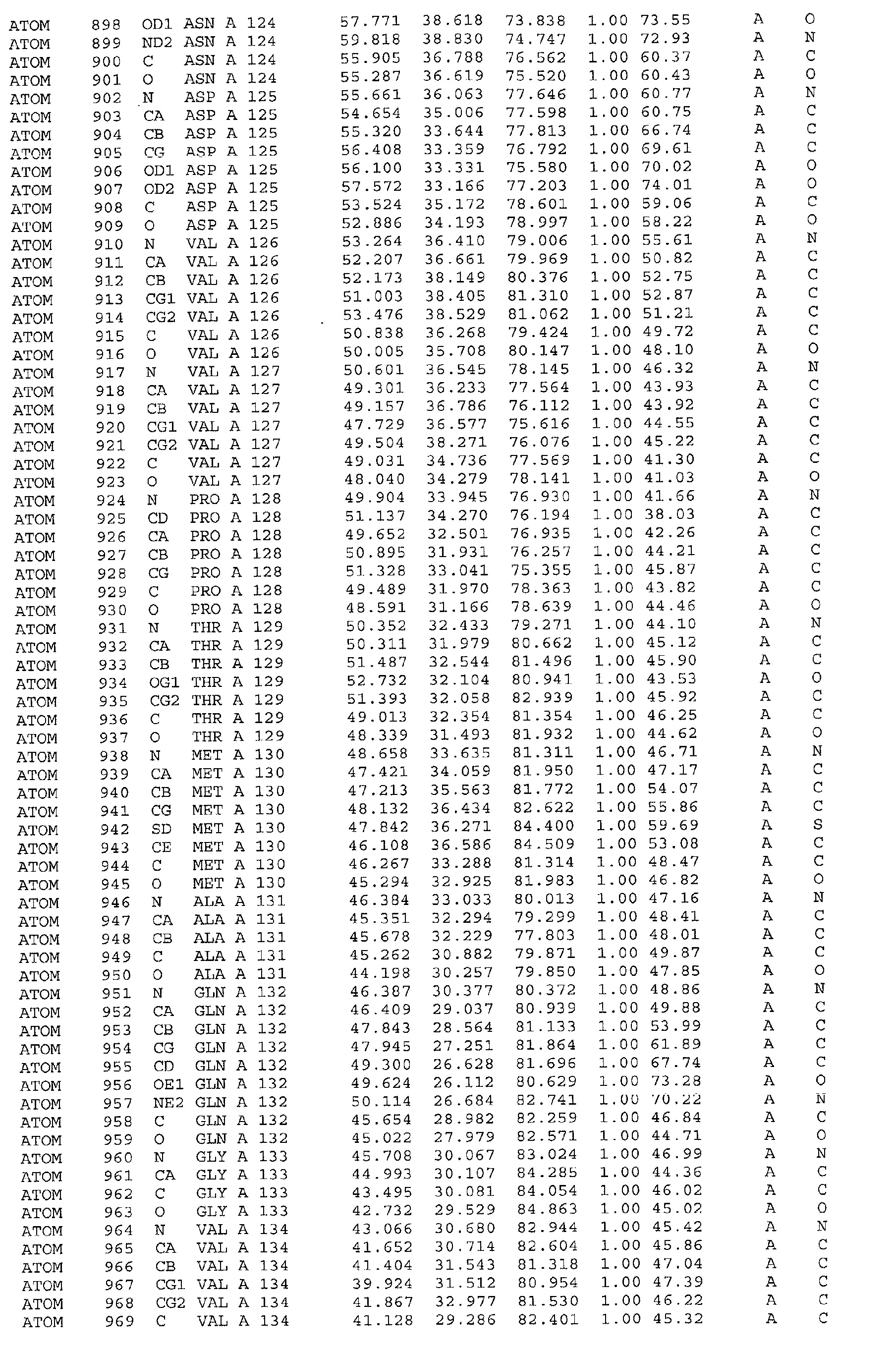

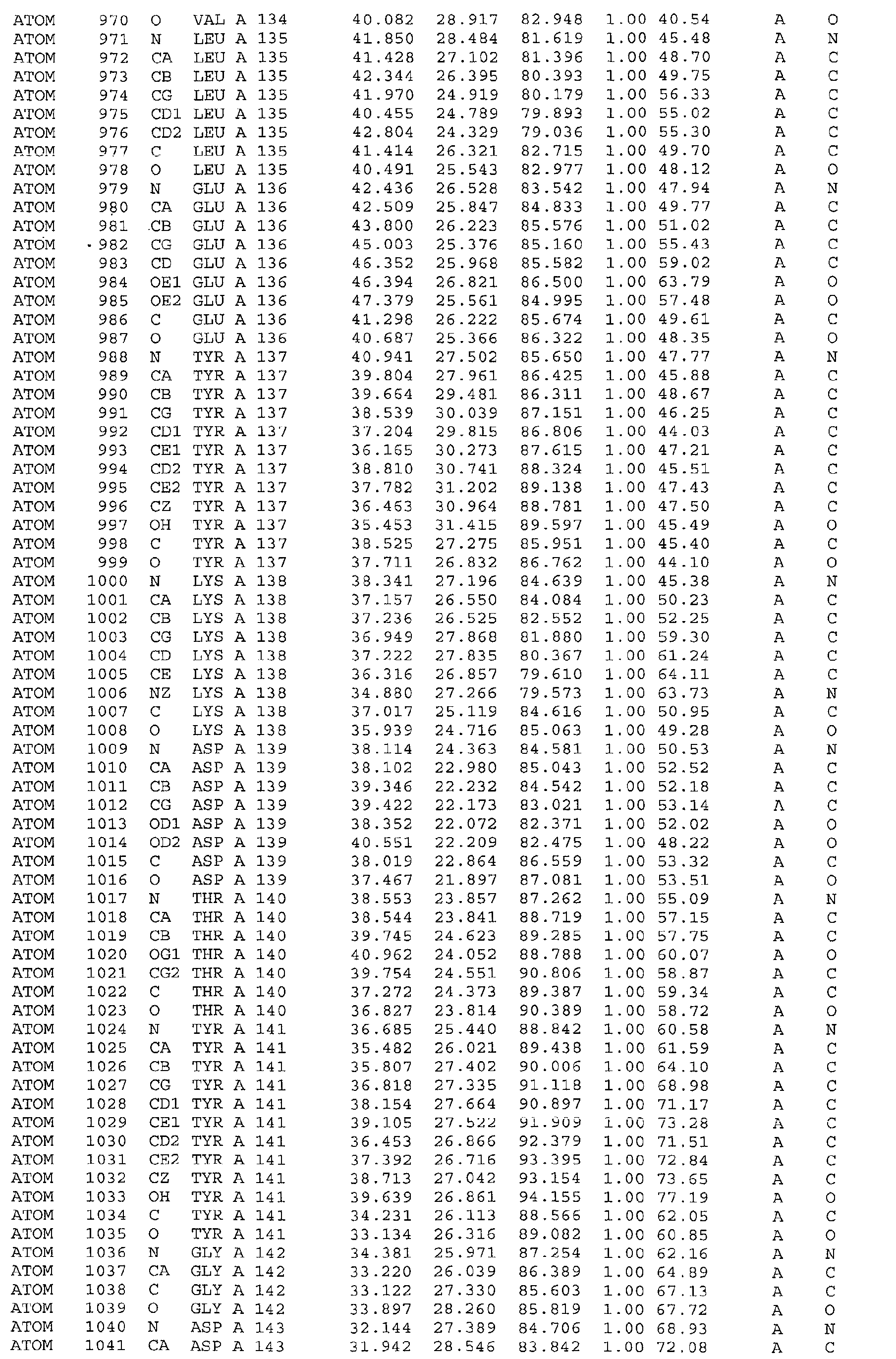

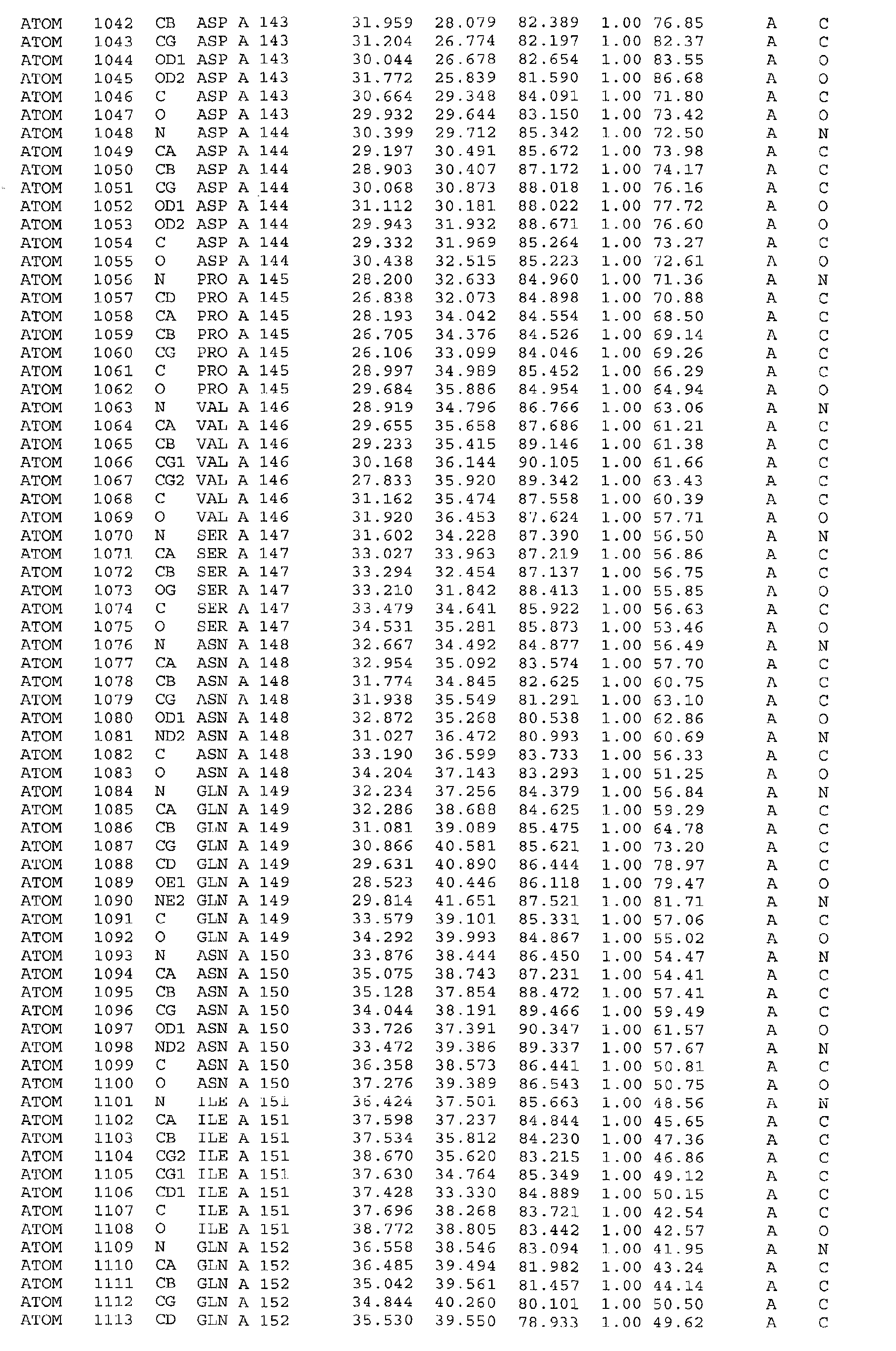

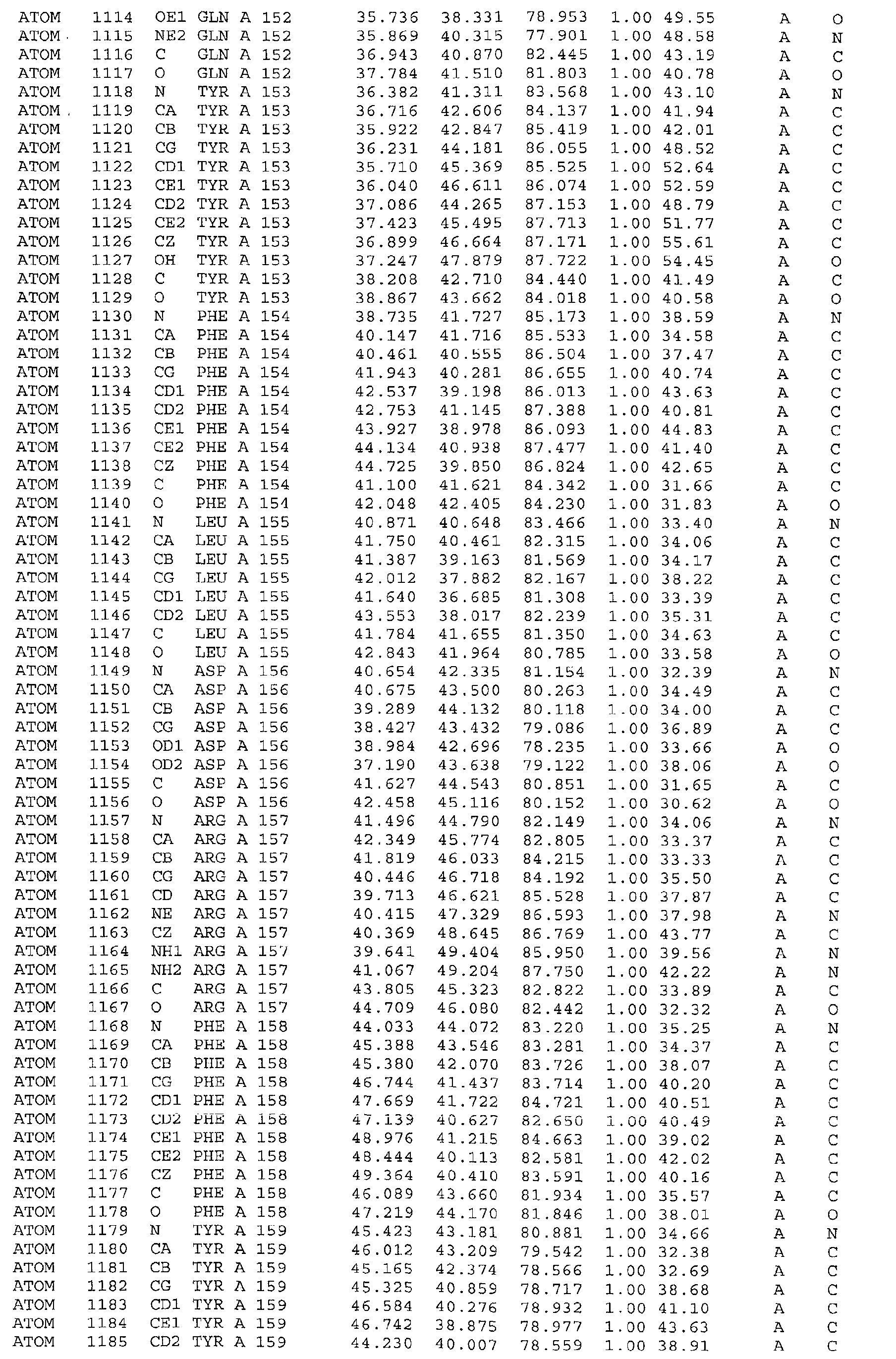

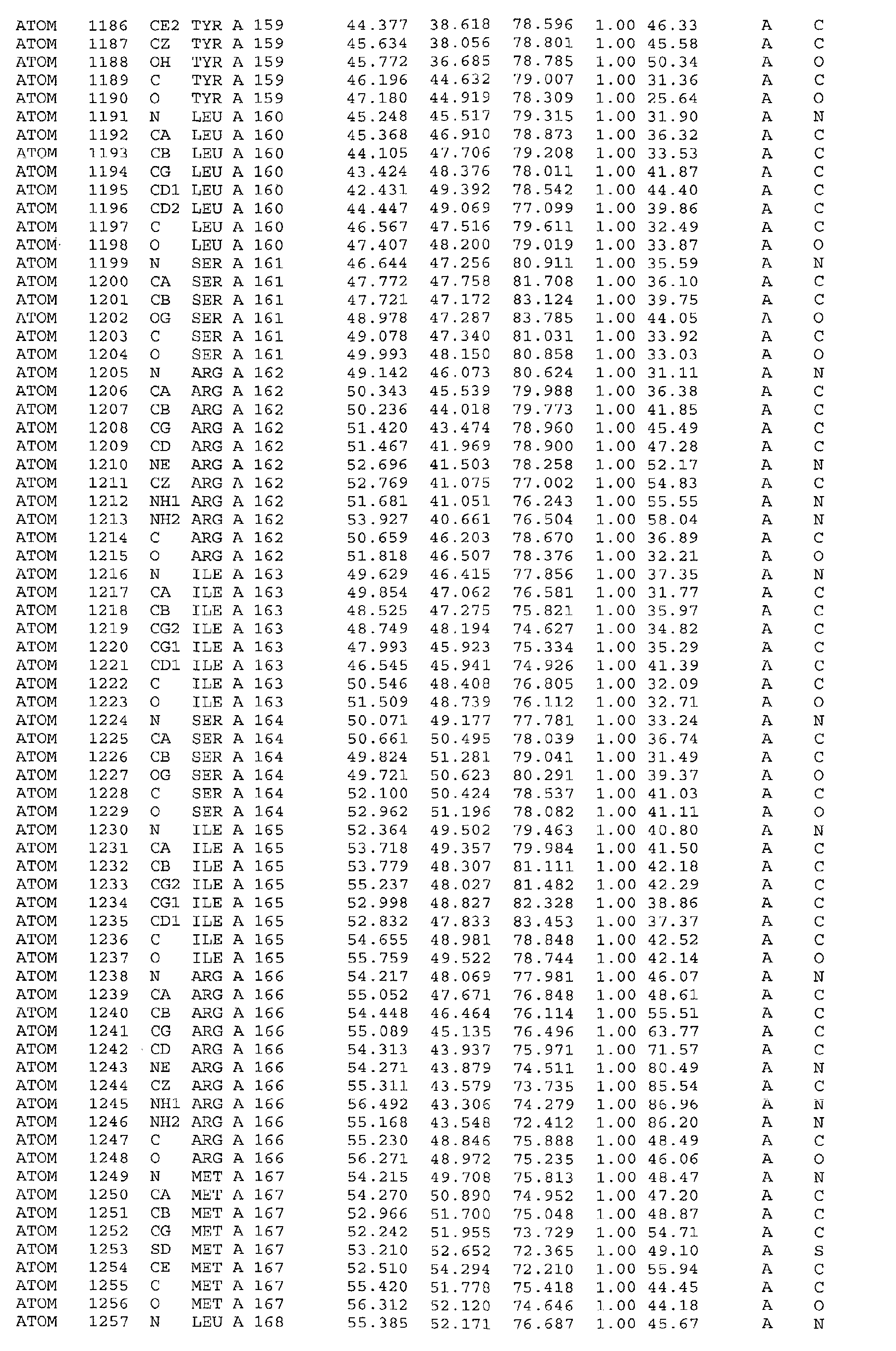

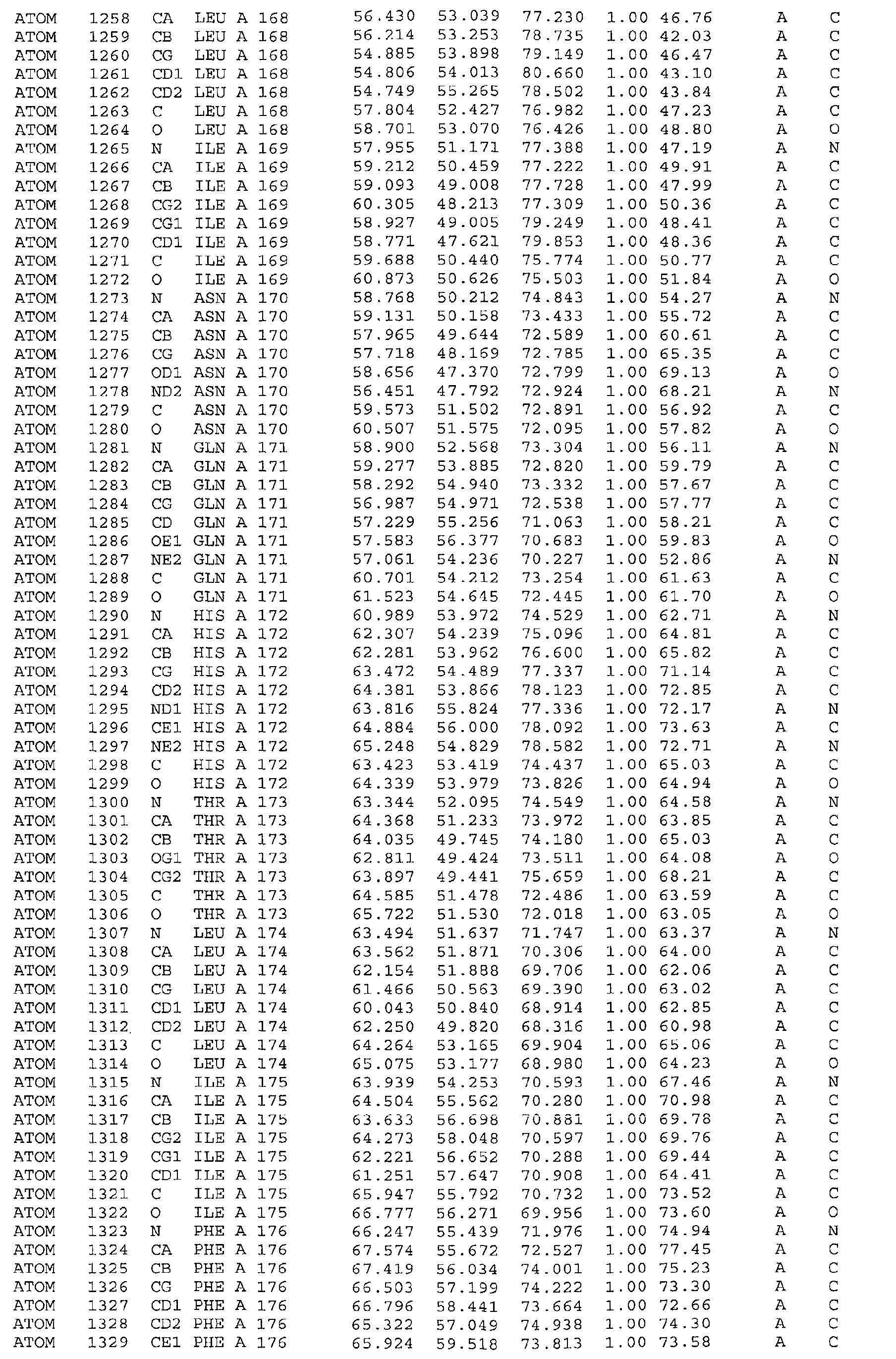

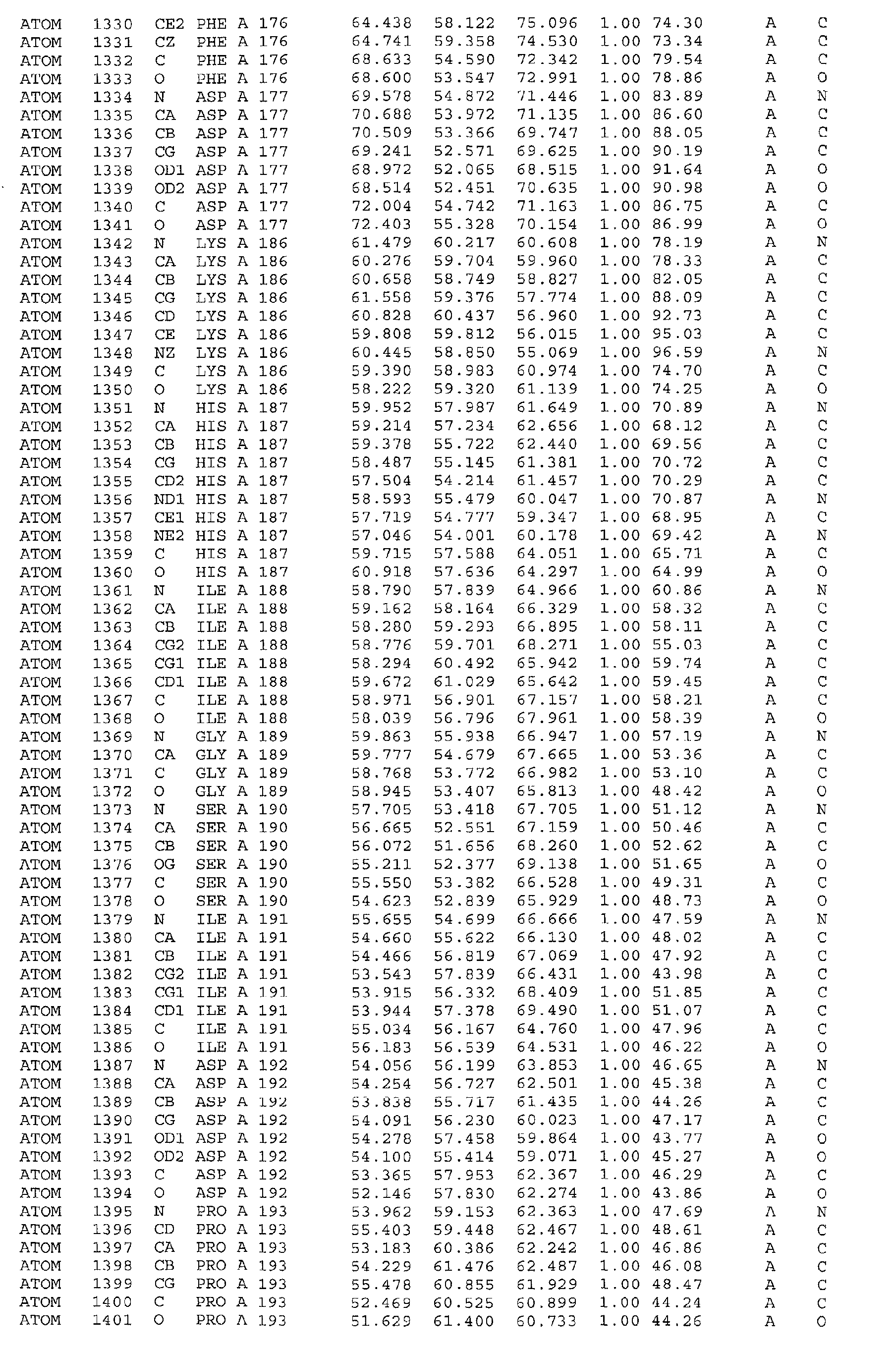

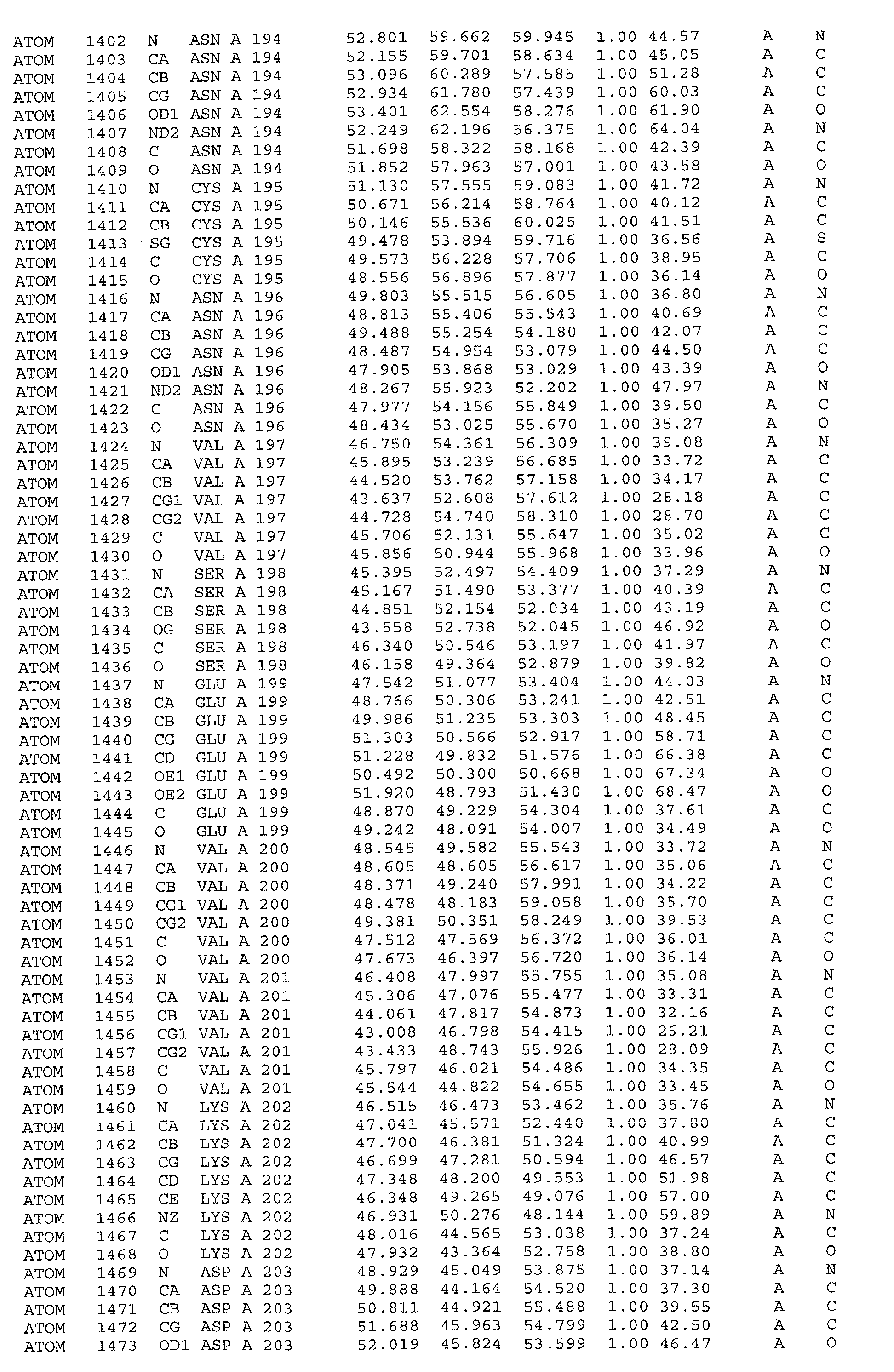

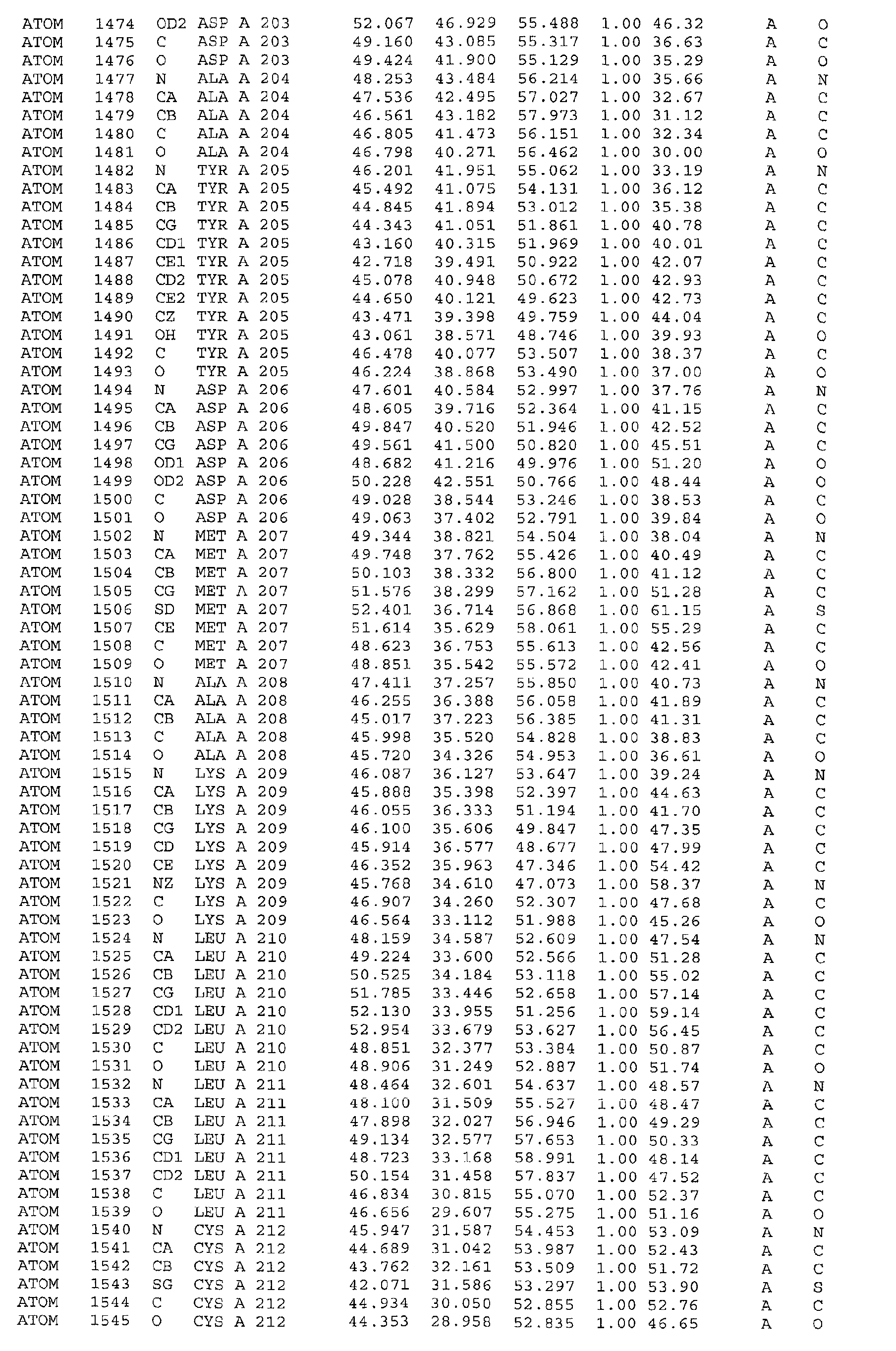

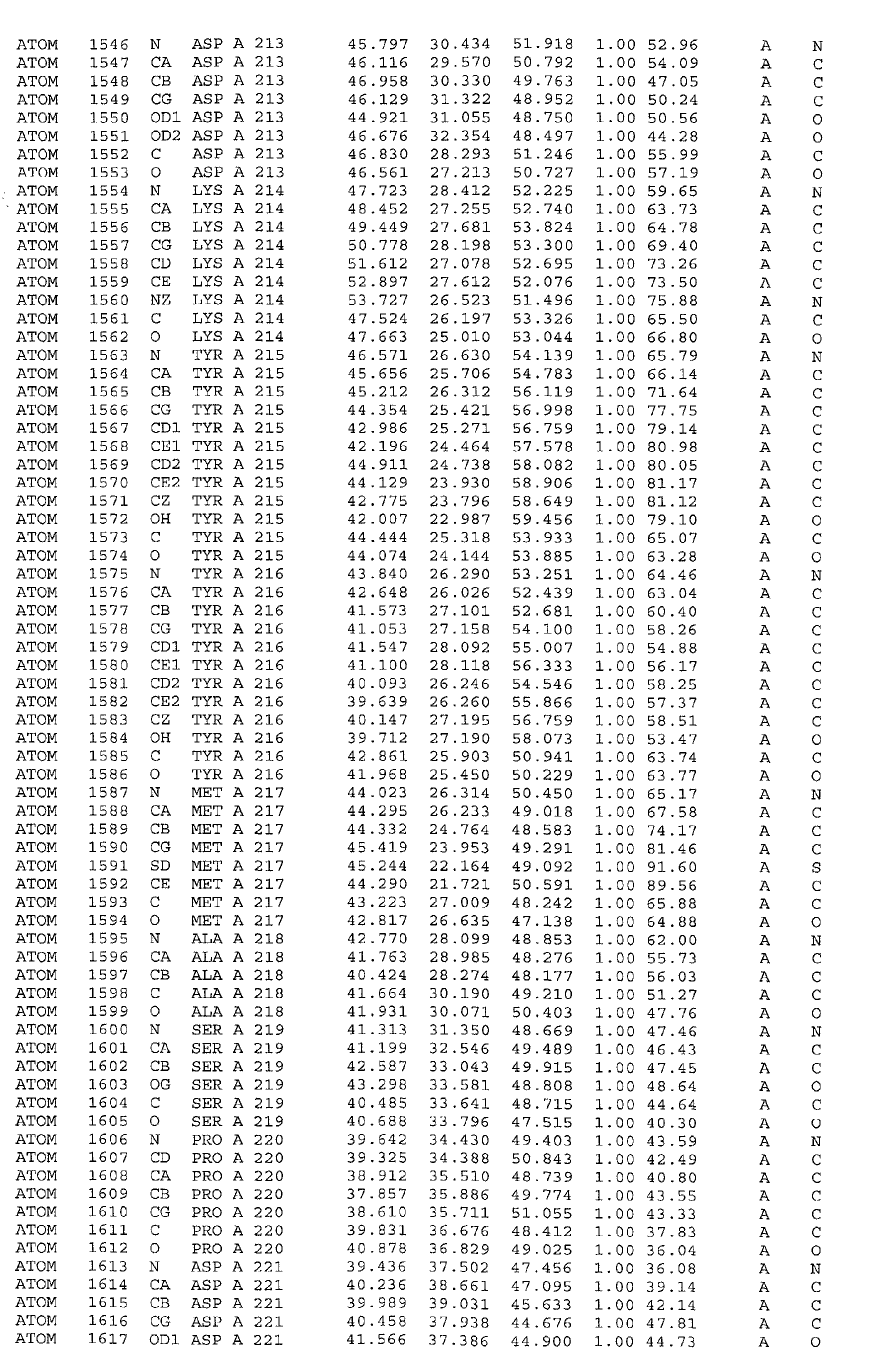

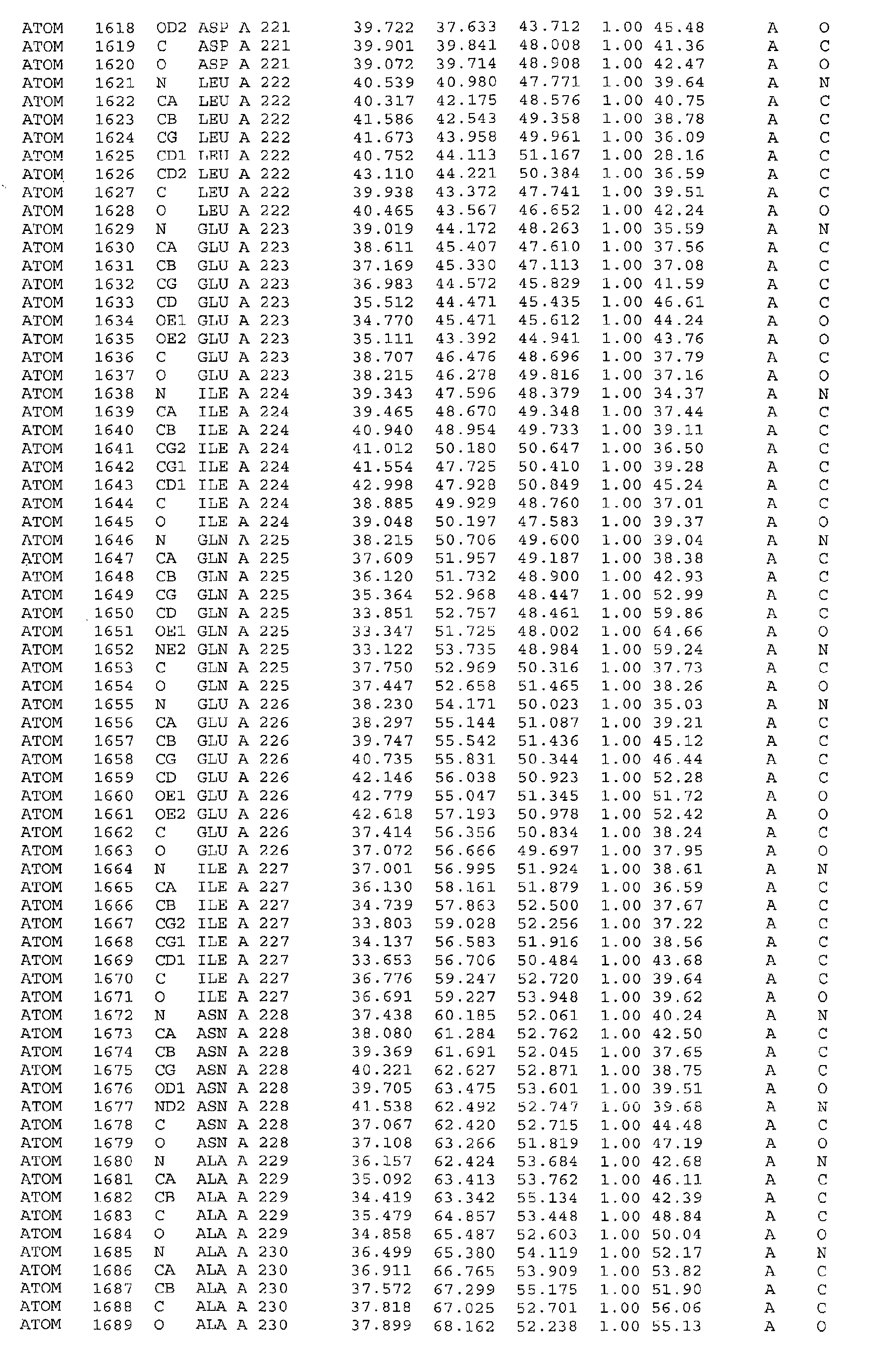

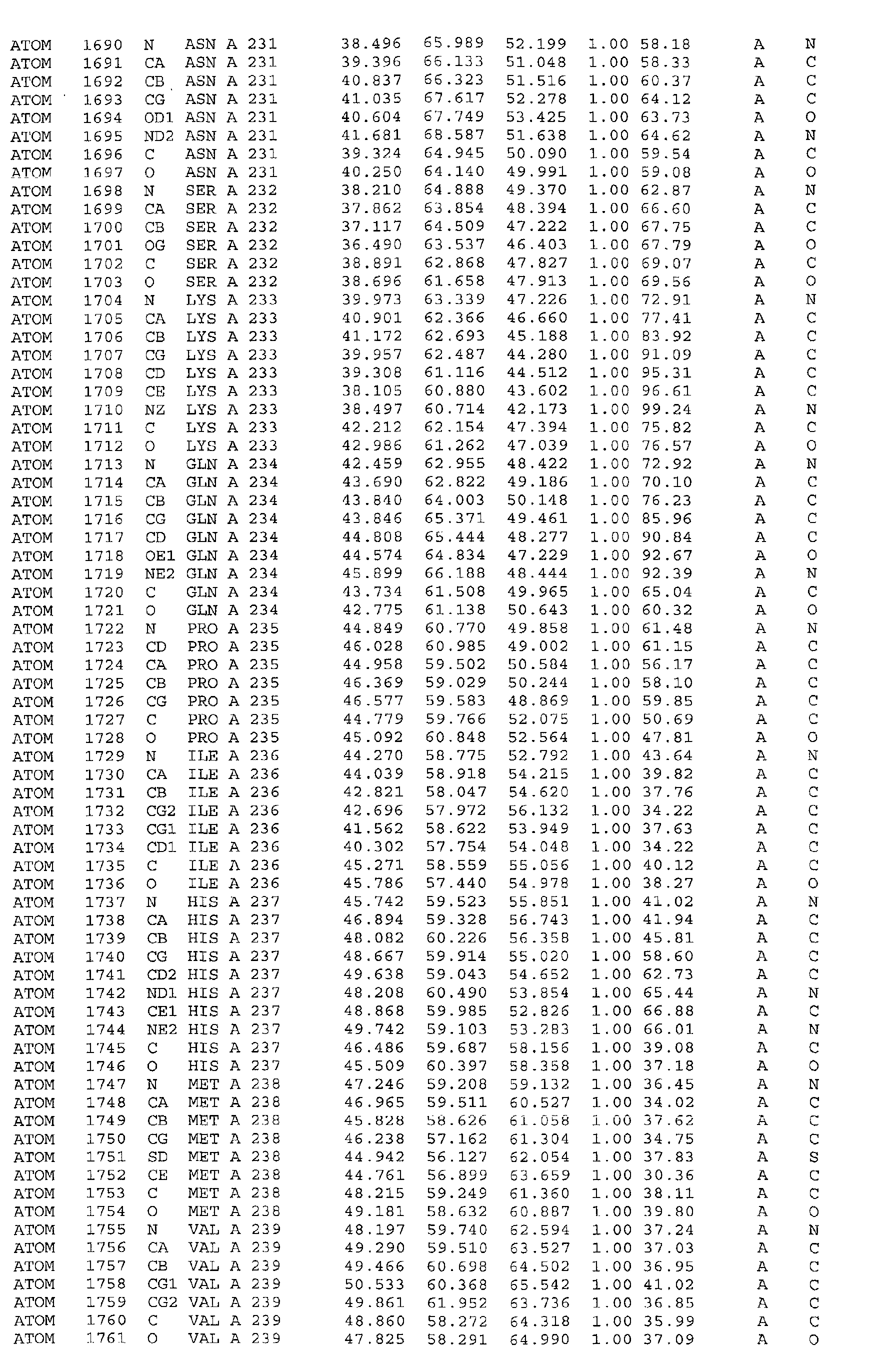

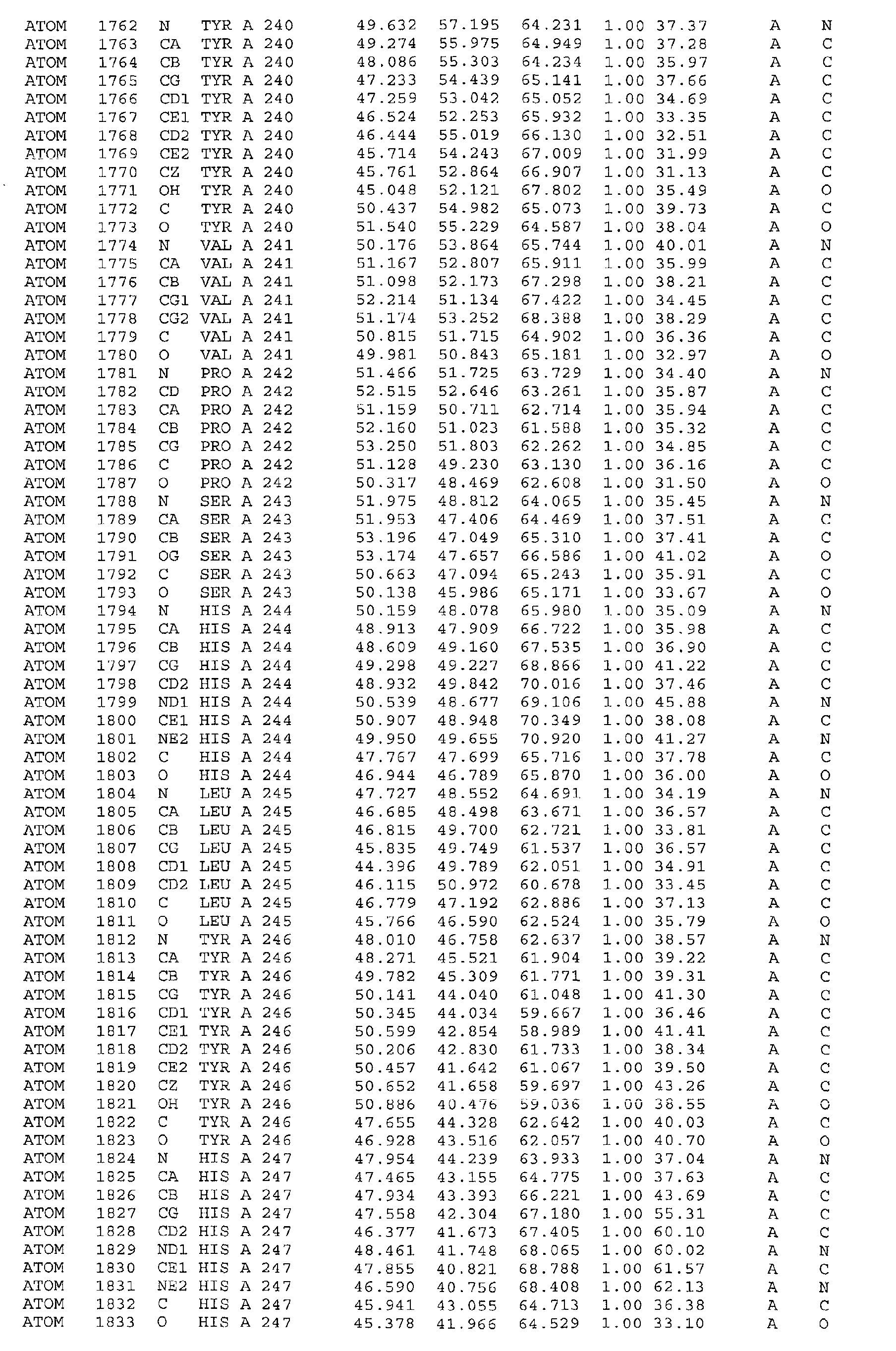

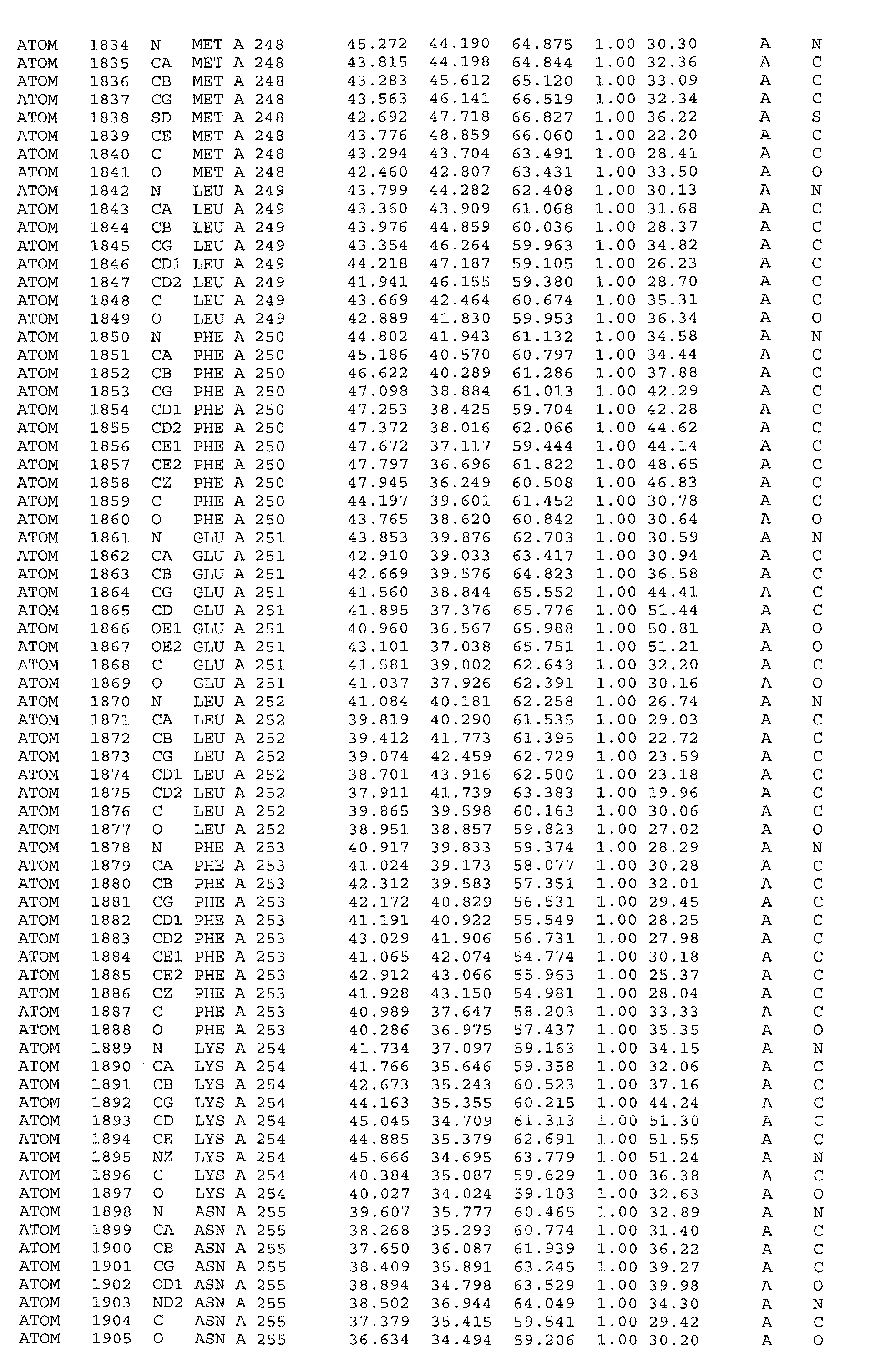

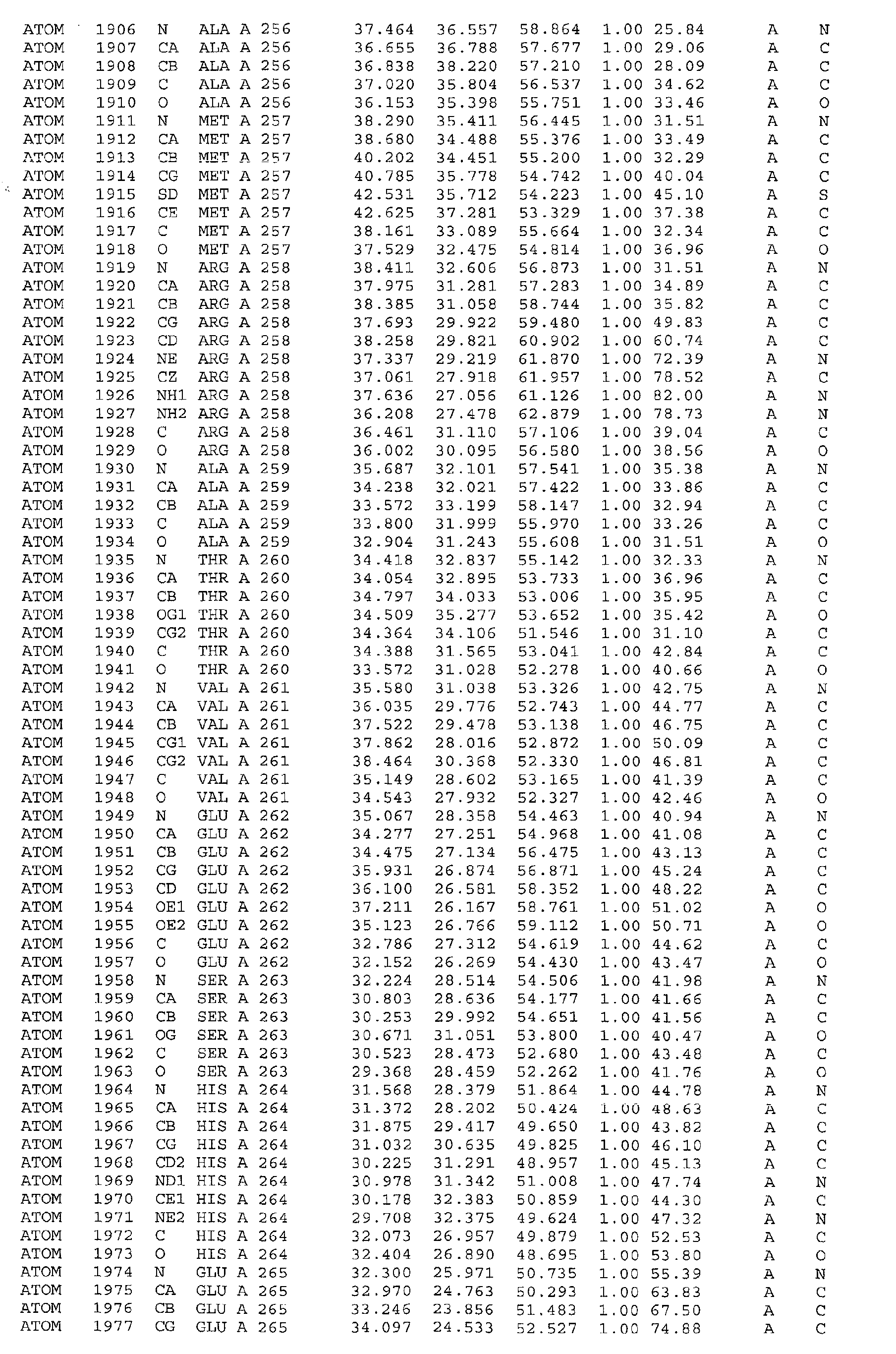

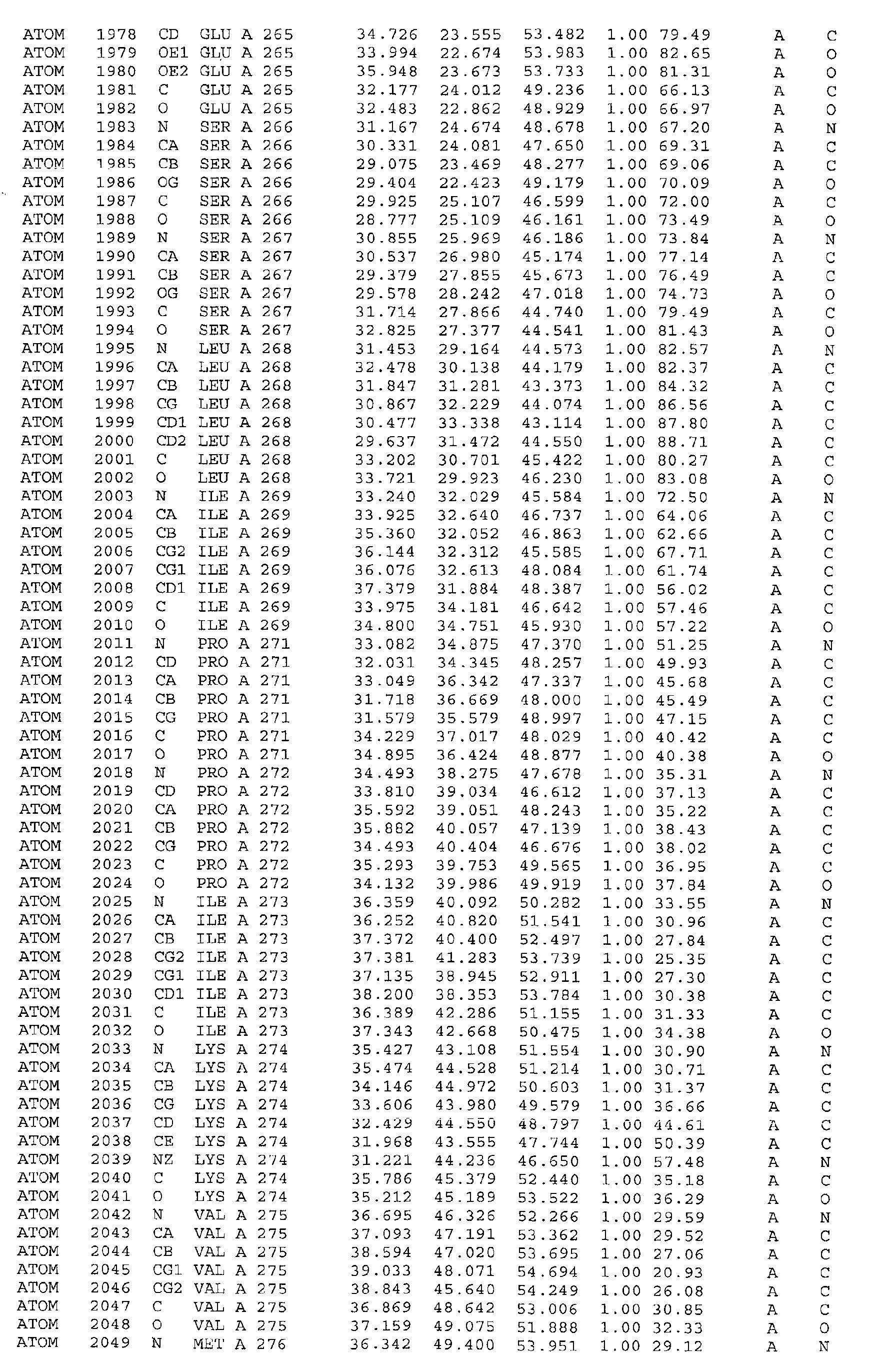

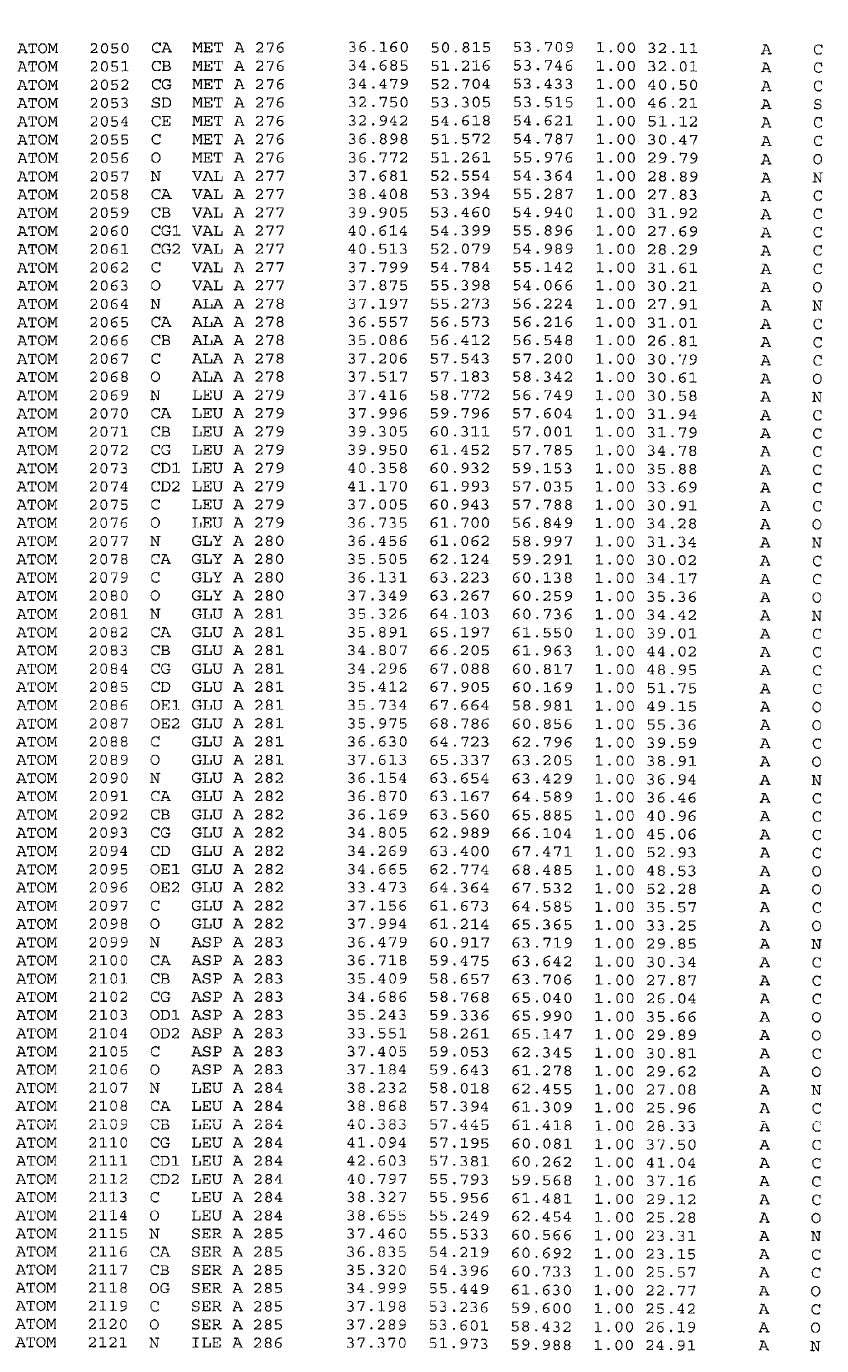

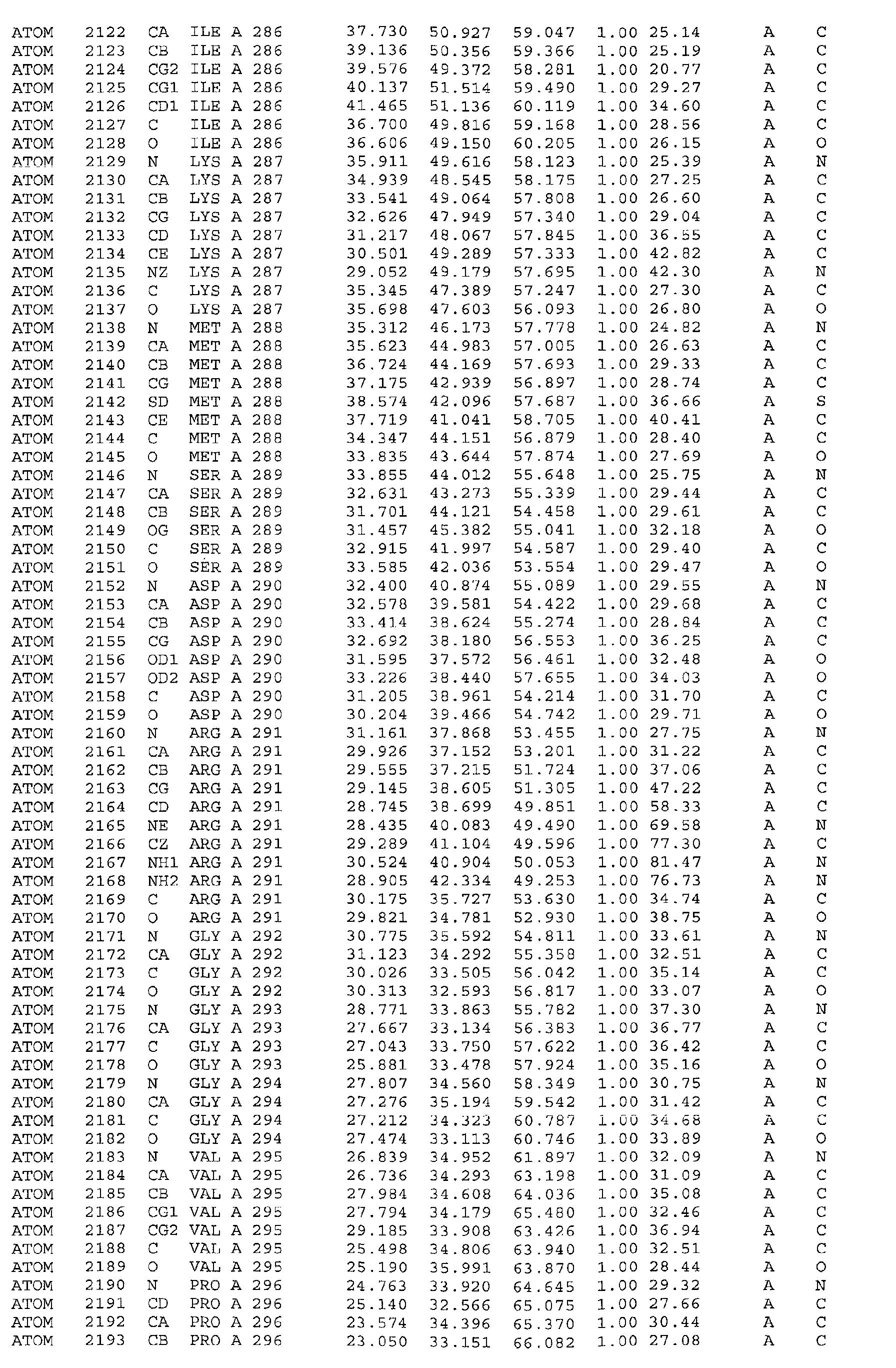

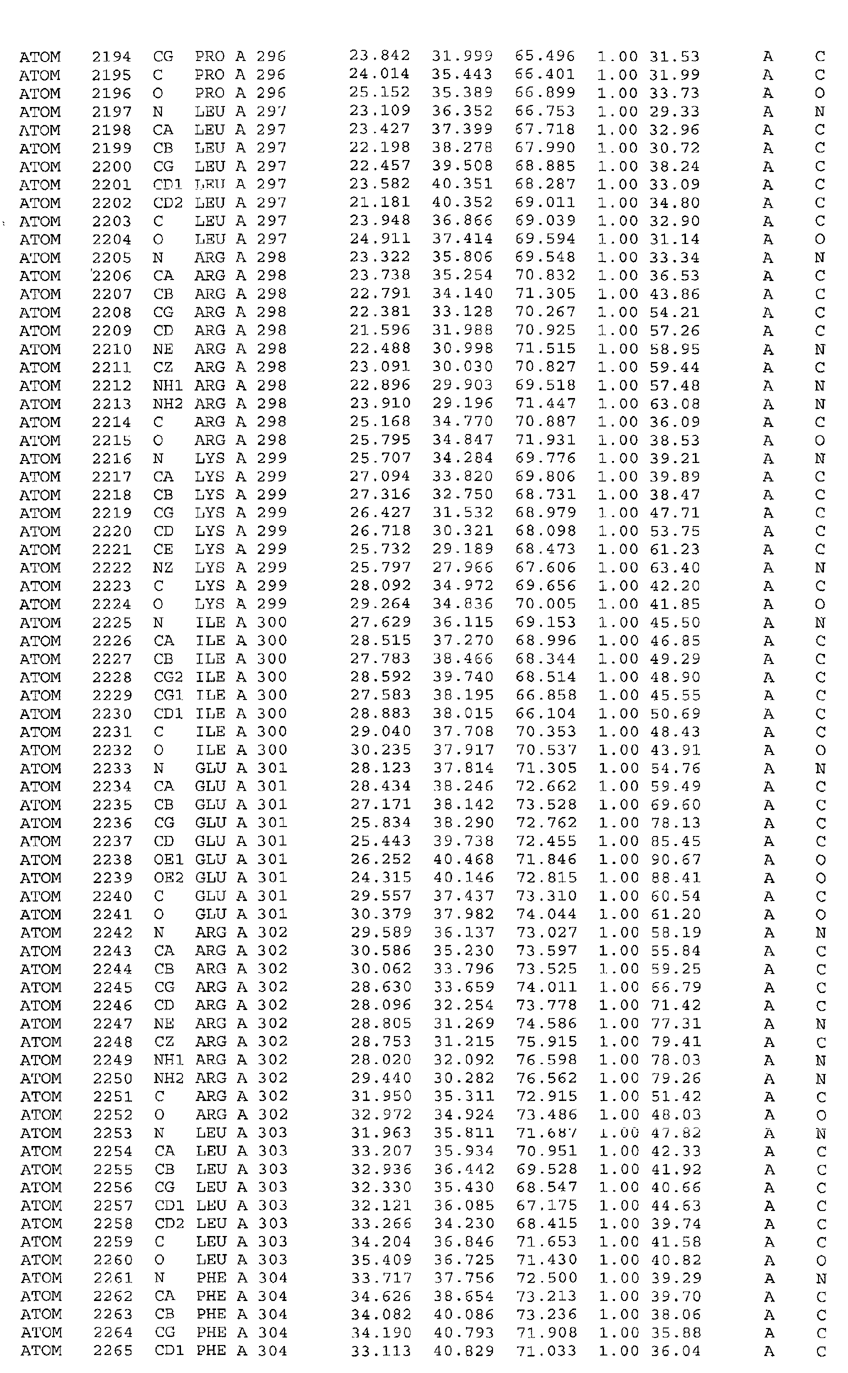

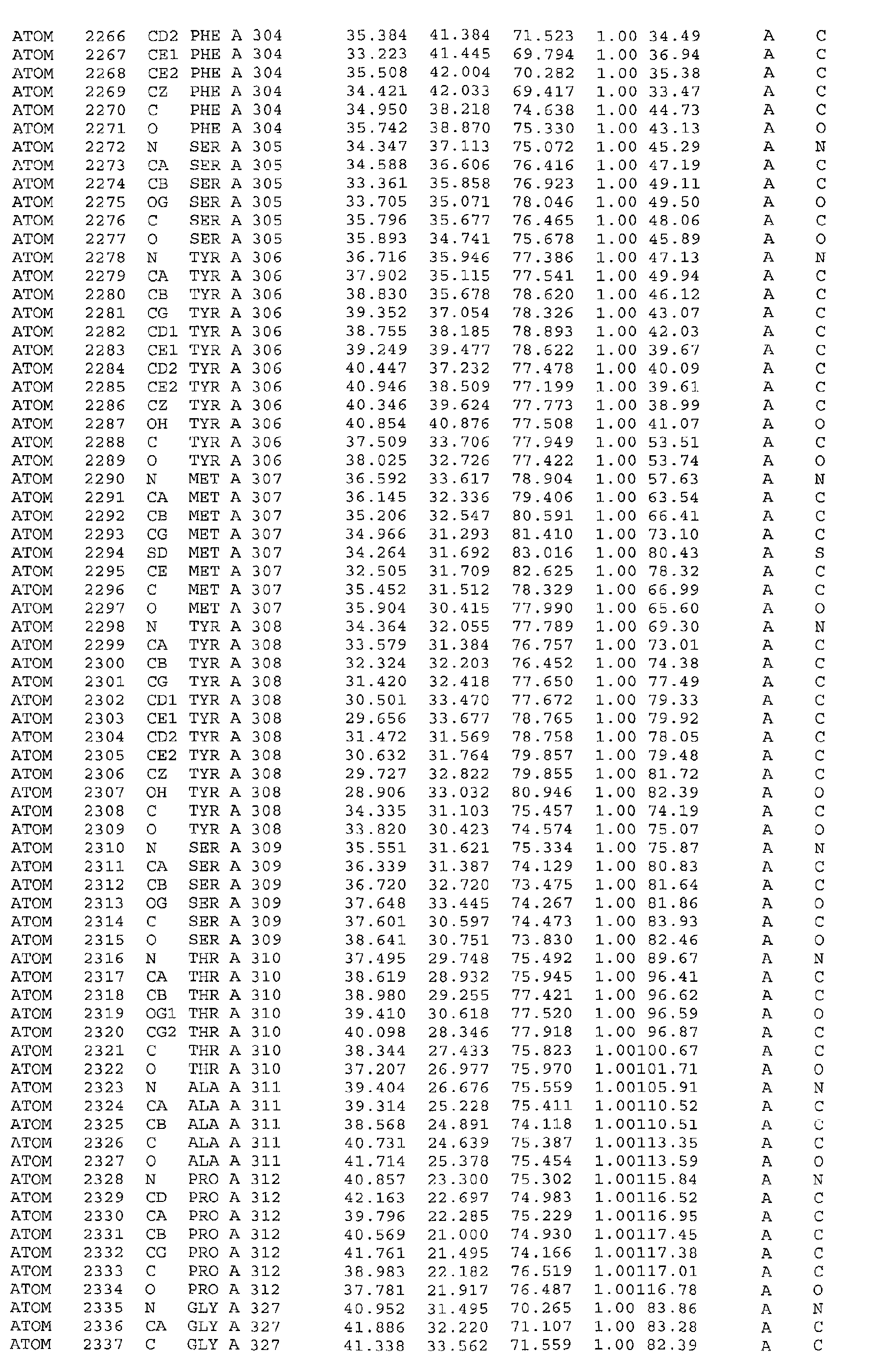

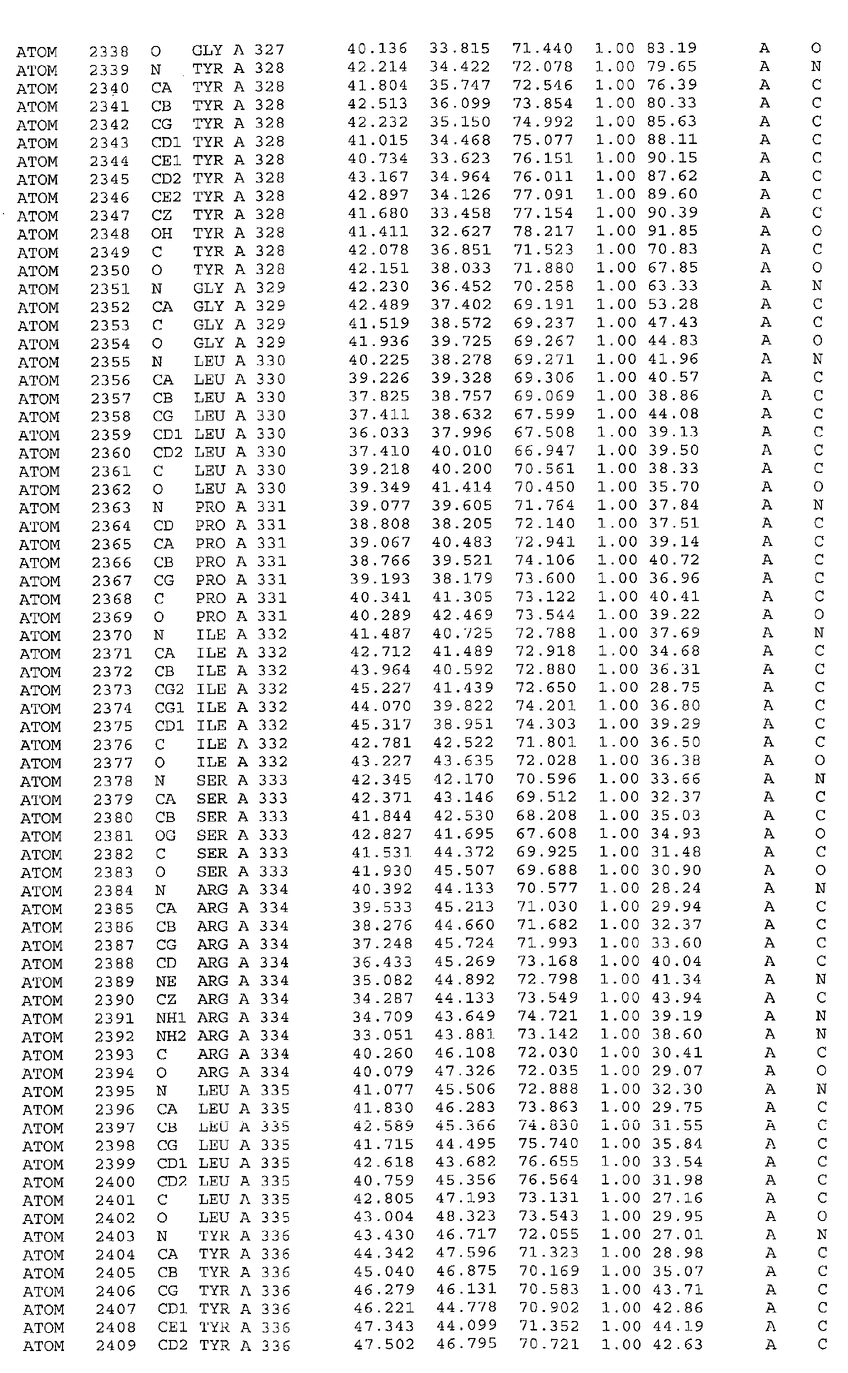

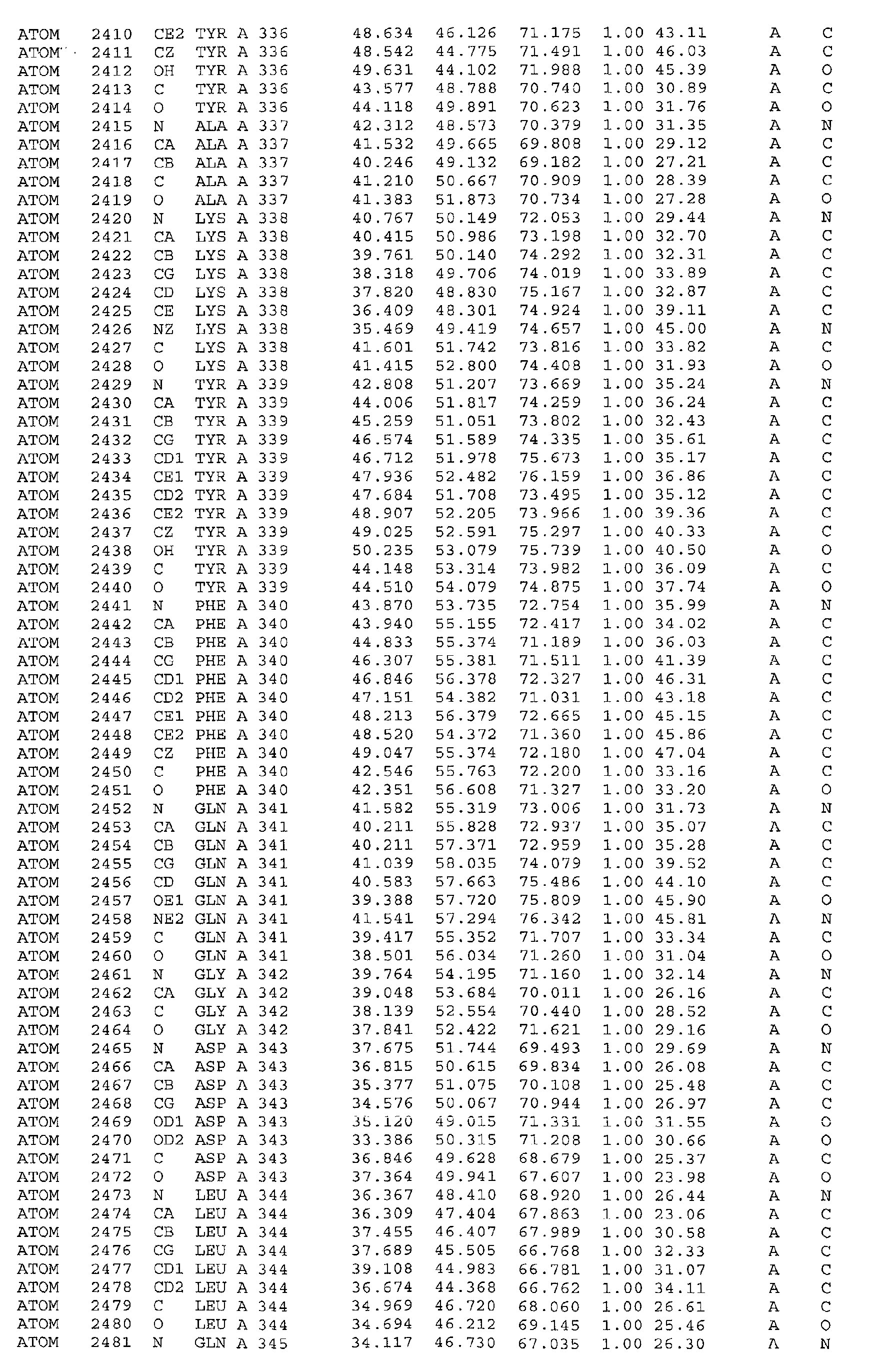

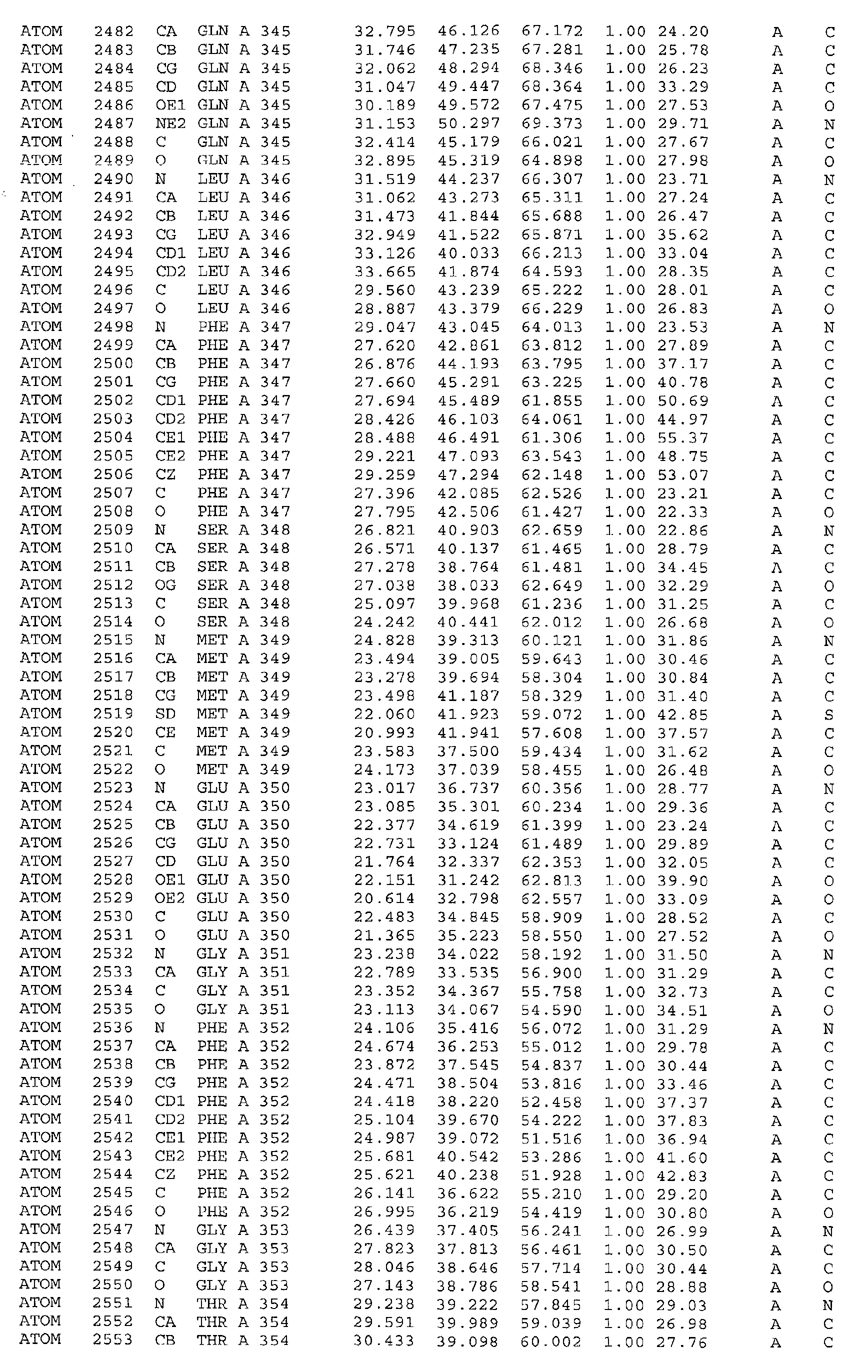

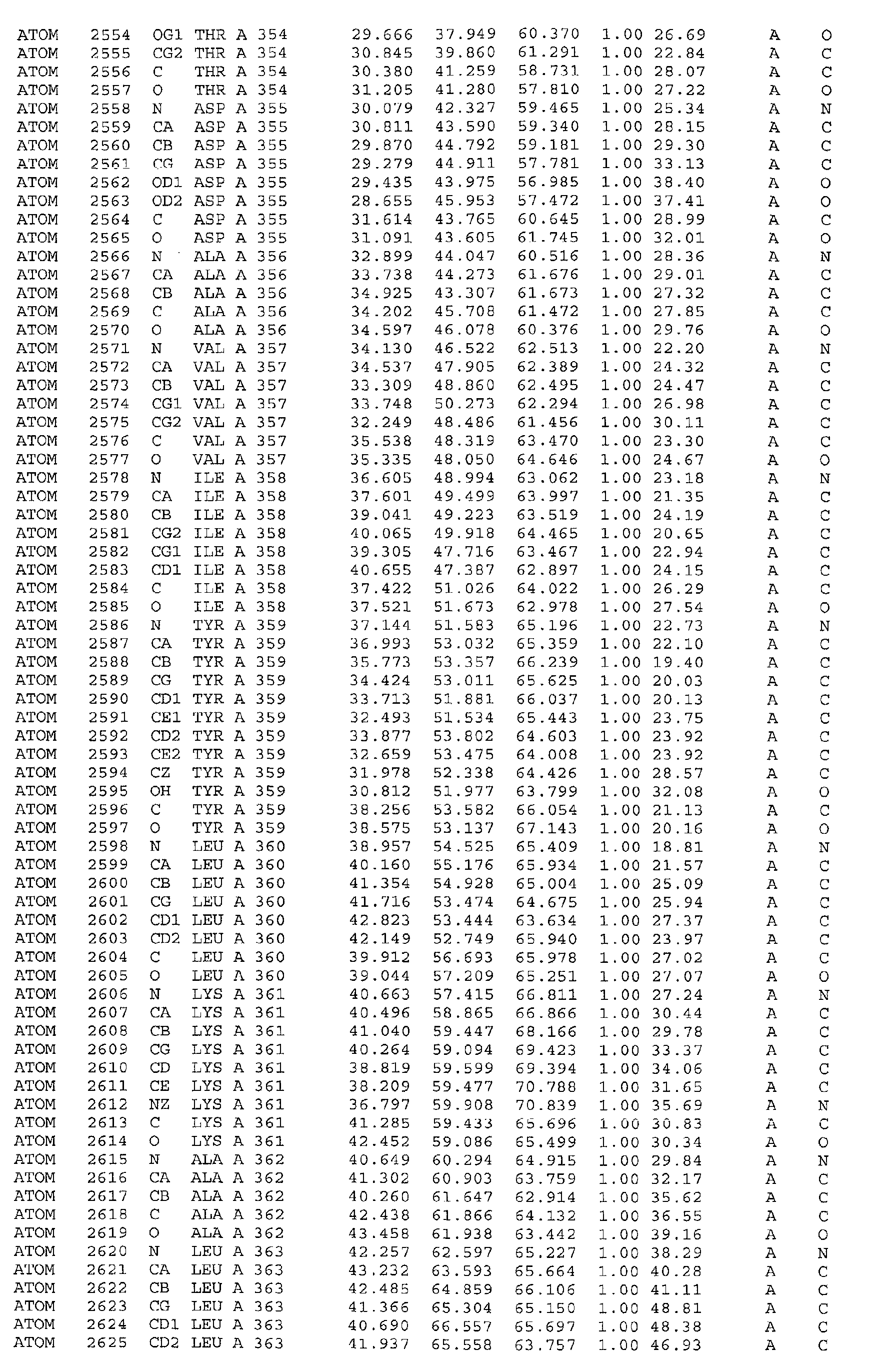

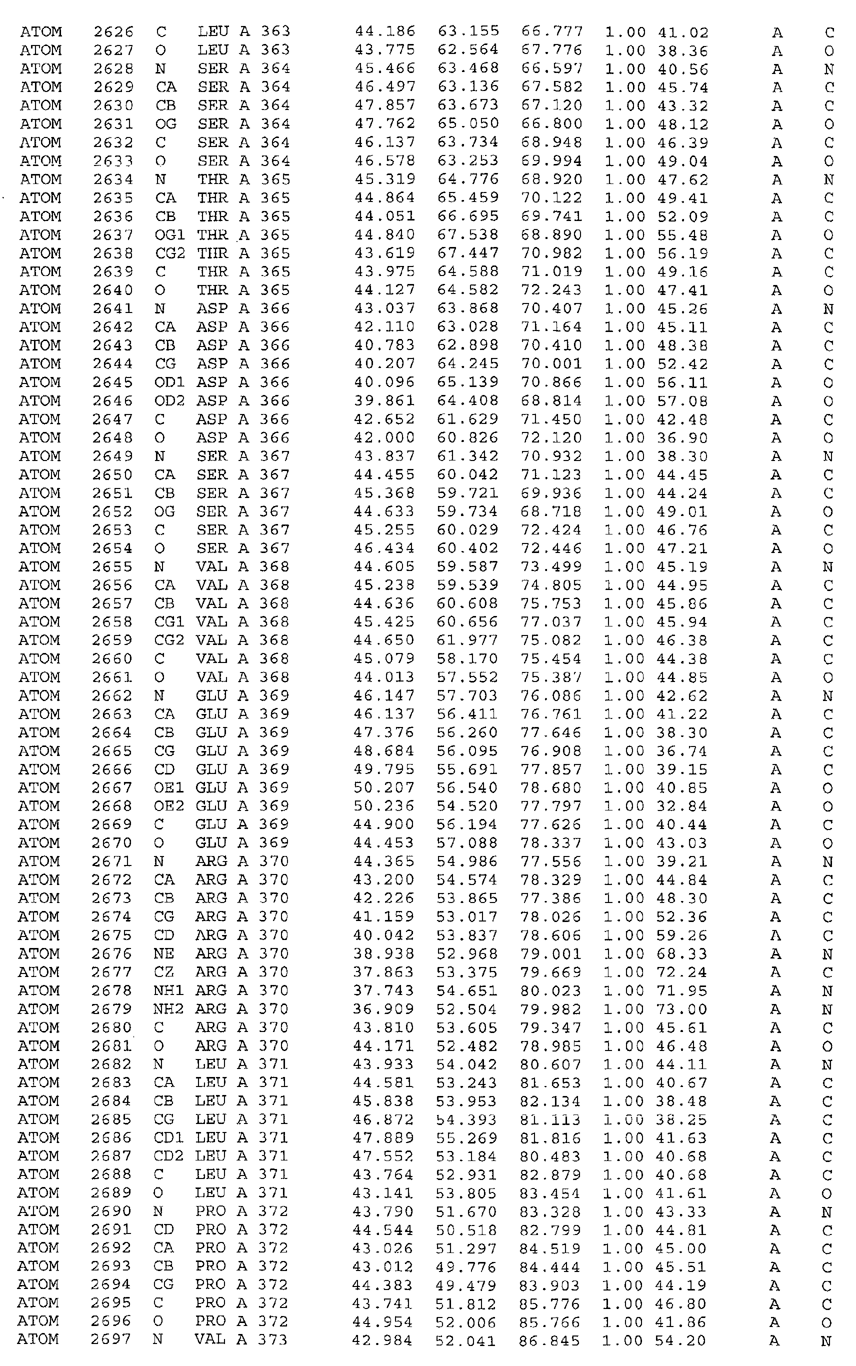

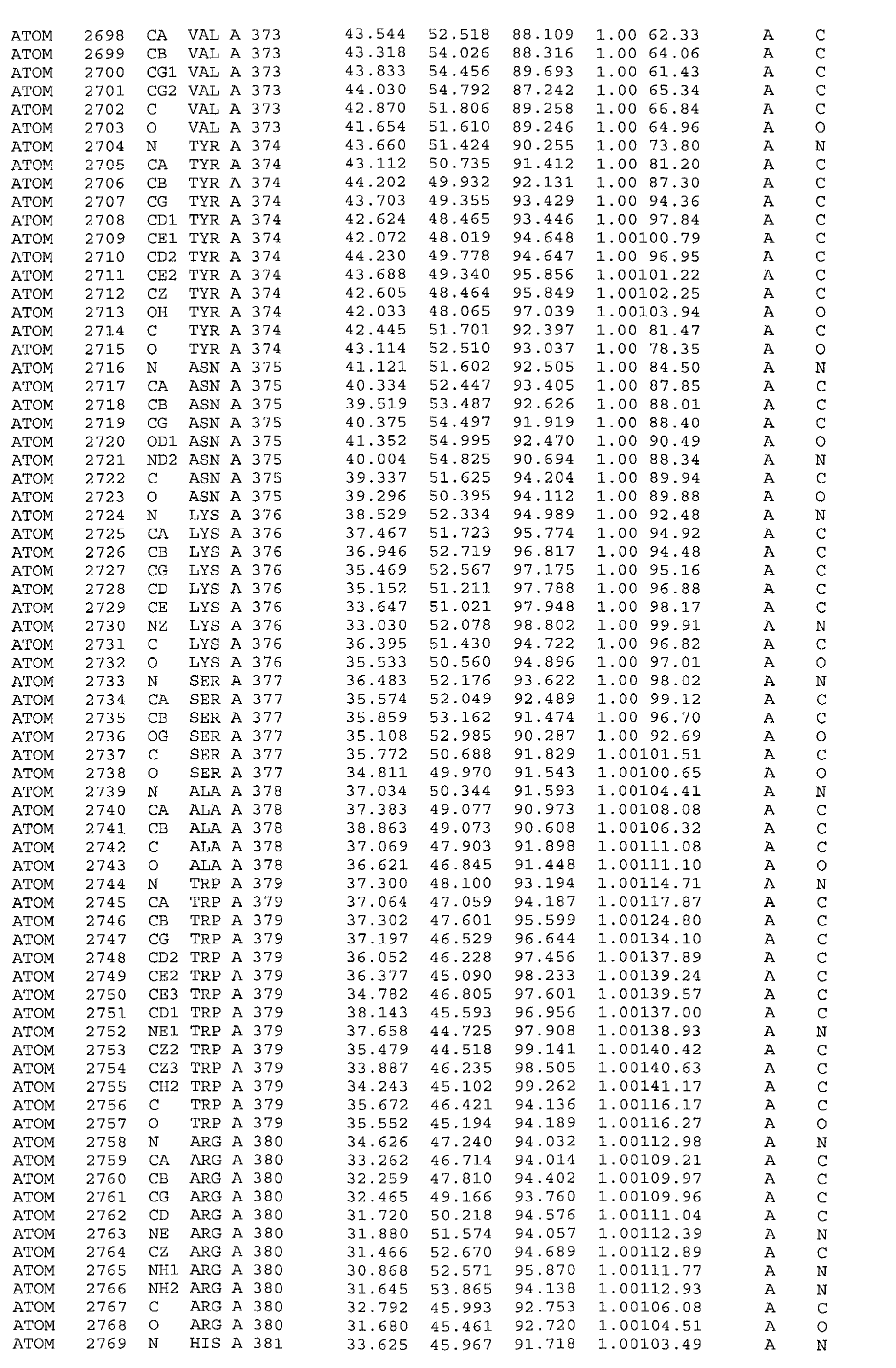

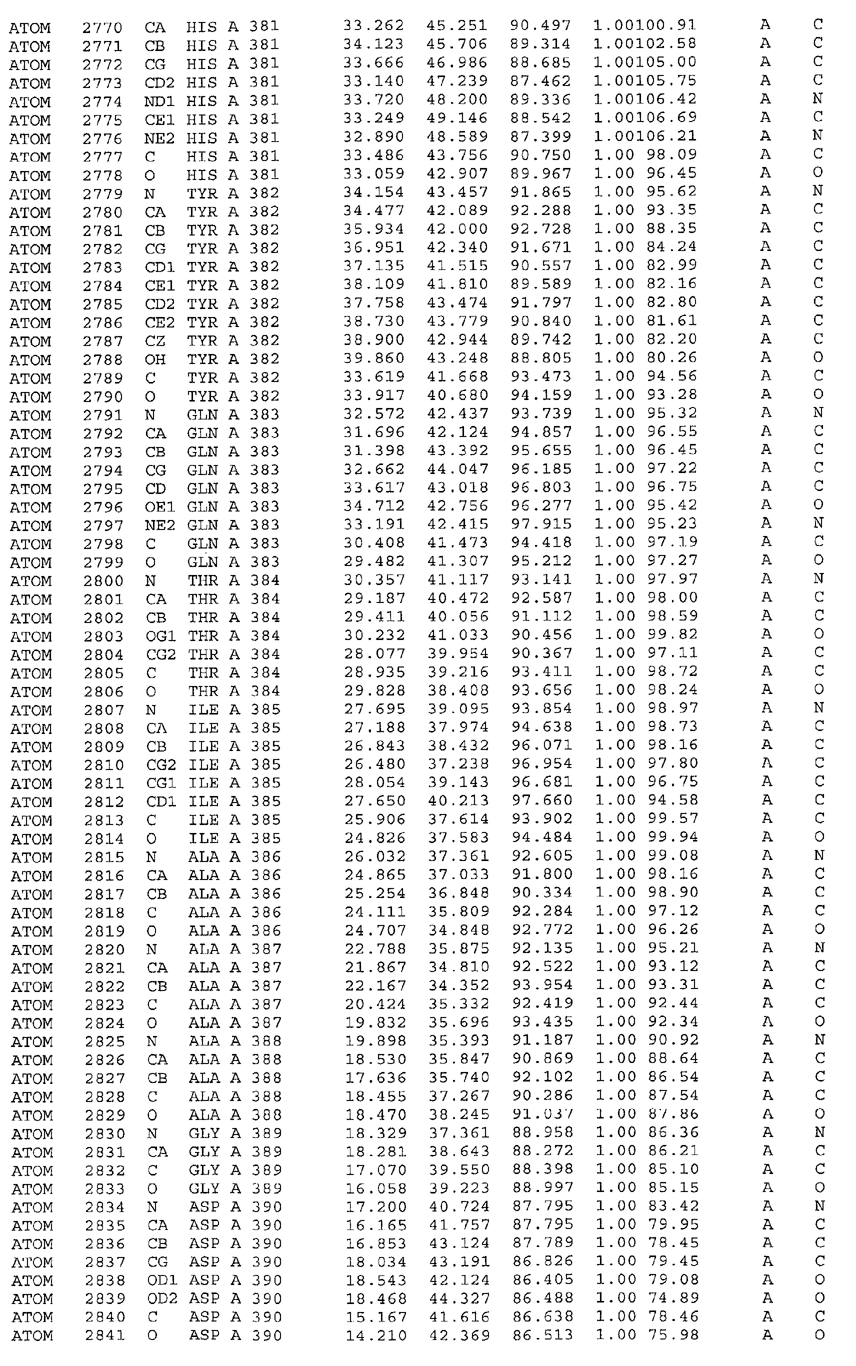

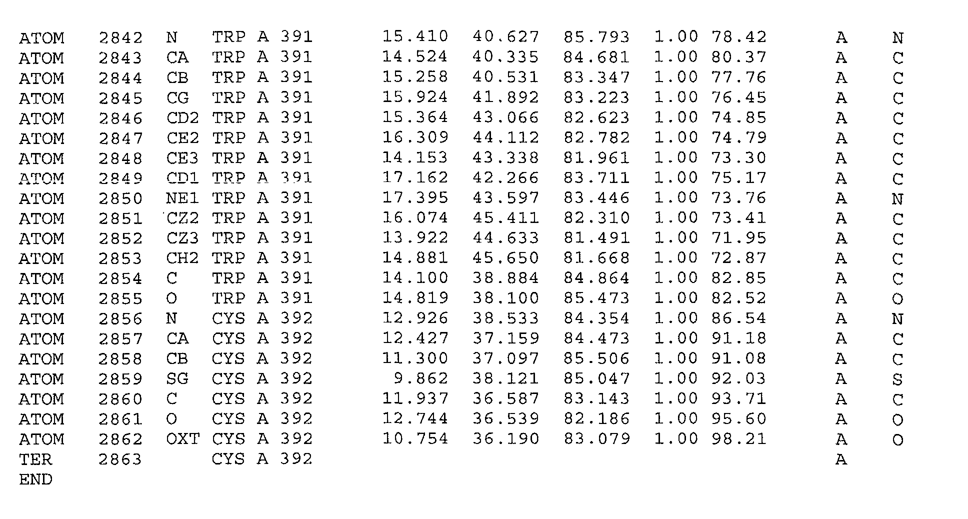

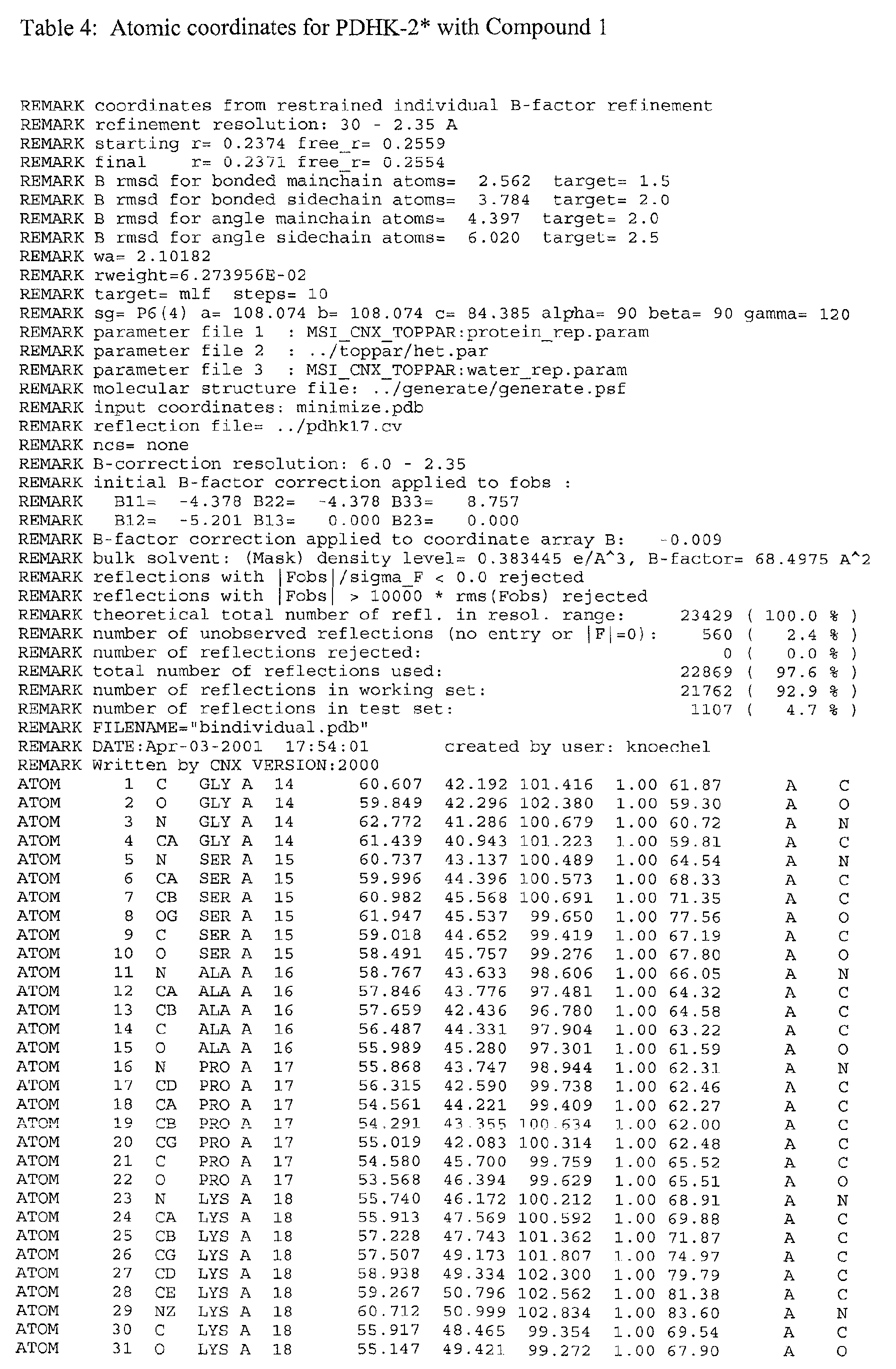

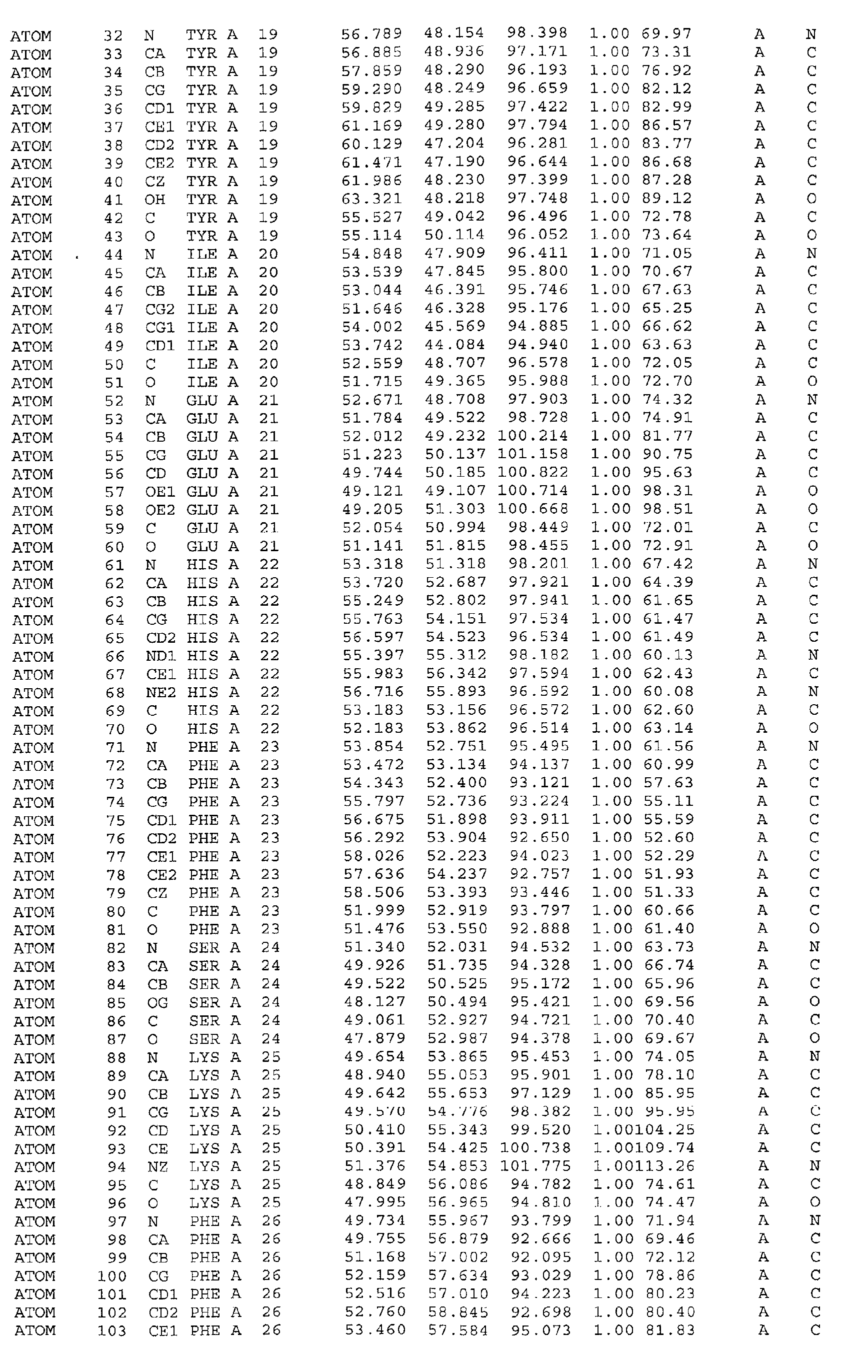

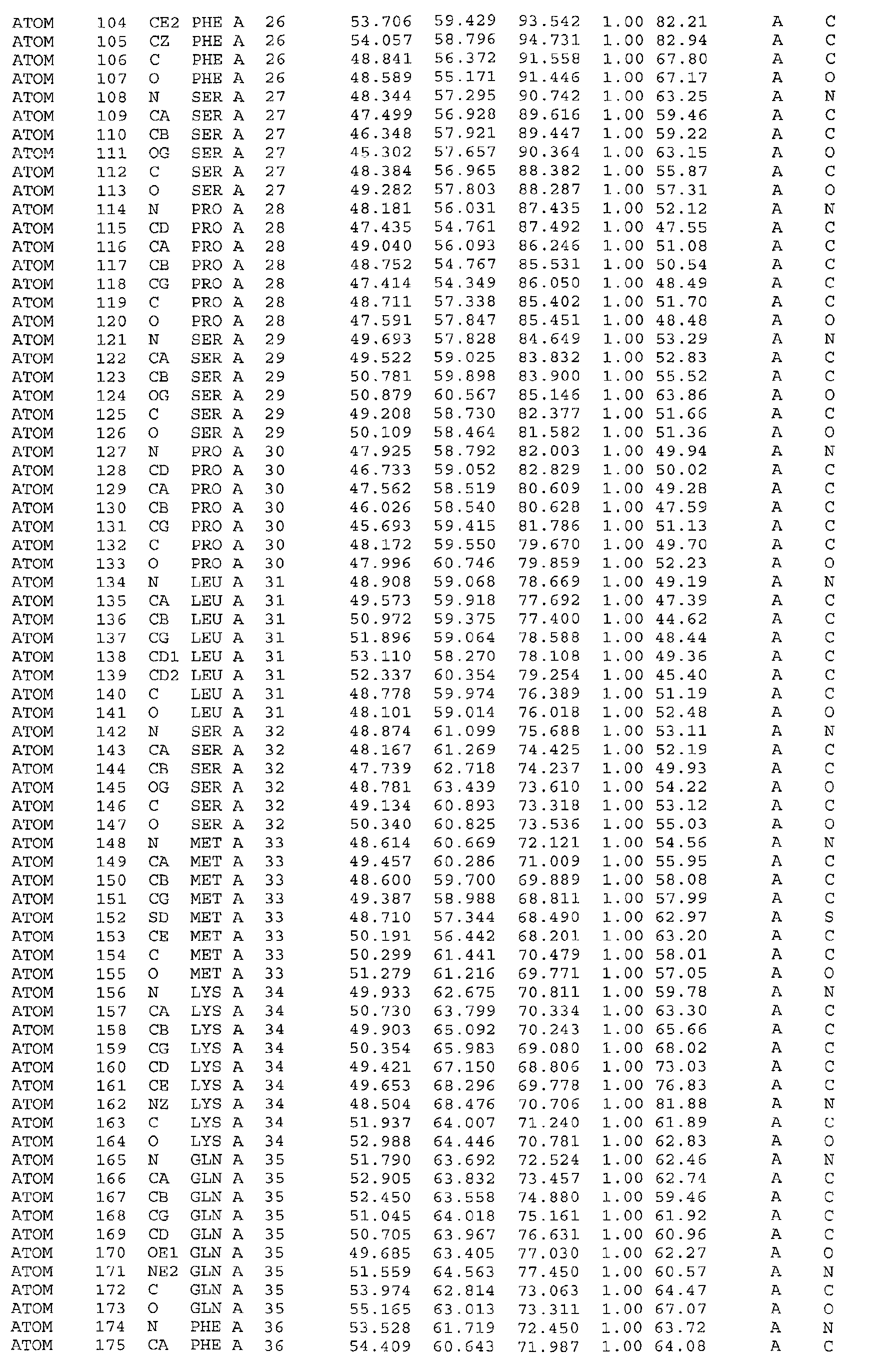

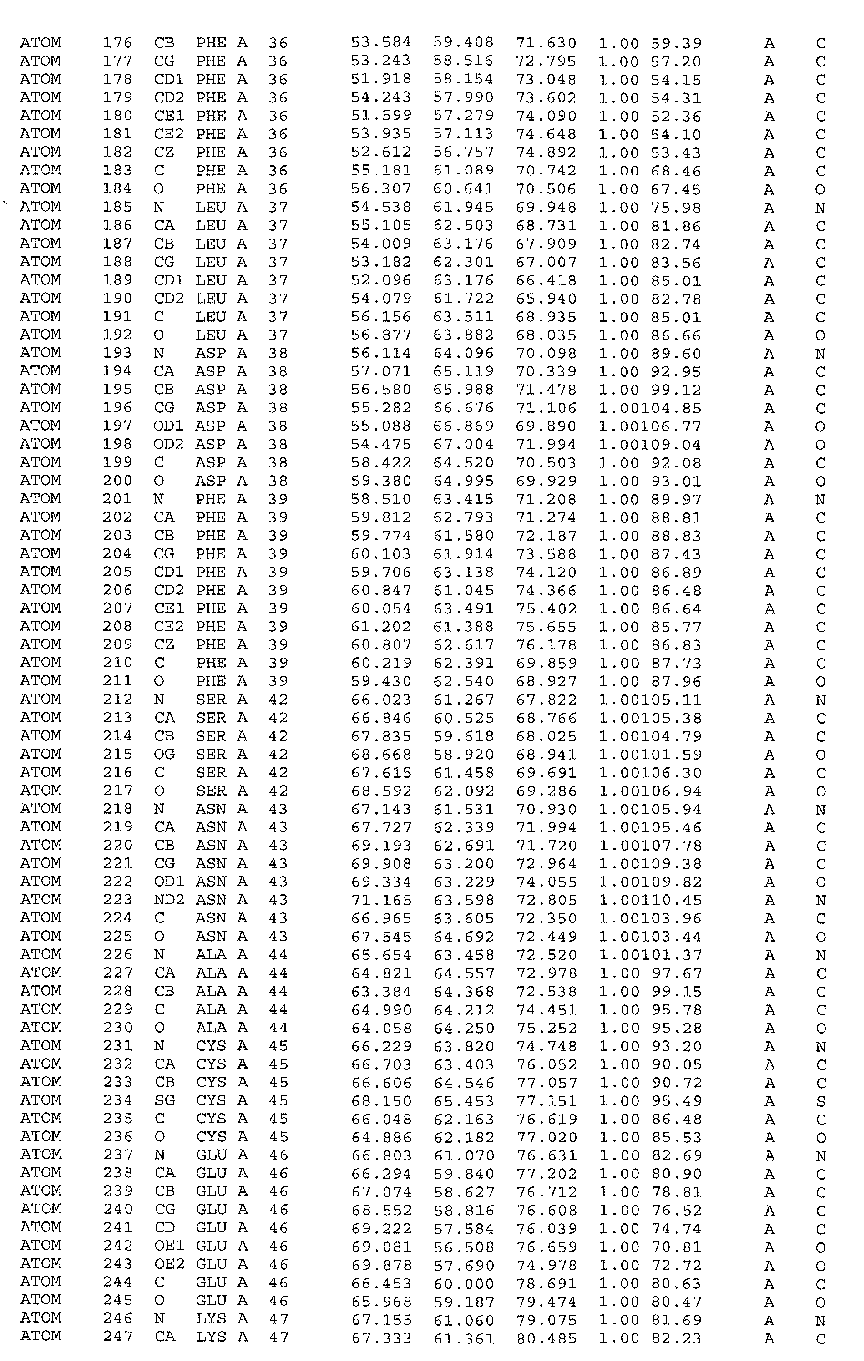

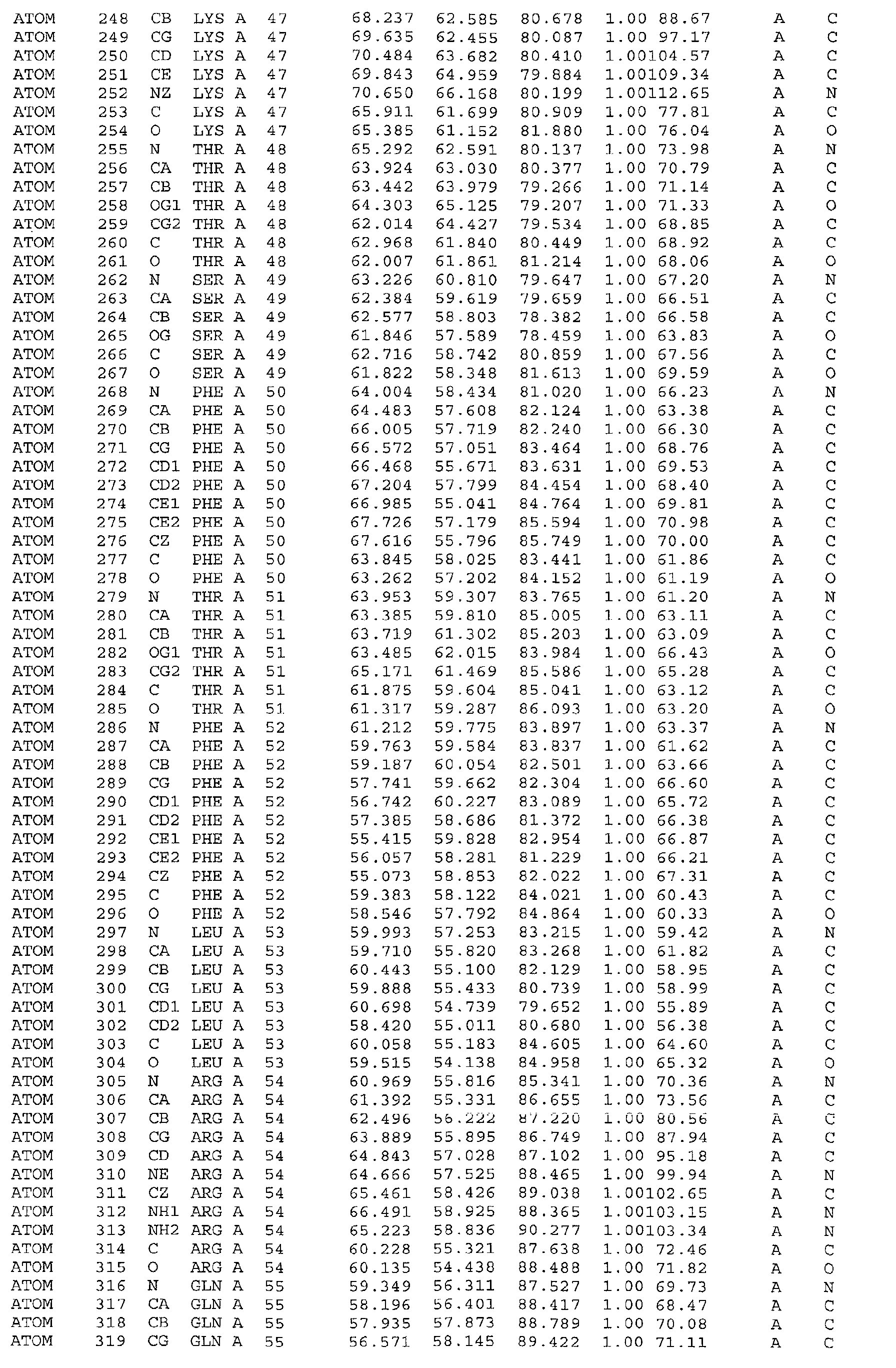

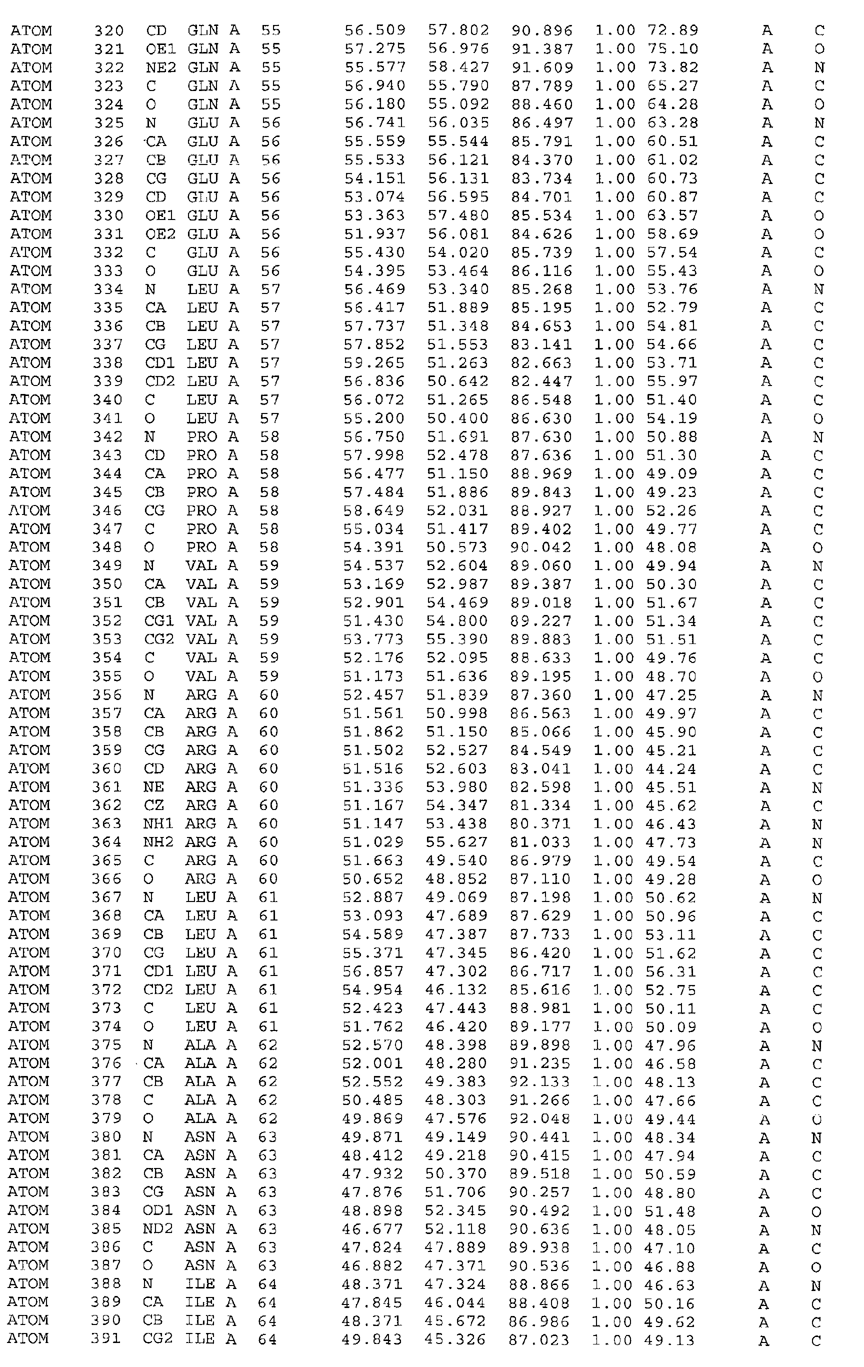

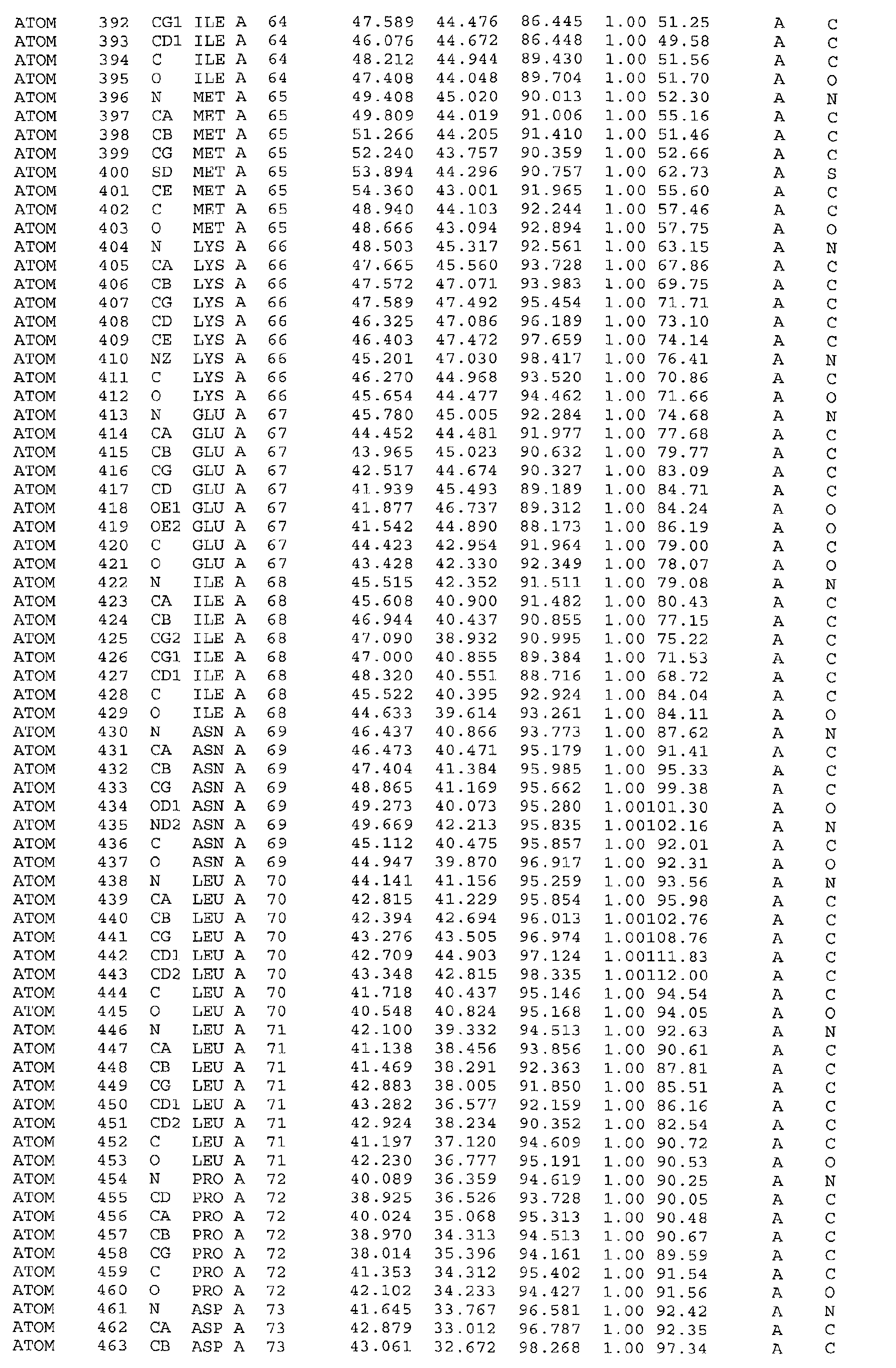

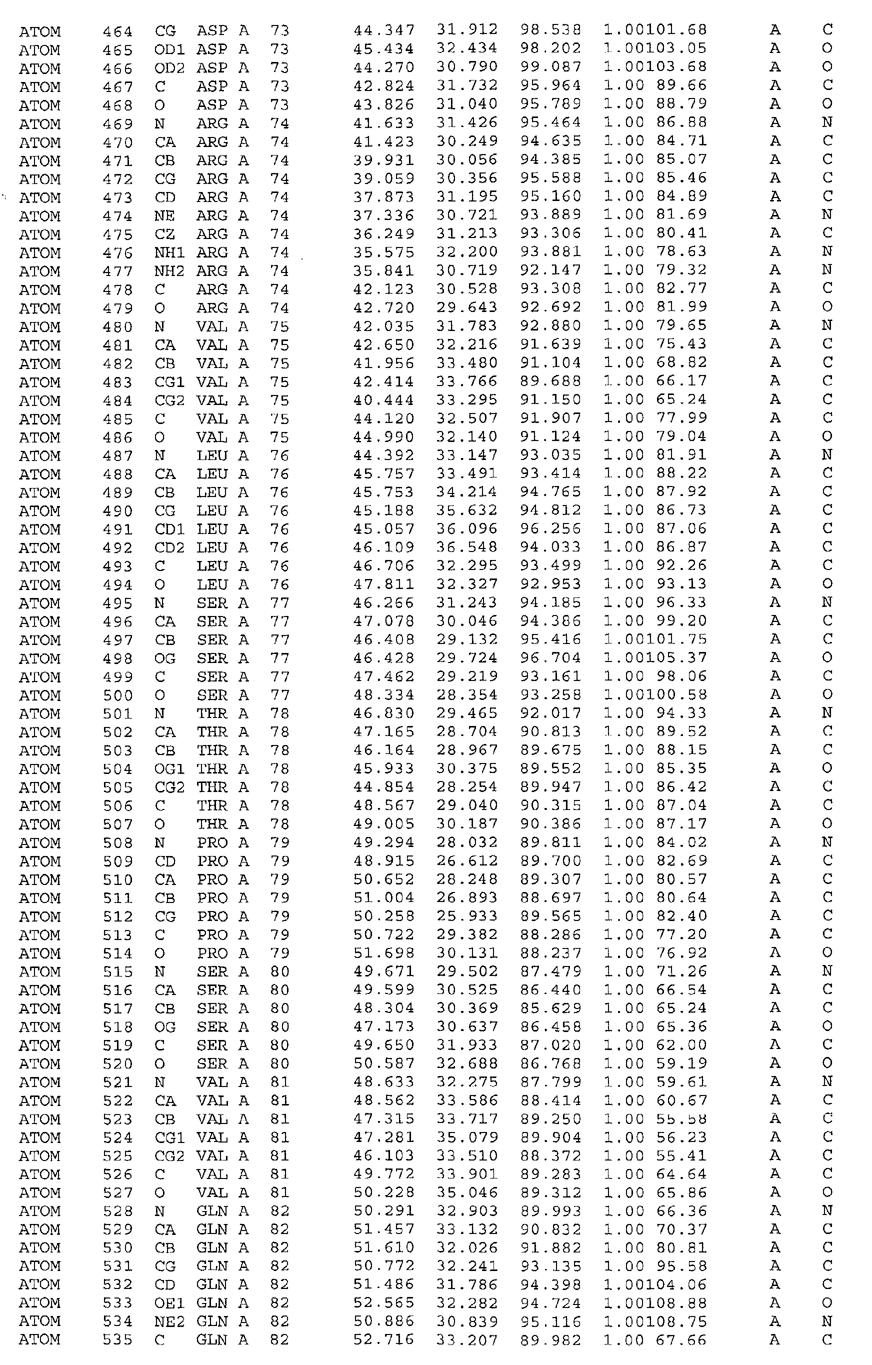

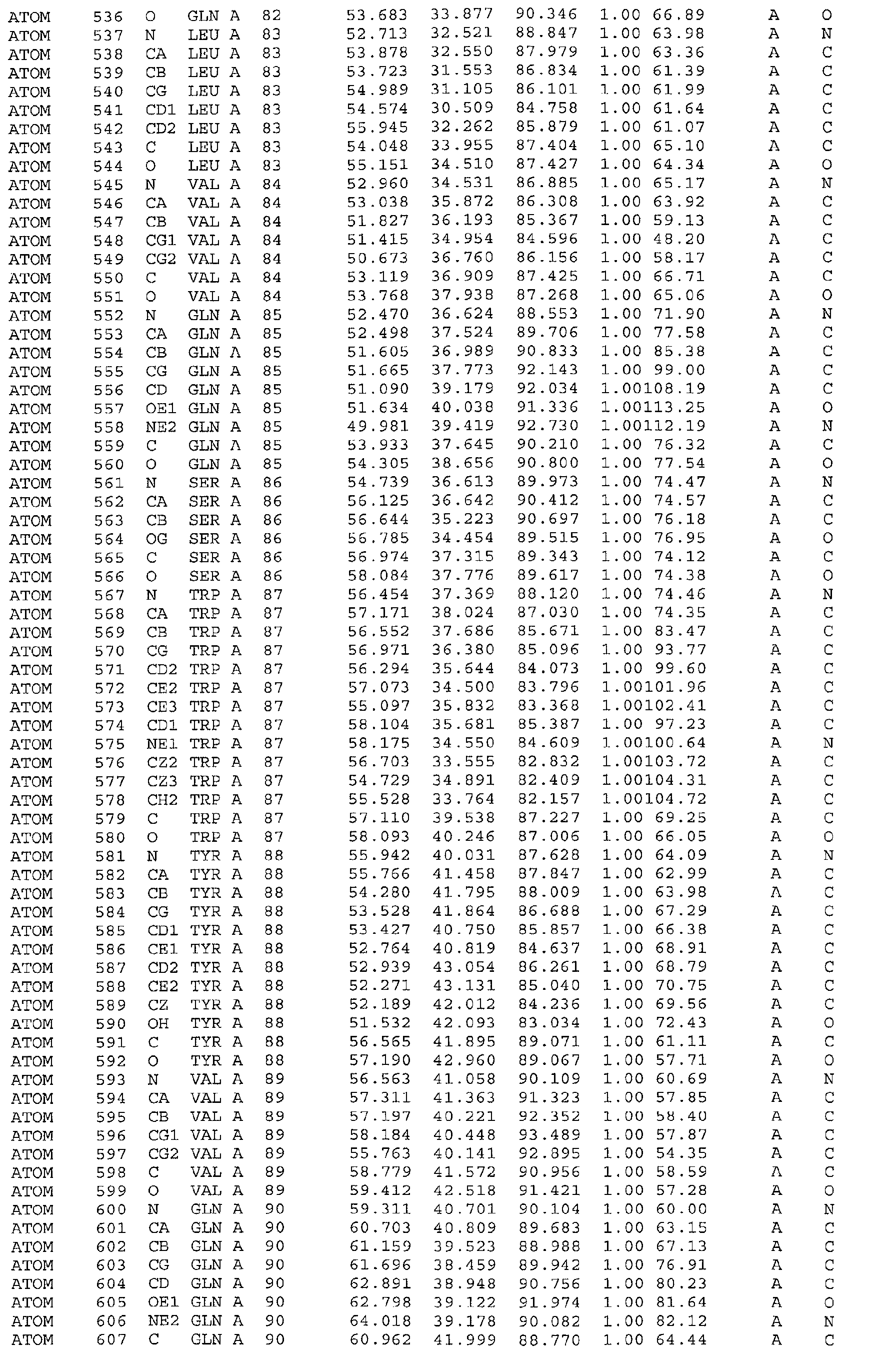

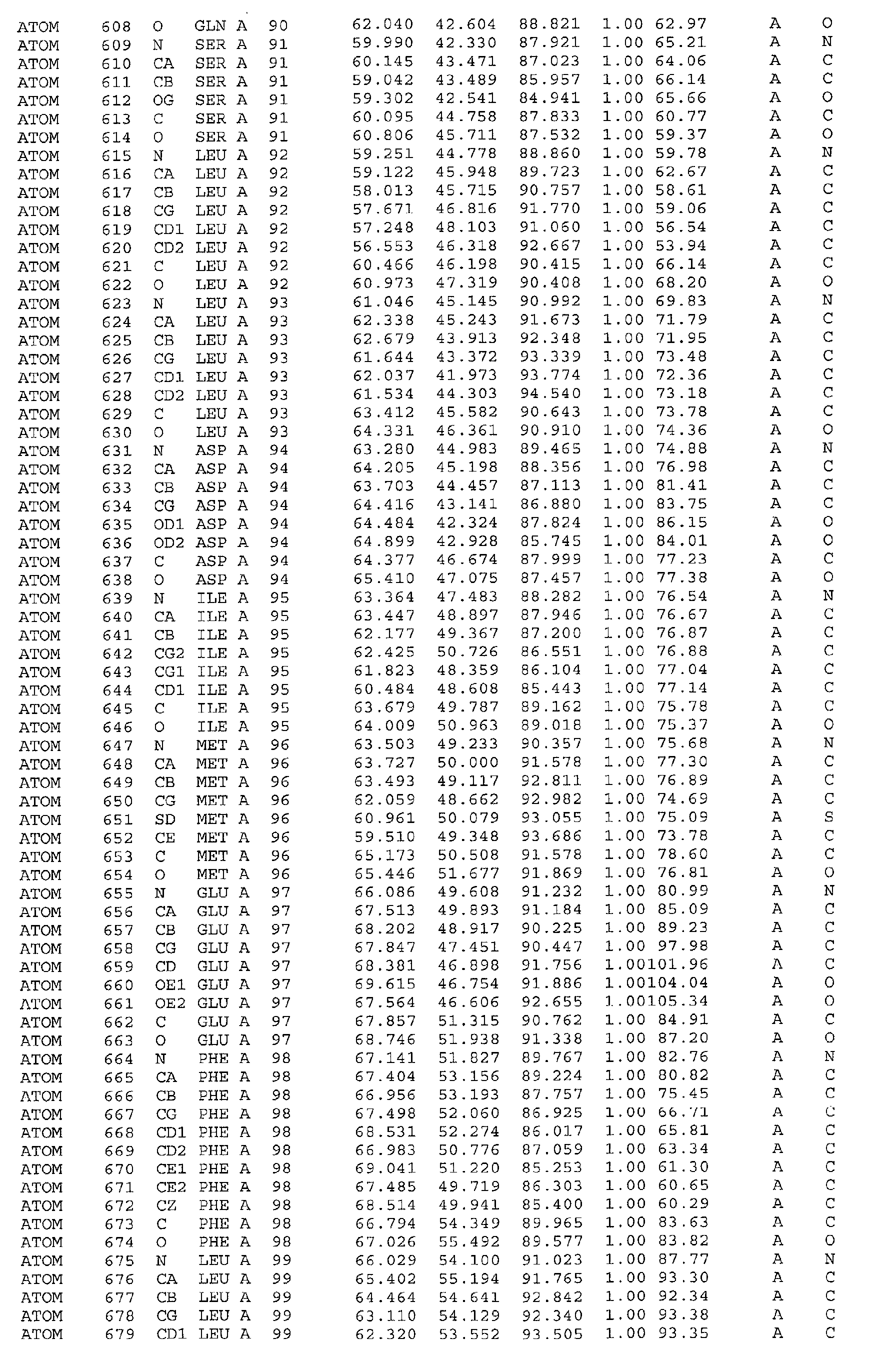

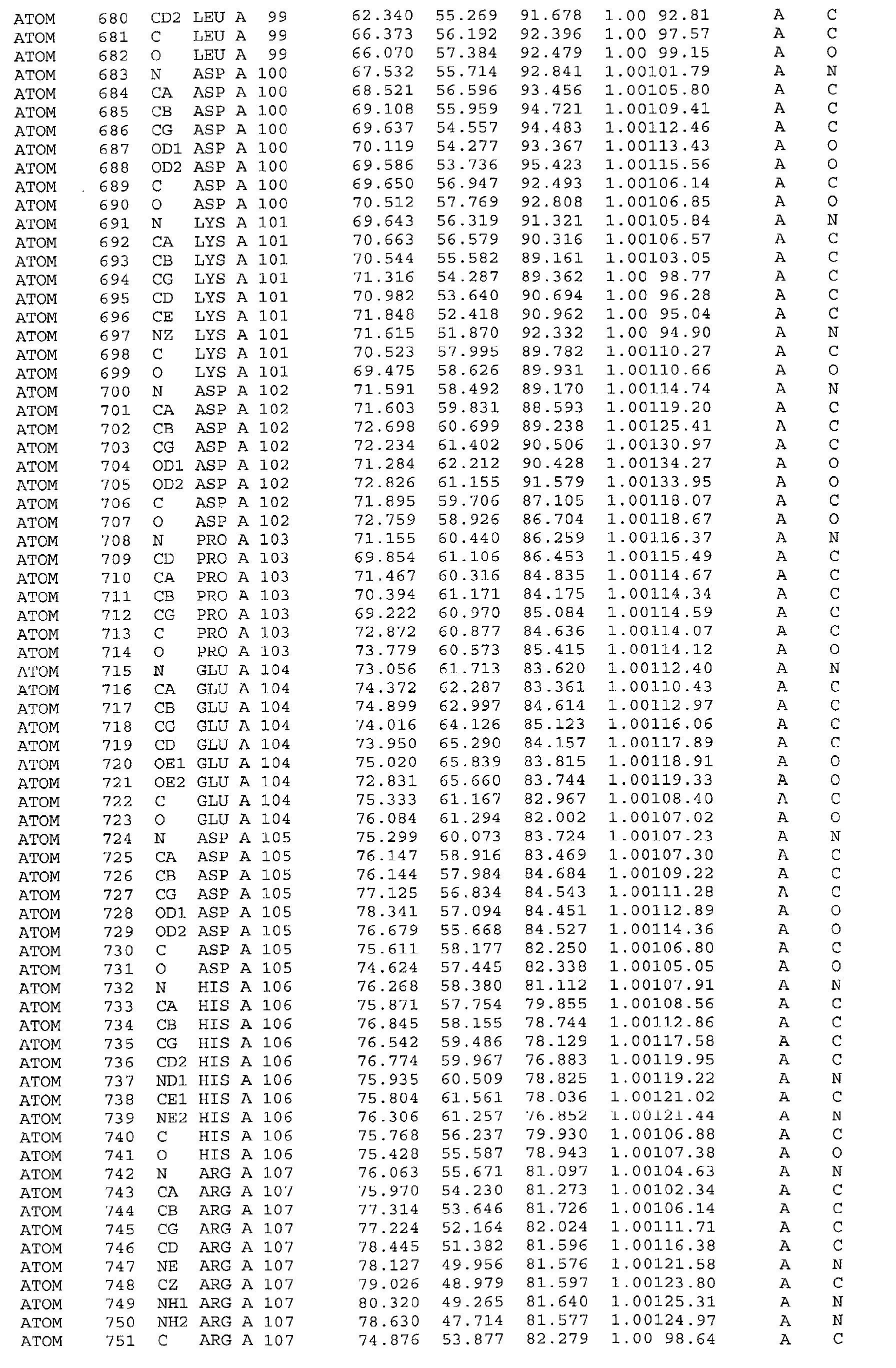

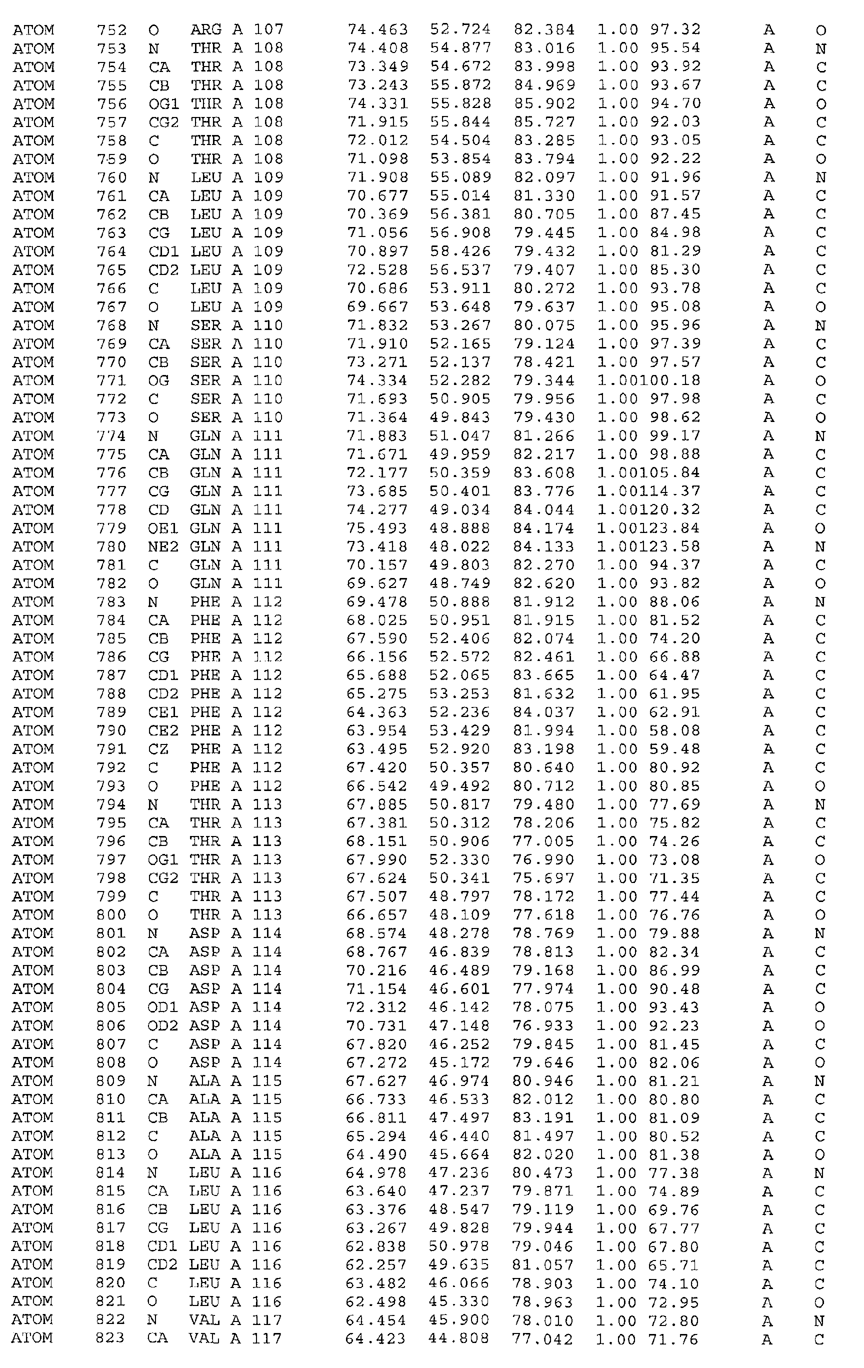

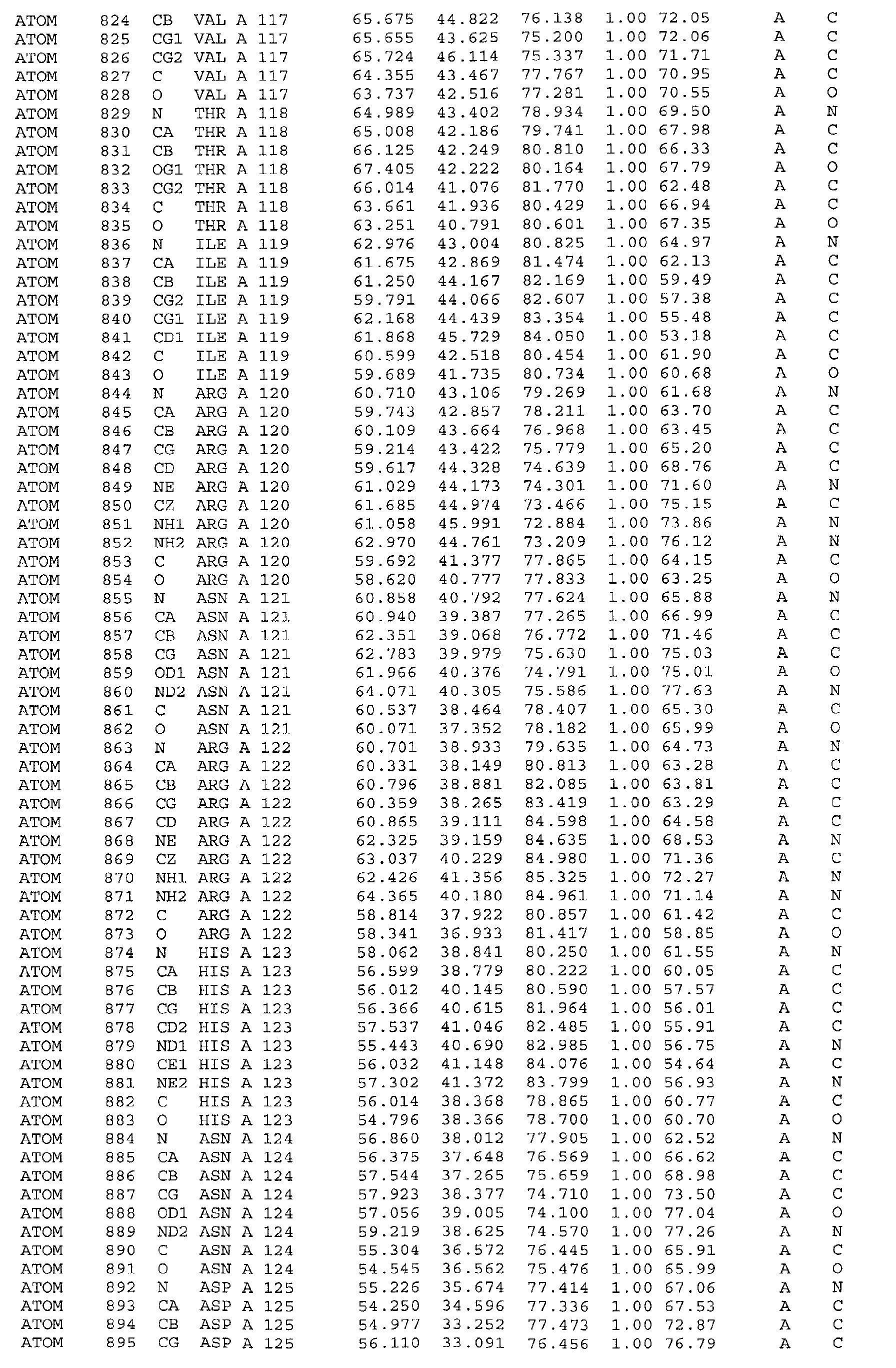

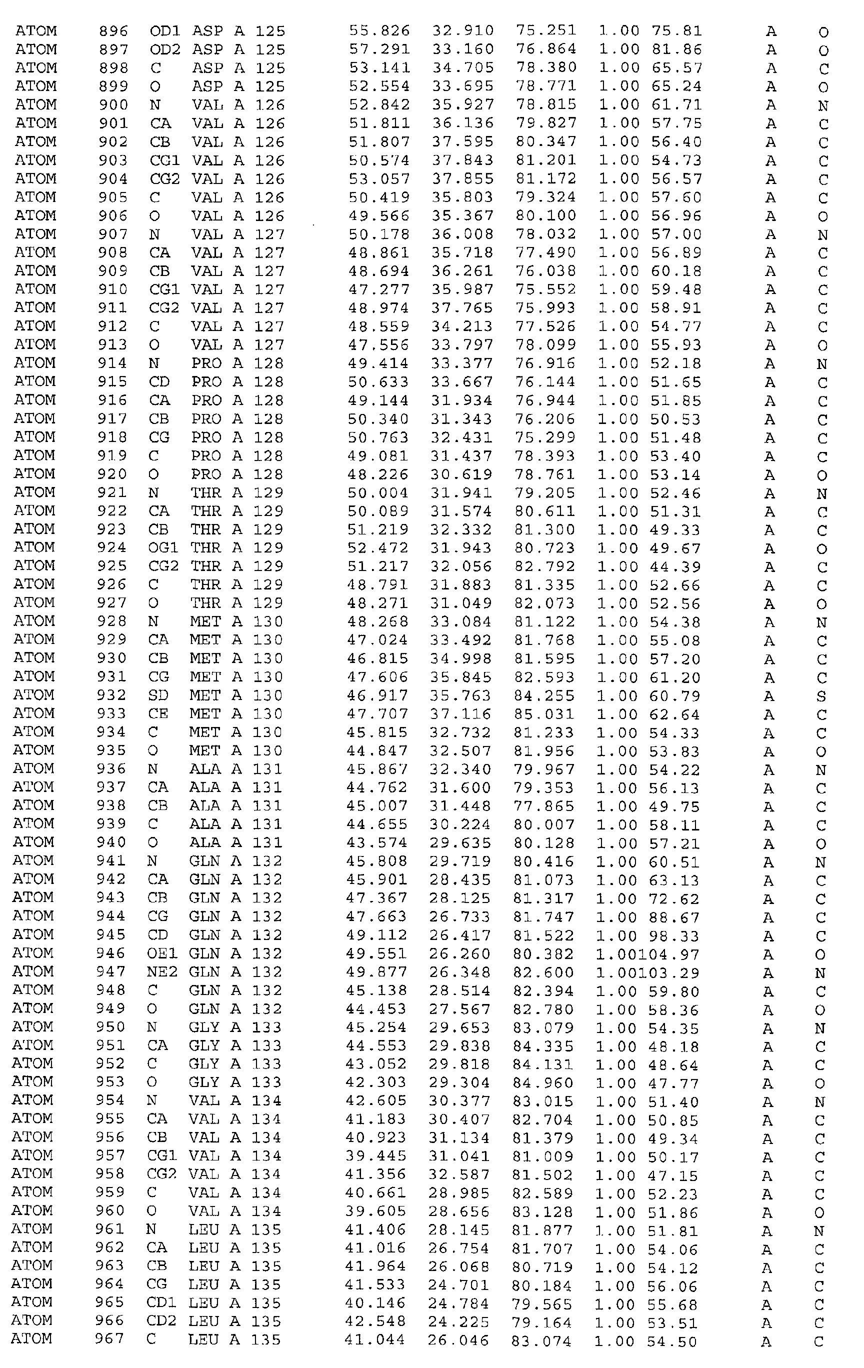

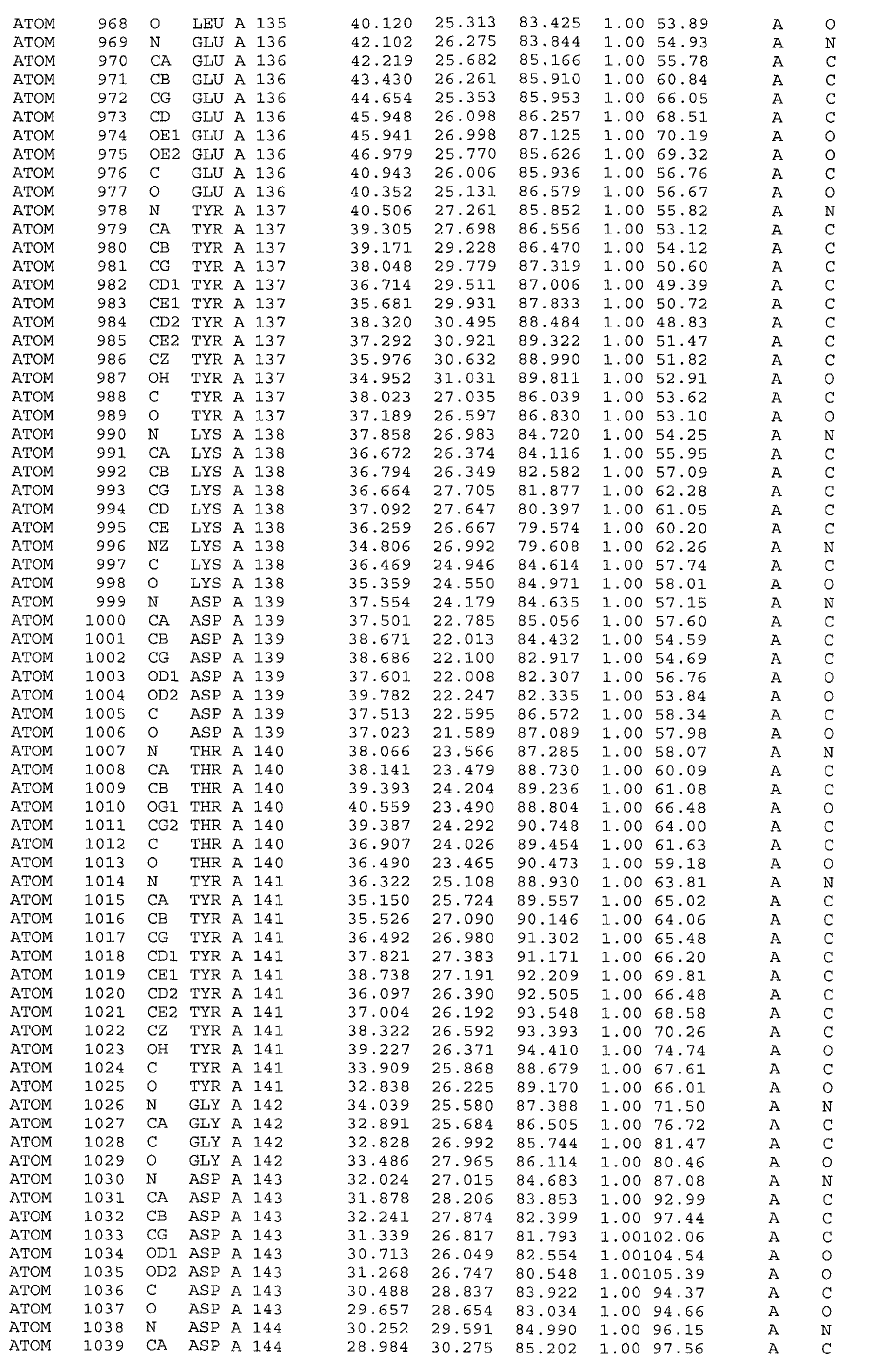

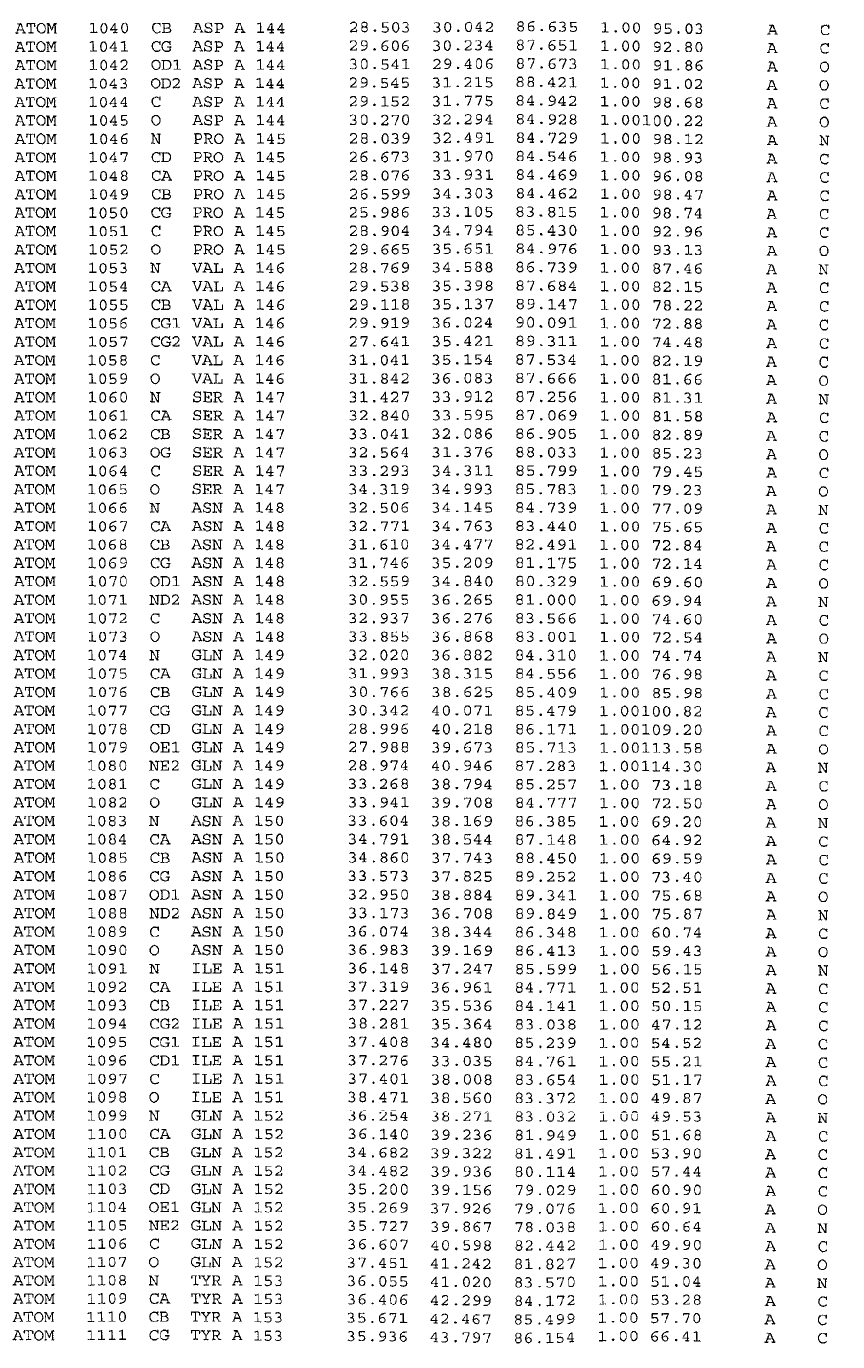

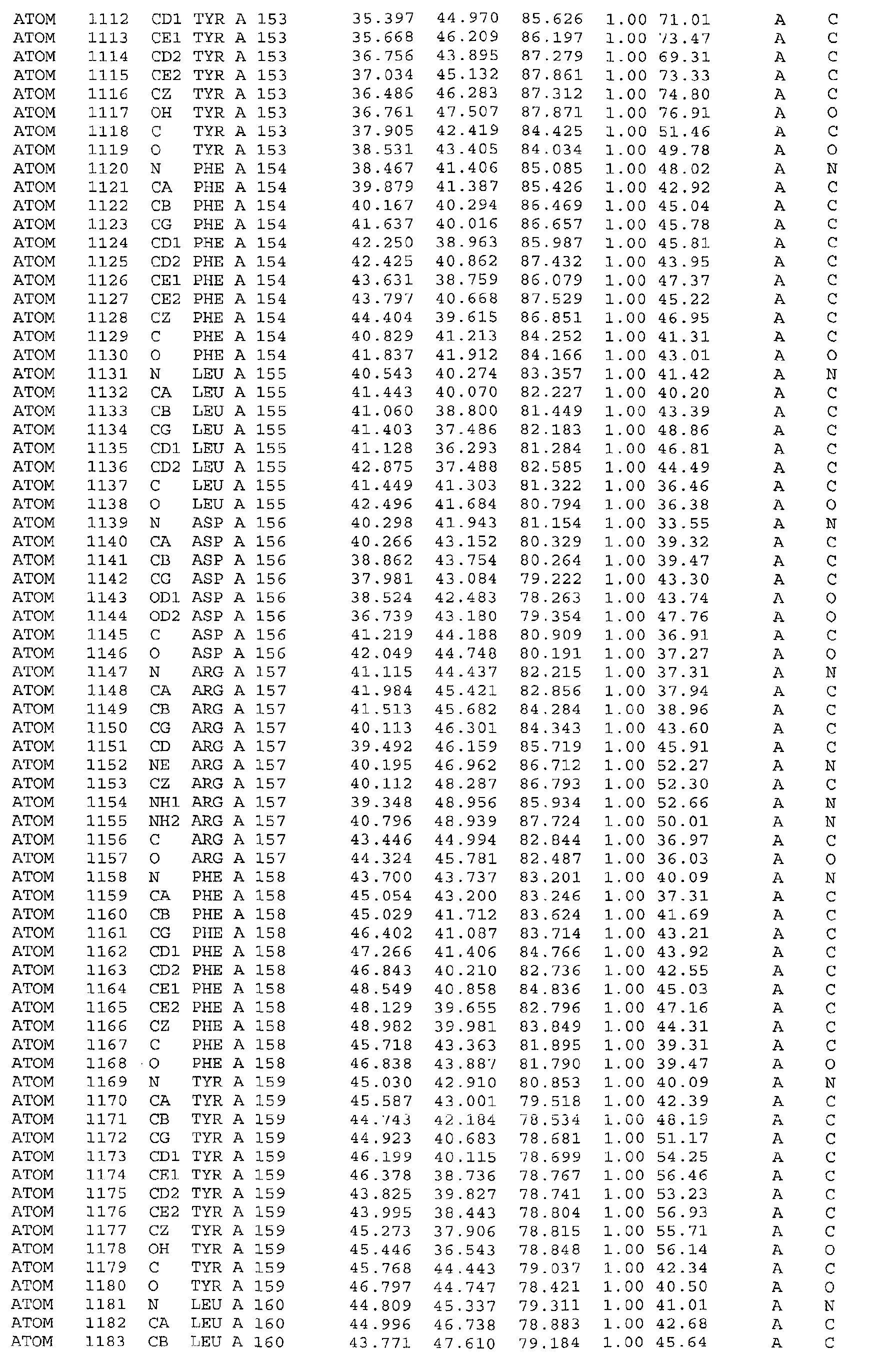

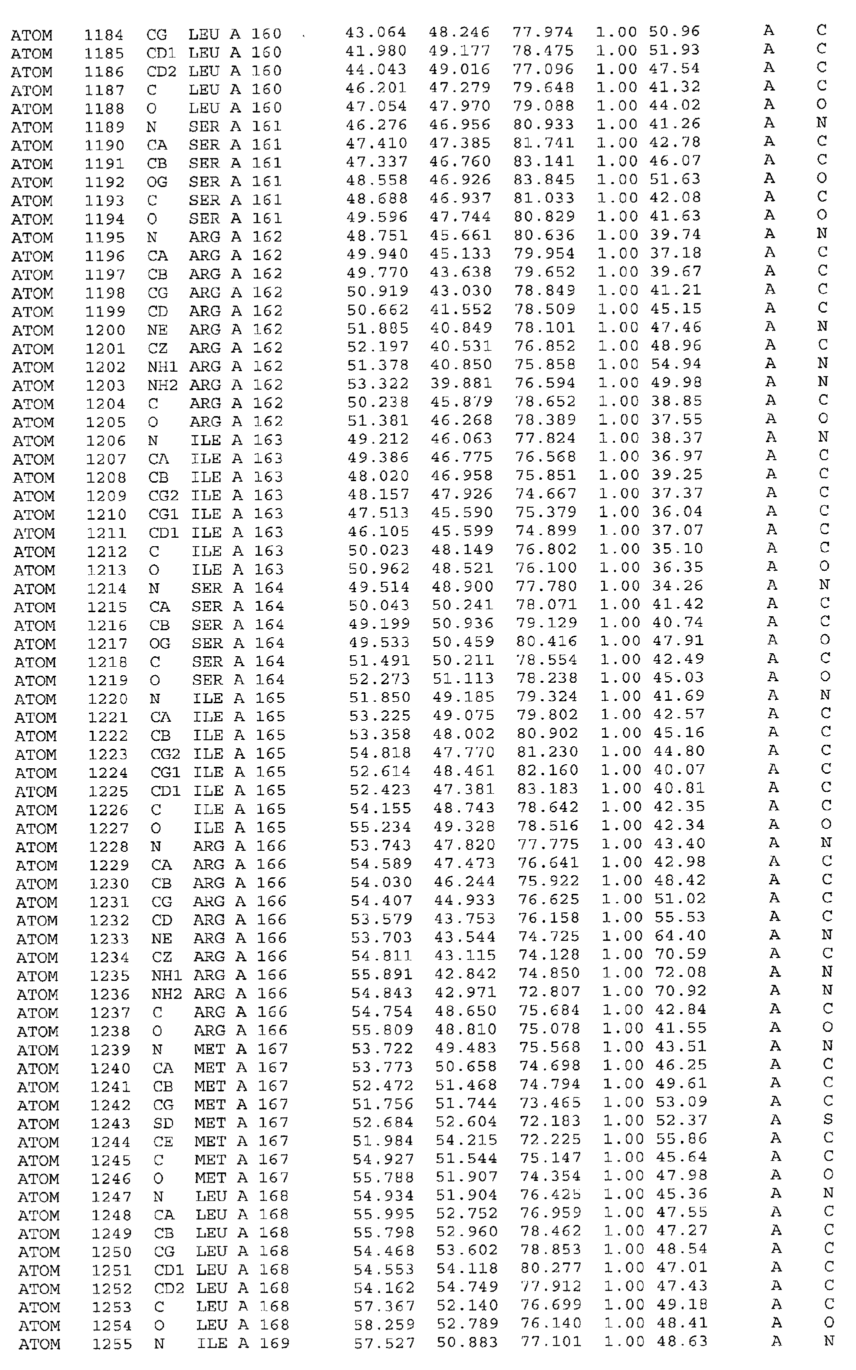

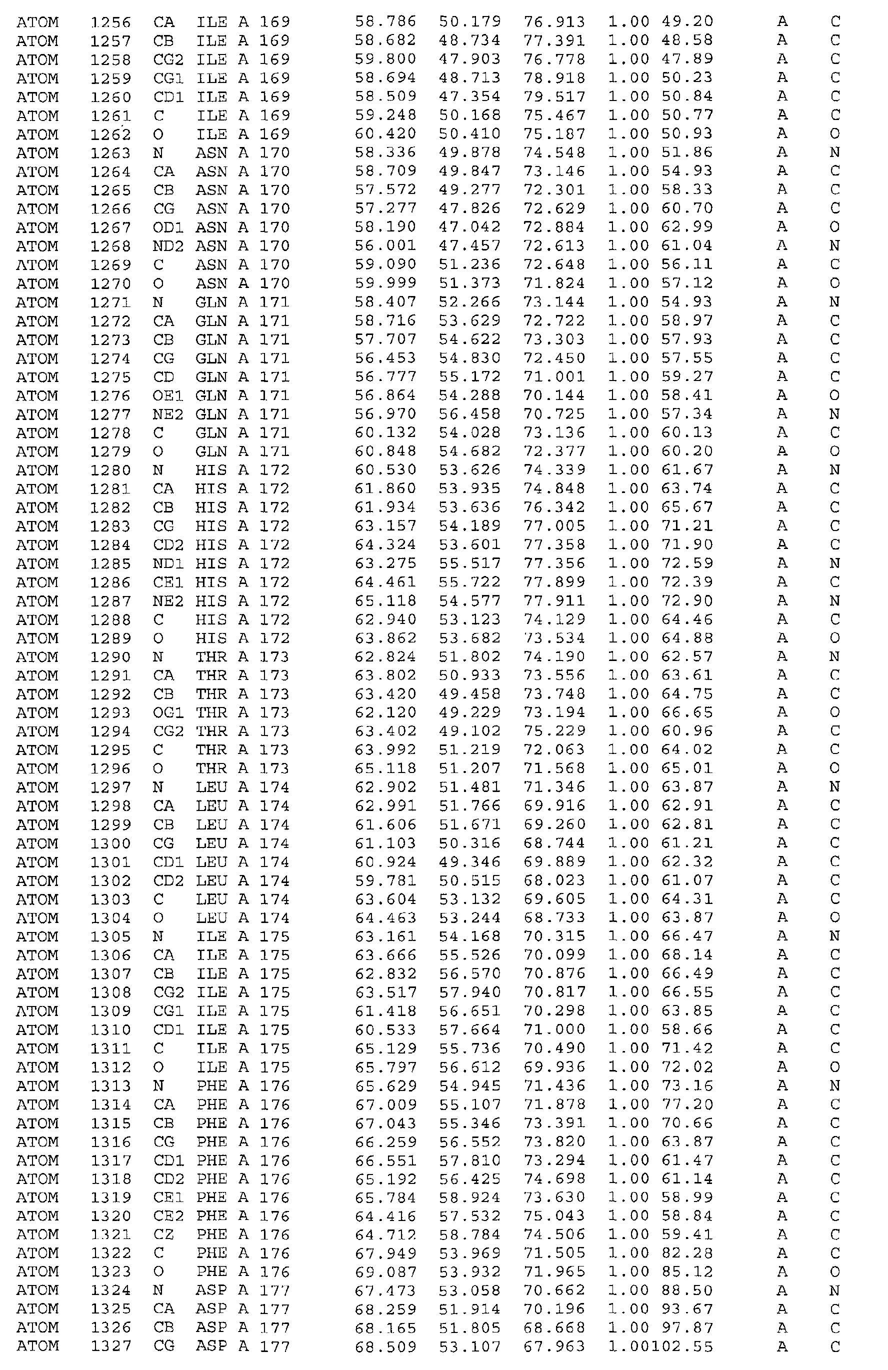

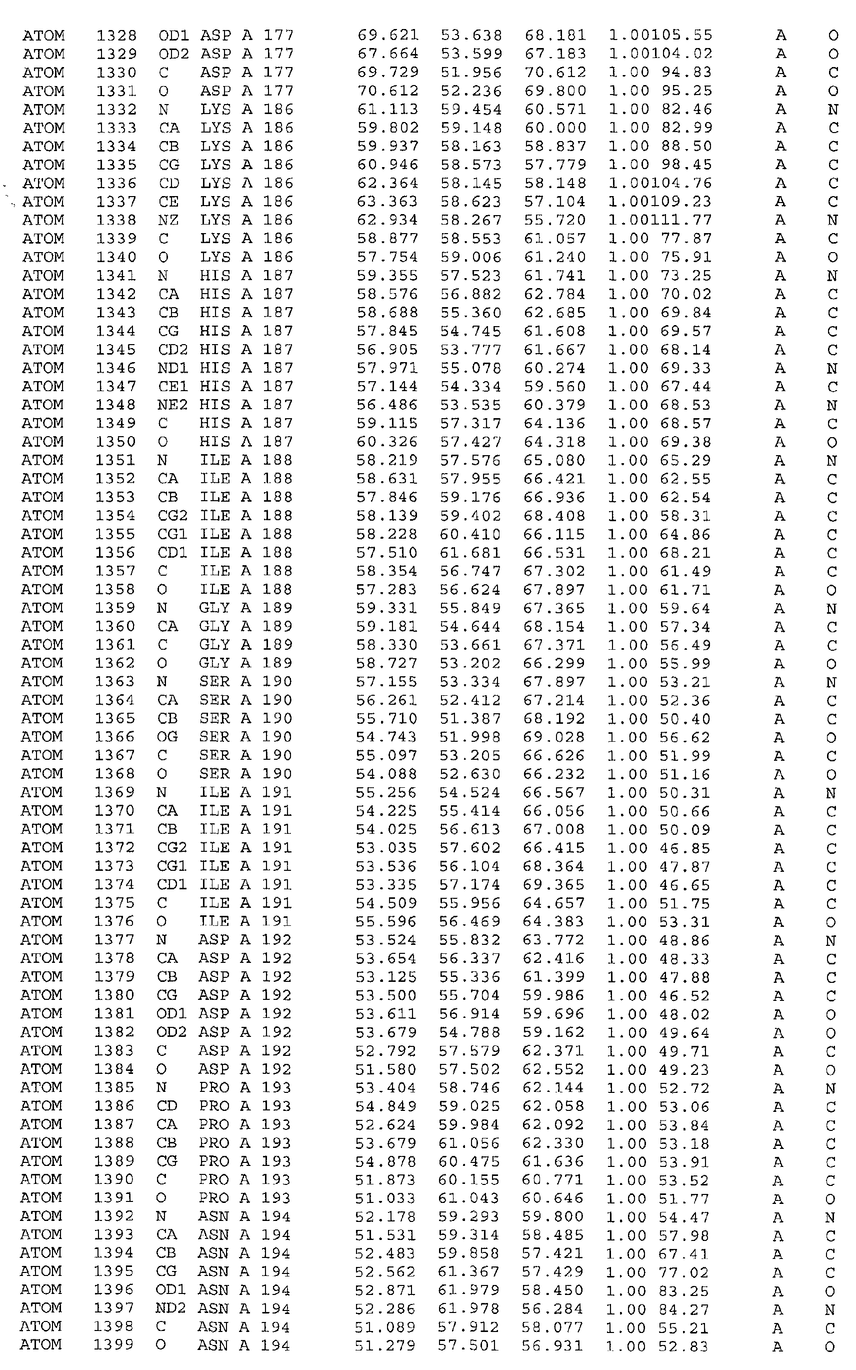

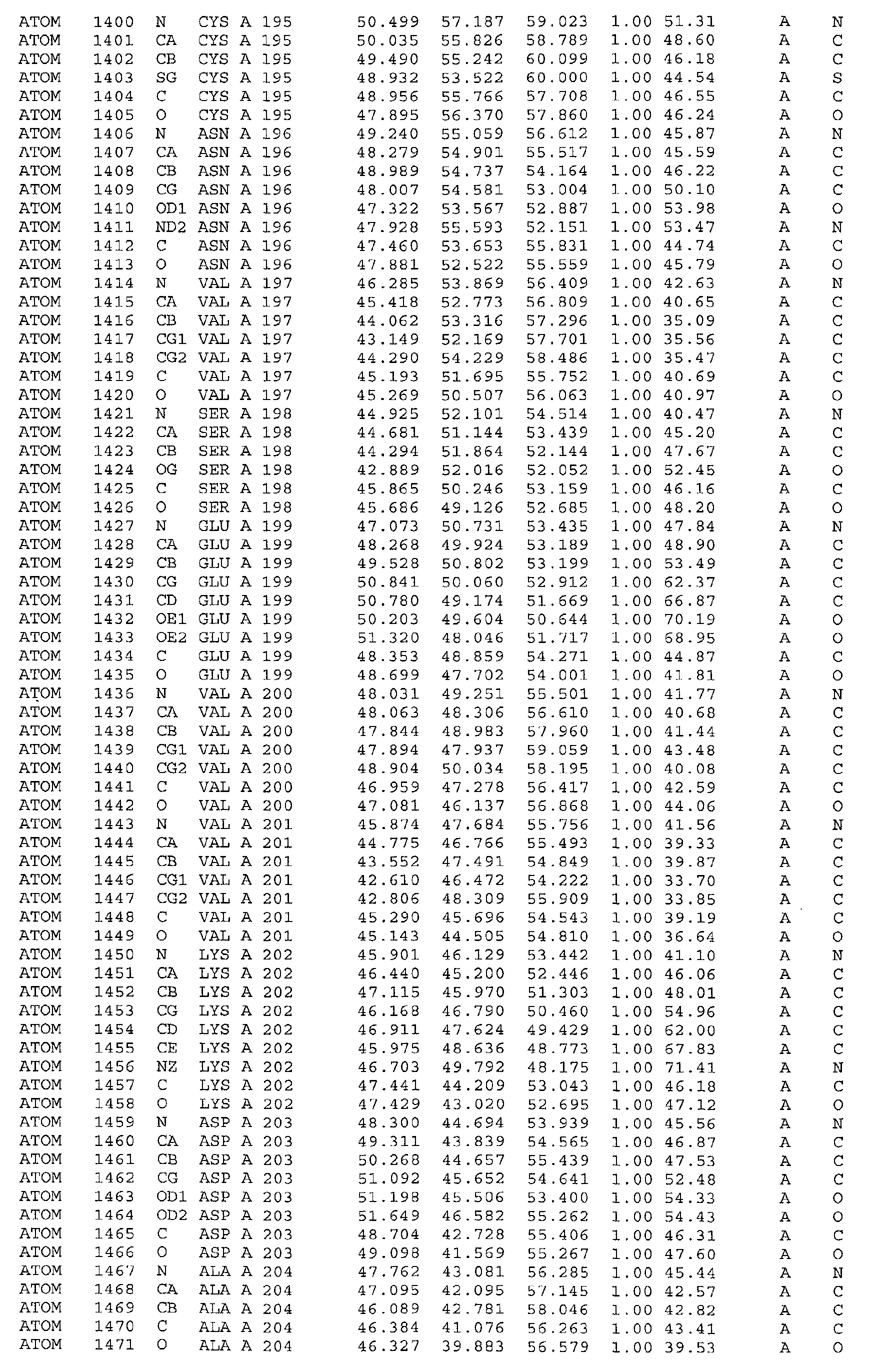

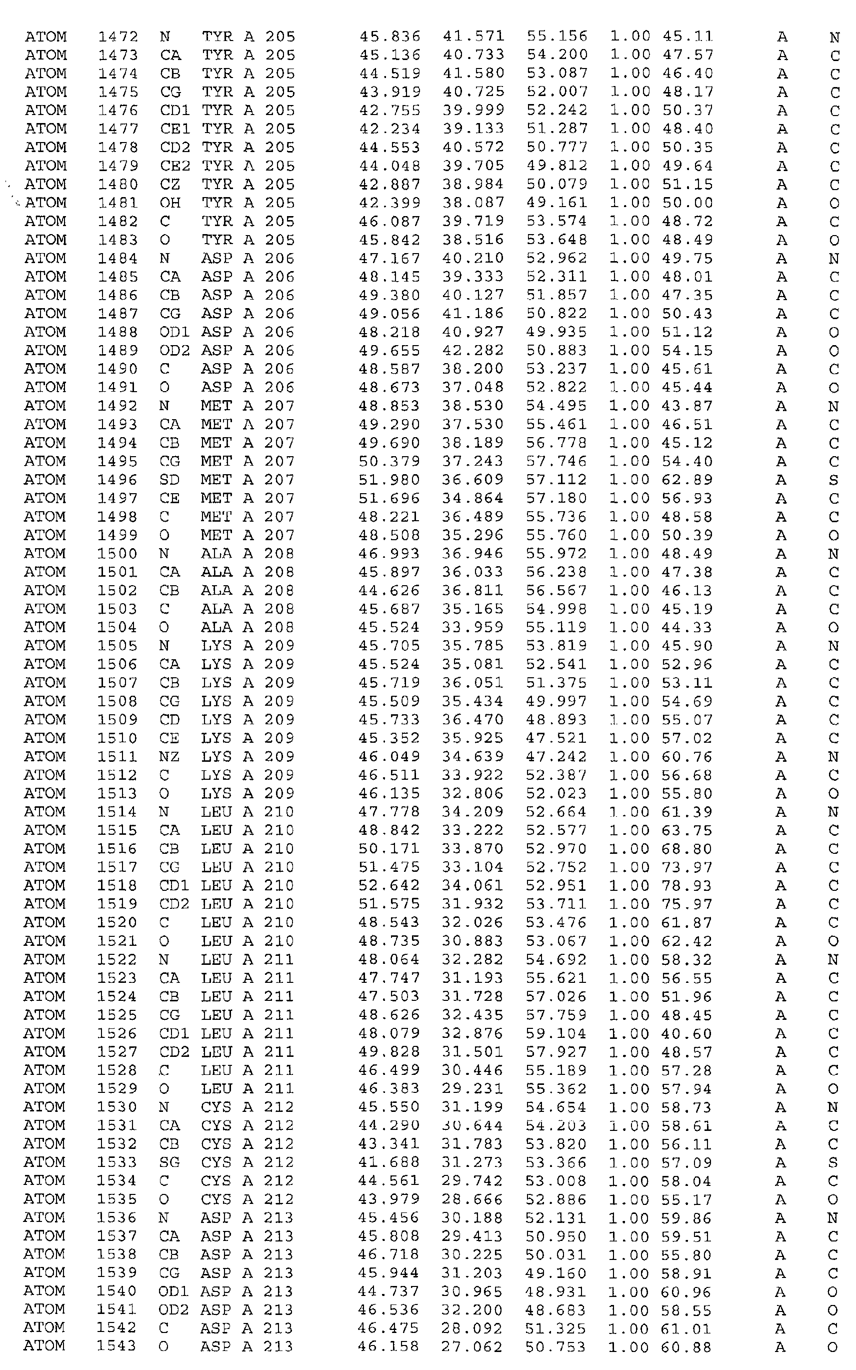

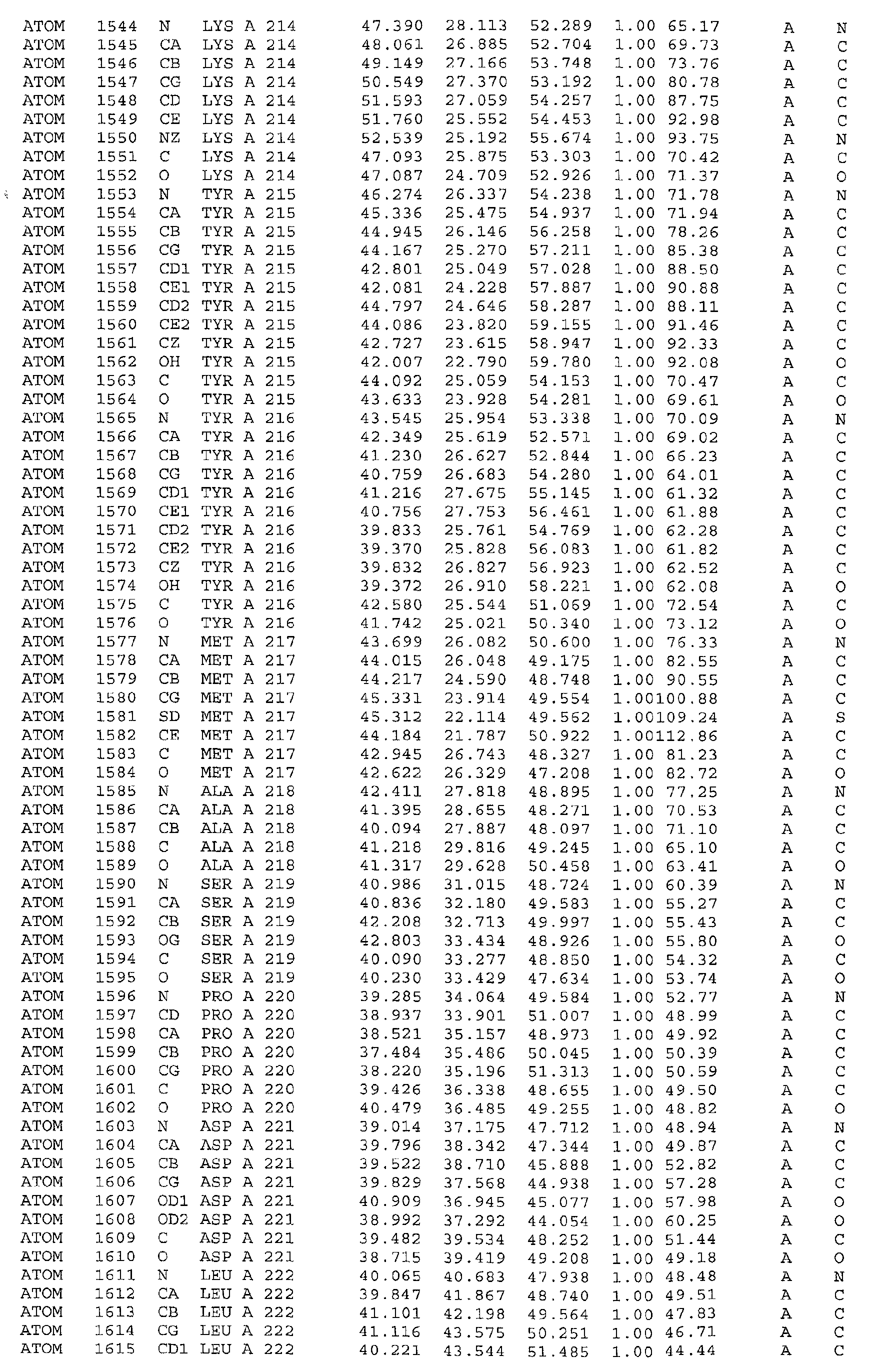

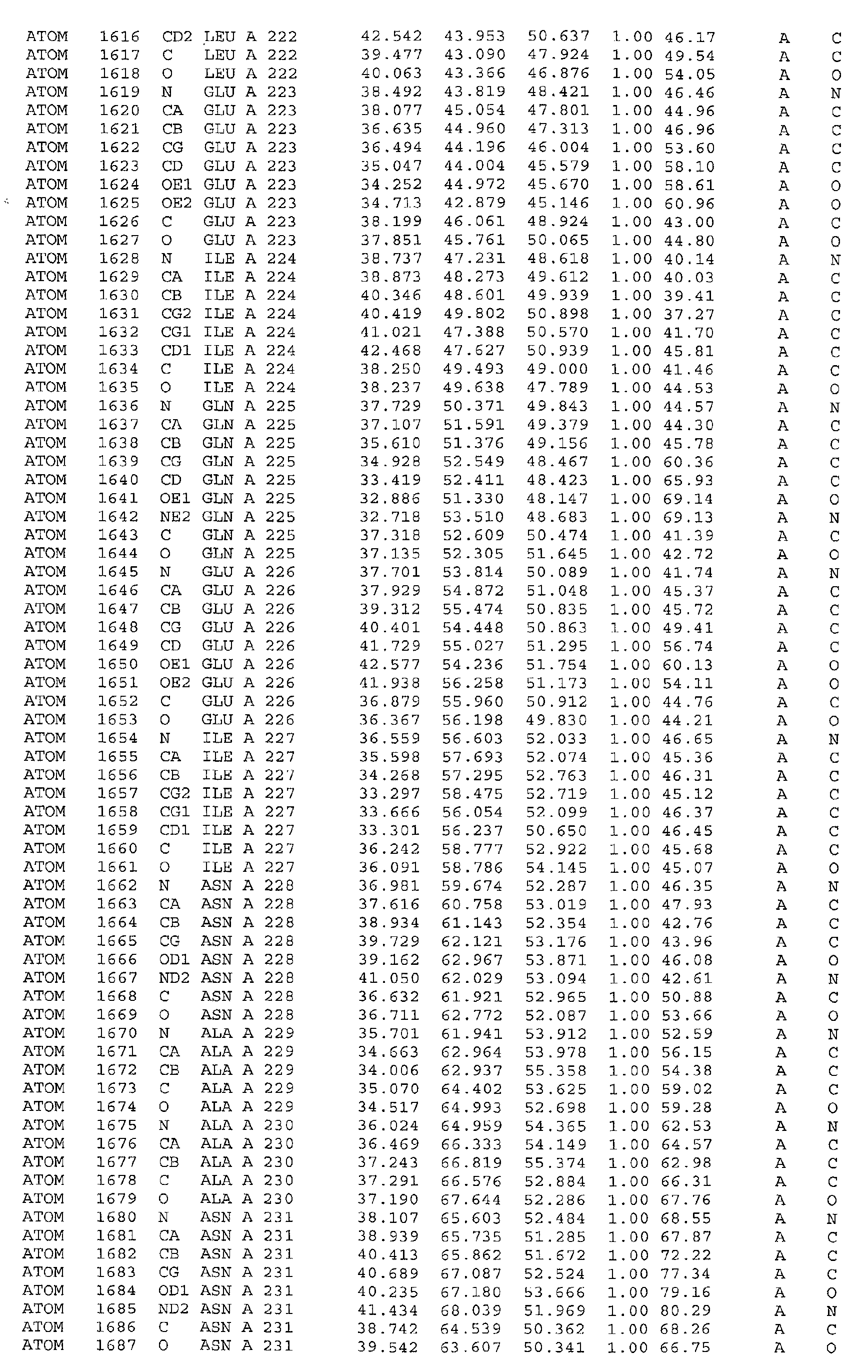

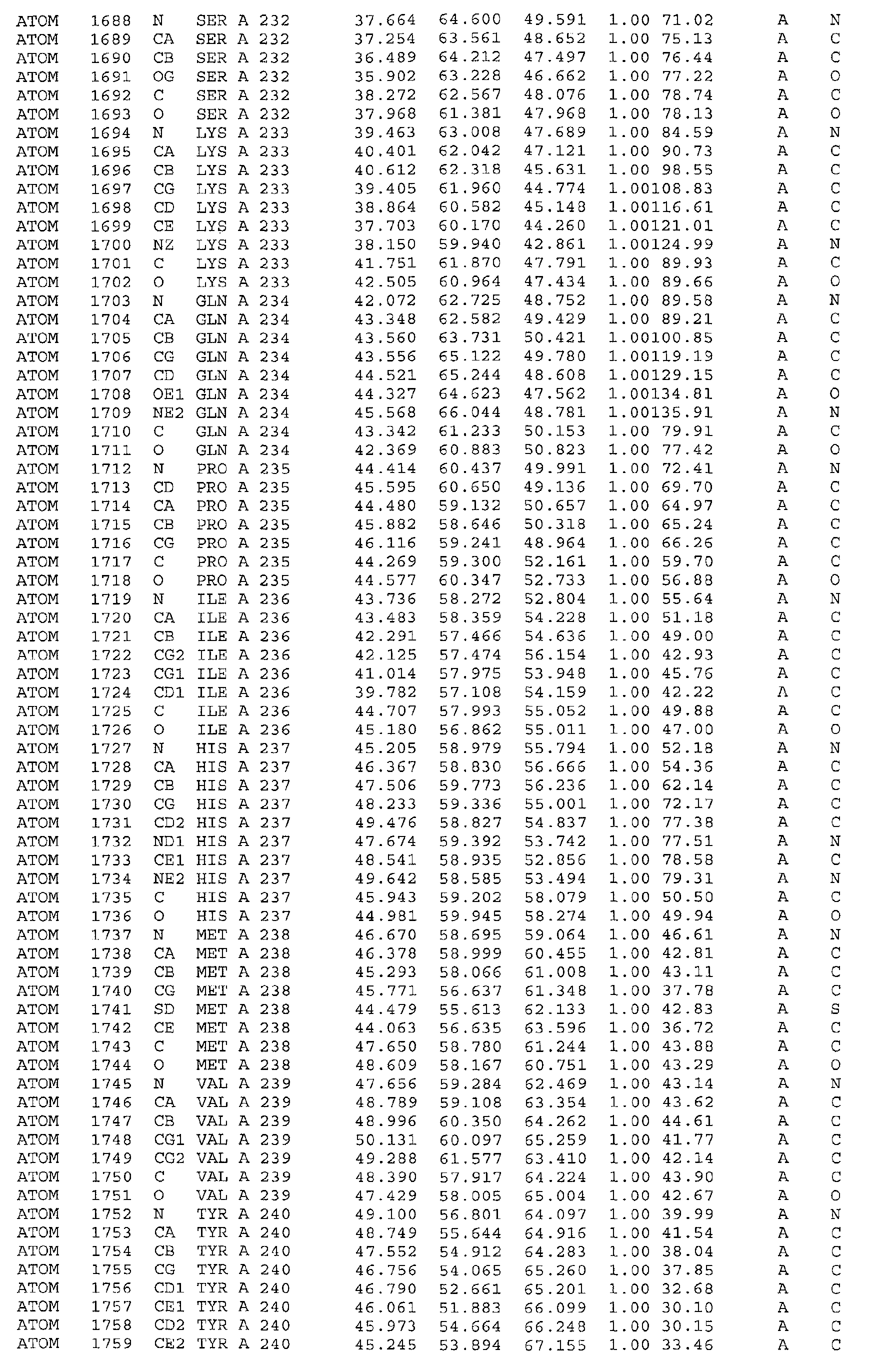

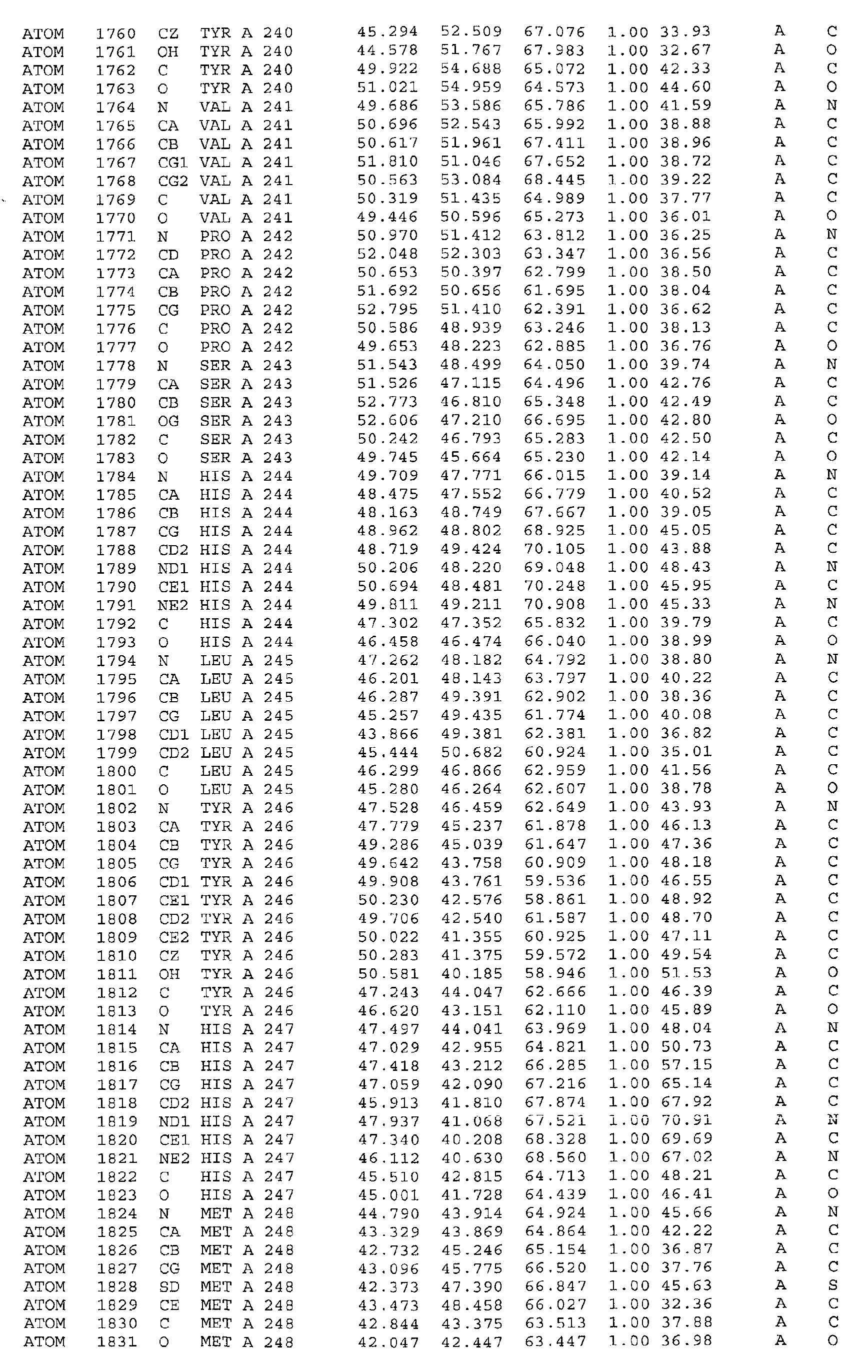

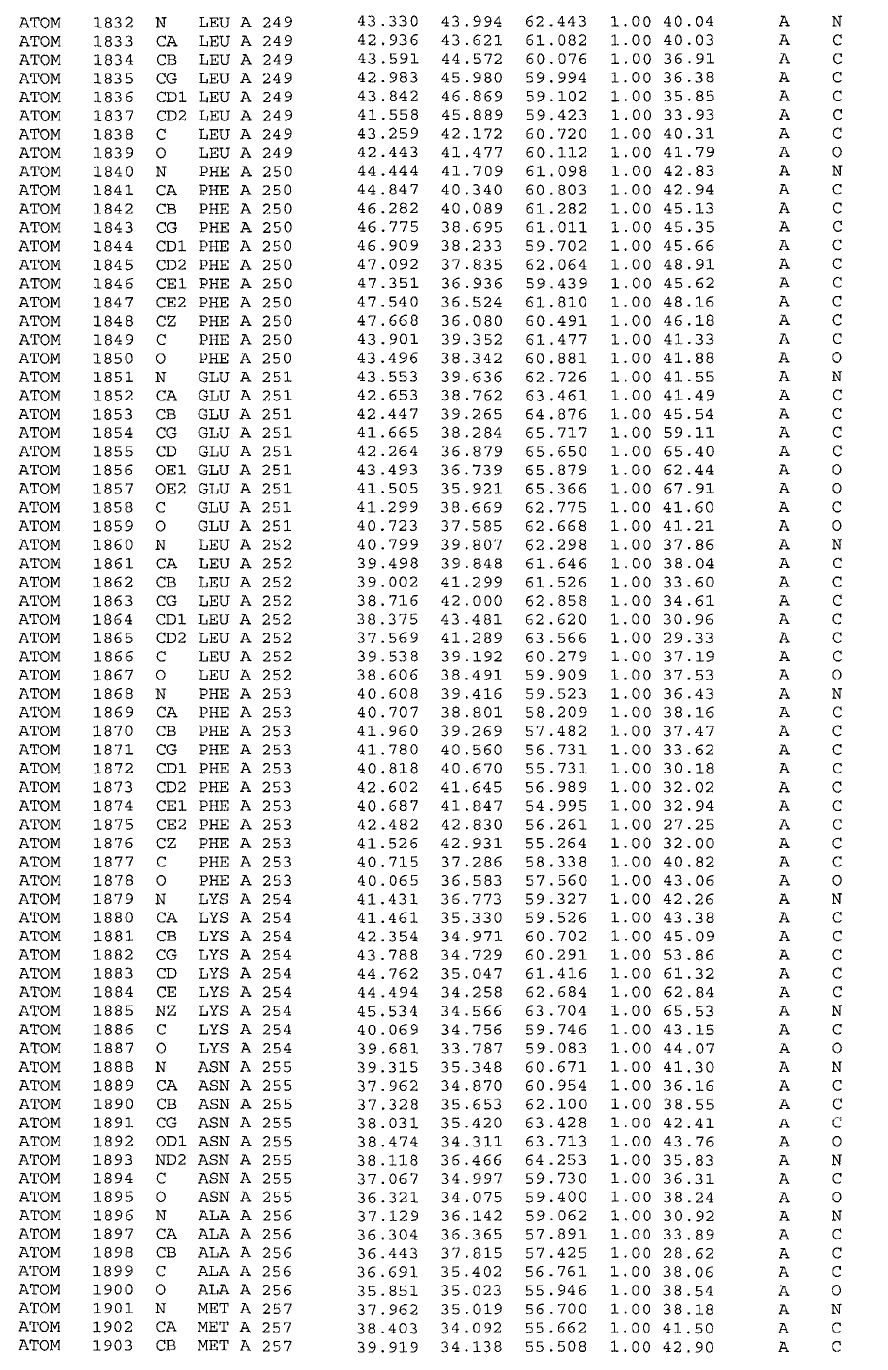

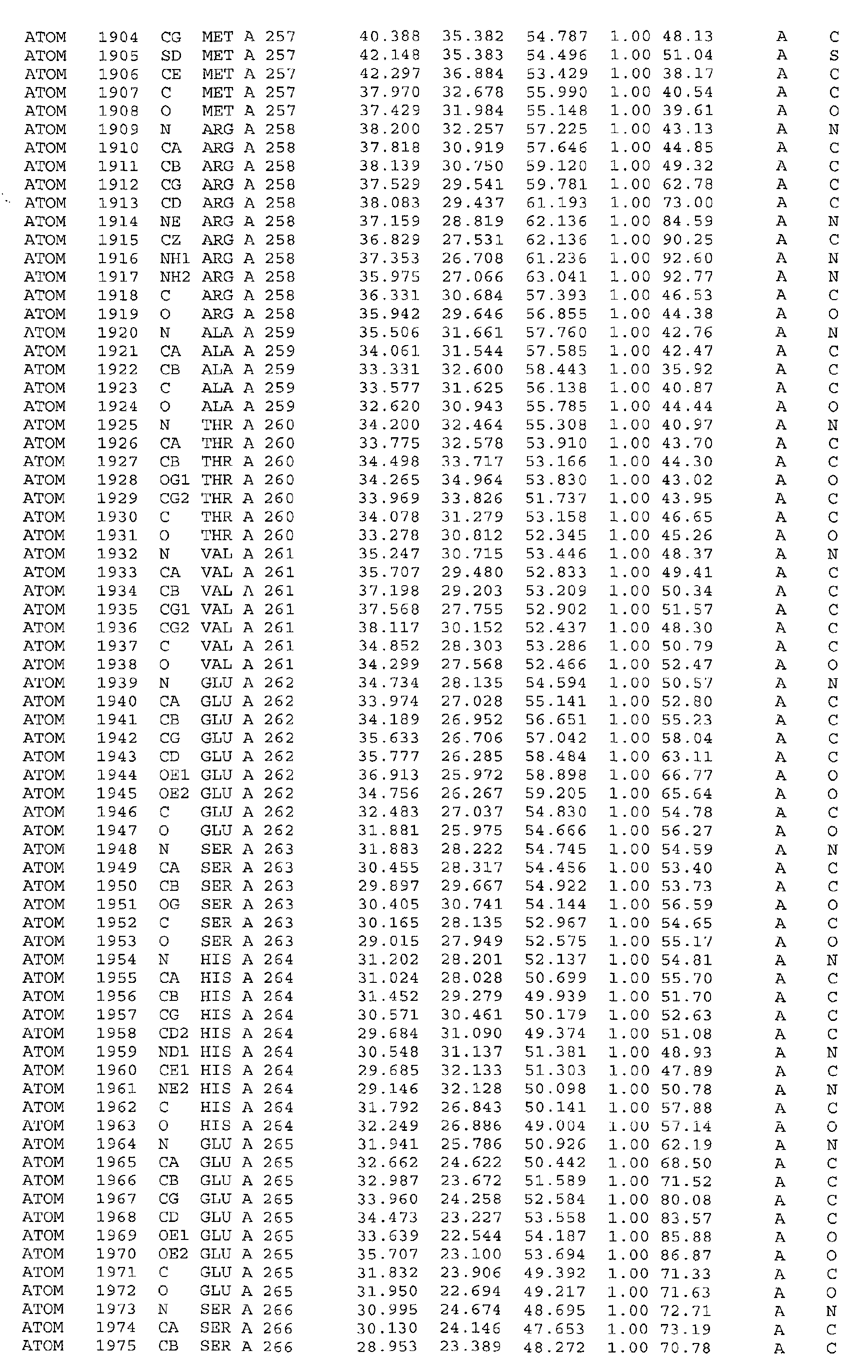

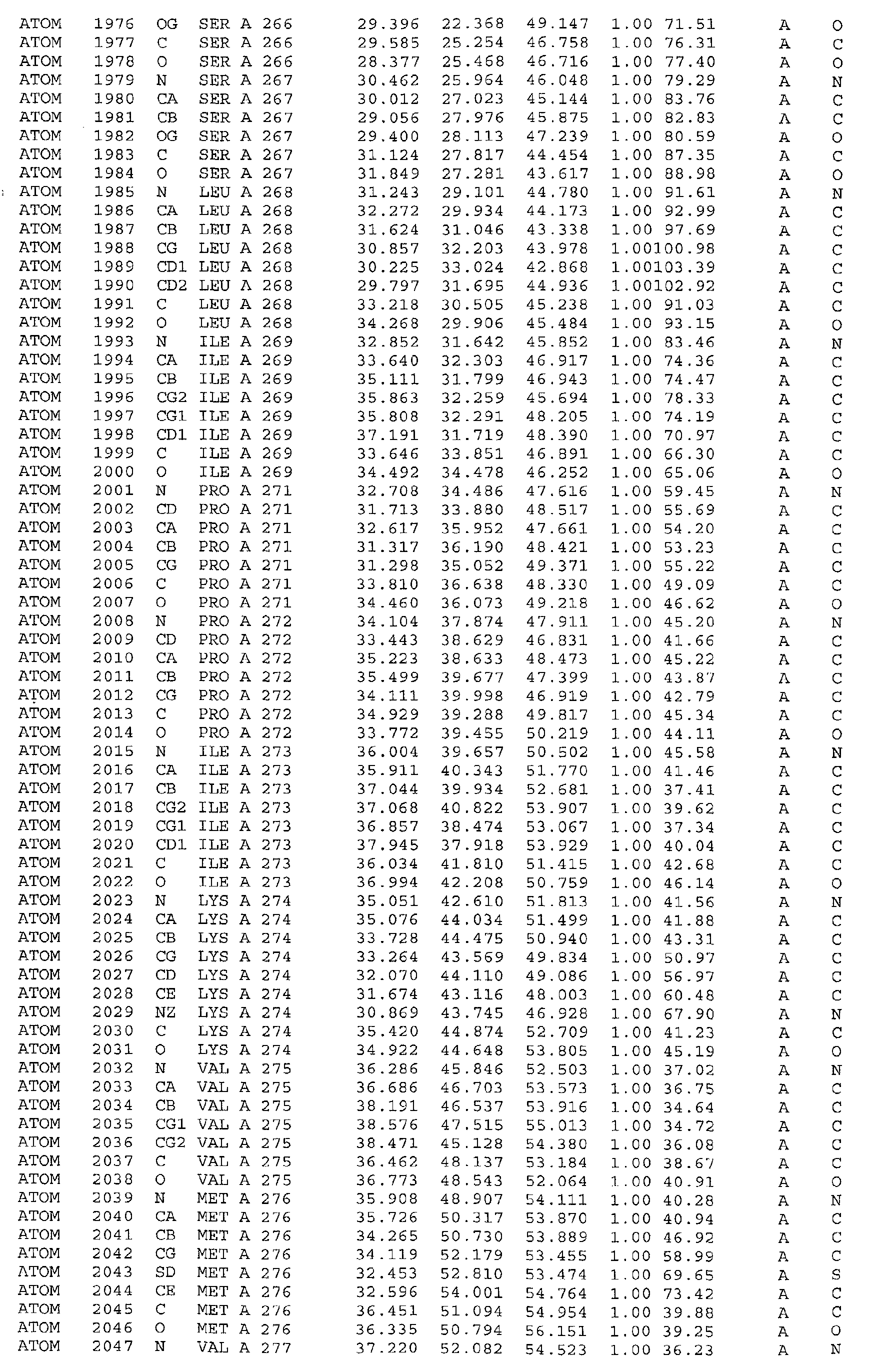

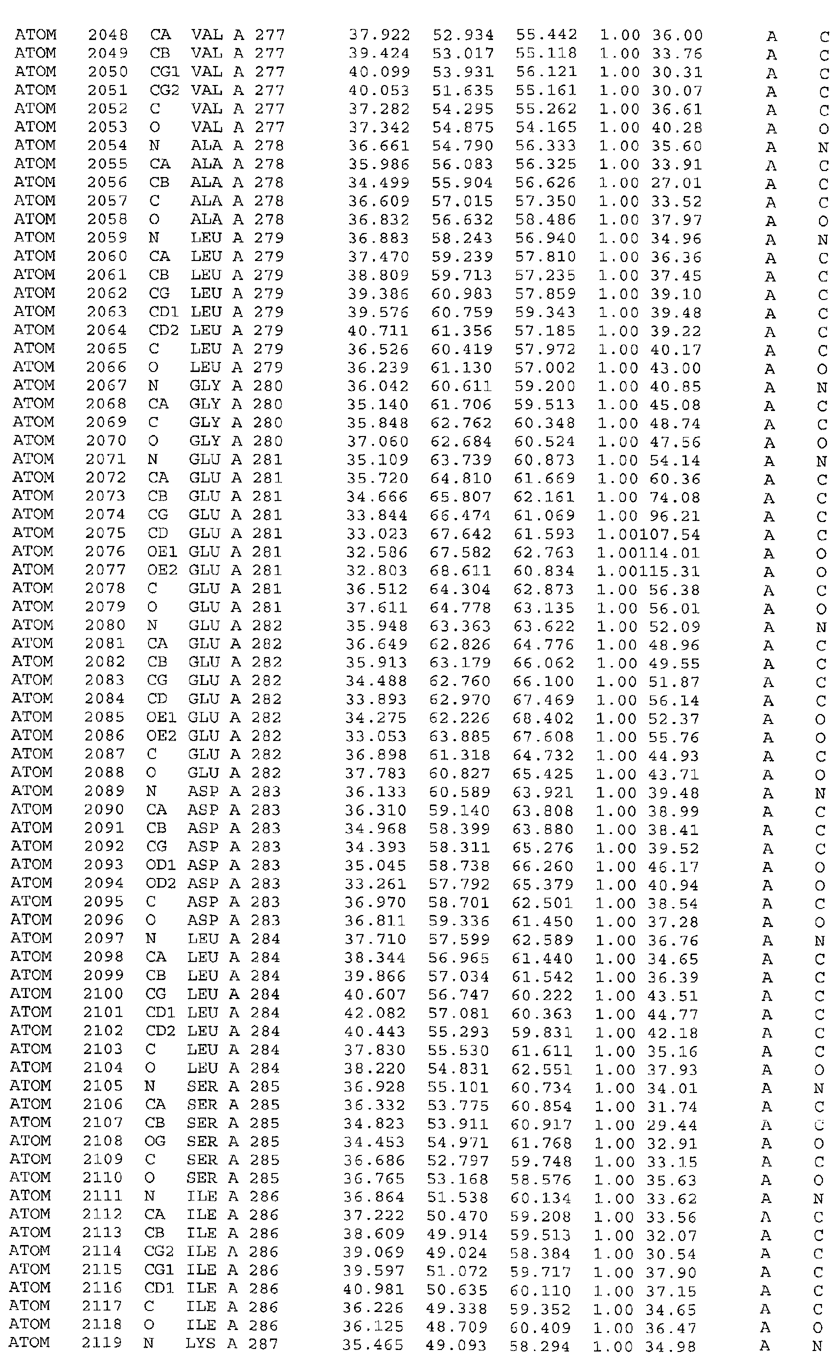

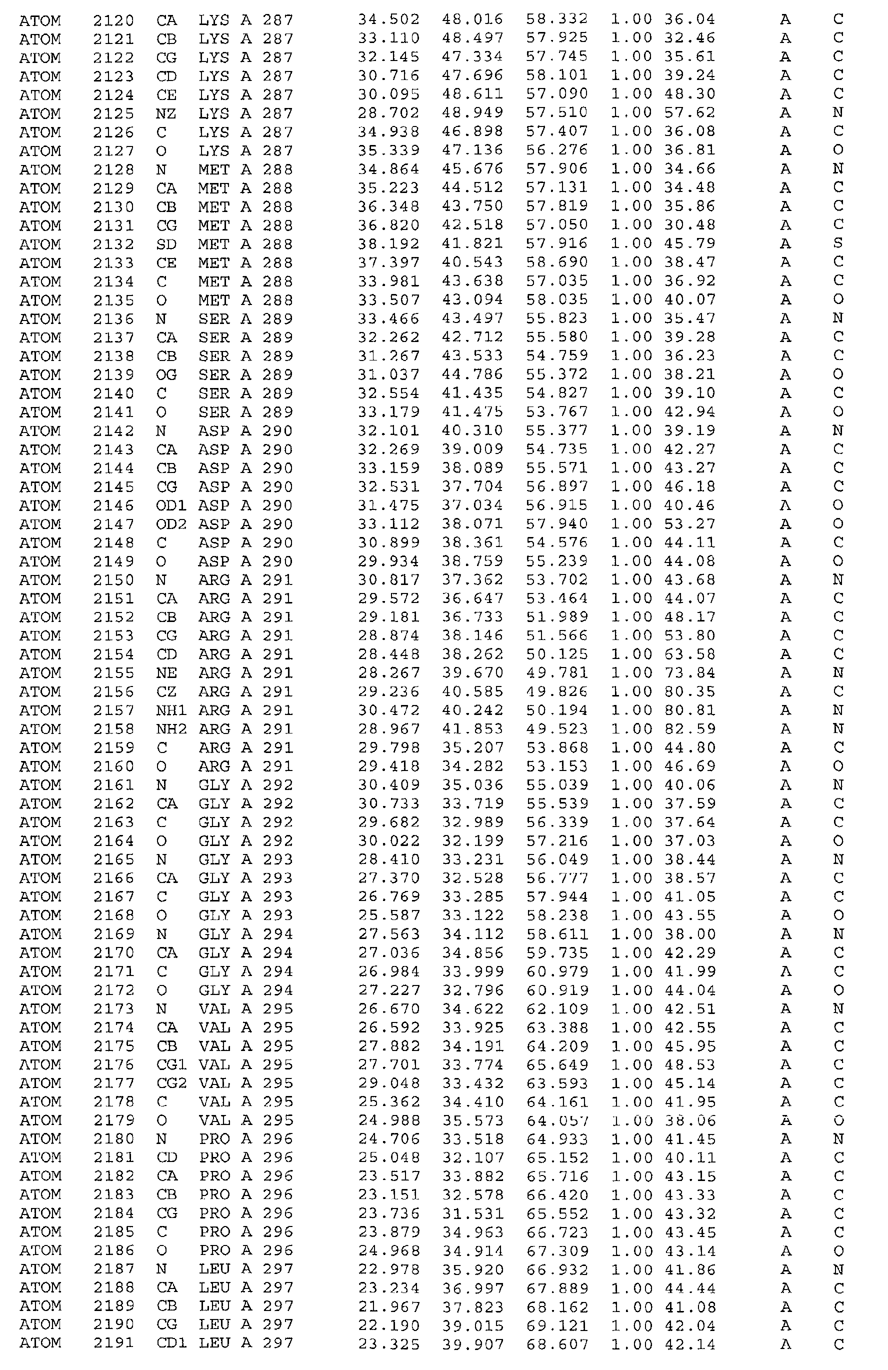

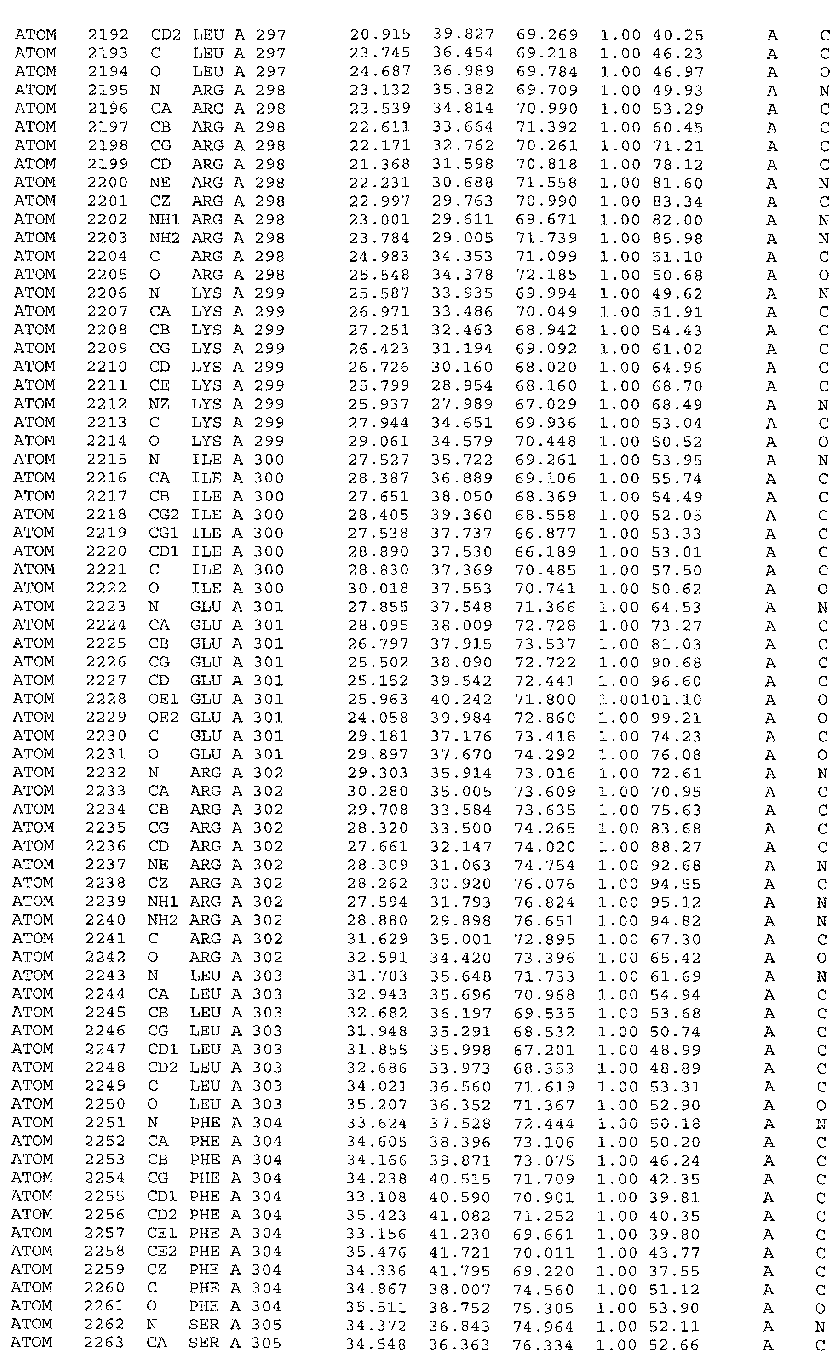

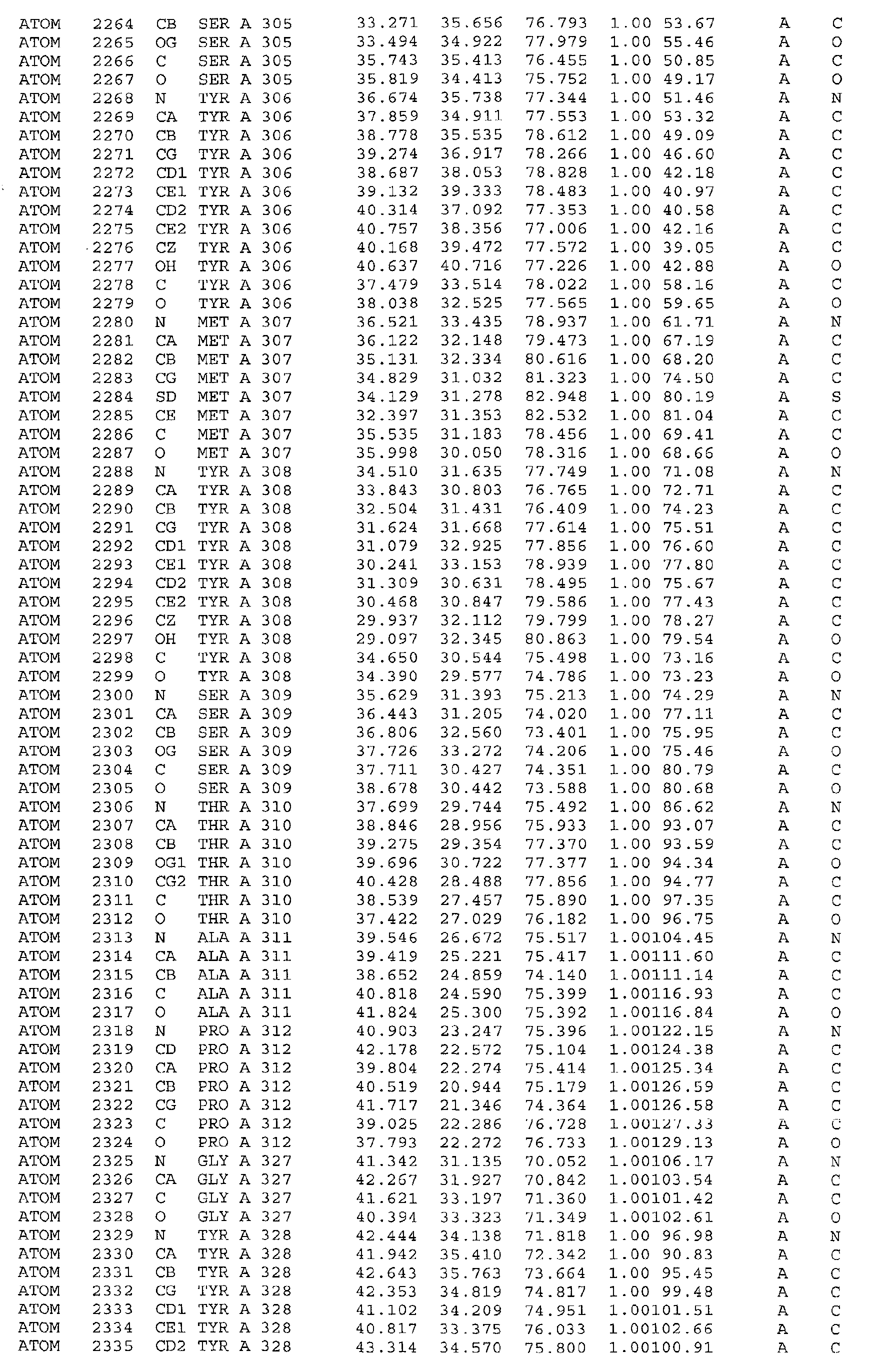

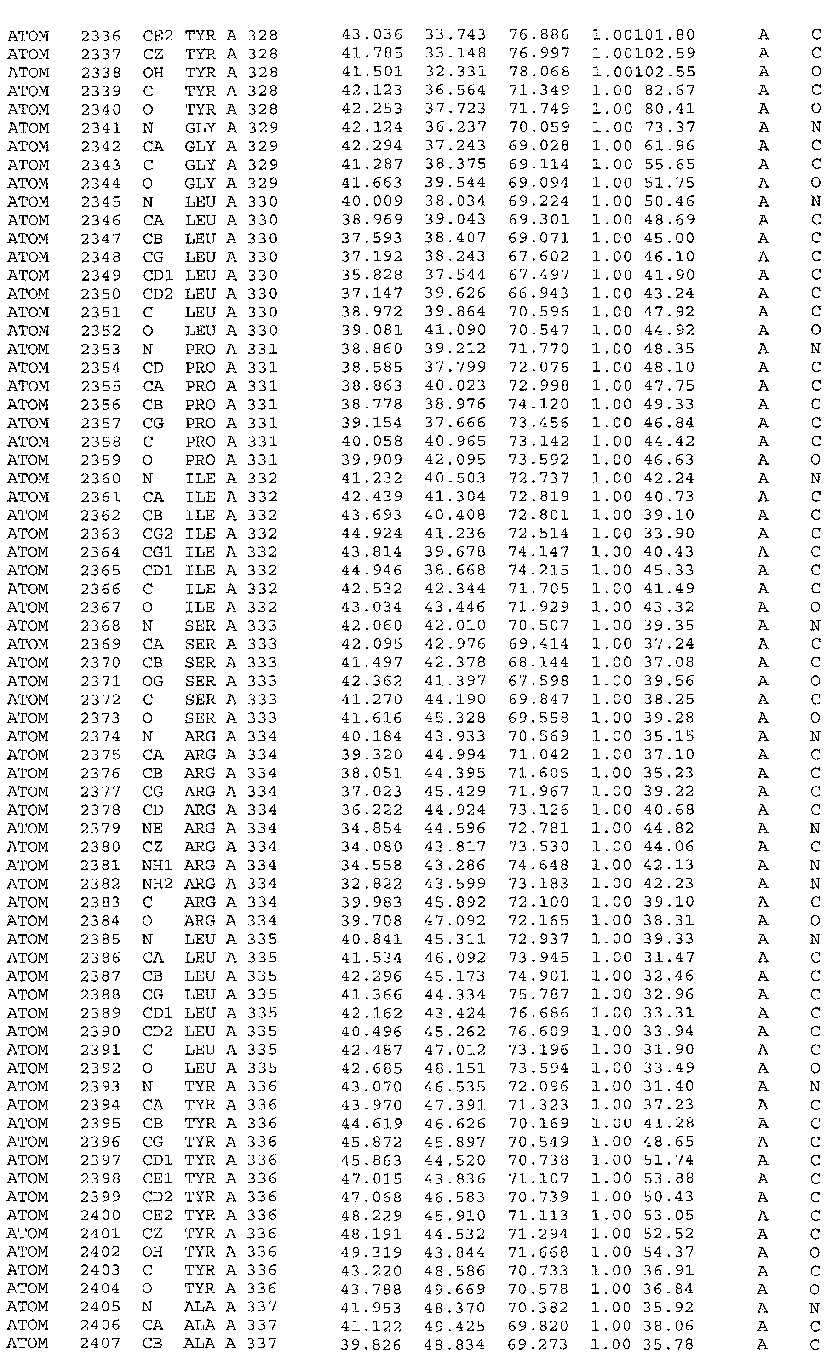

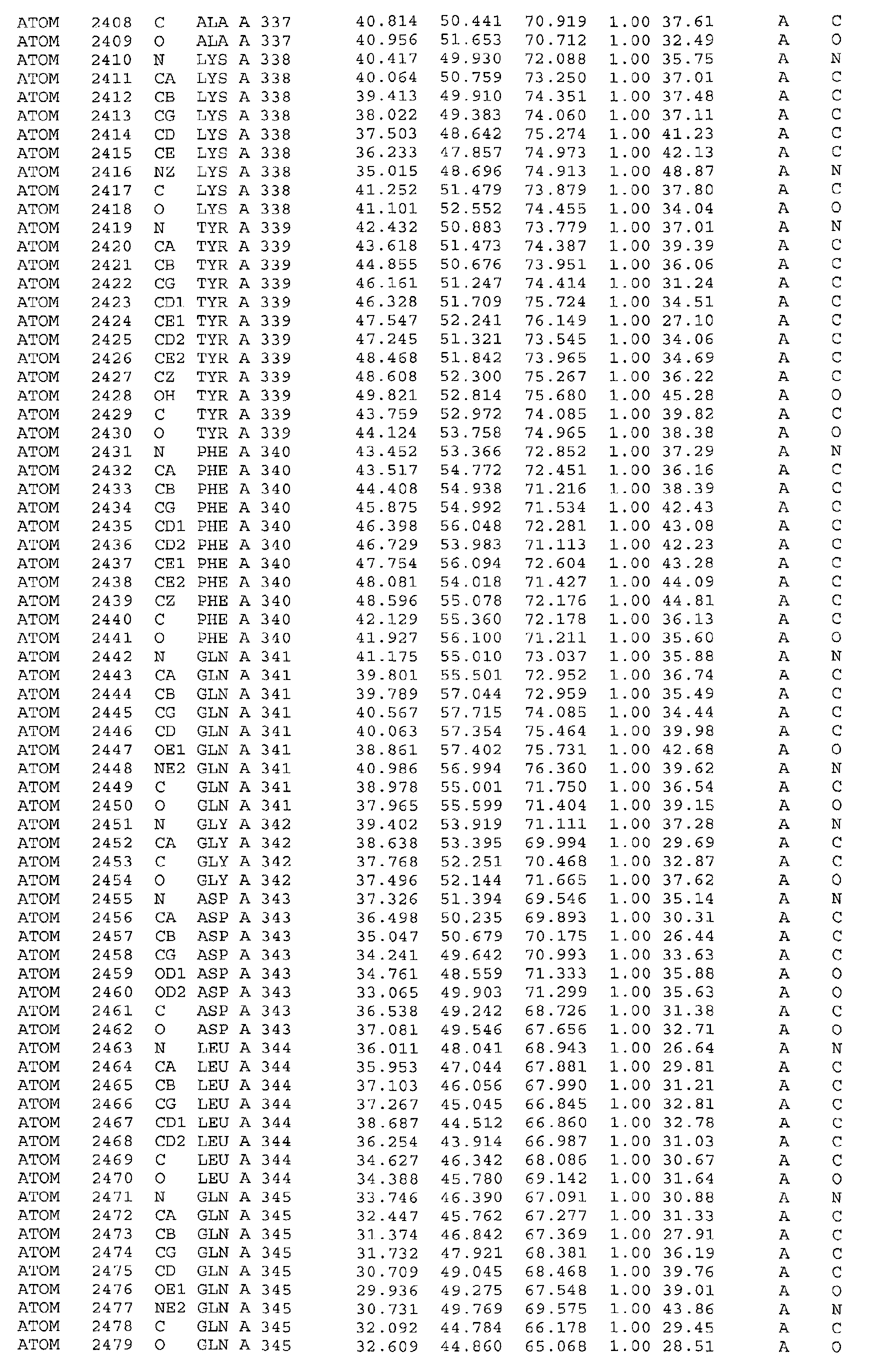

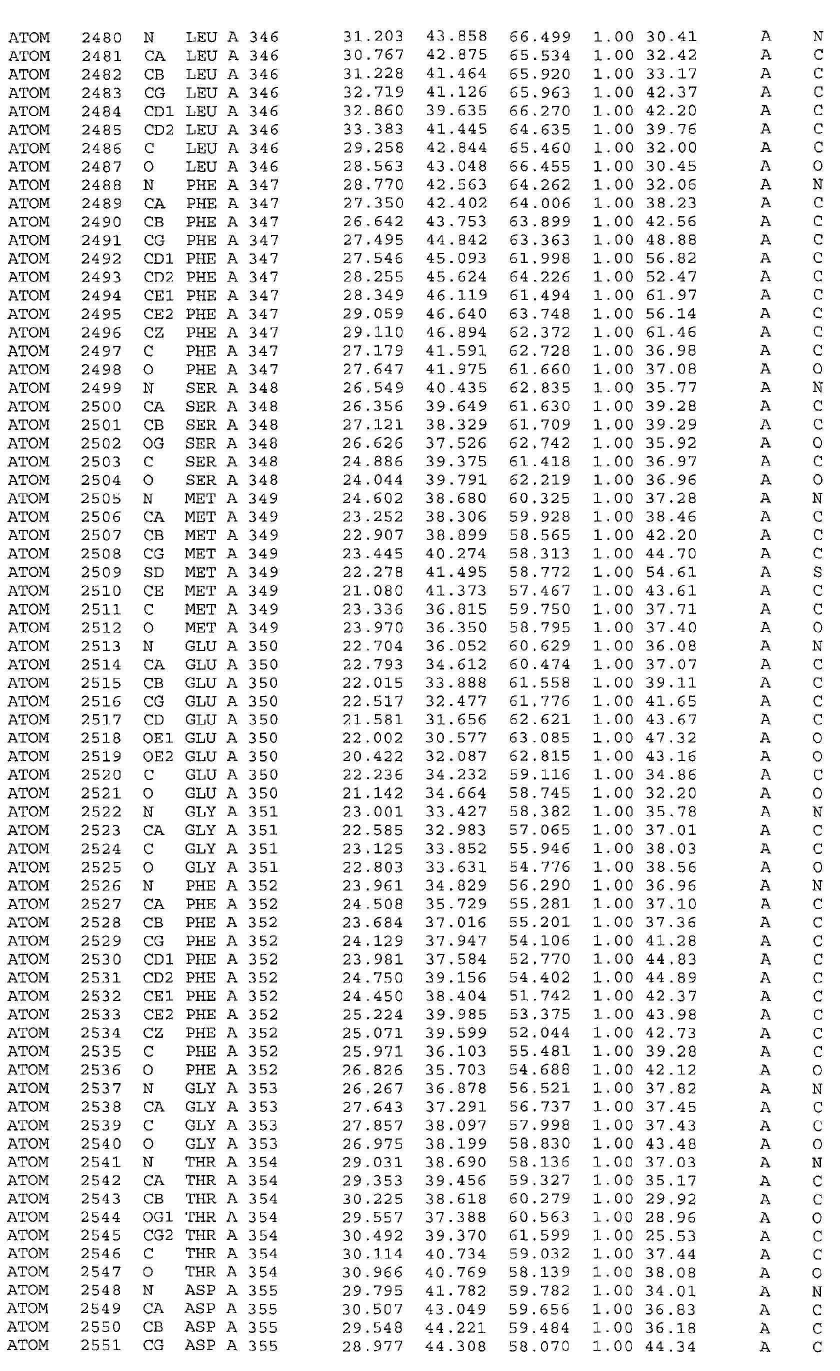

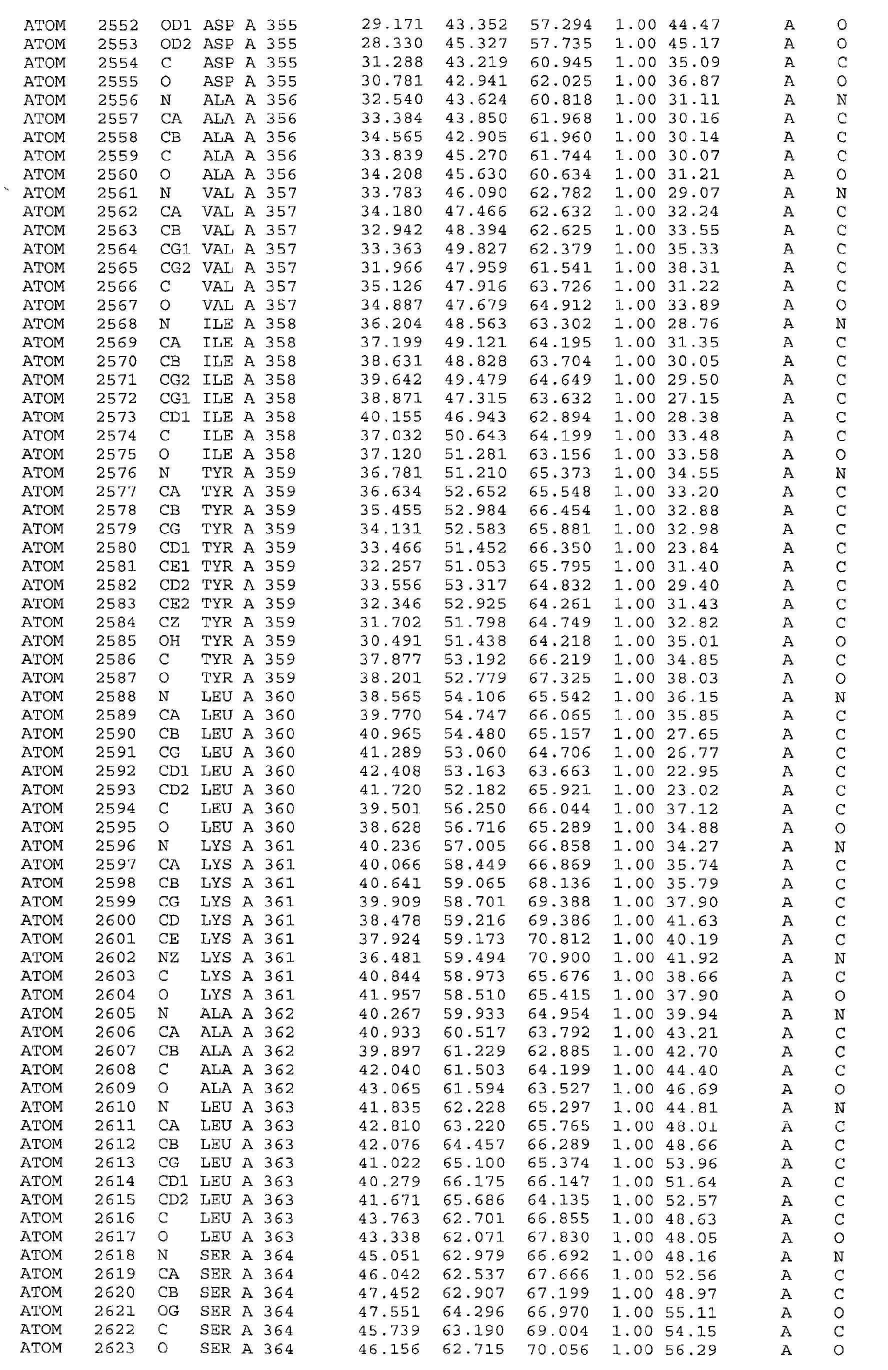

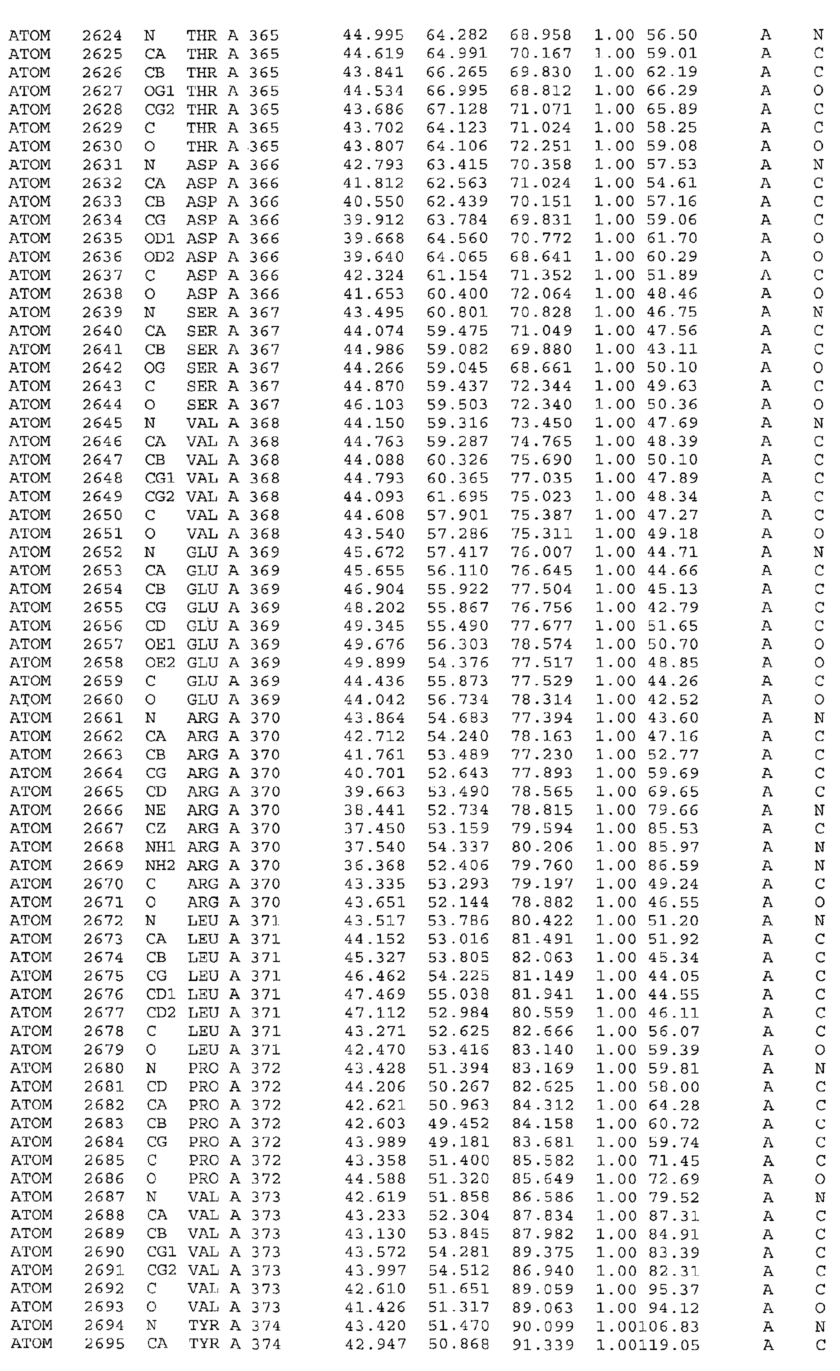

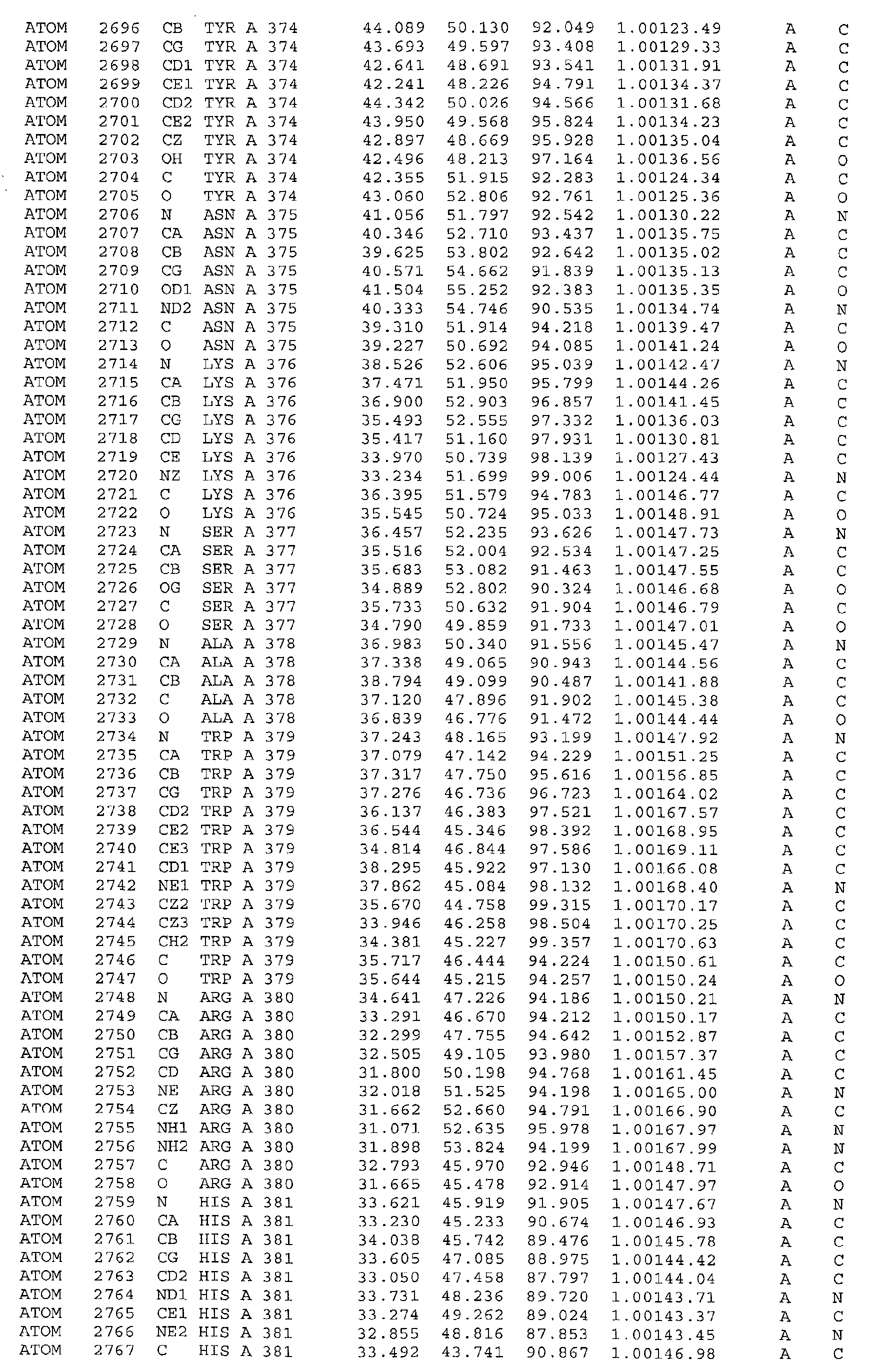

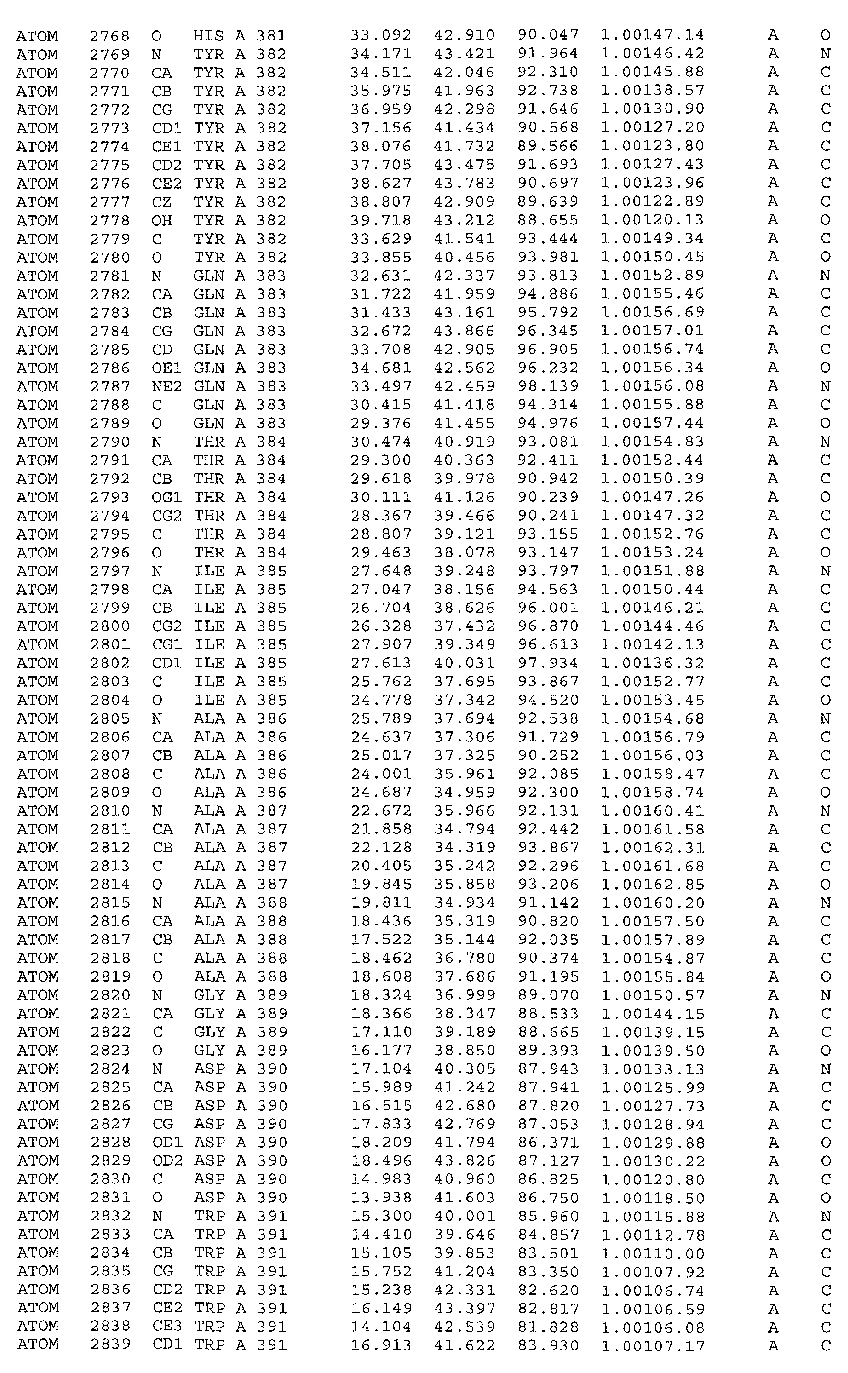

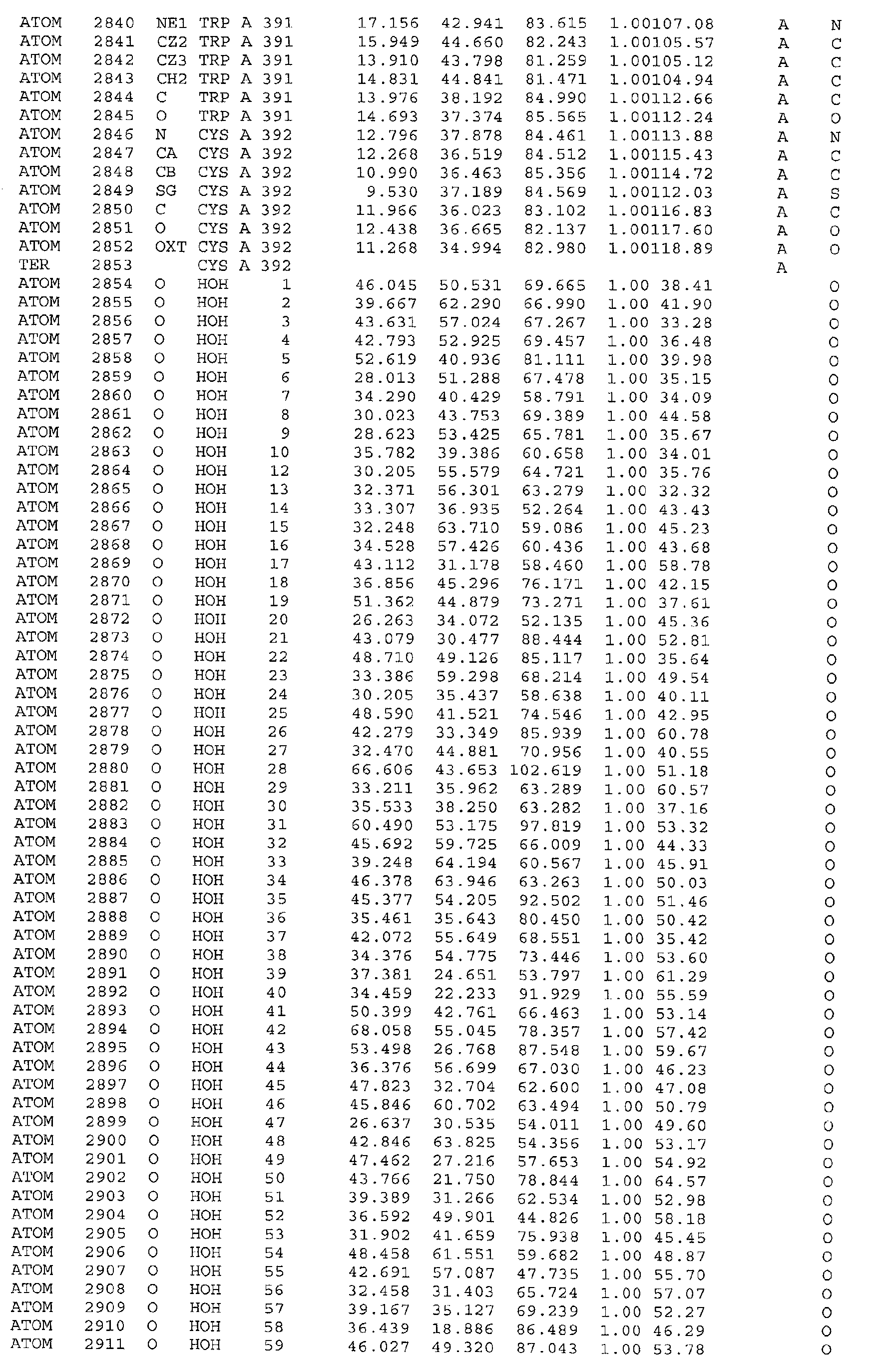

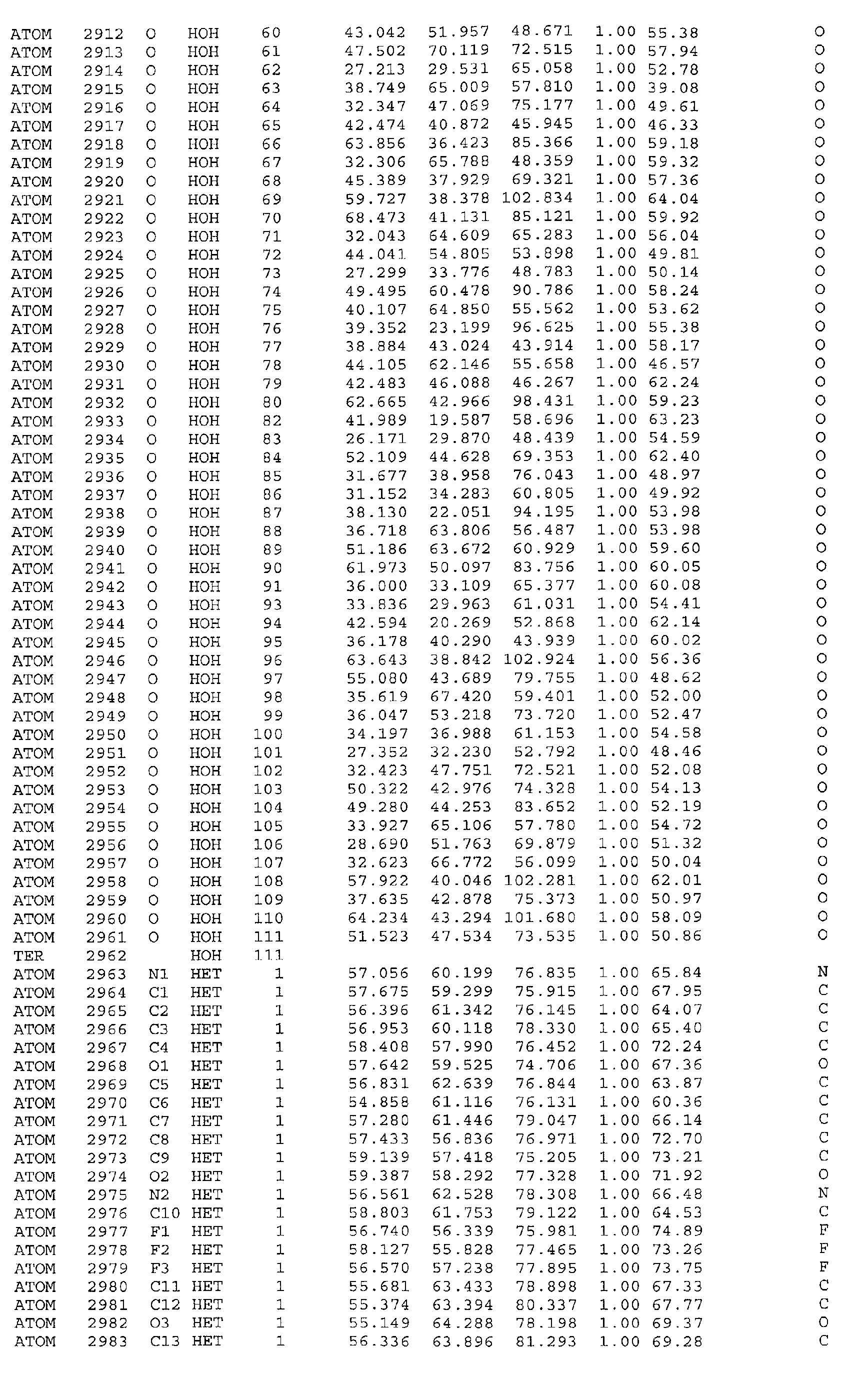

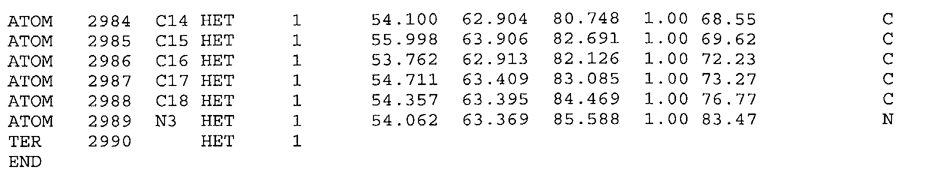

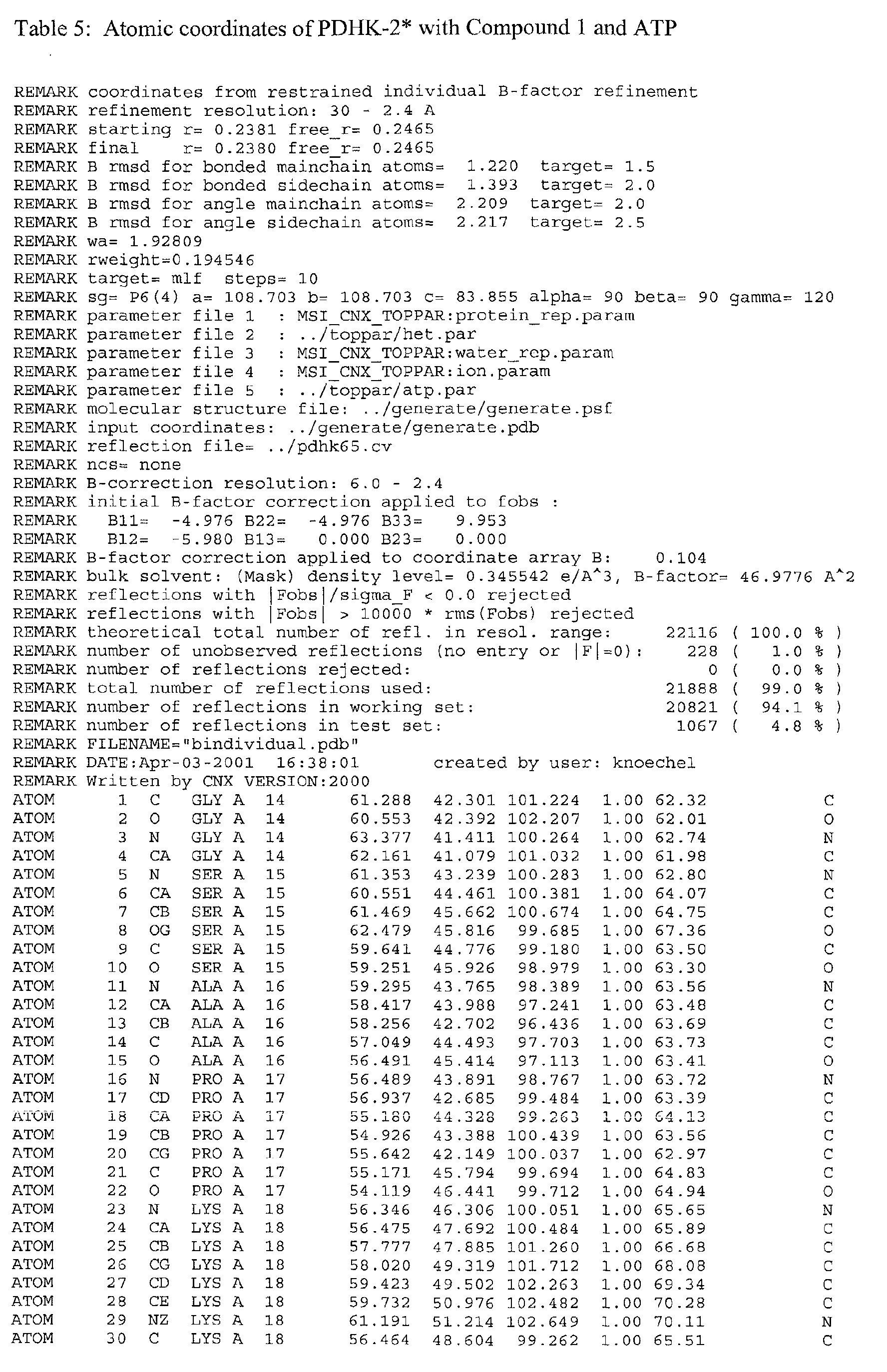

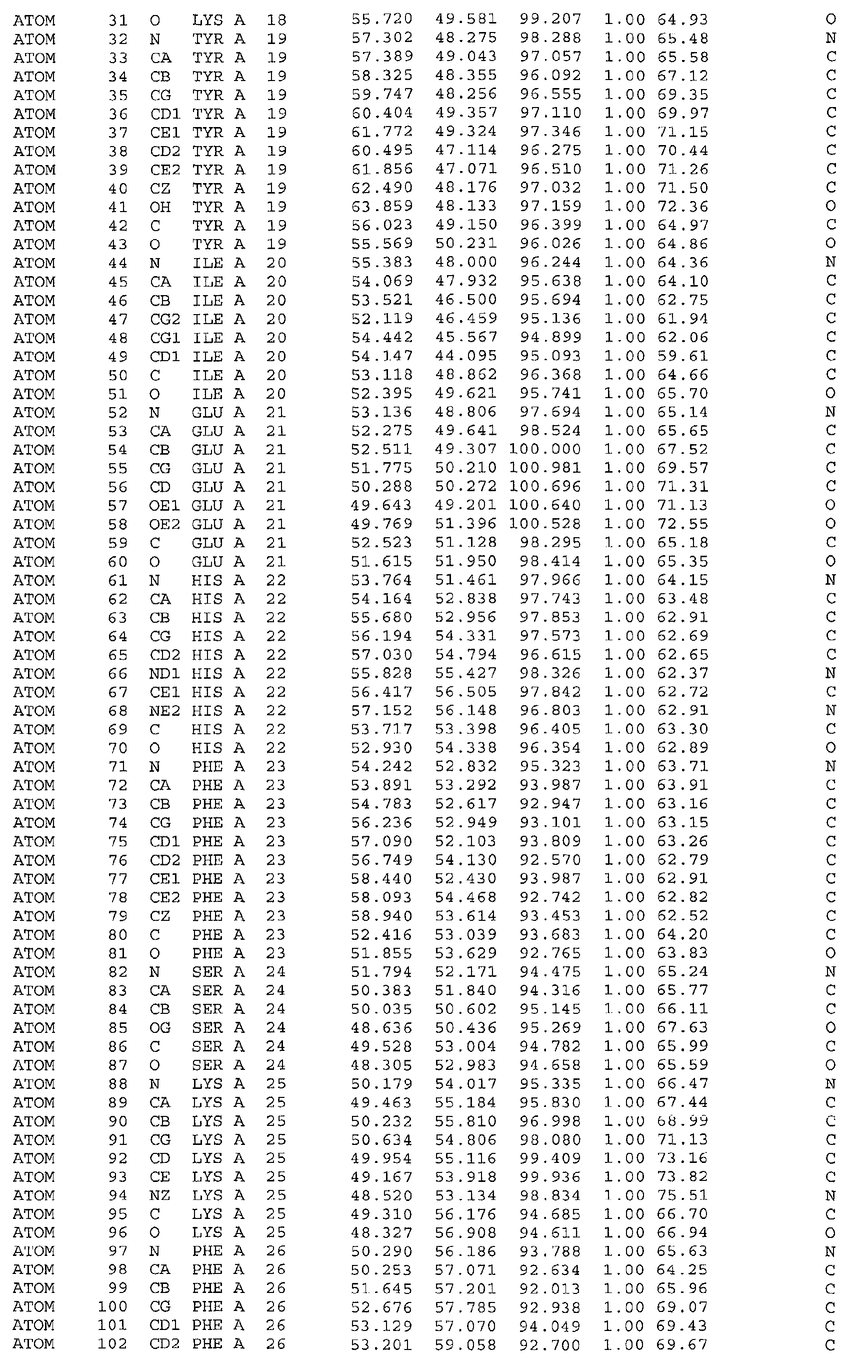

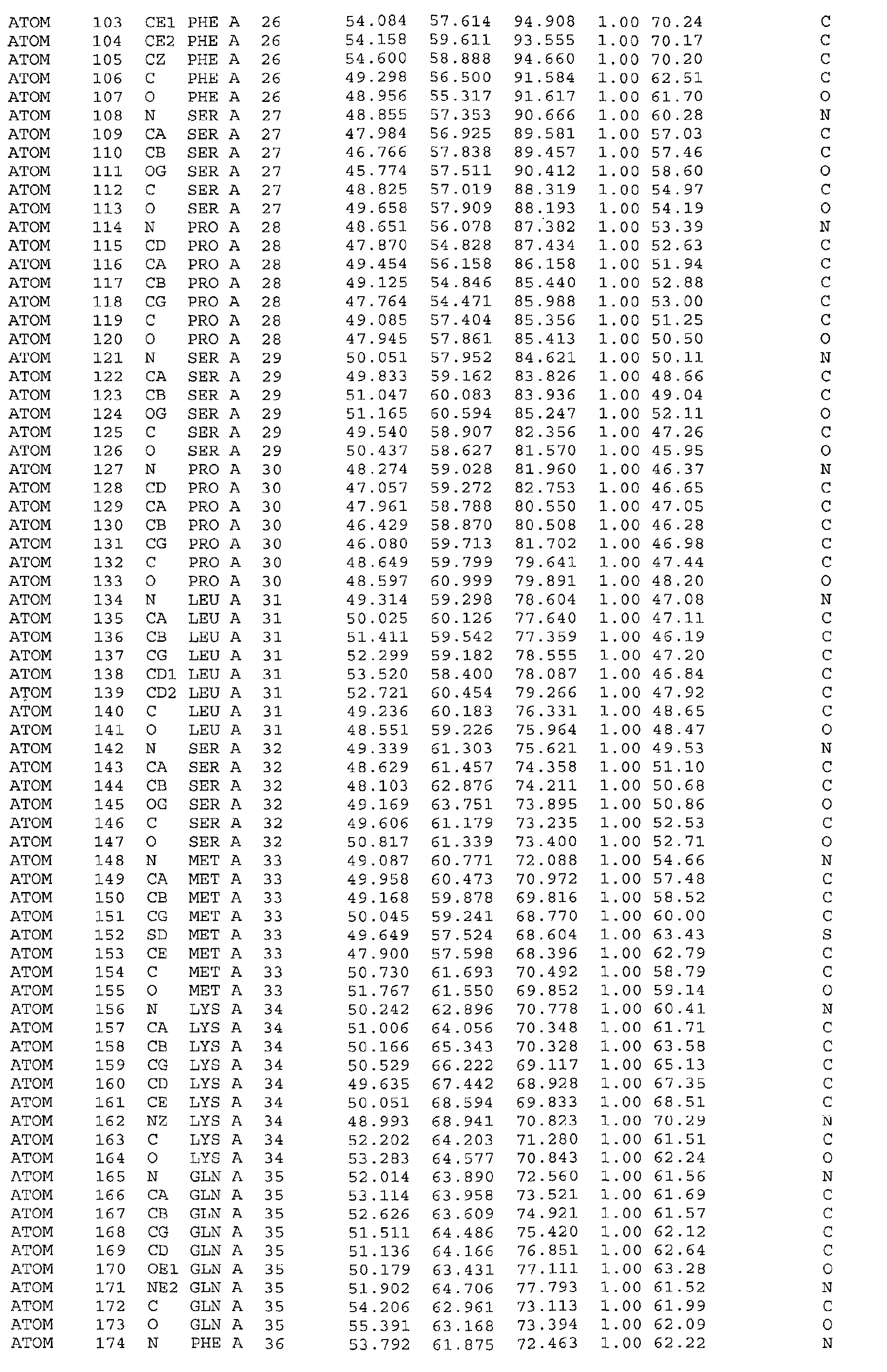

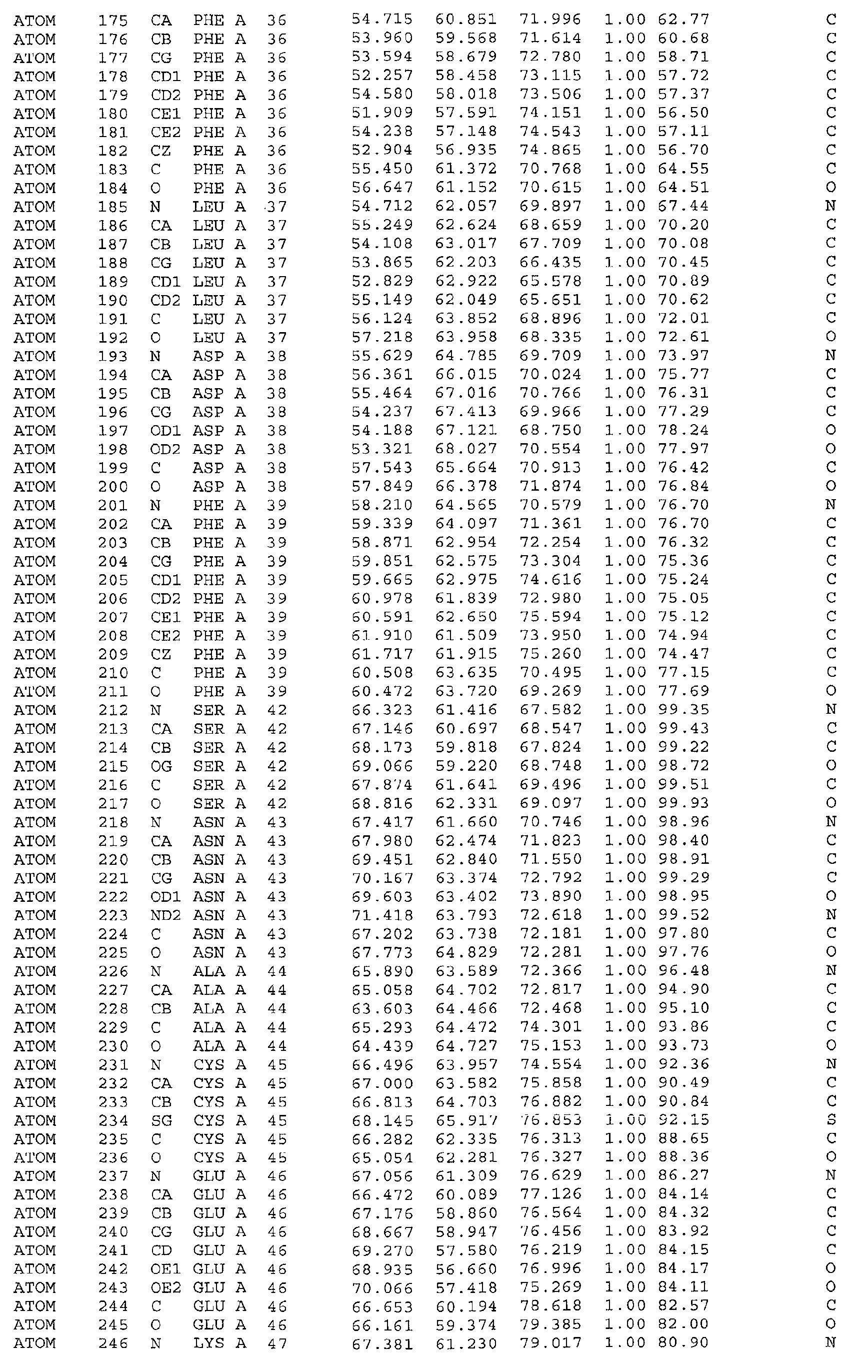

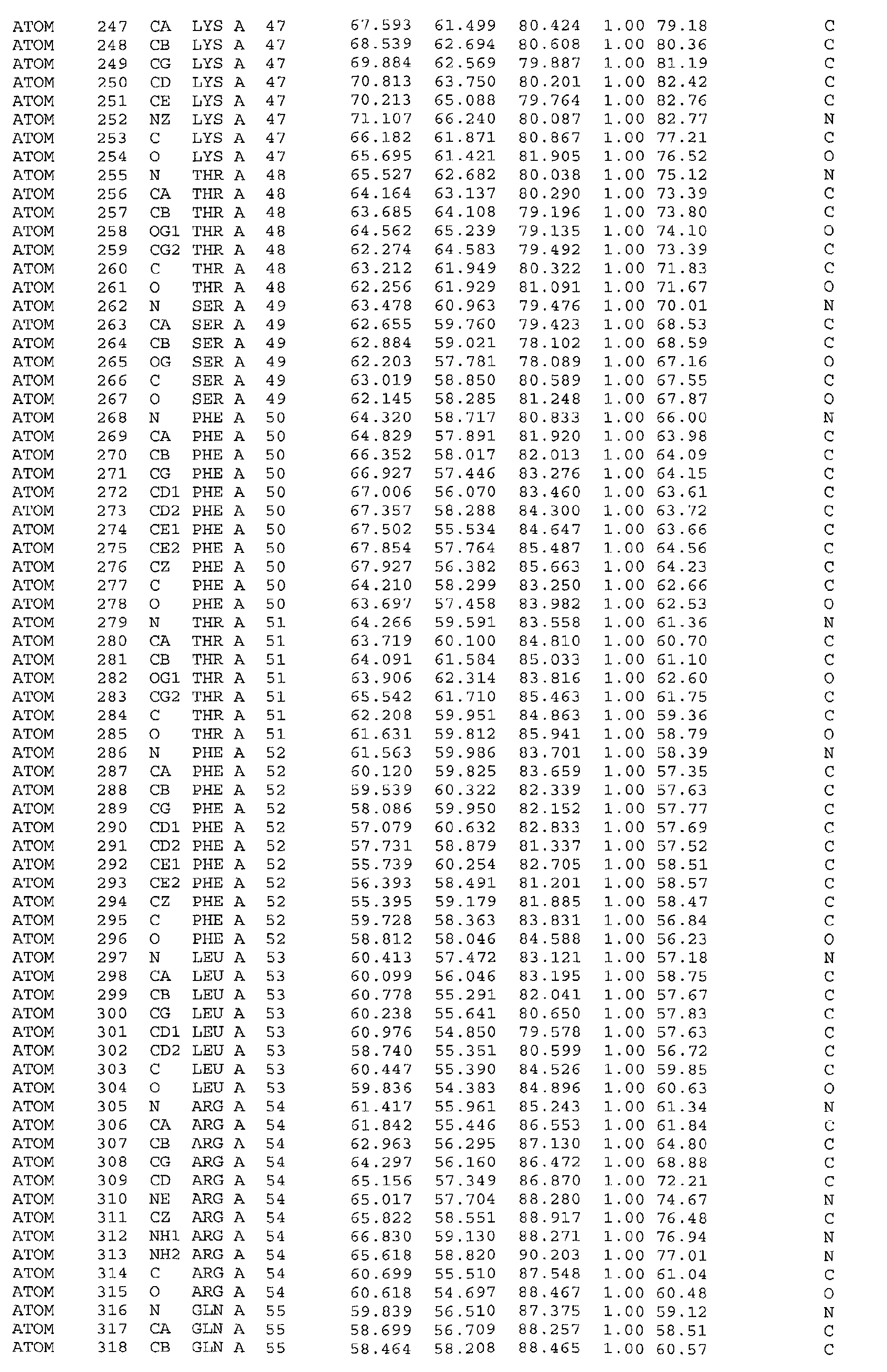

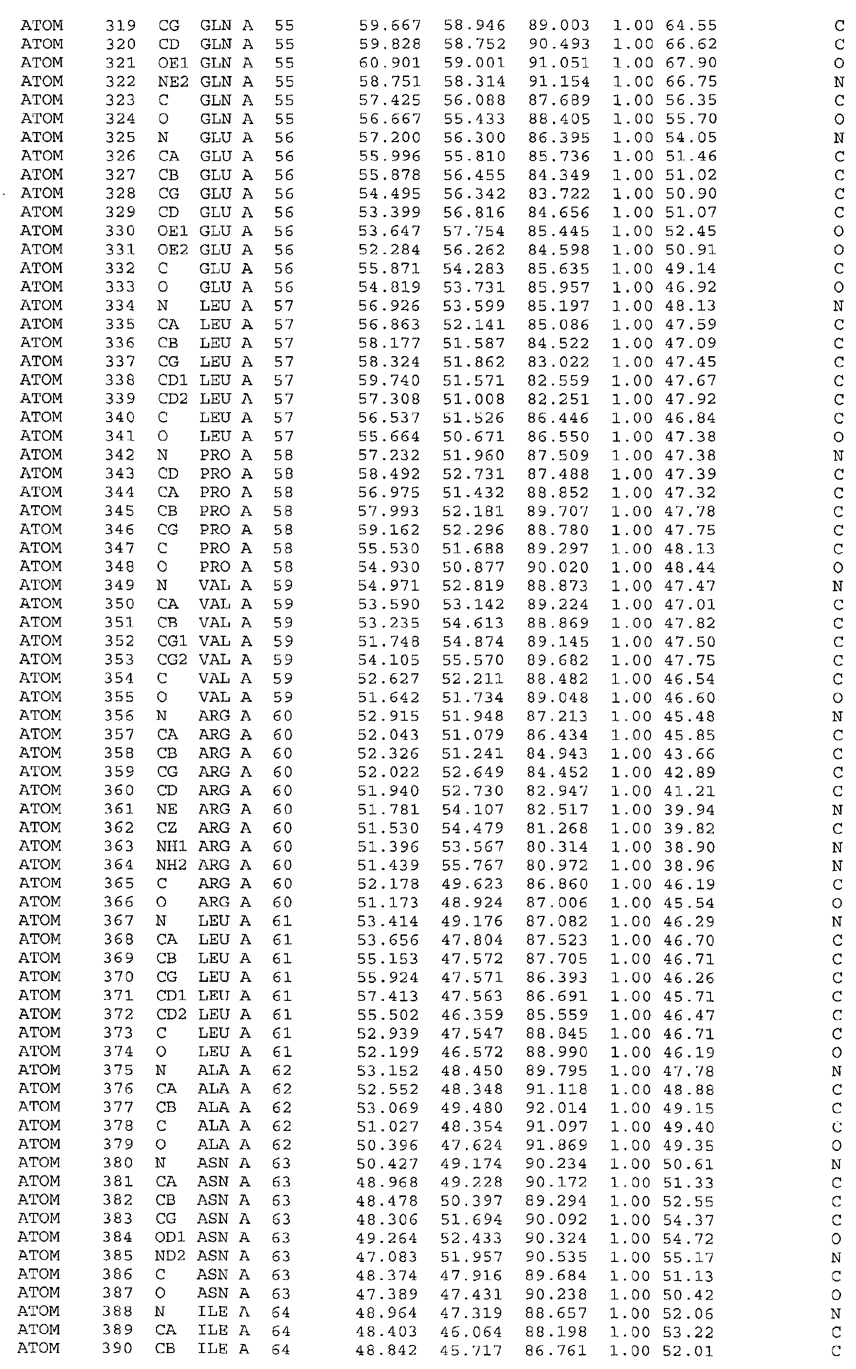

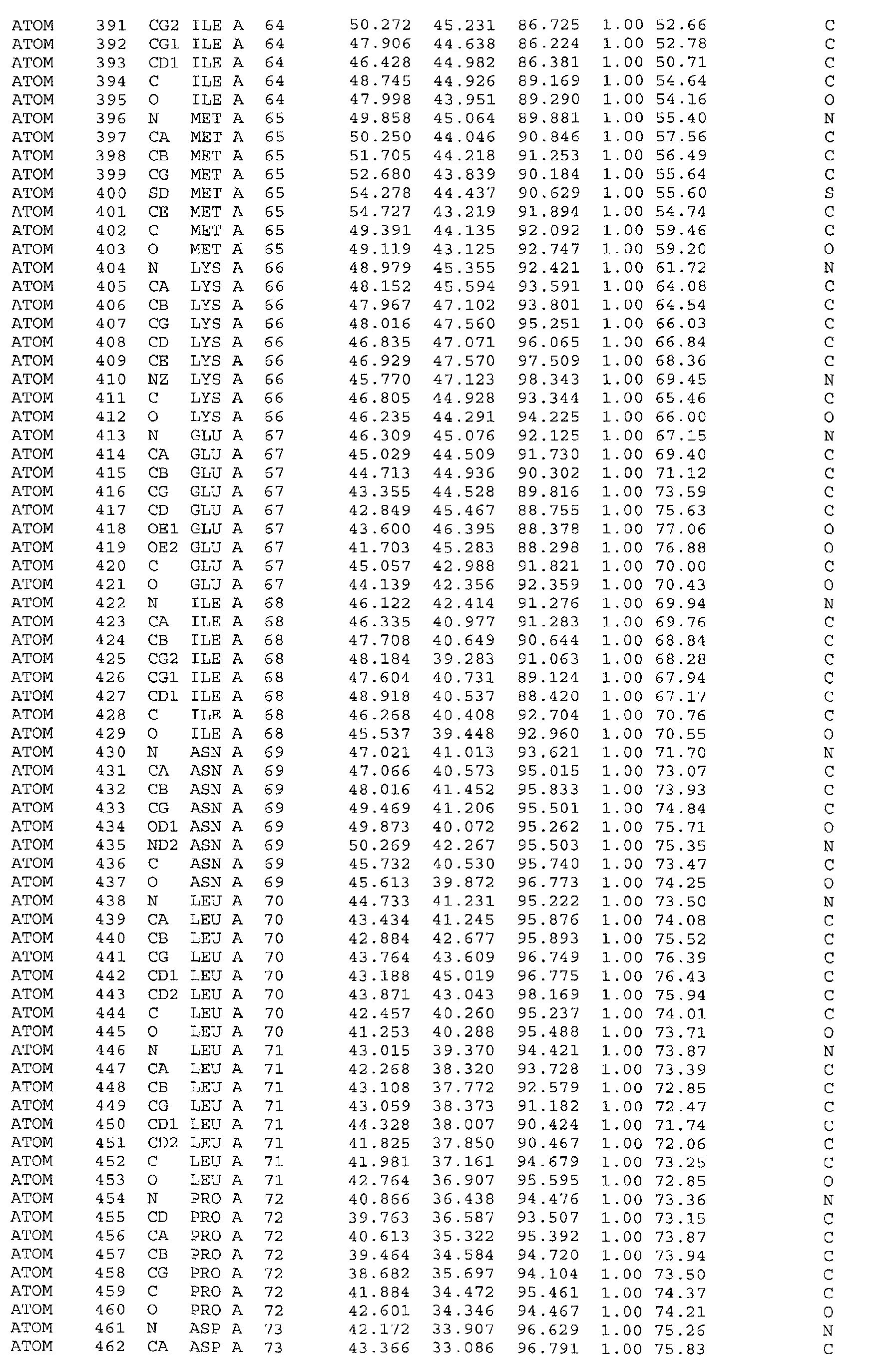

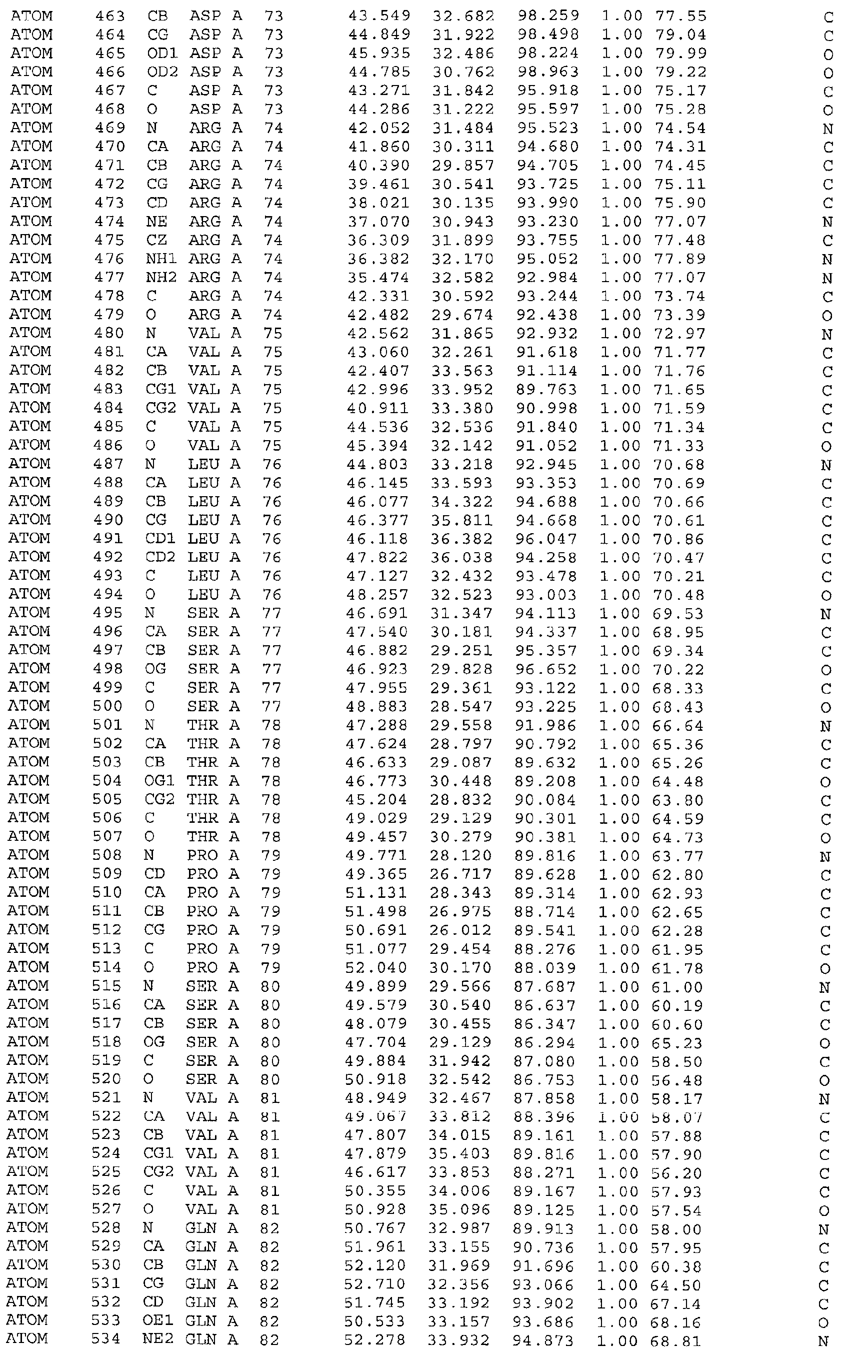

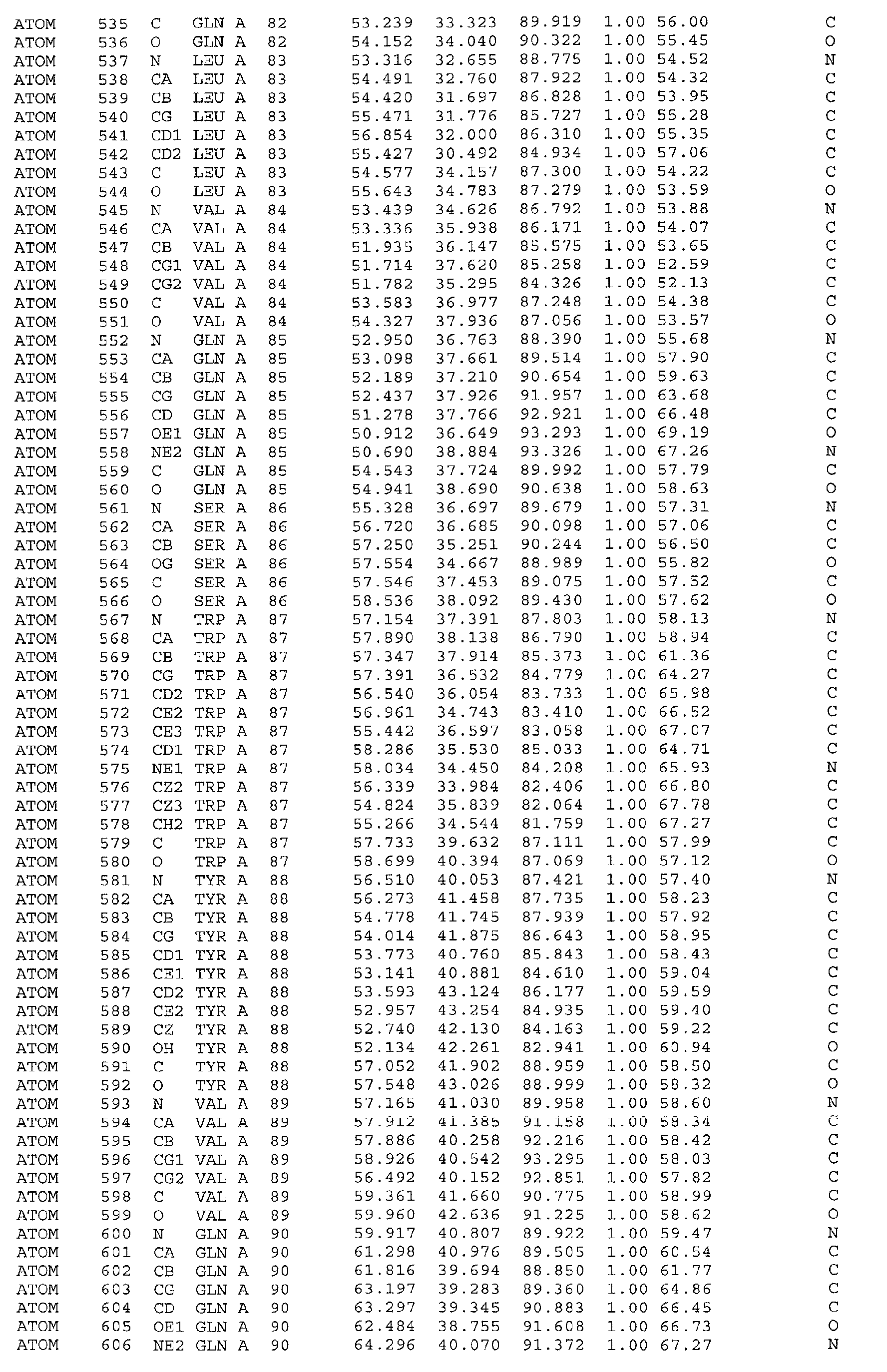

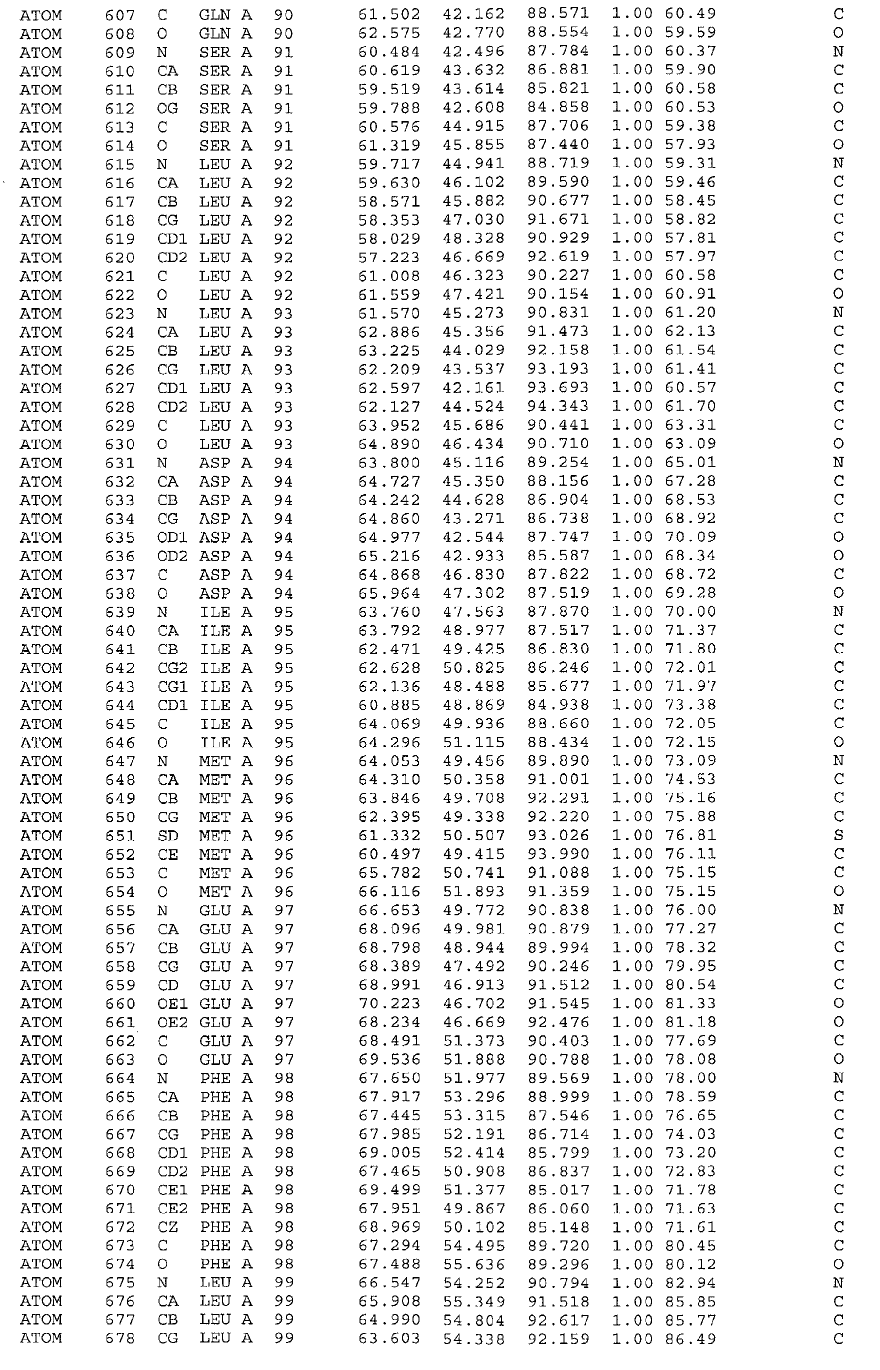

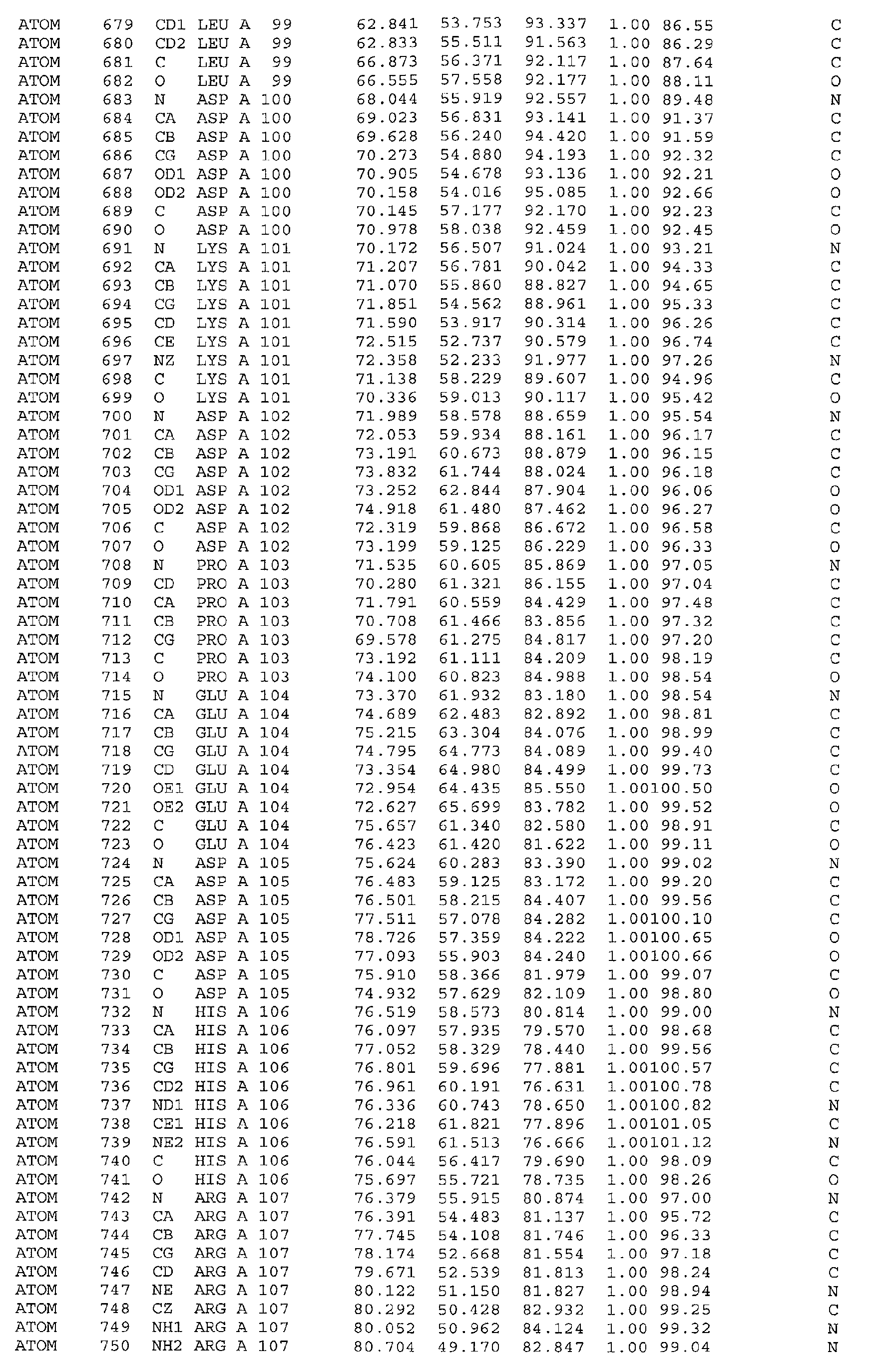

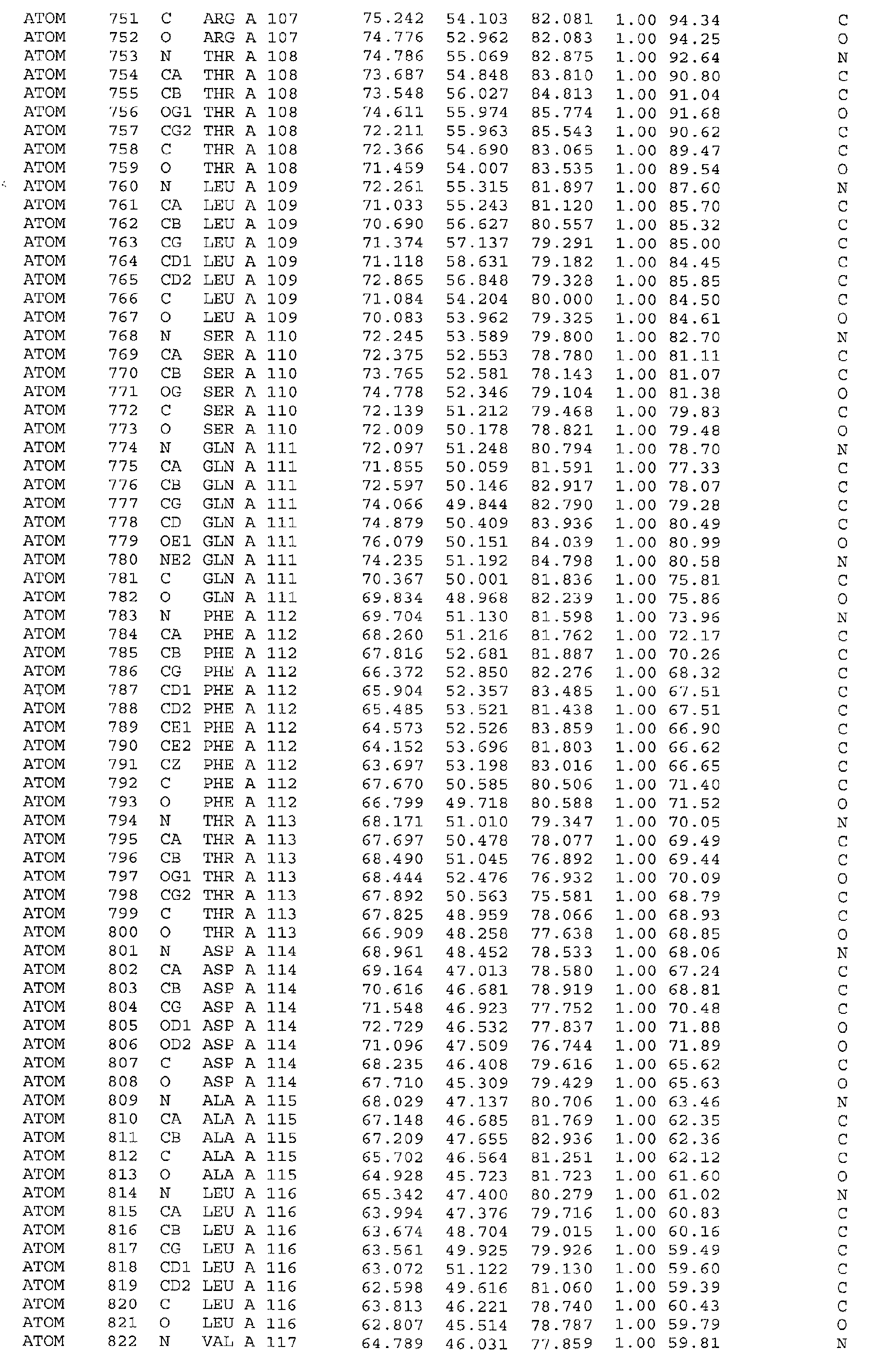

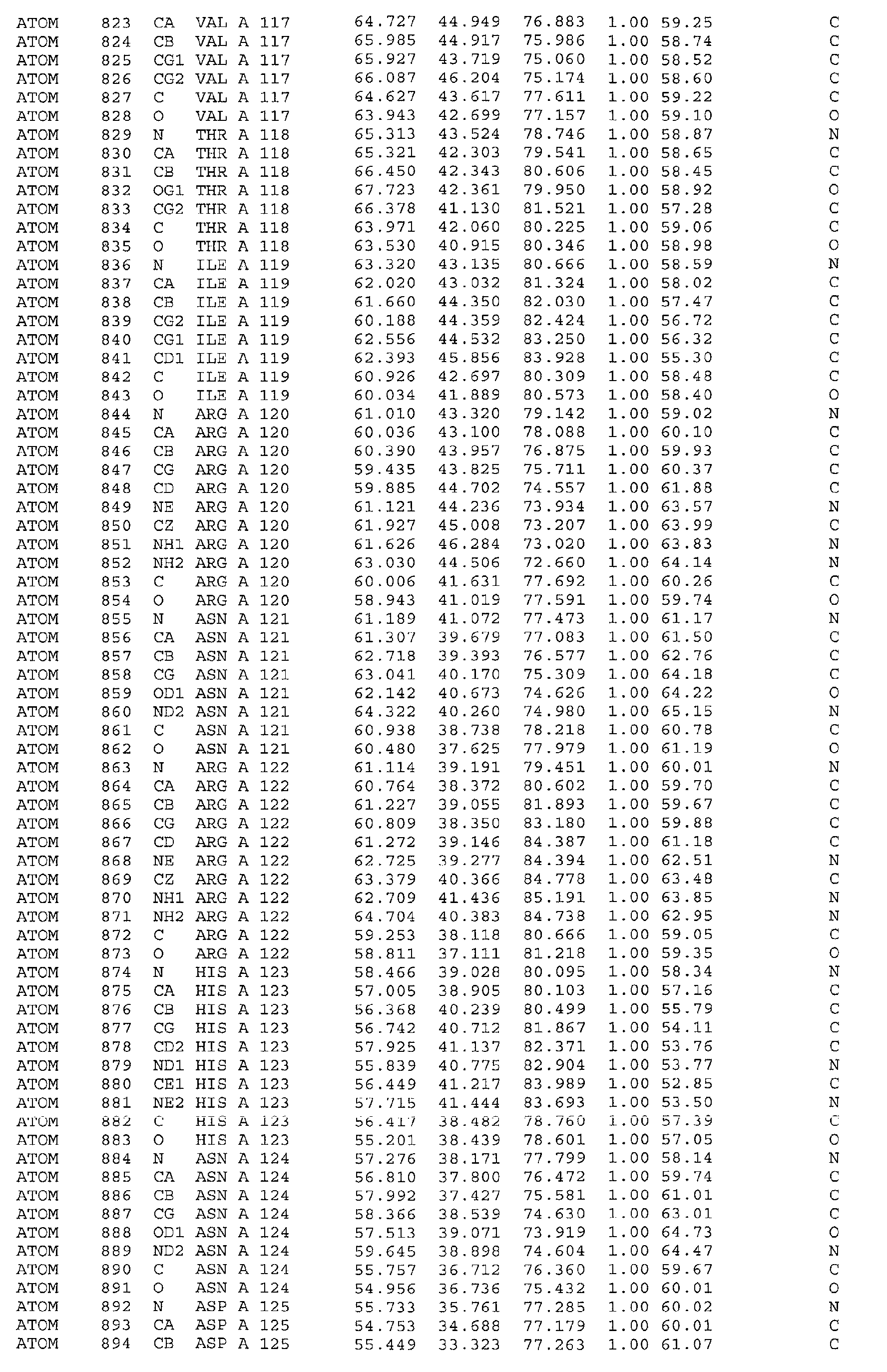

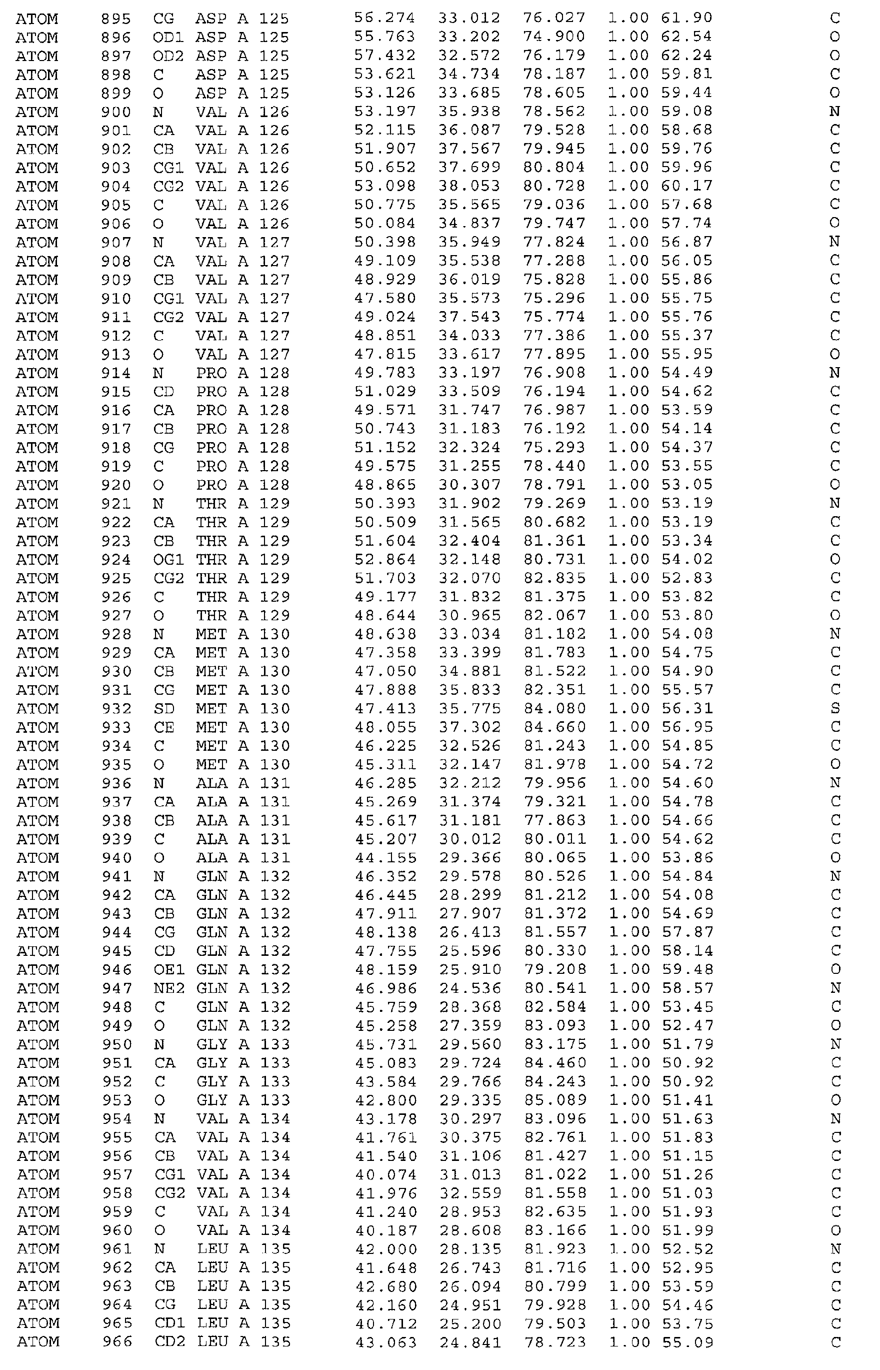

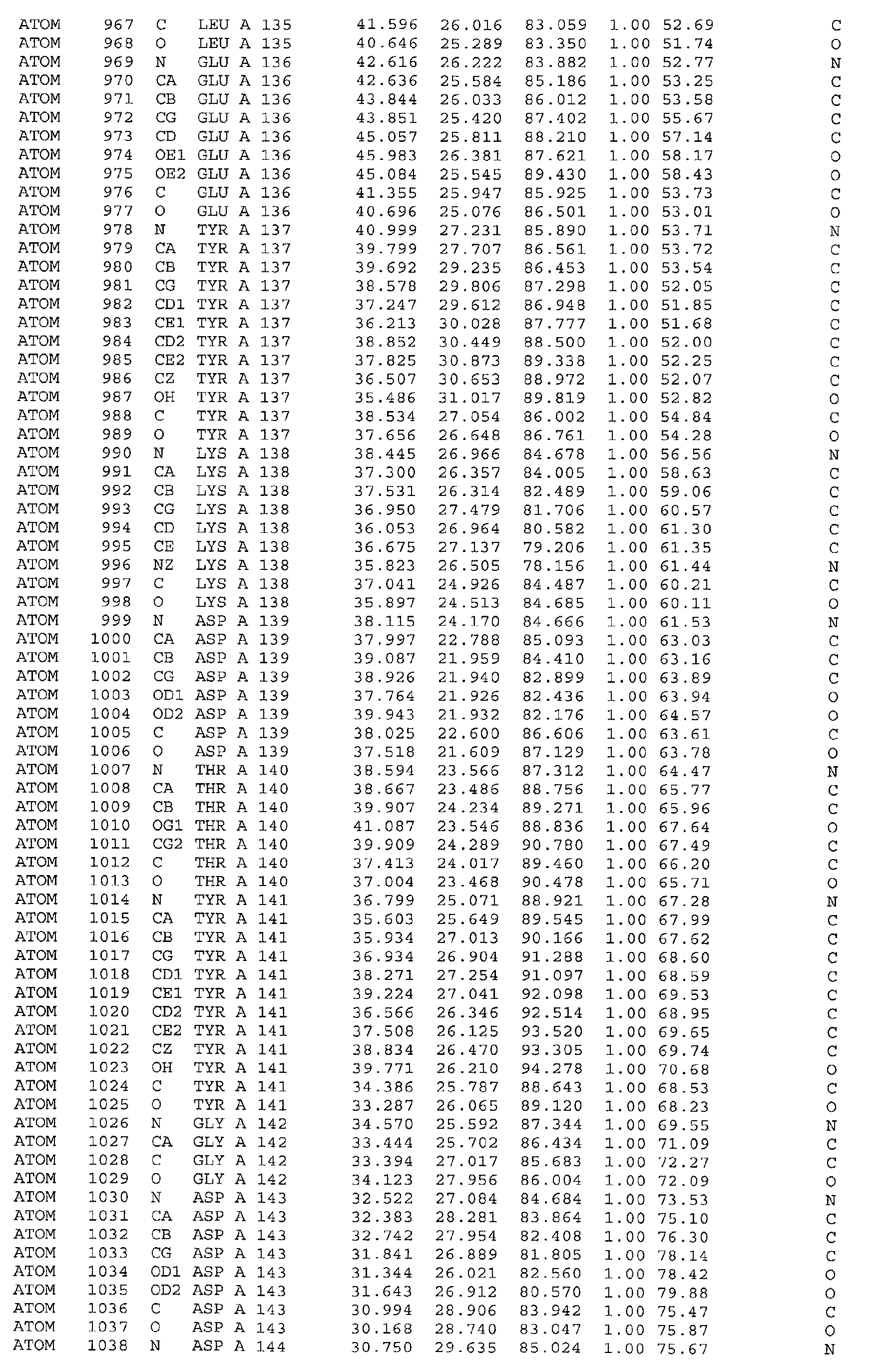

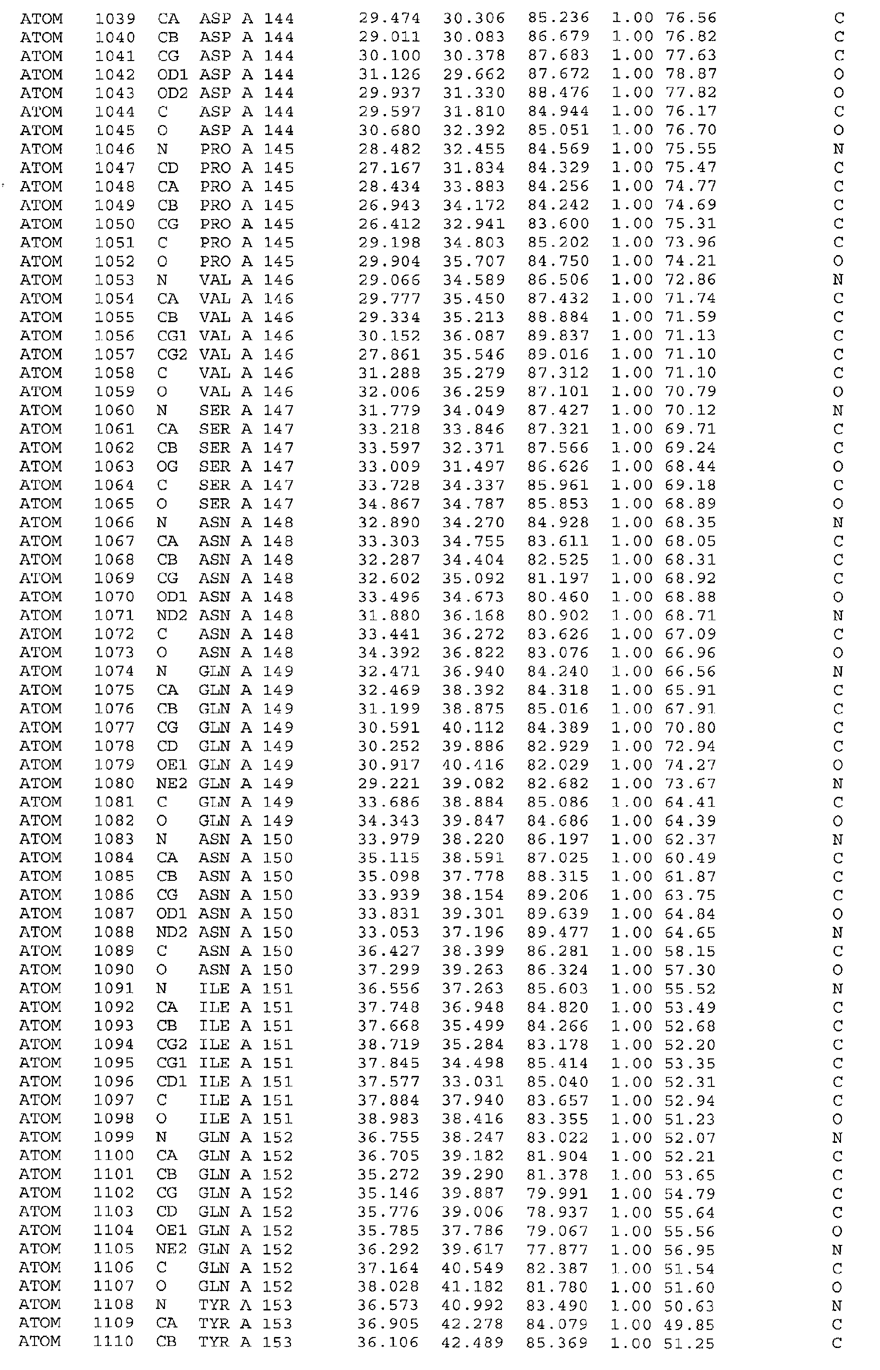

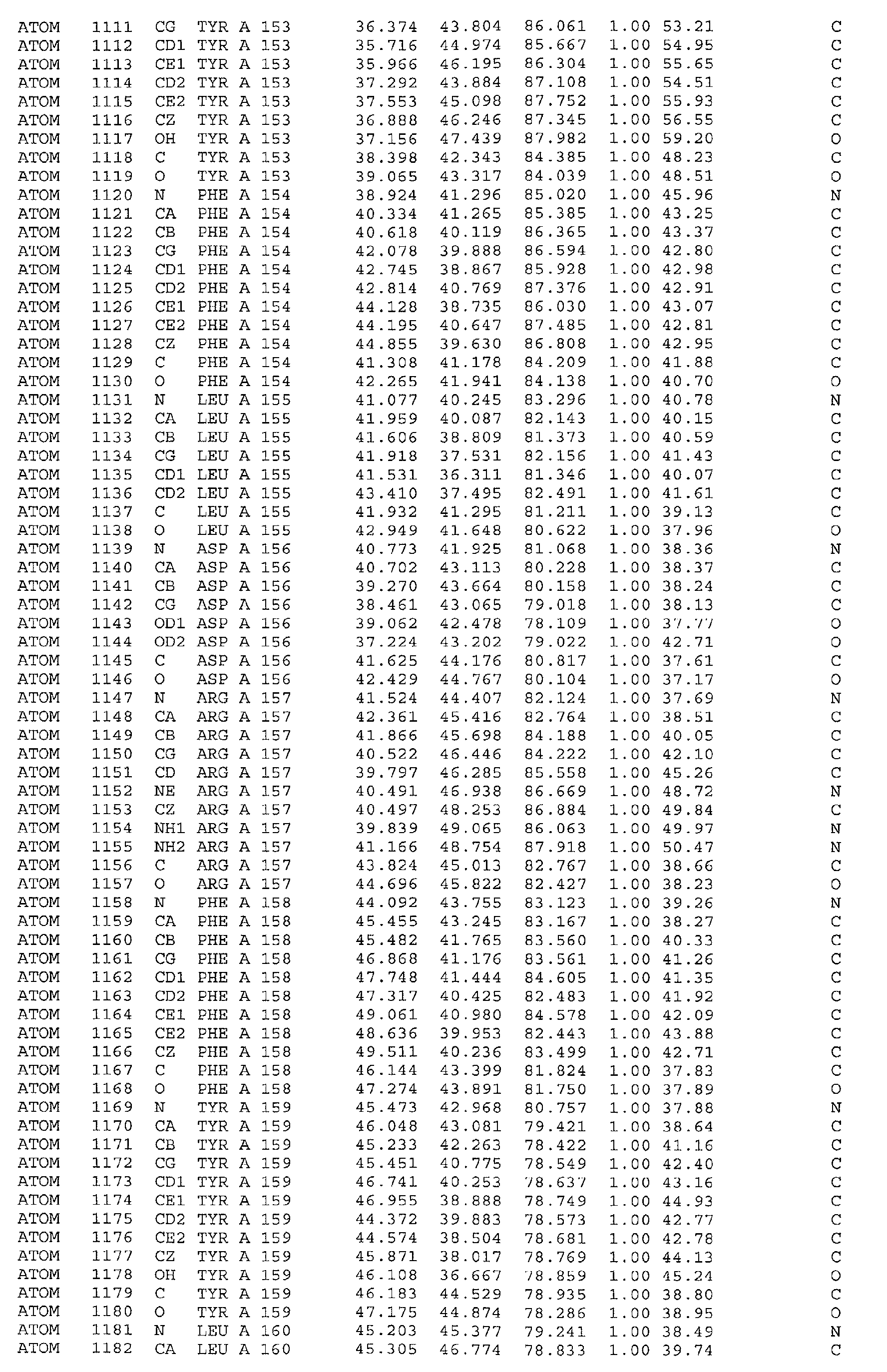

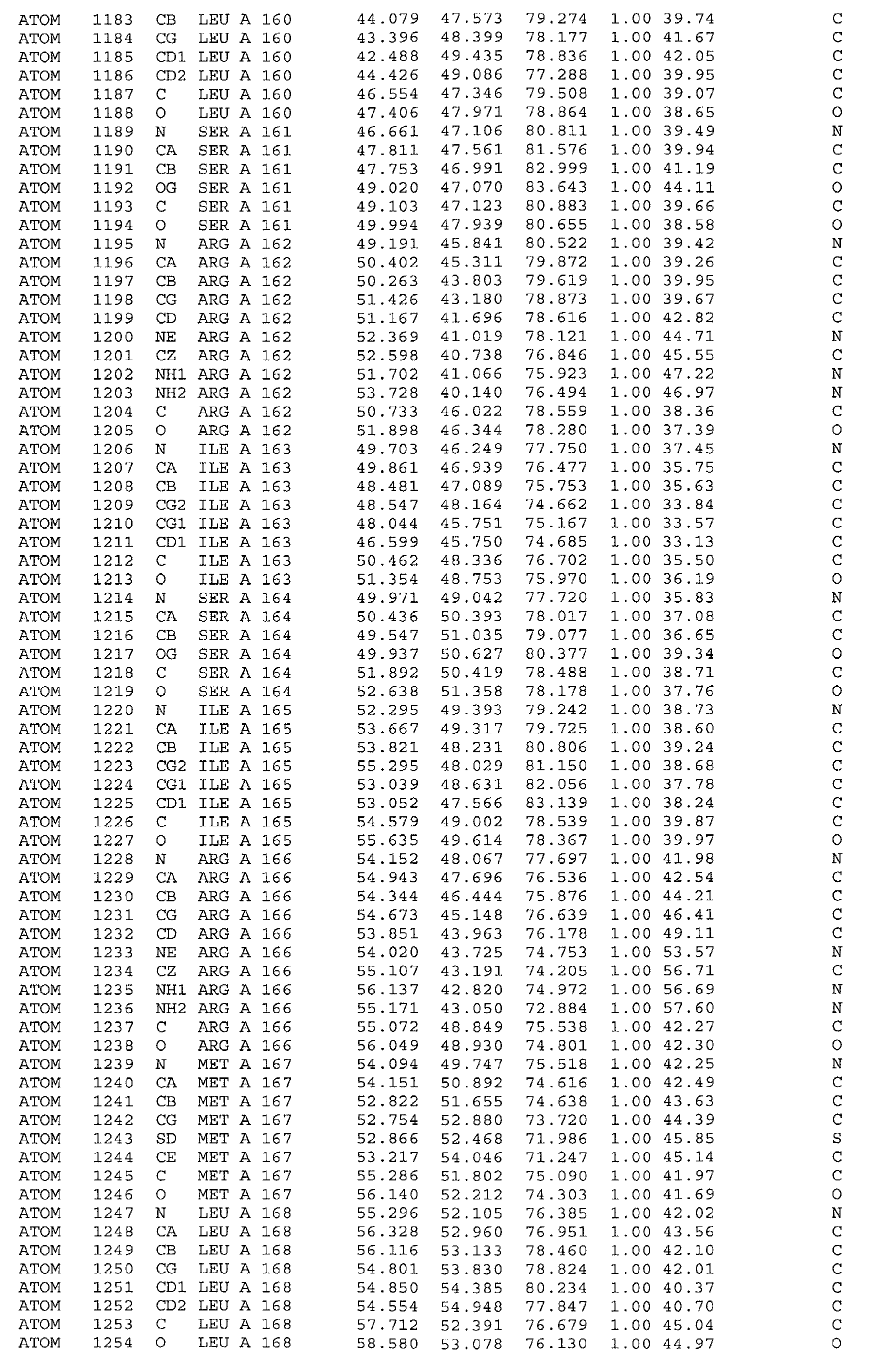

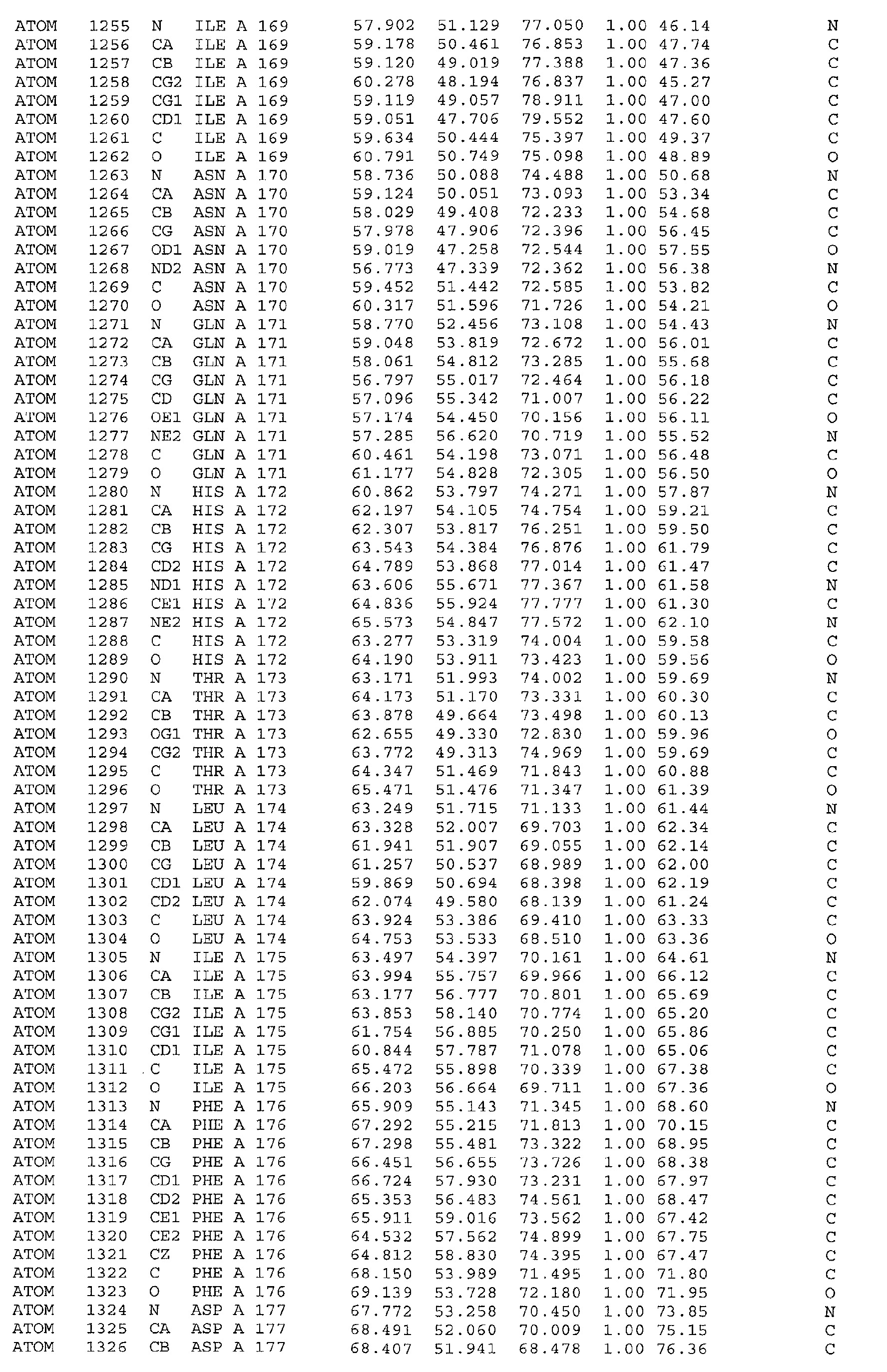

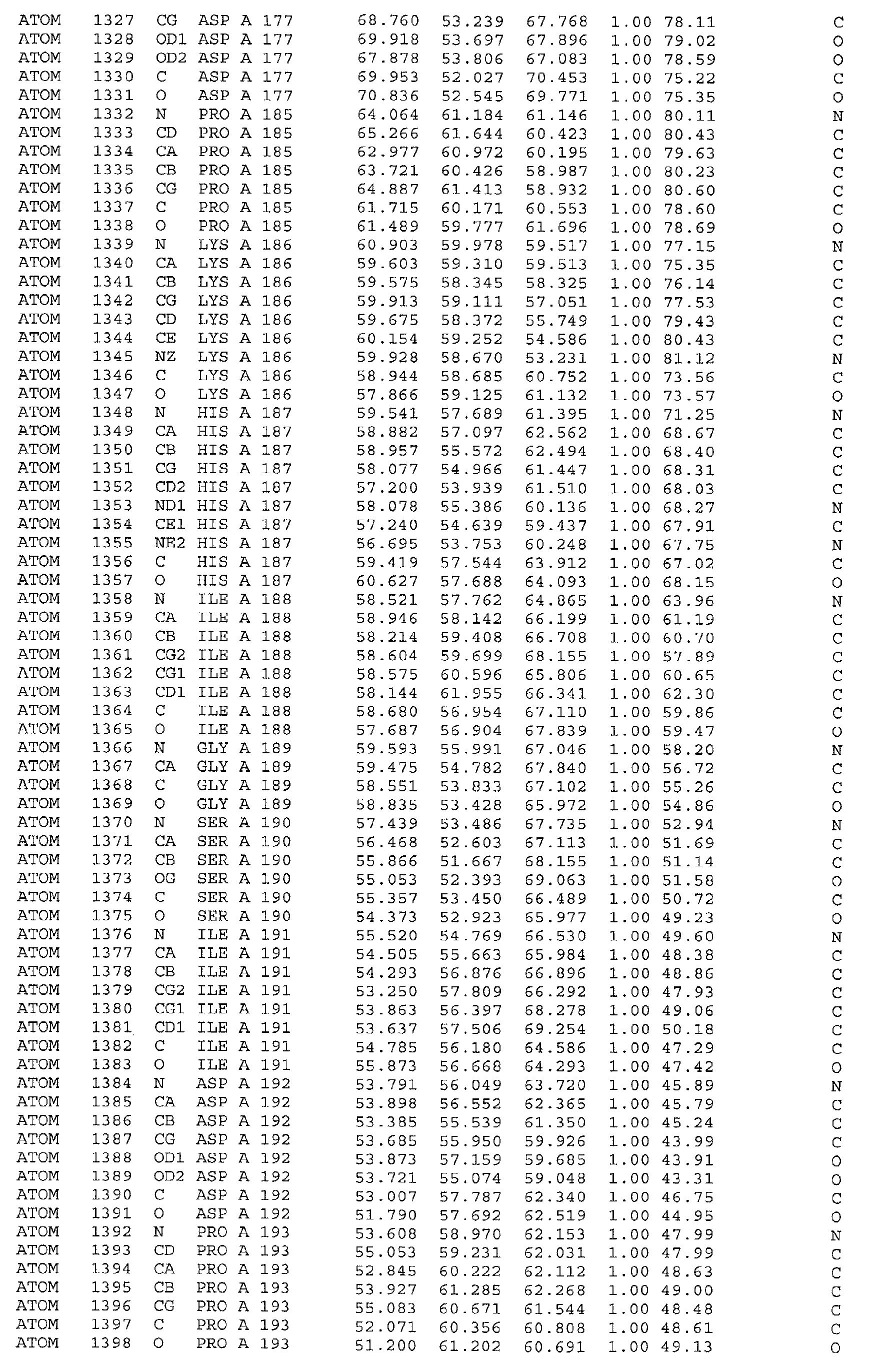

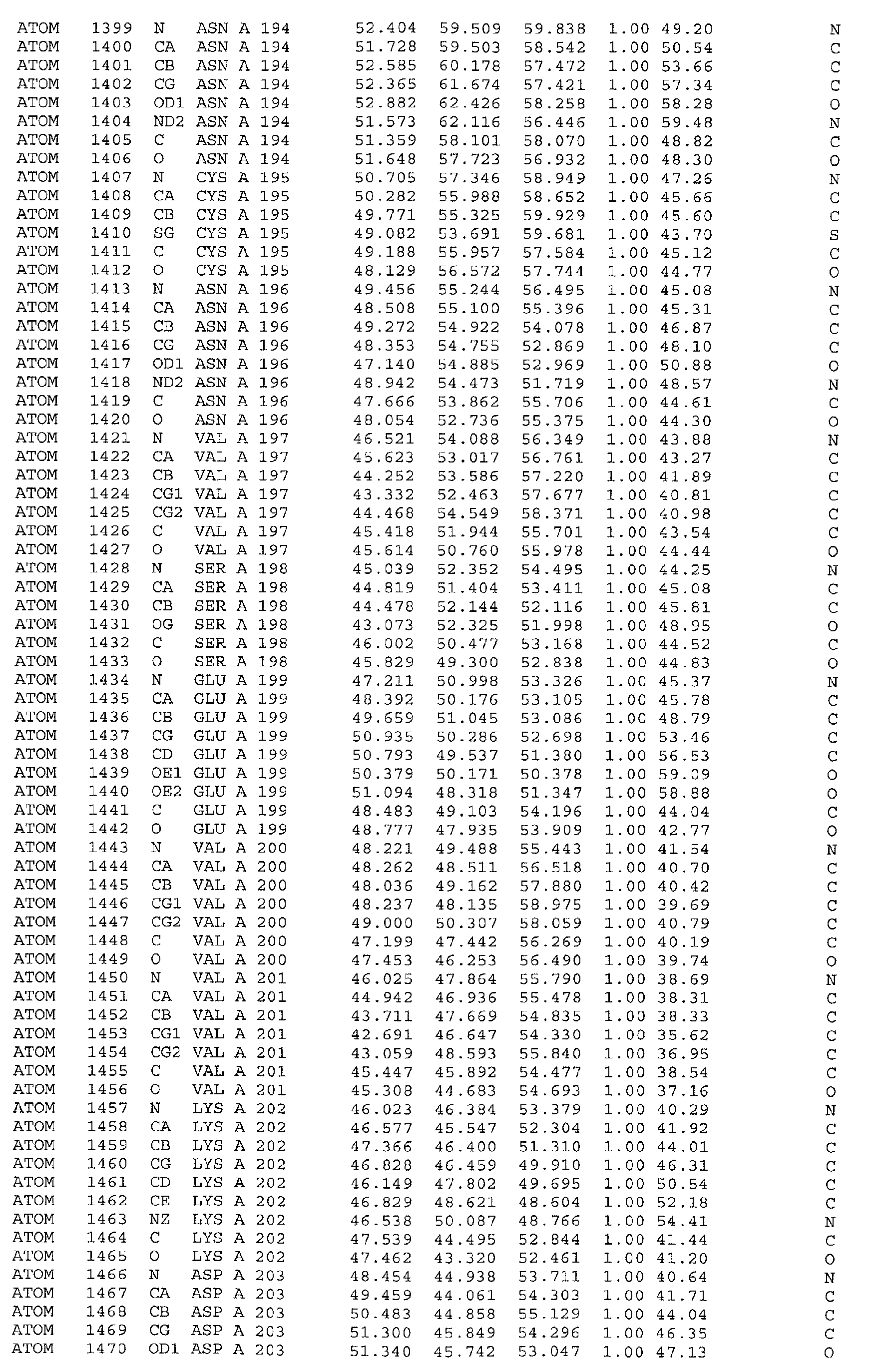

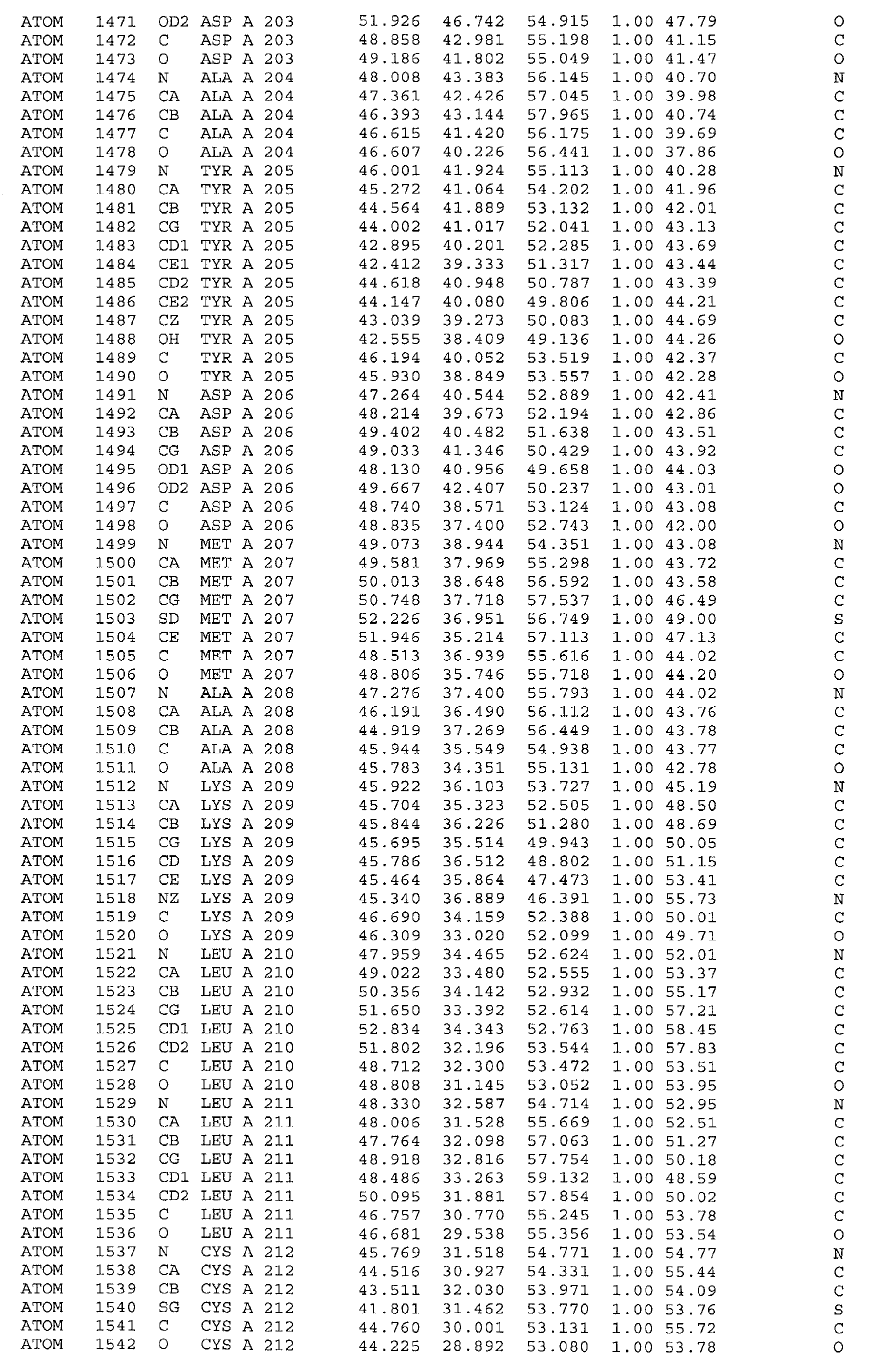

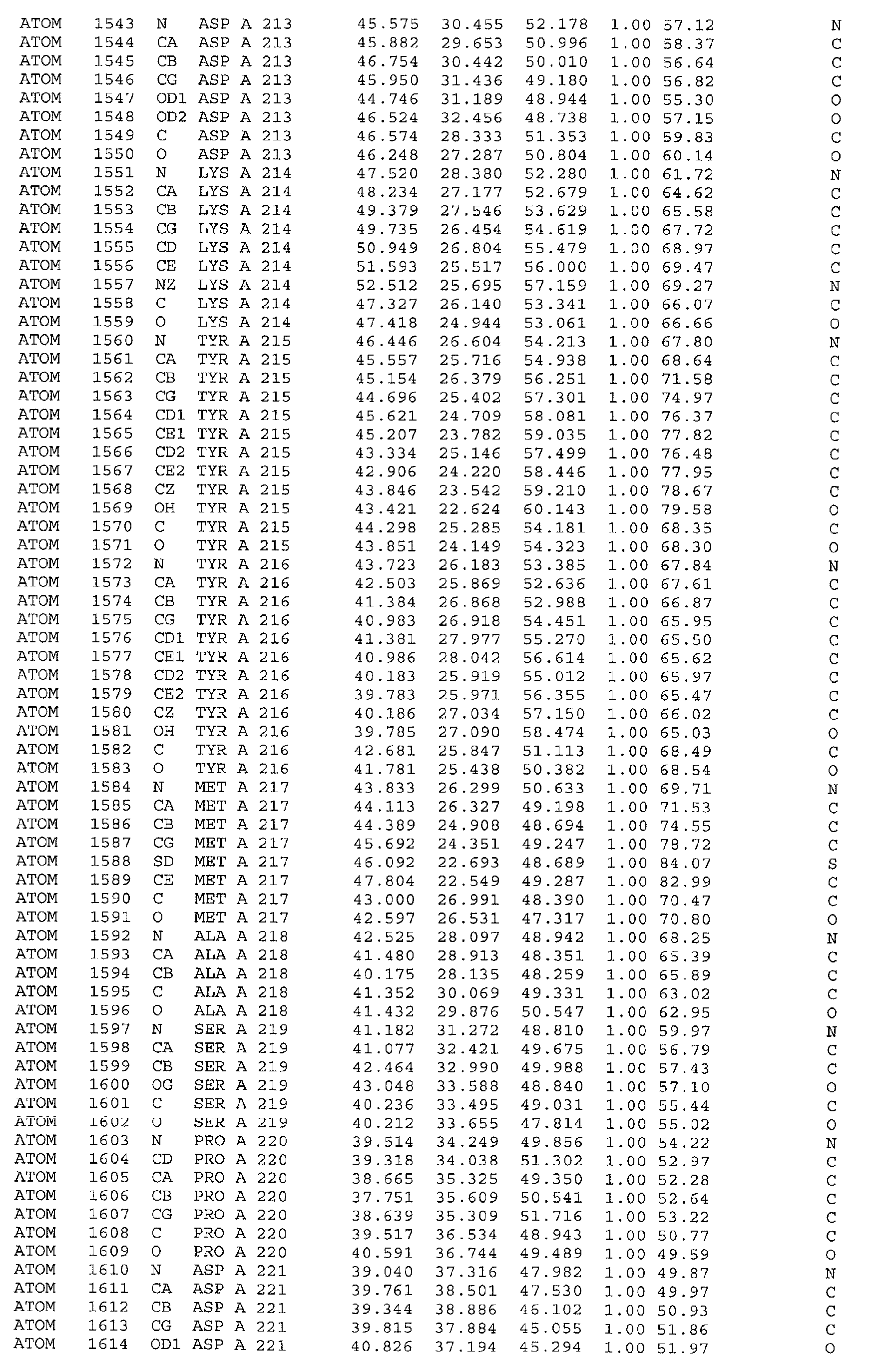

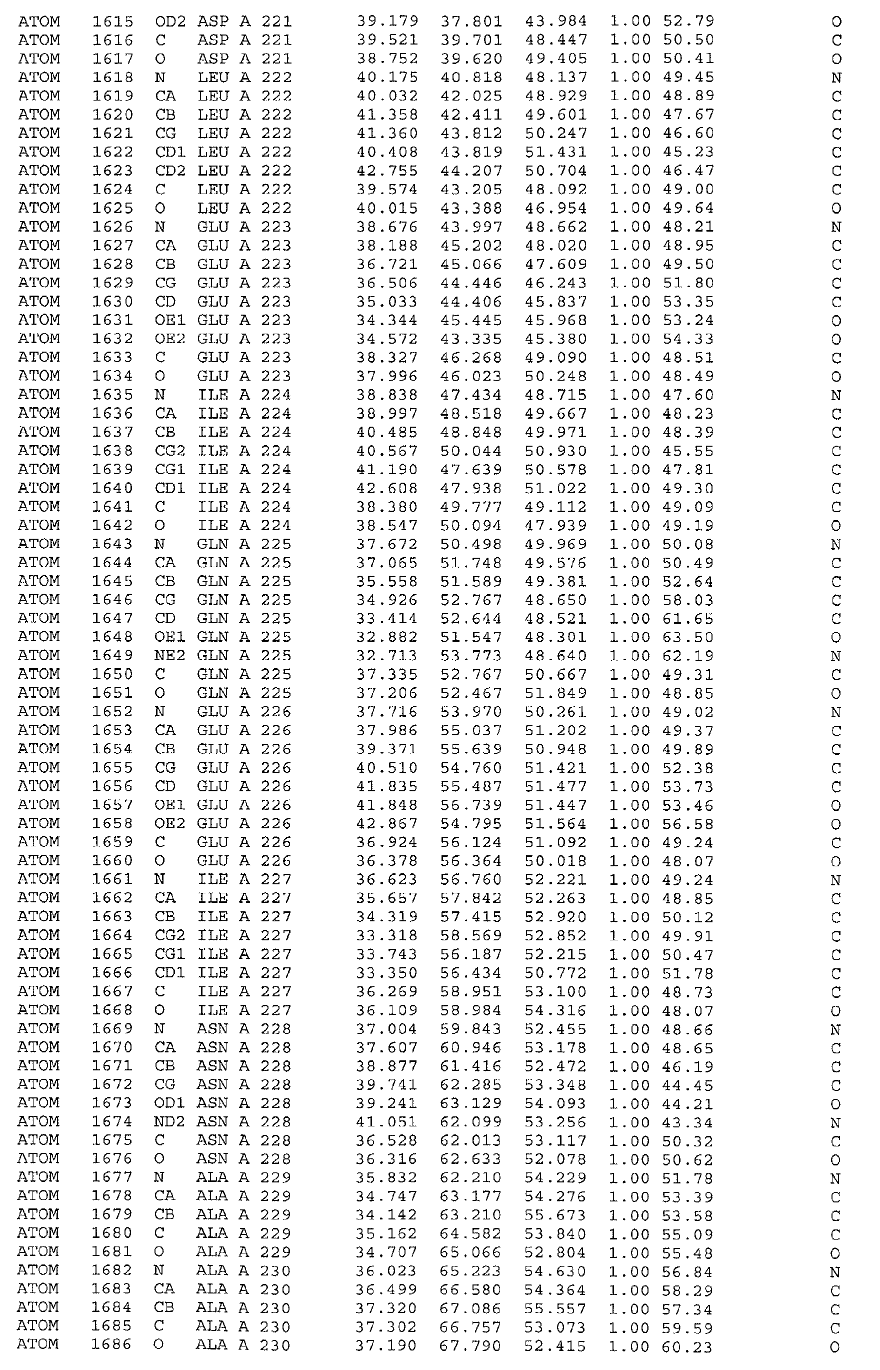

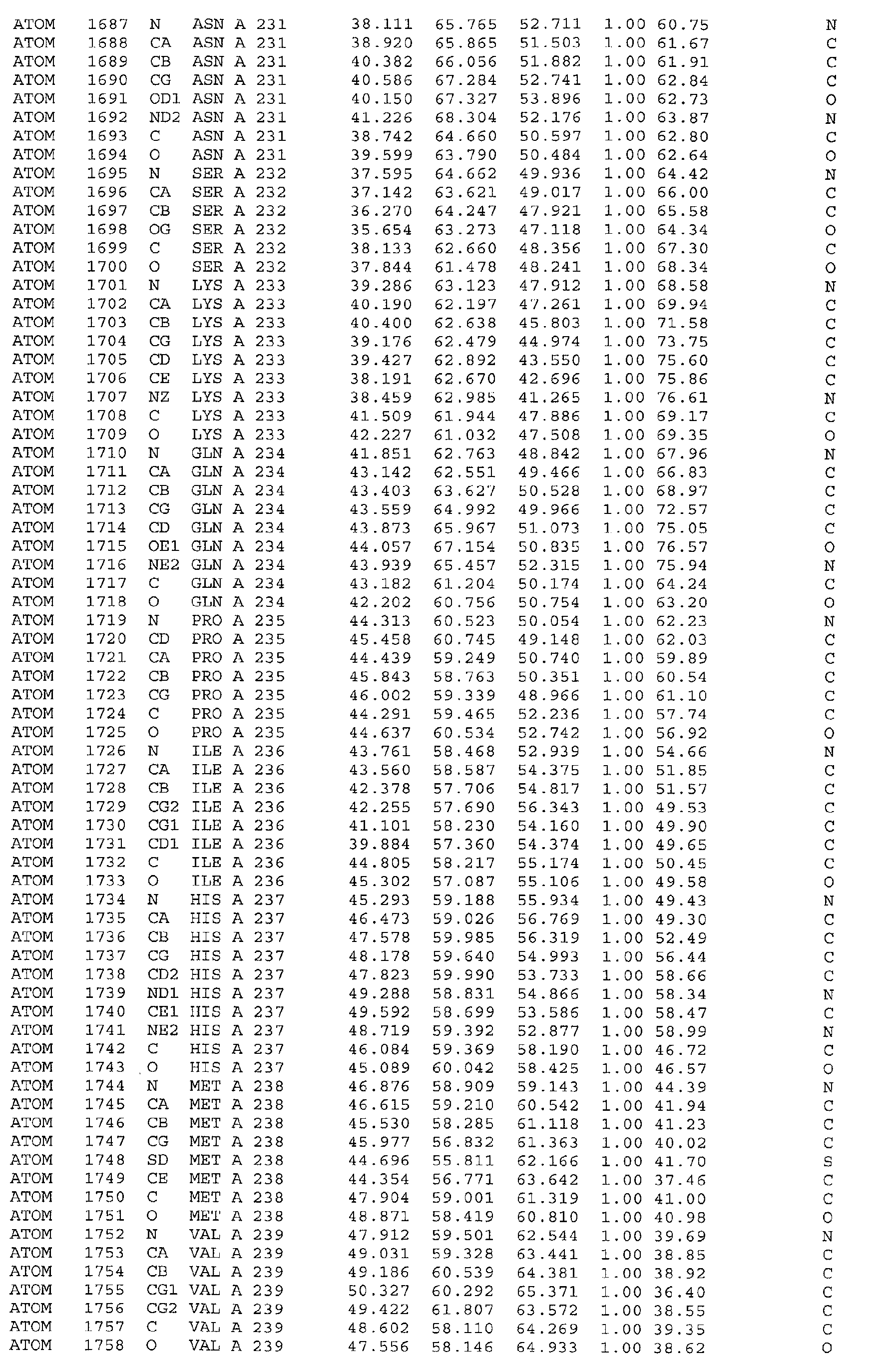

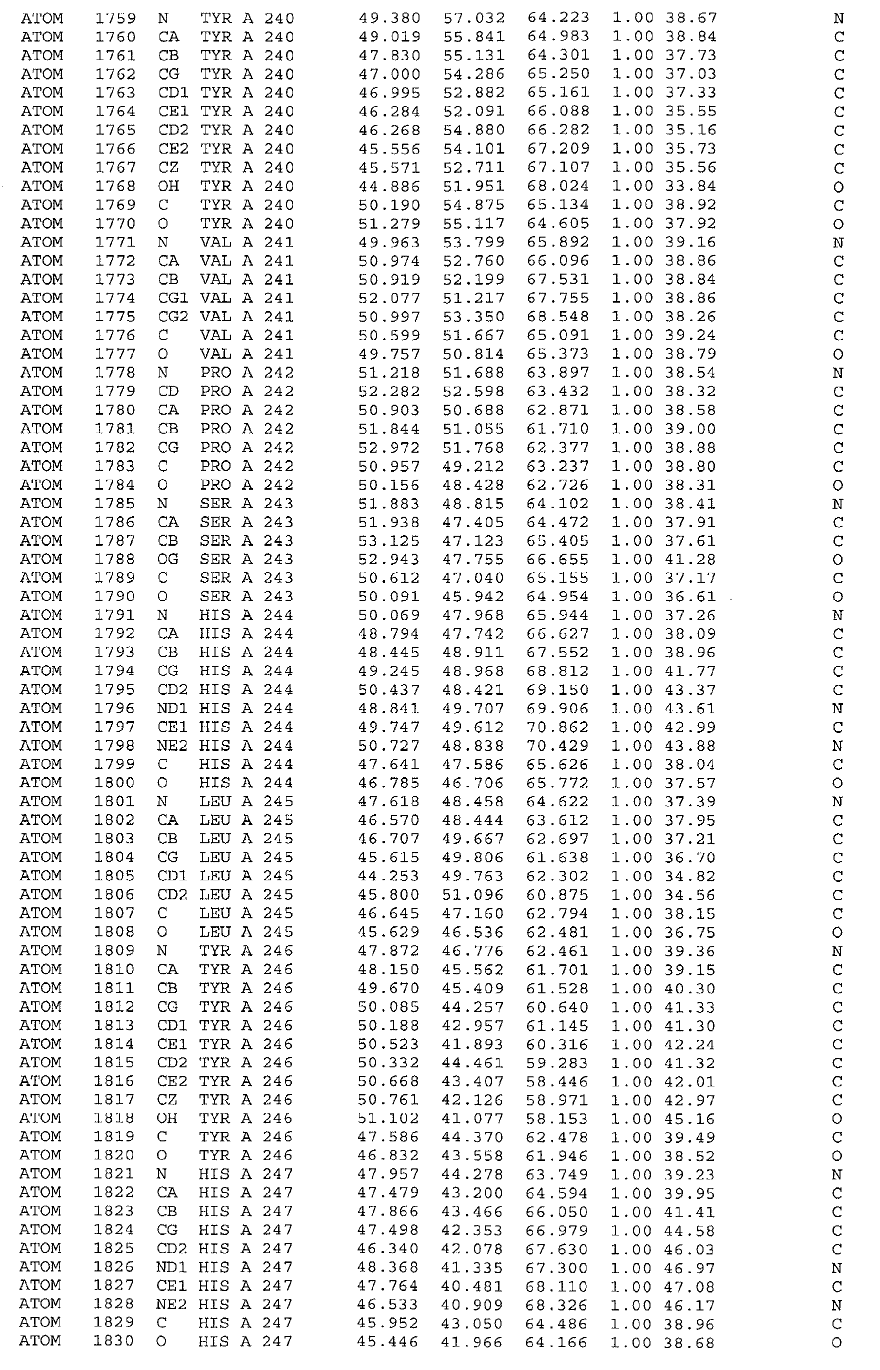

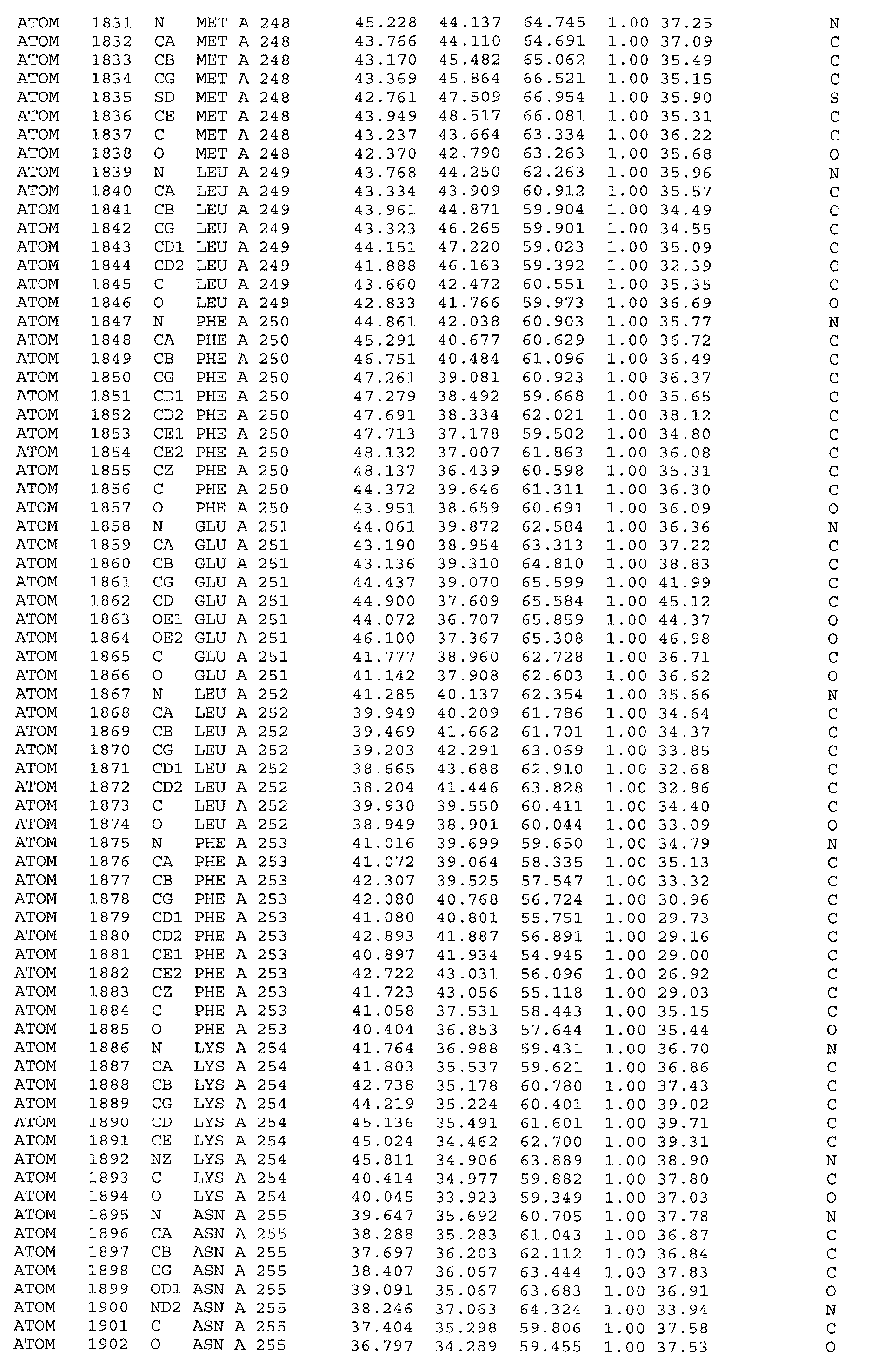

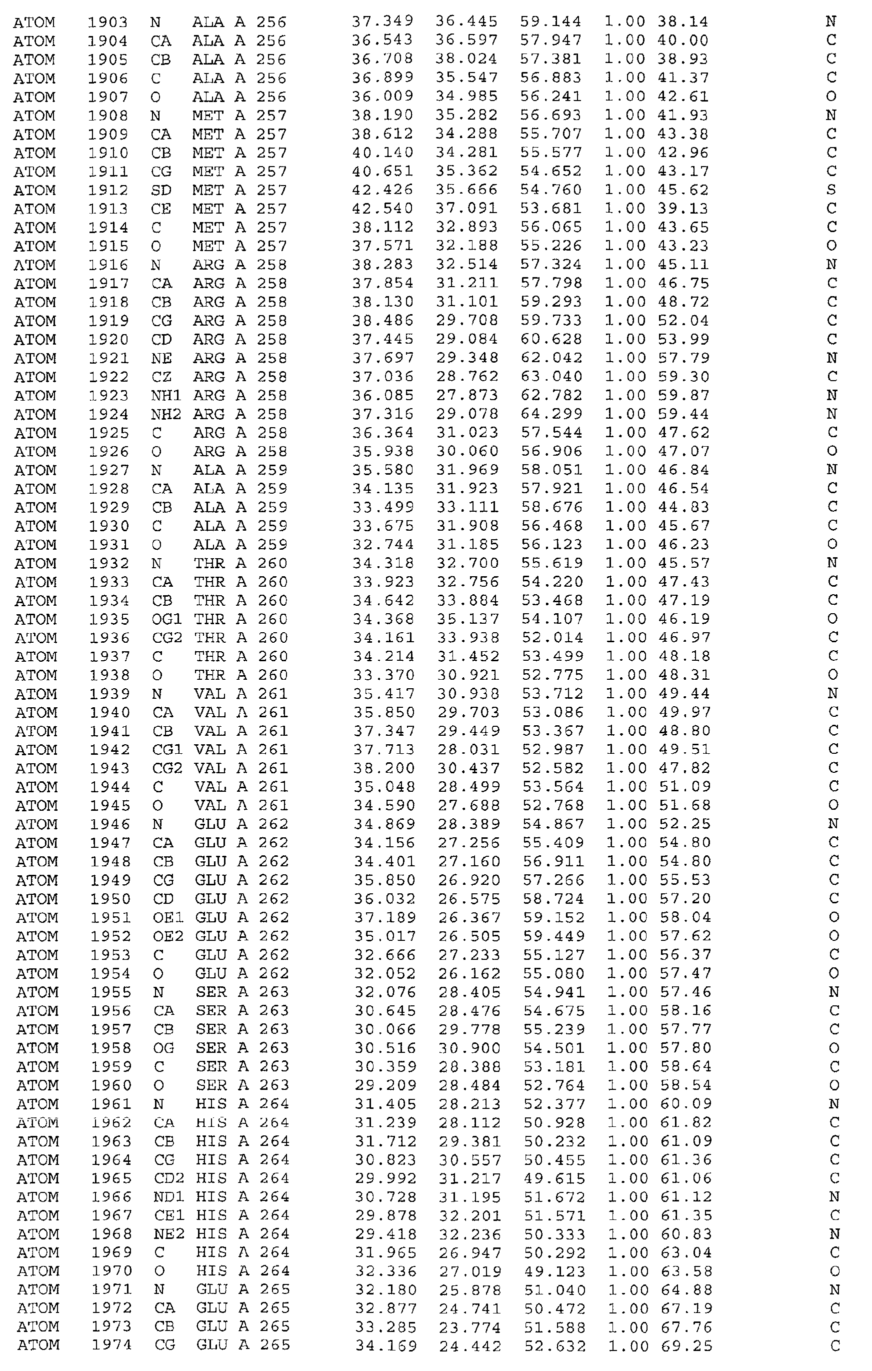

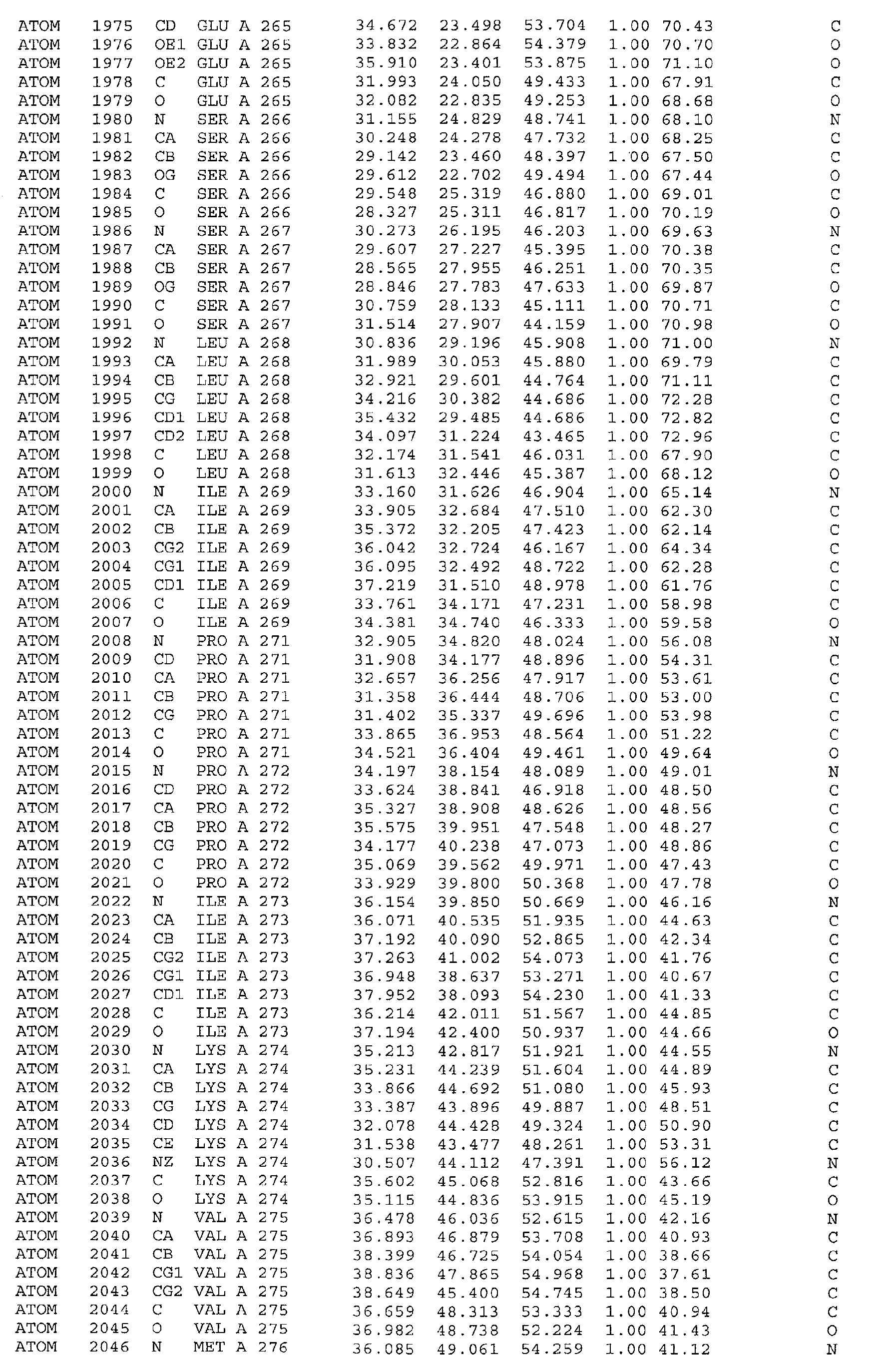

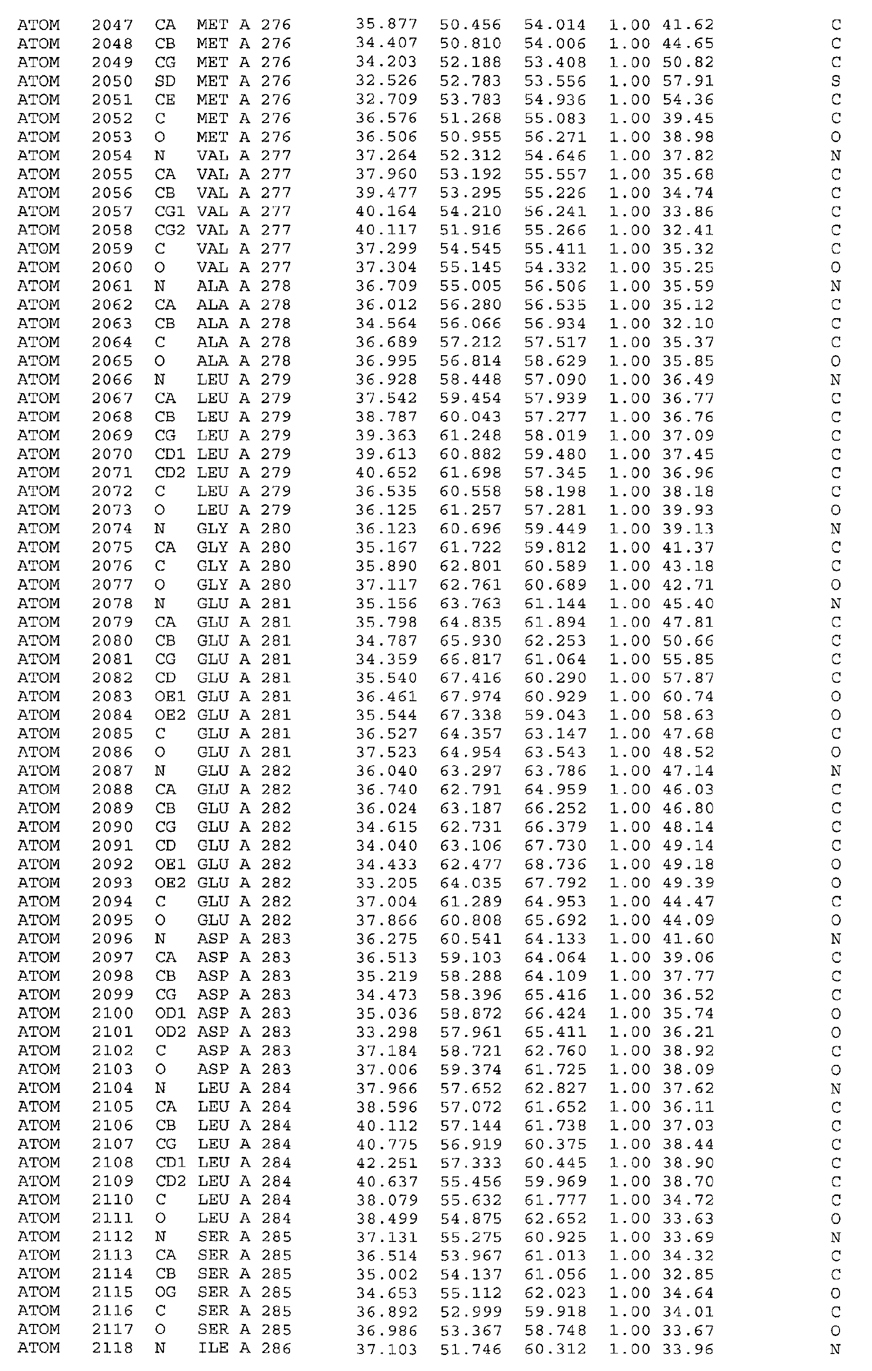

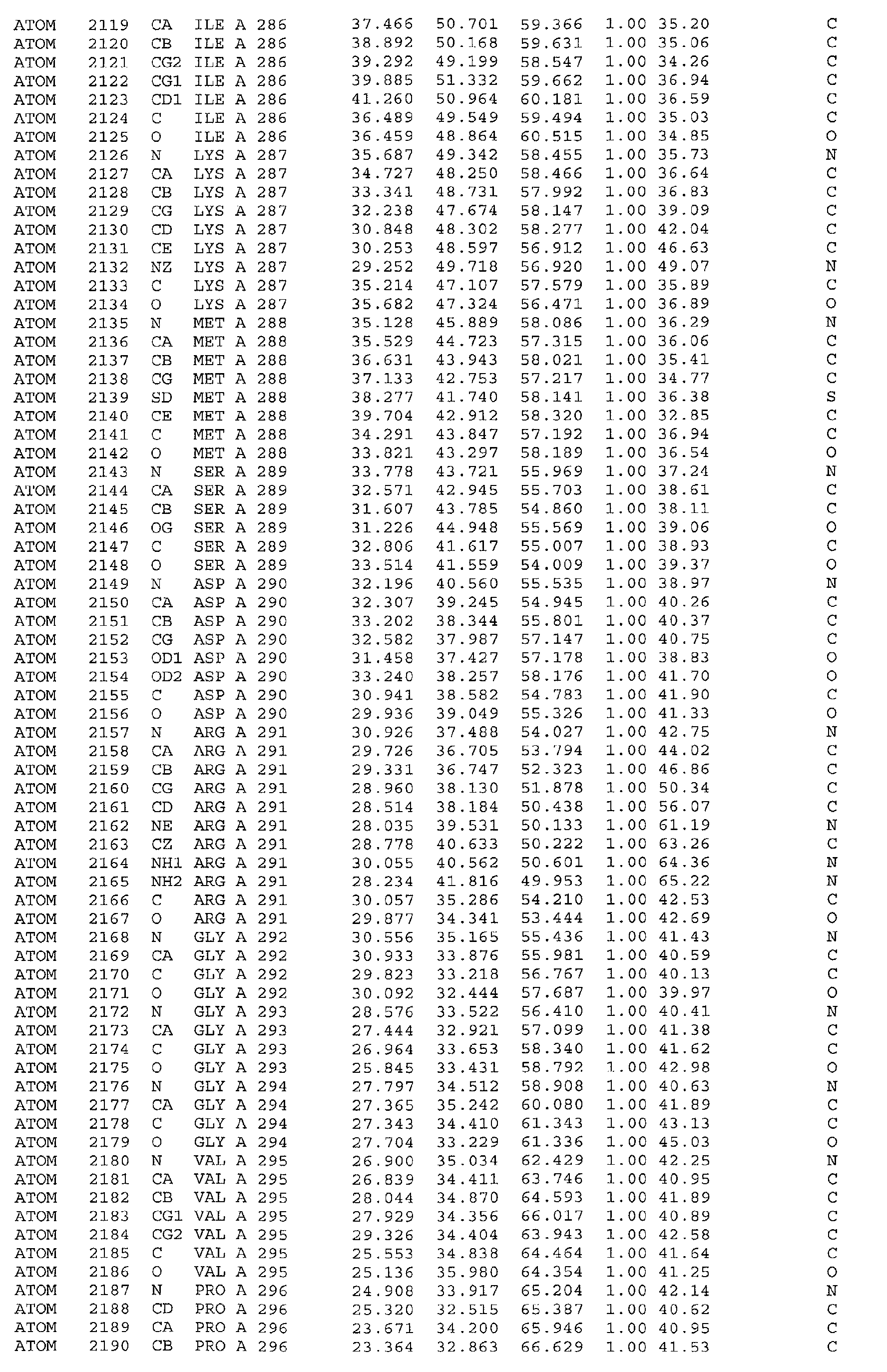

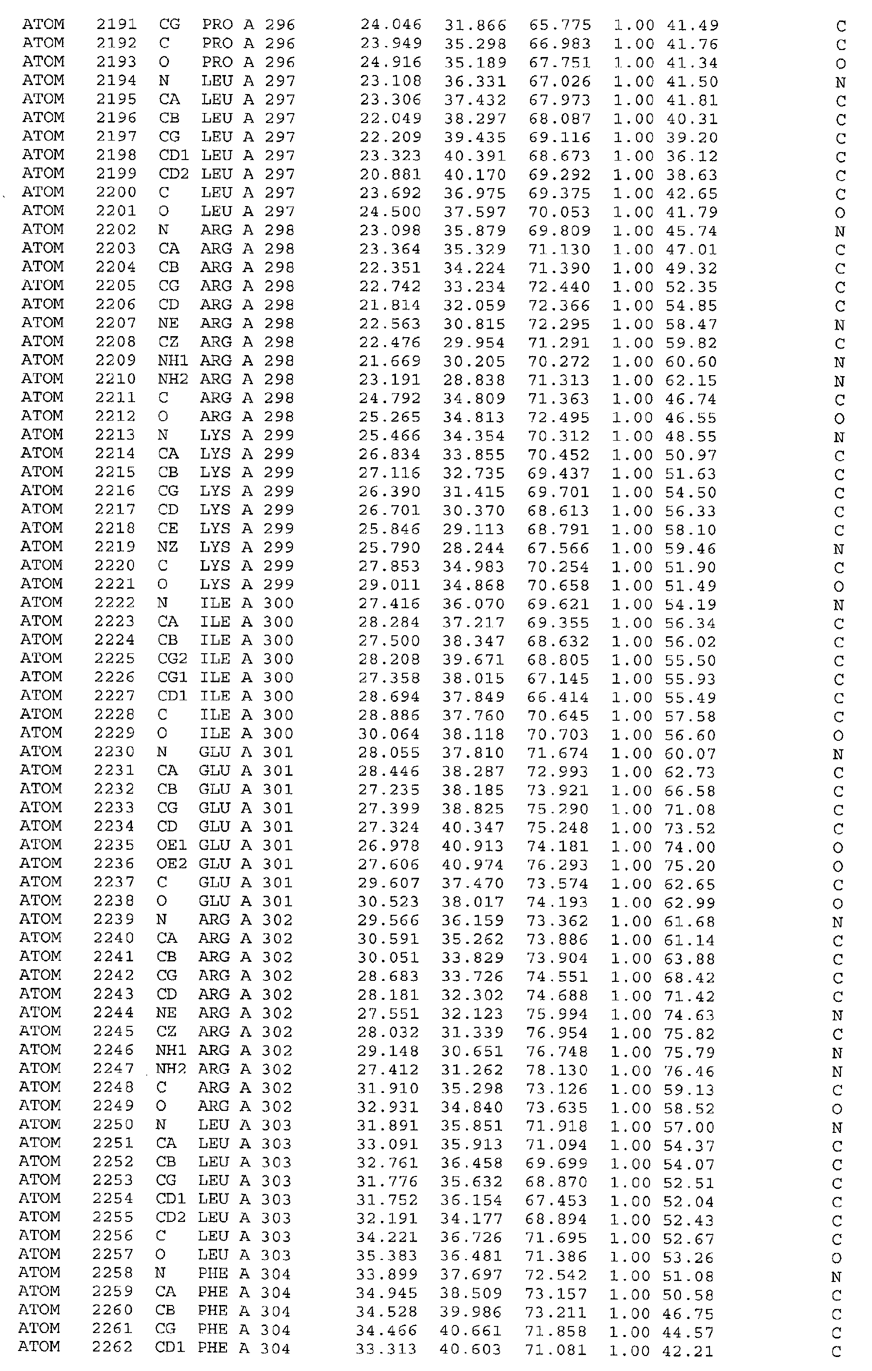

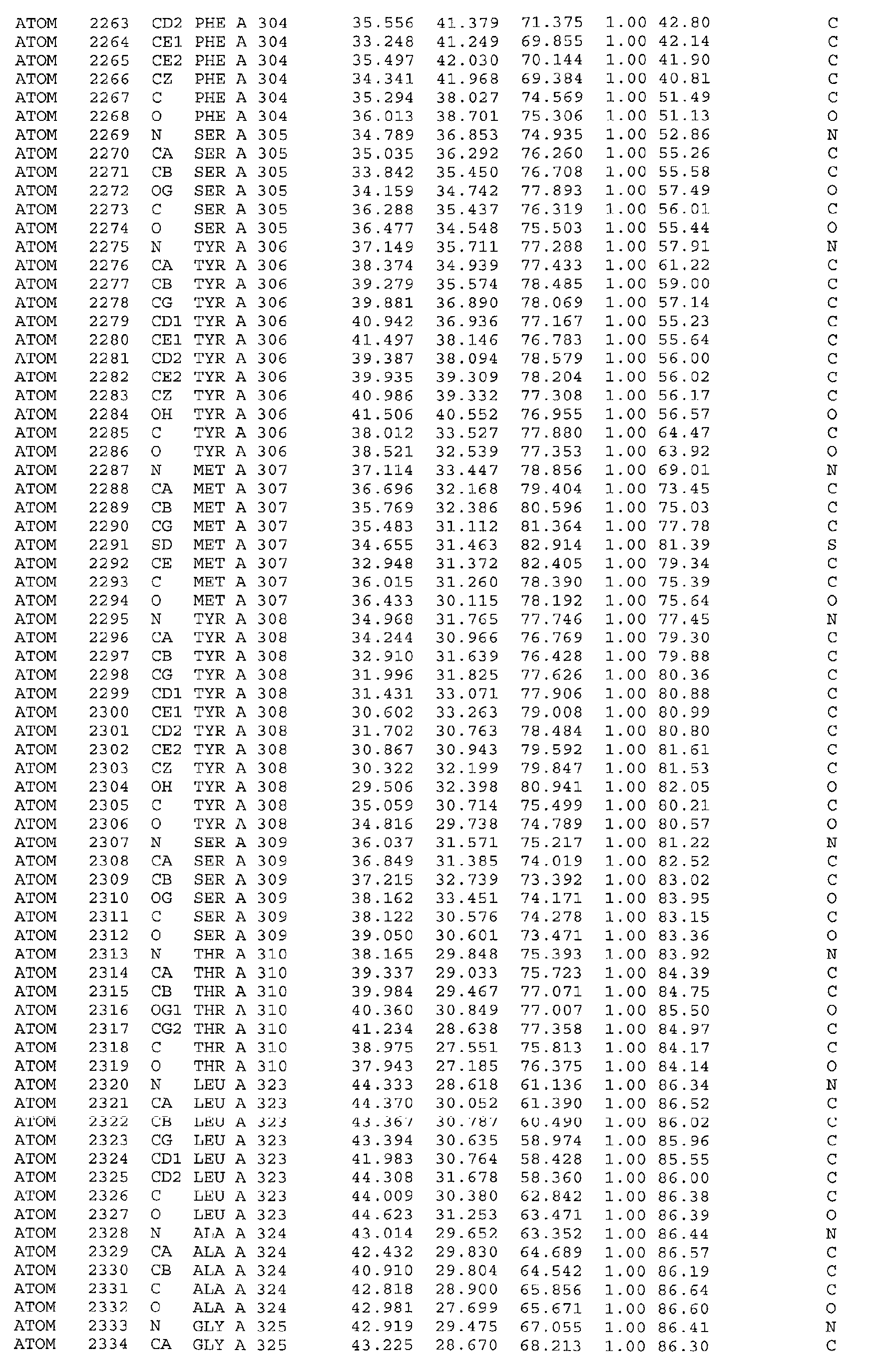

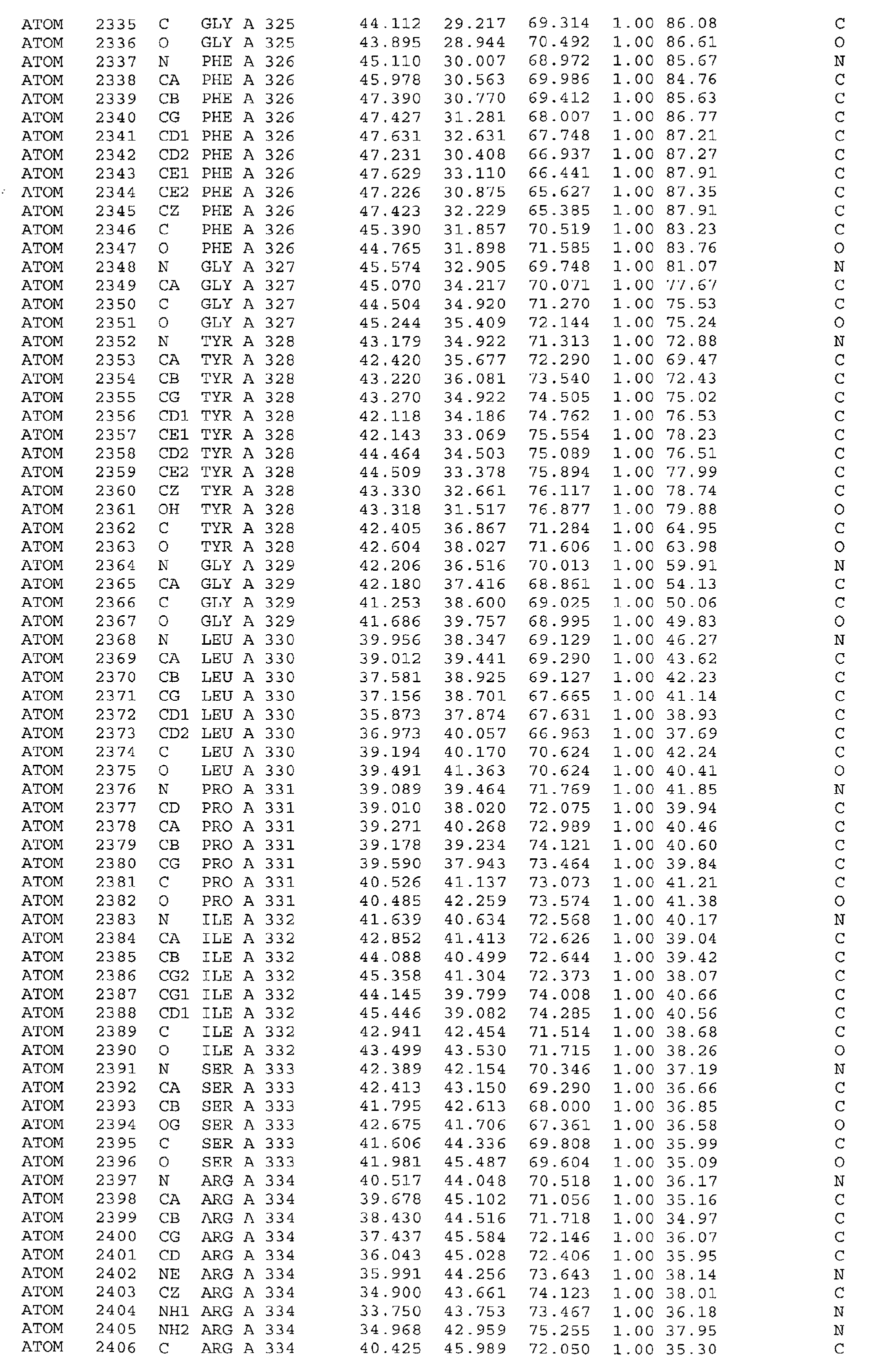

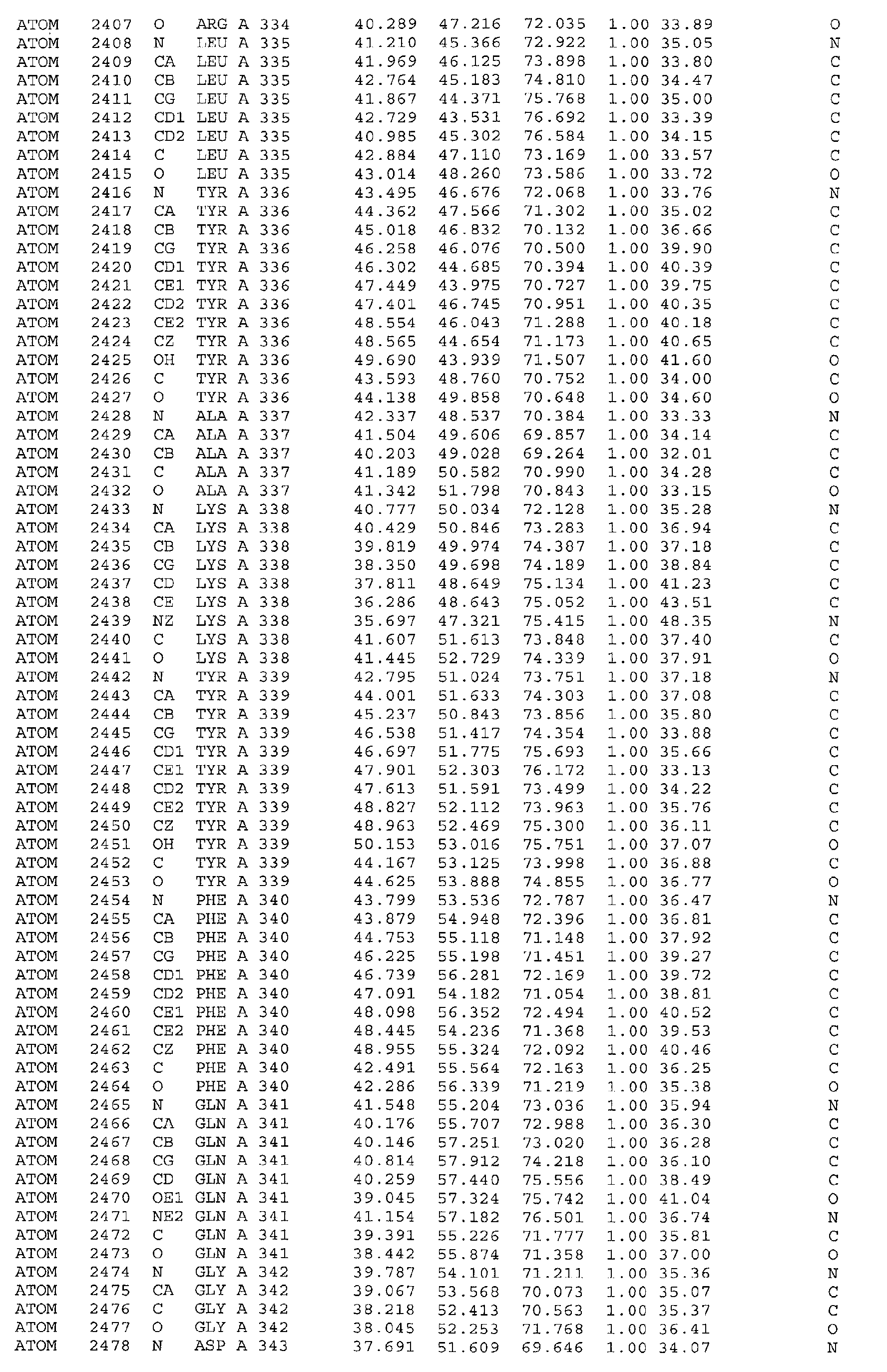

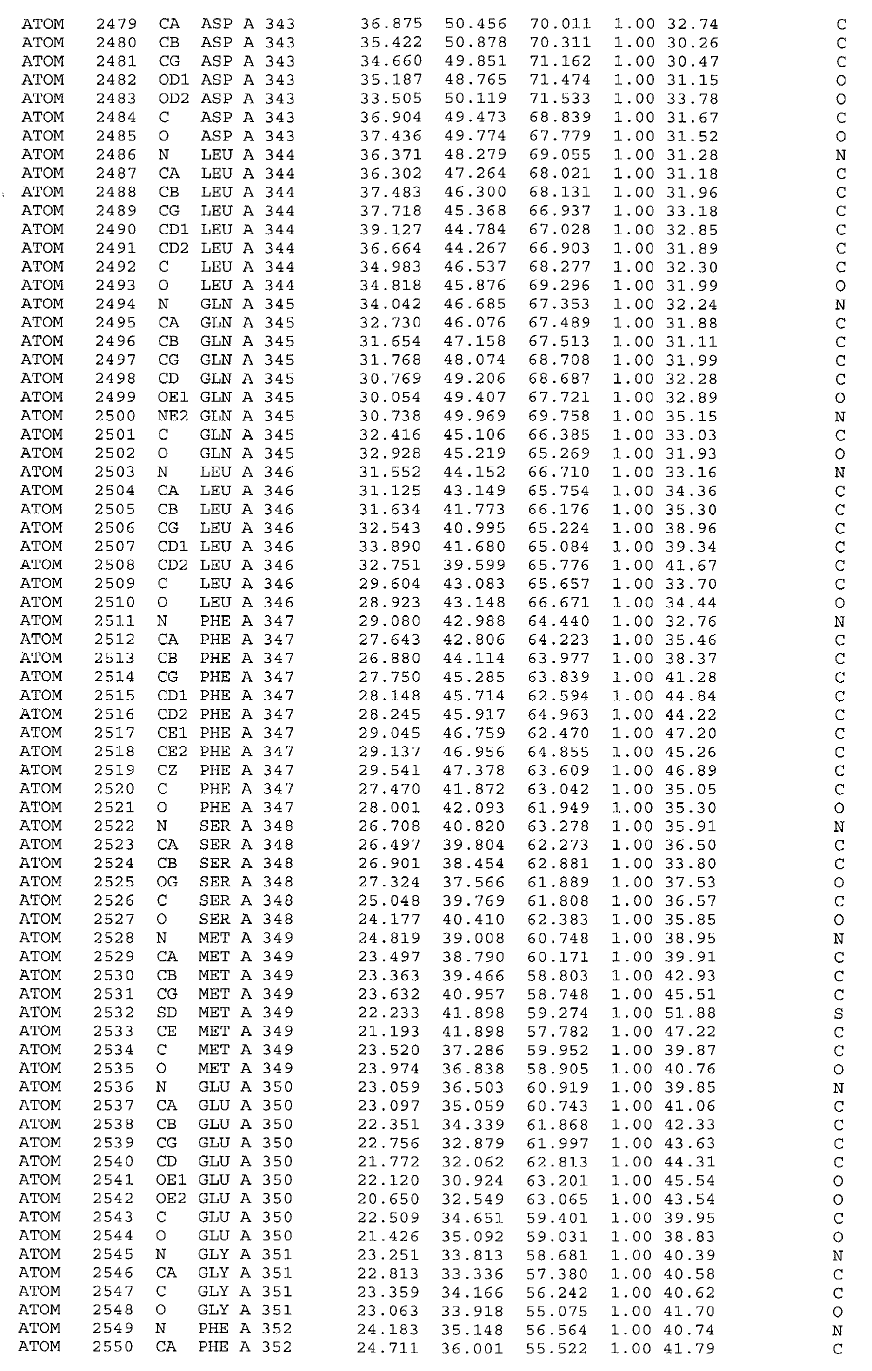

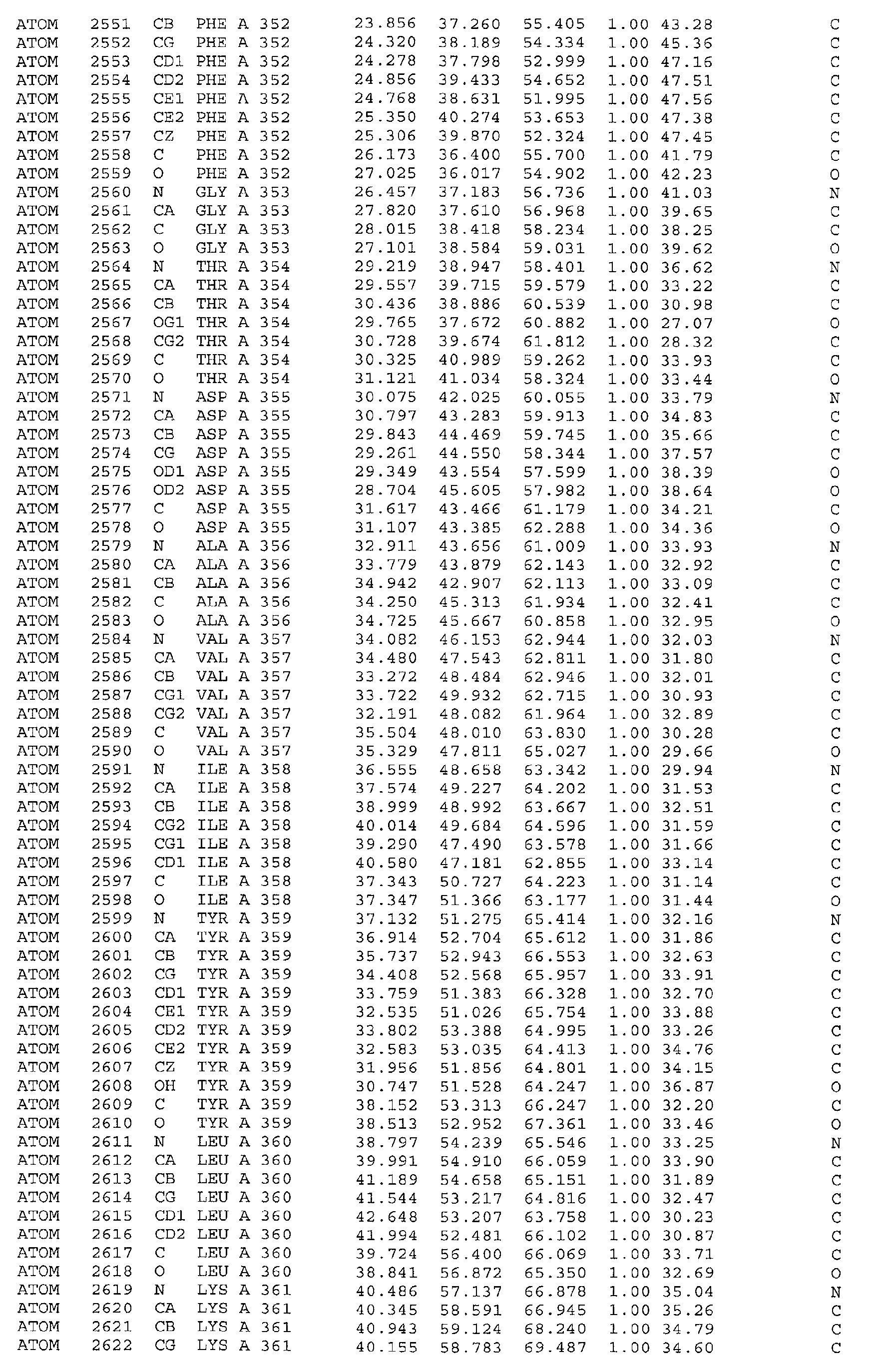

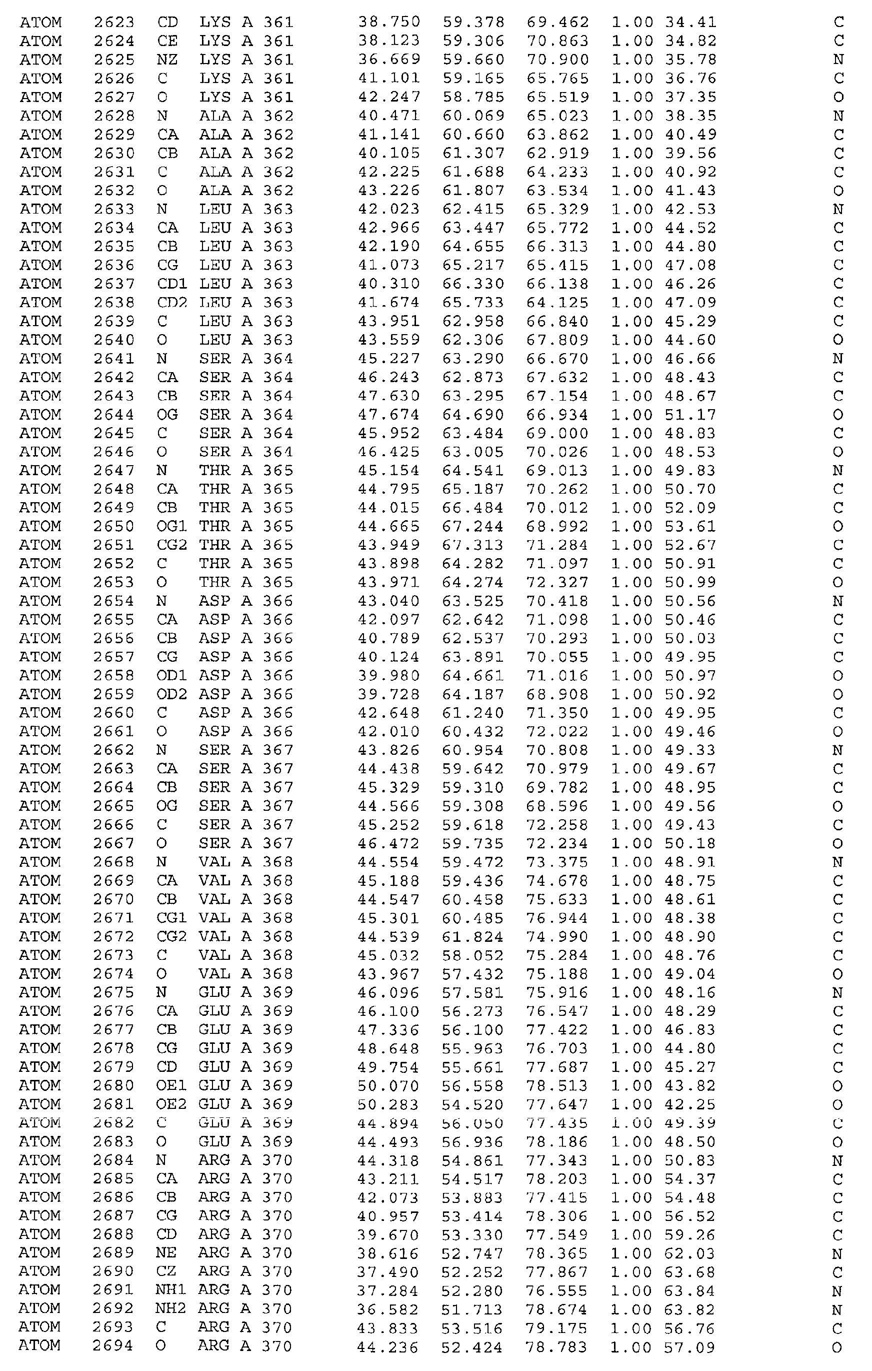

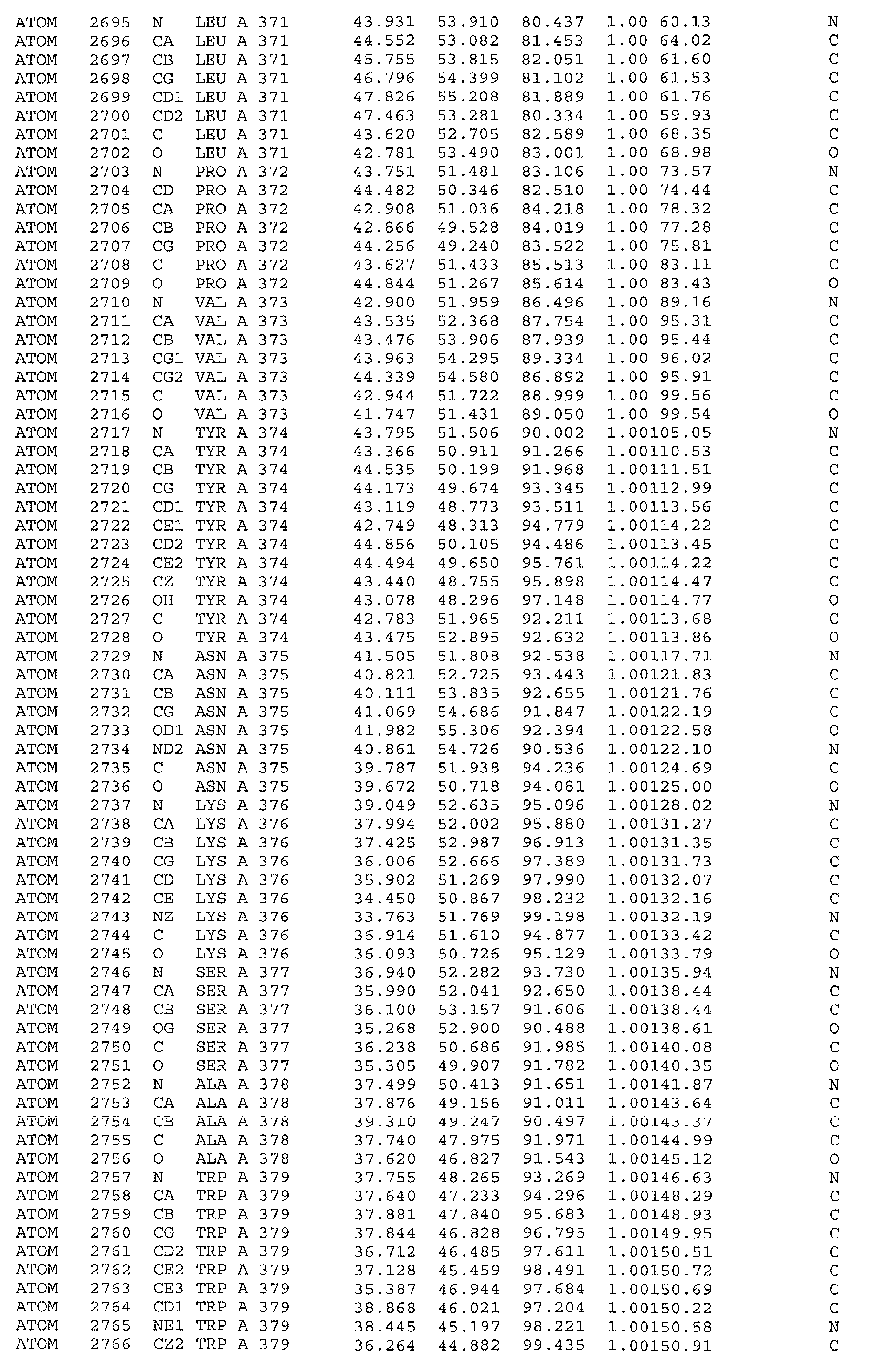

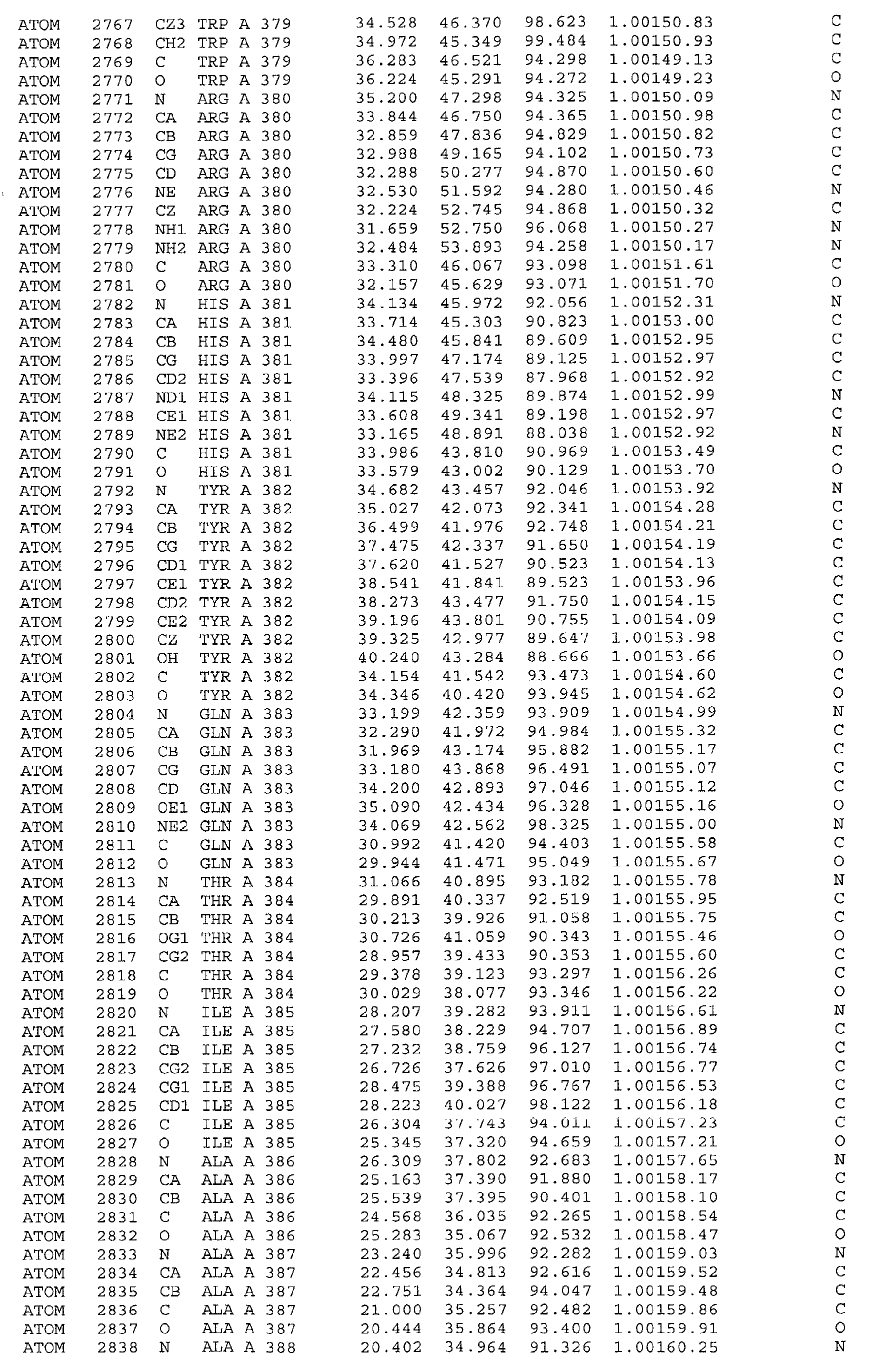

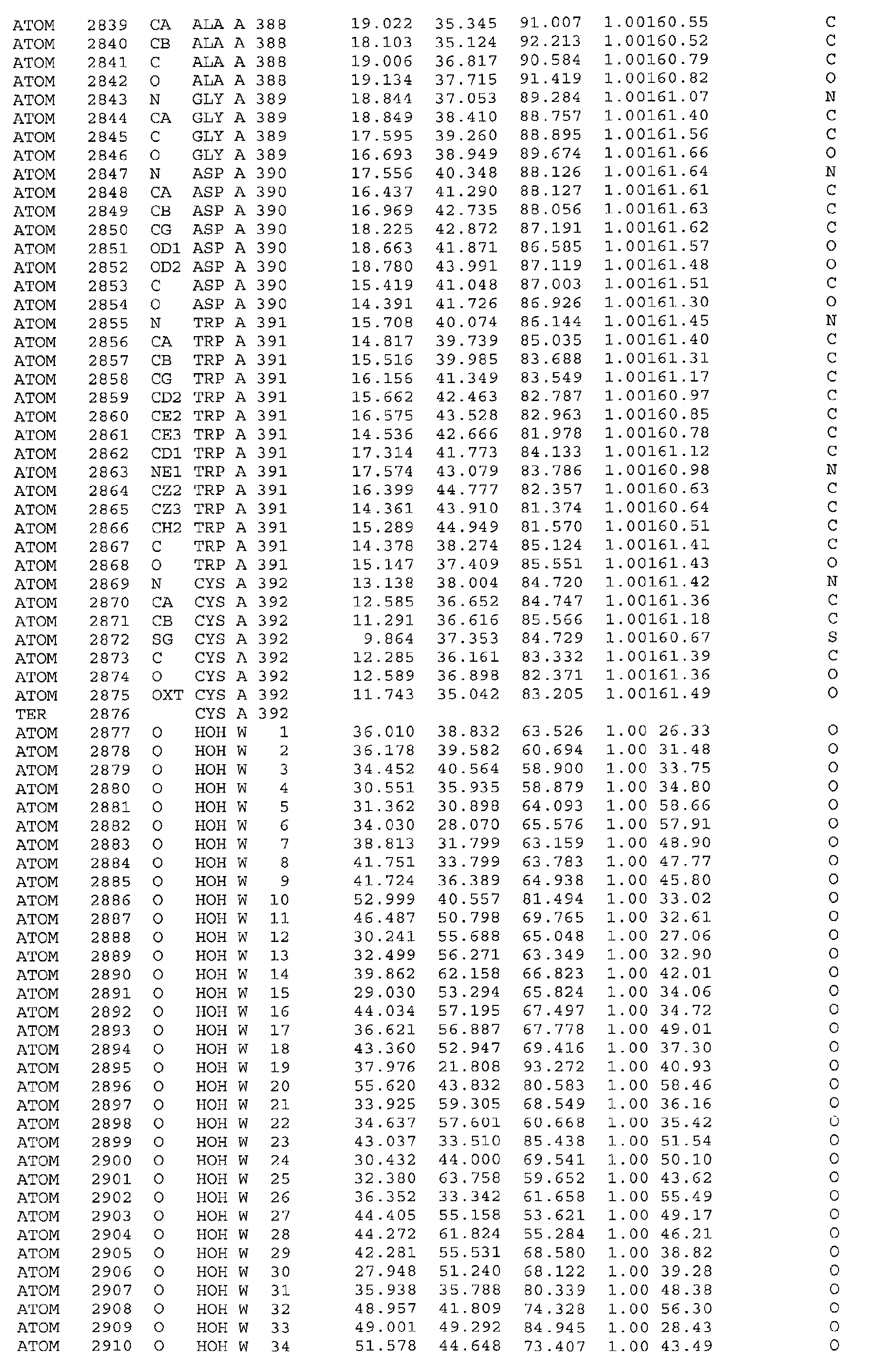

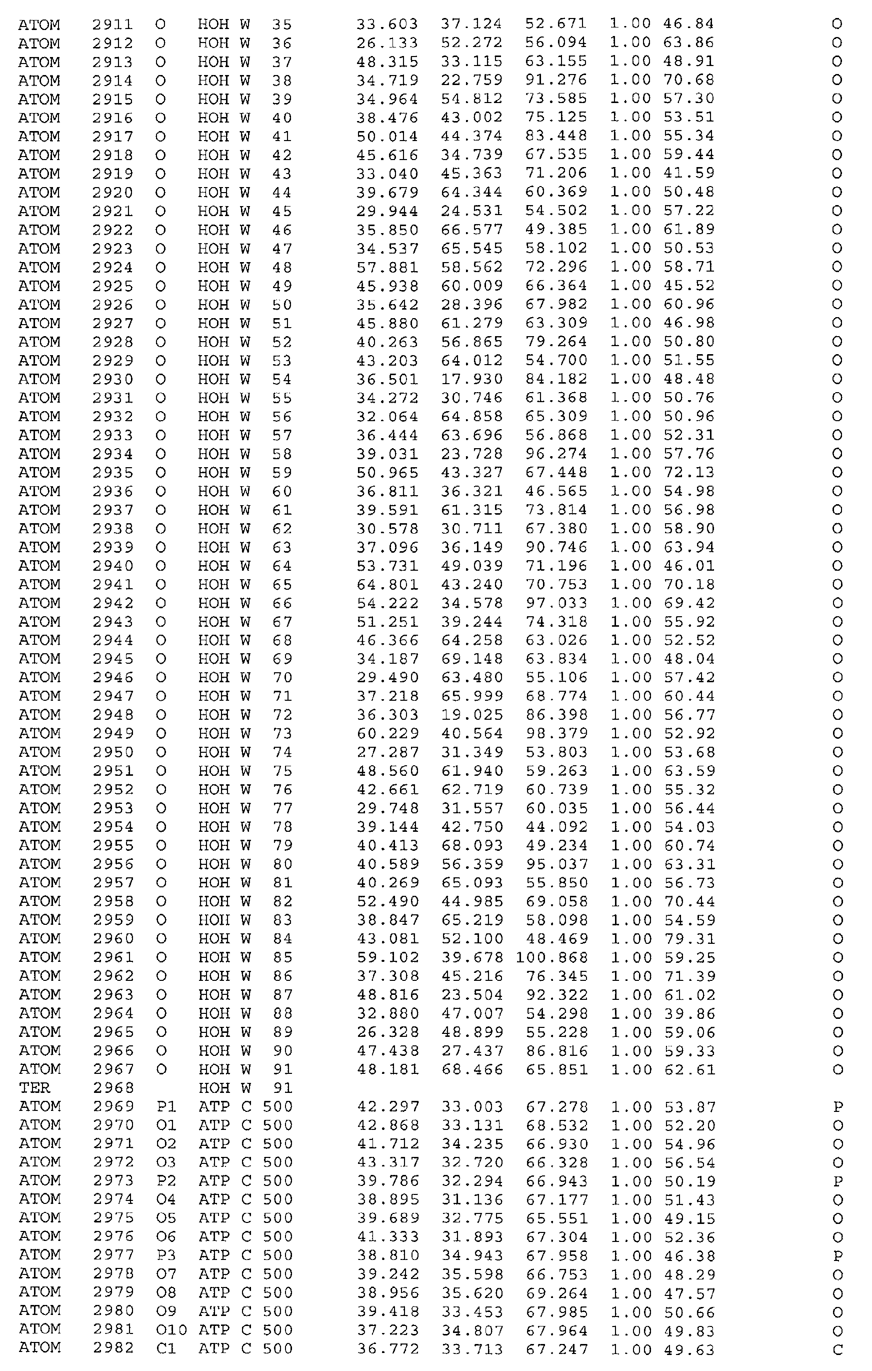

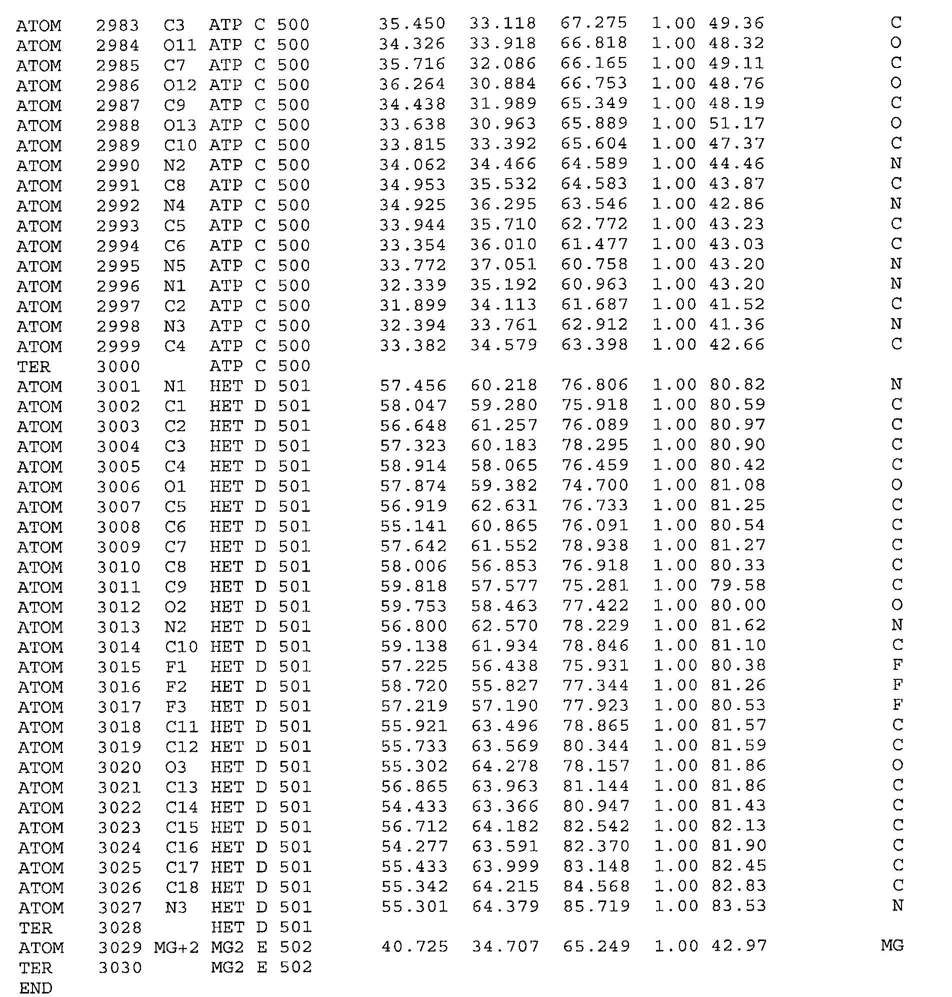

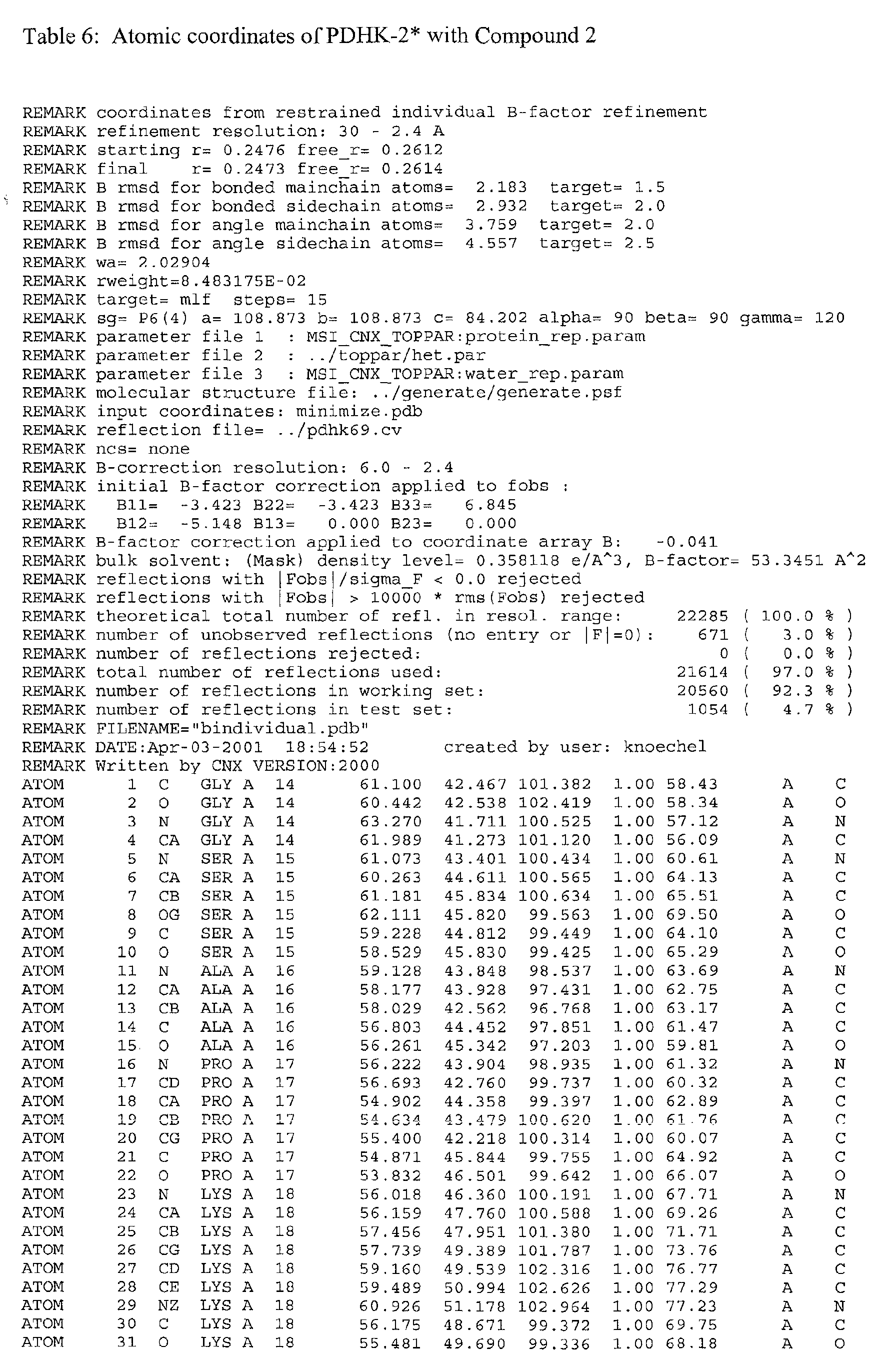

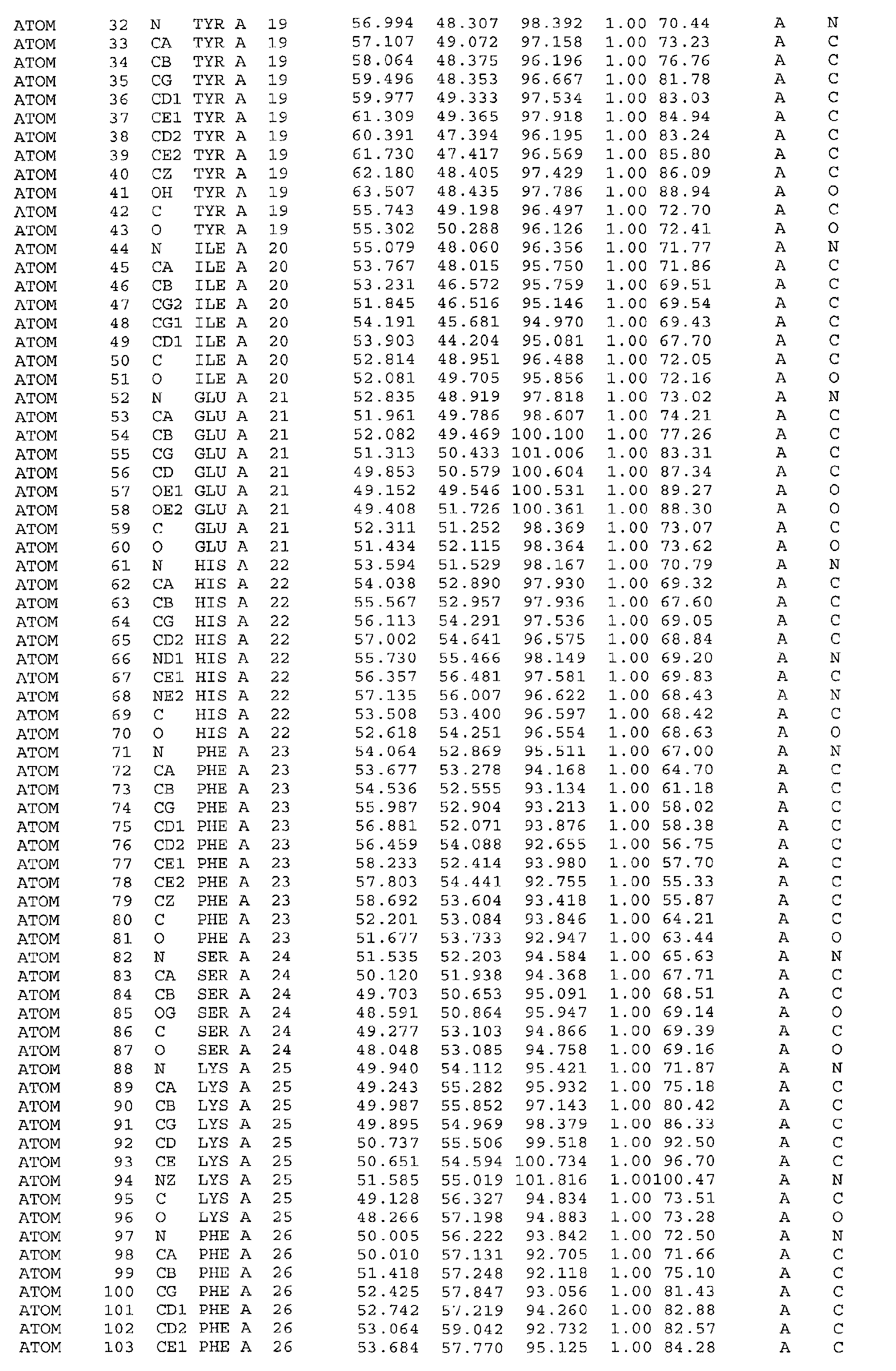

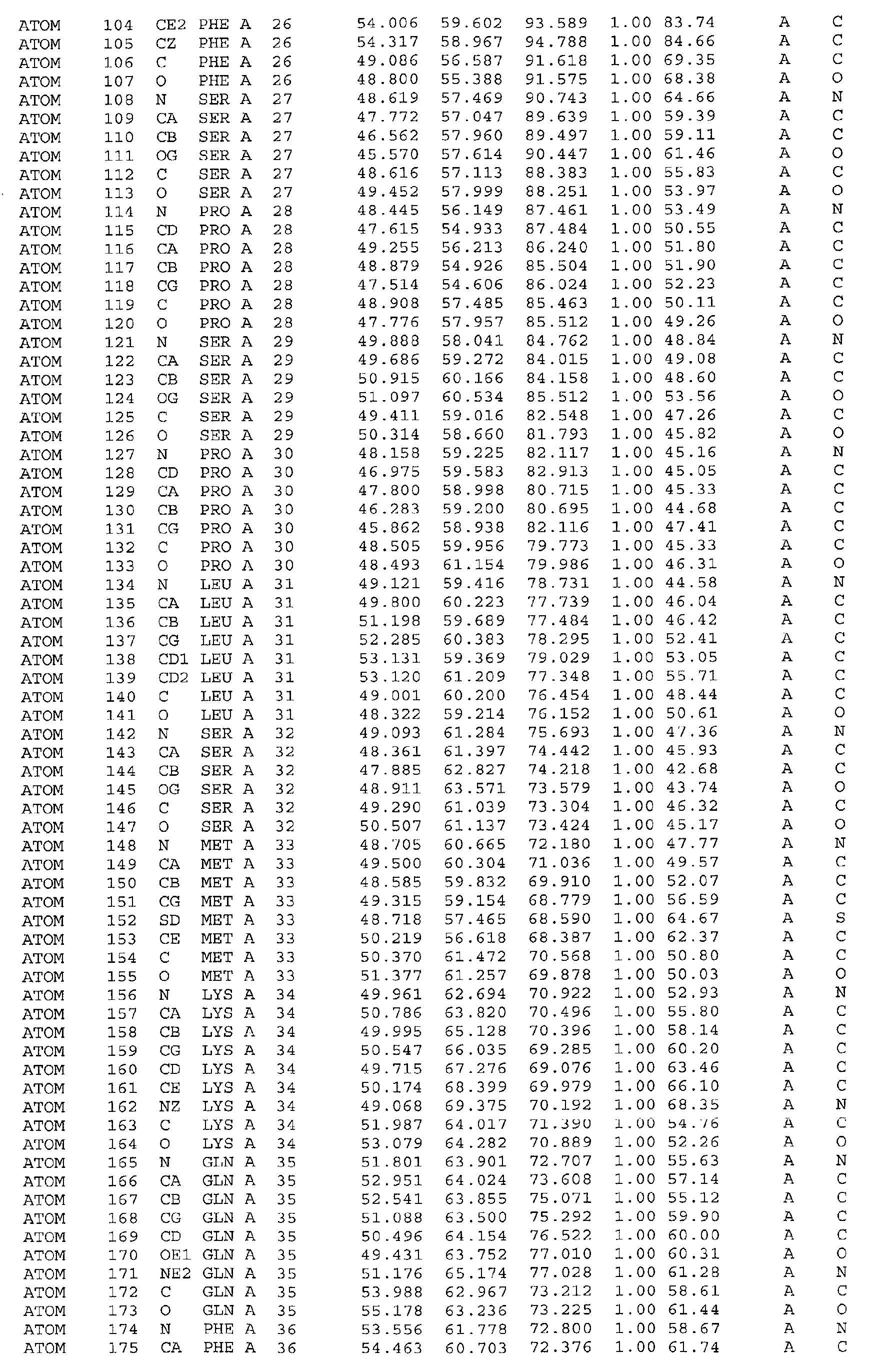

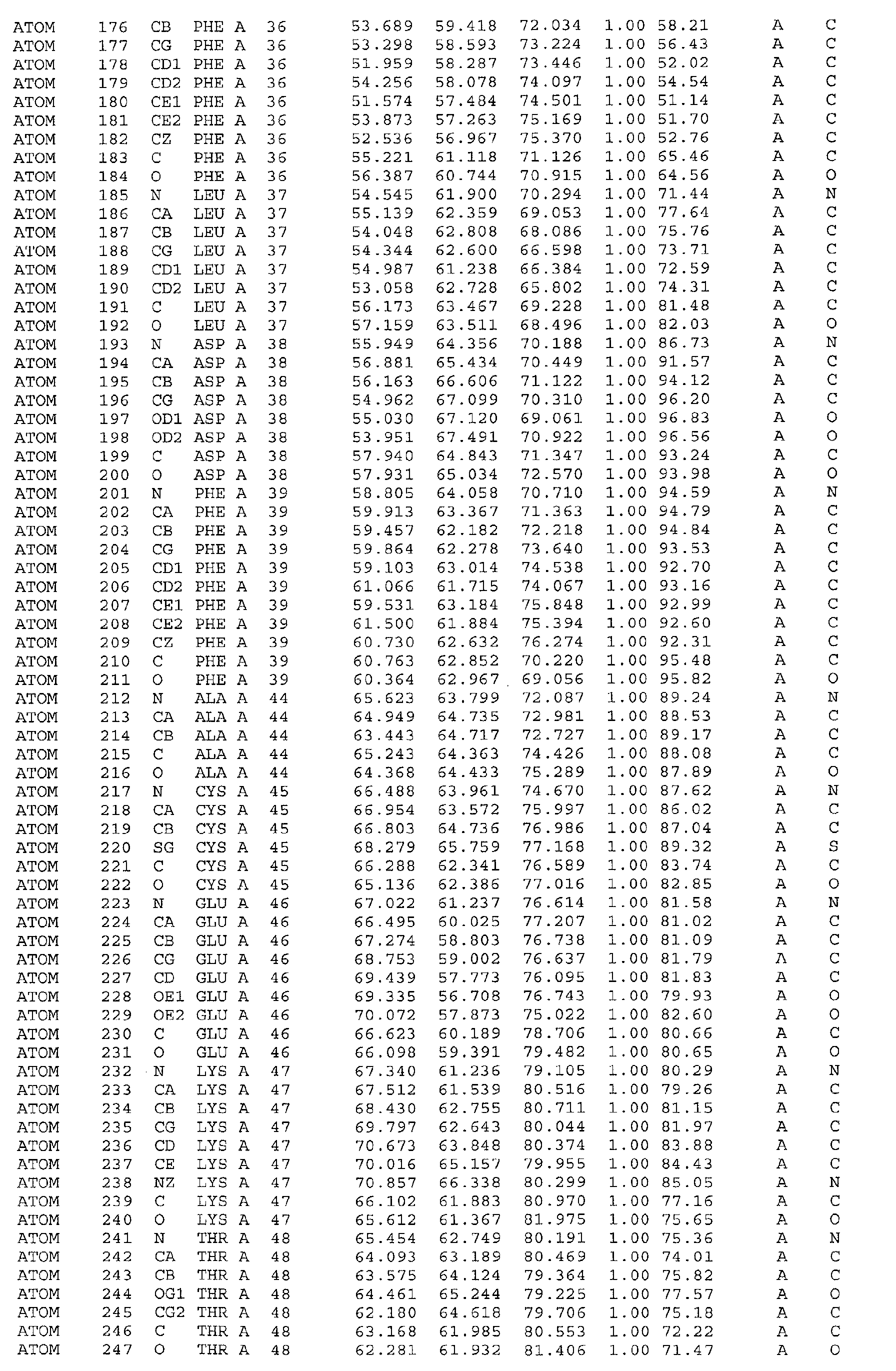

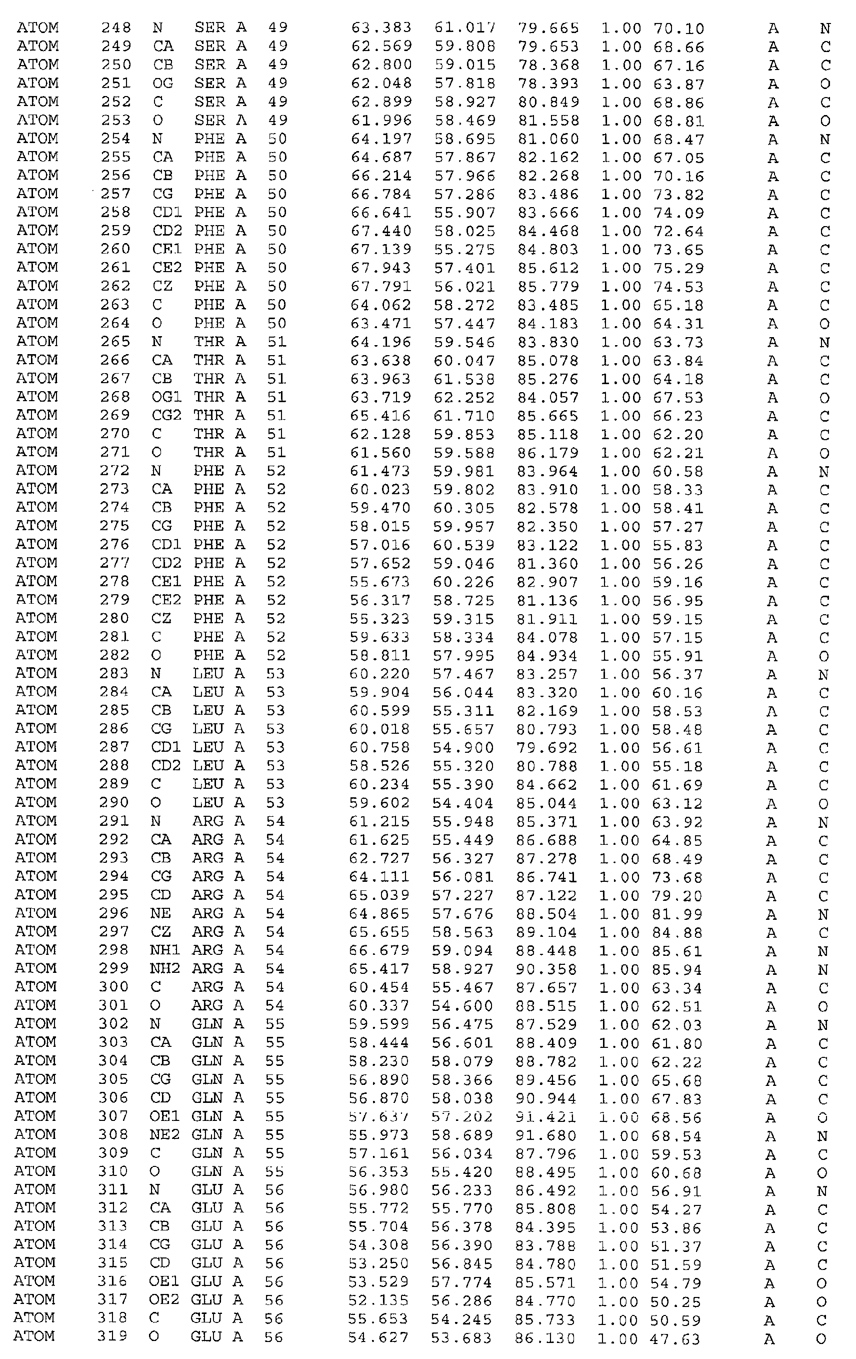

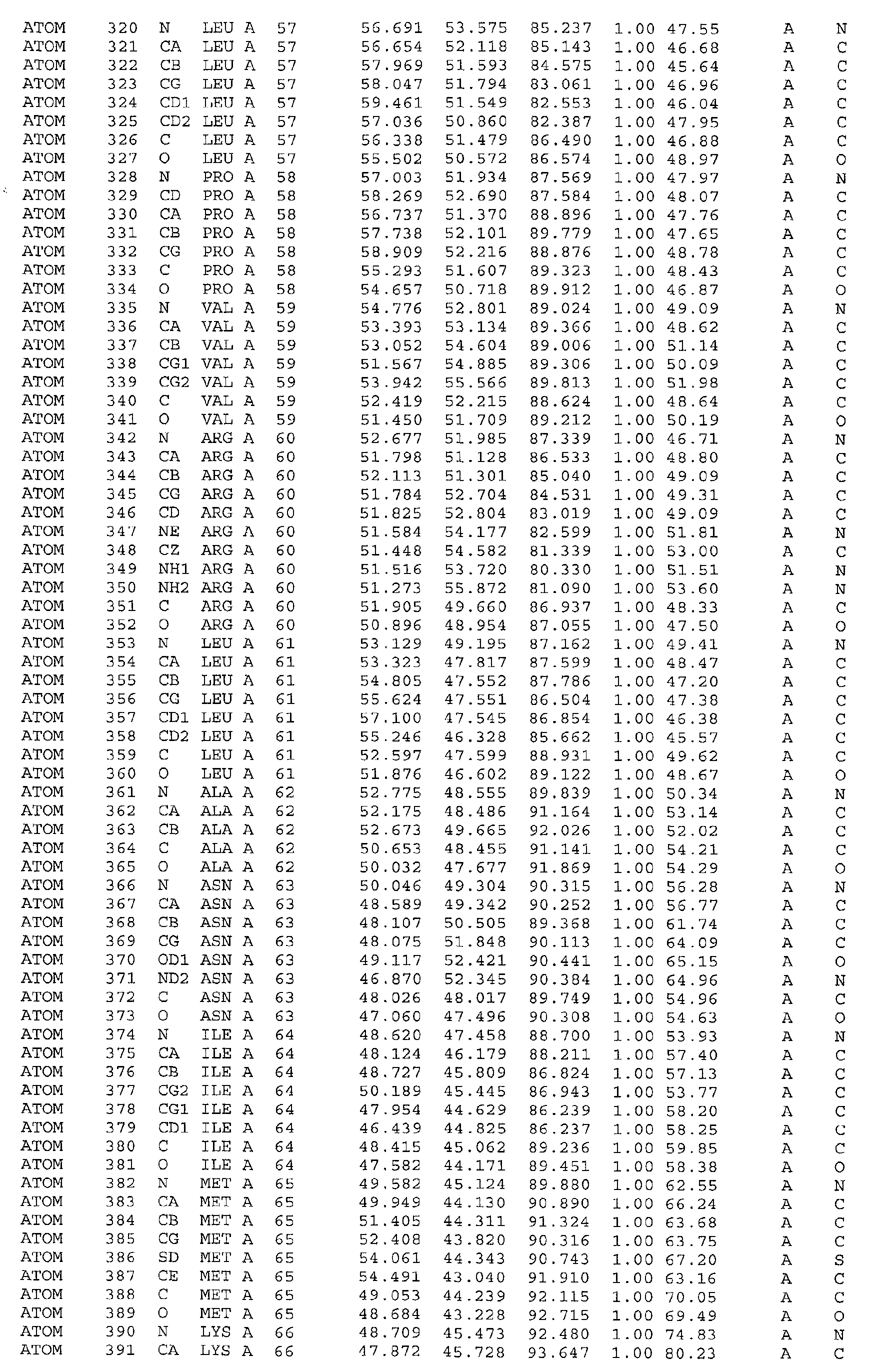

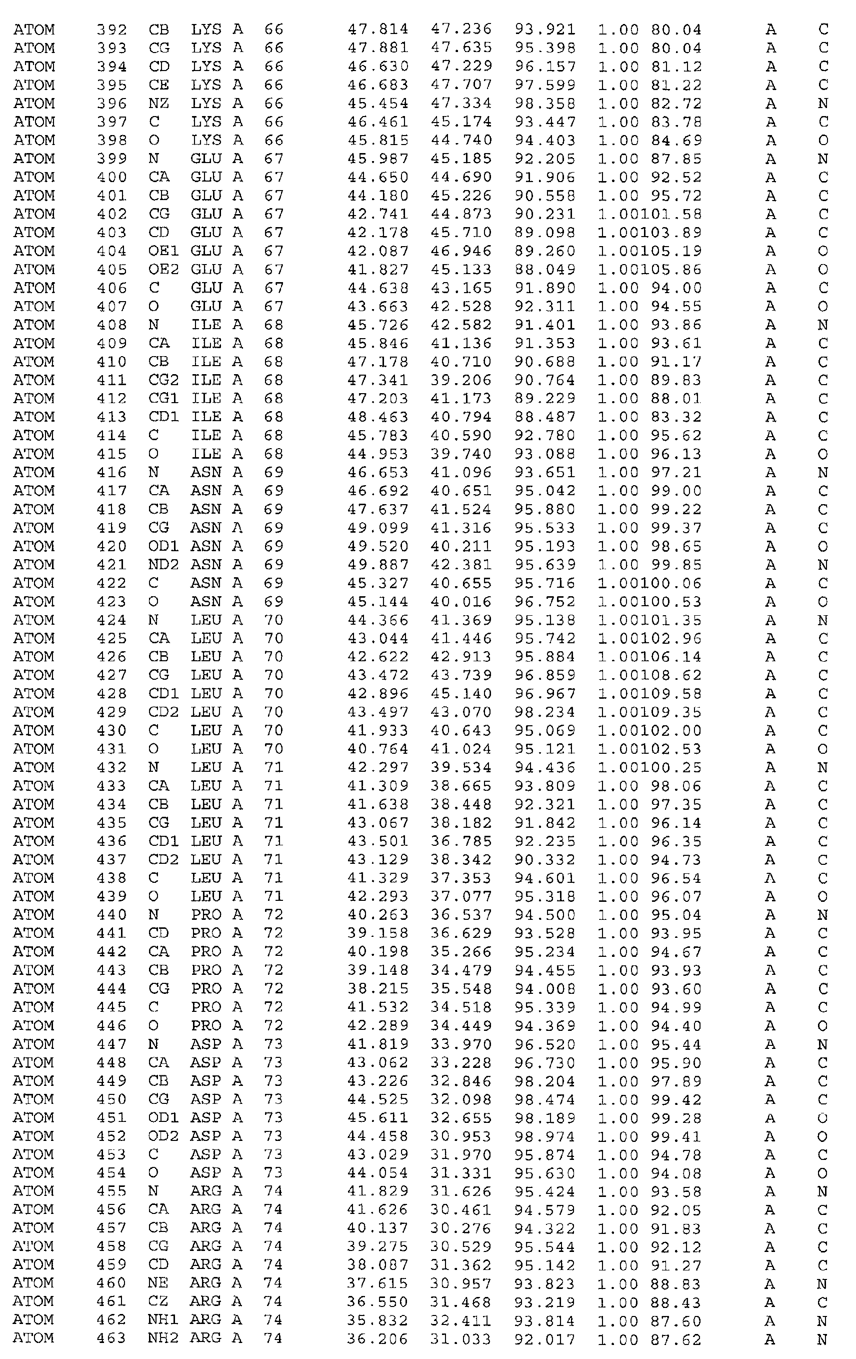

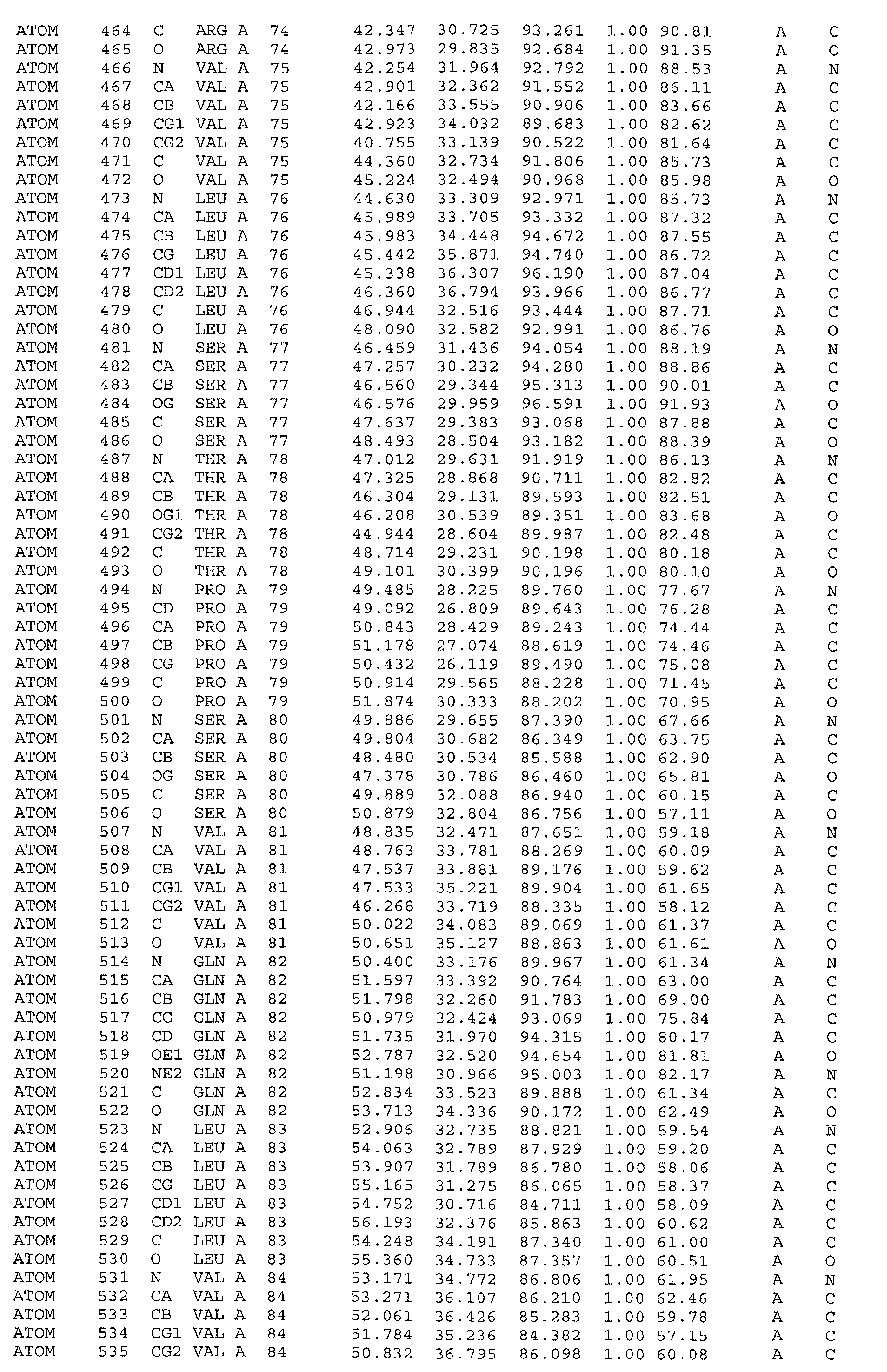

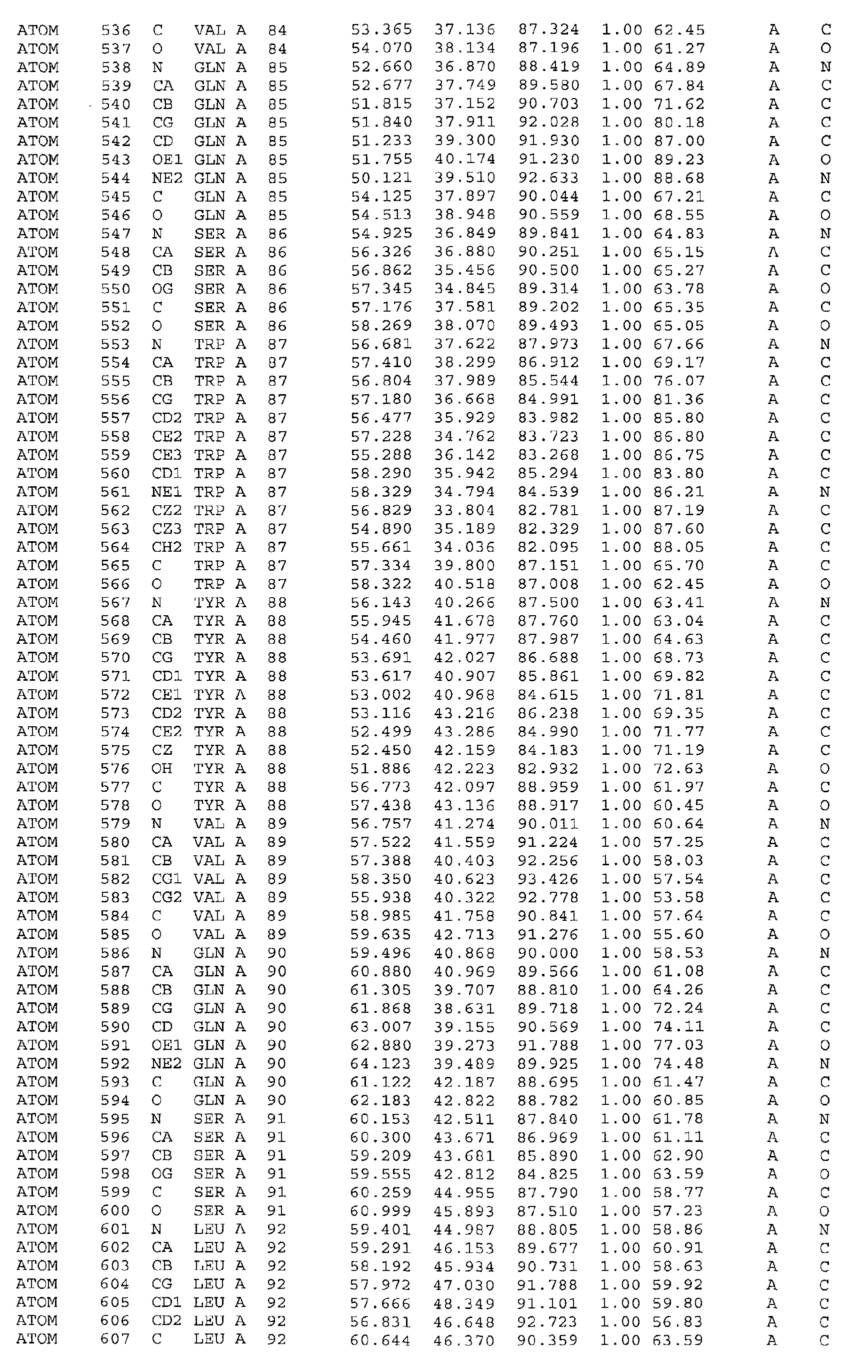

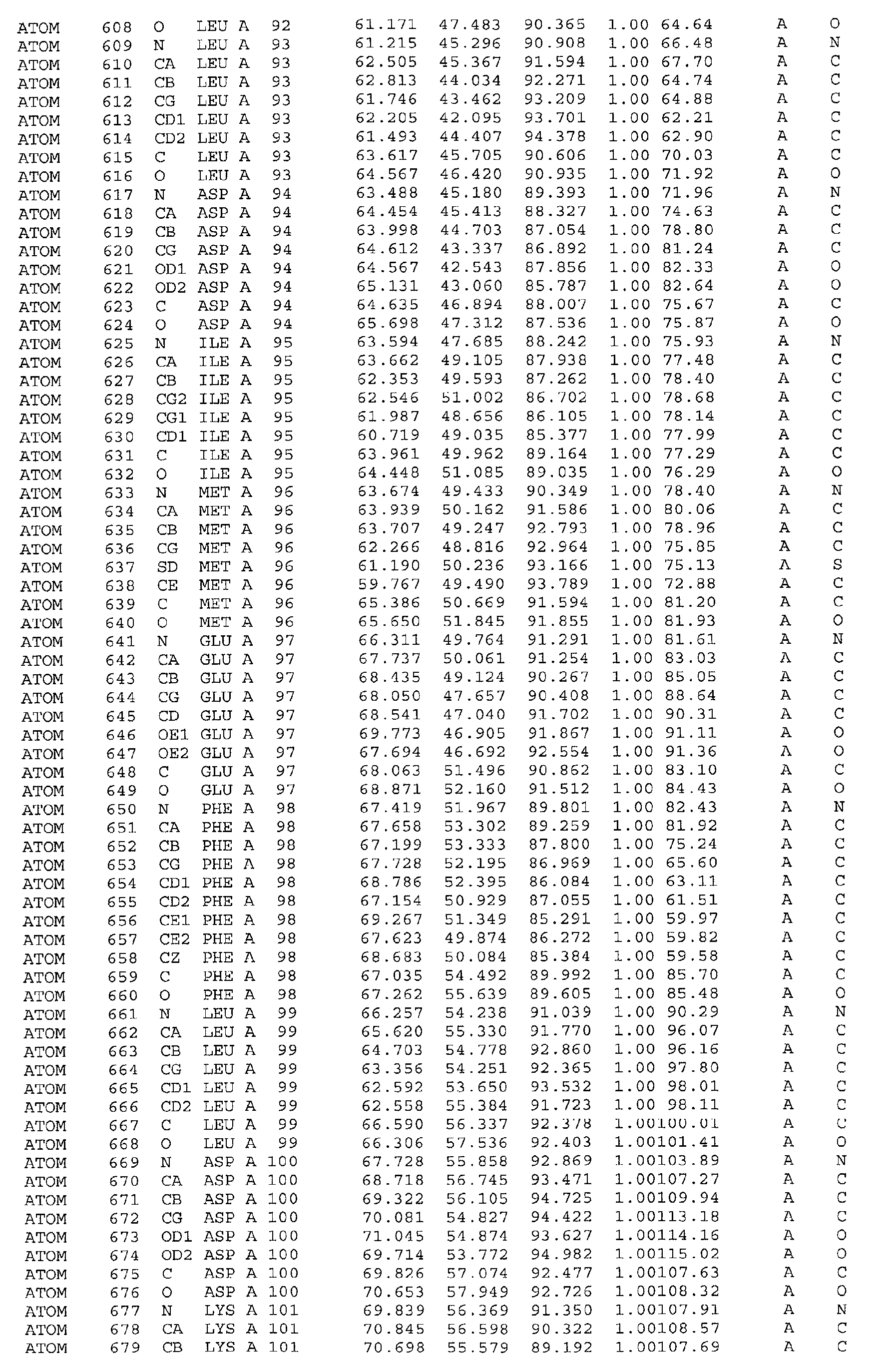

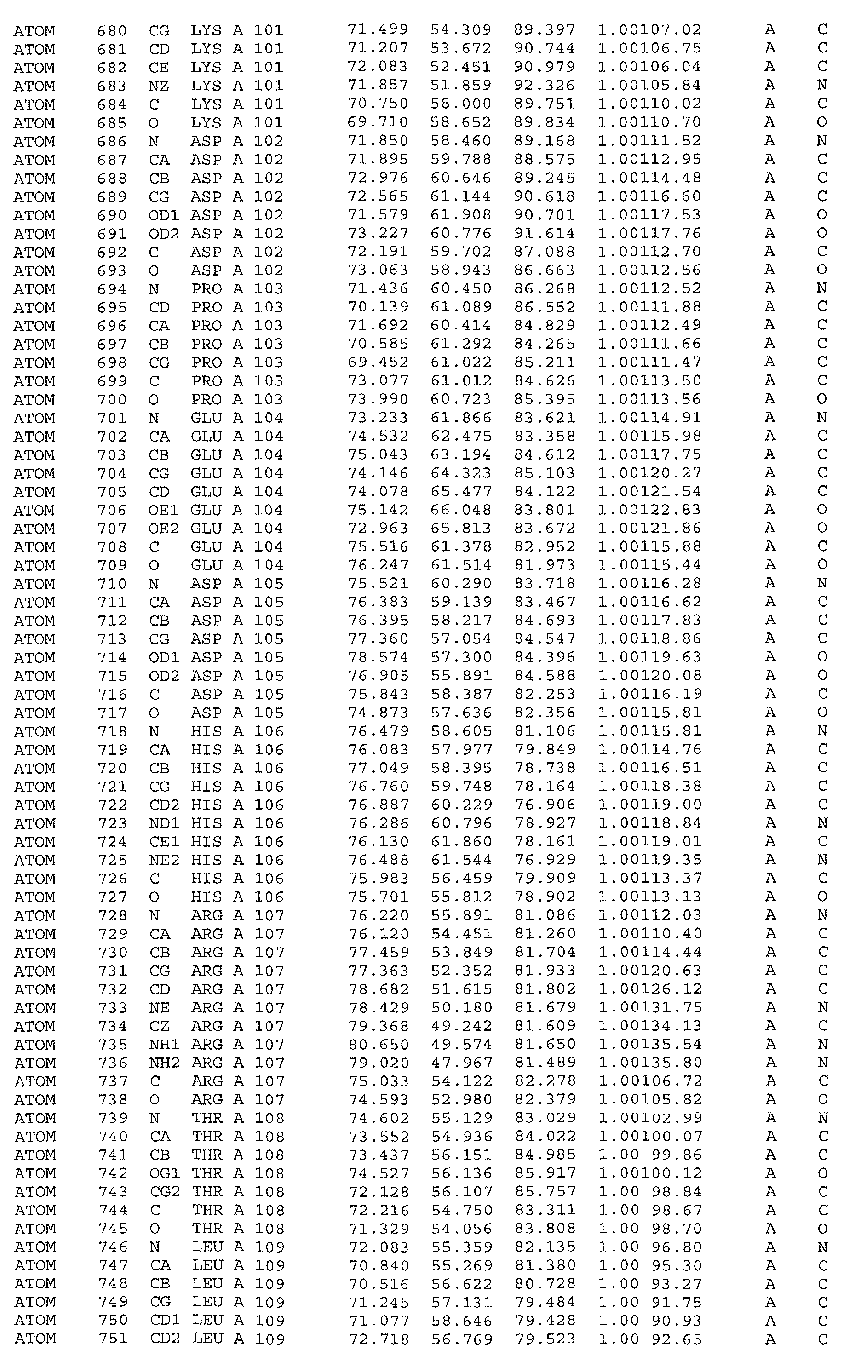

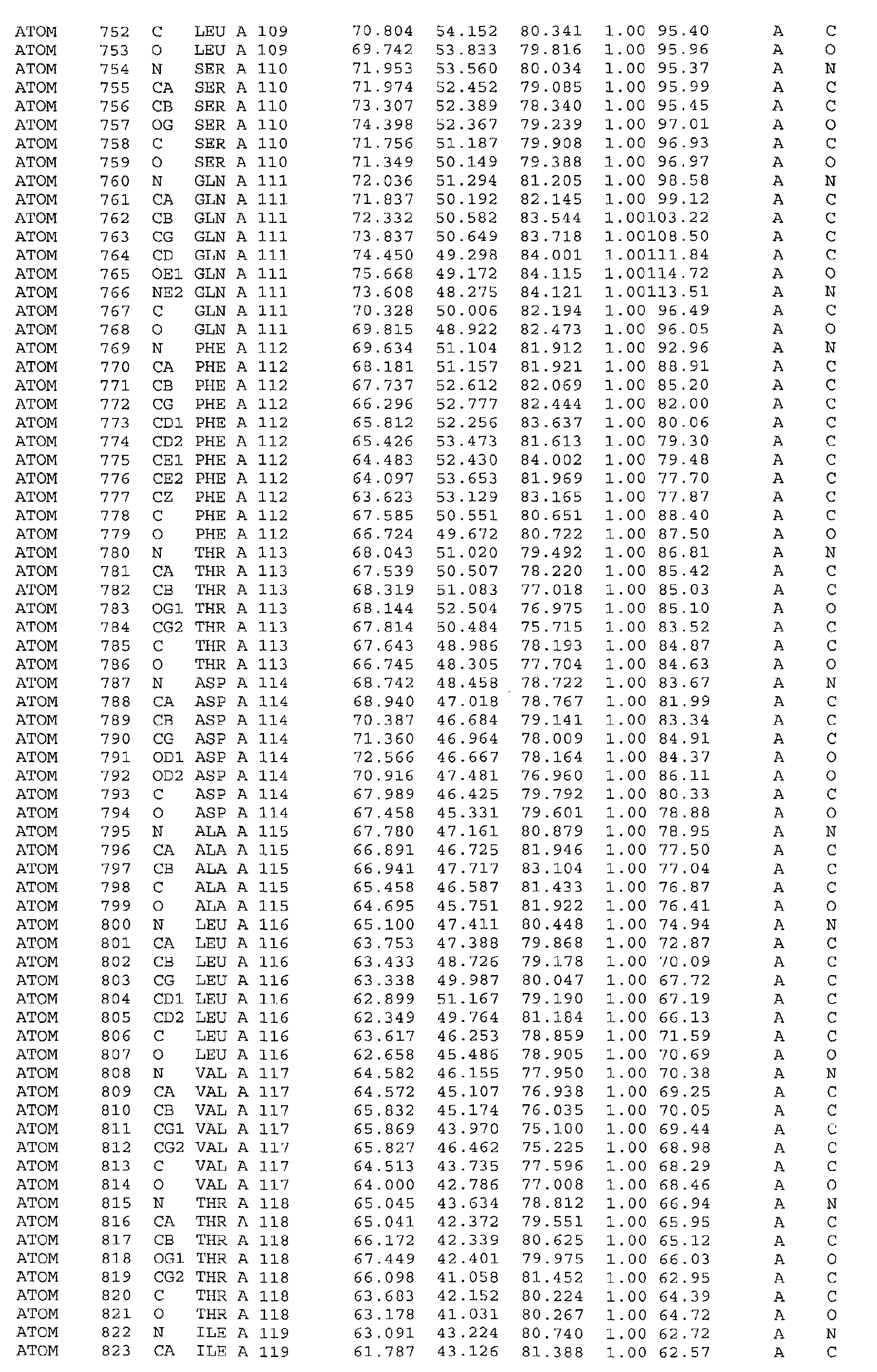

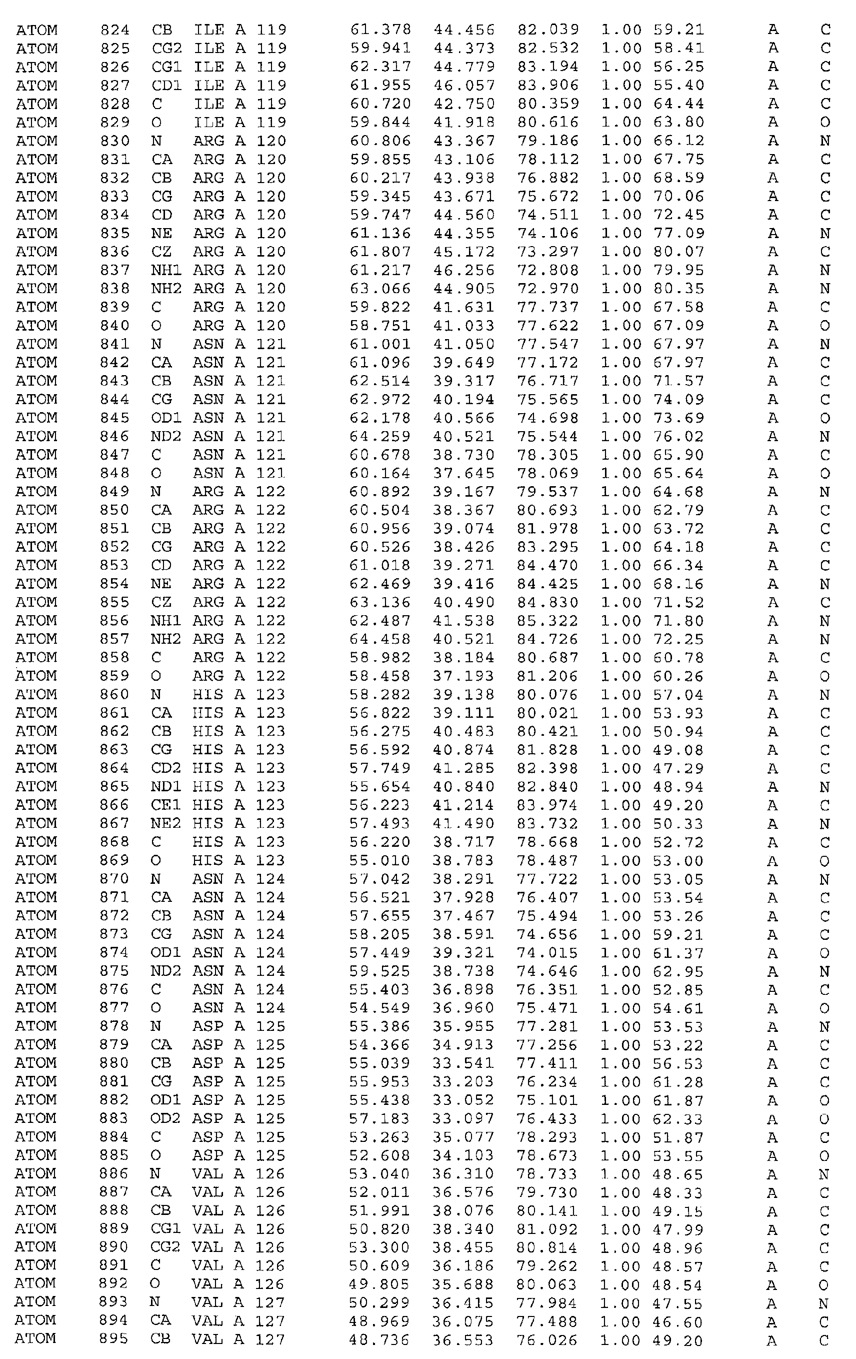

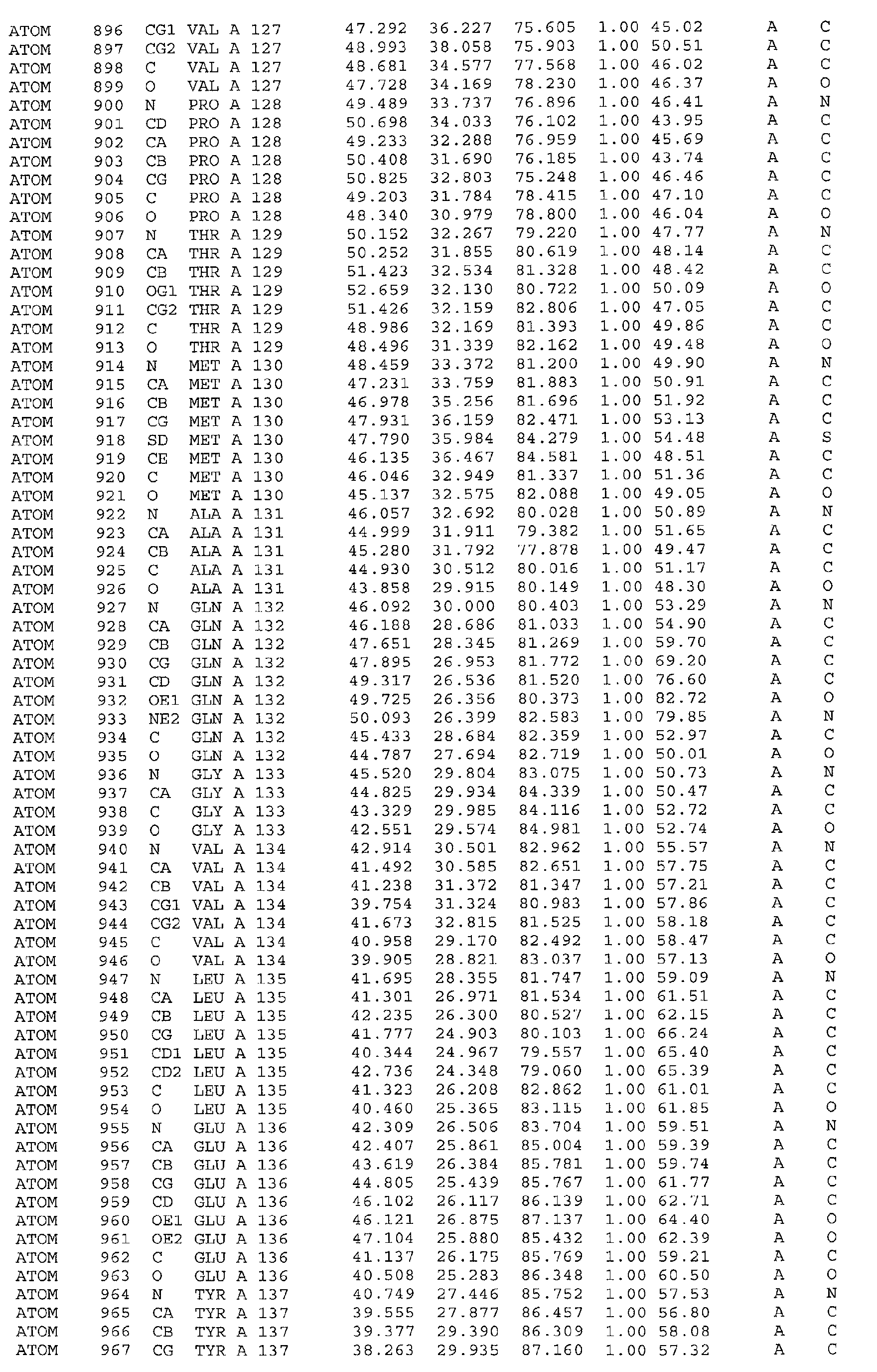

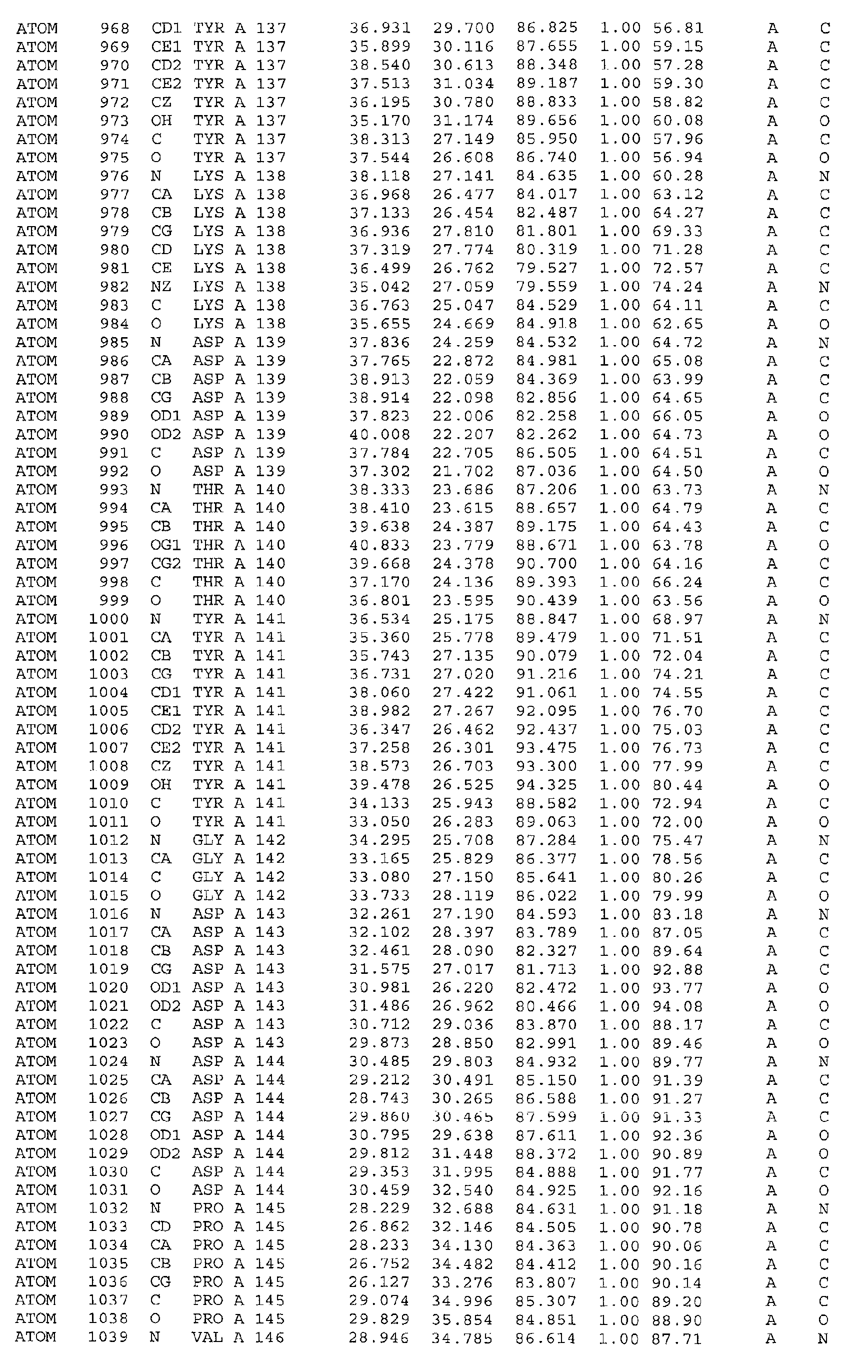

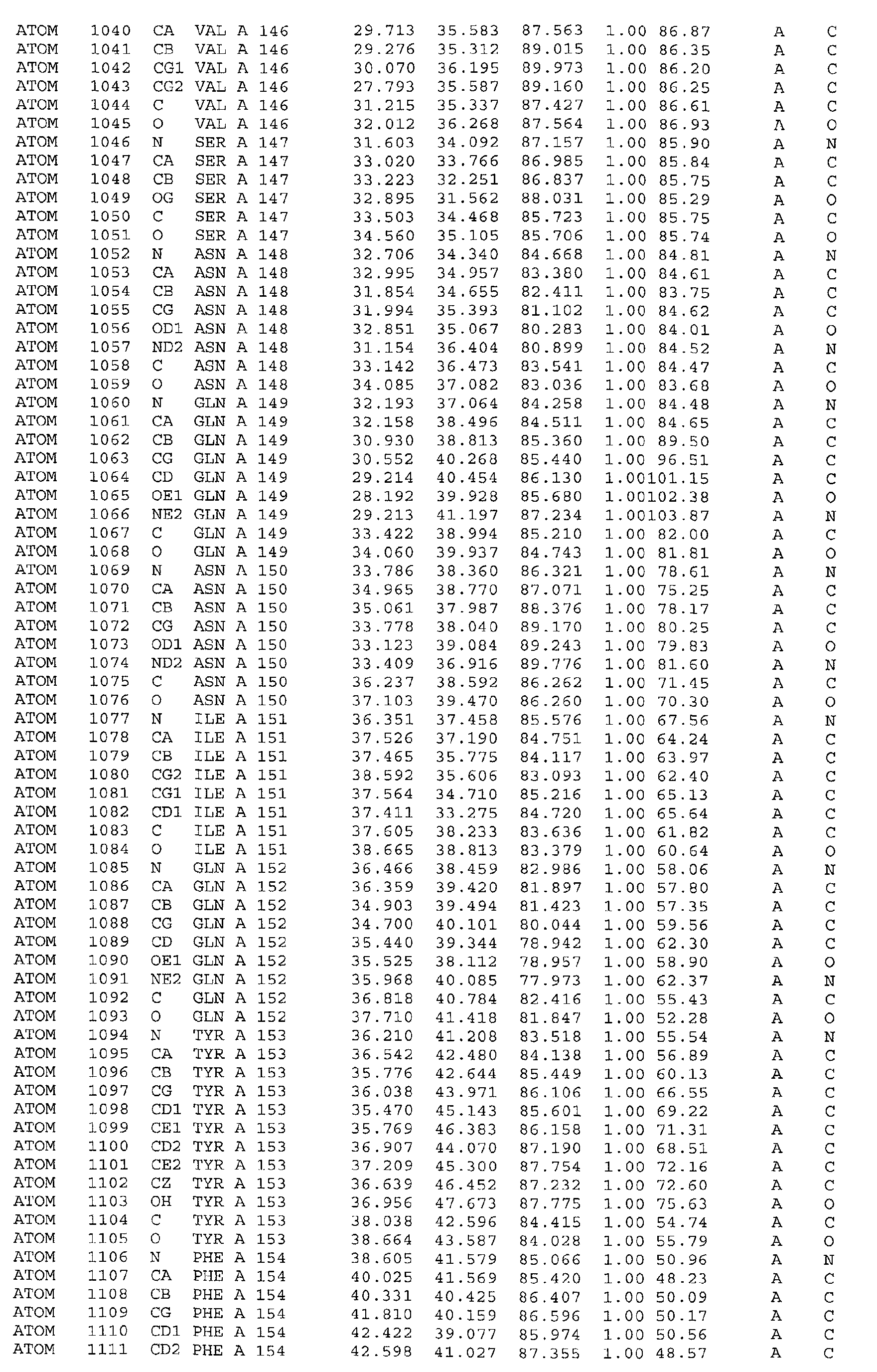

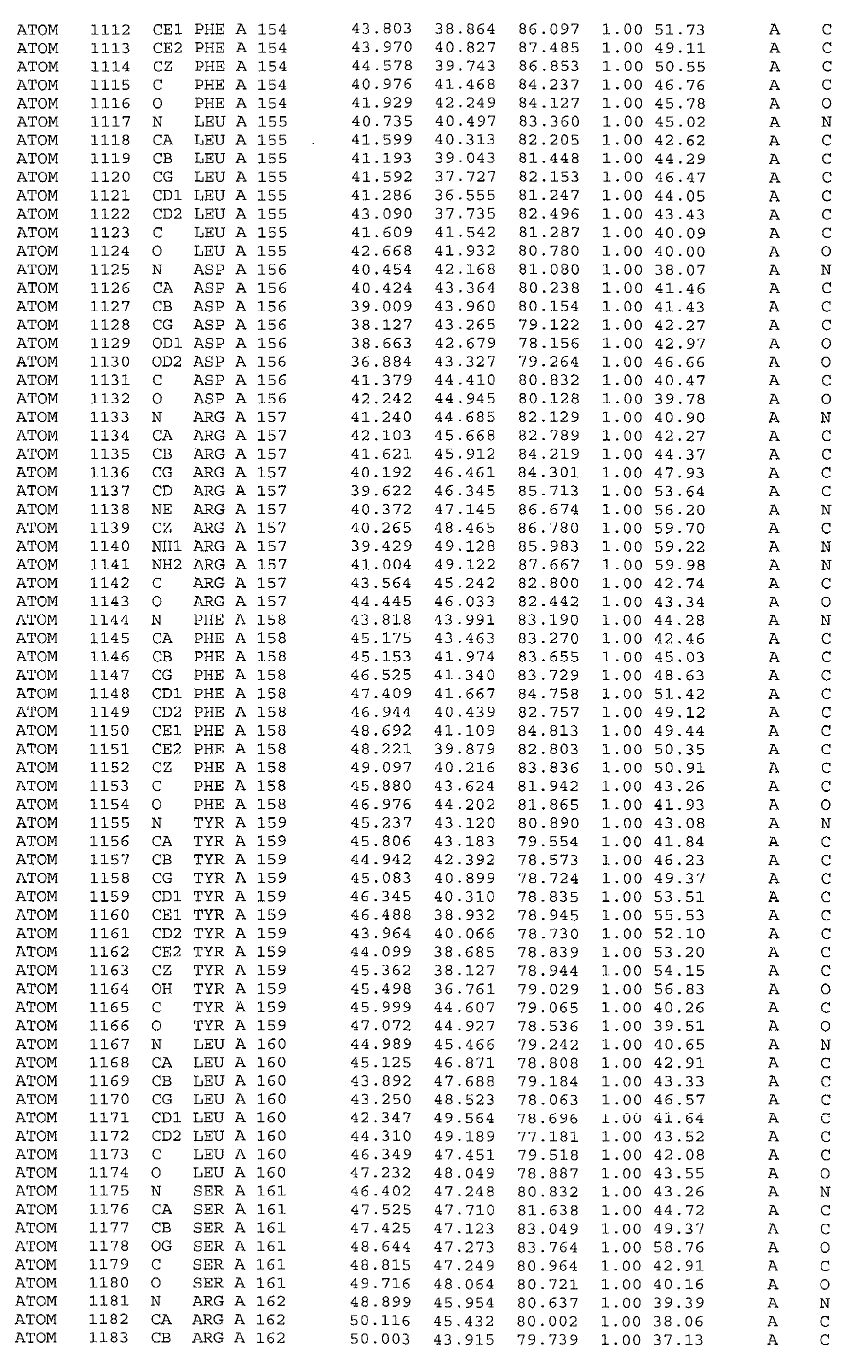

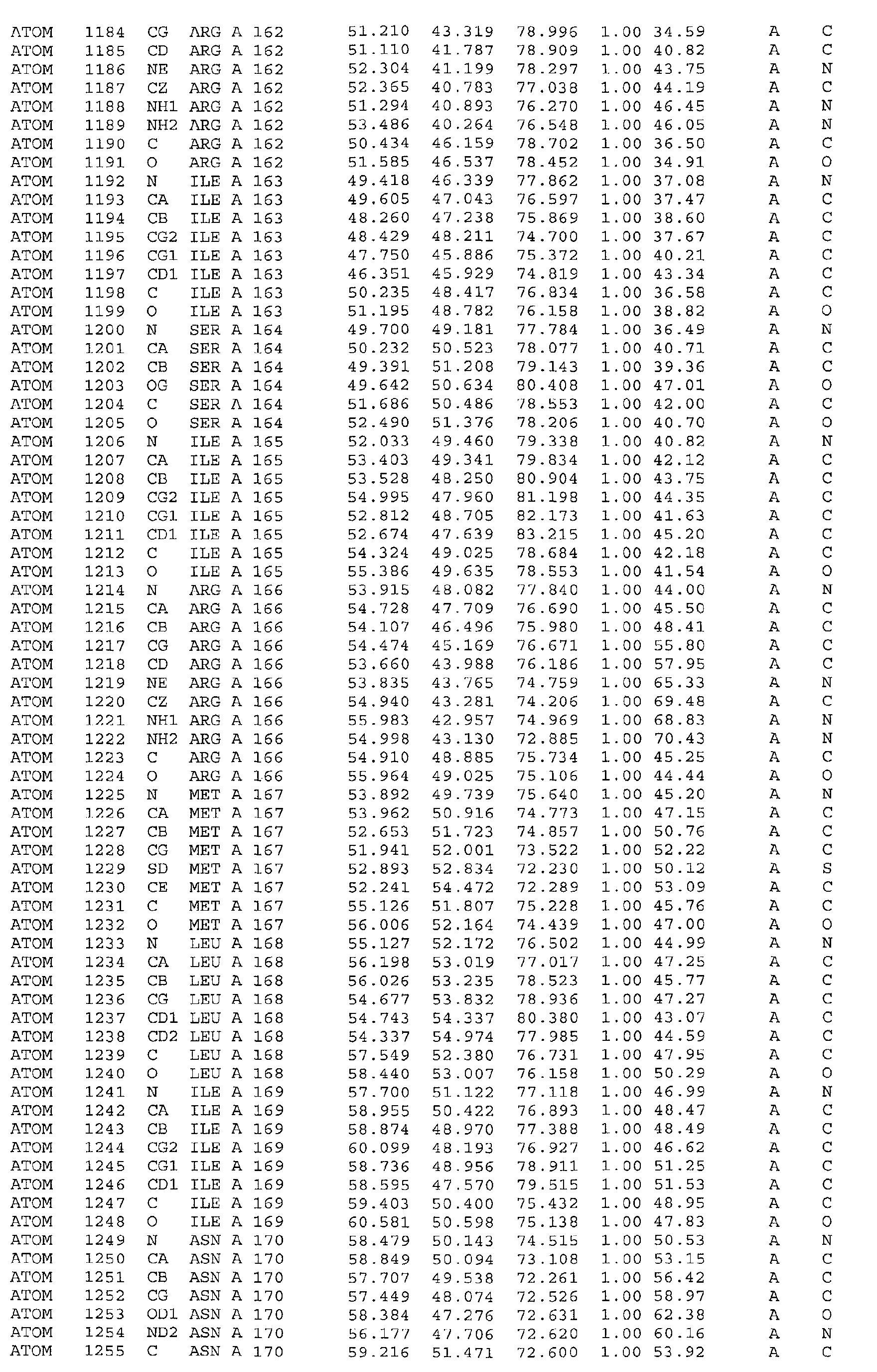

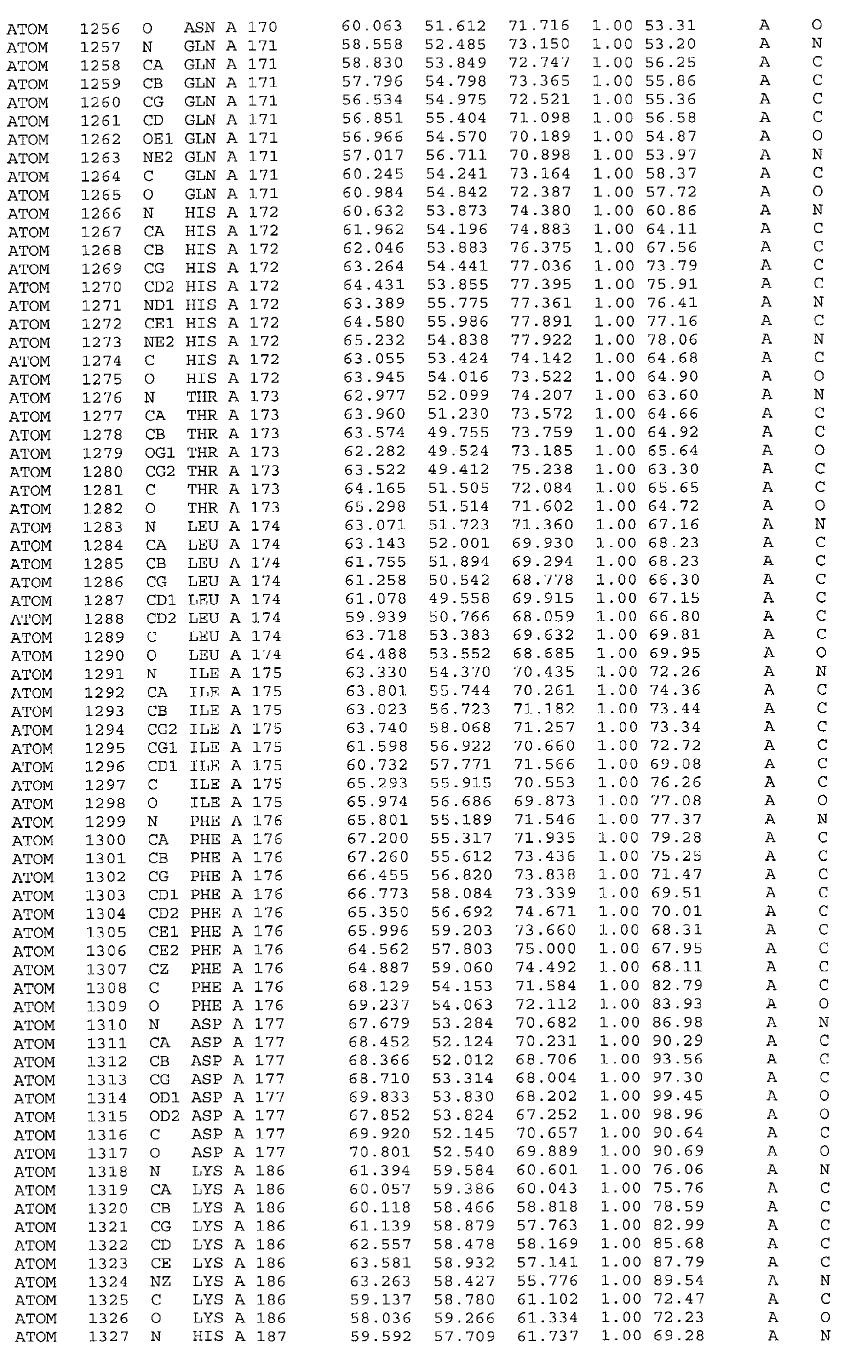

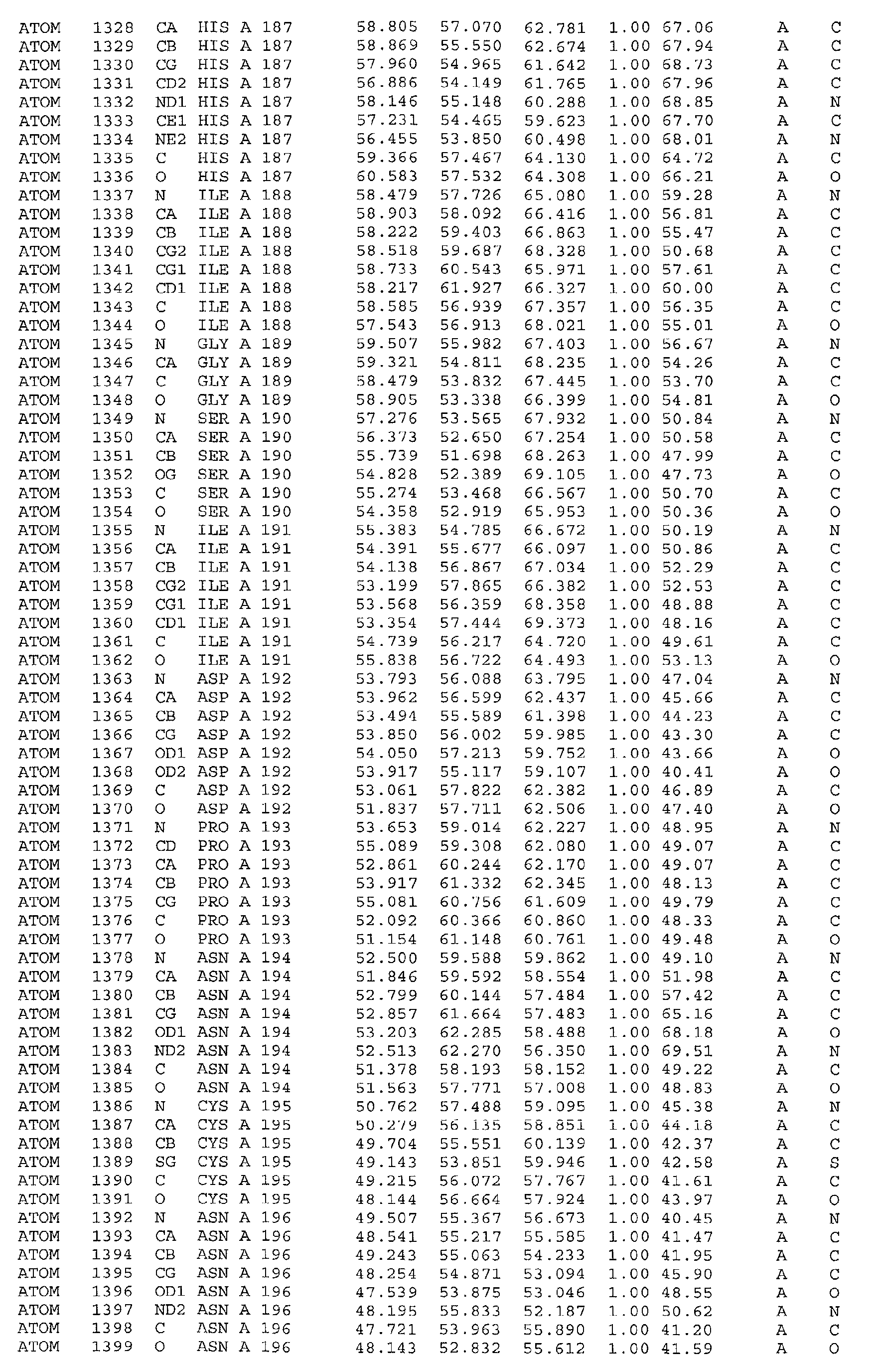

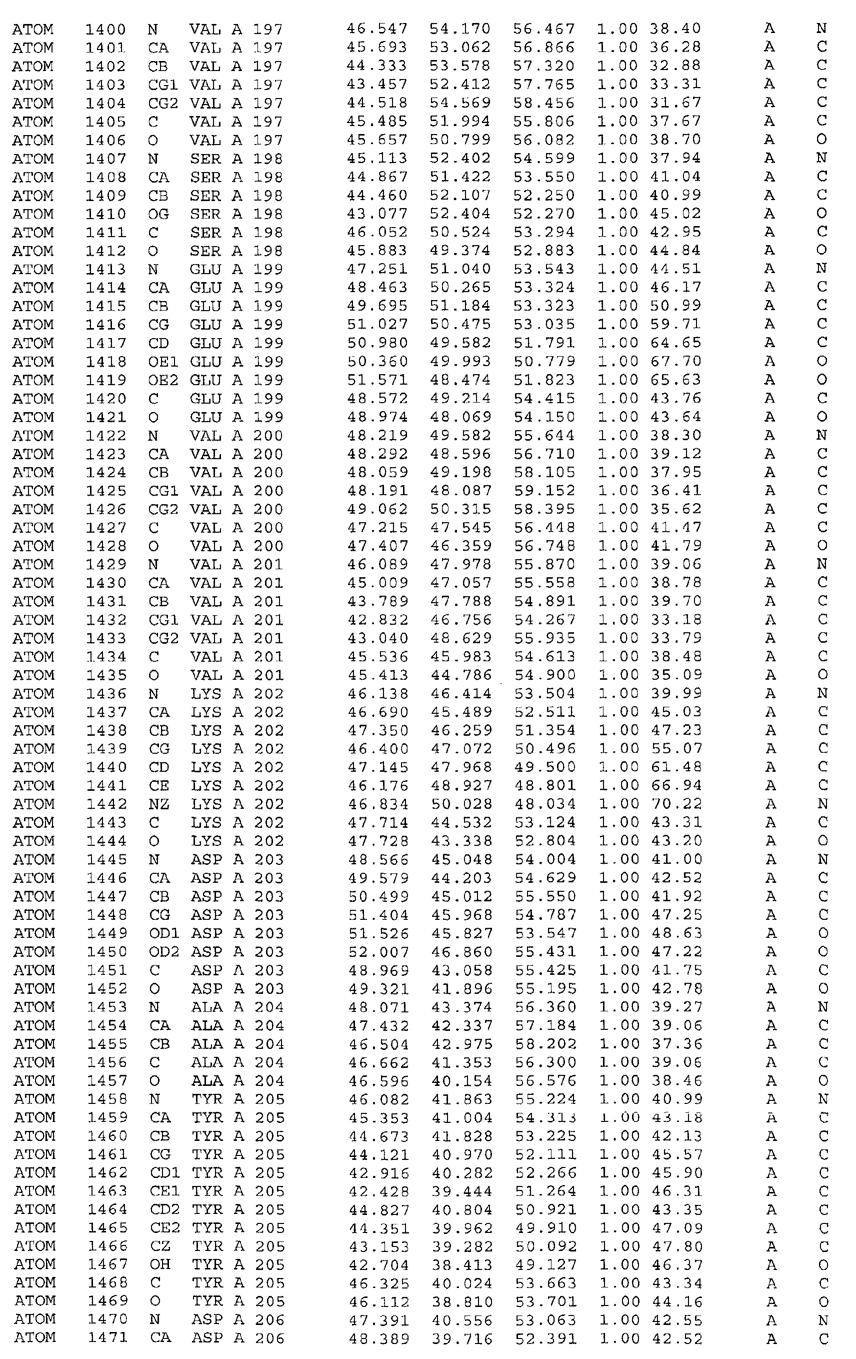

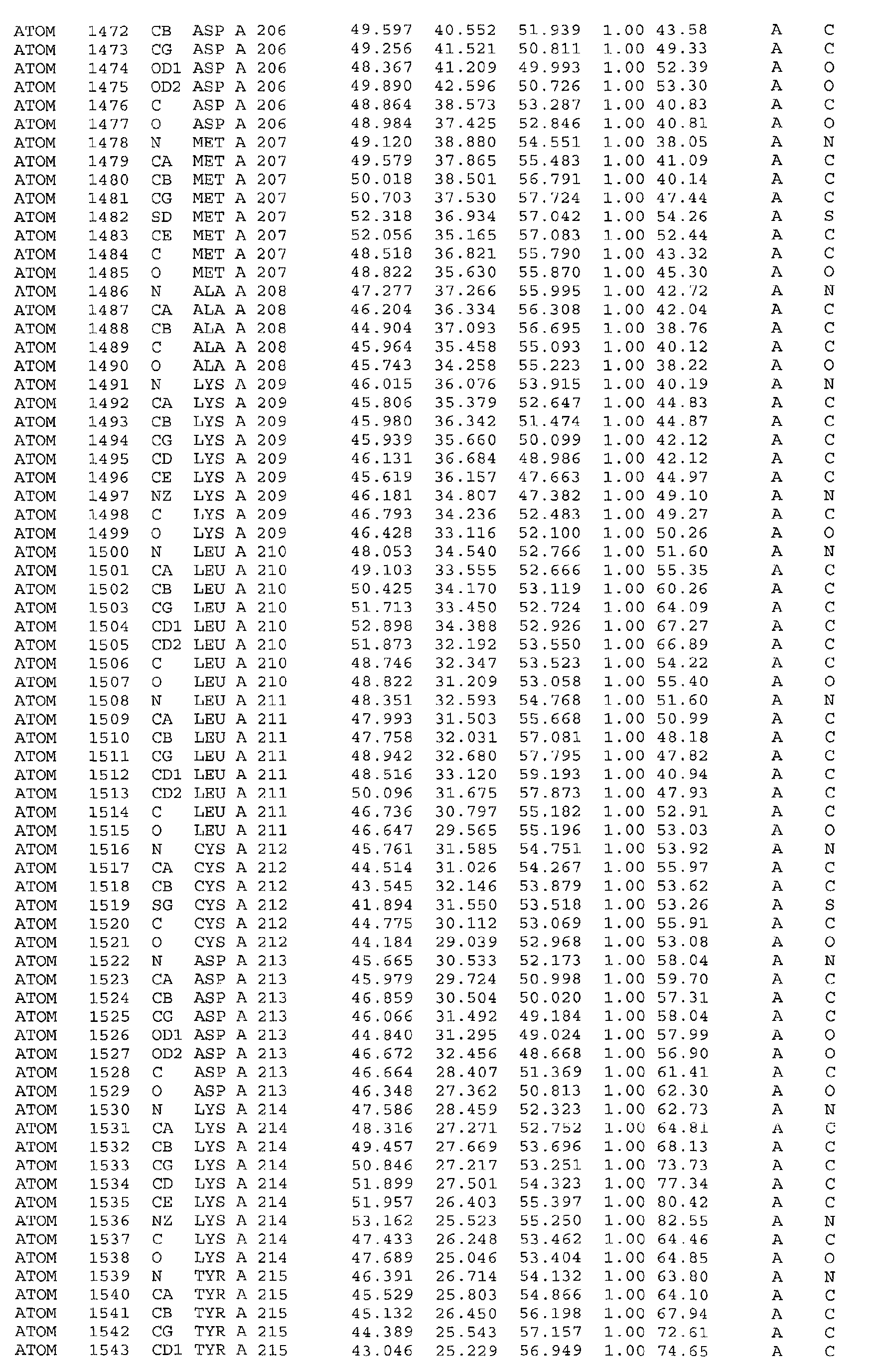

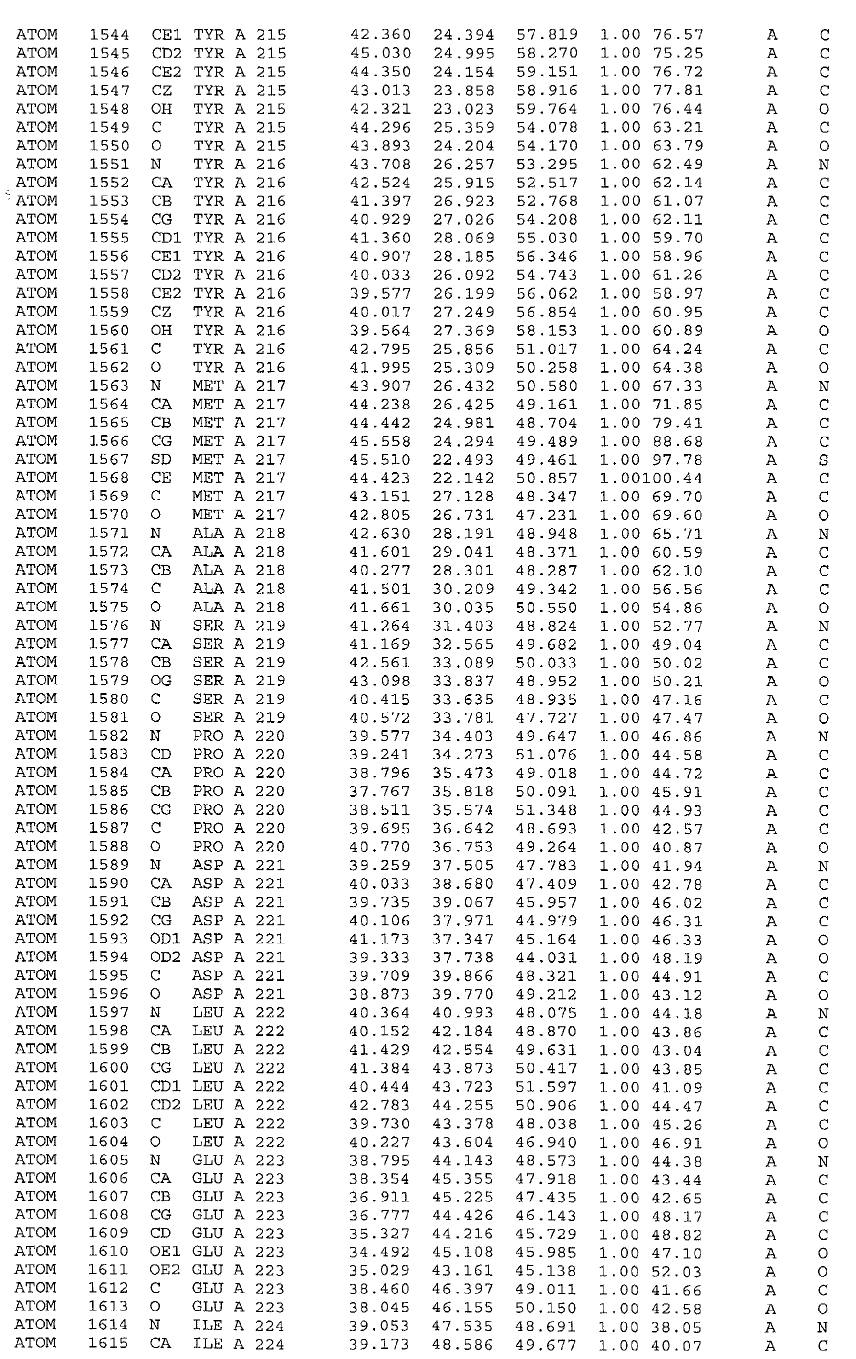

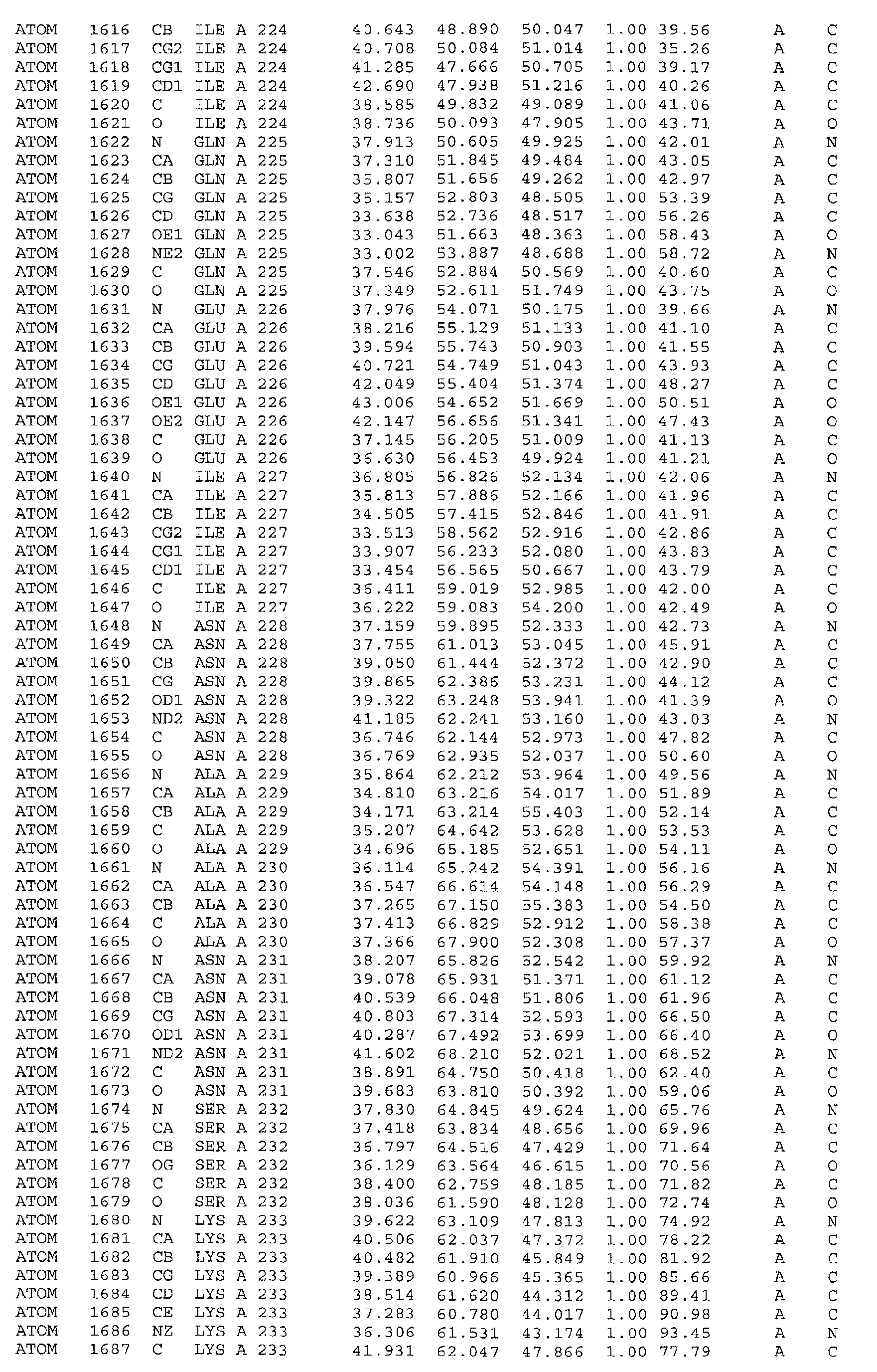

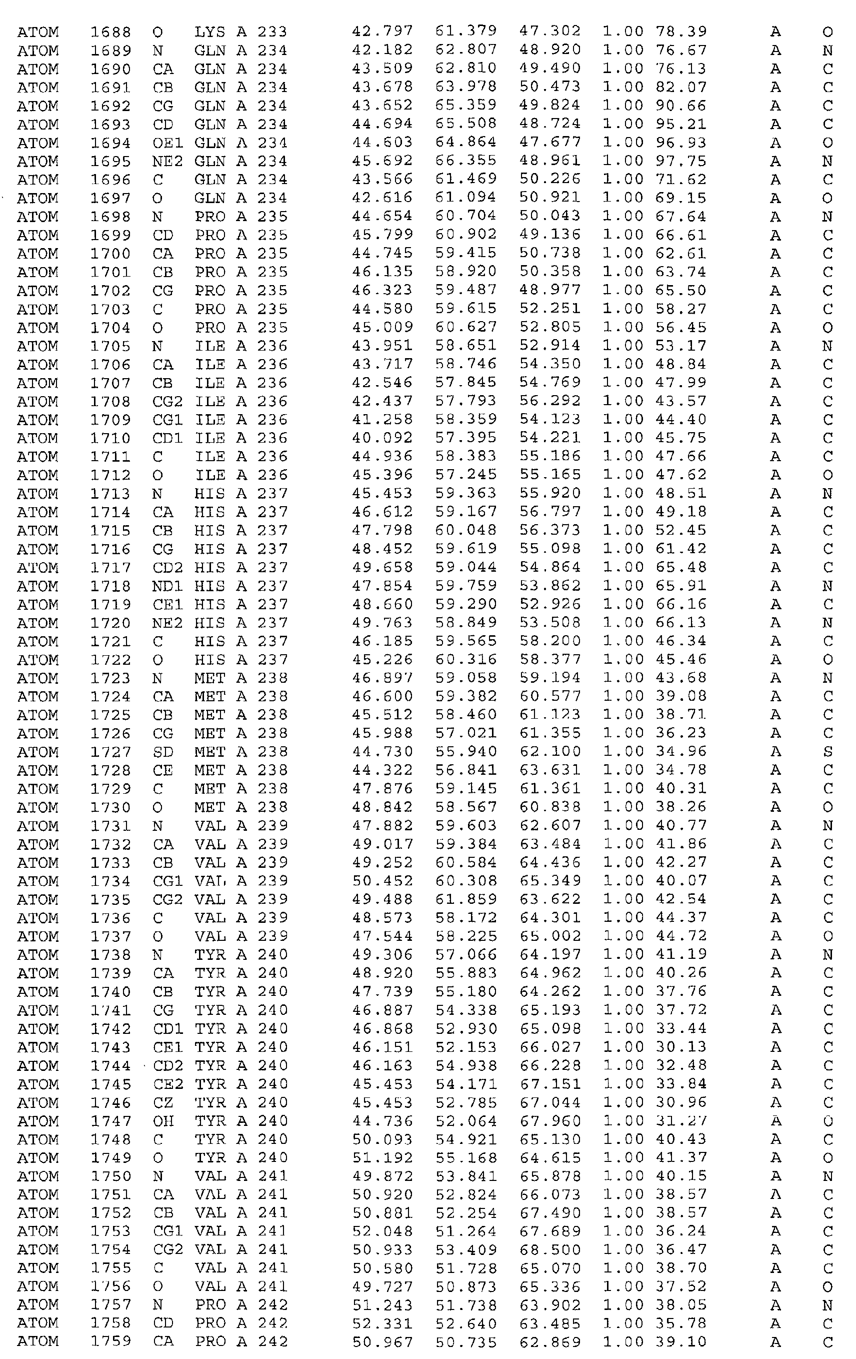

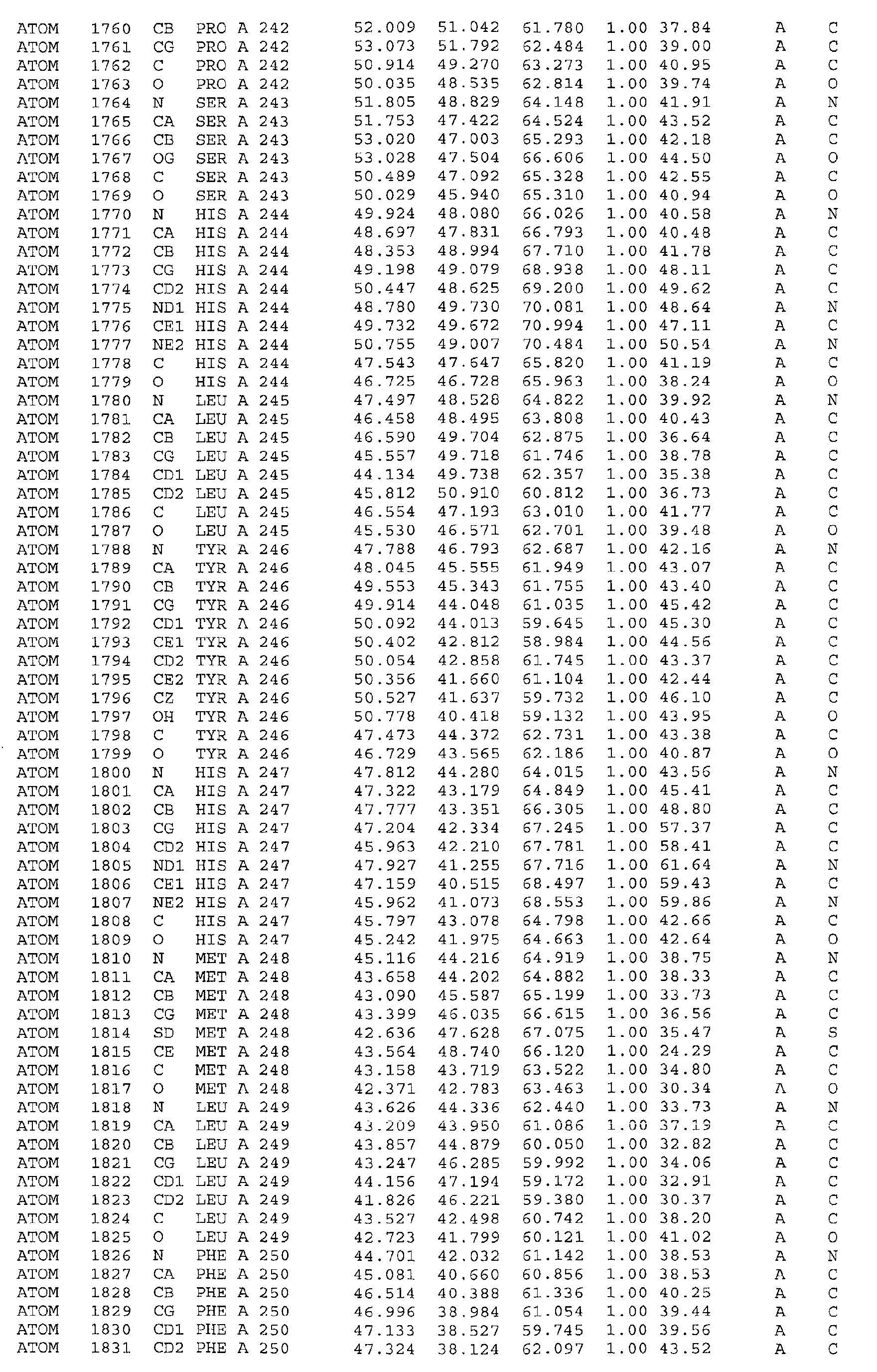

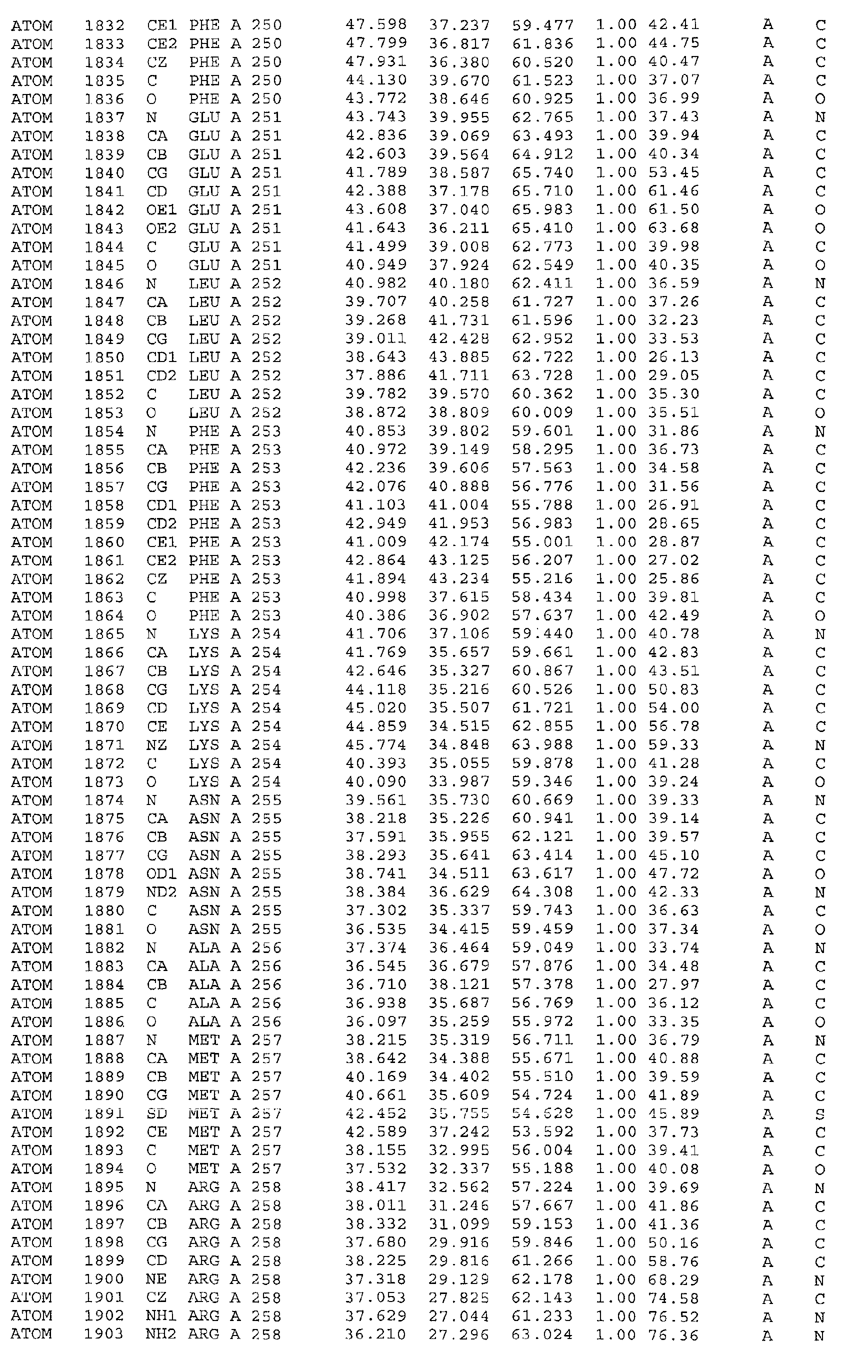

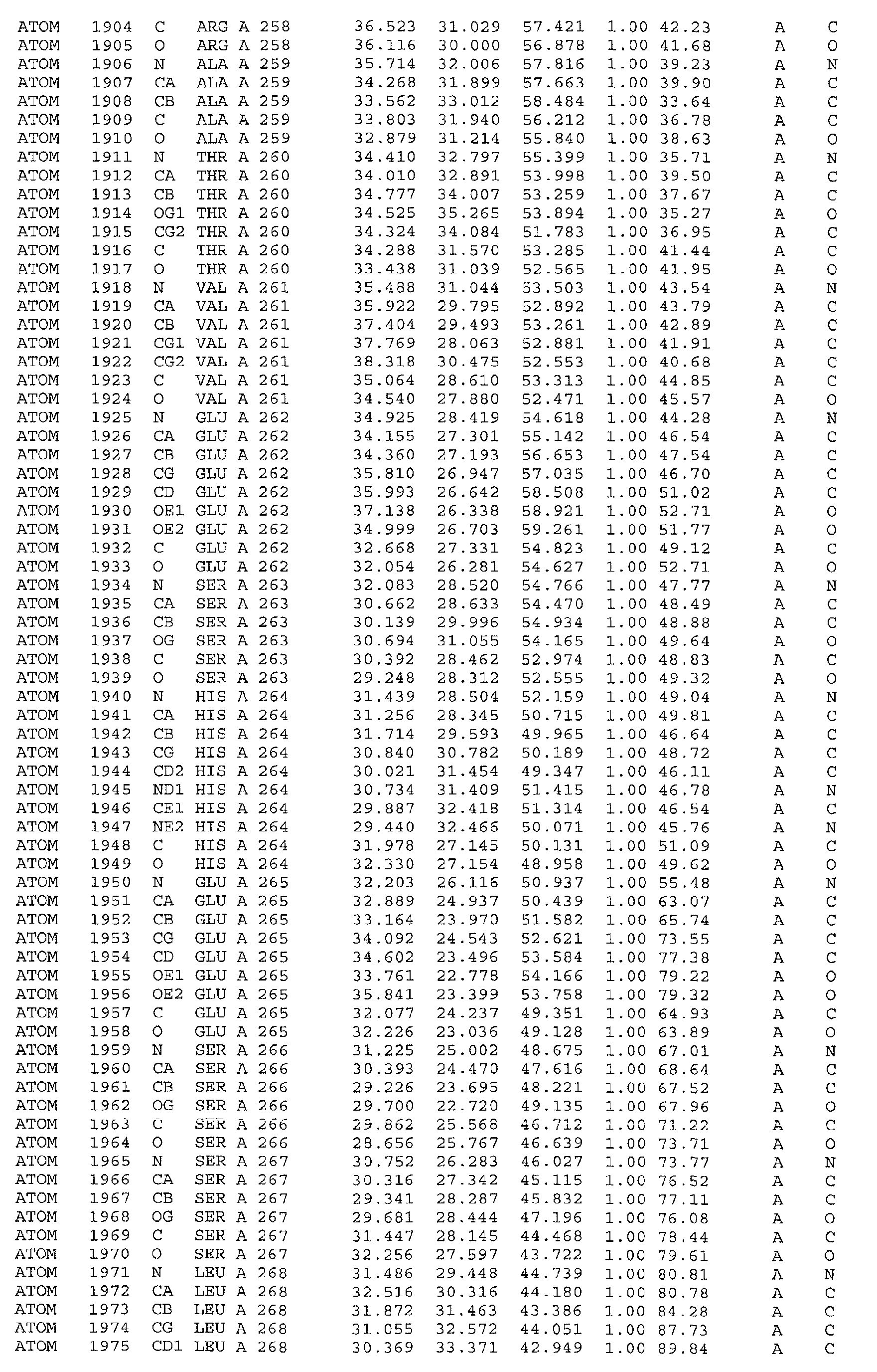

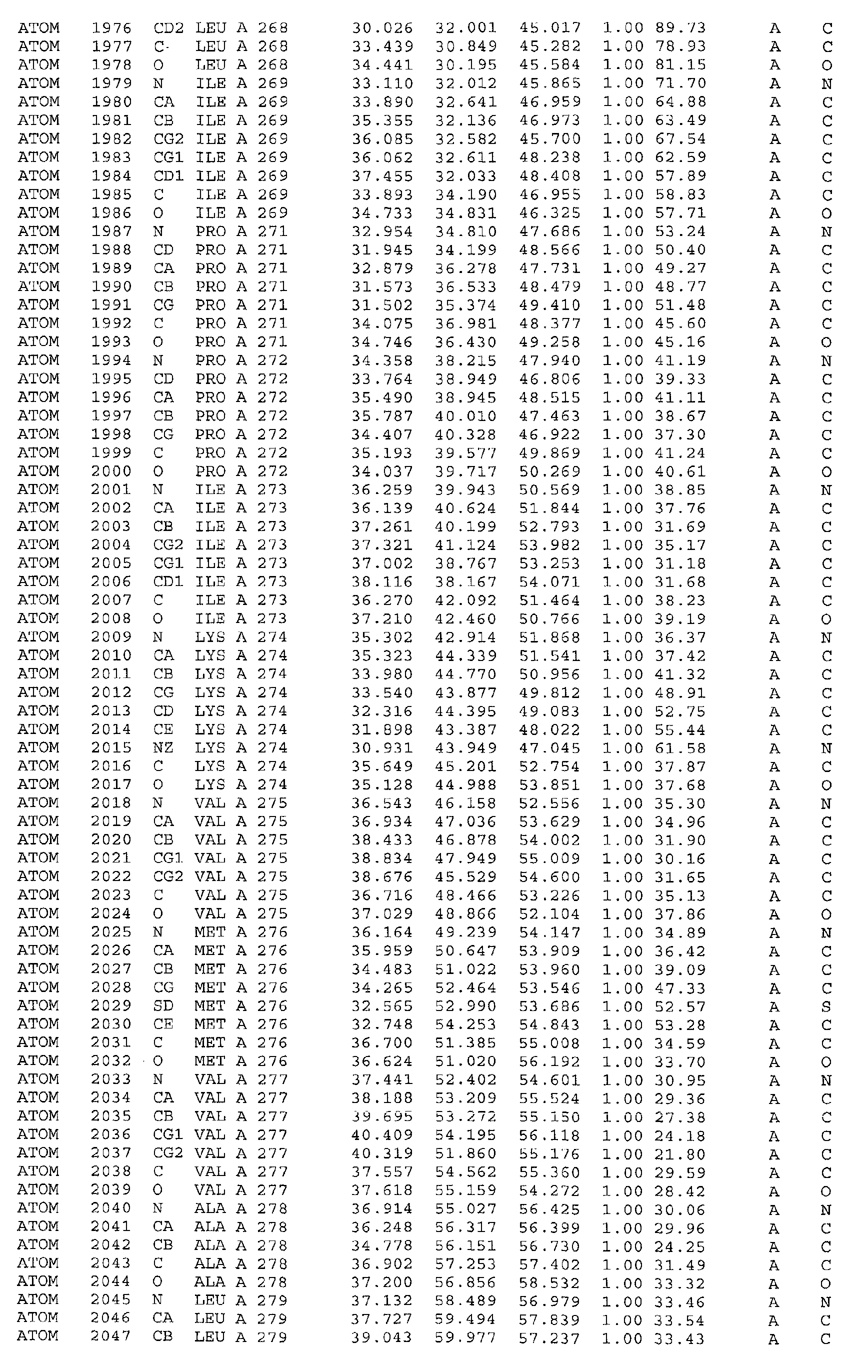

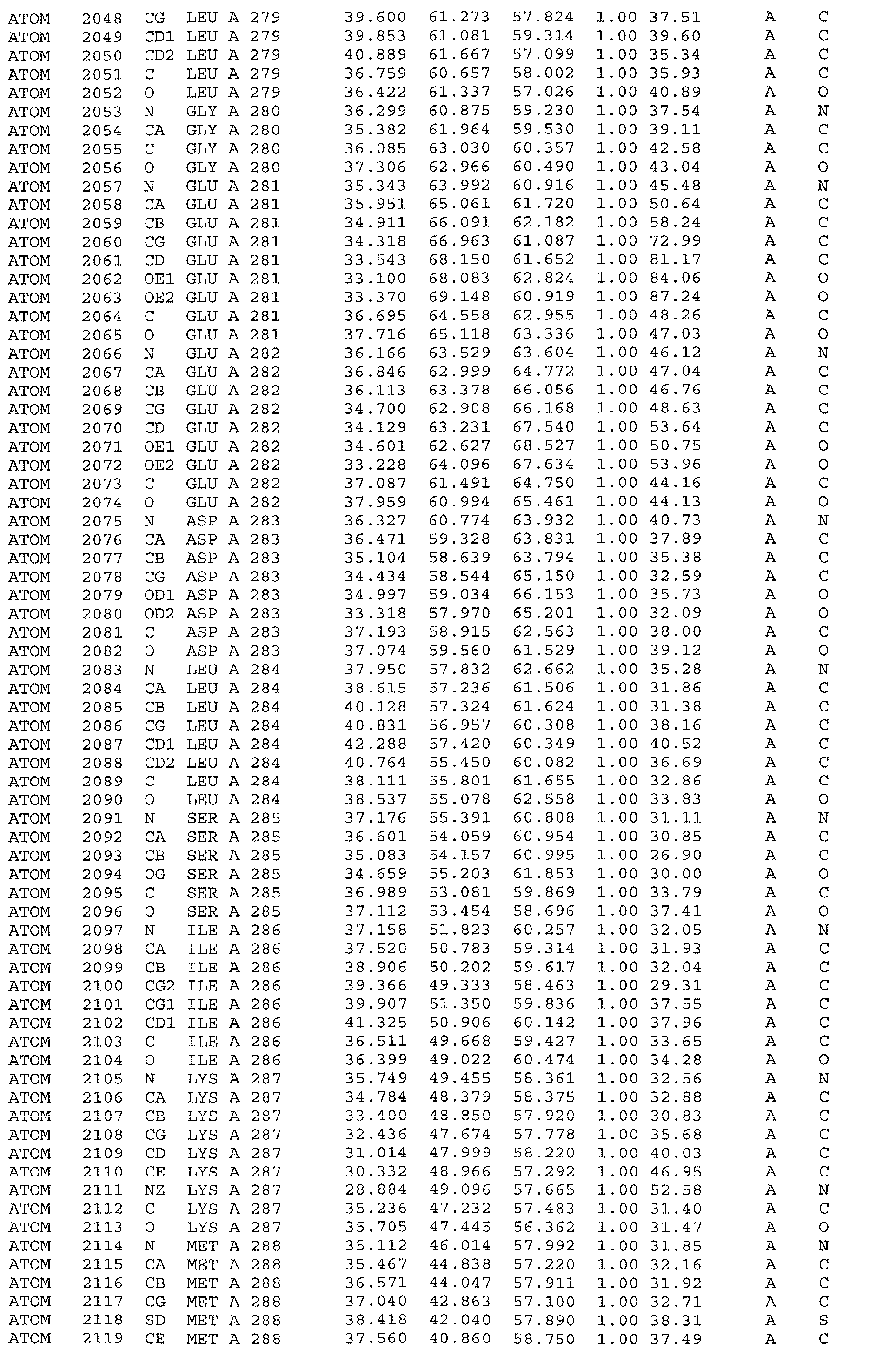

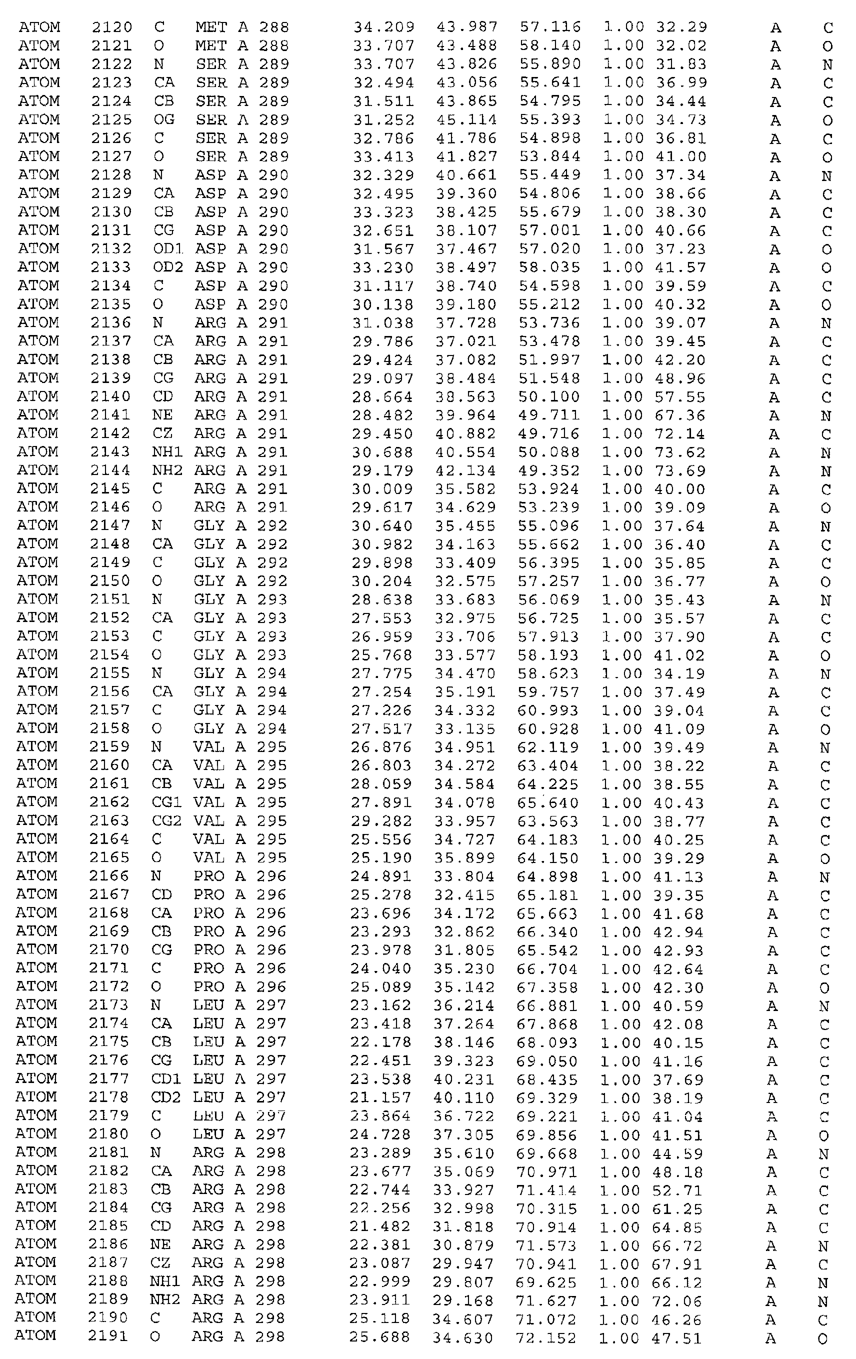

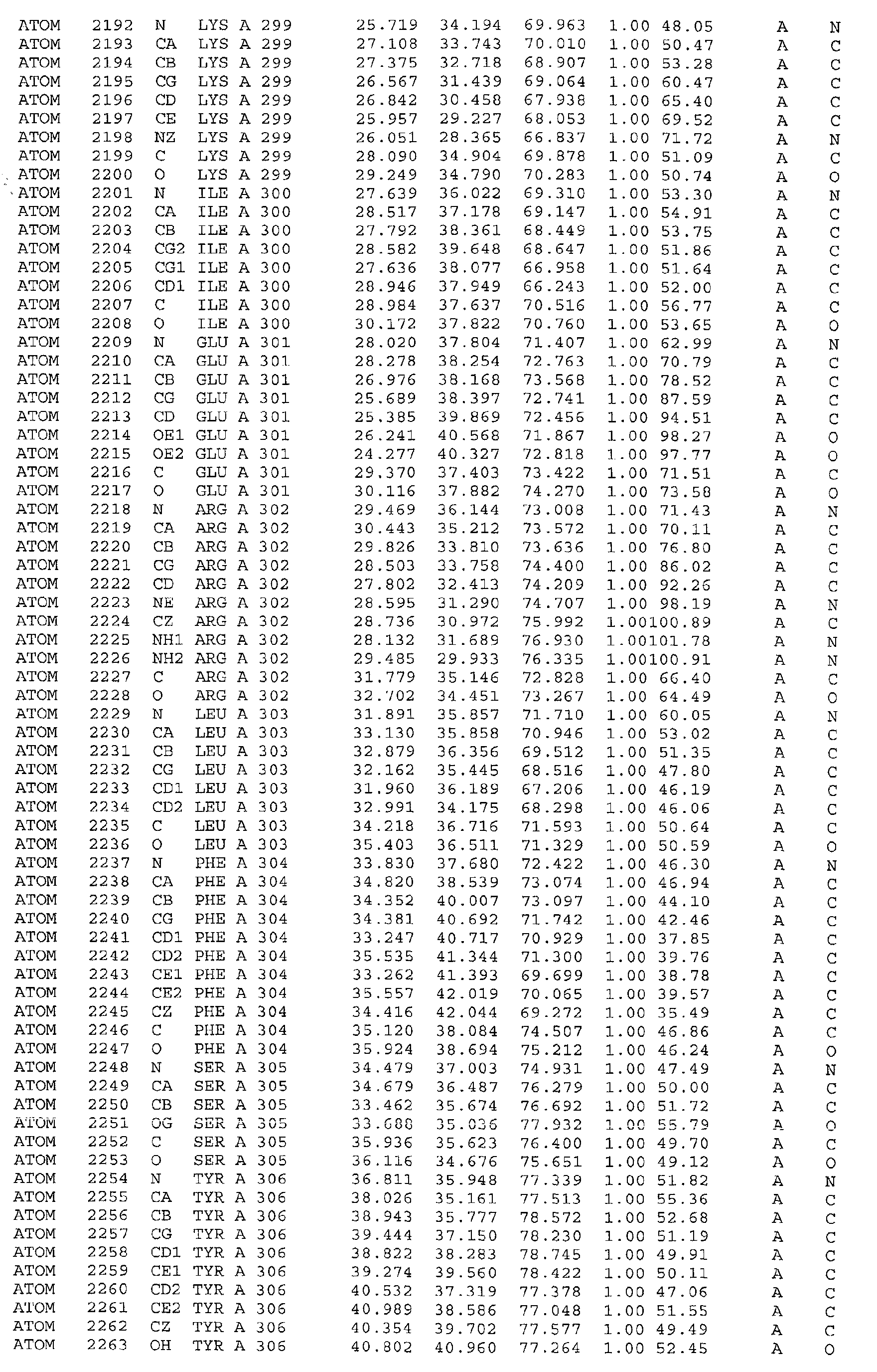

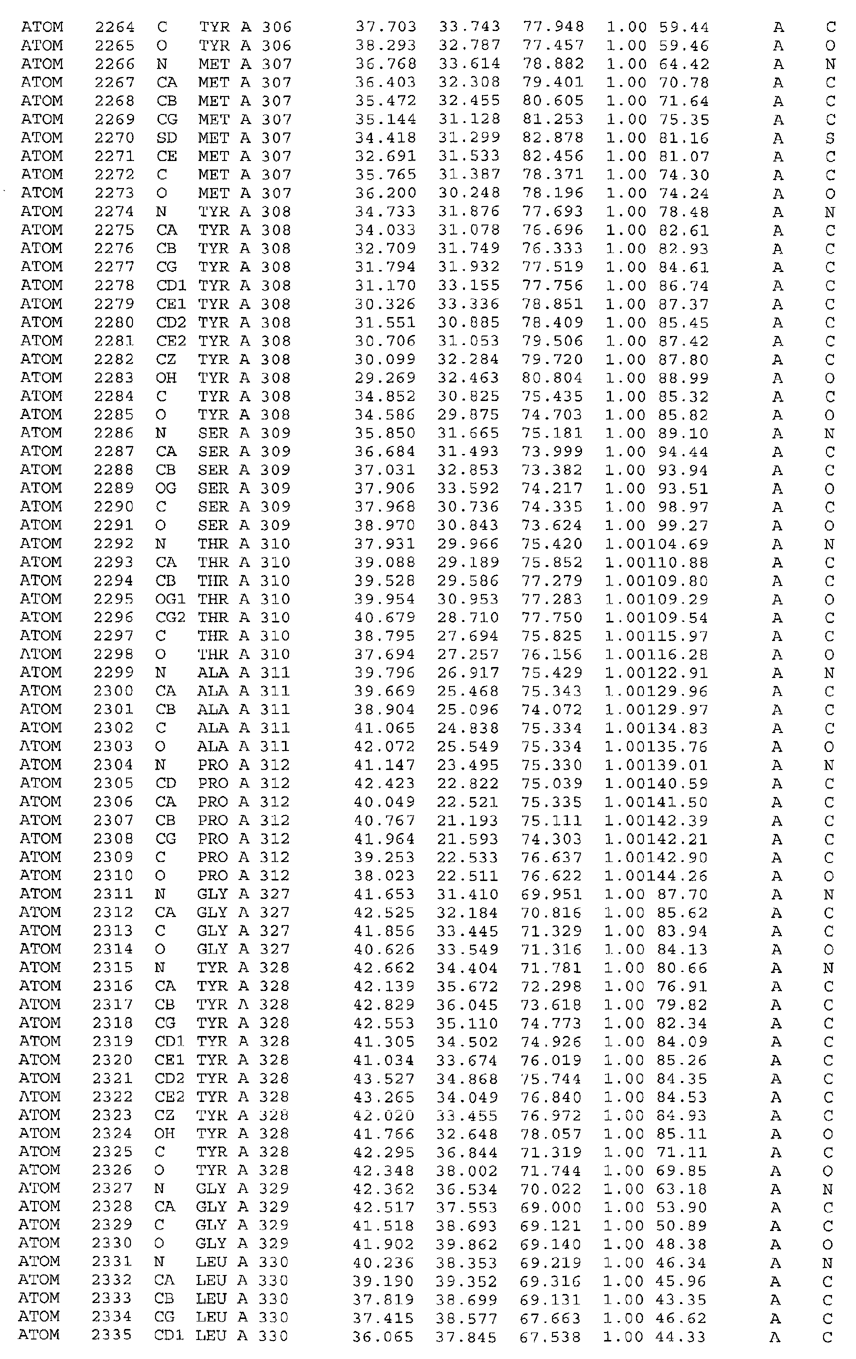

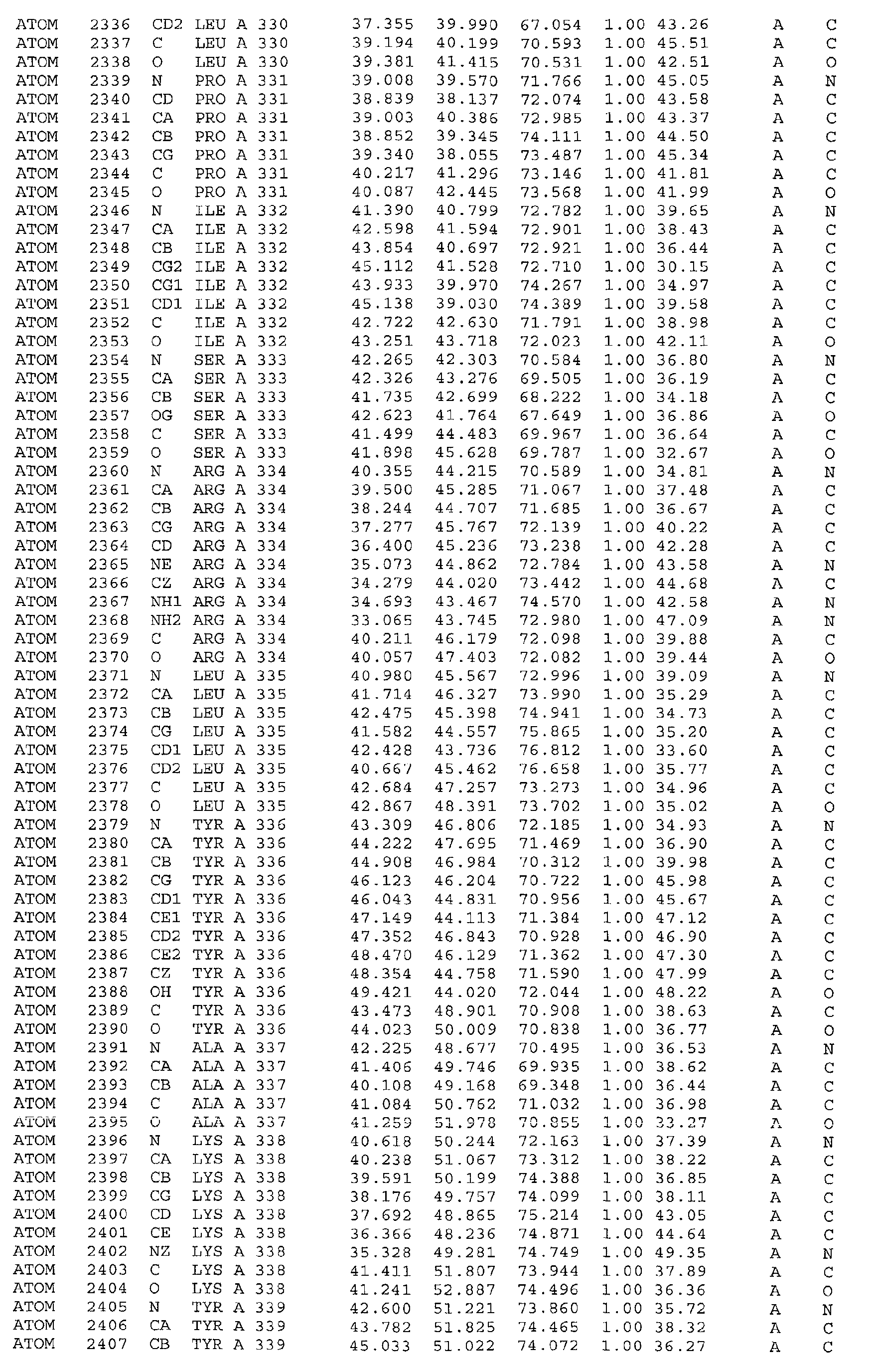

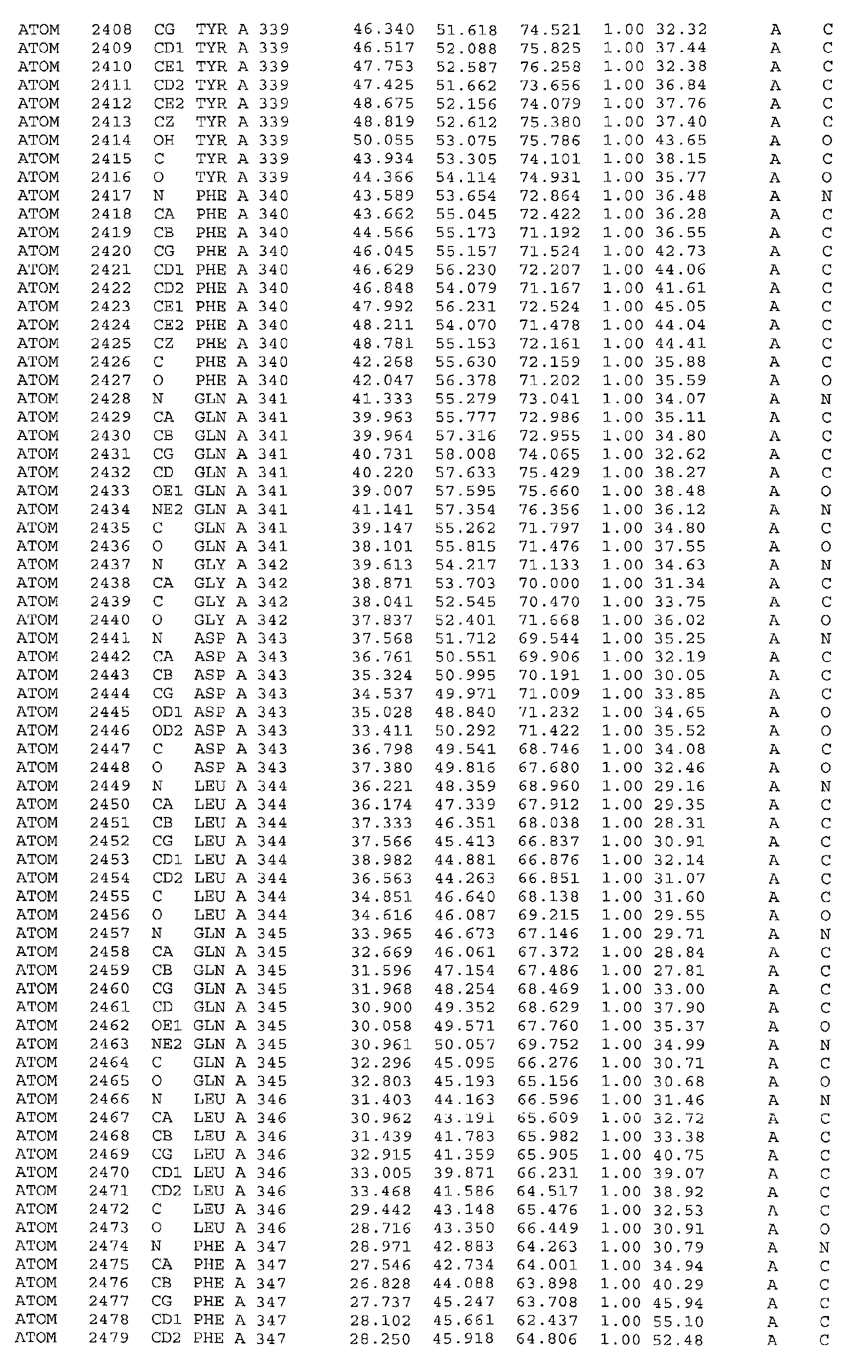

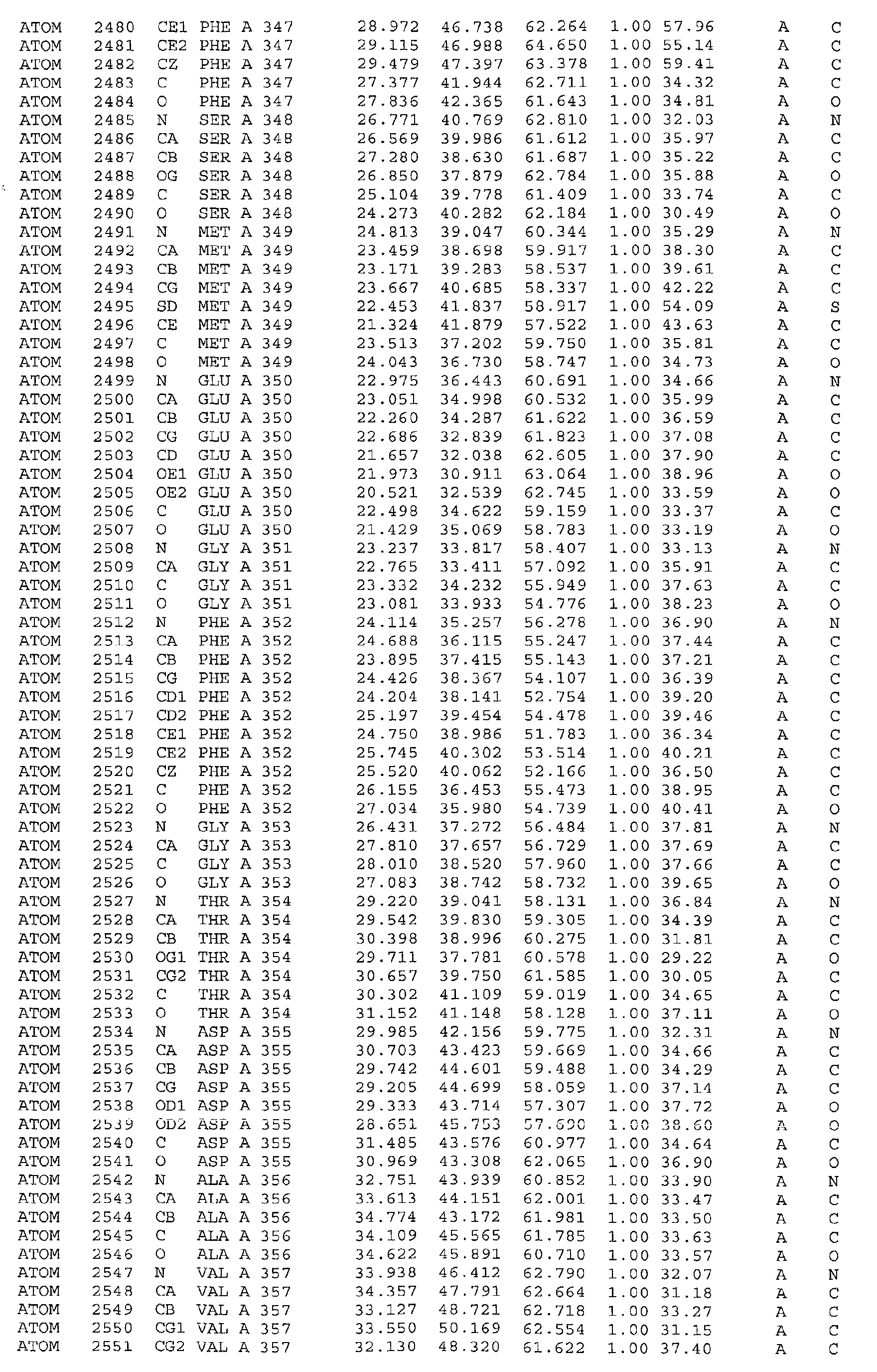

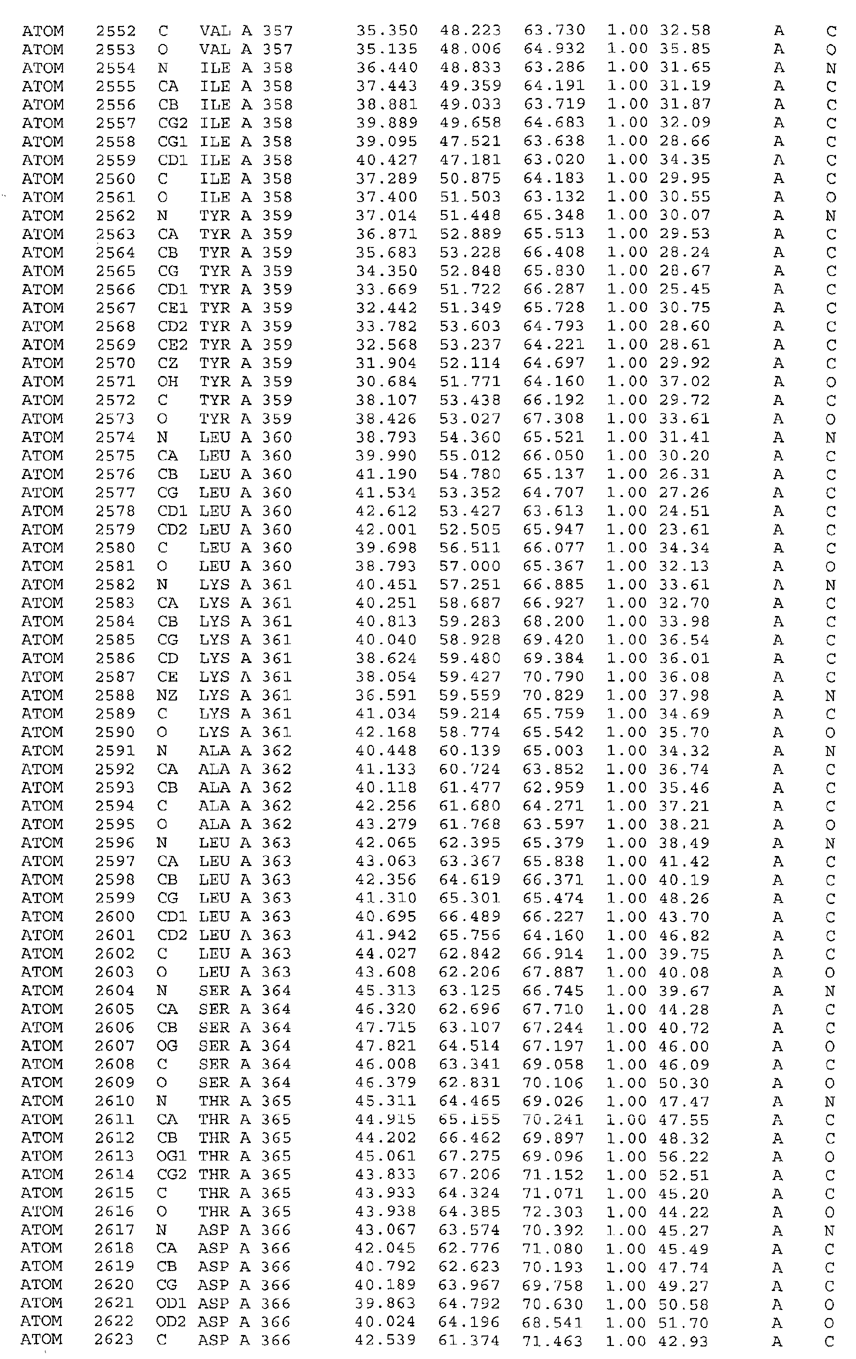

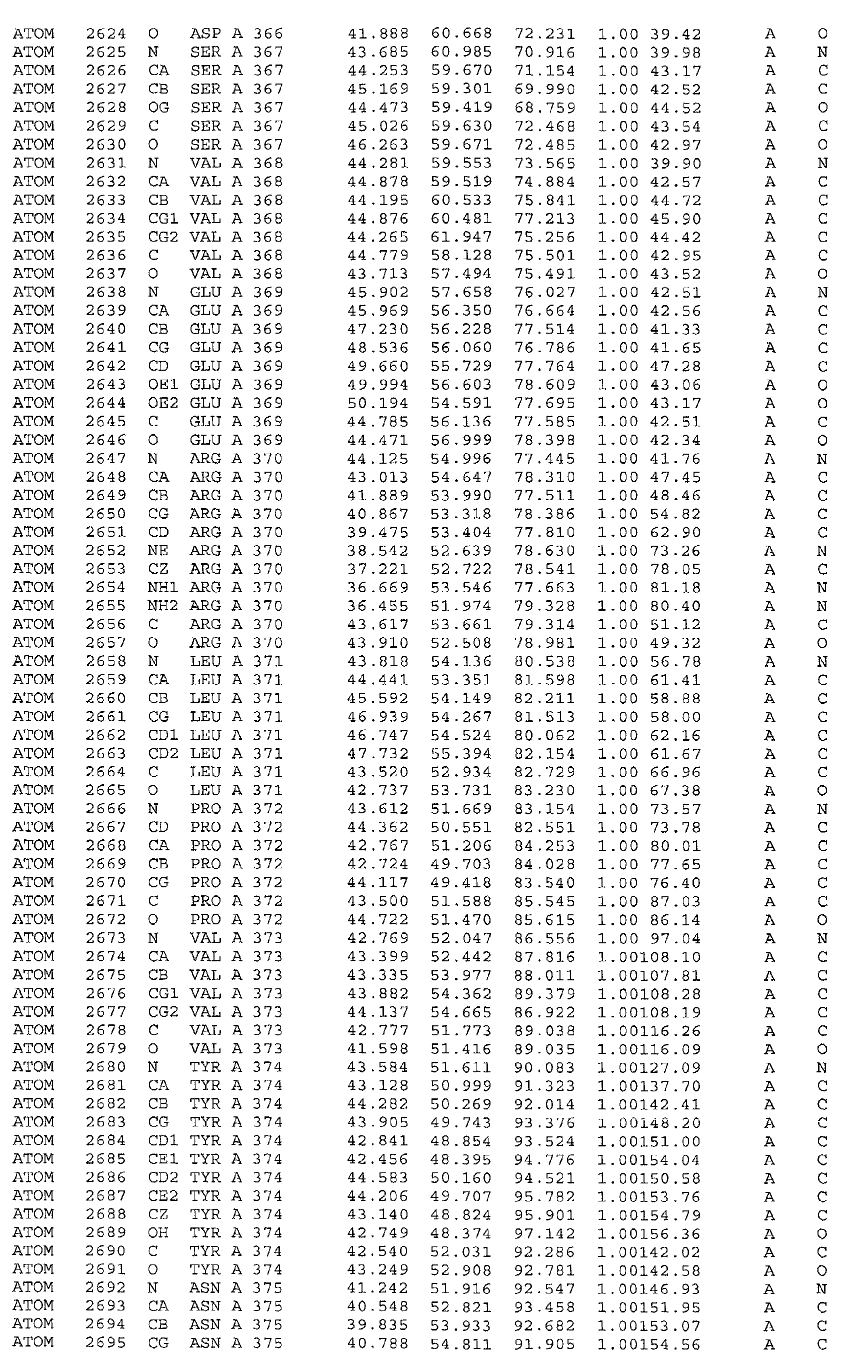

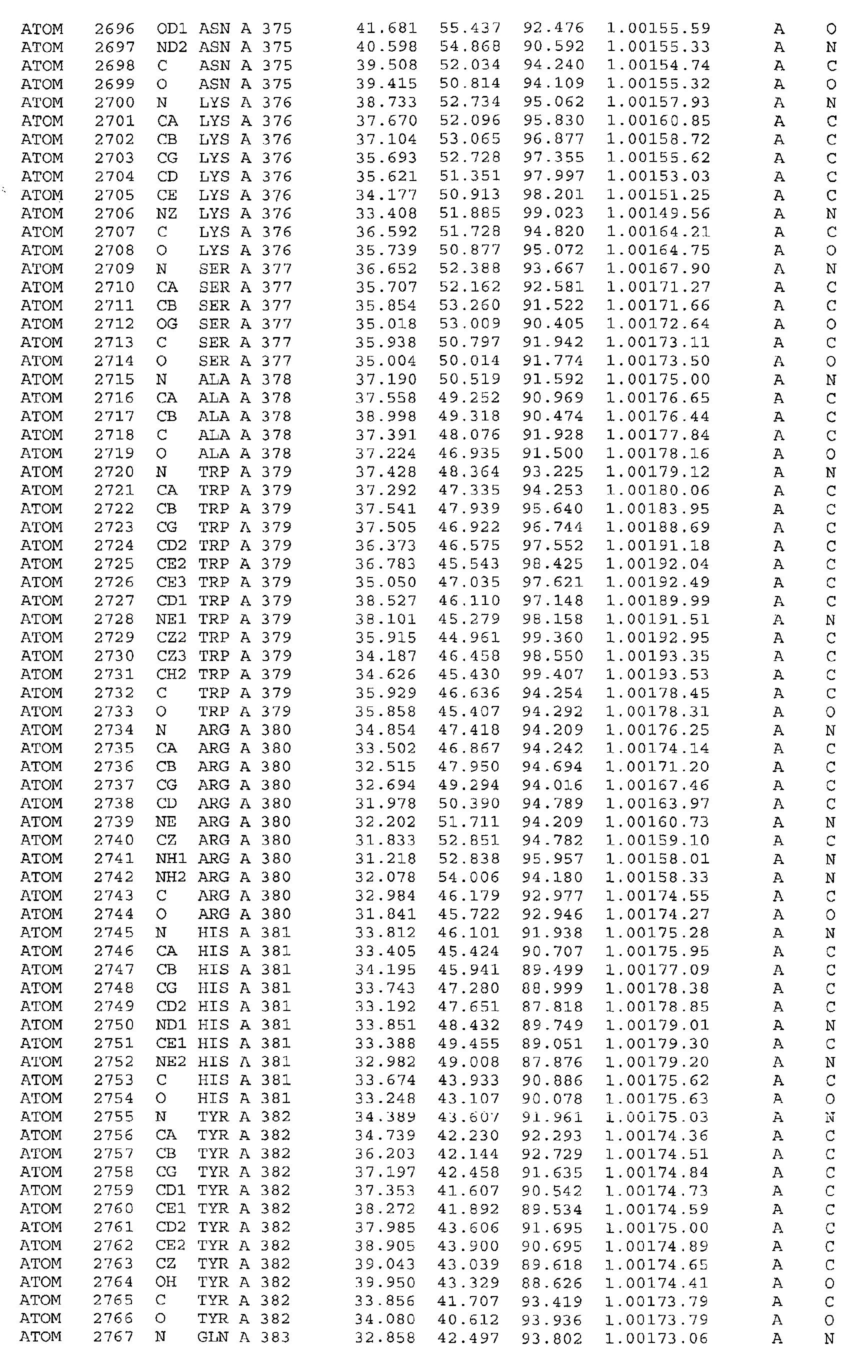

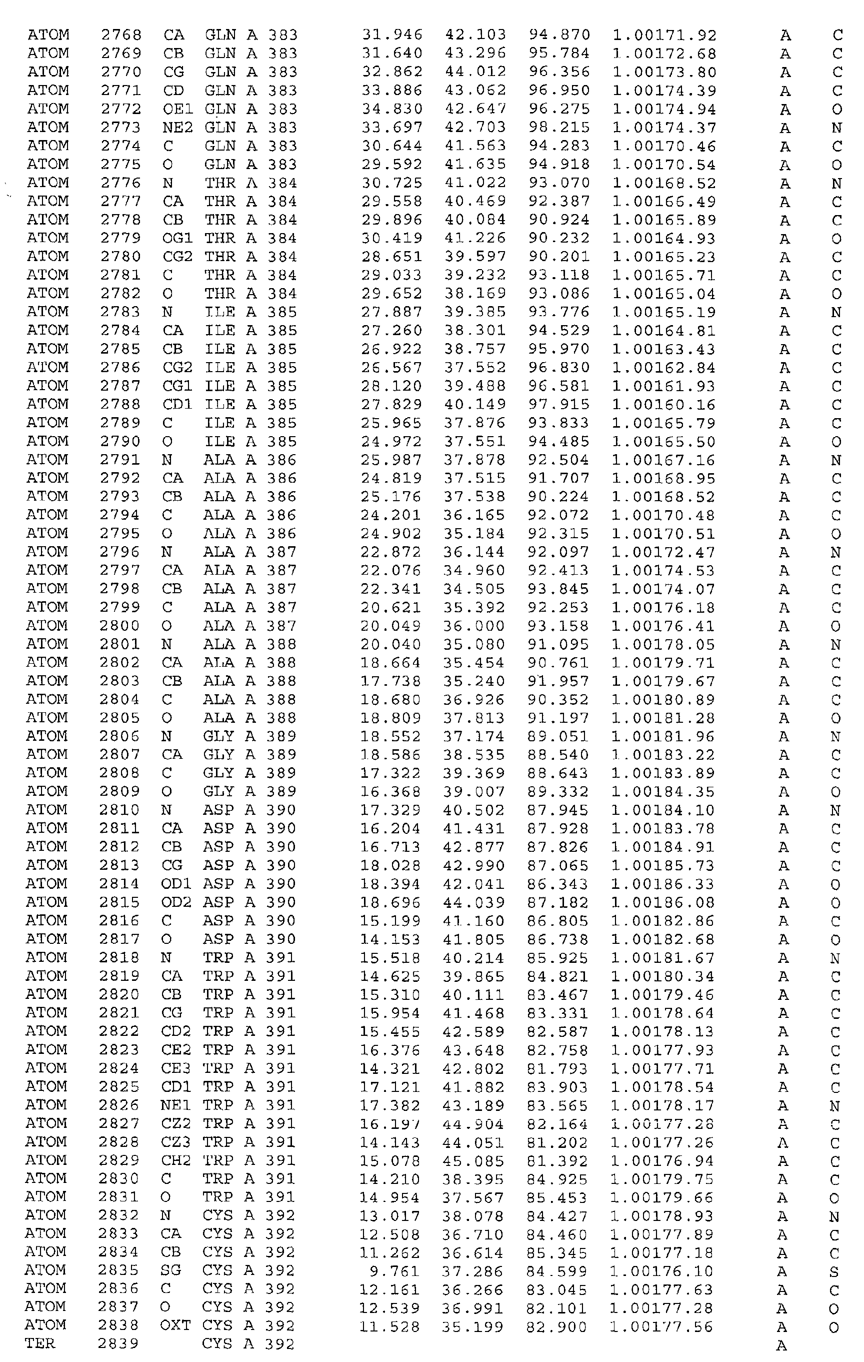

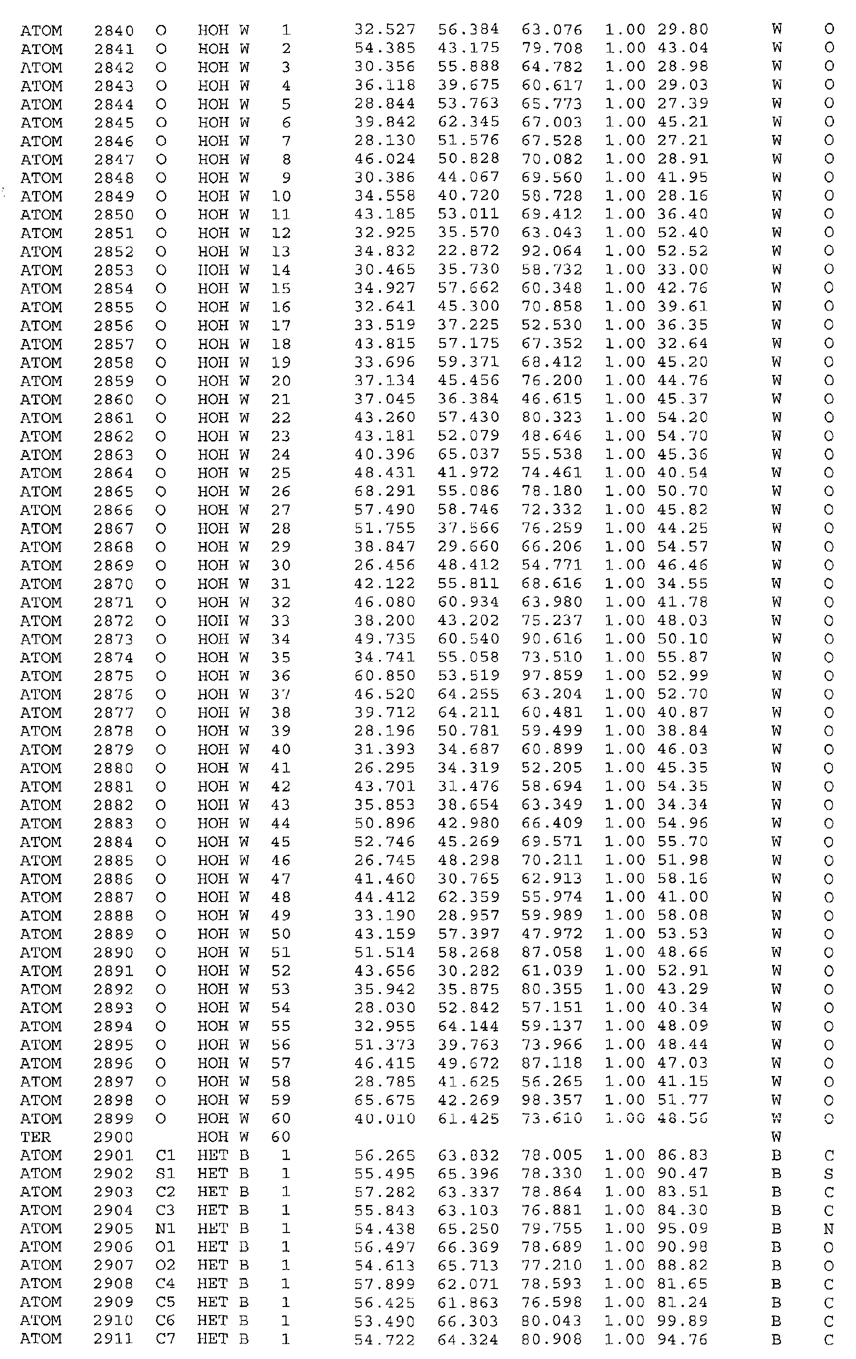

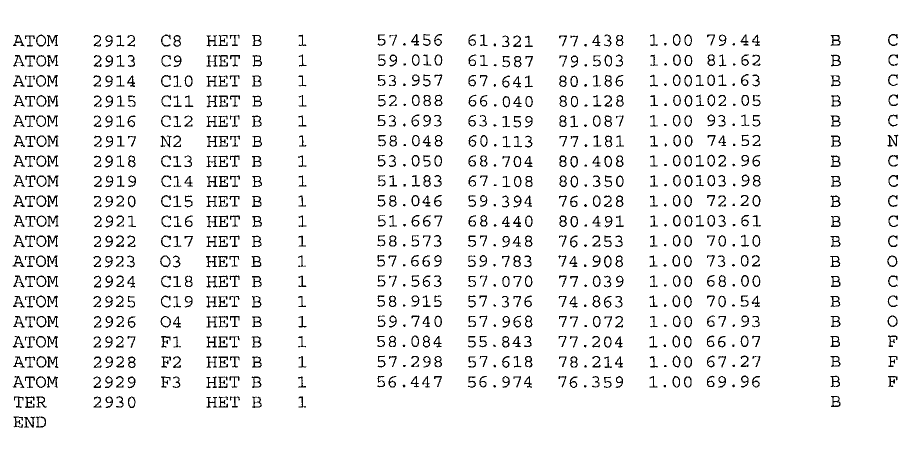

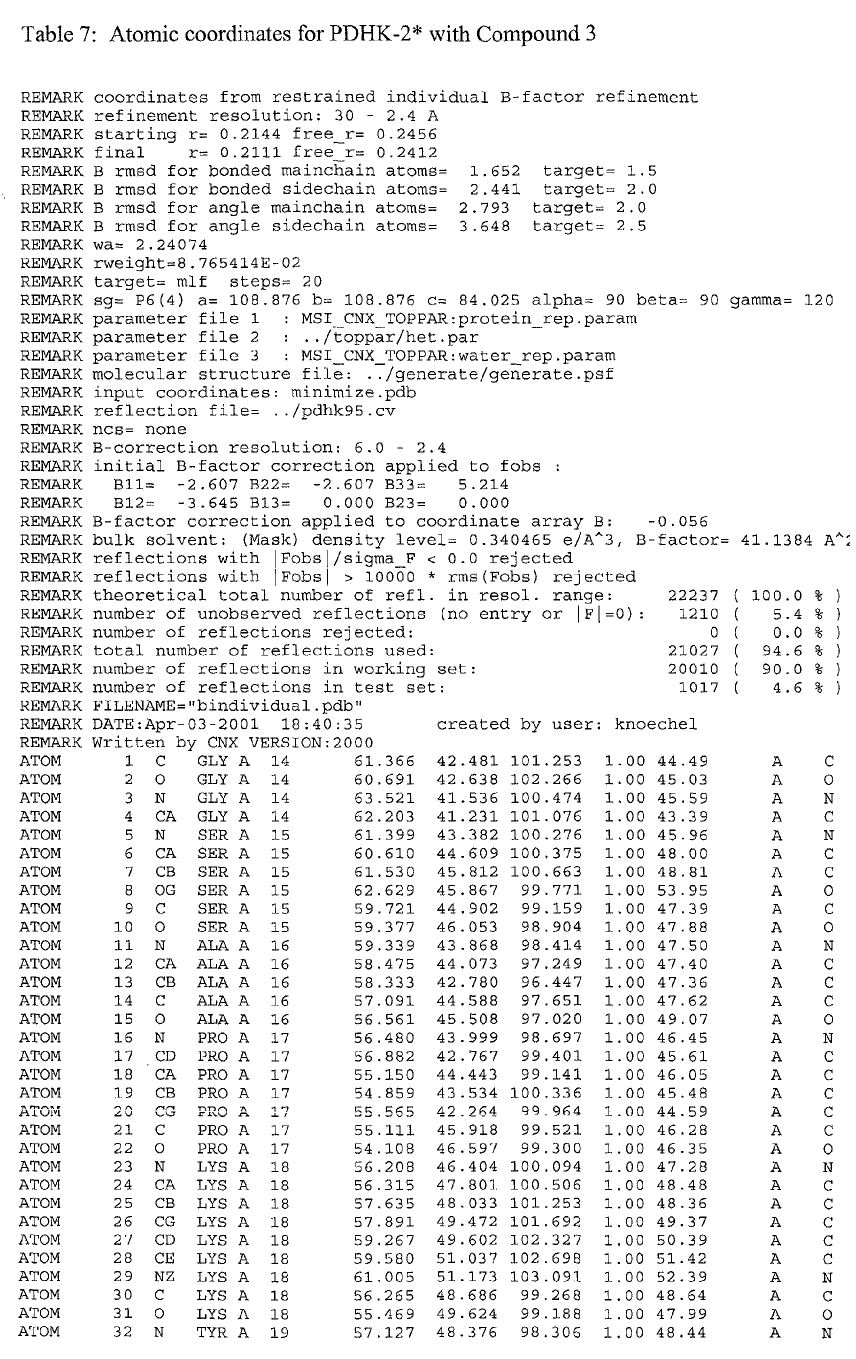

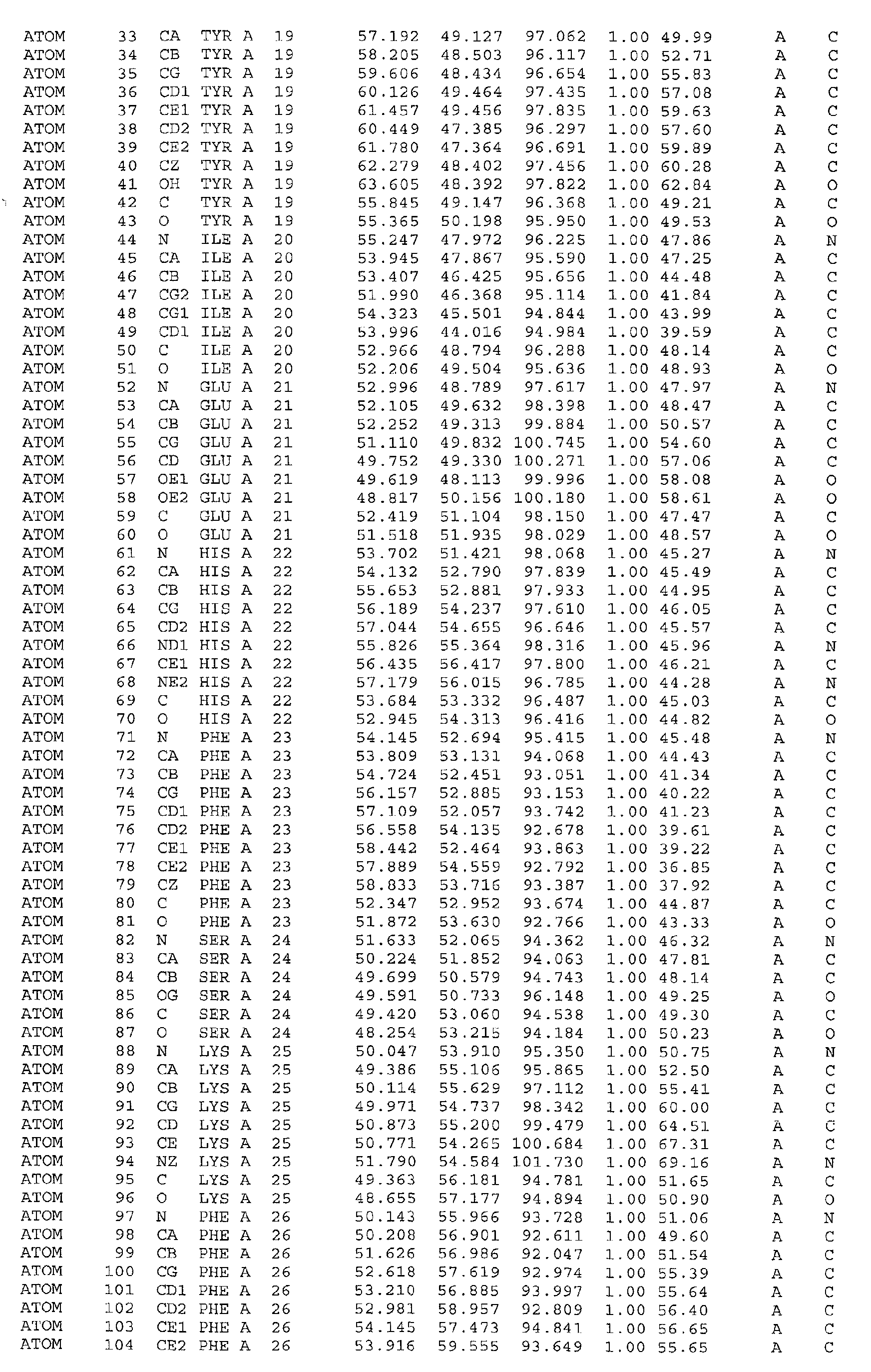

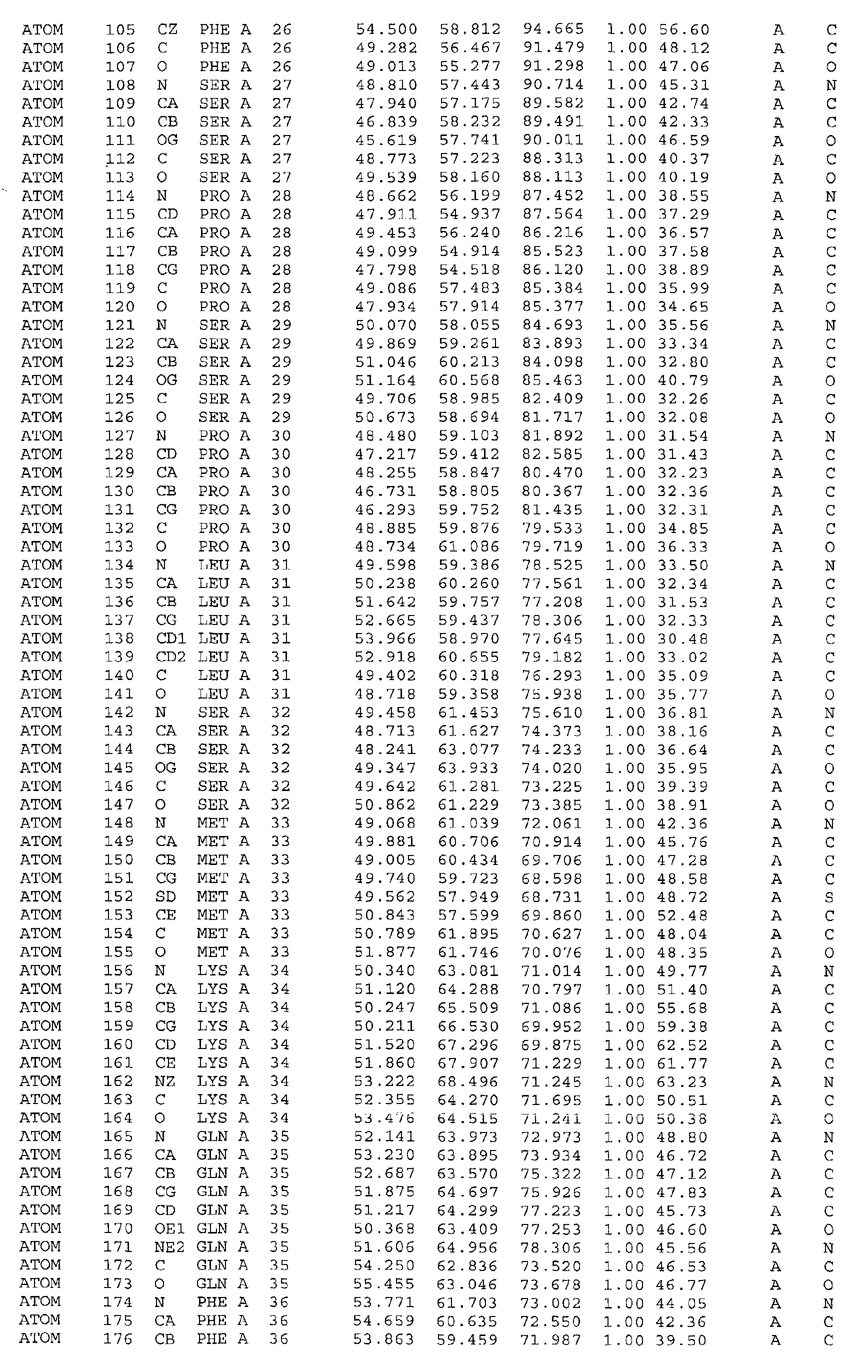

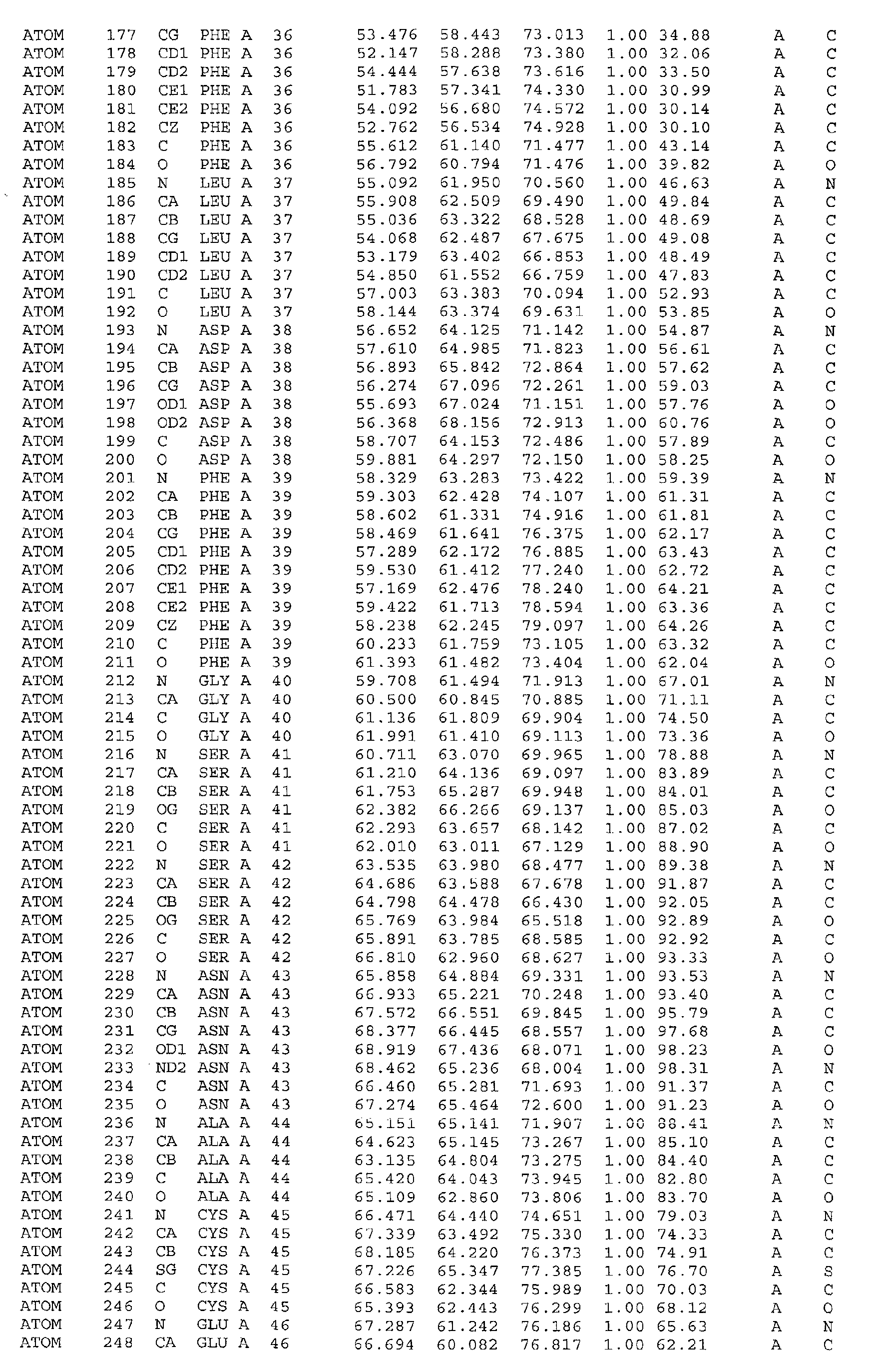

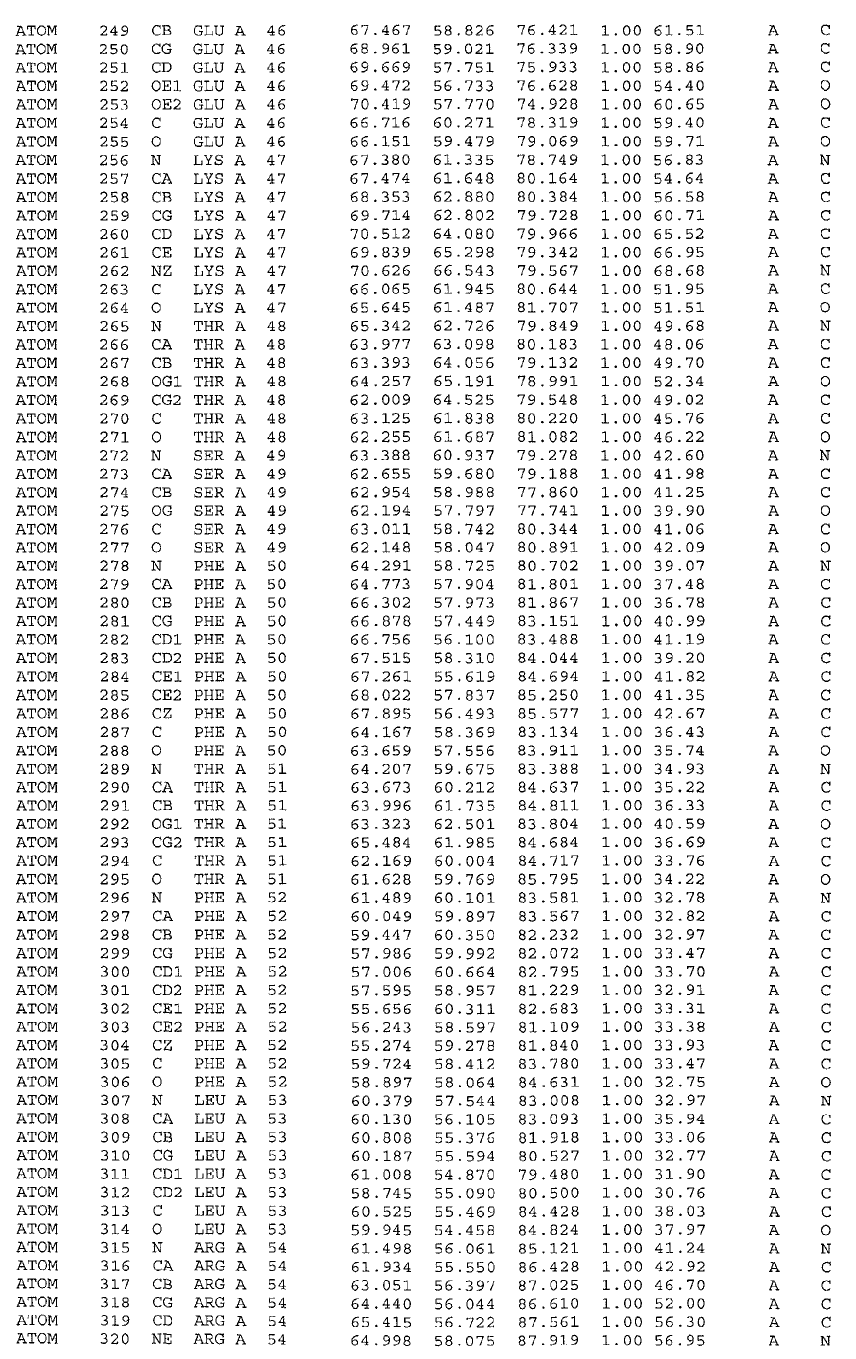

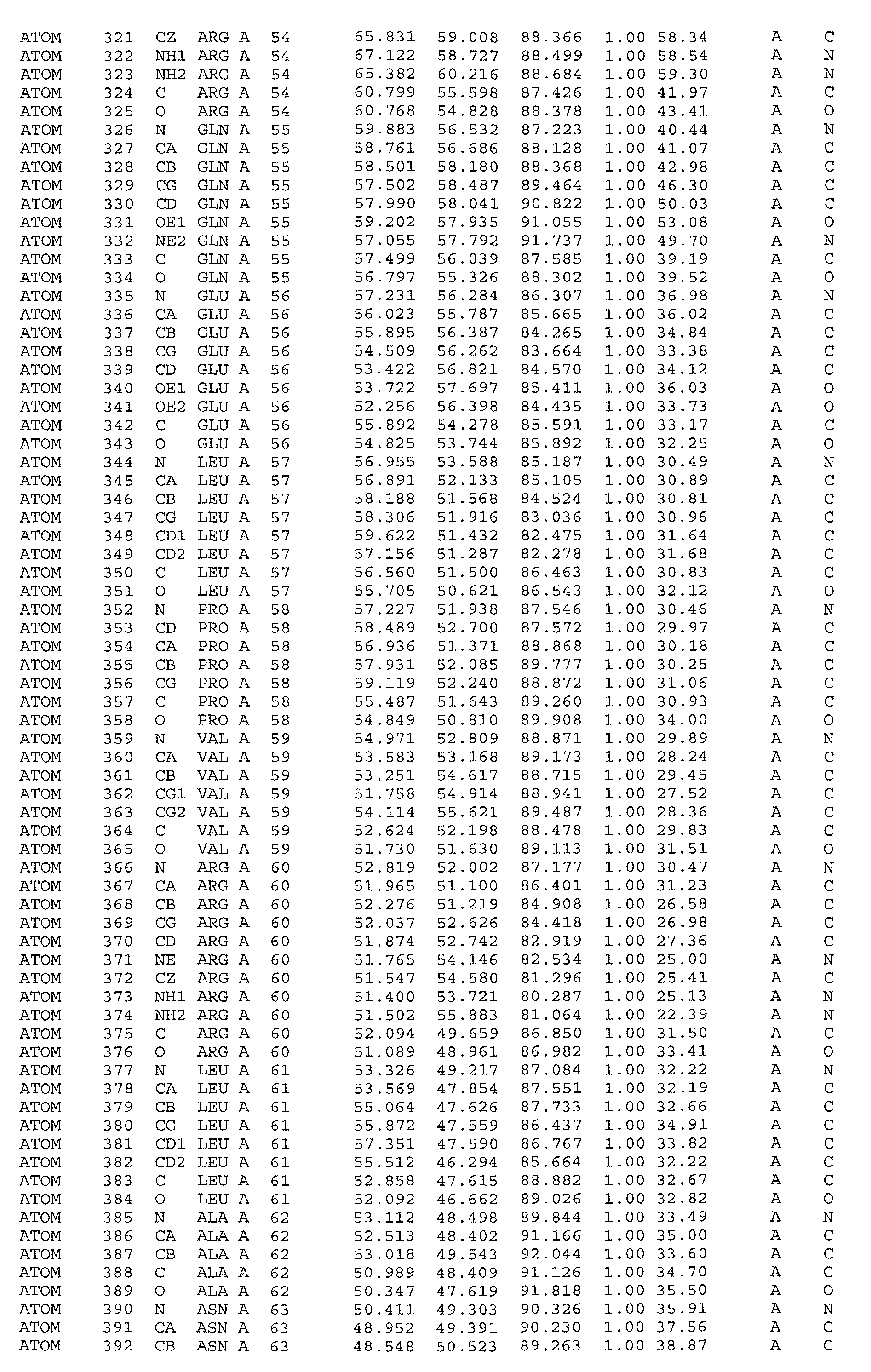

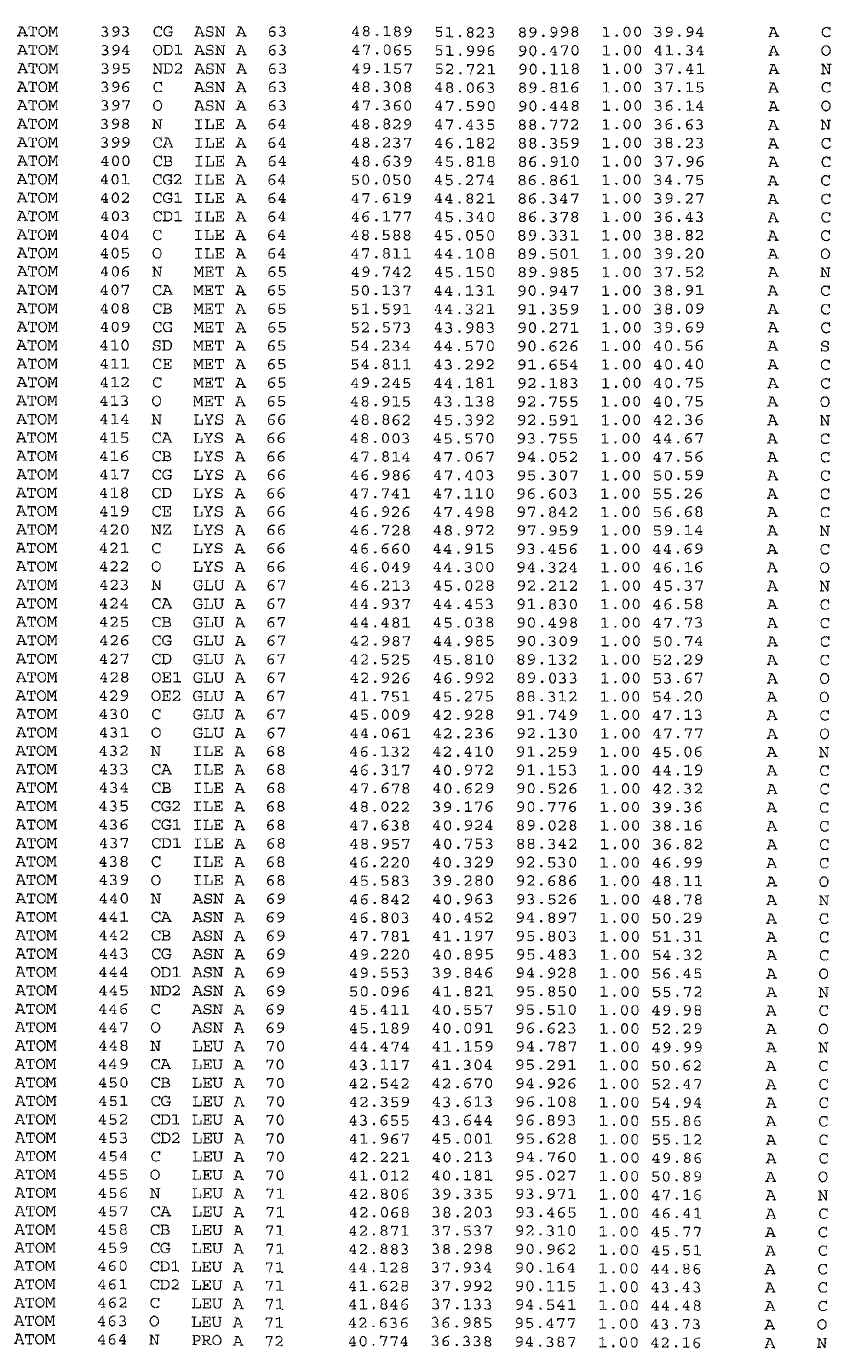

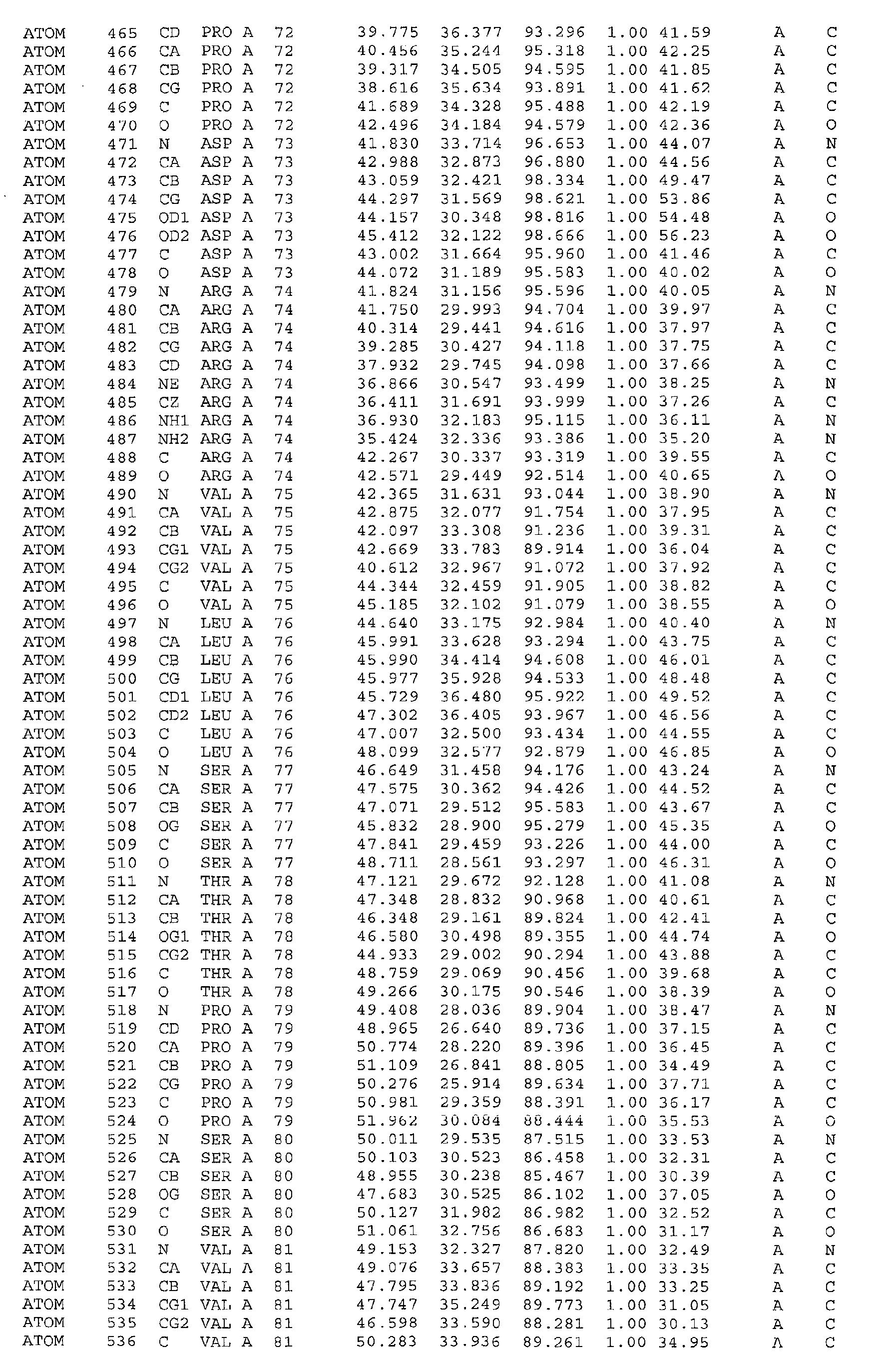

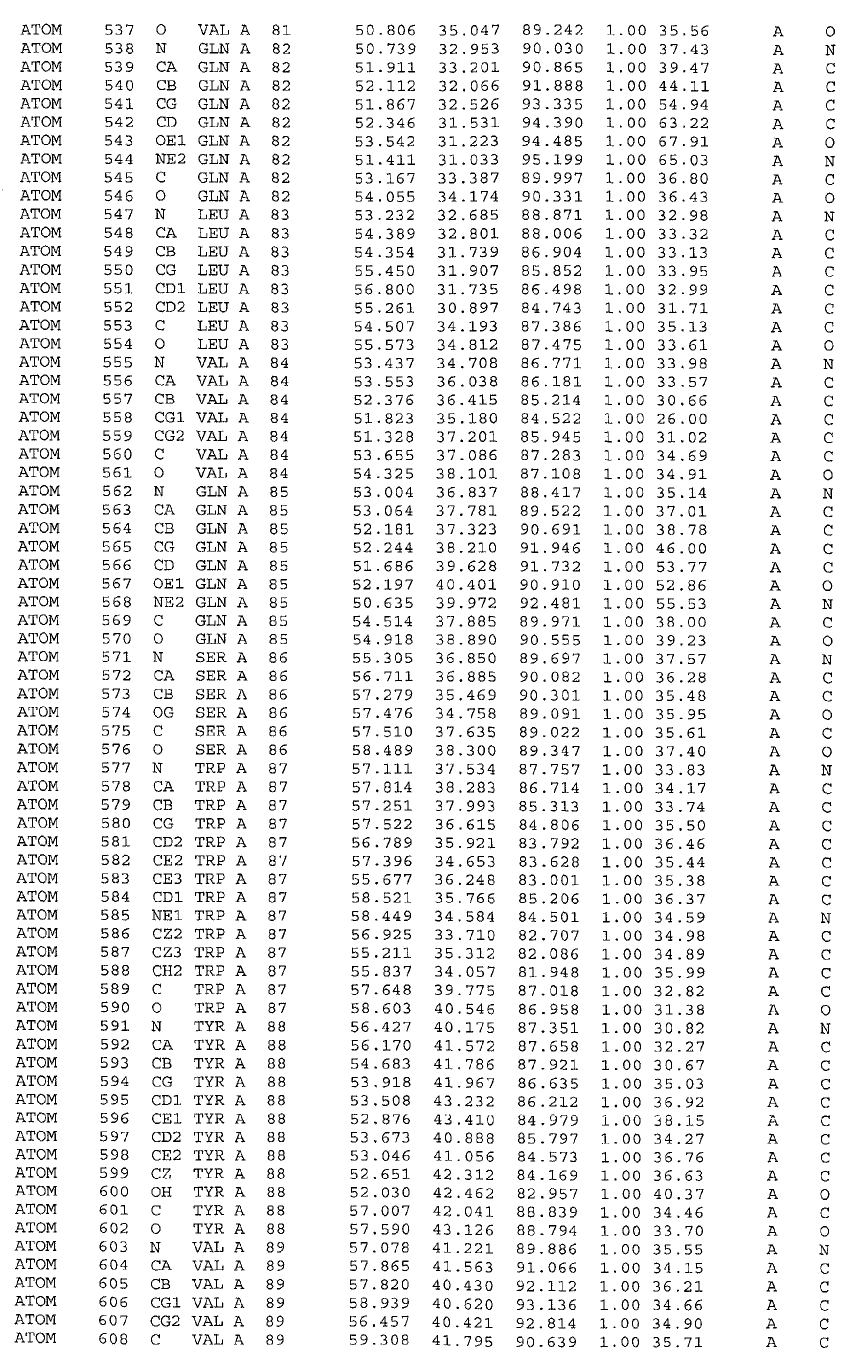

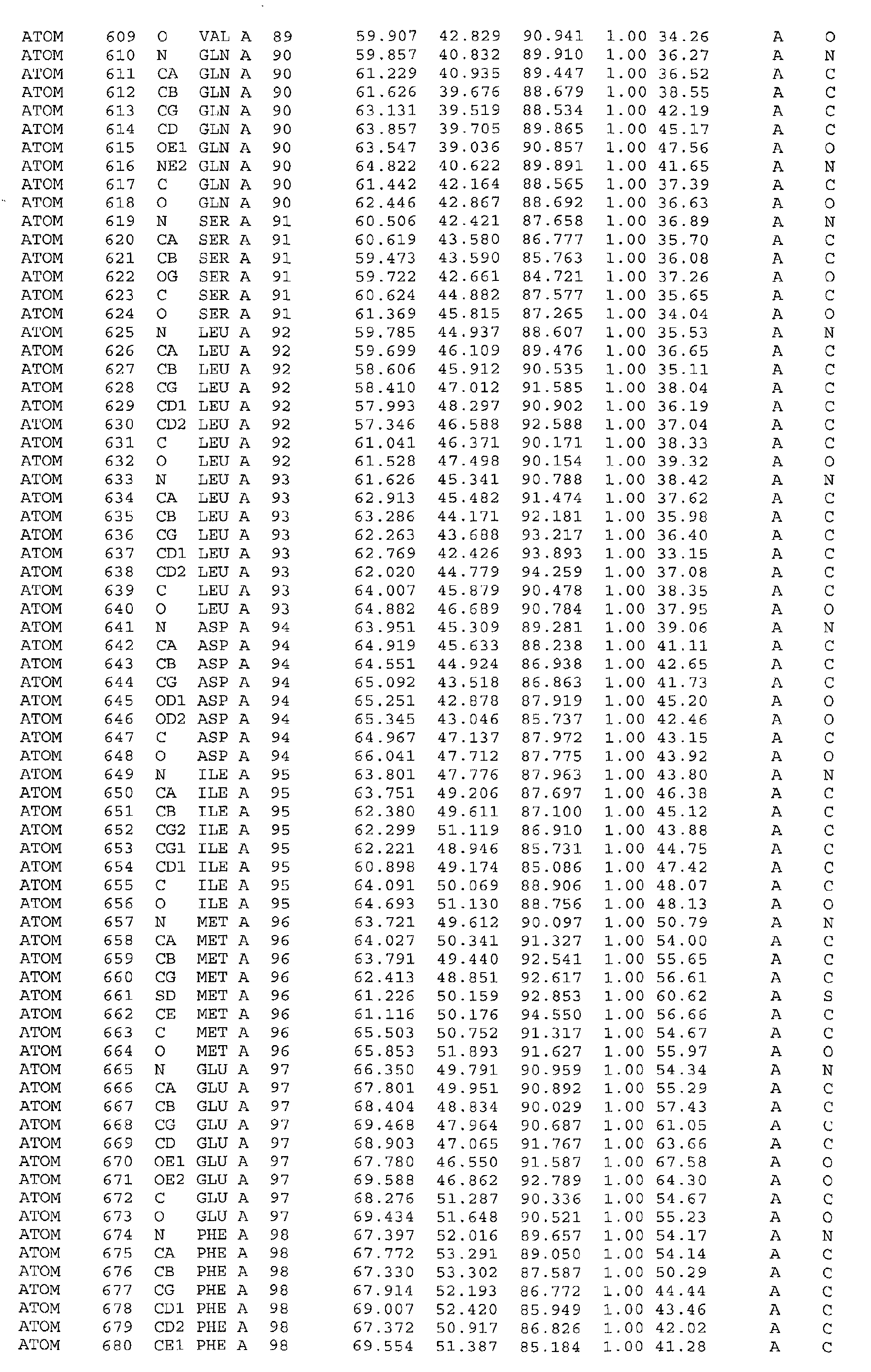

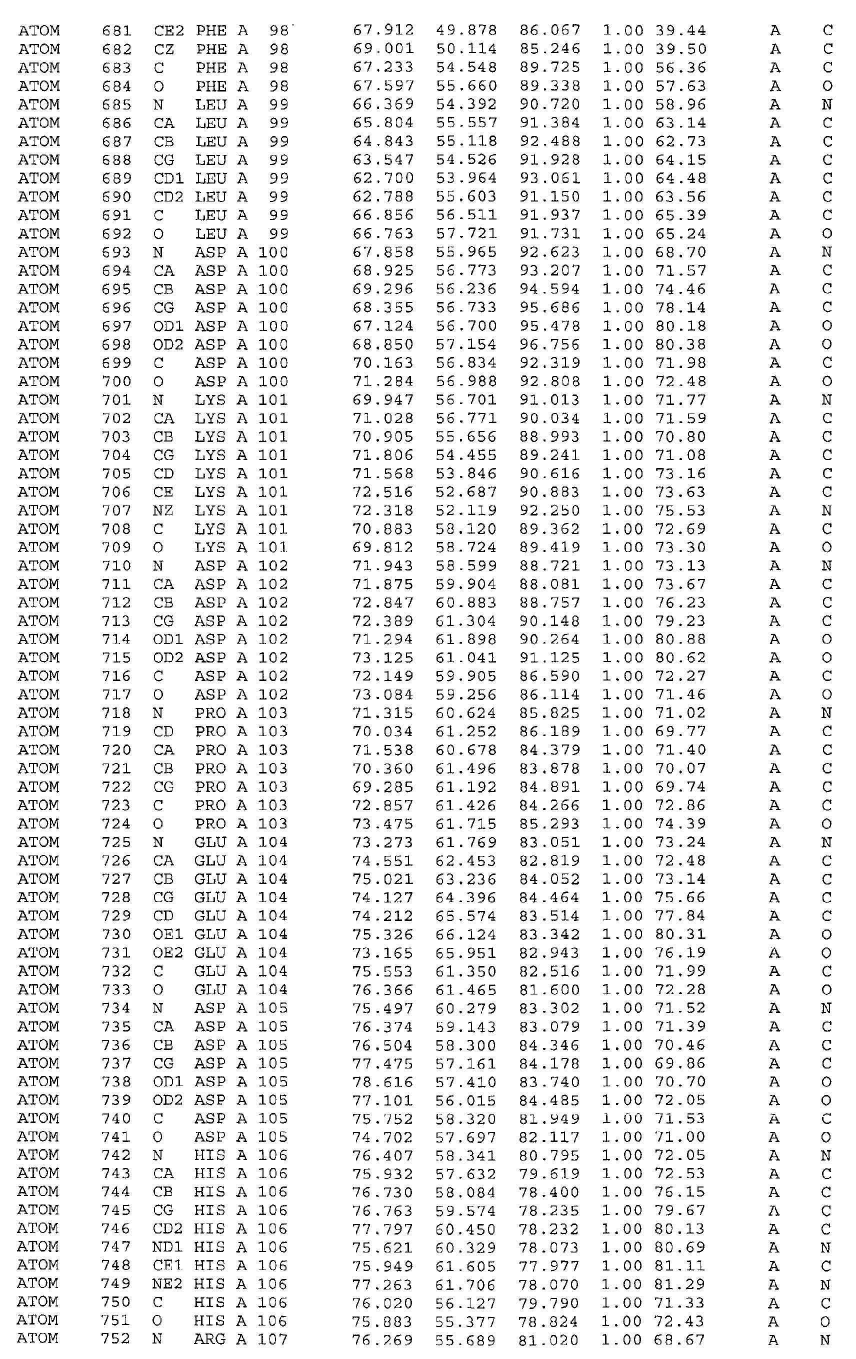

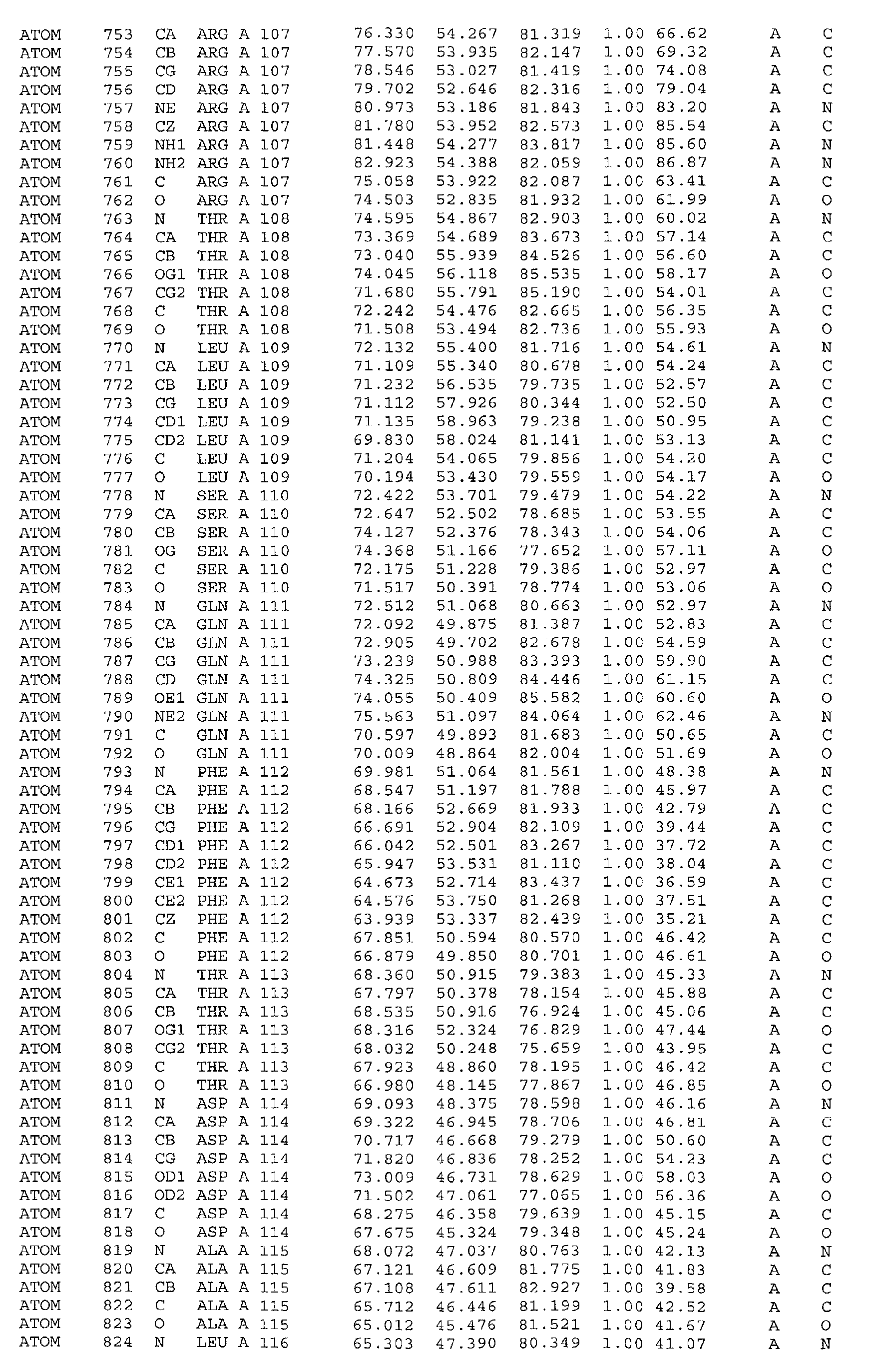

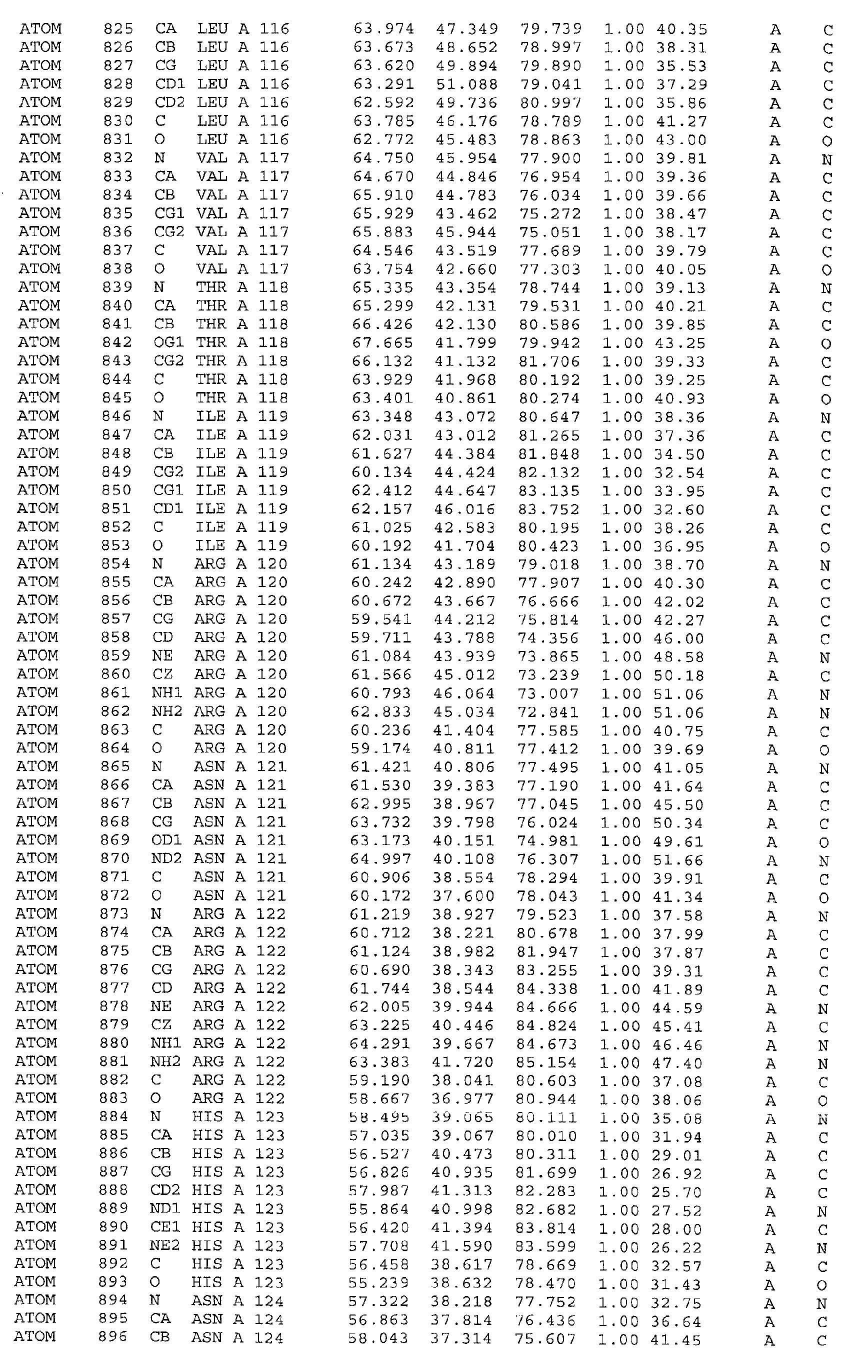

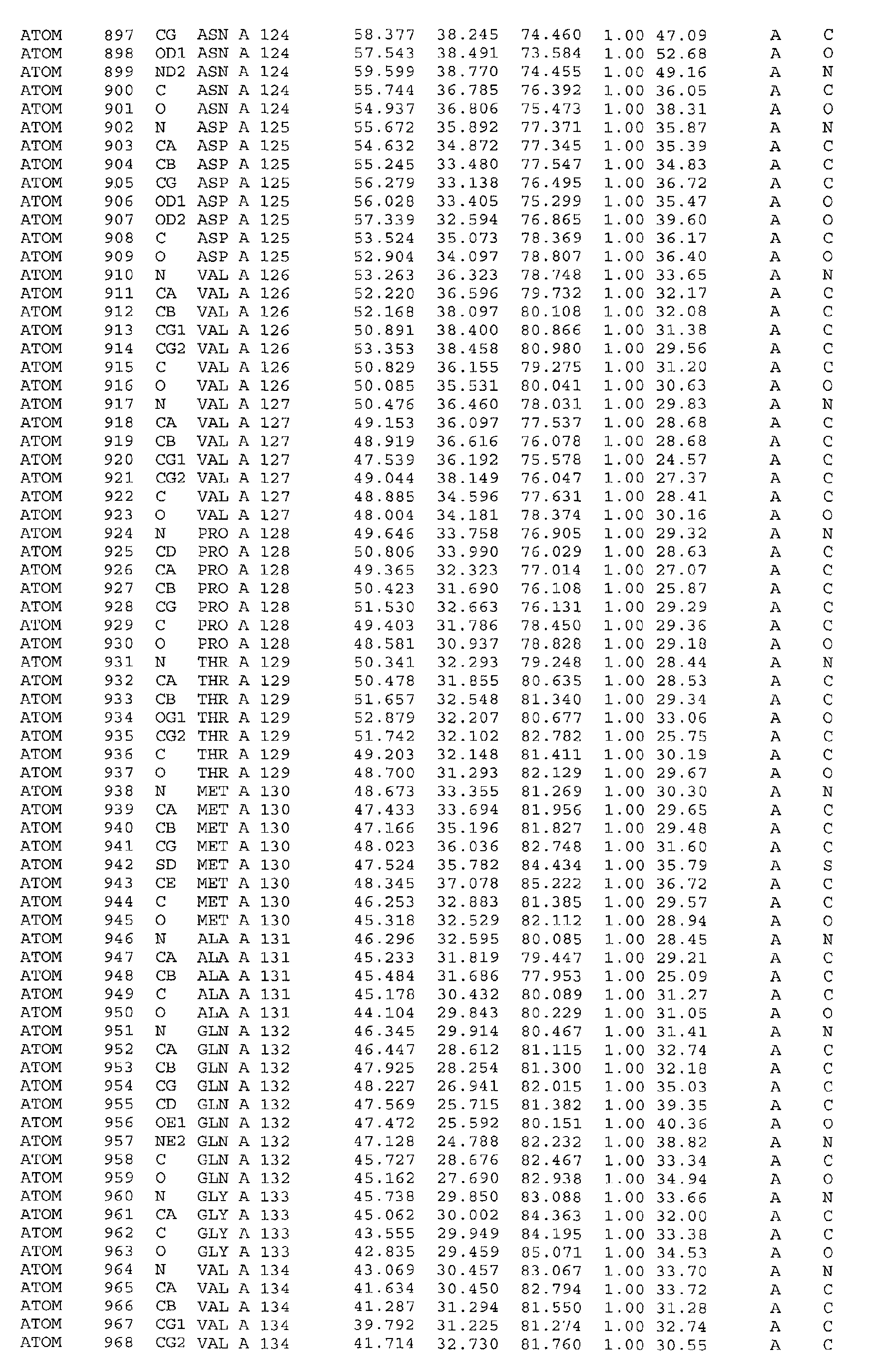

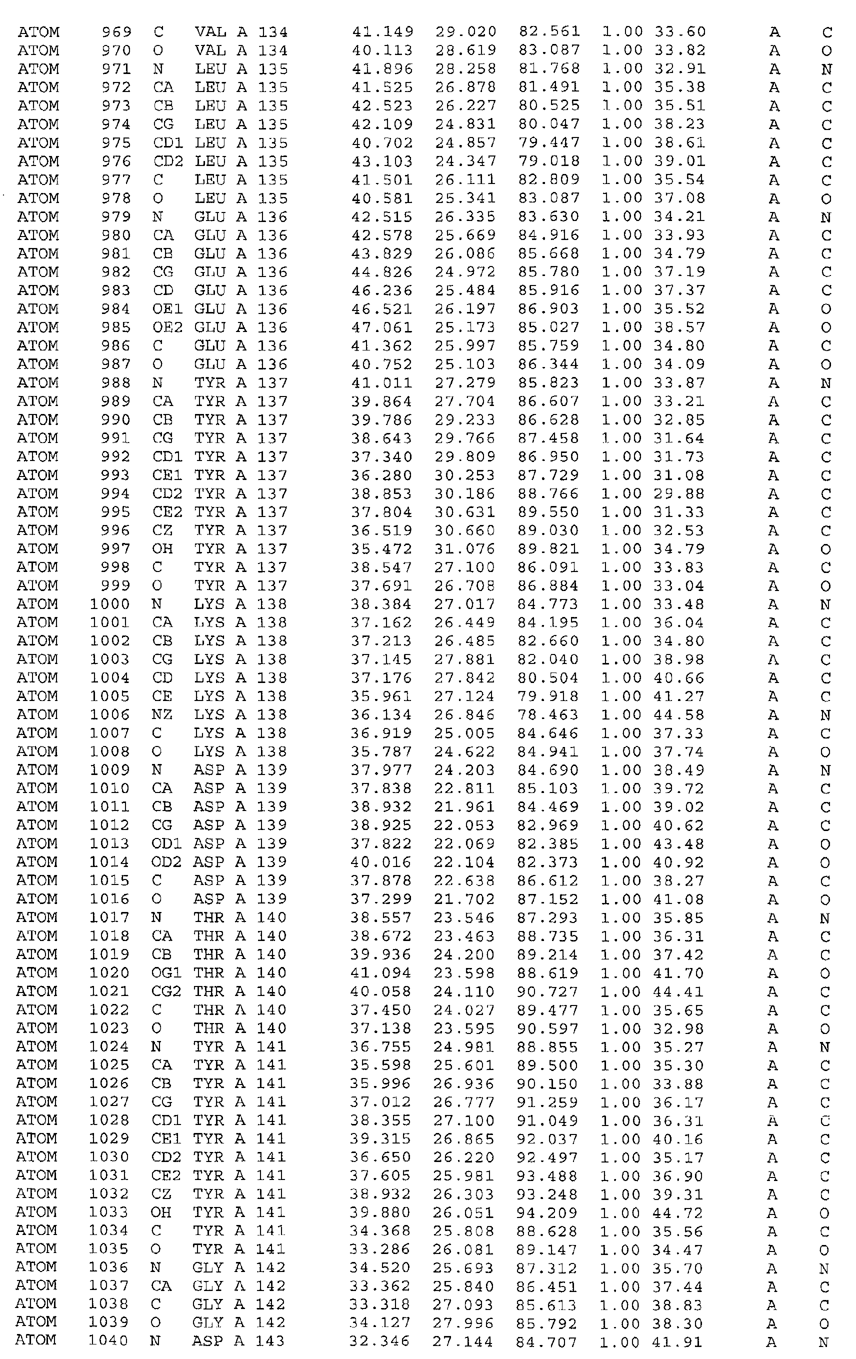

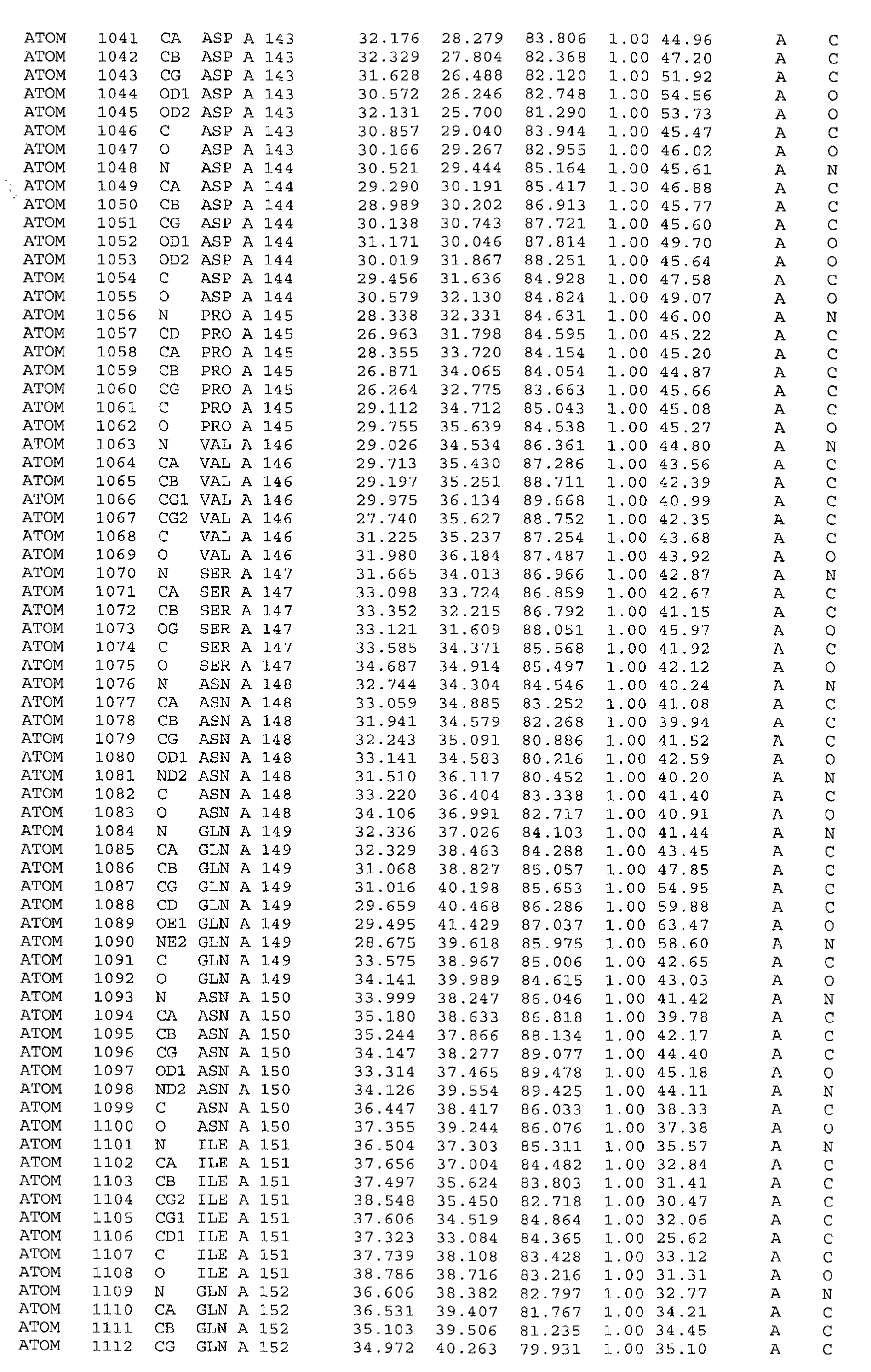

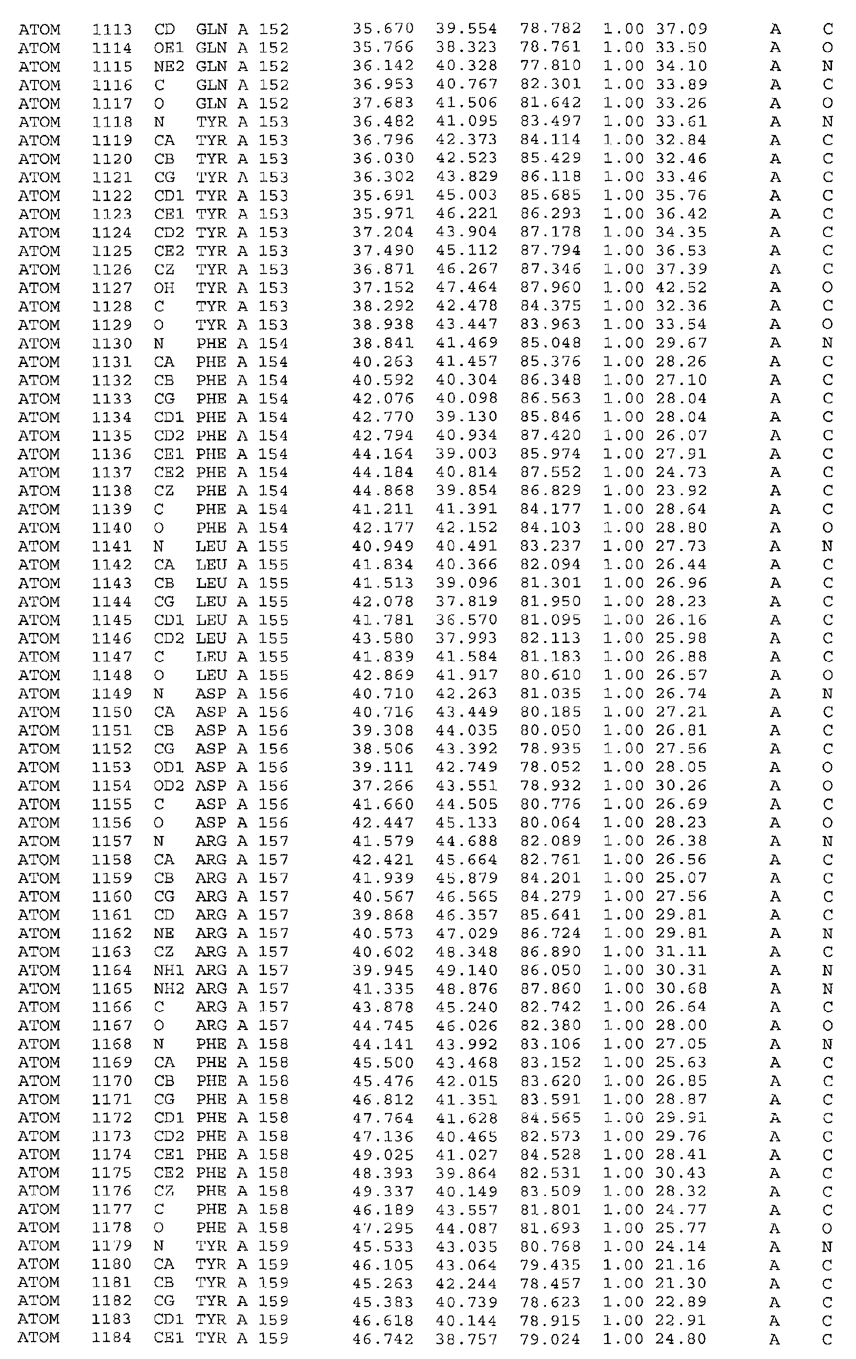

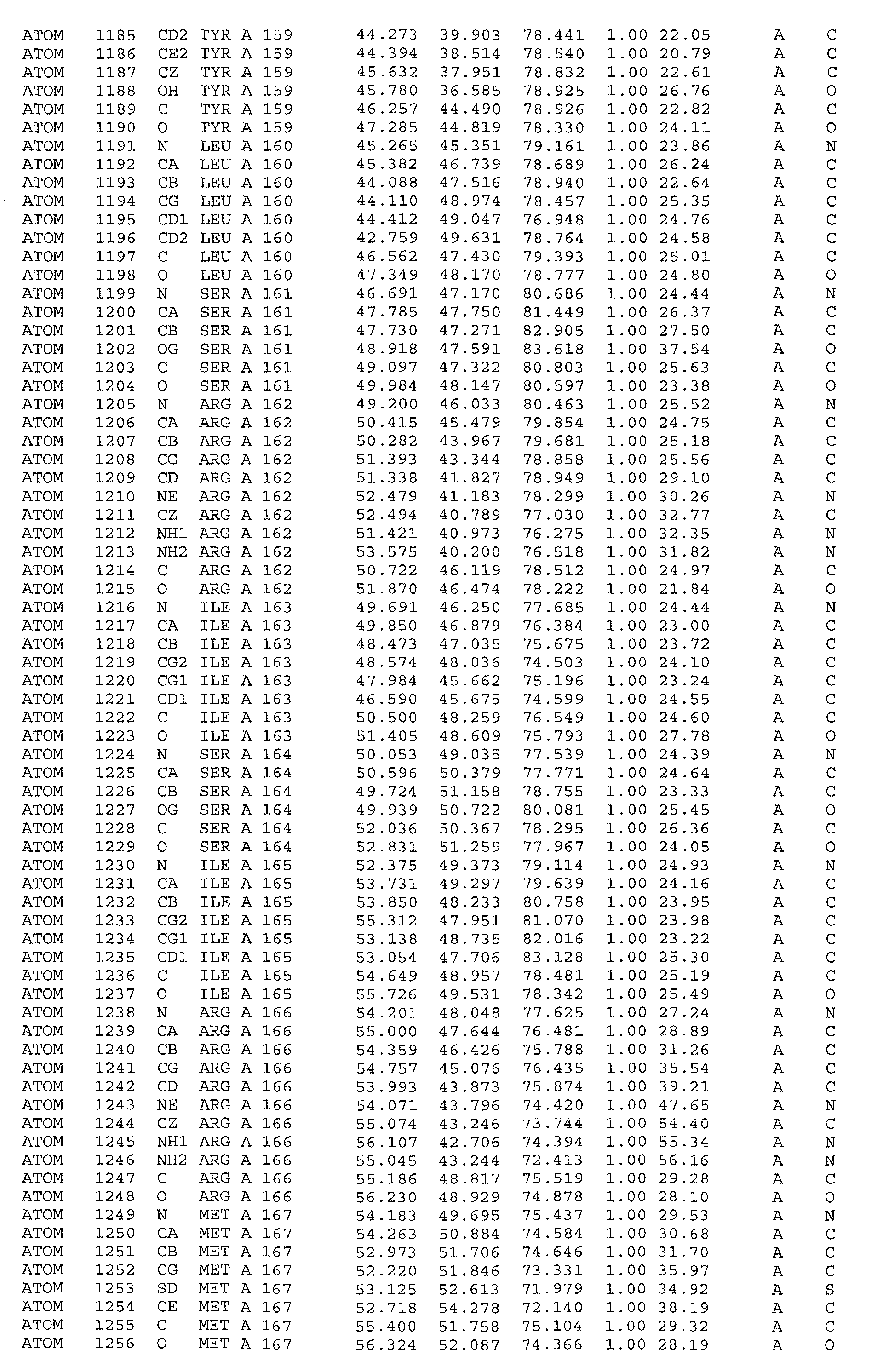

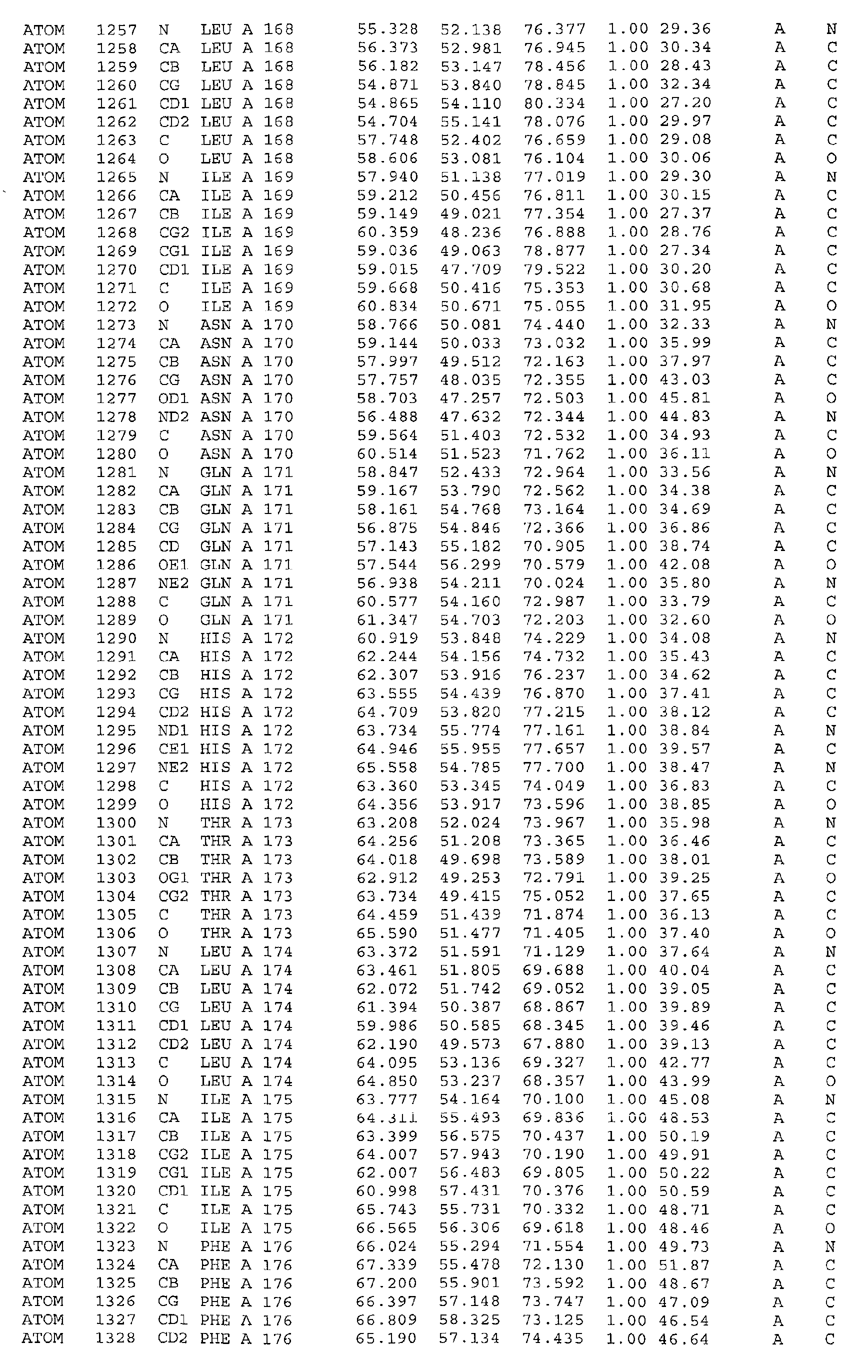

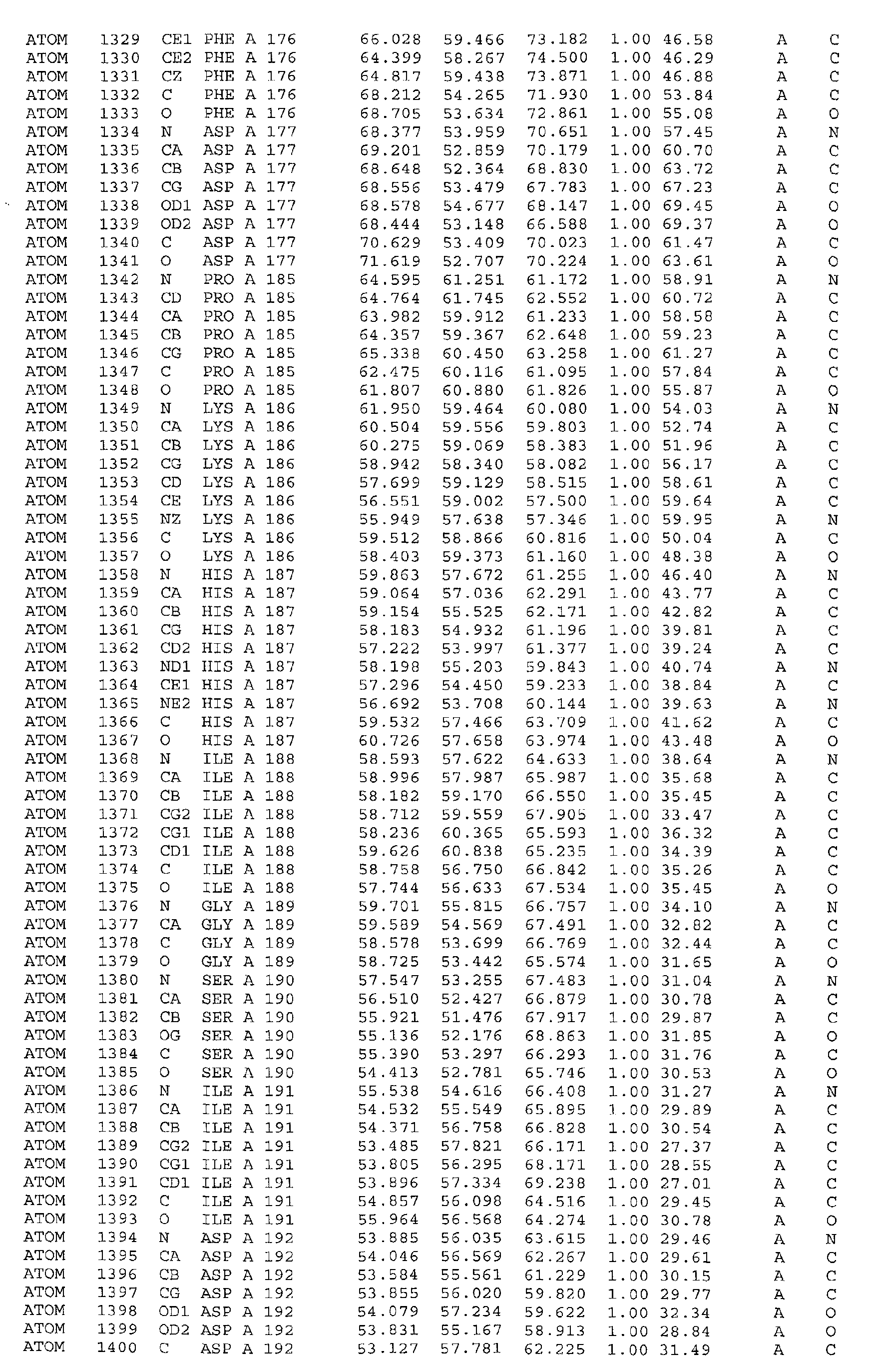

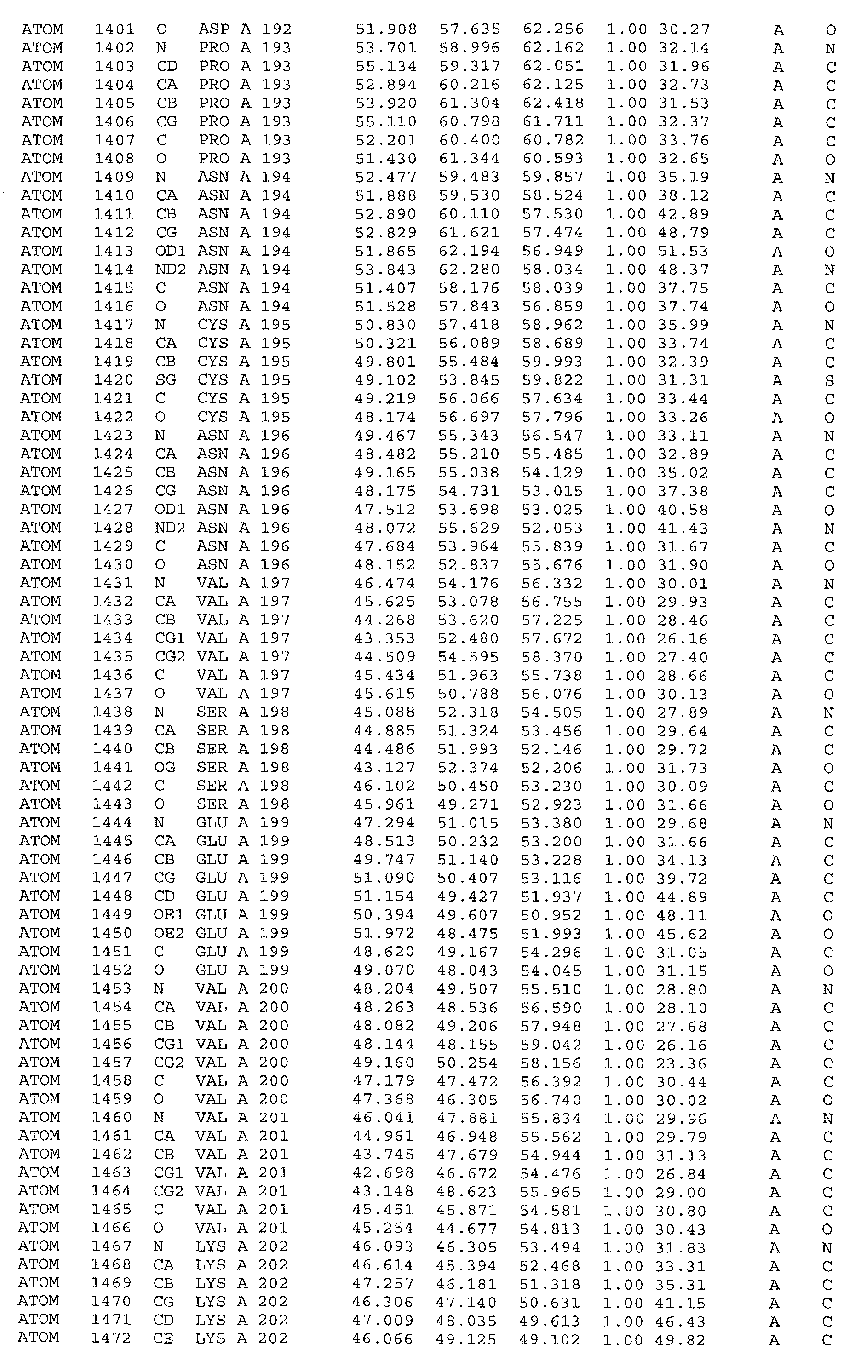

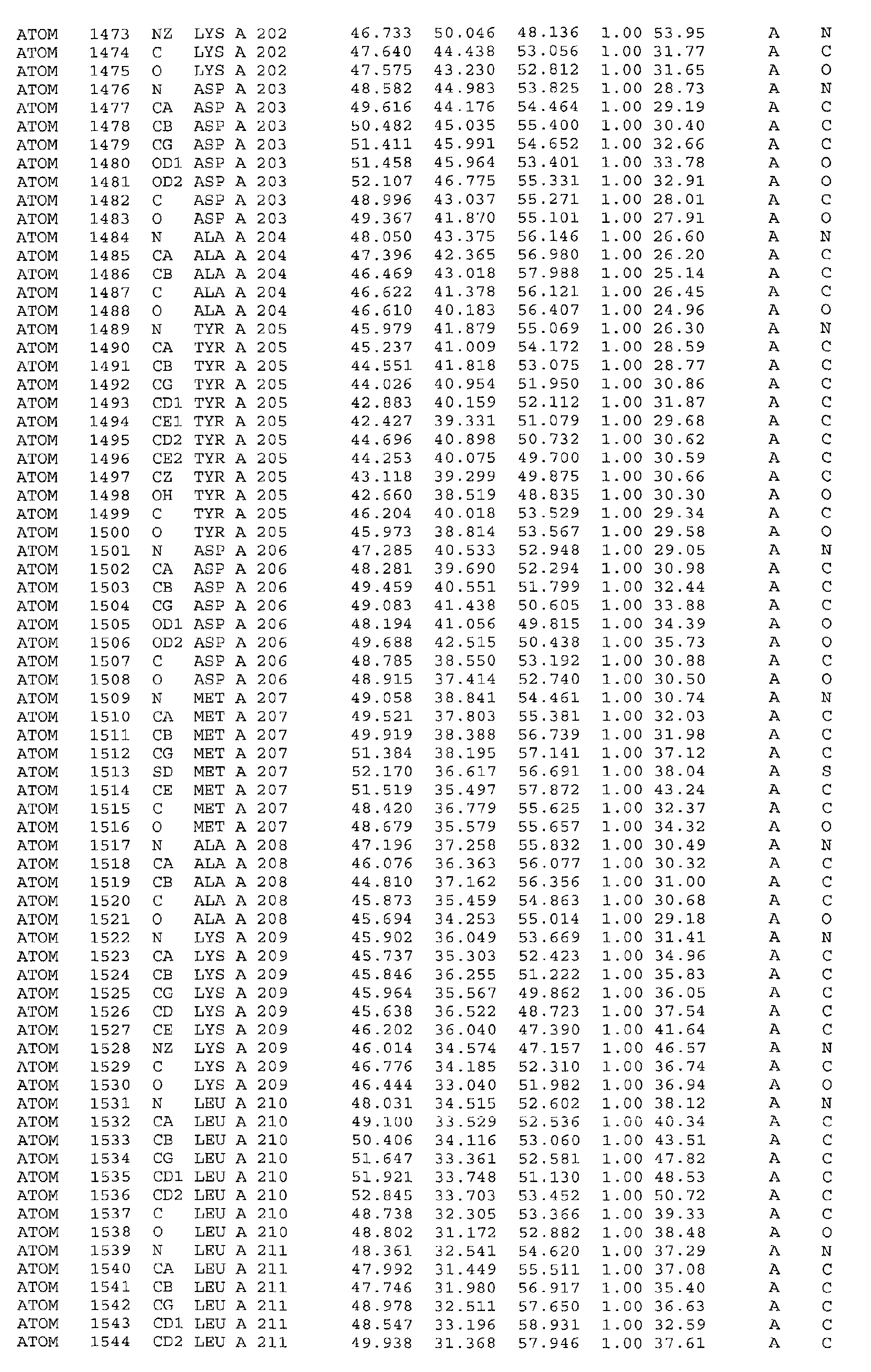

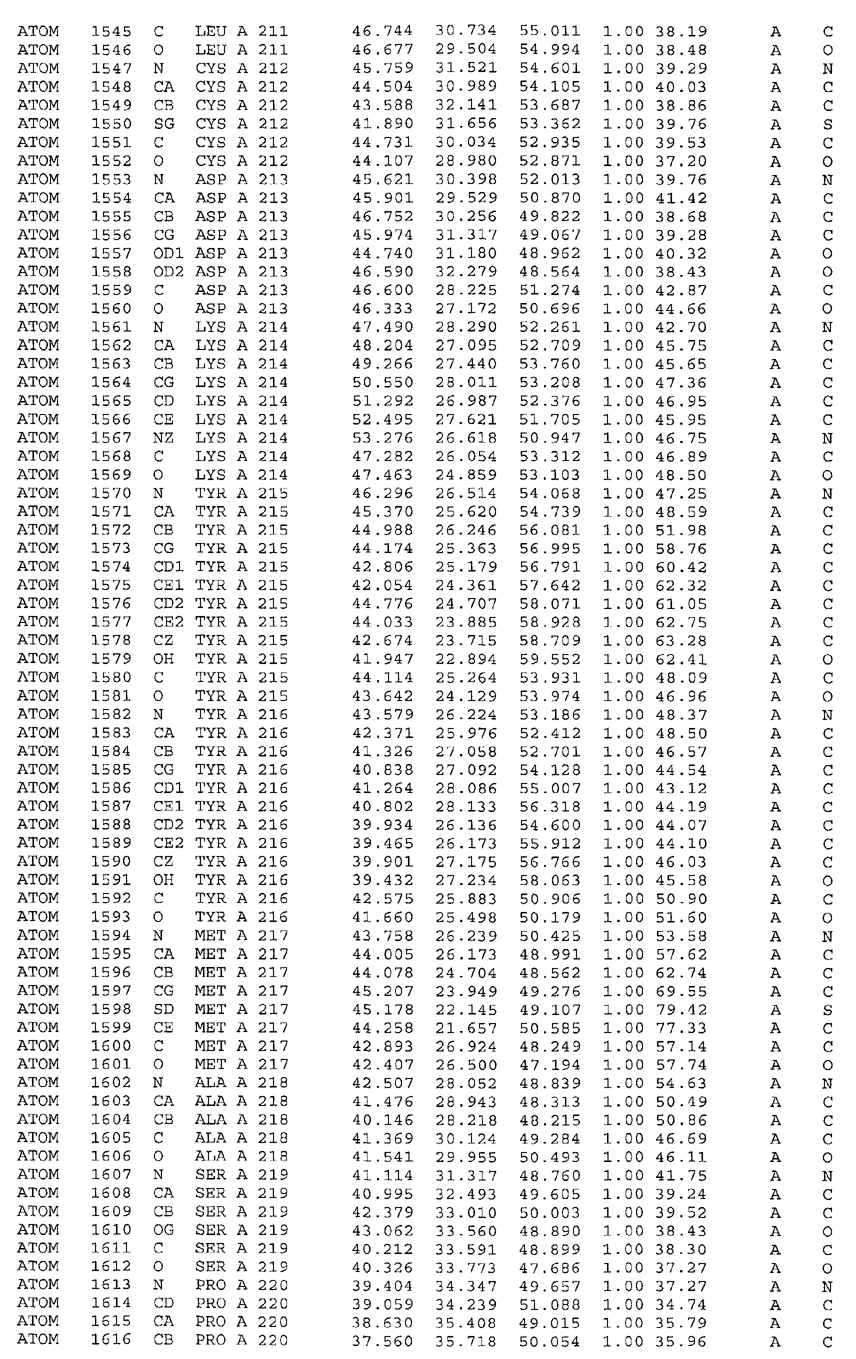

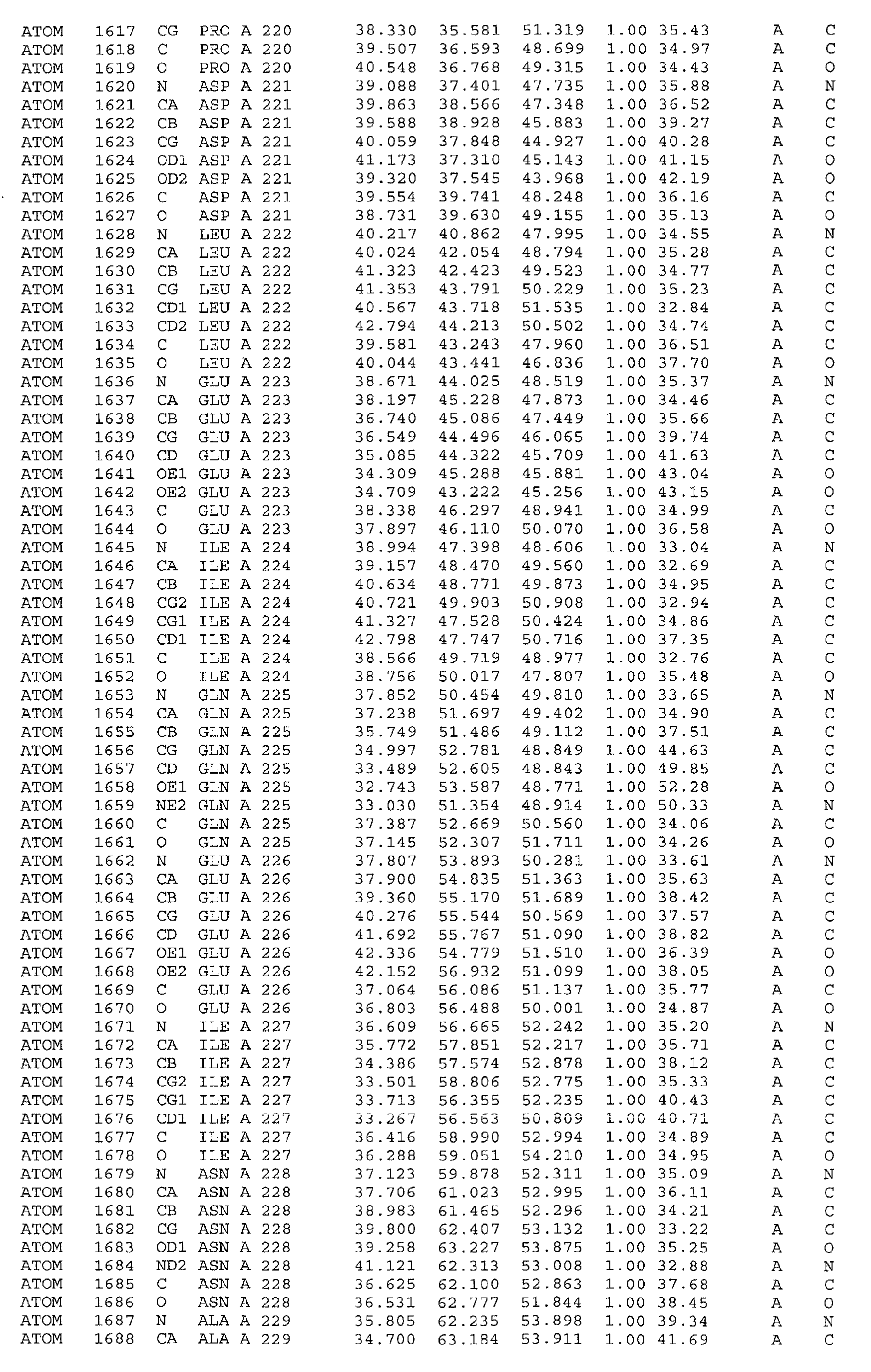

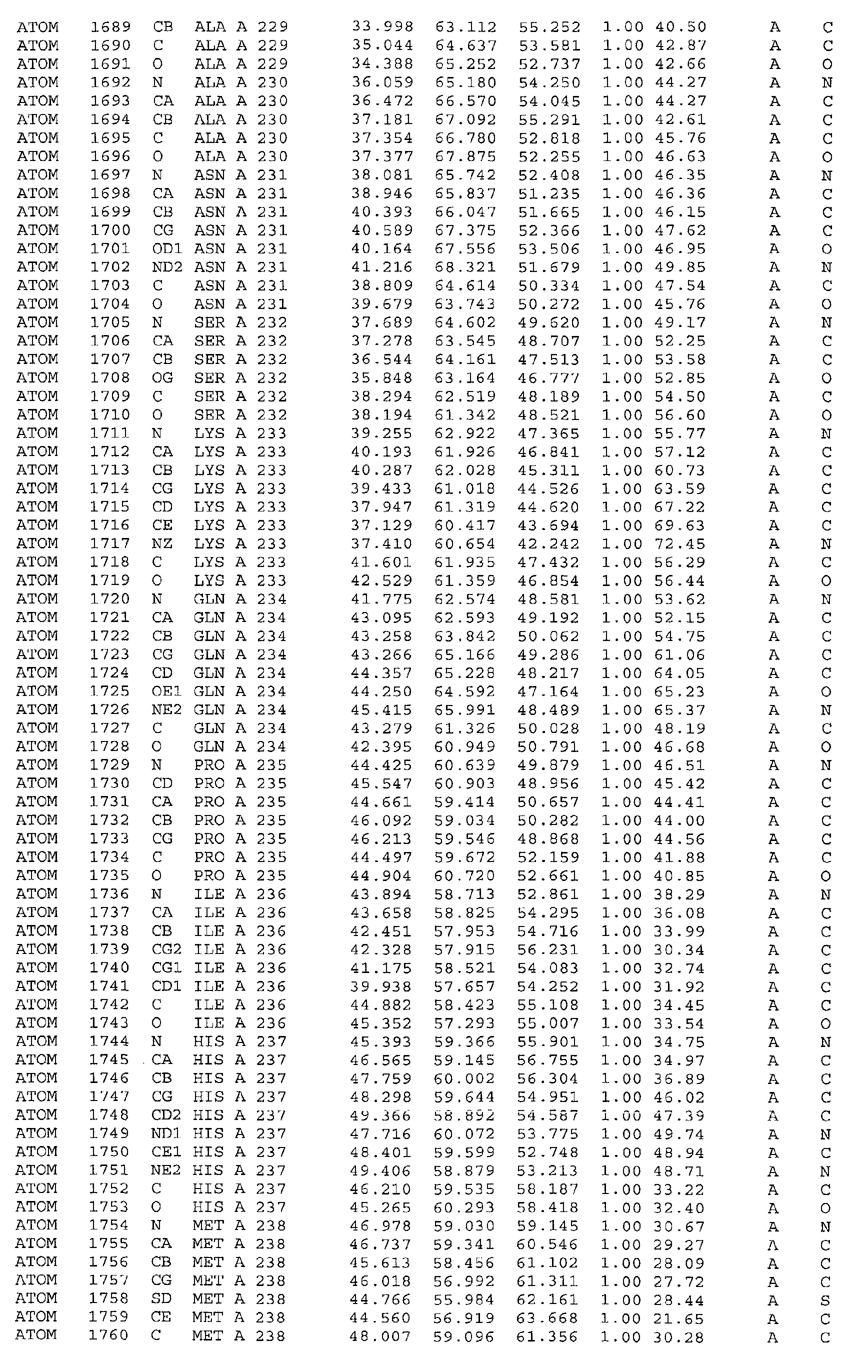

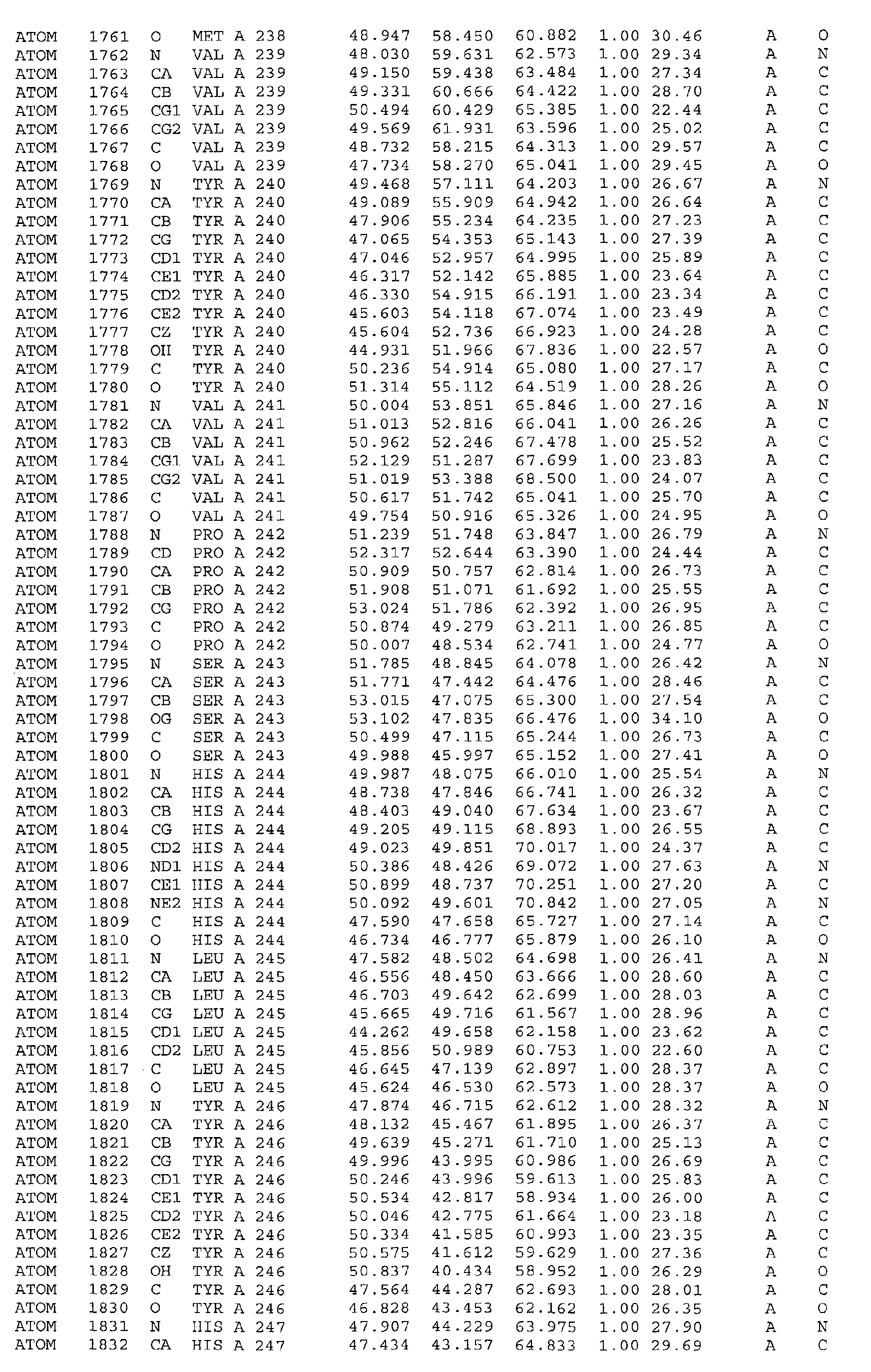

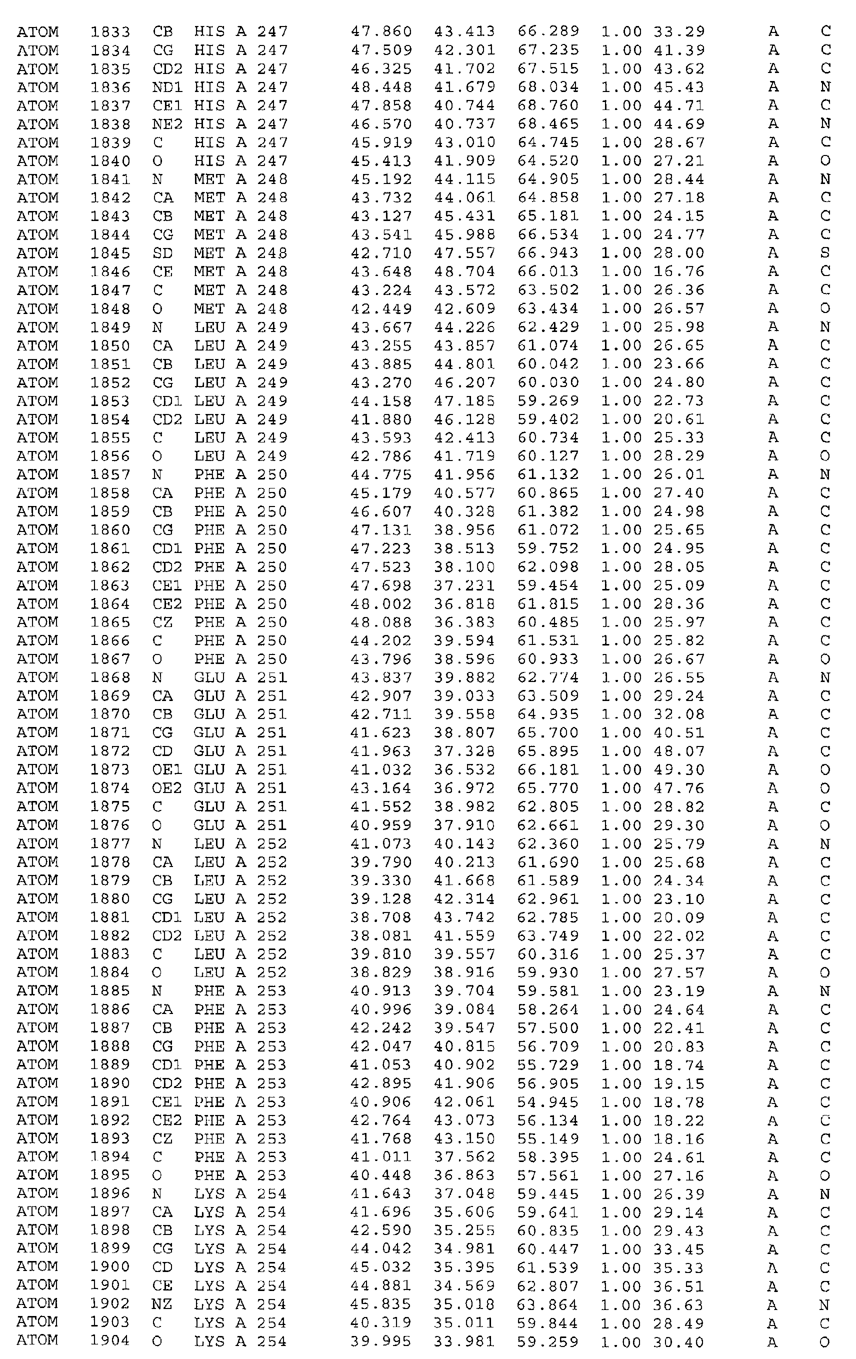

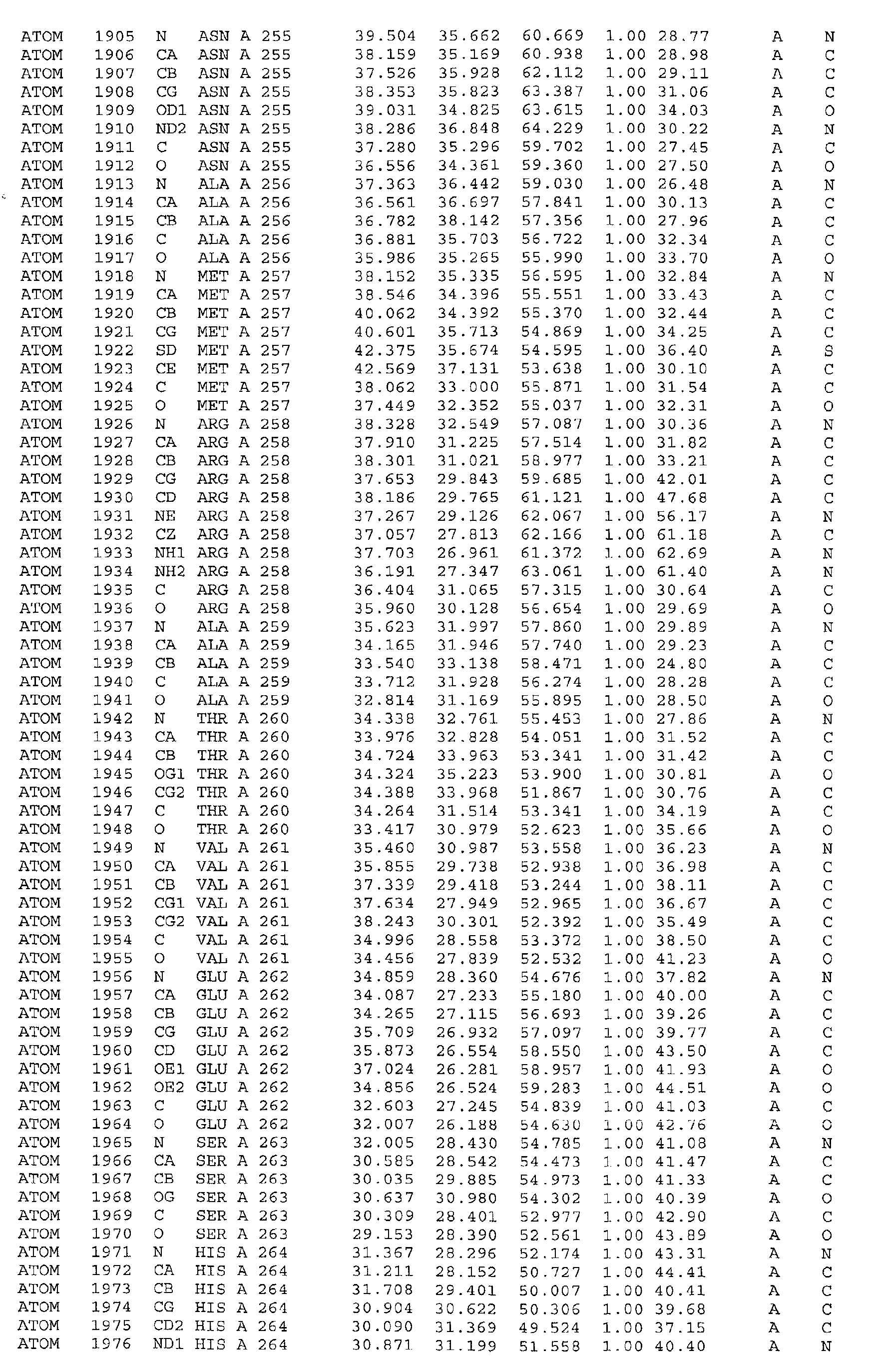

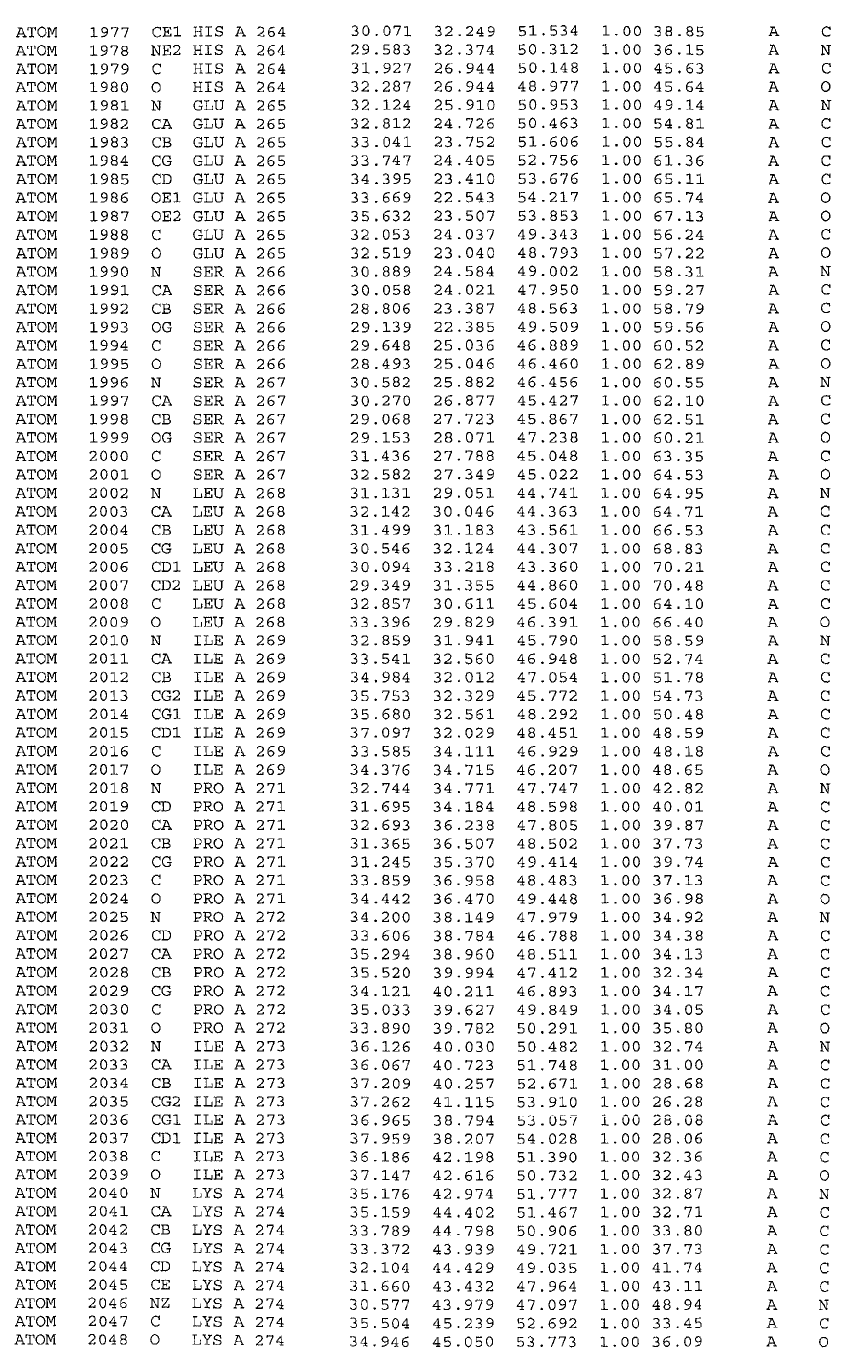

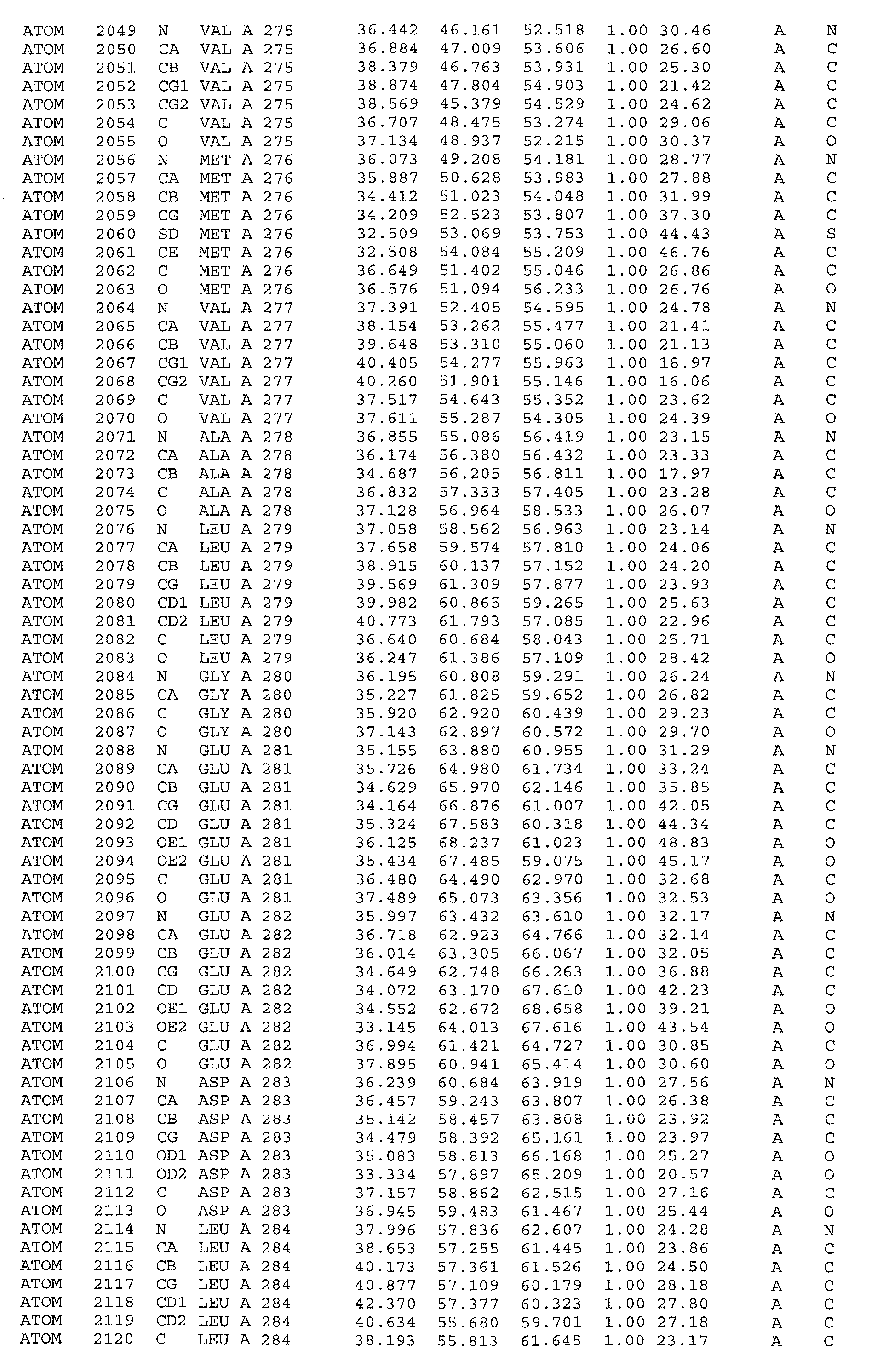

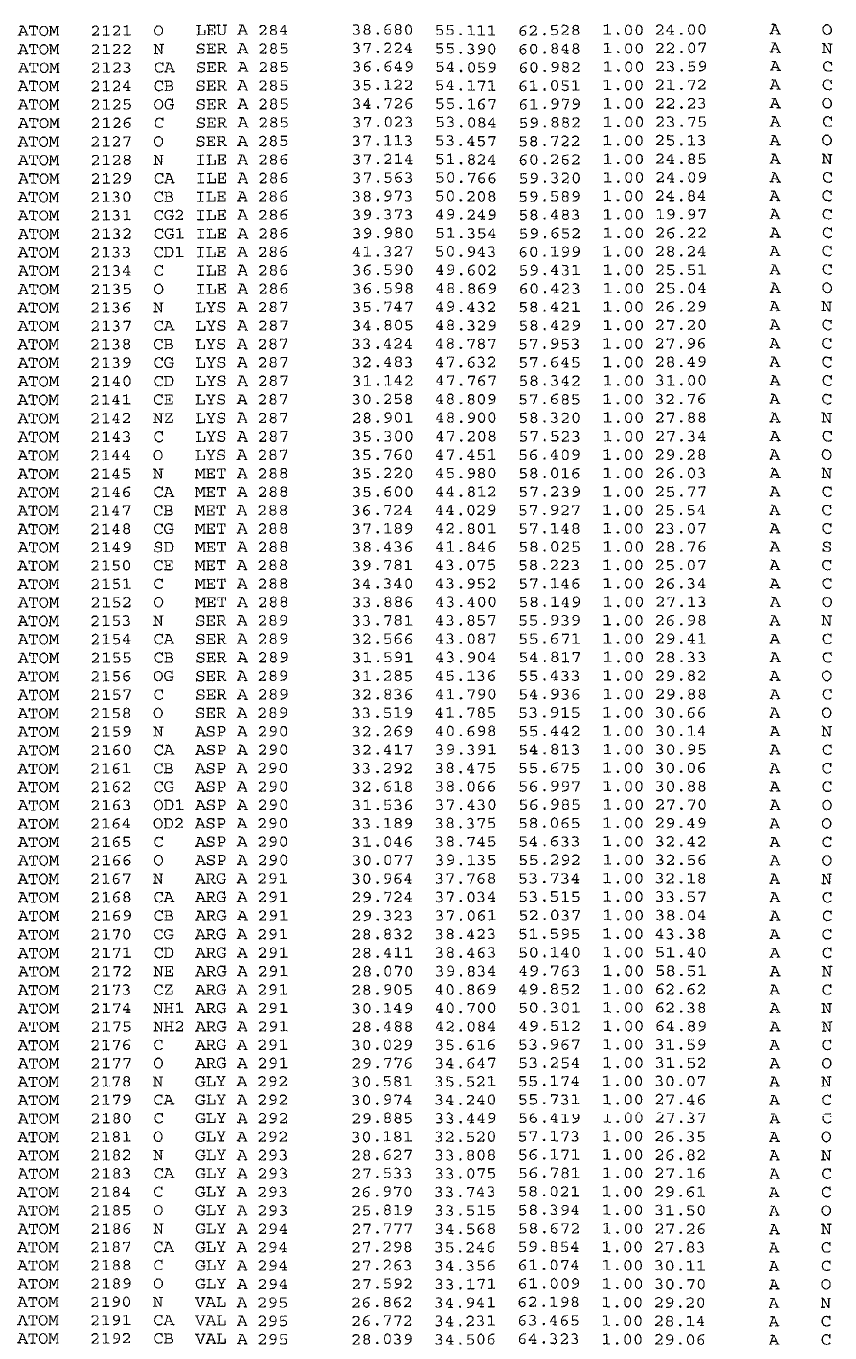

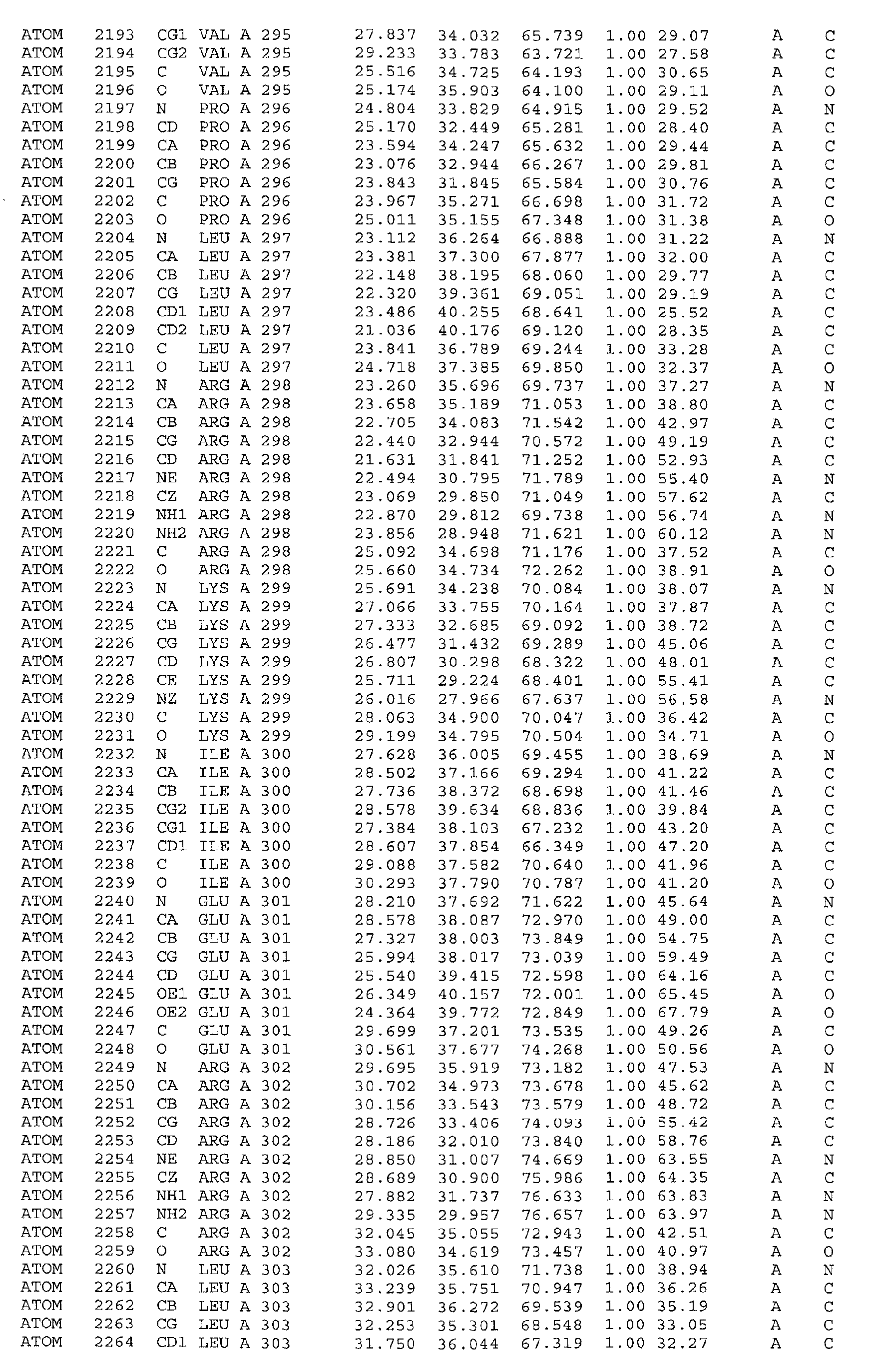

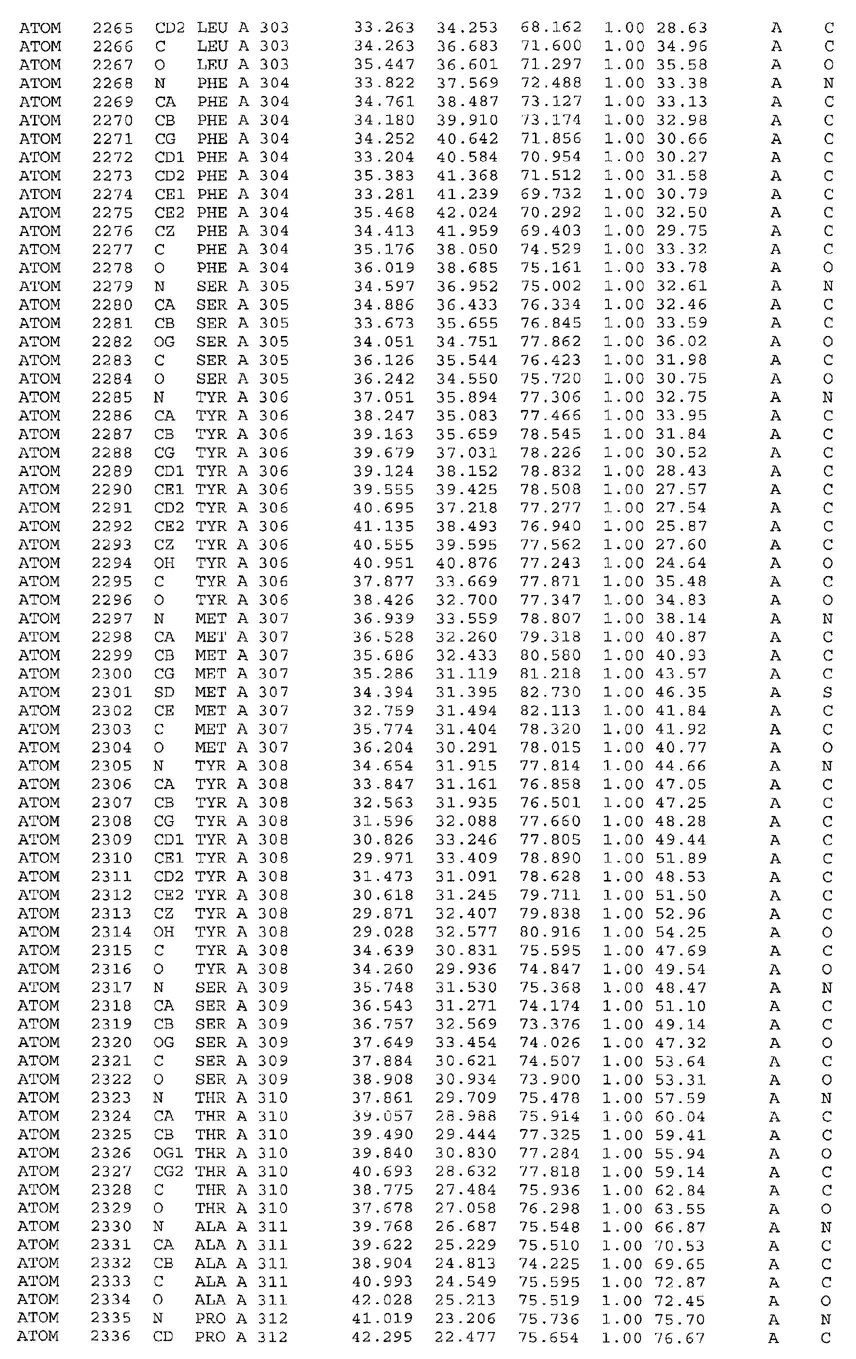

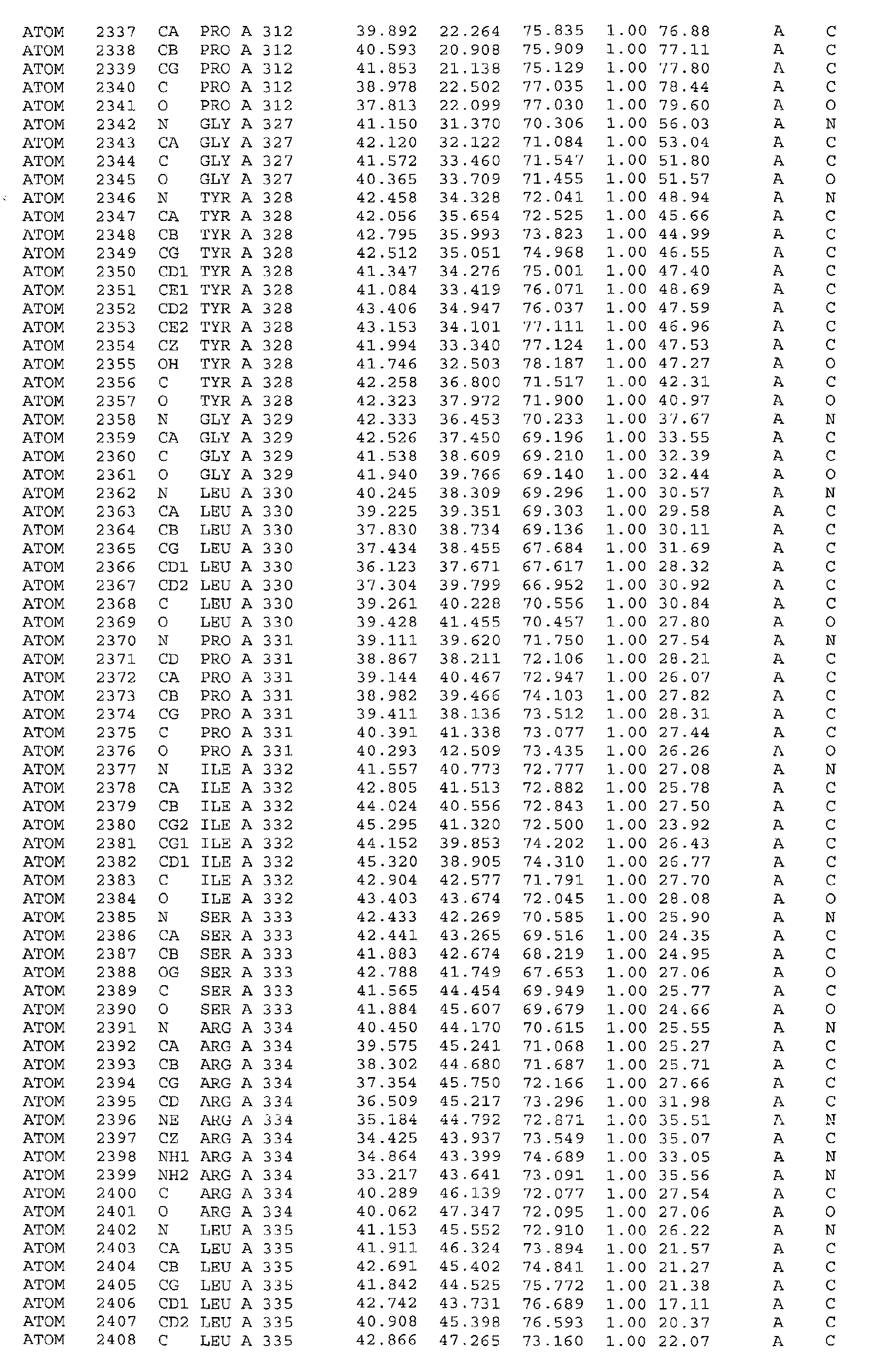

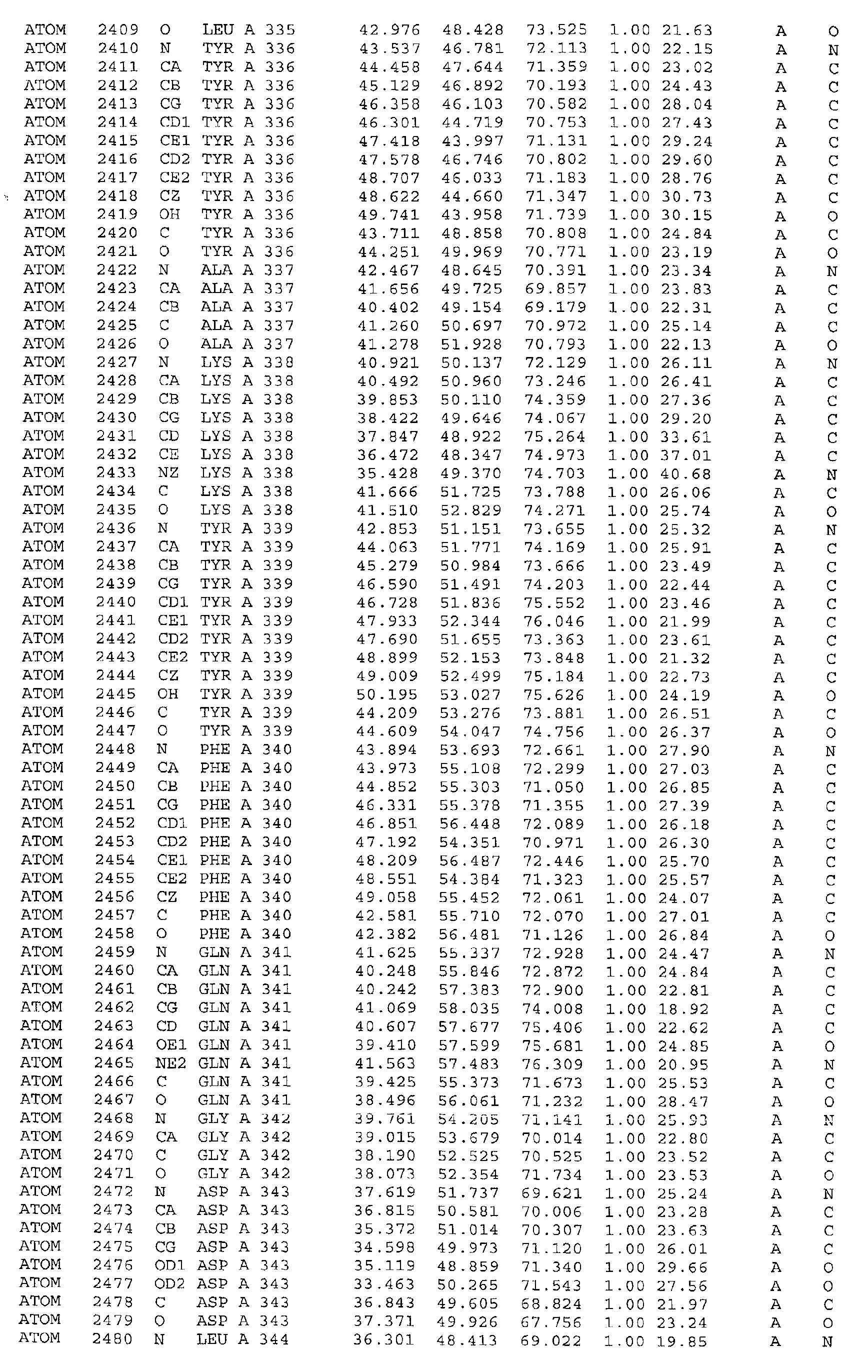

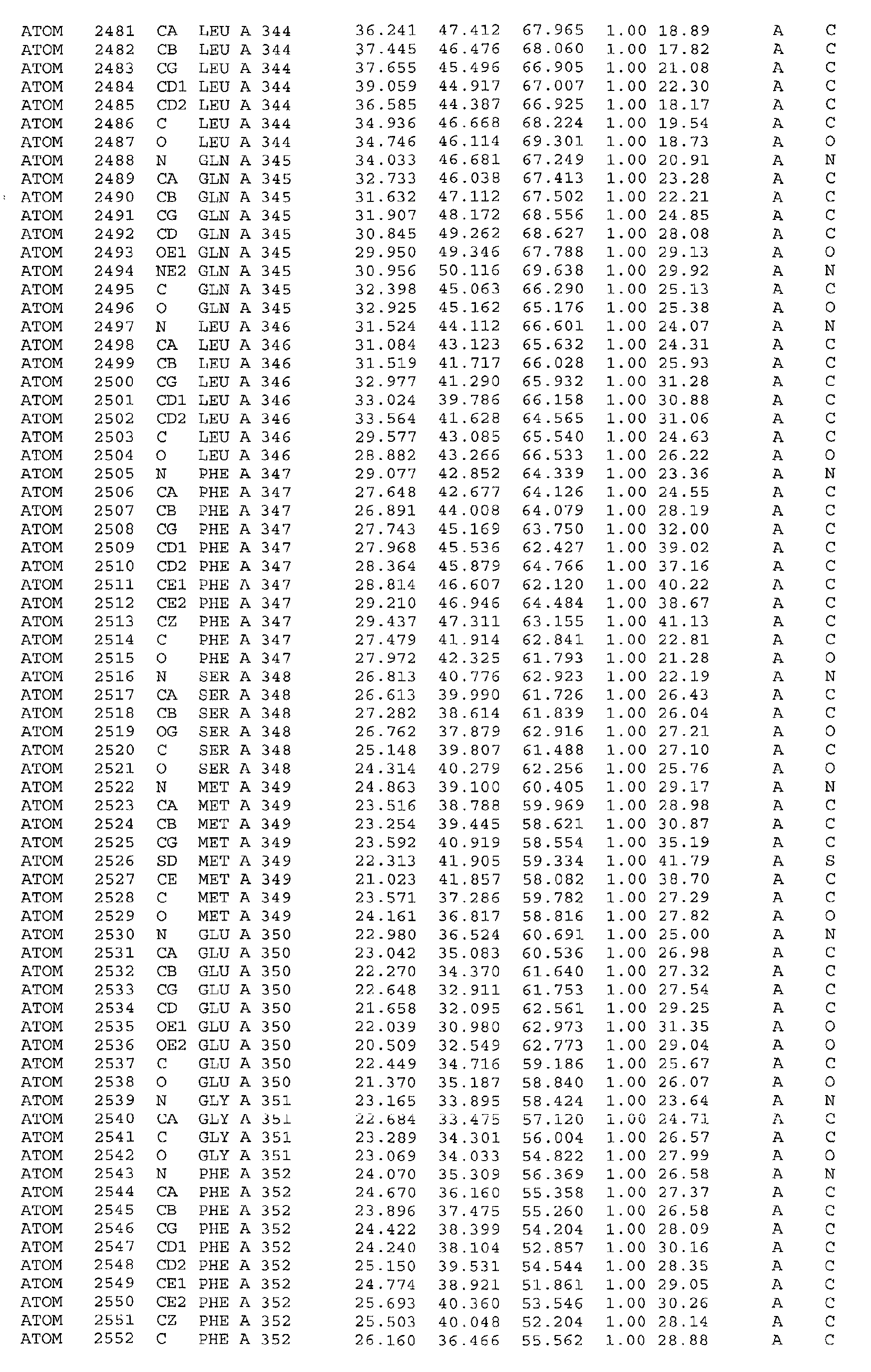

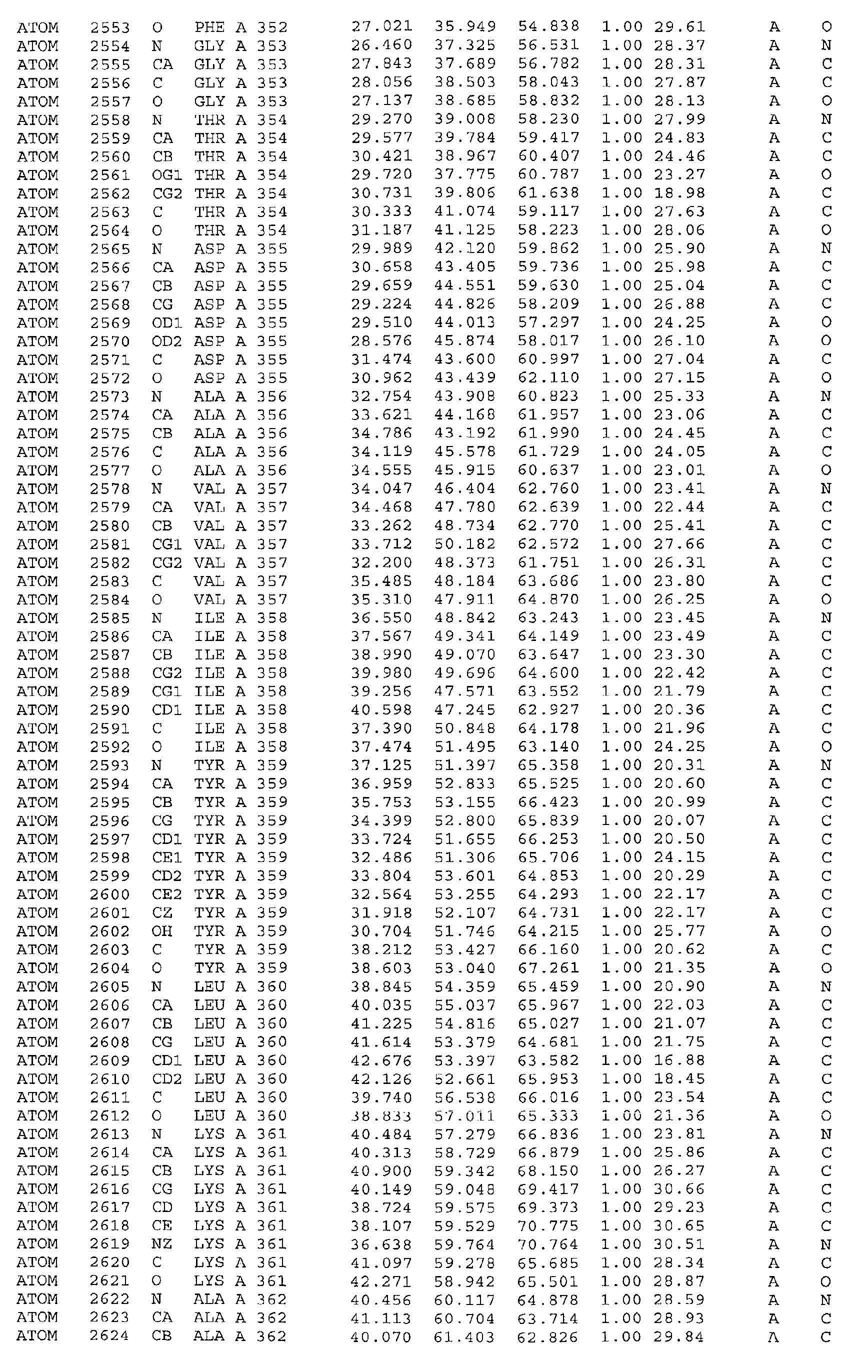

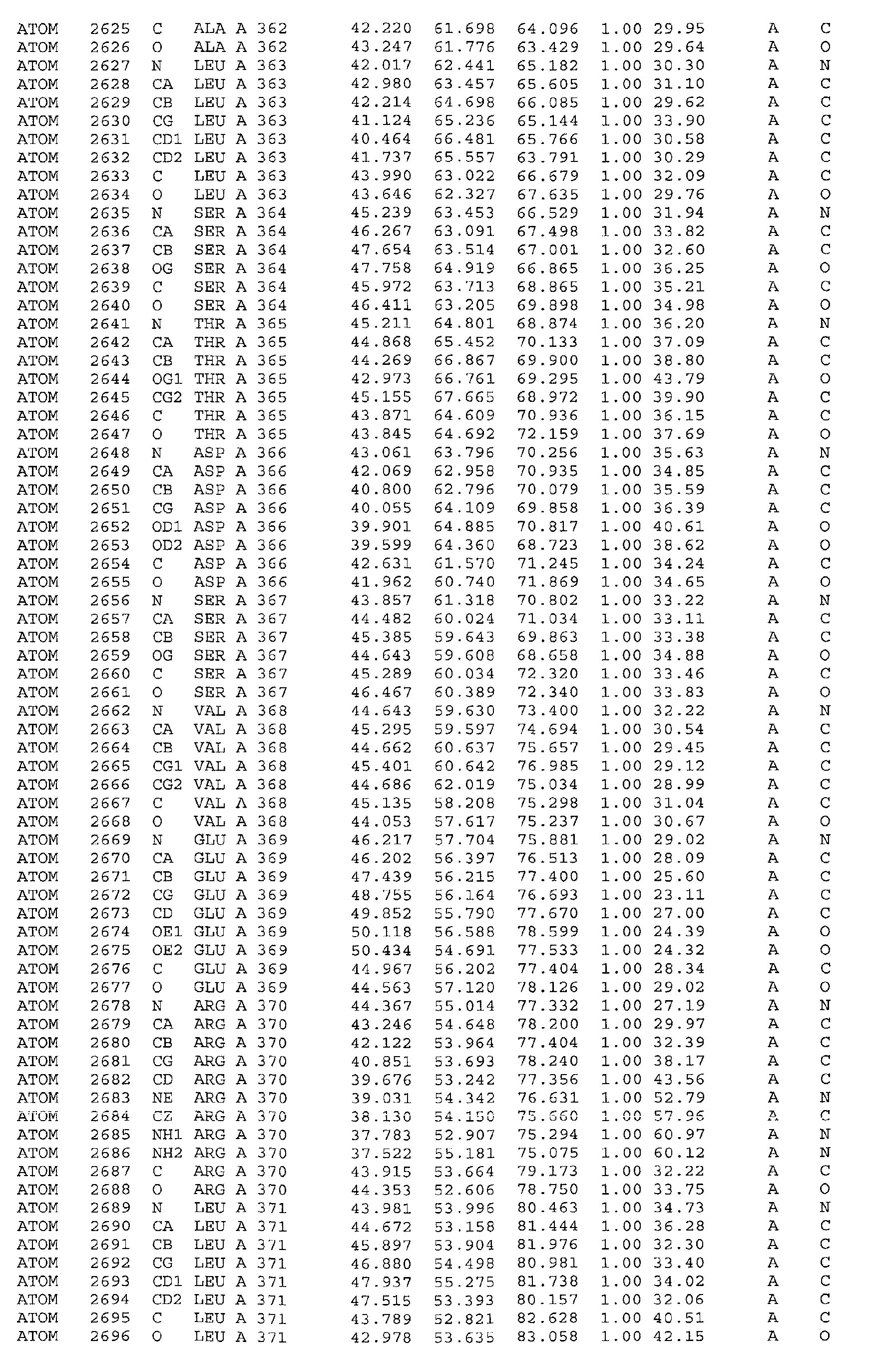

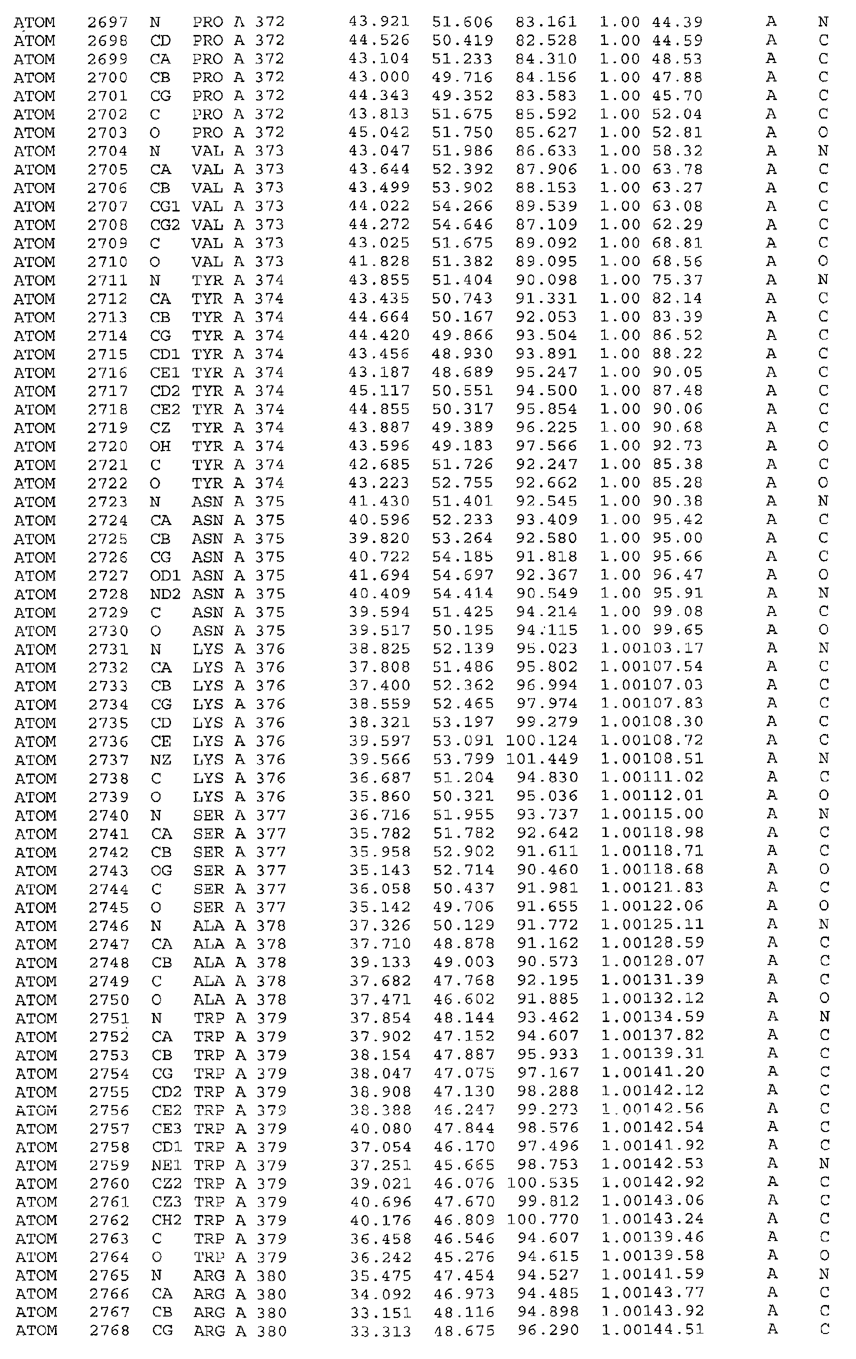

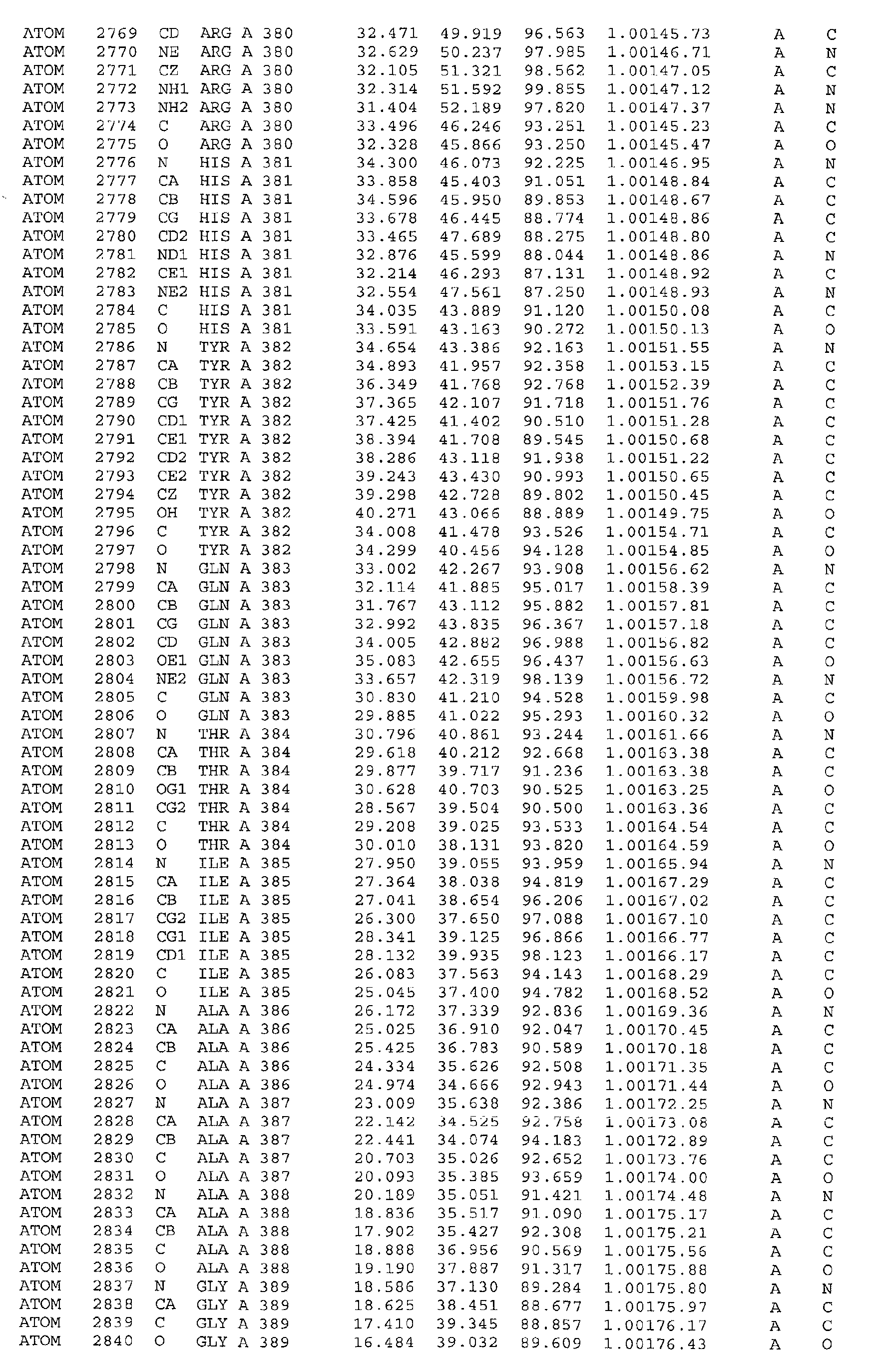

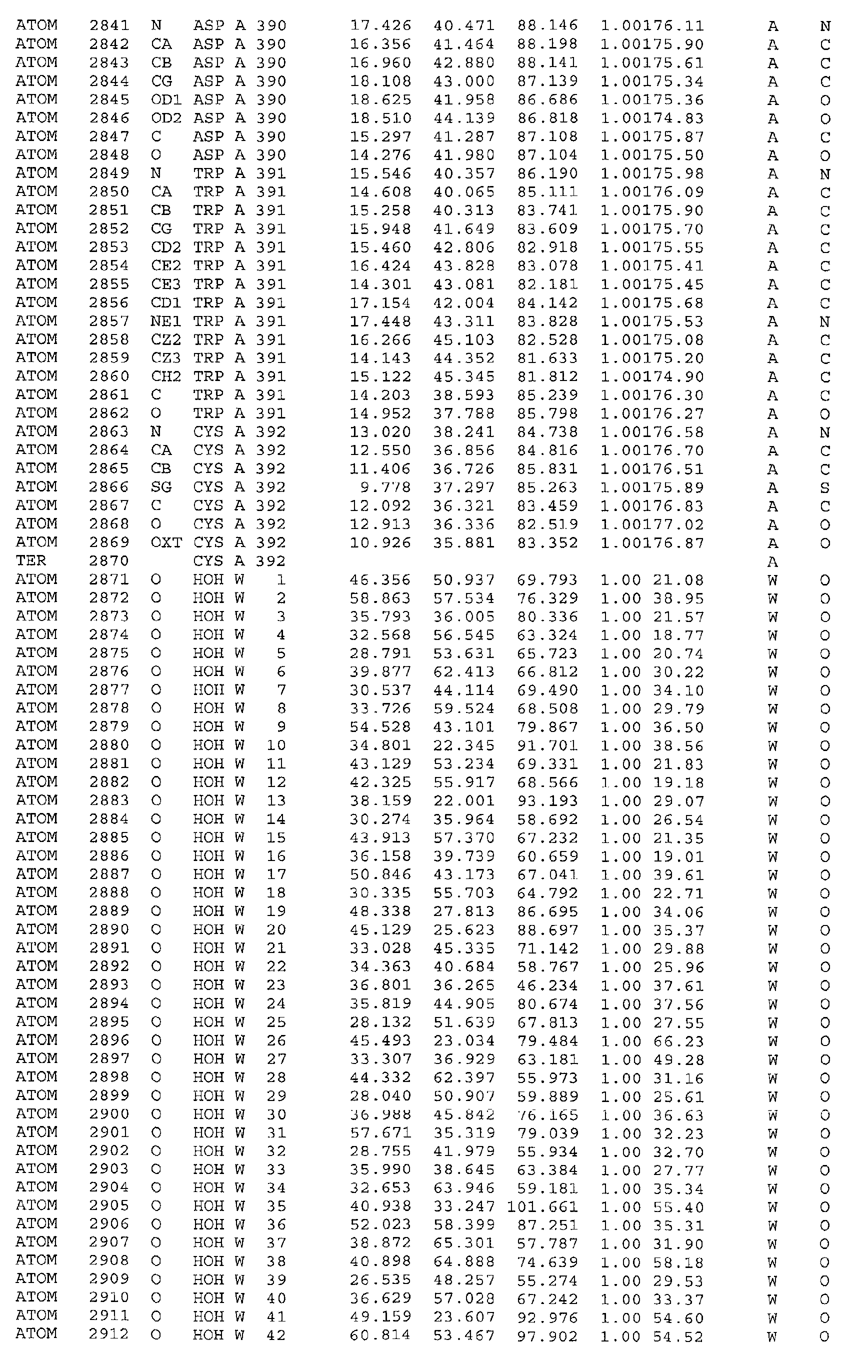

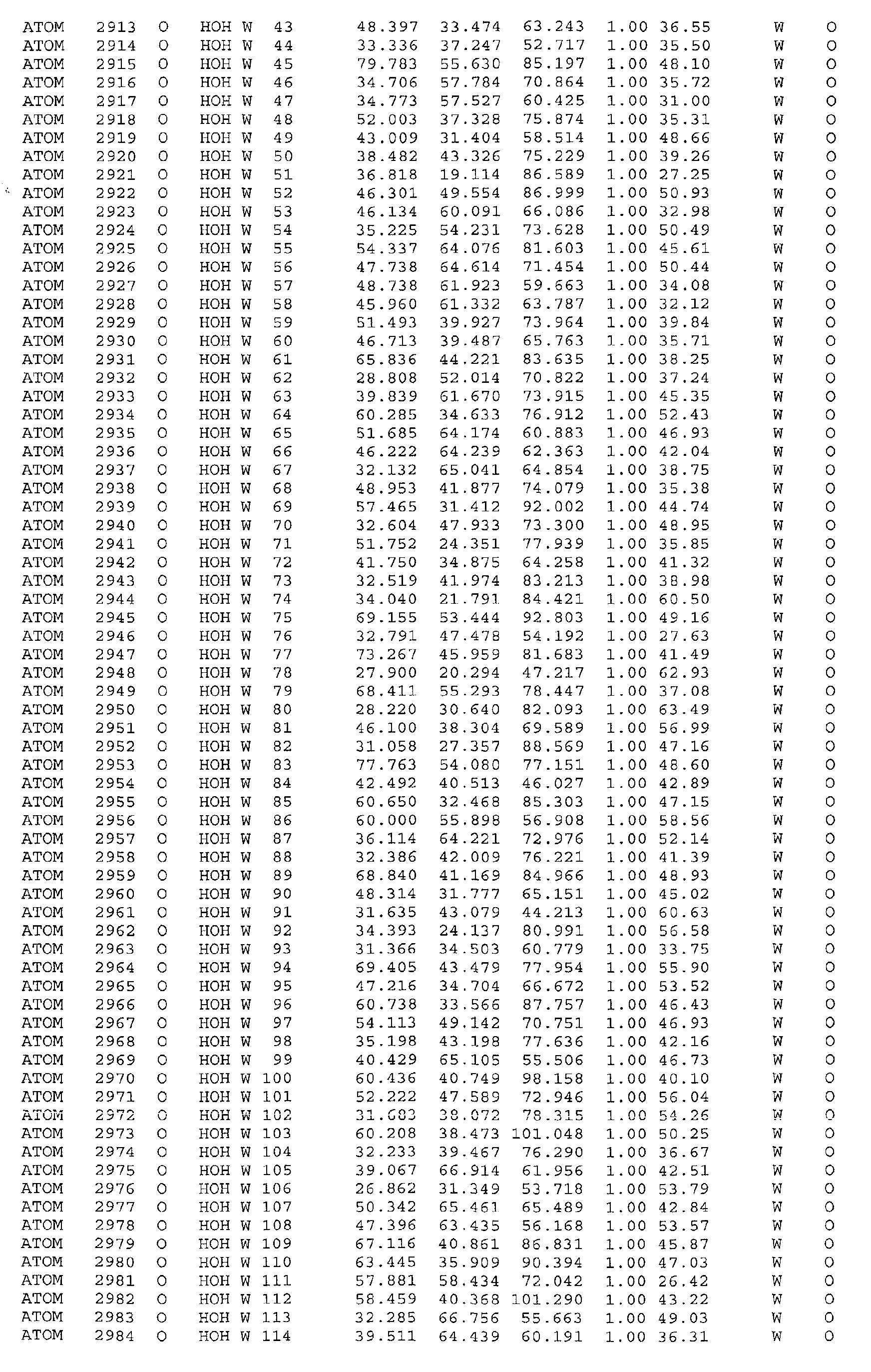

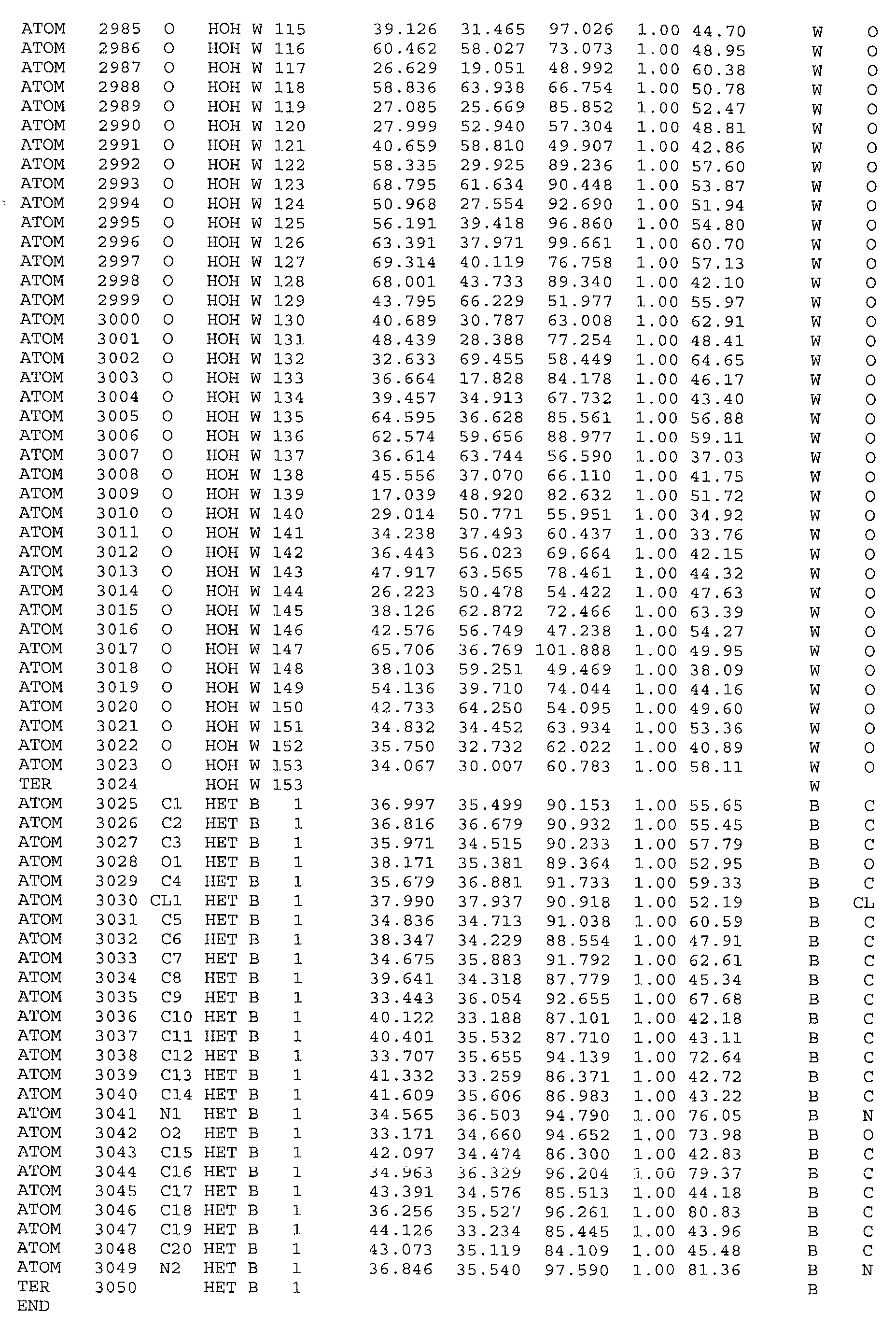

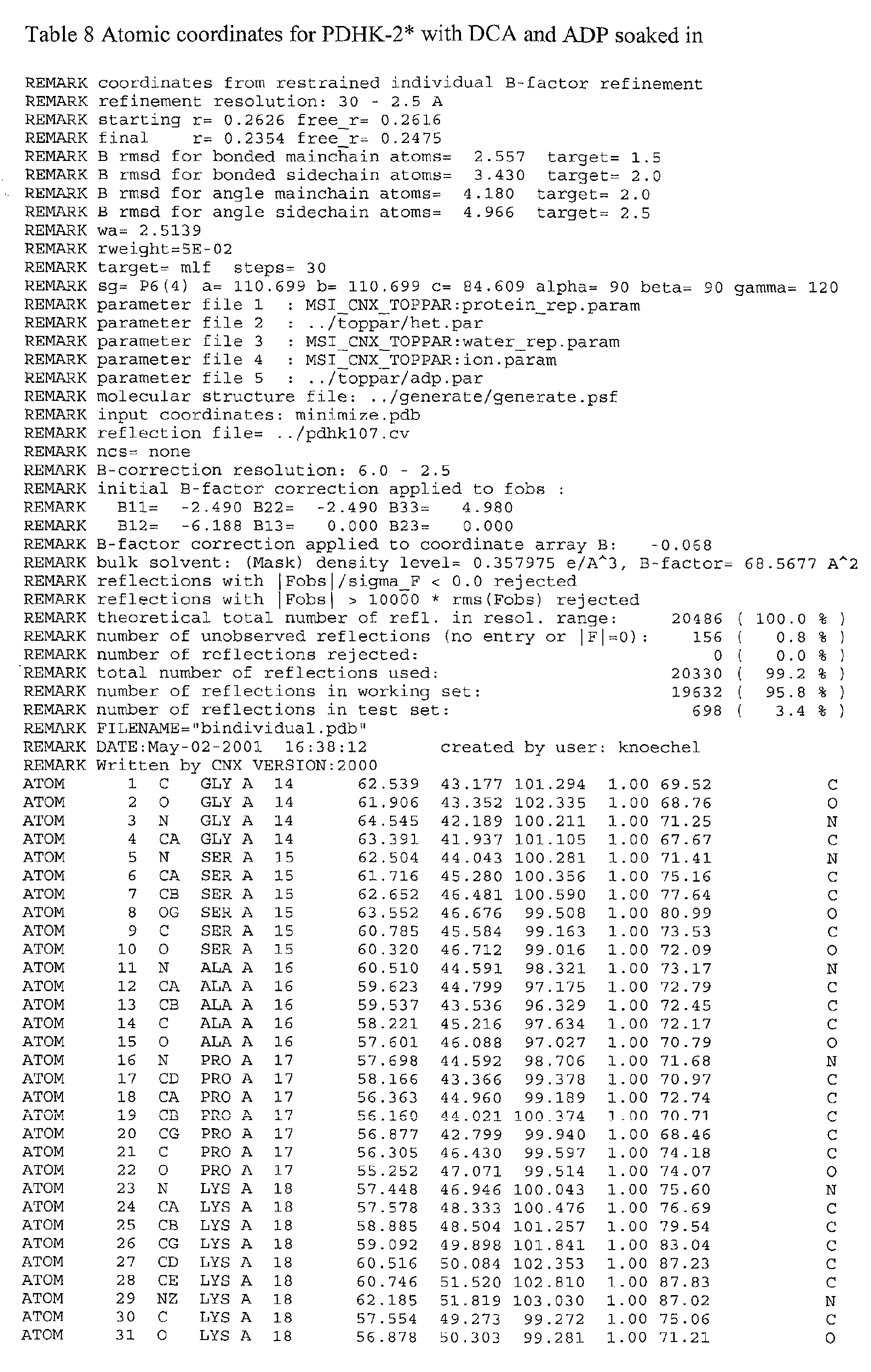

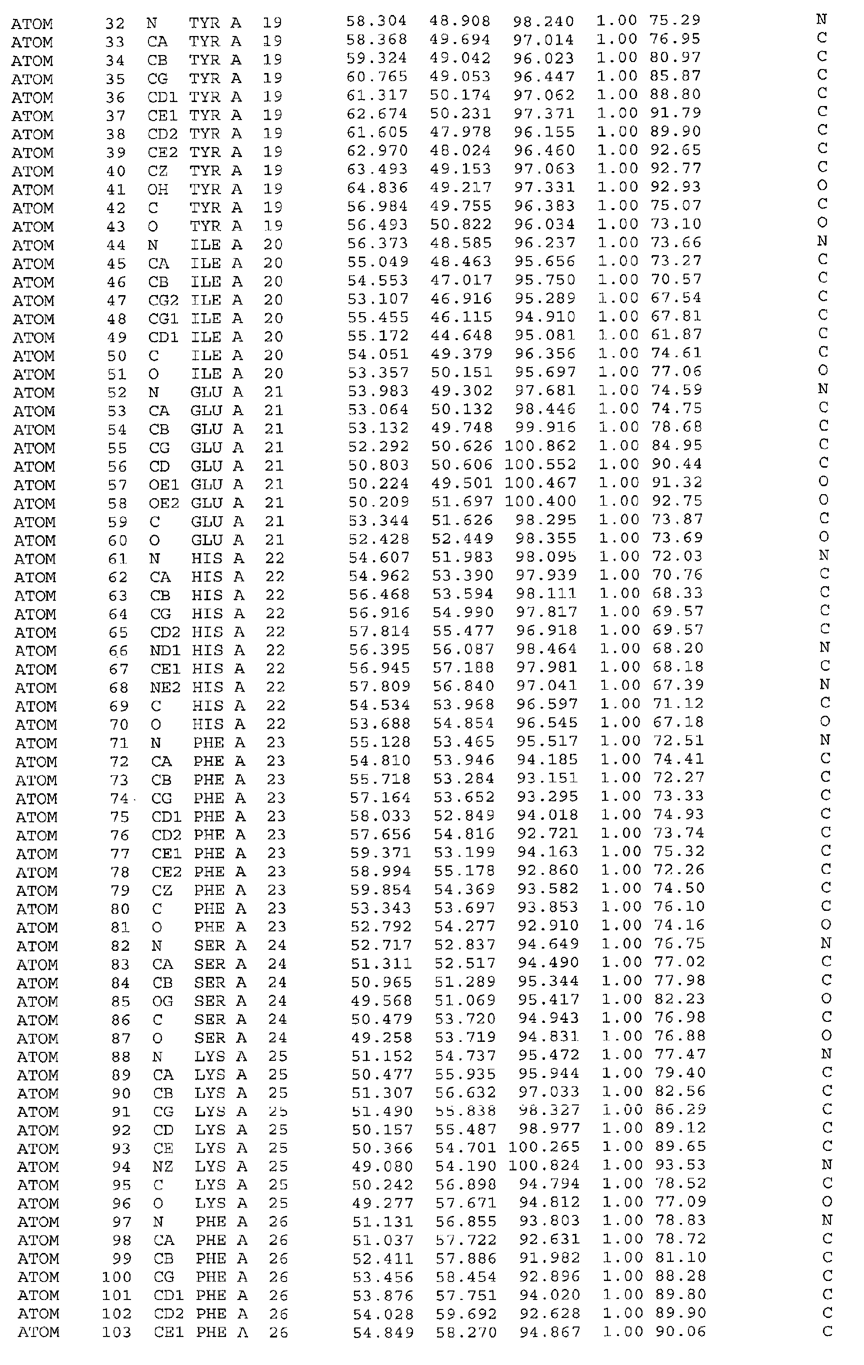

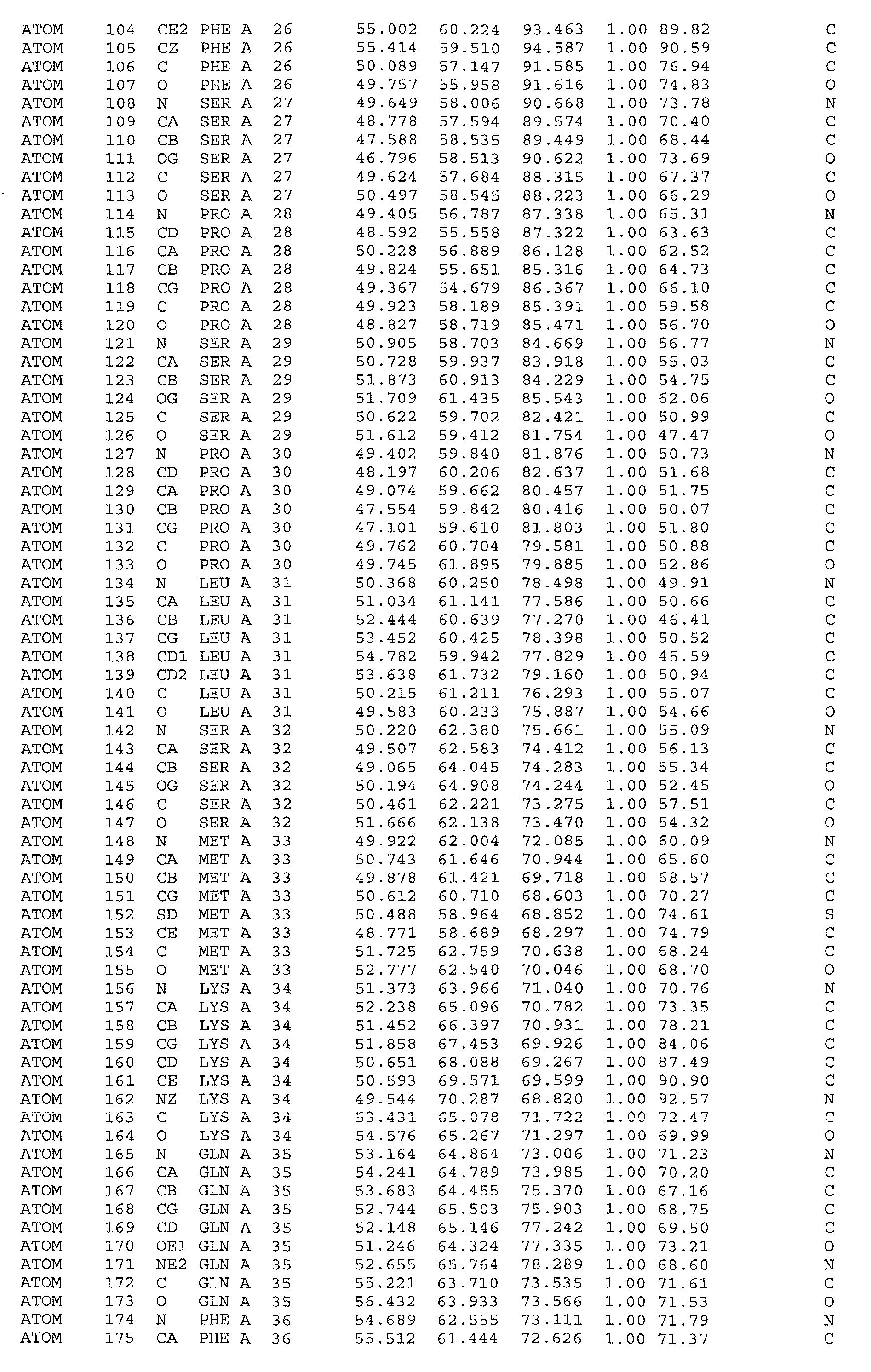

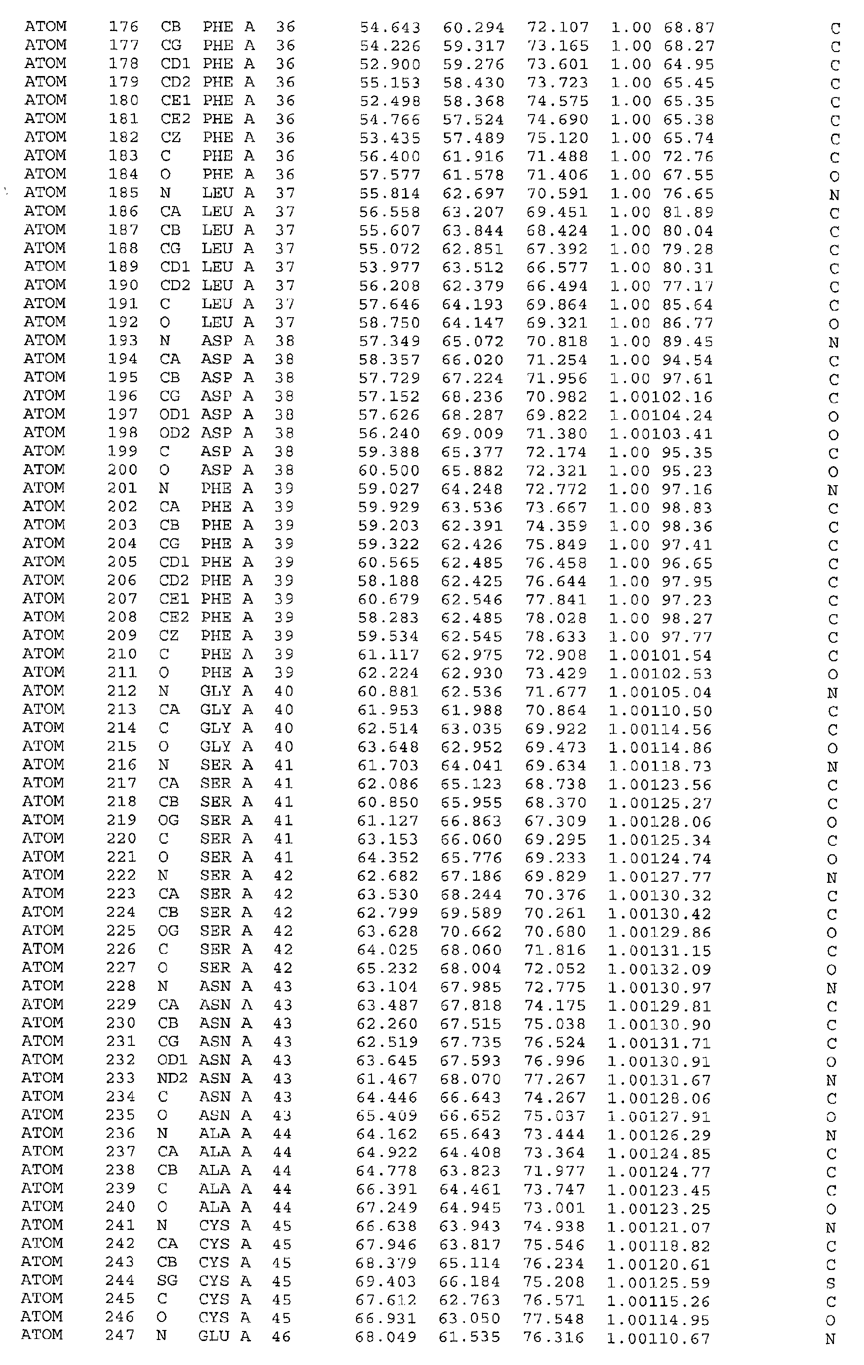

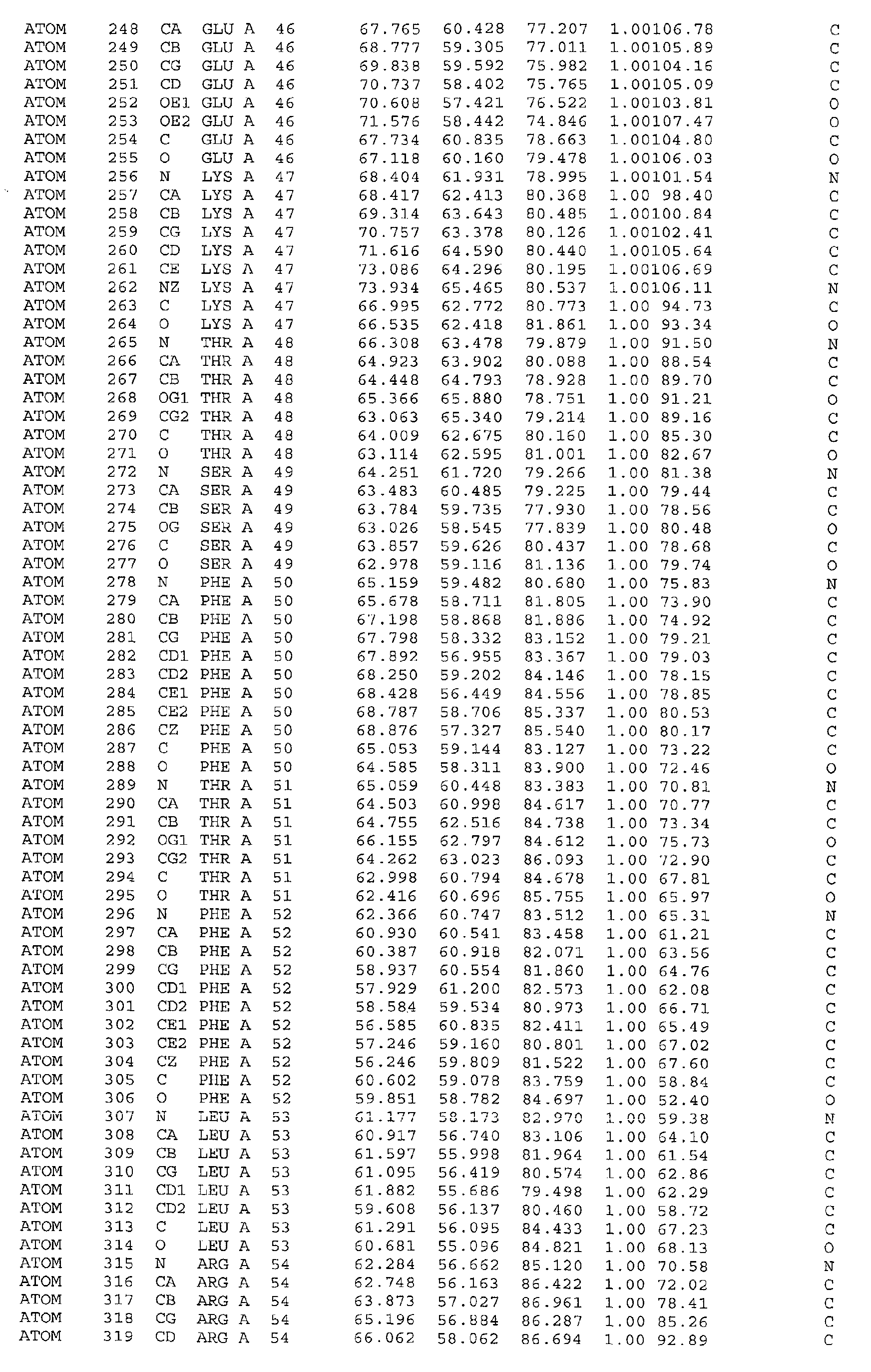

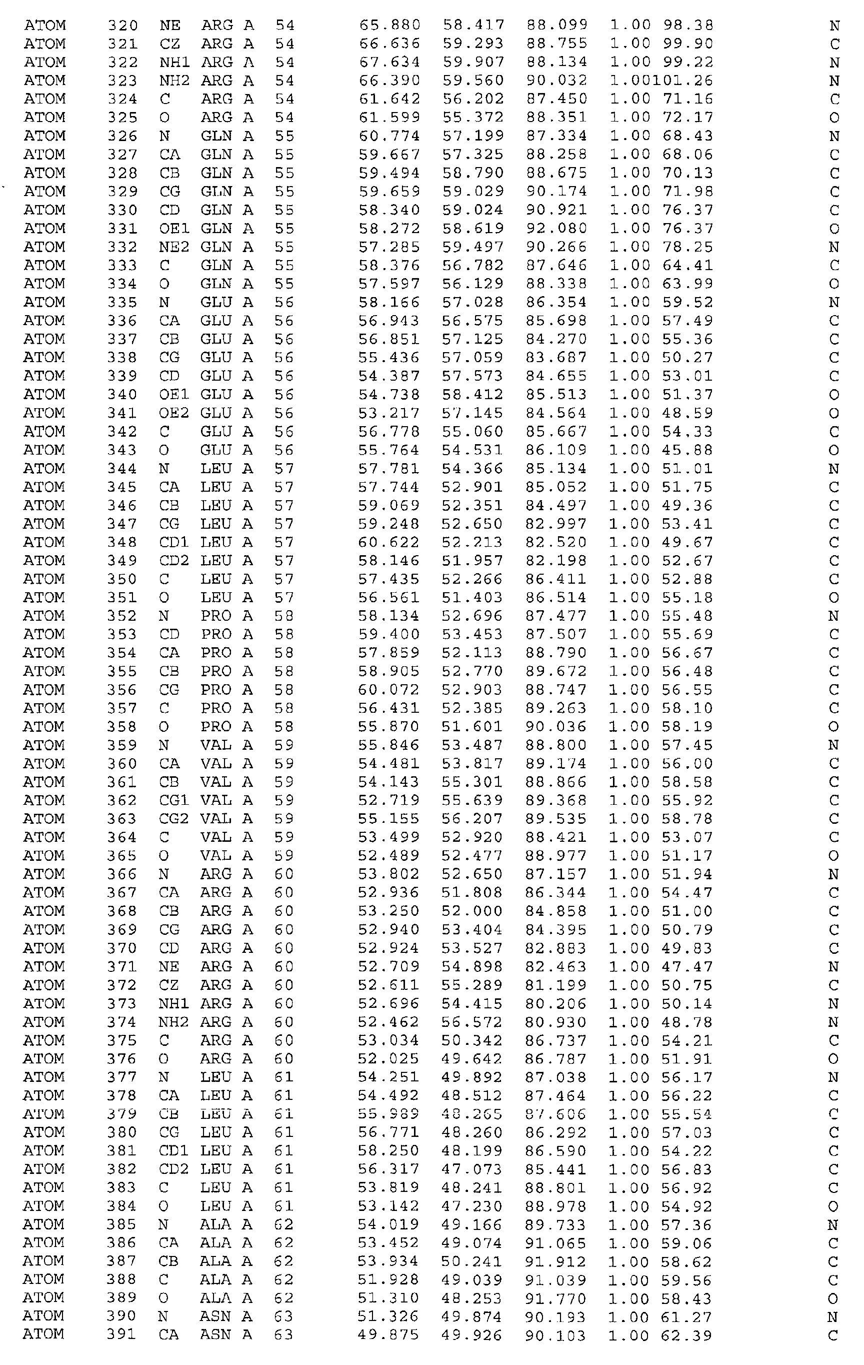

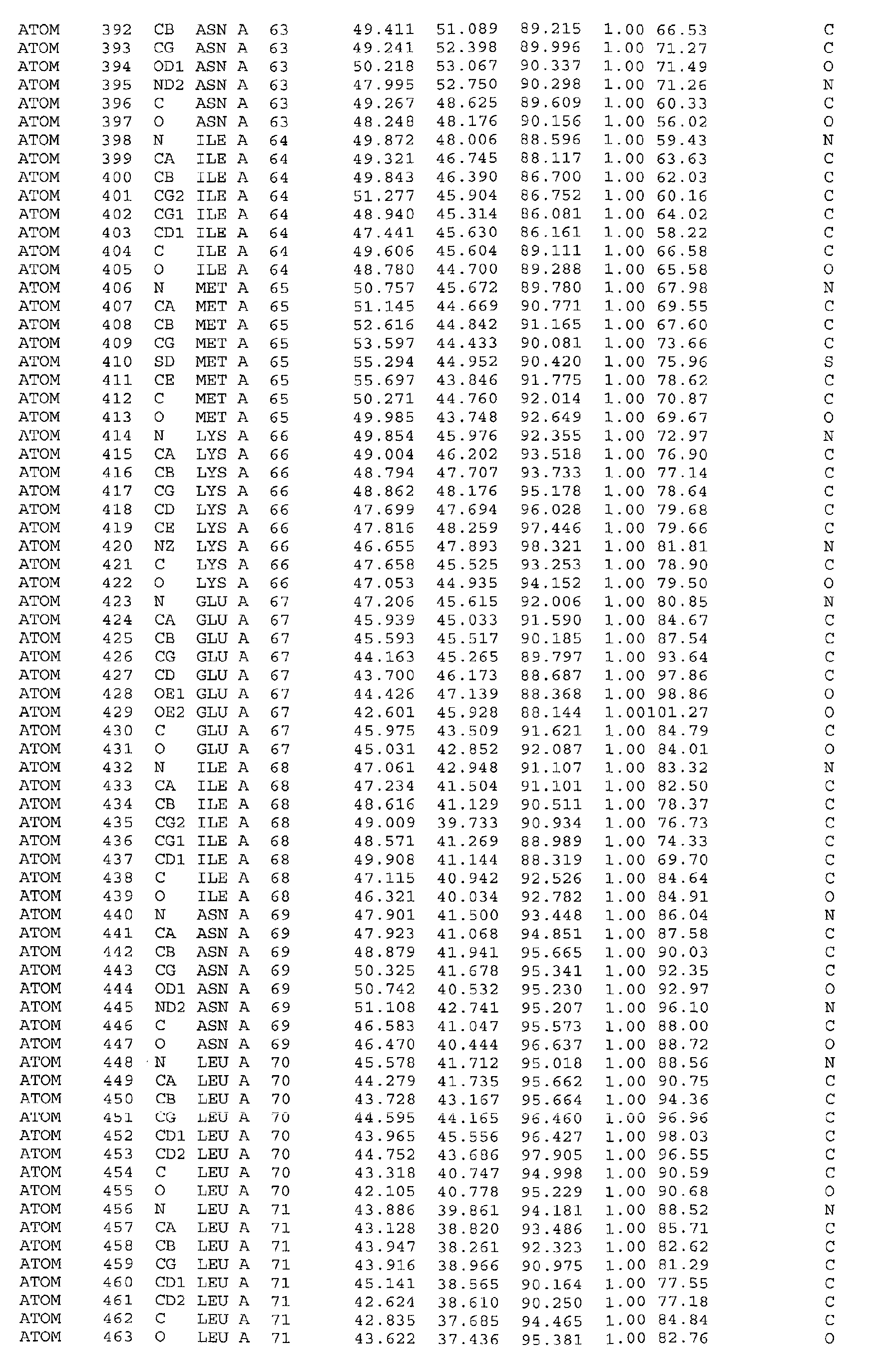

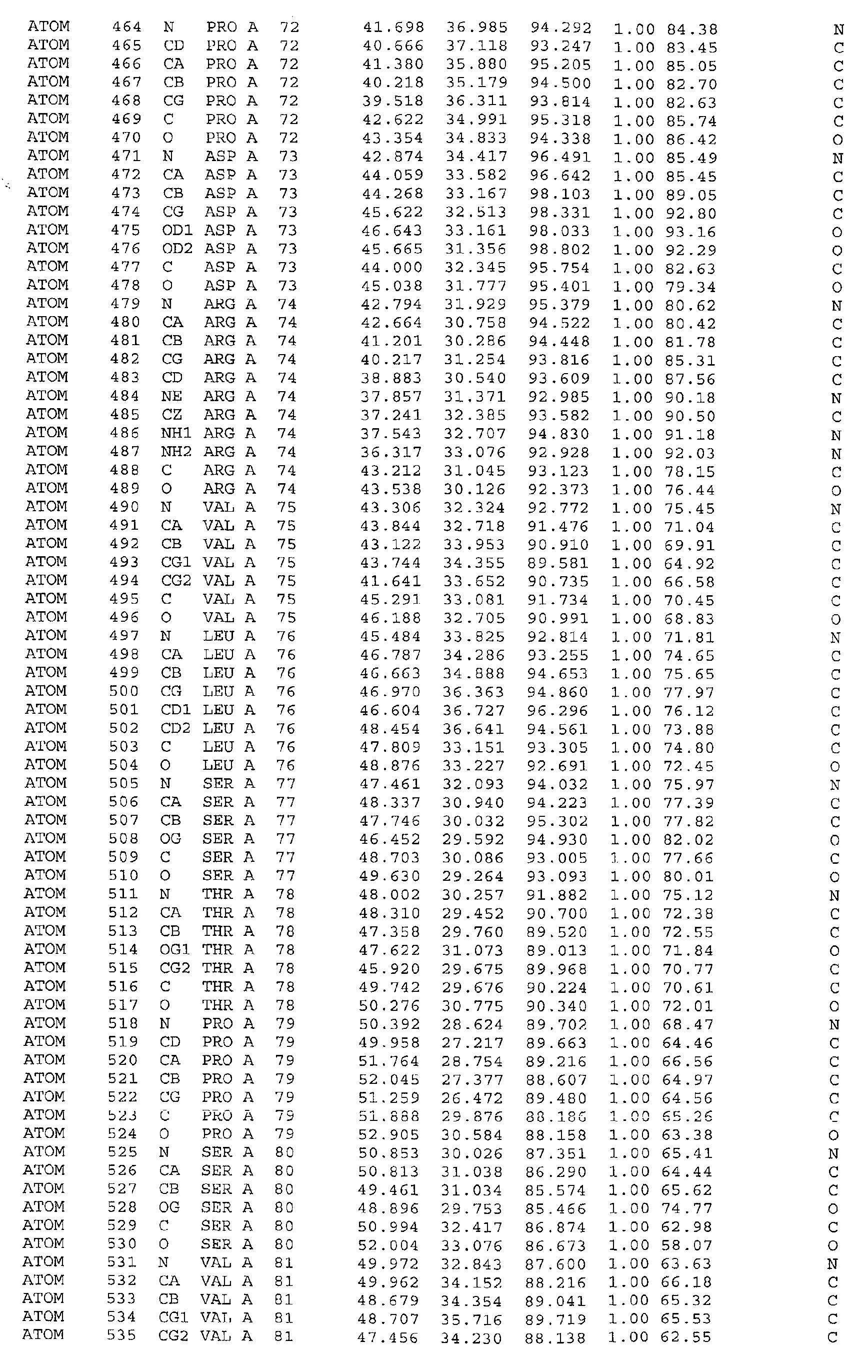

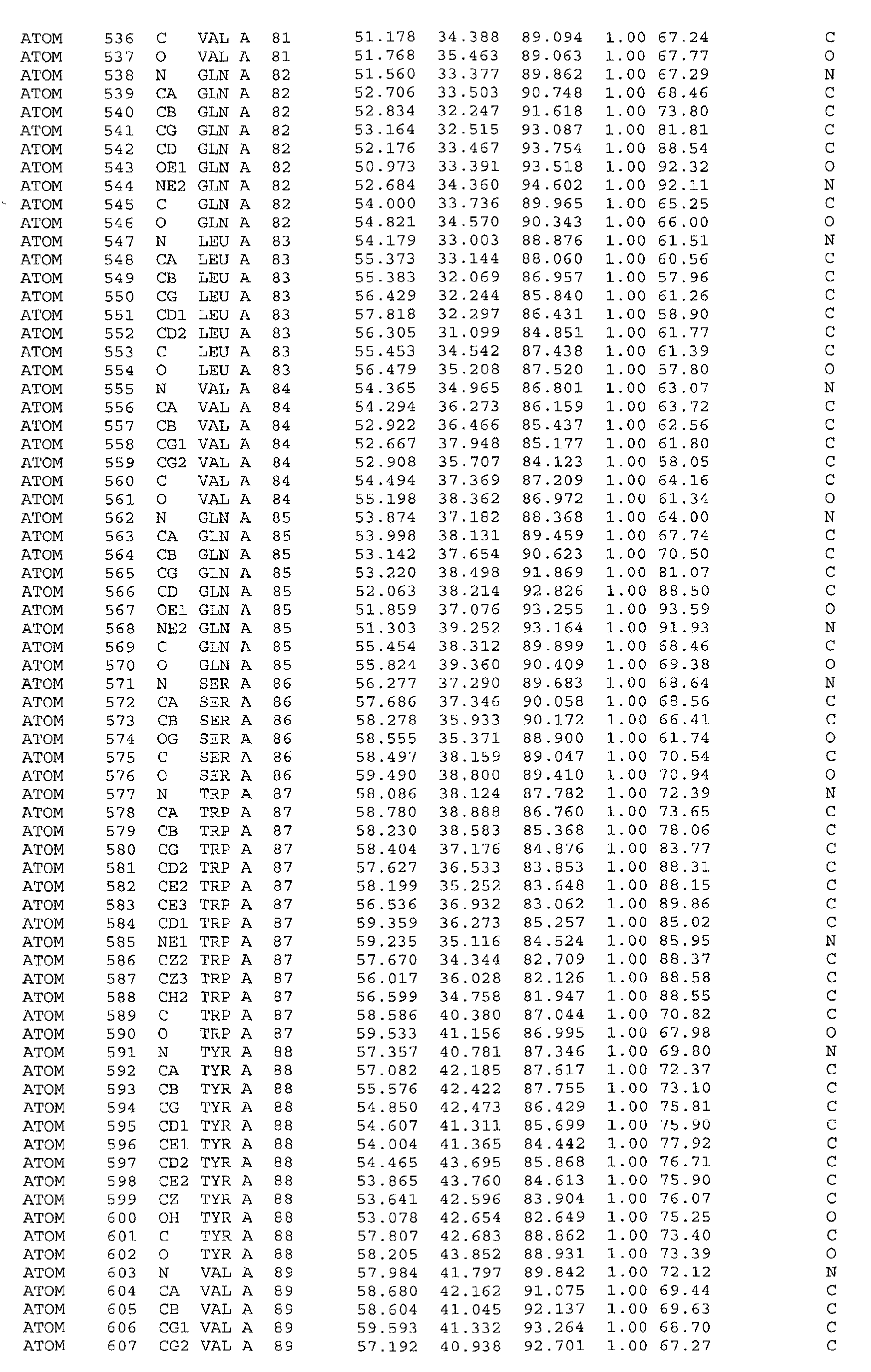

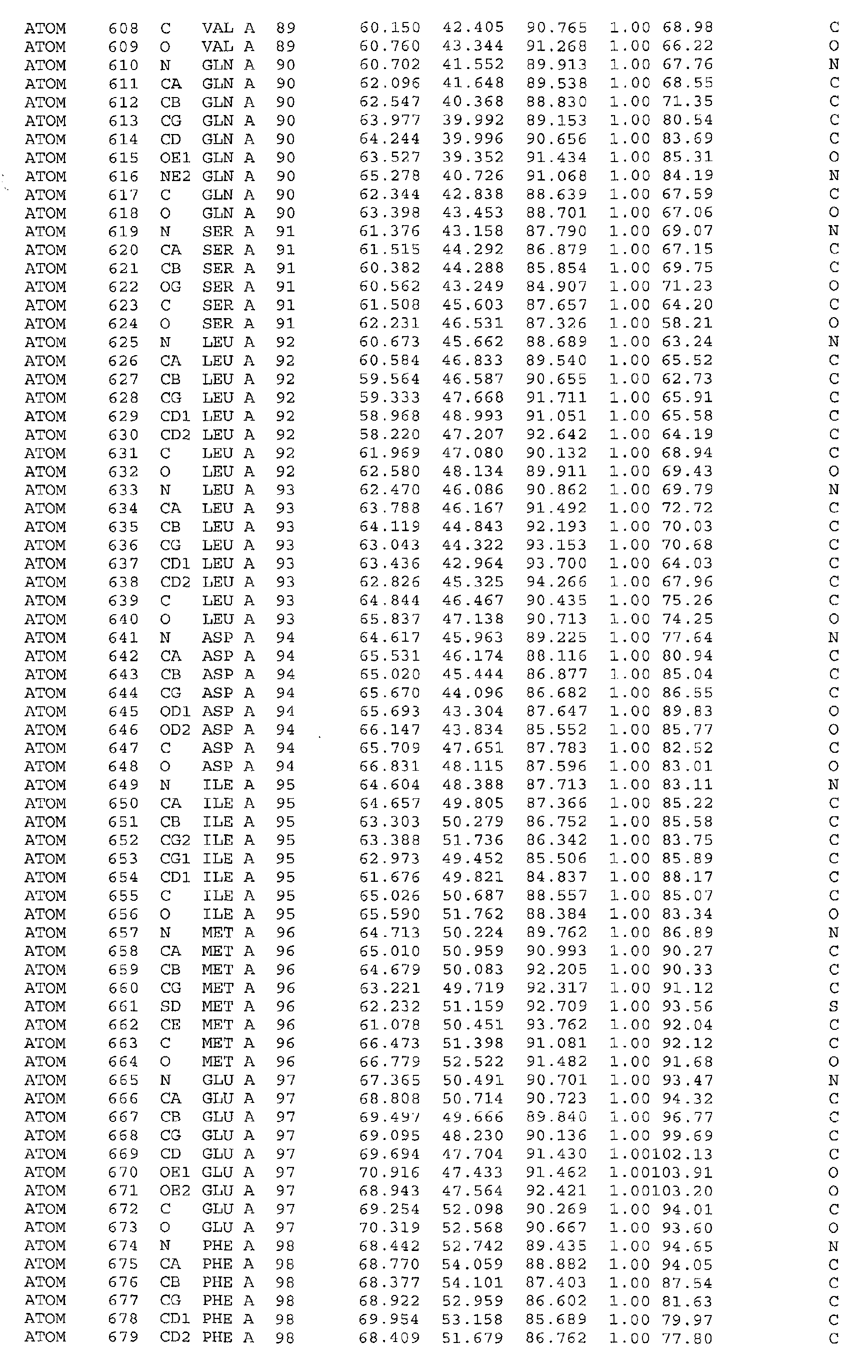

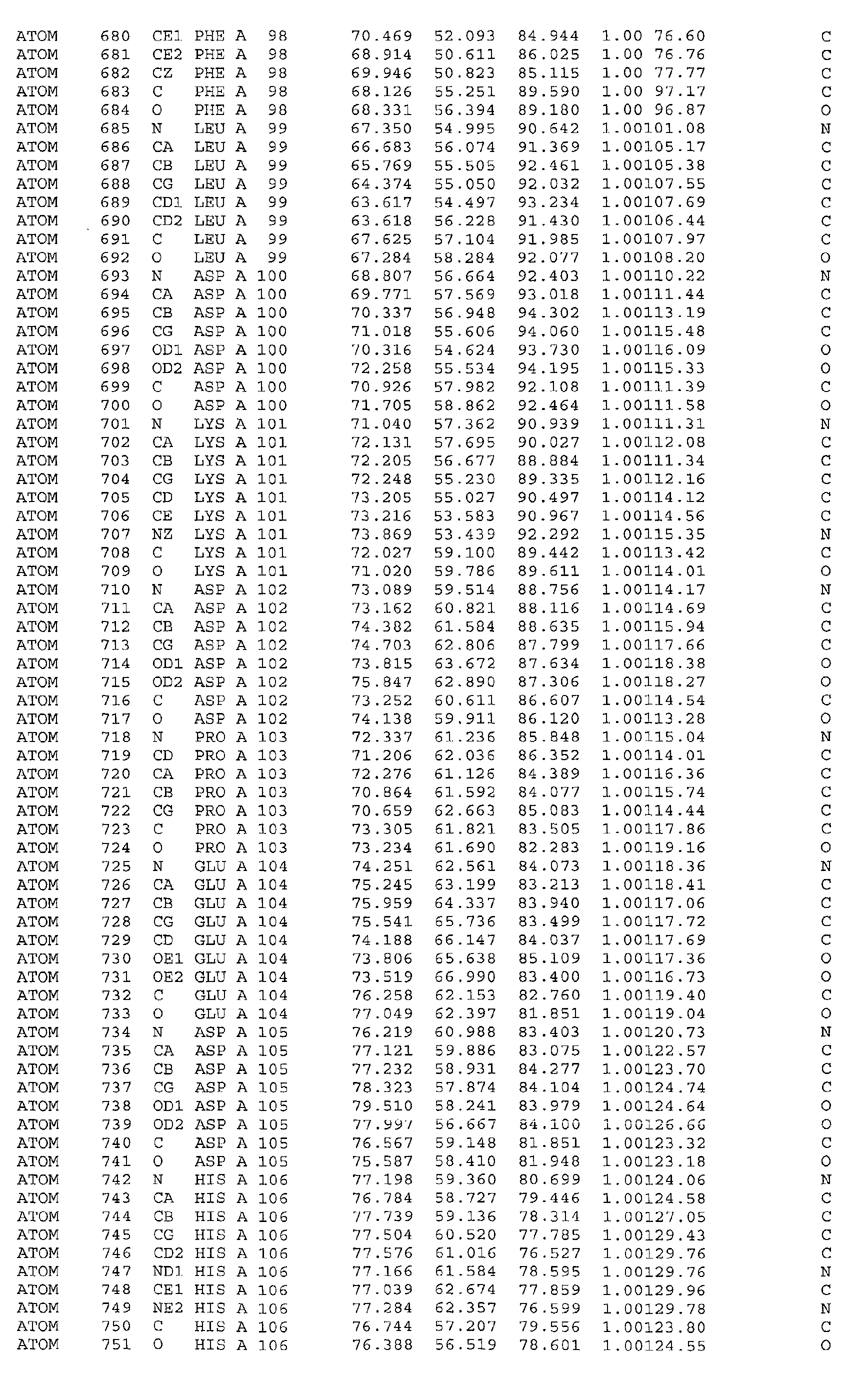

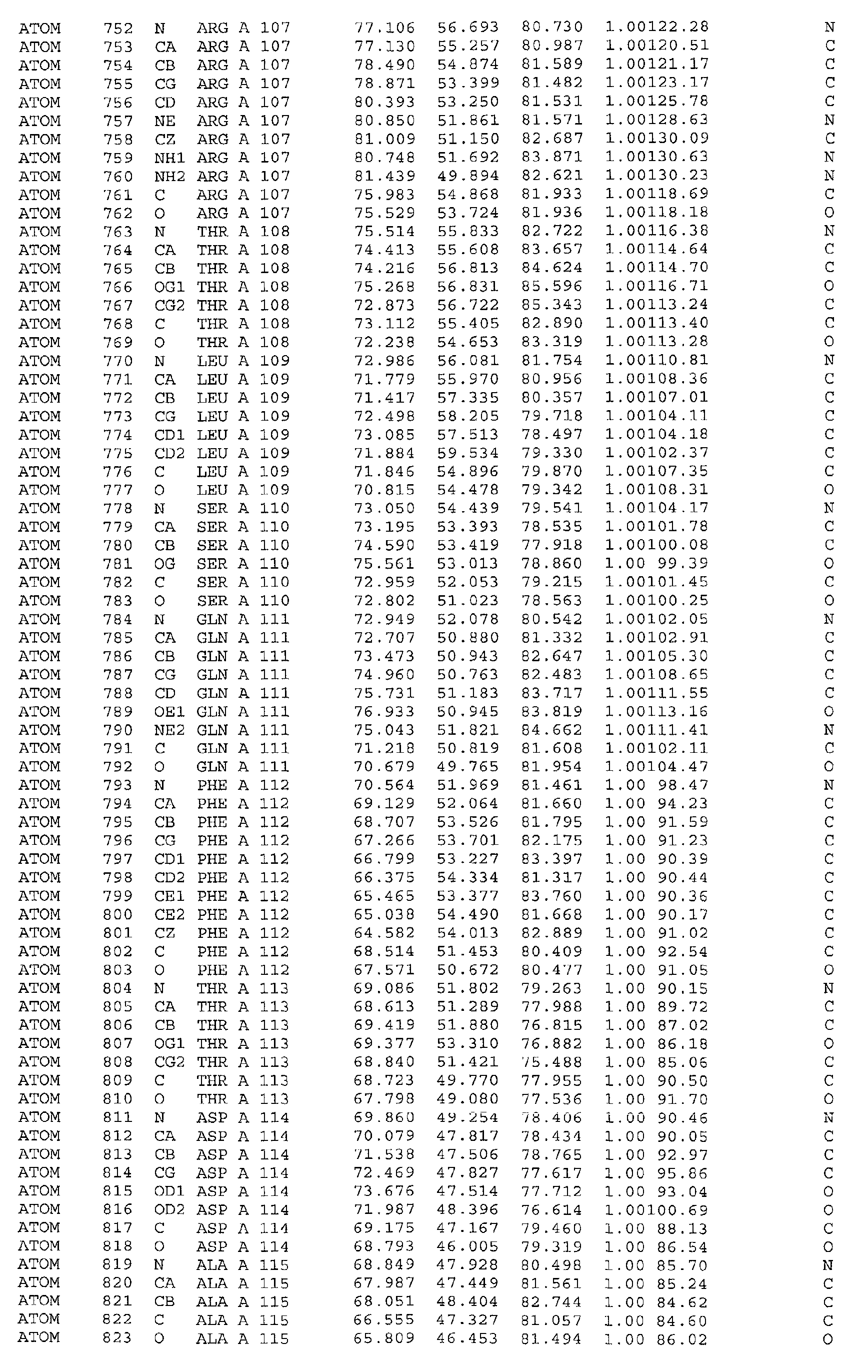

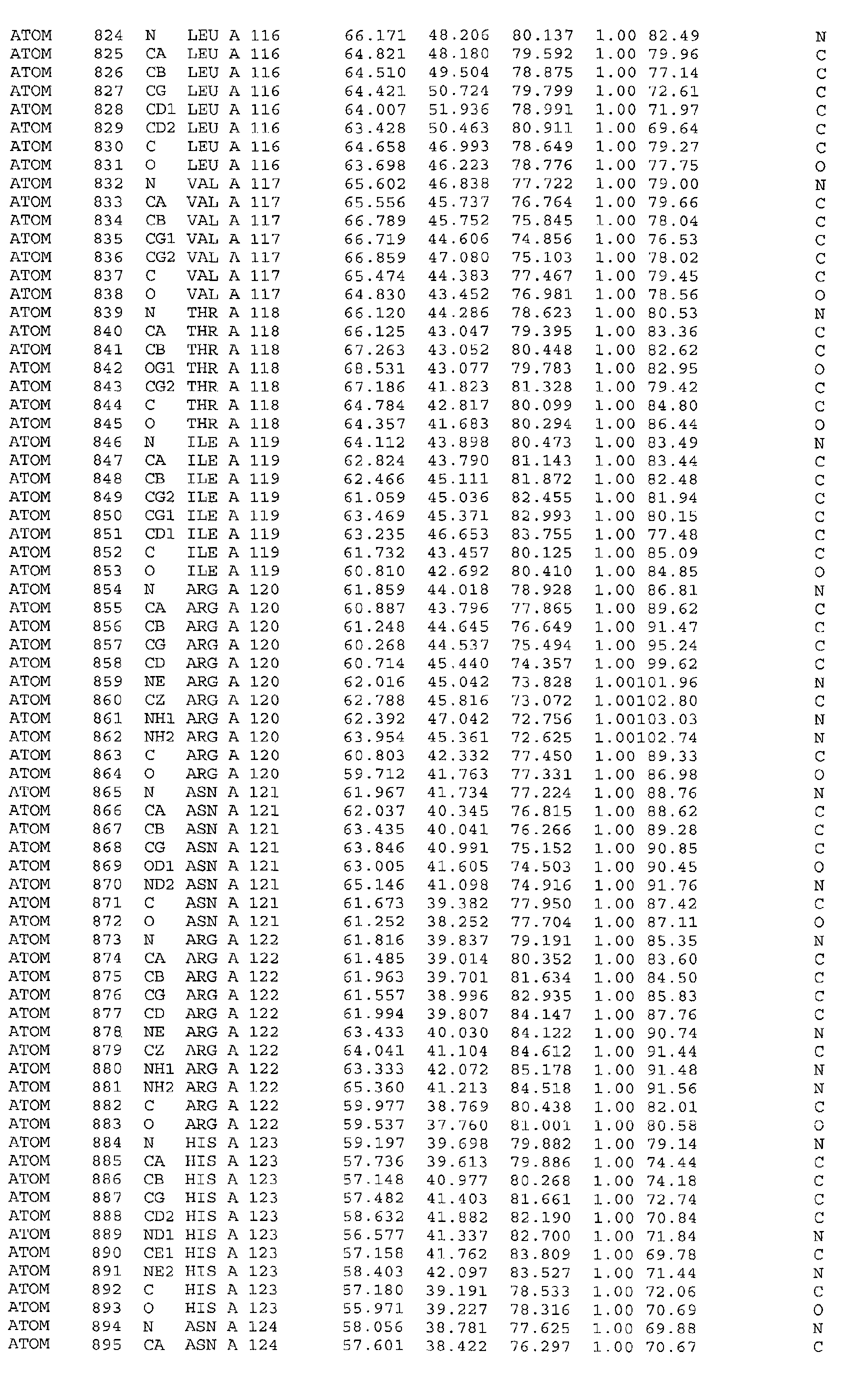

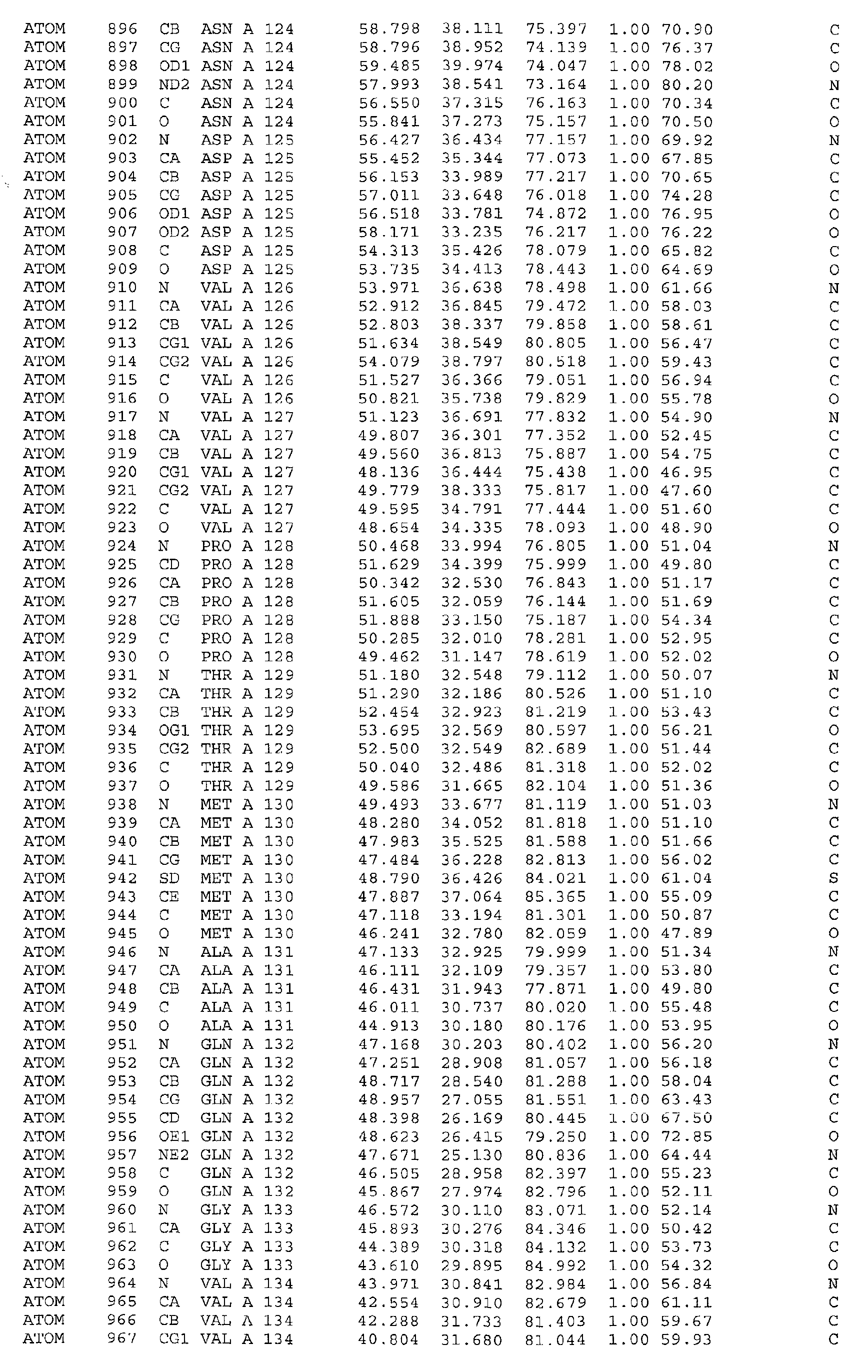

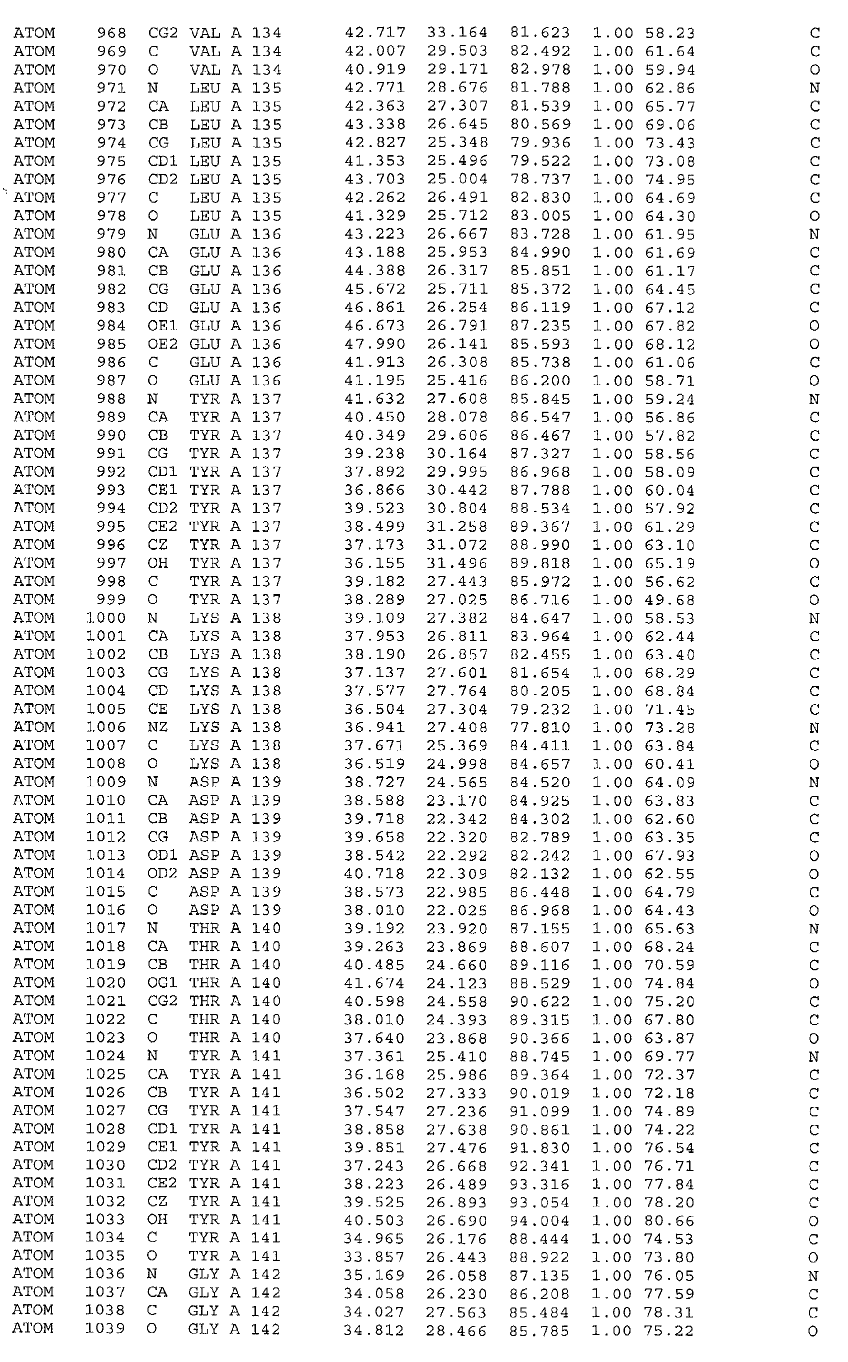

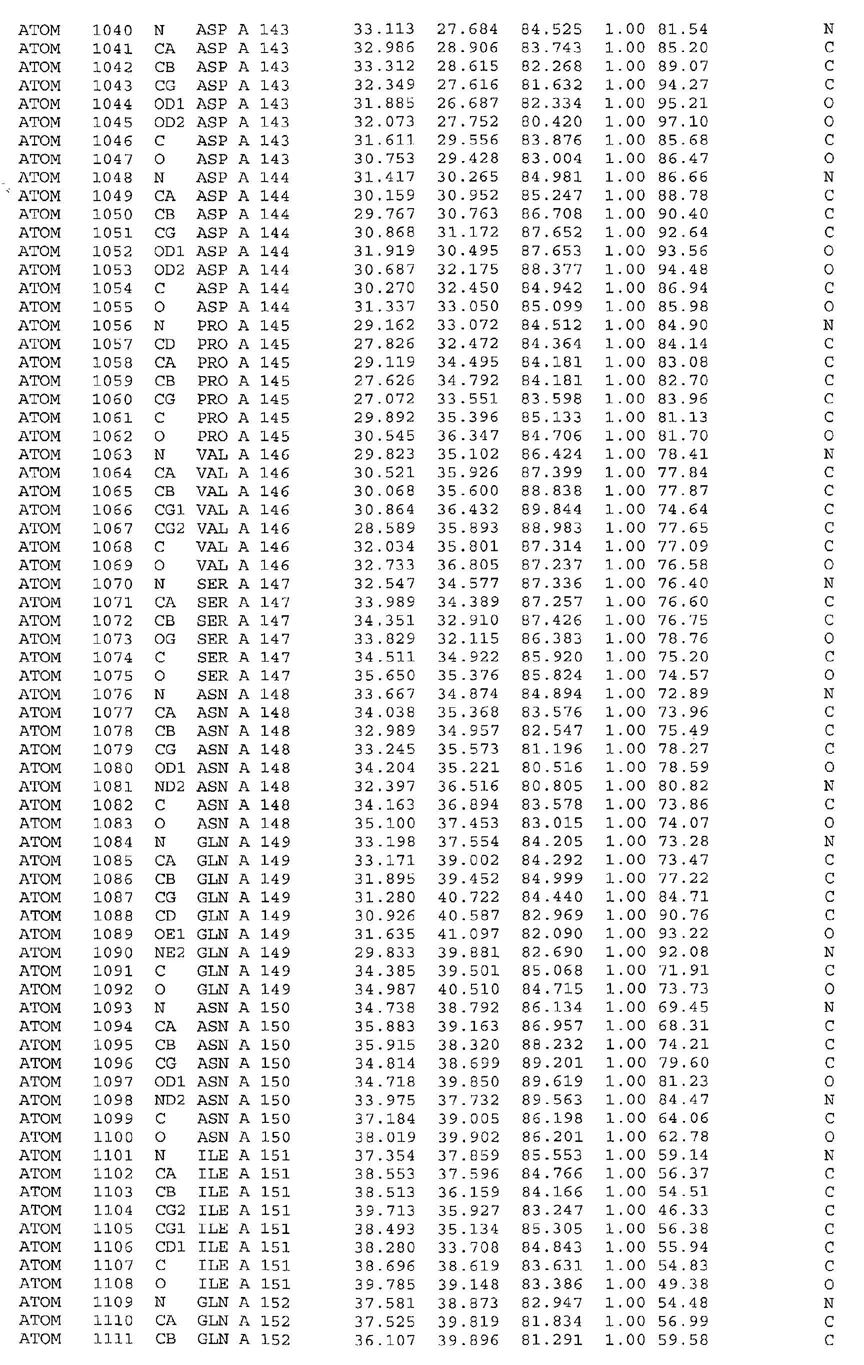

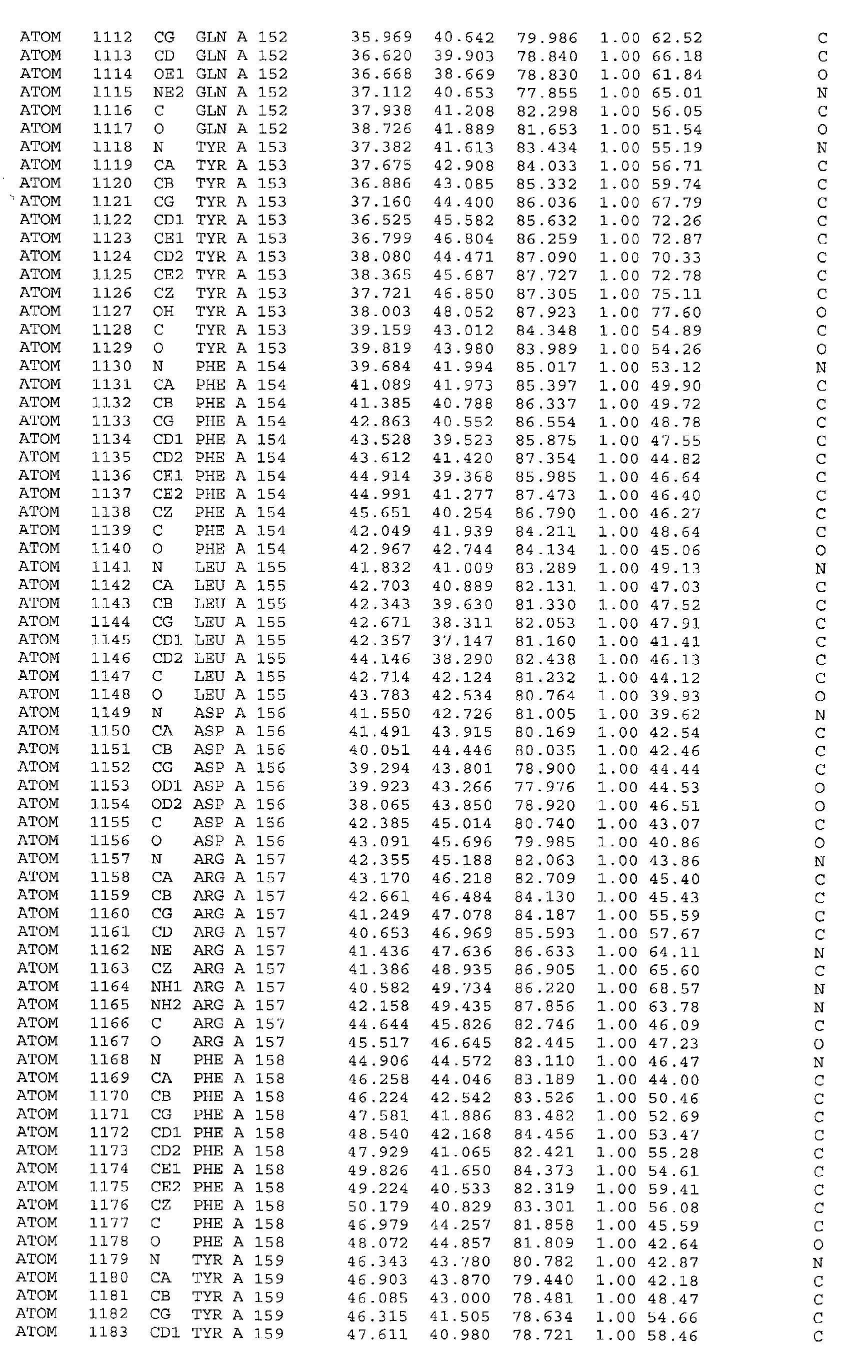

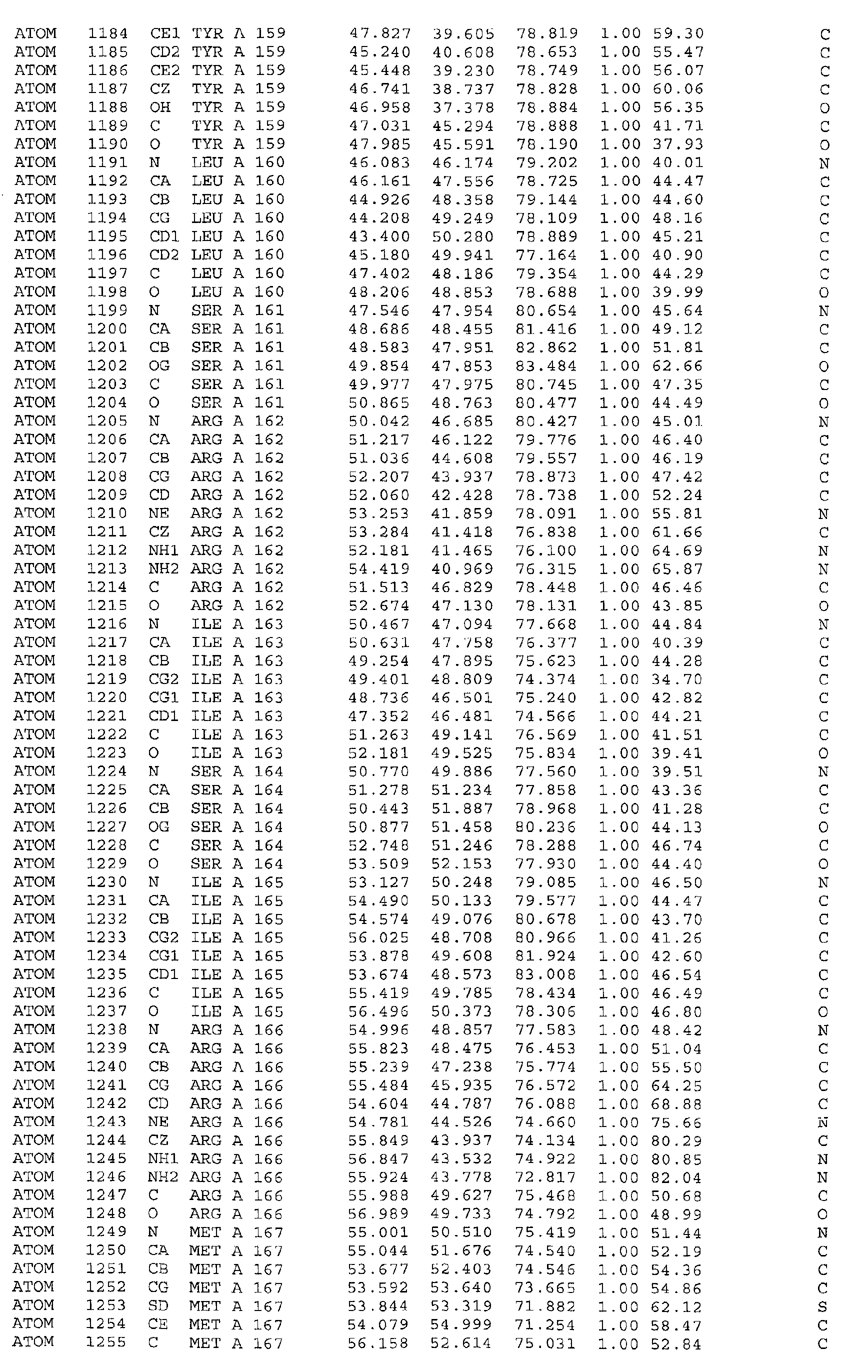

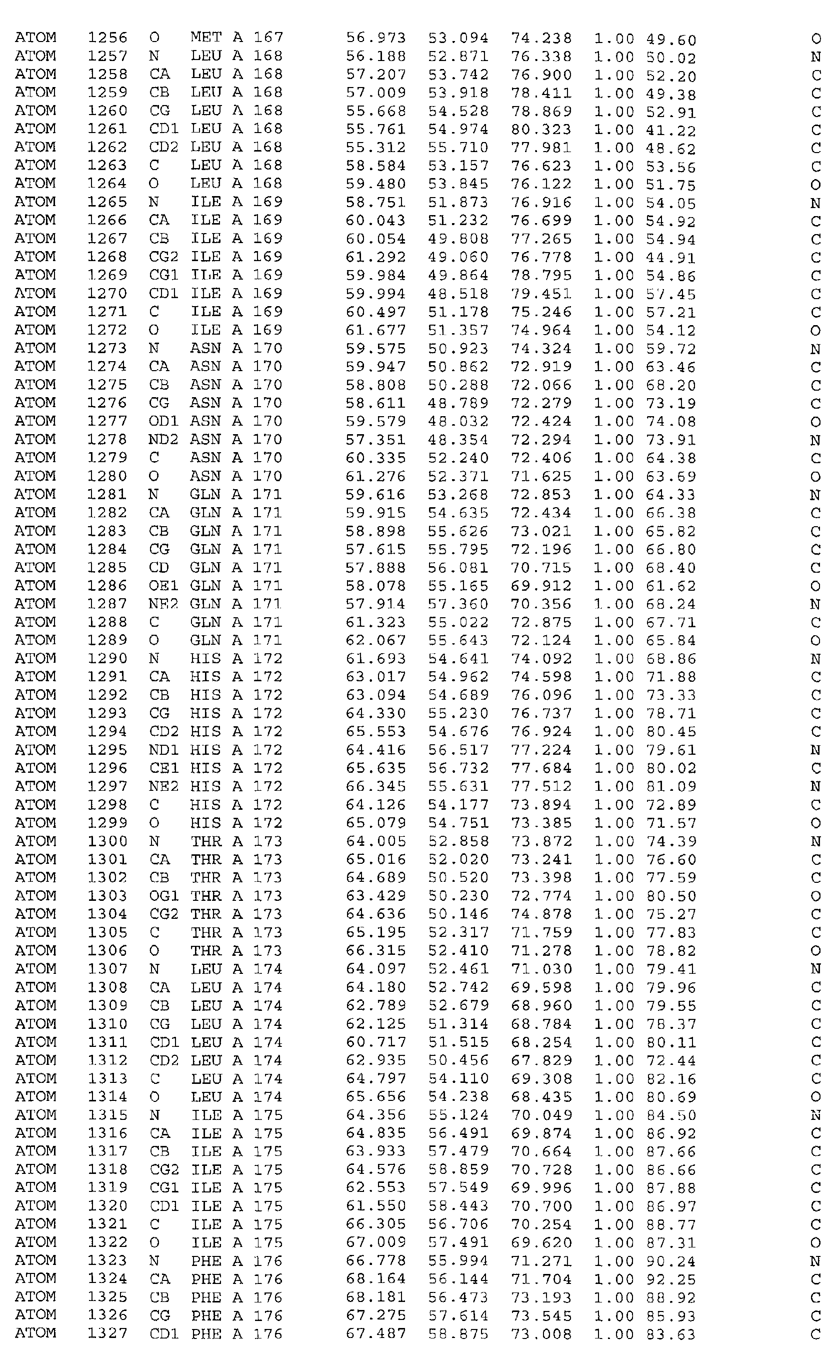

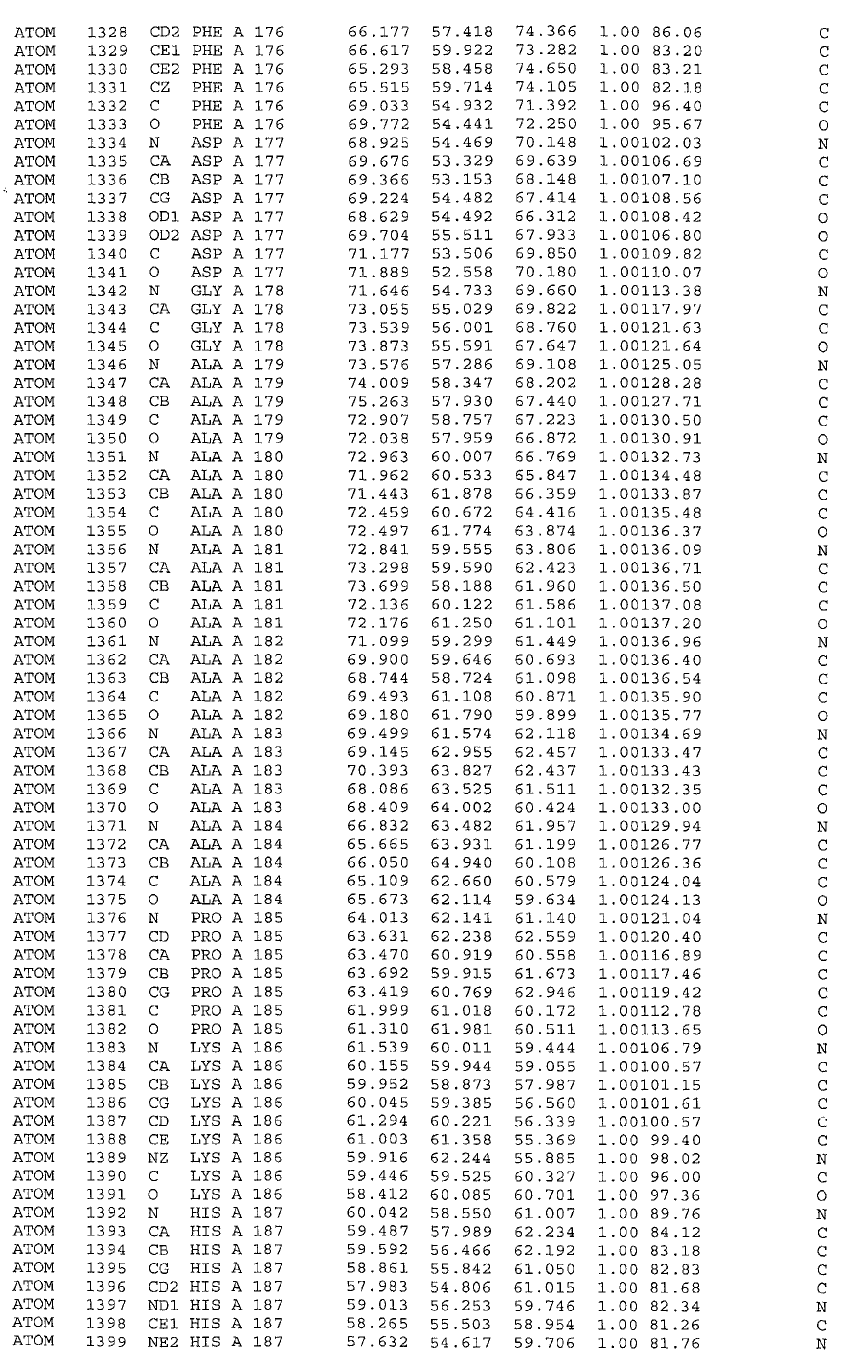

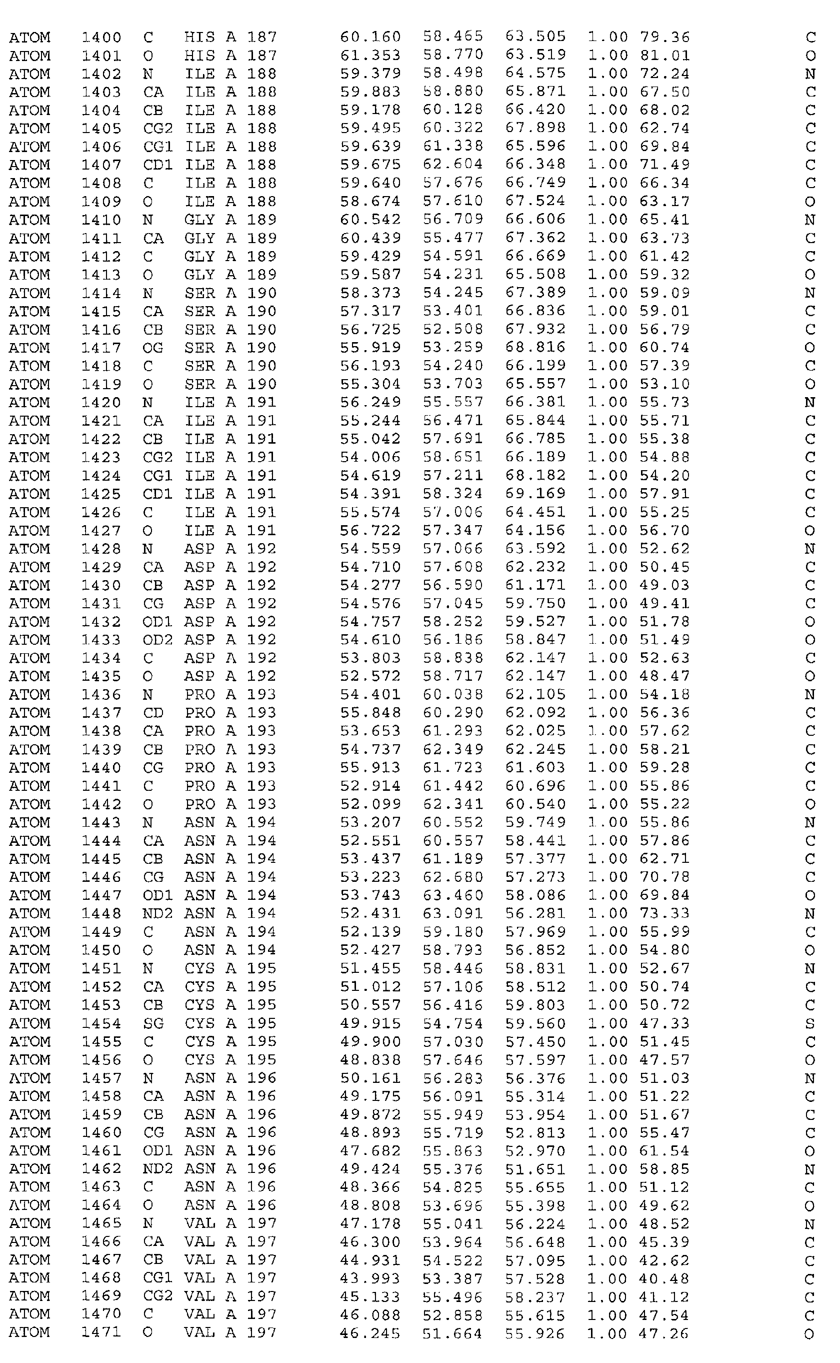

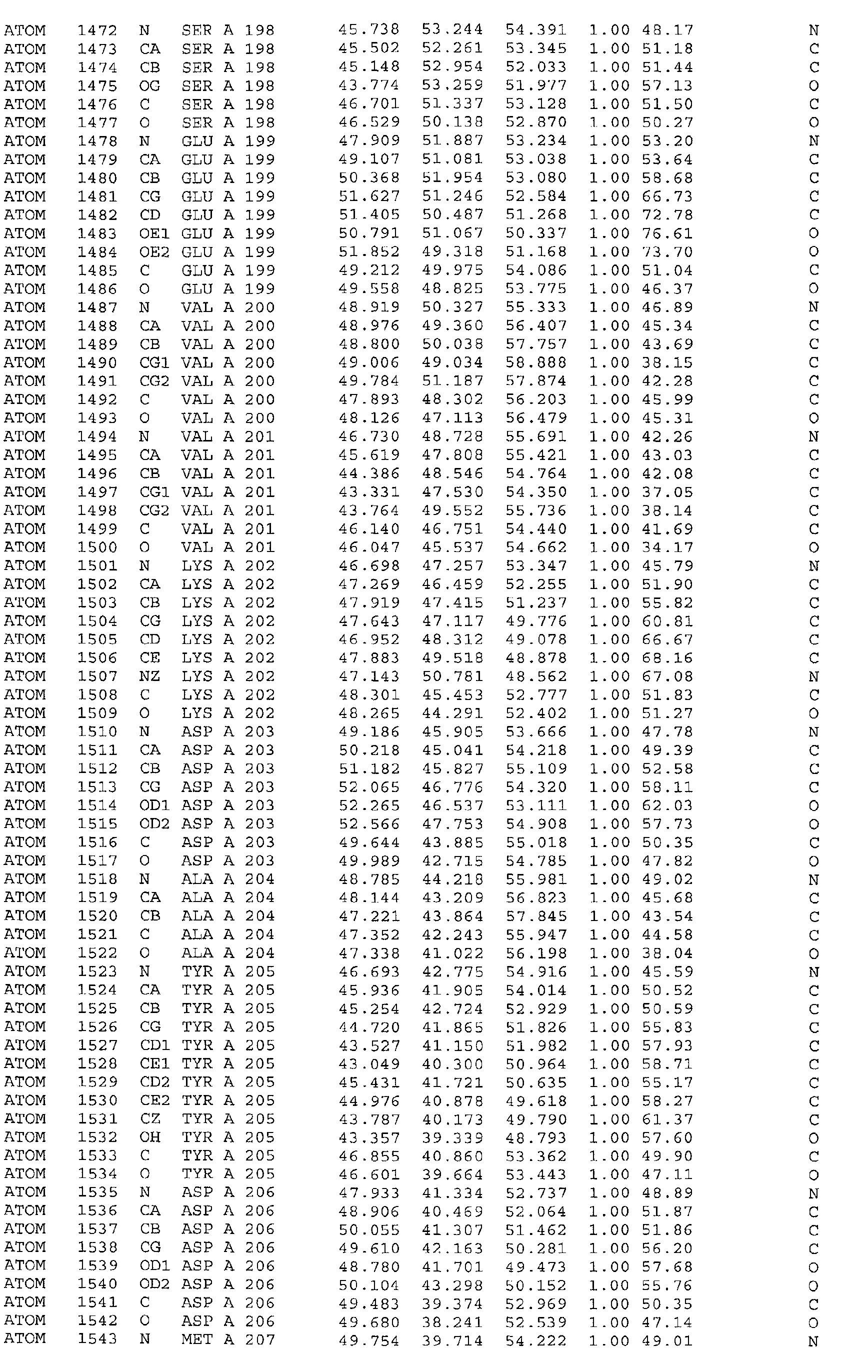

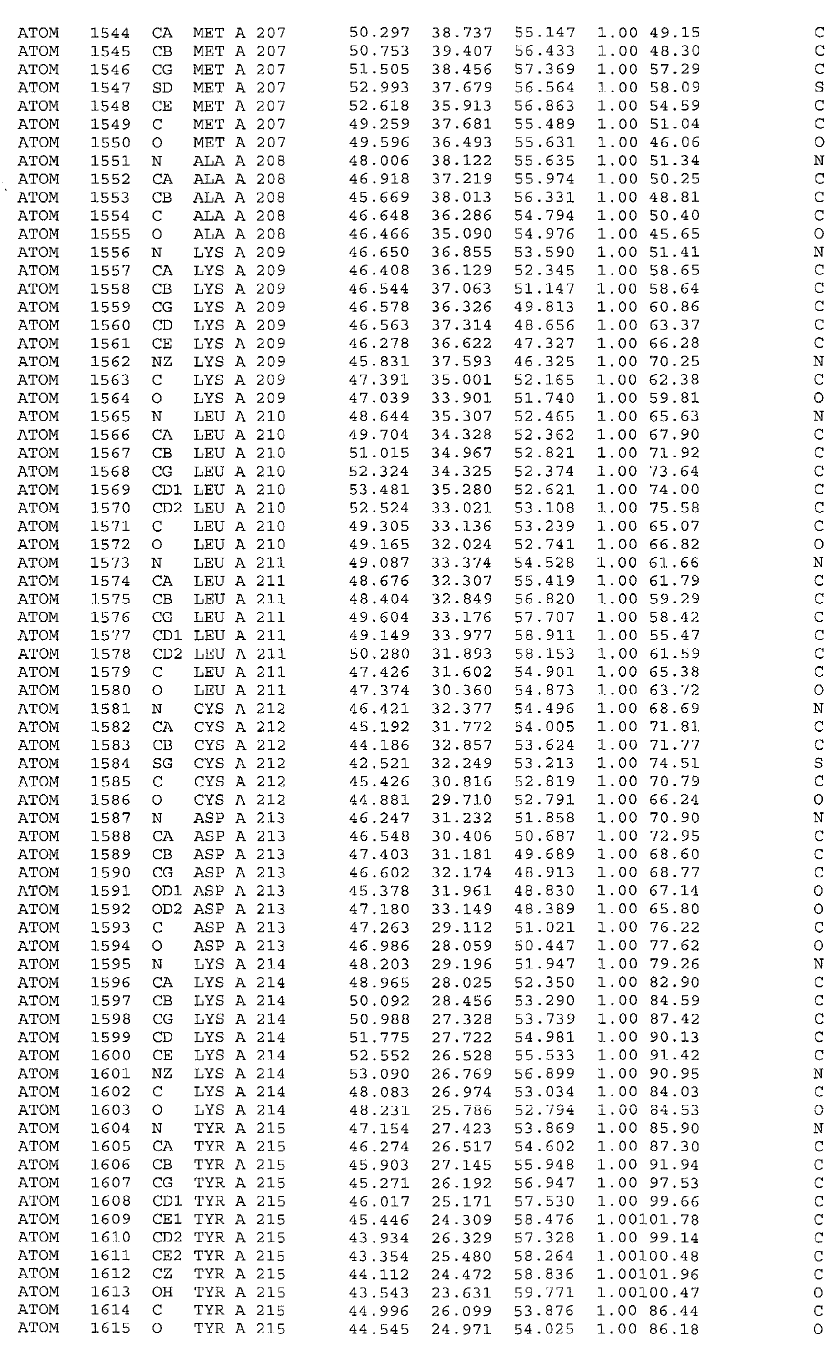

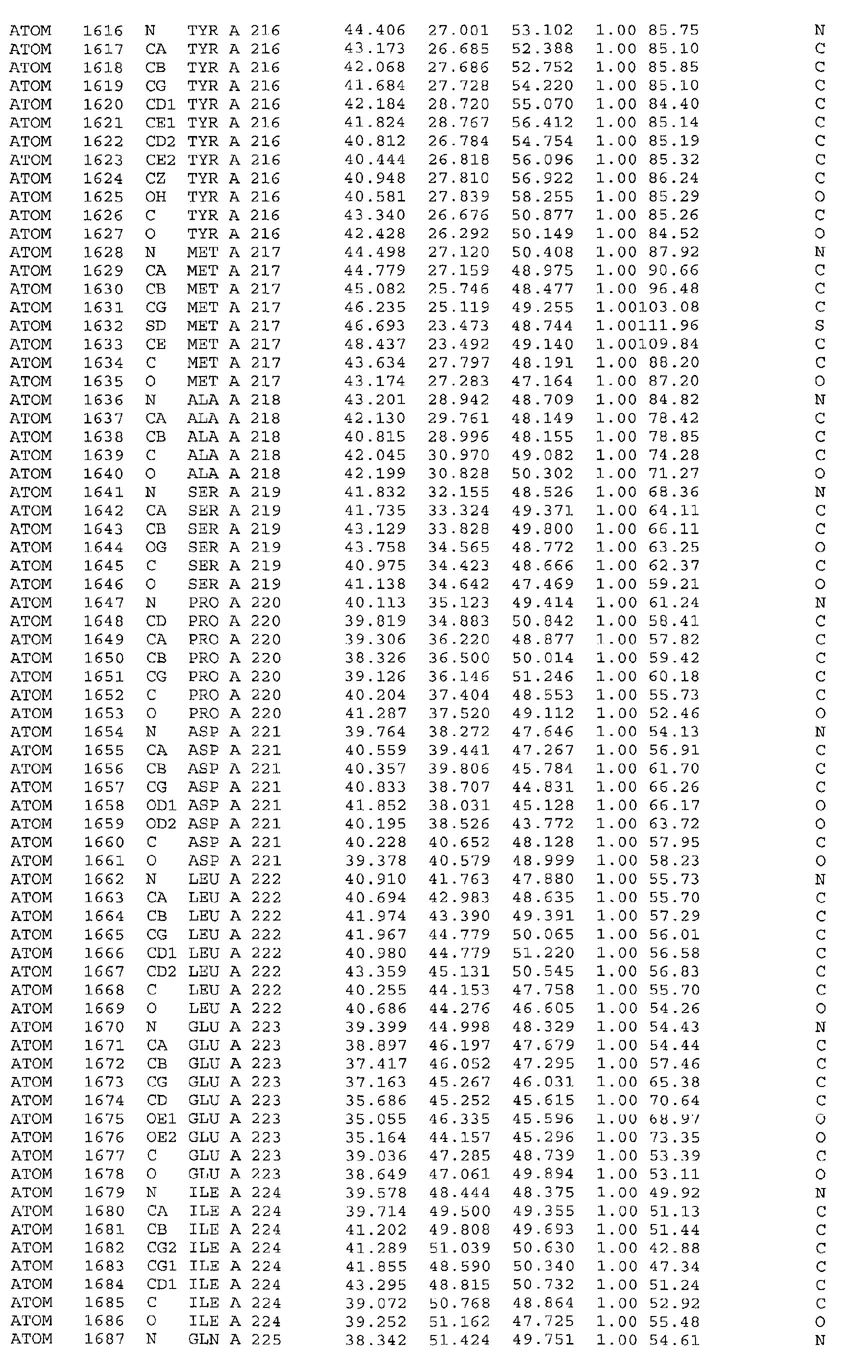

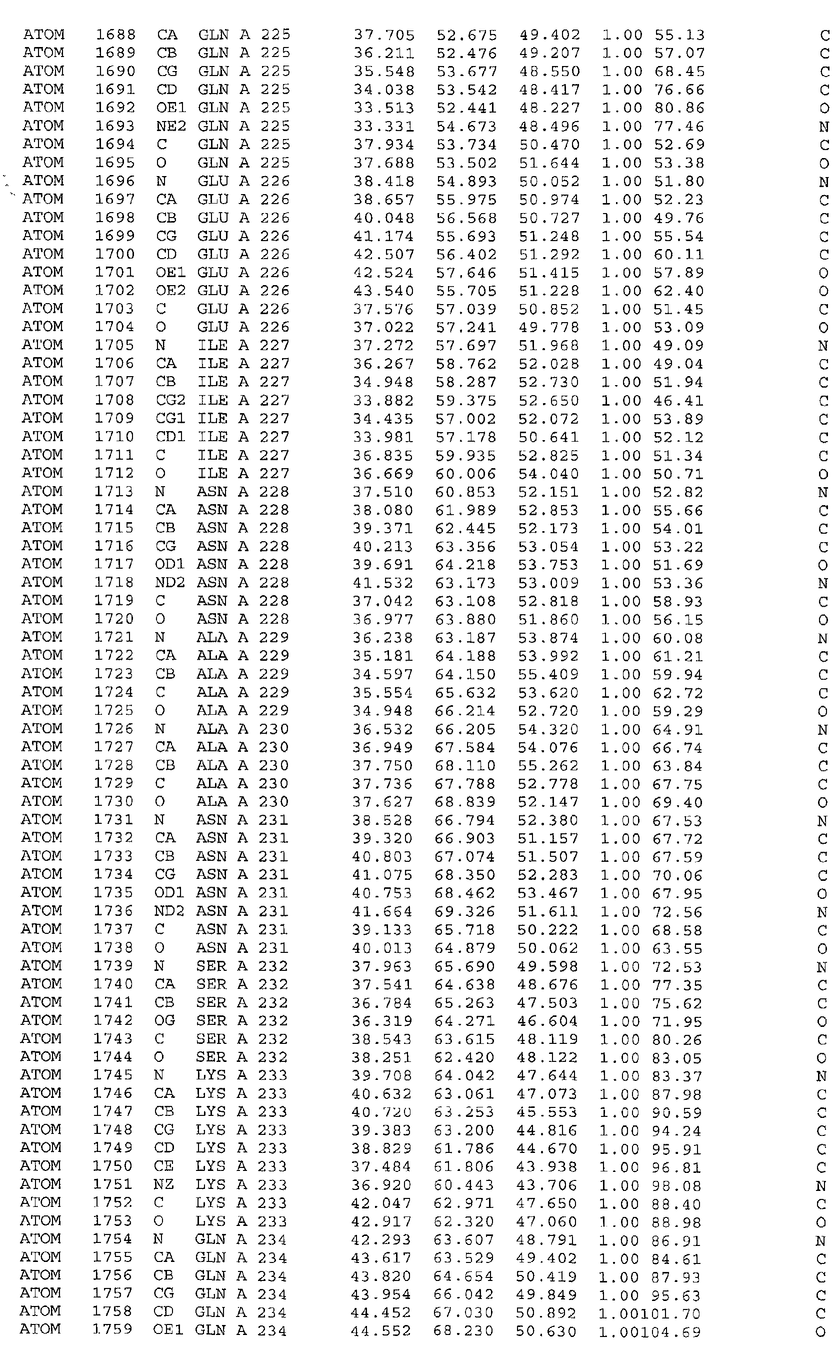

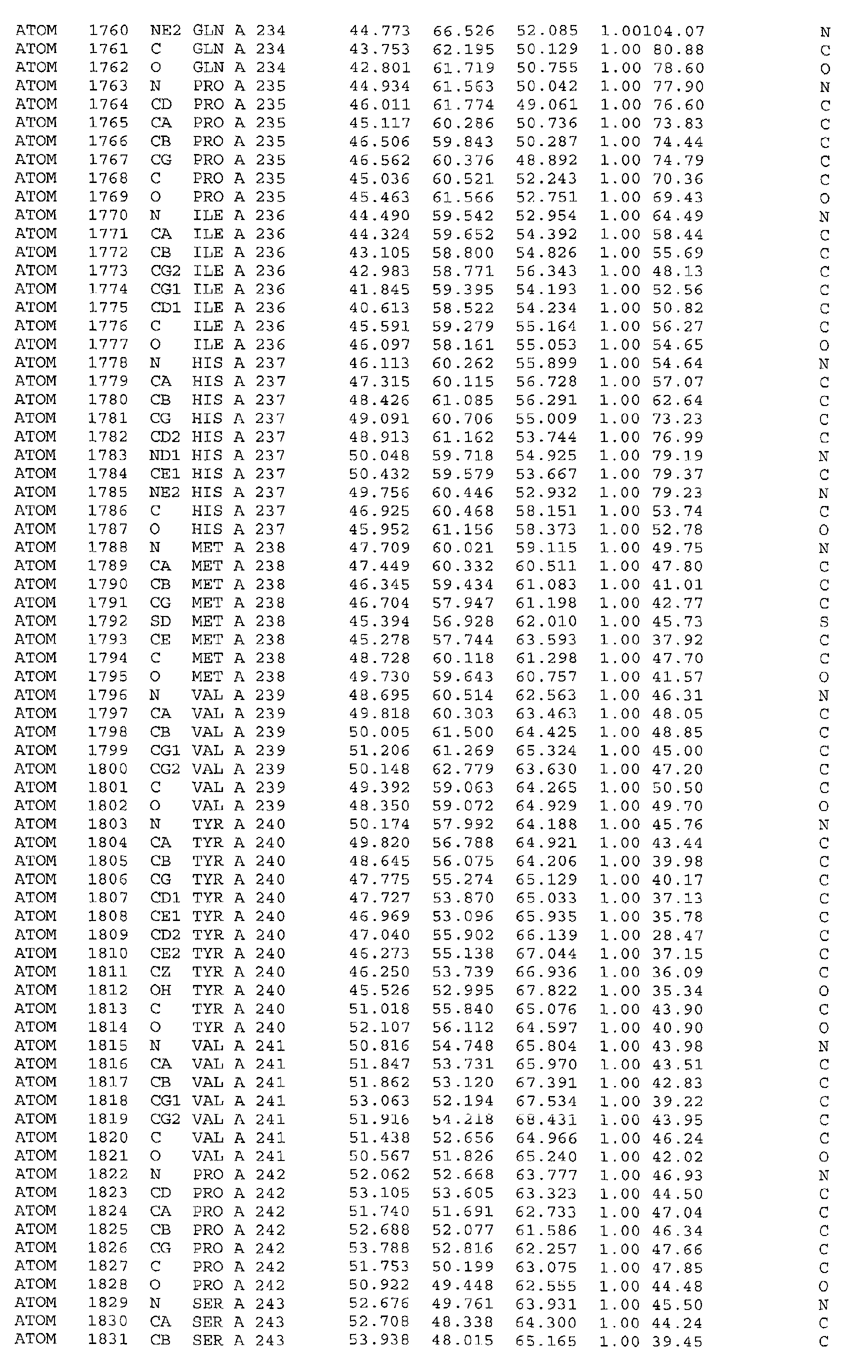

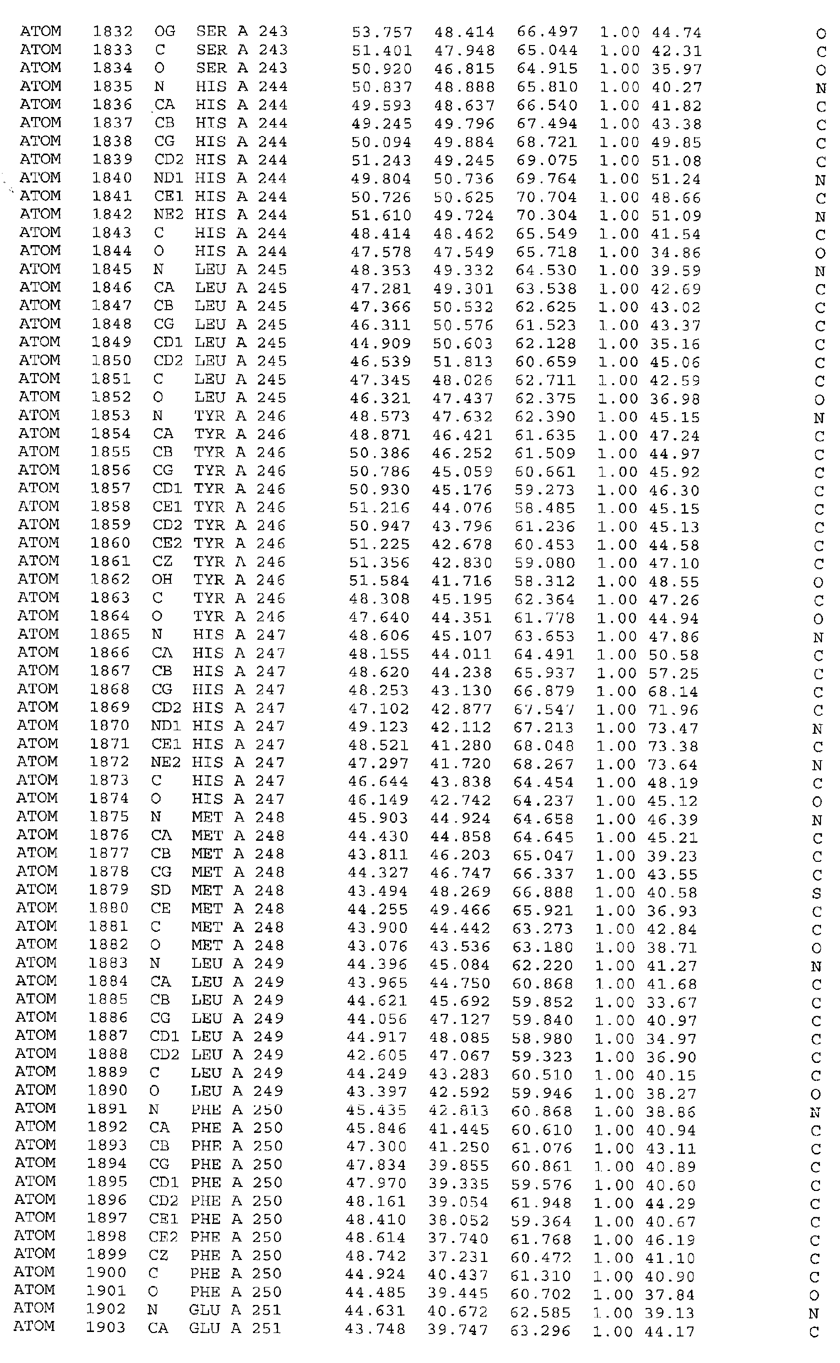

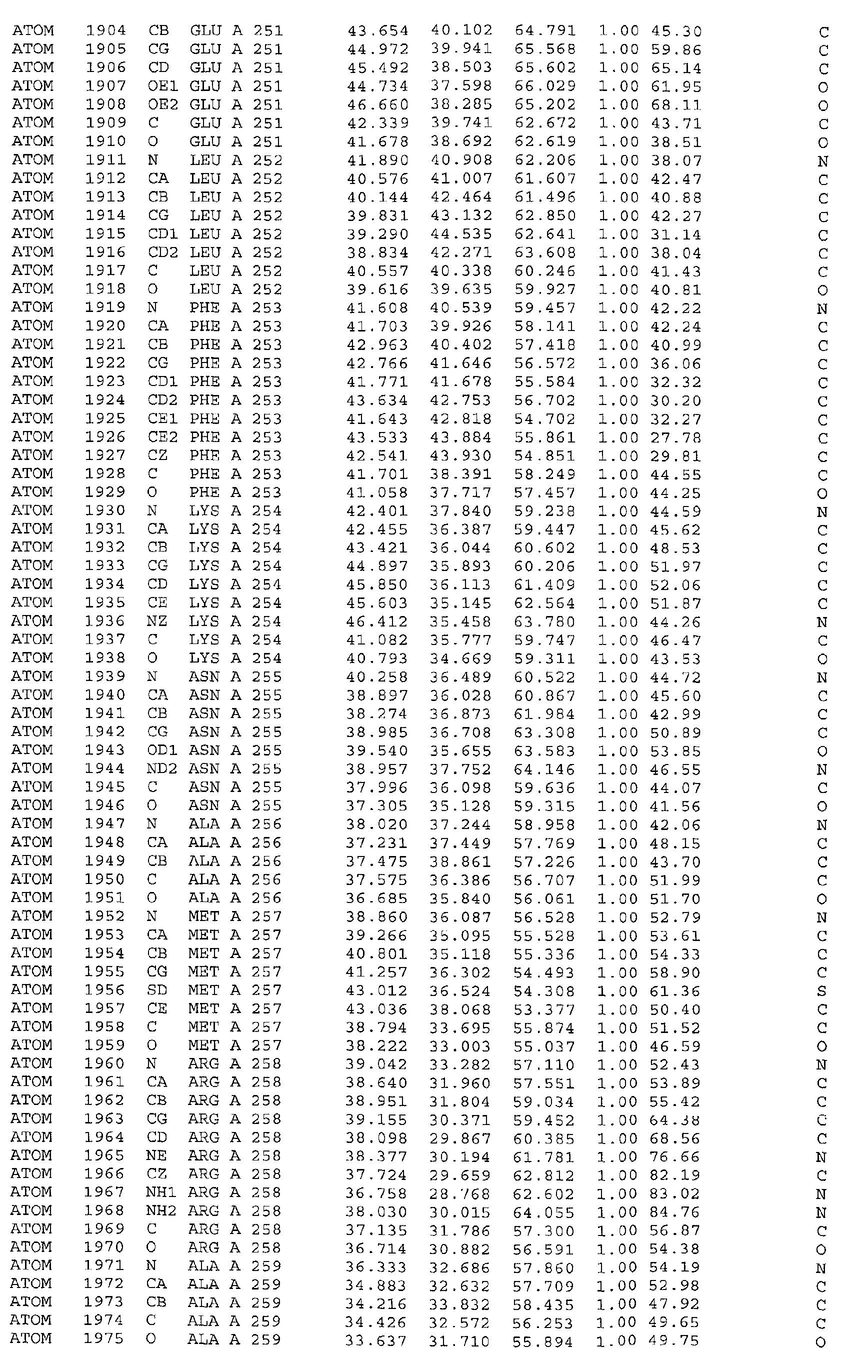

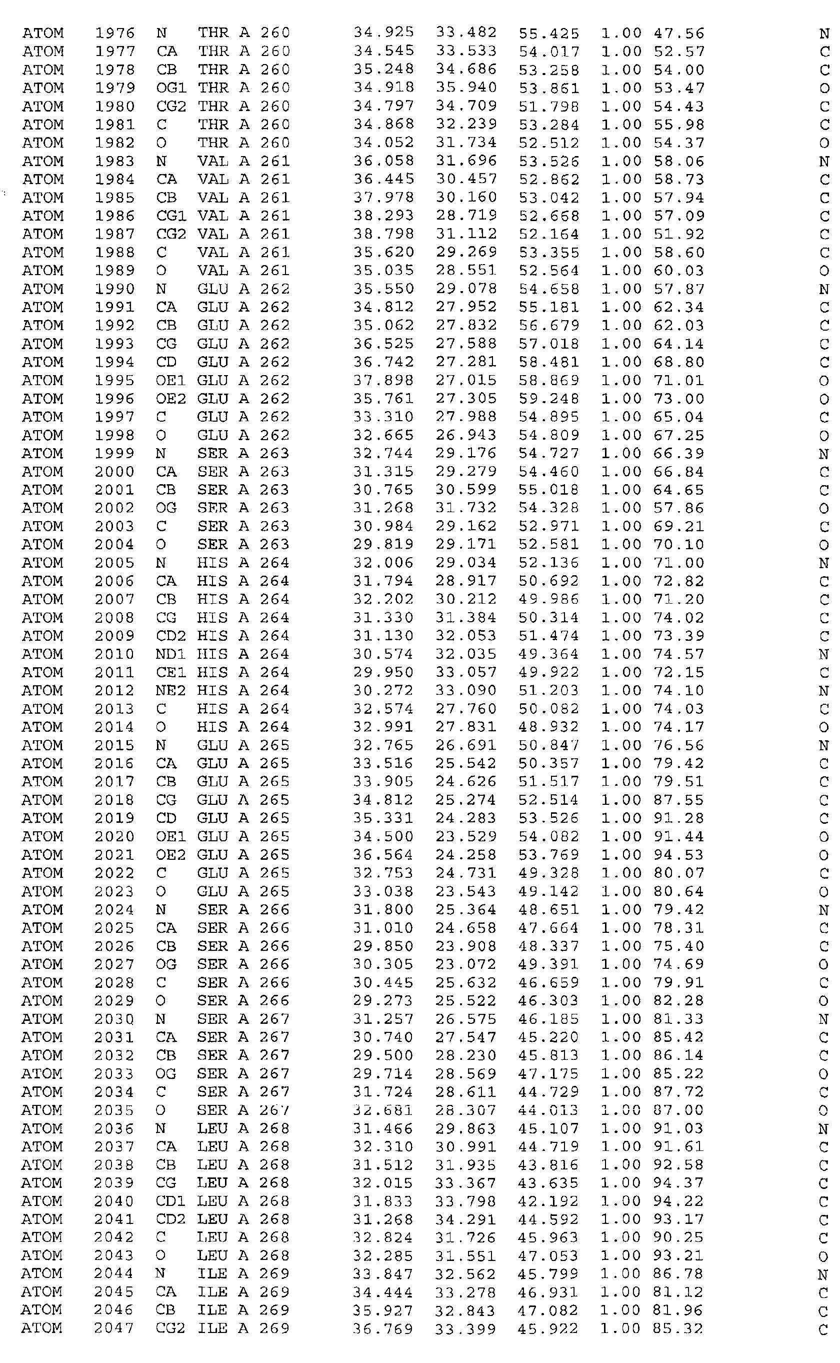

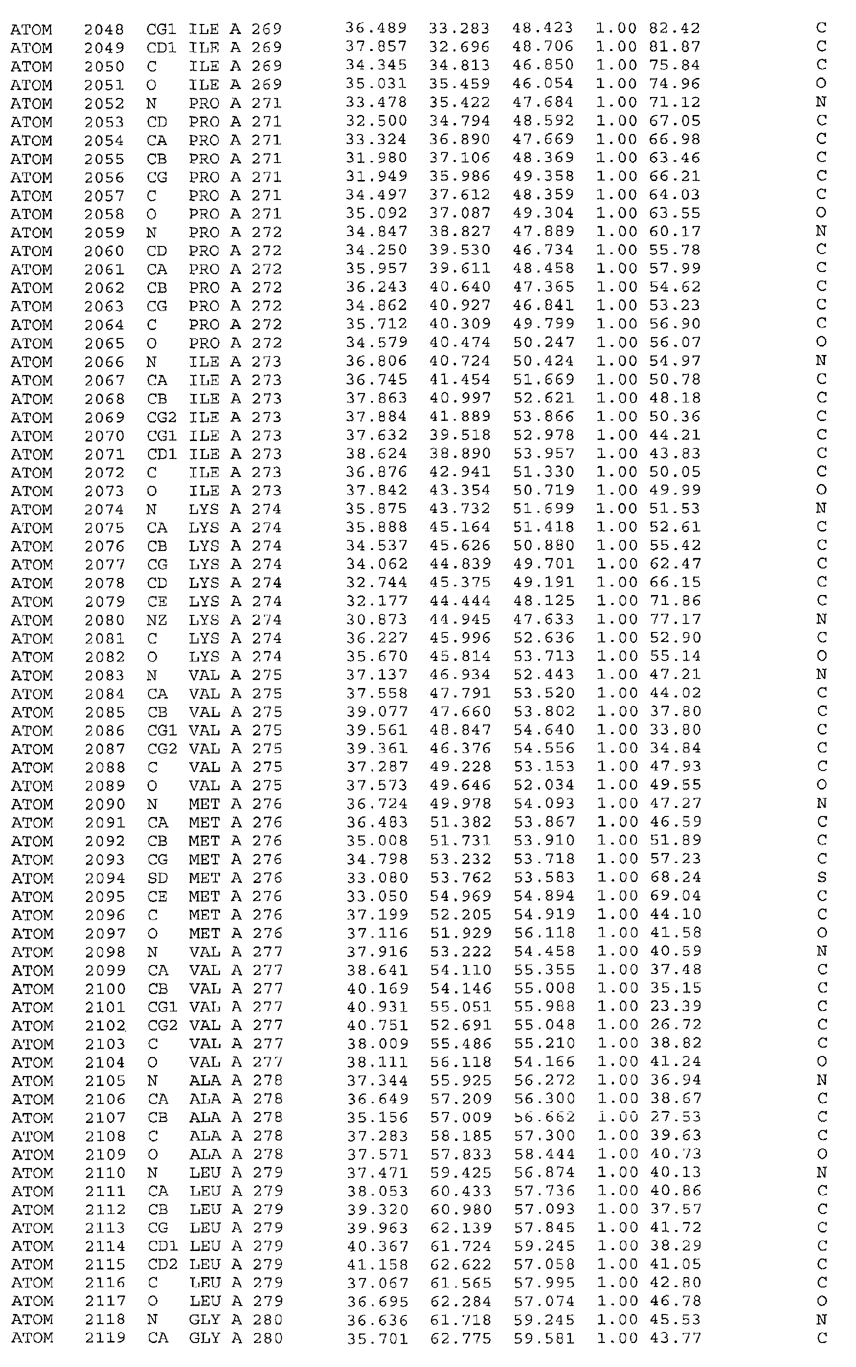

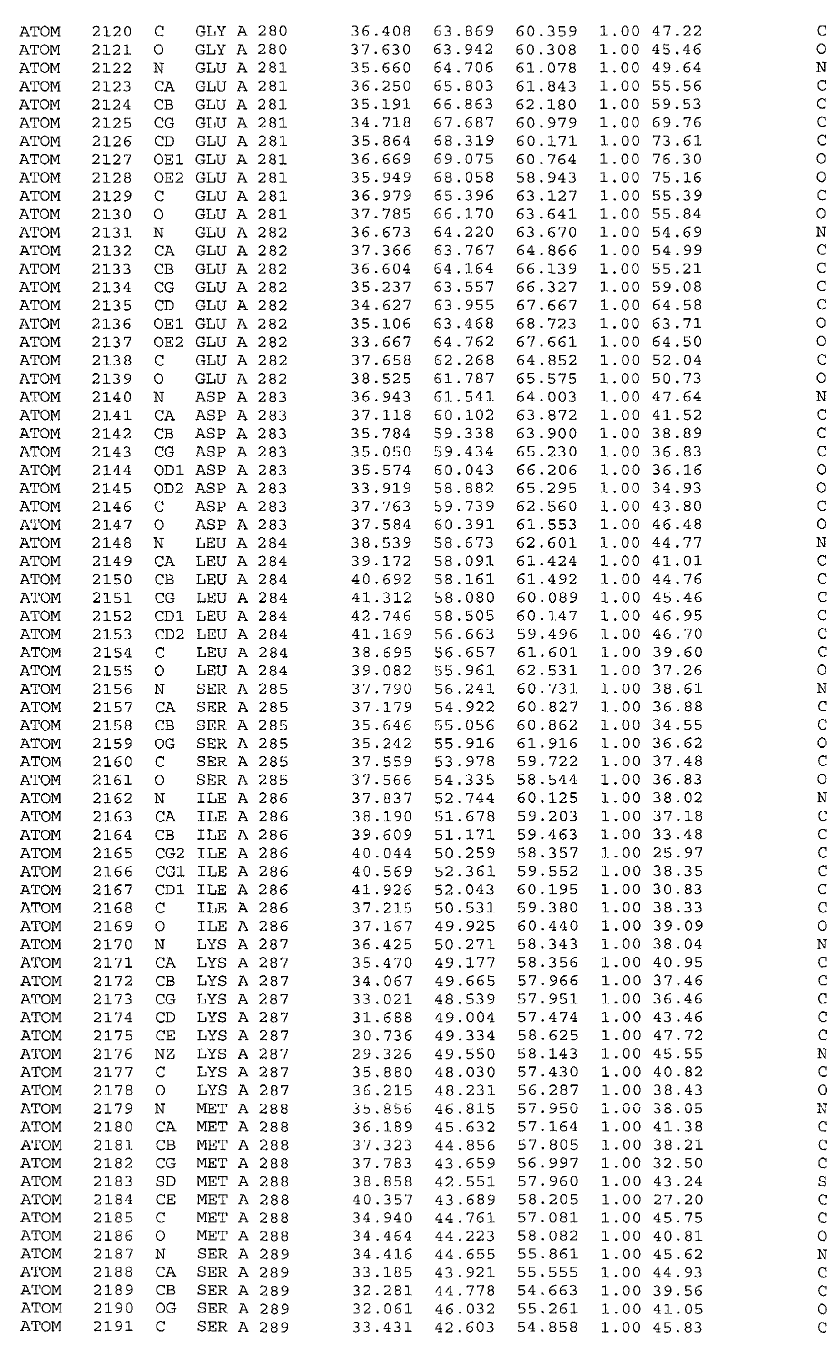

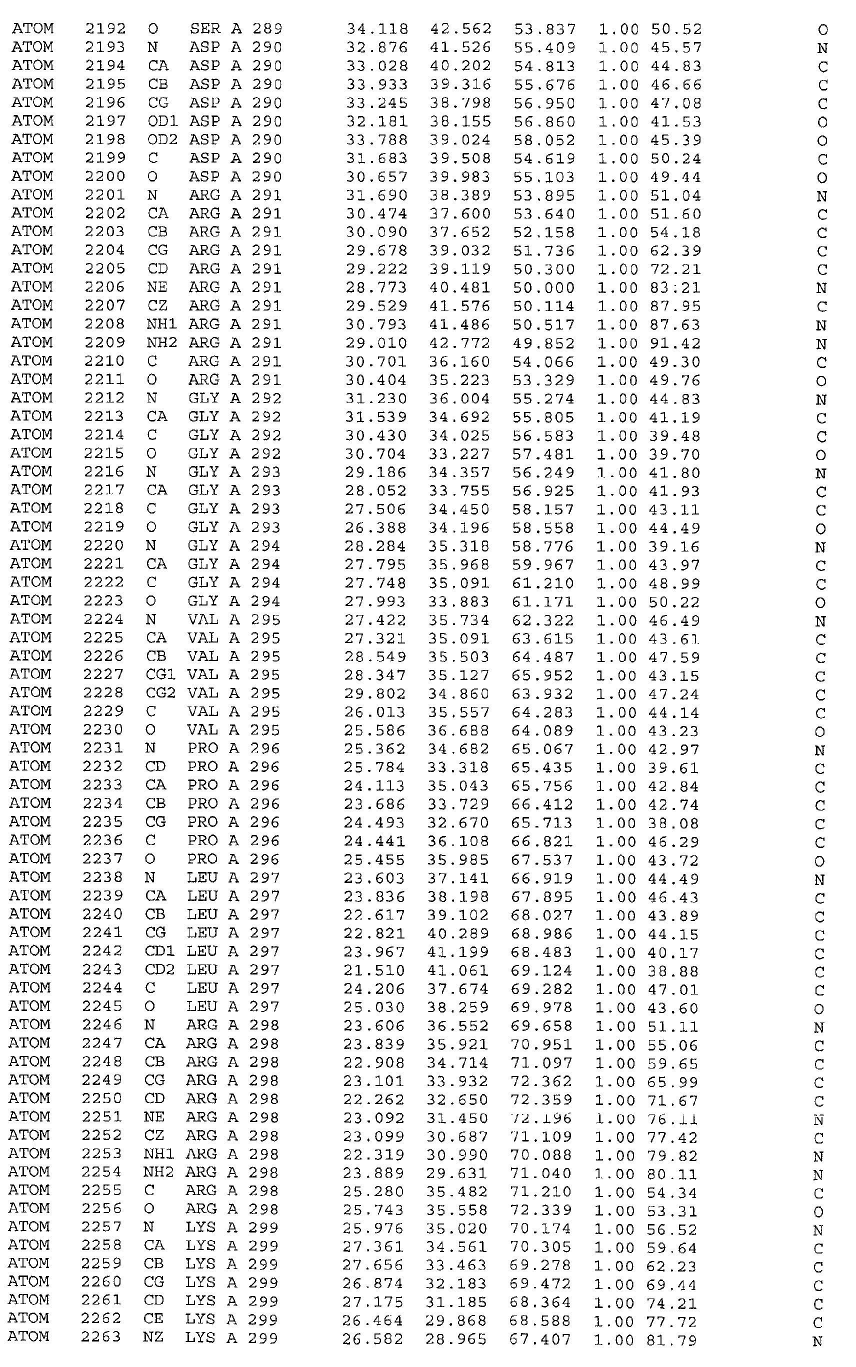

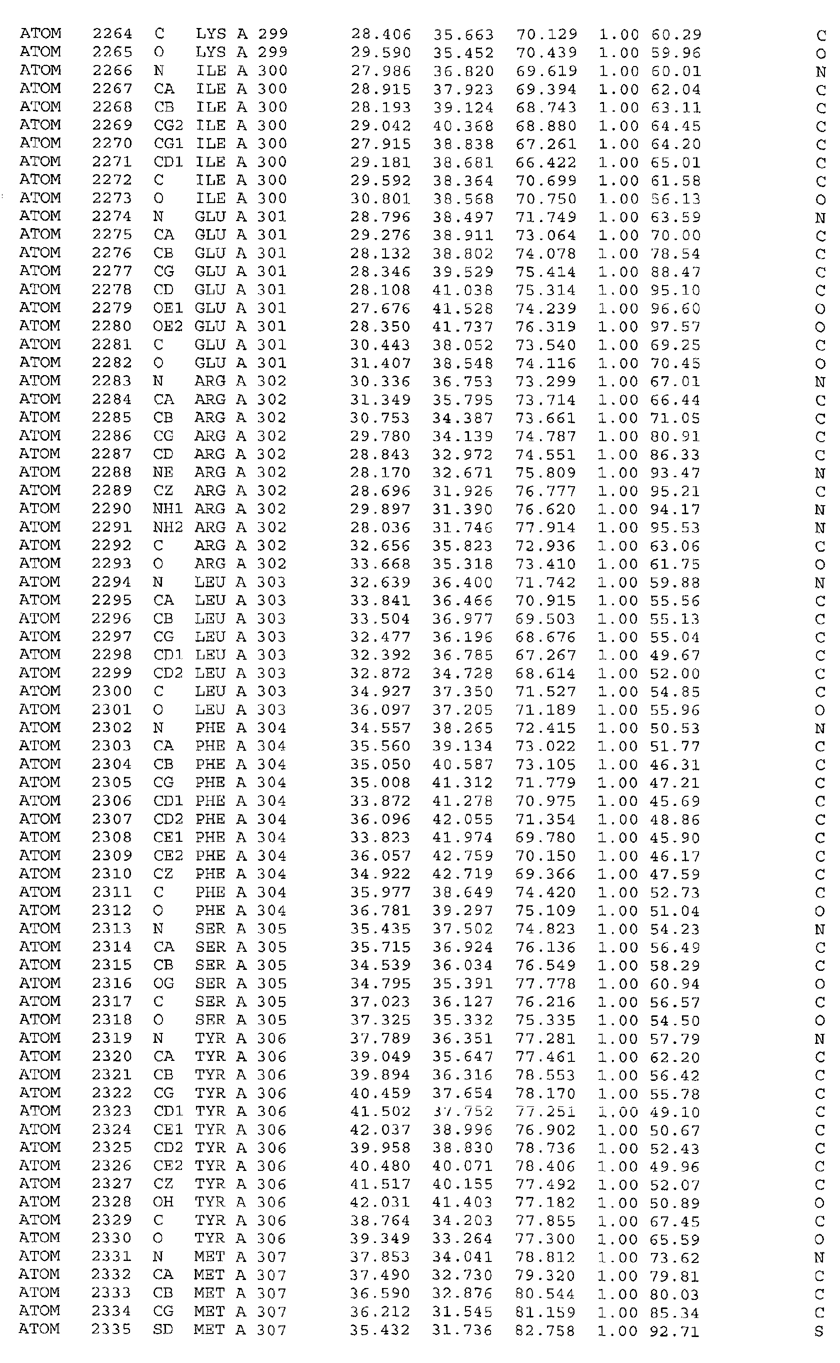

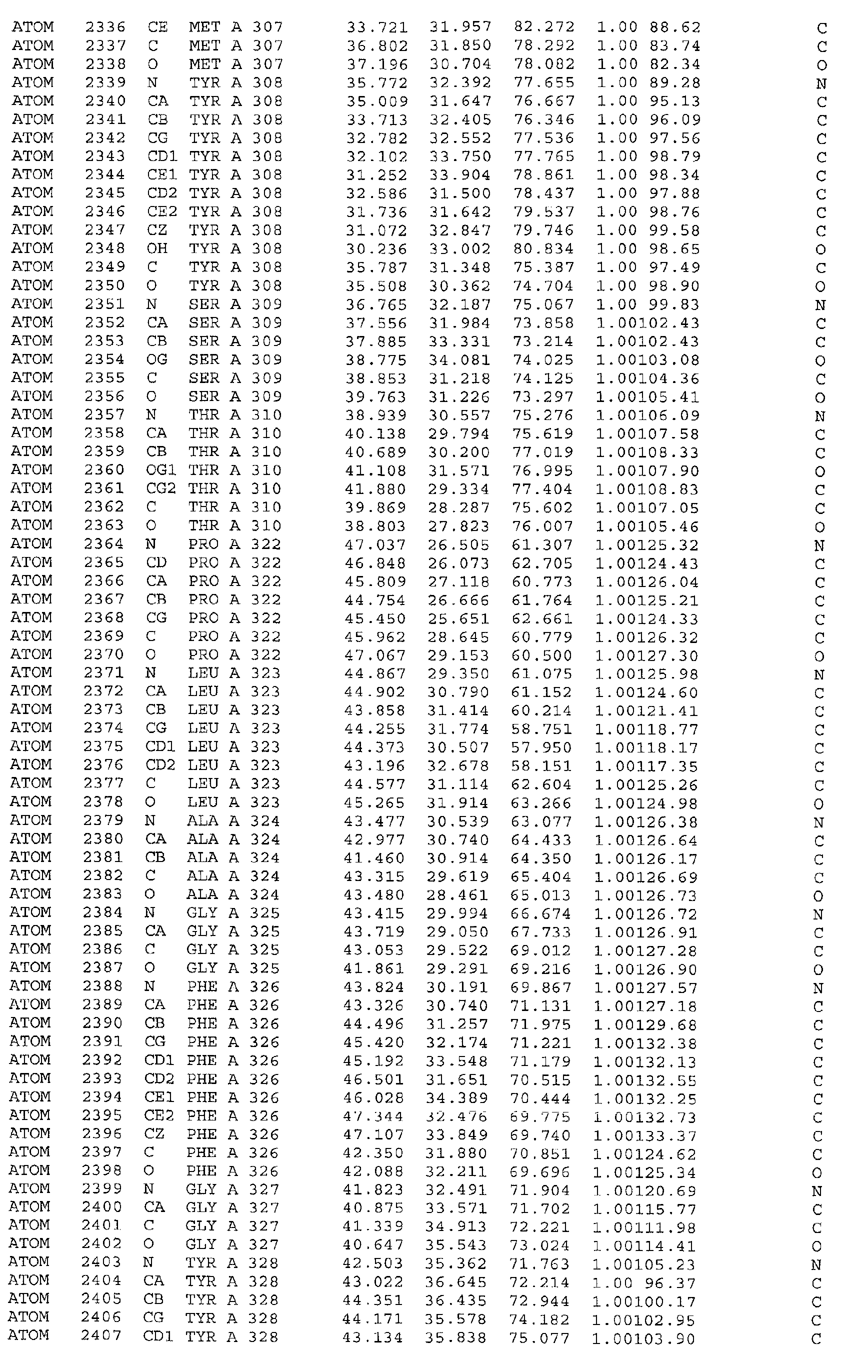

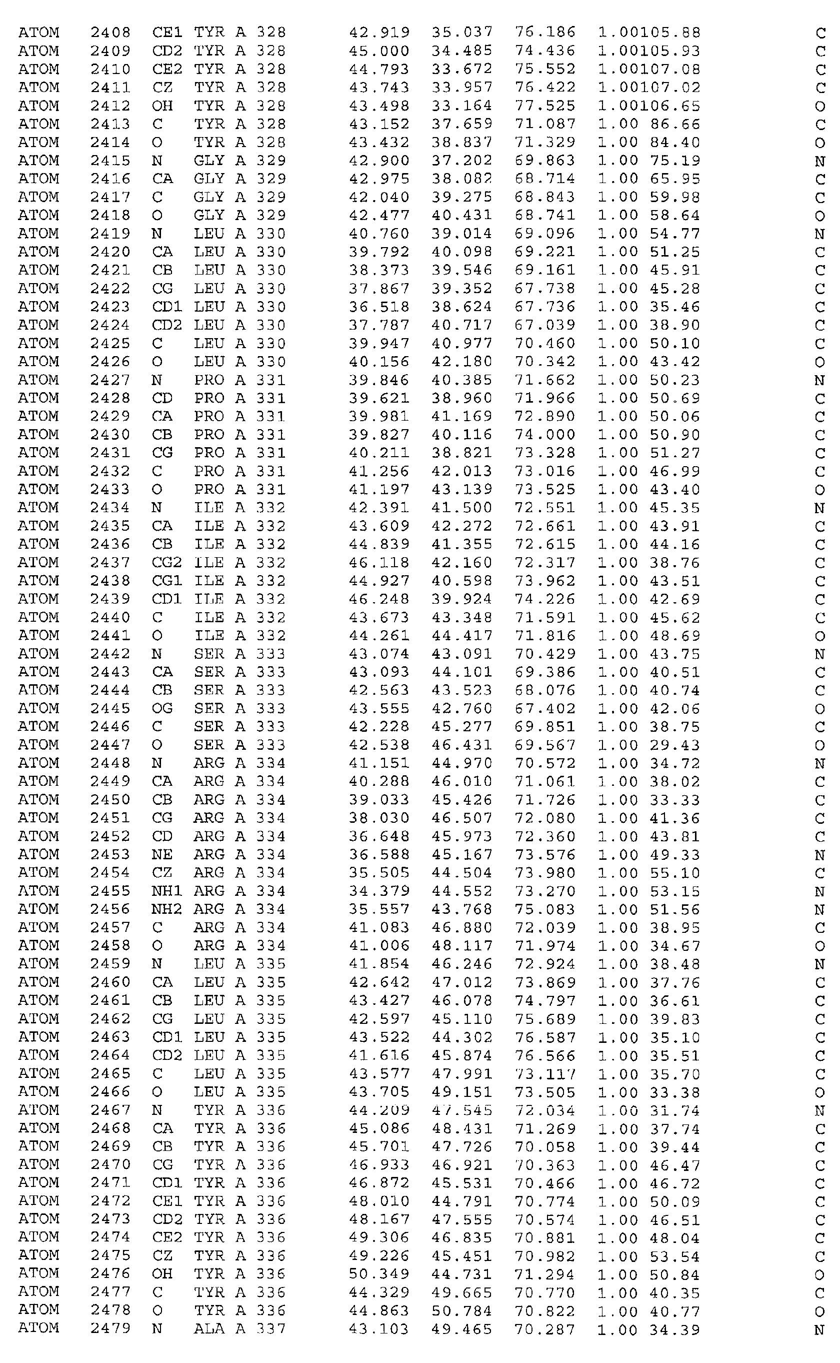

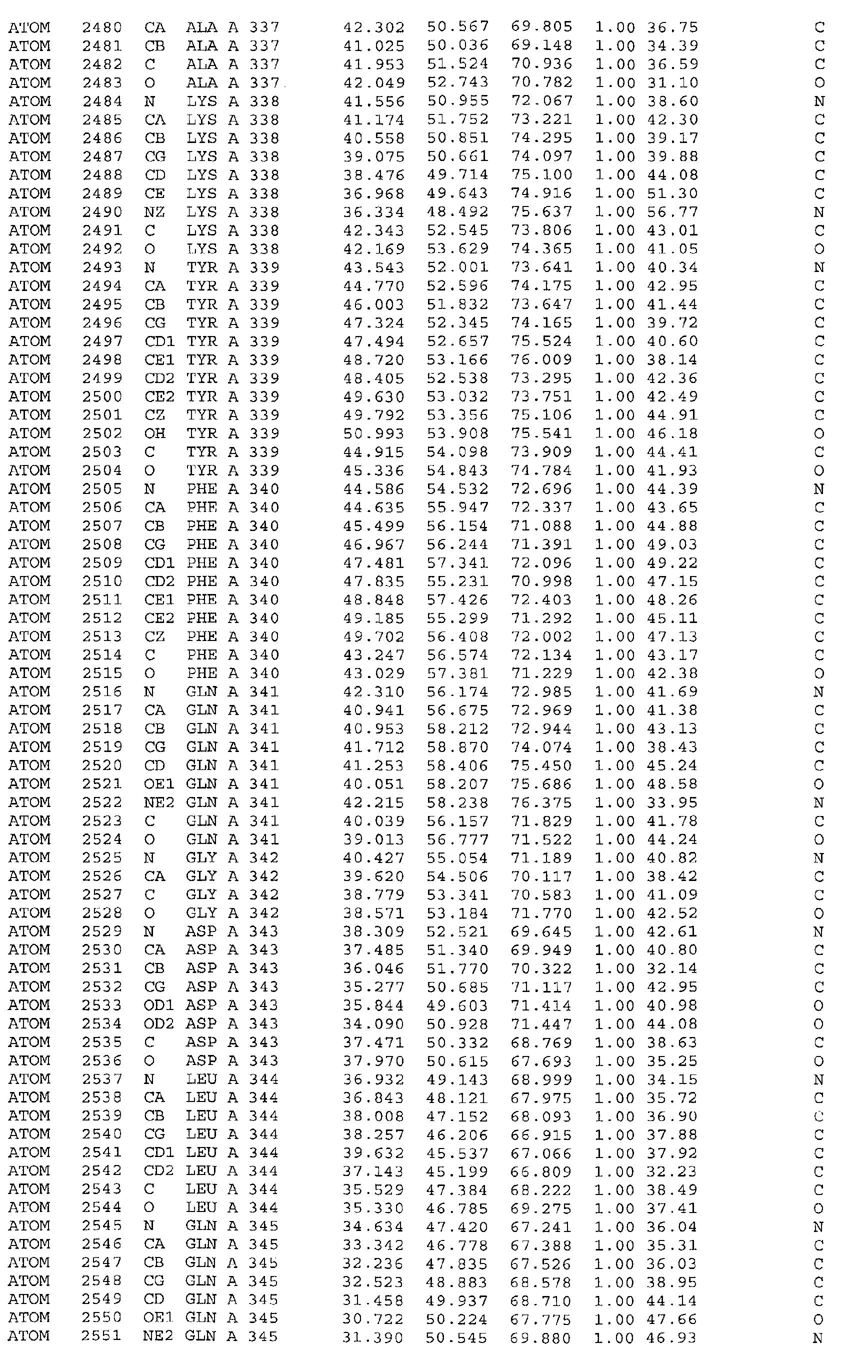

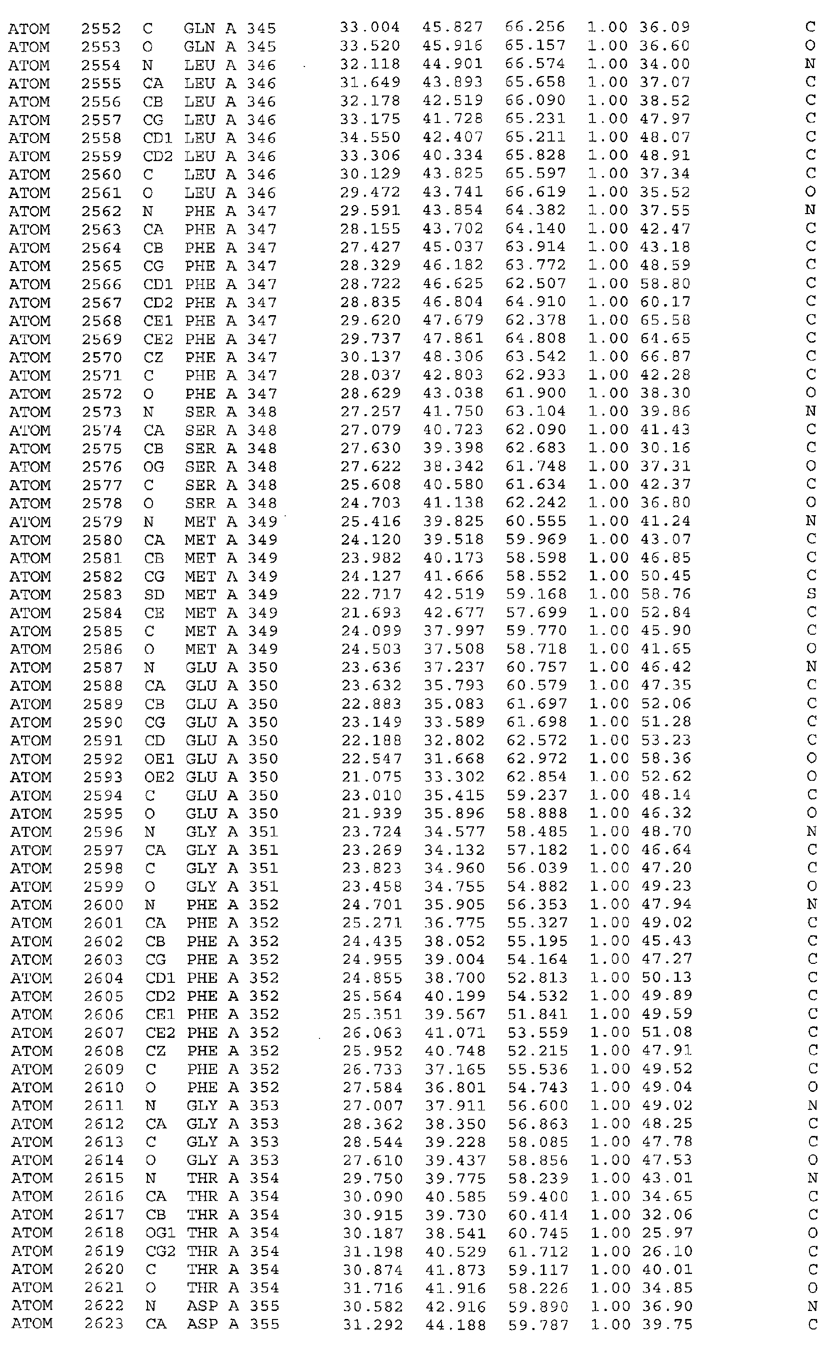

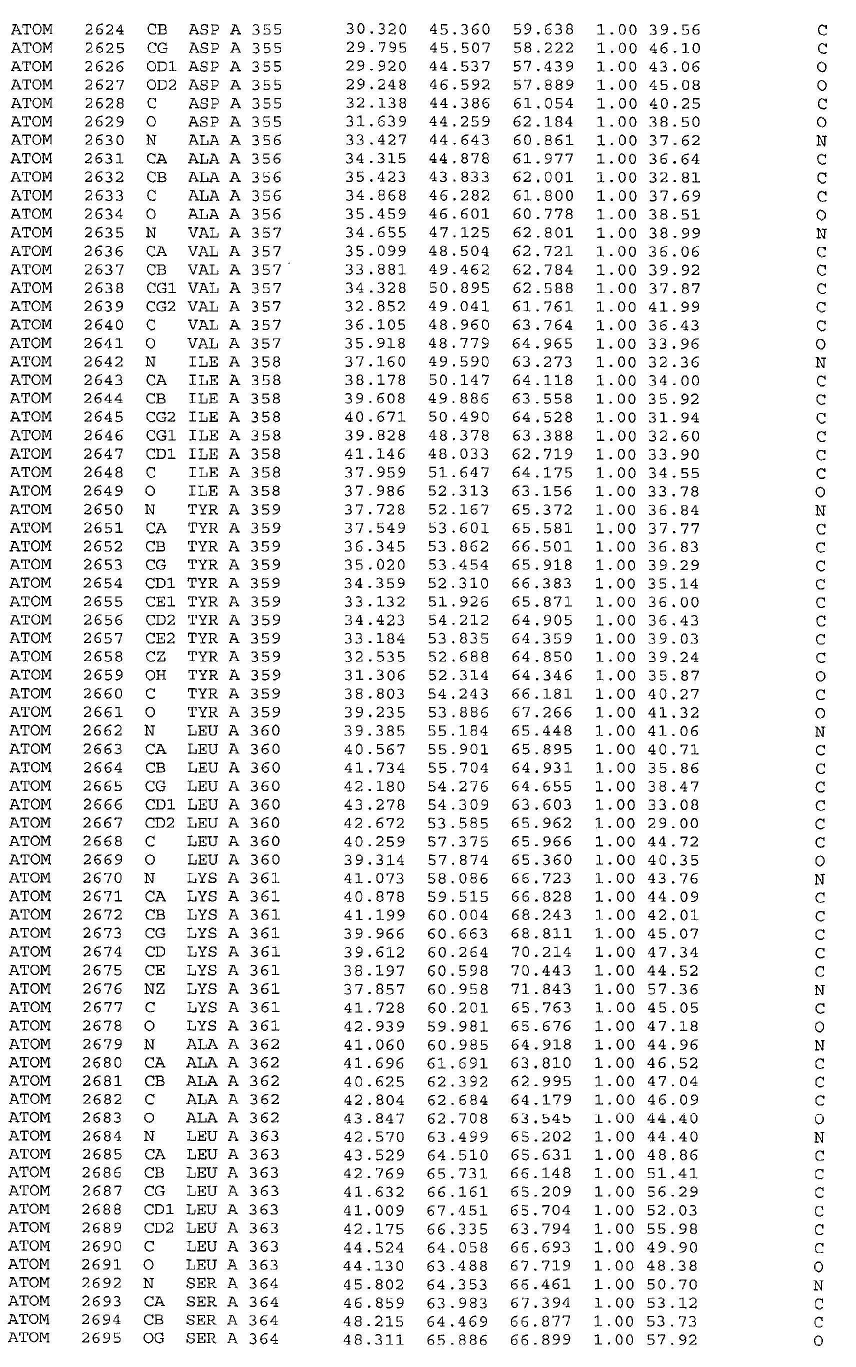

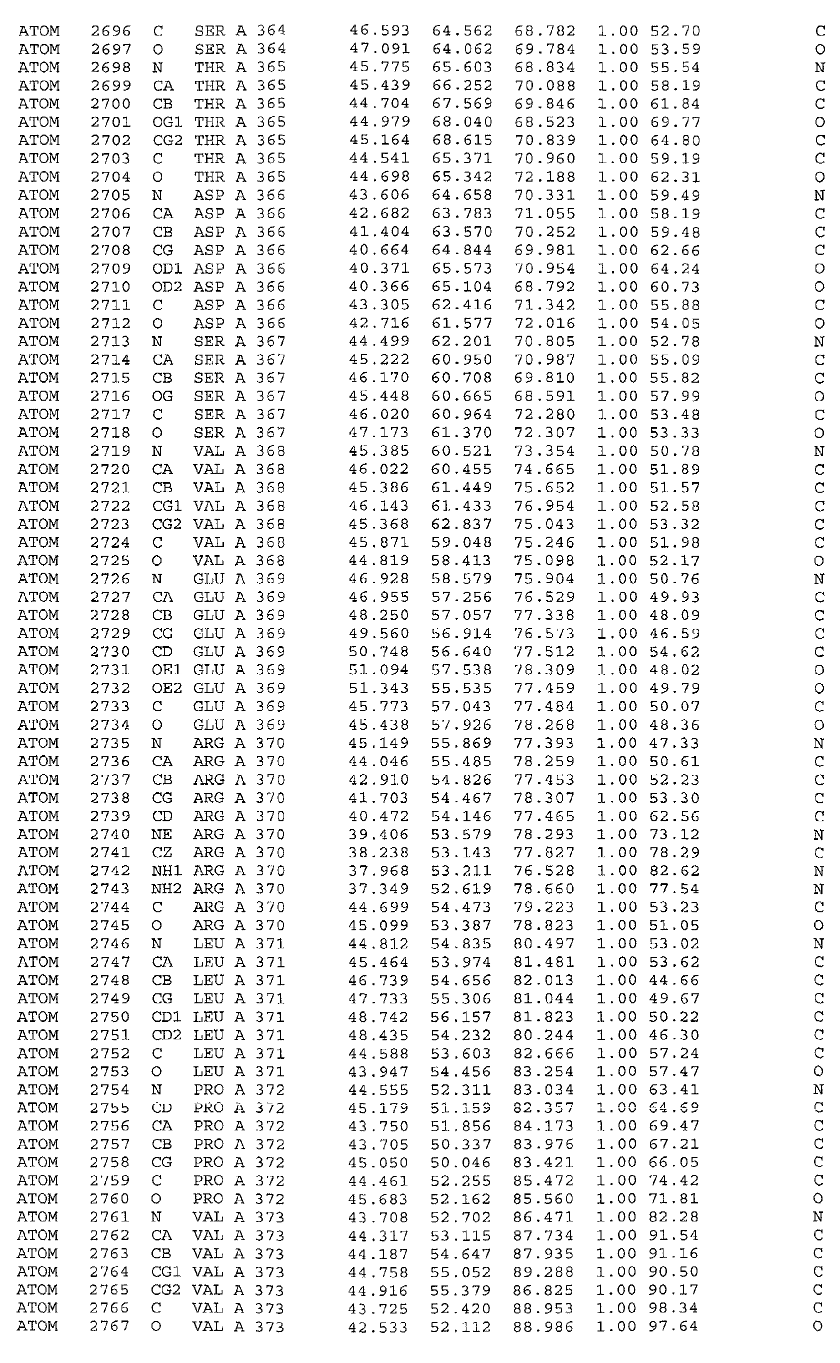

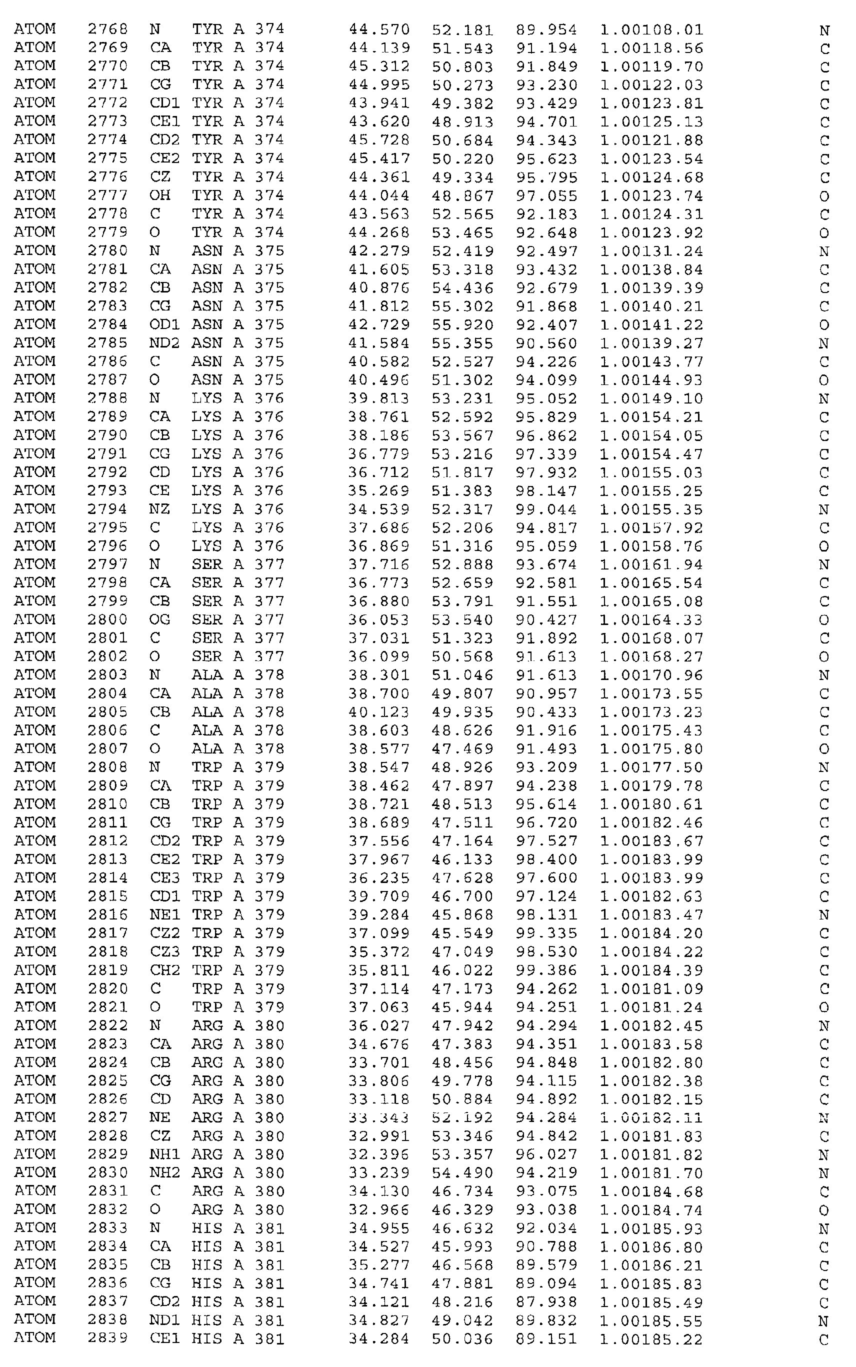

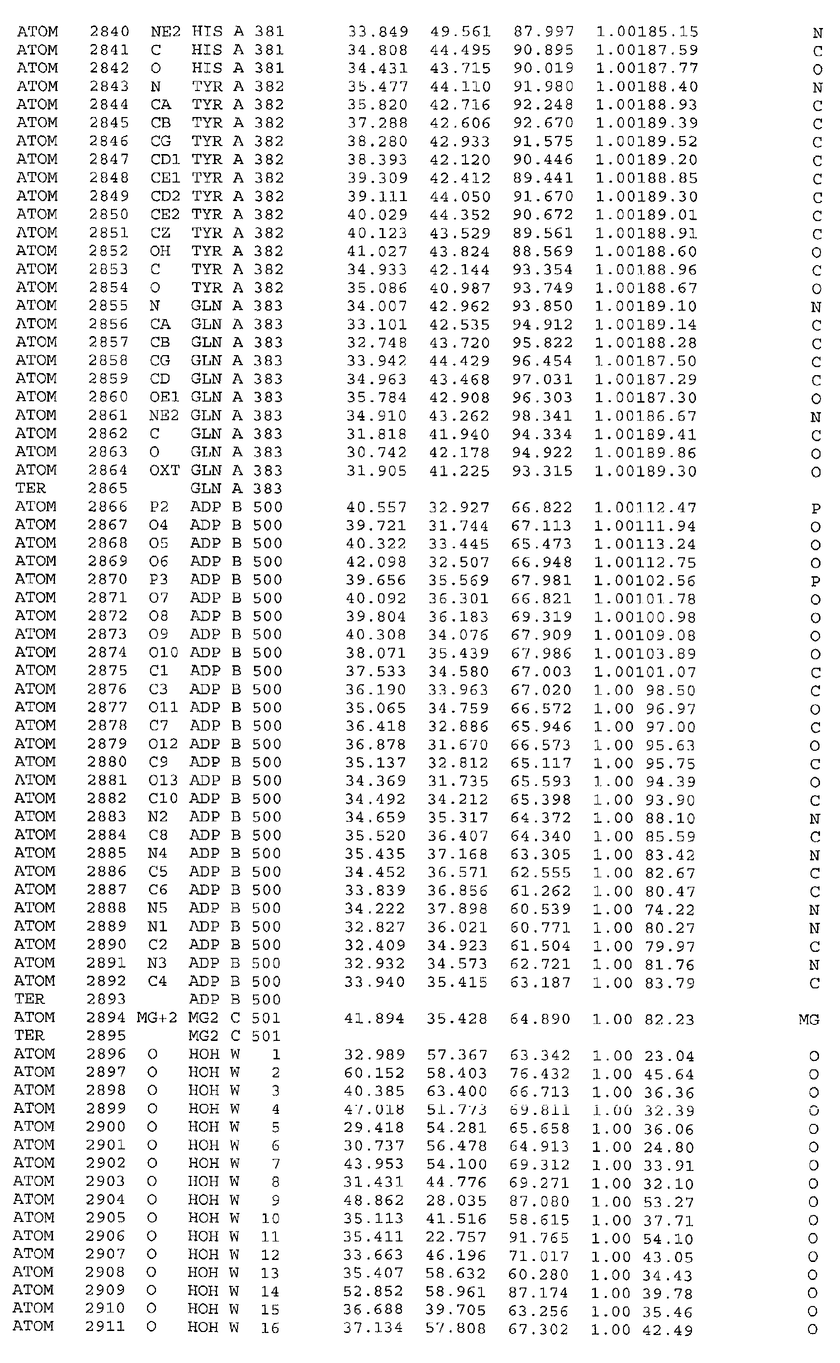

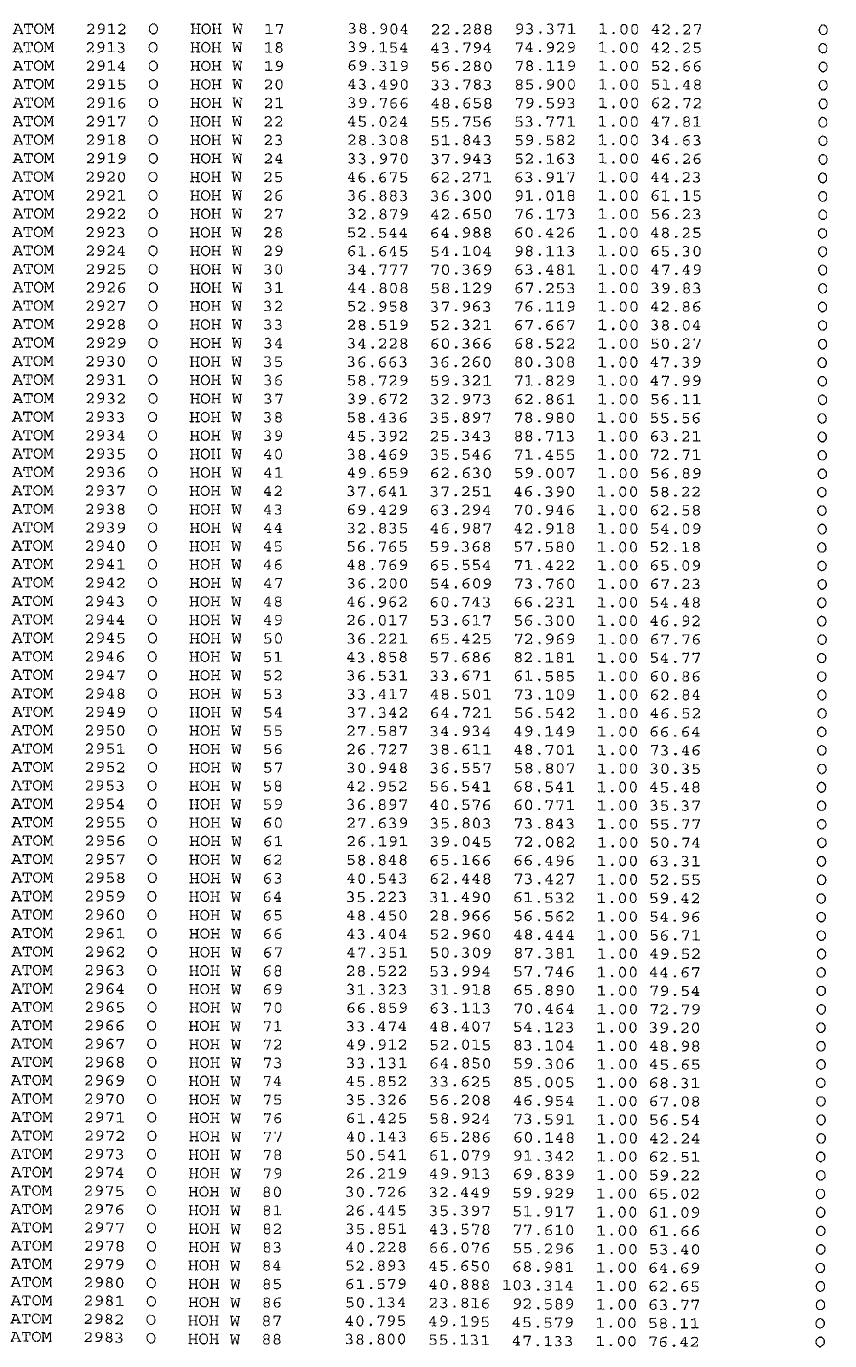

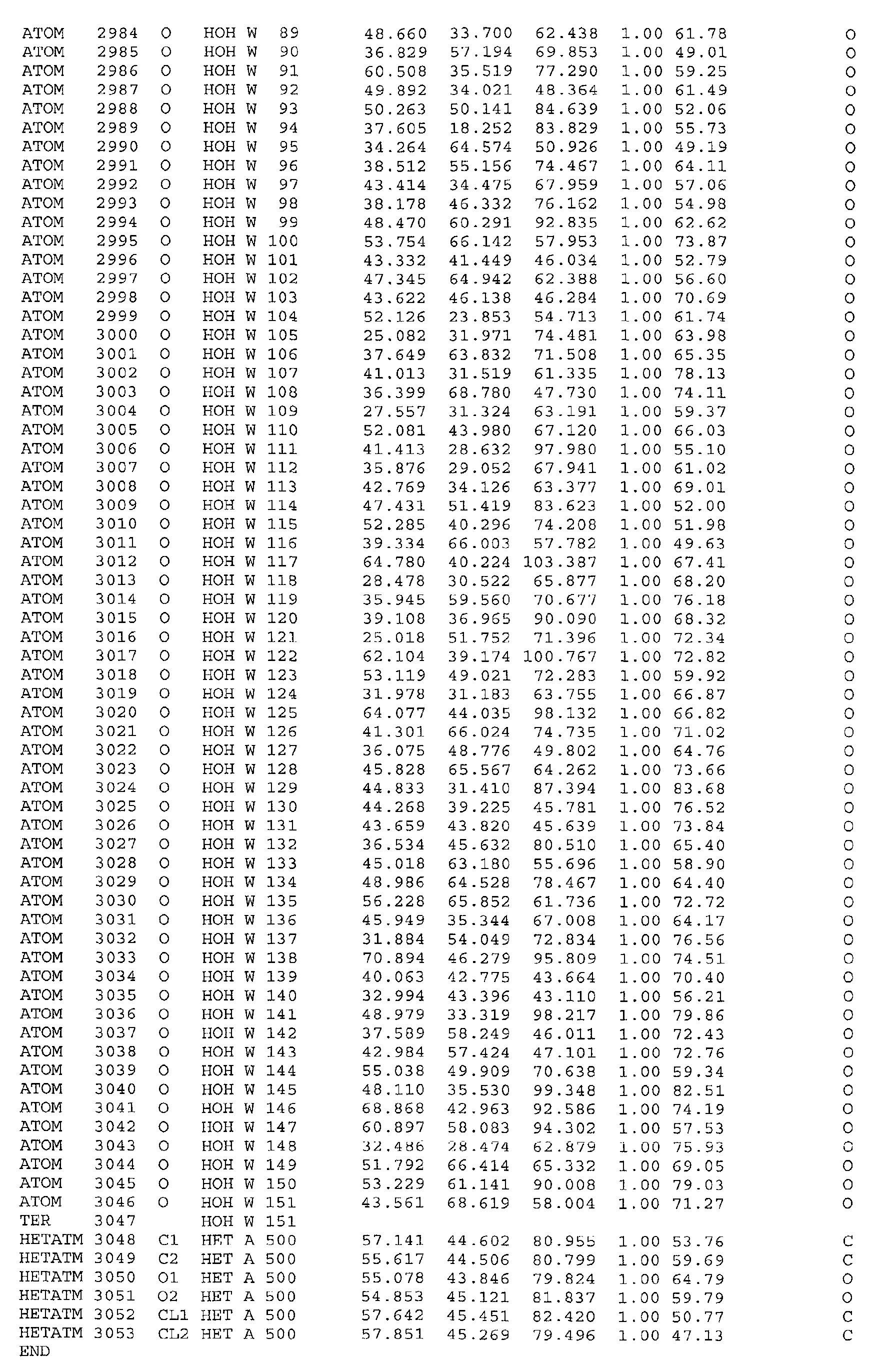

## Claims

1. A PDHK crystal wherein the PDHK protein includes PDHK-2 amino acid sequence as shown in SEQ ID NO: 2 but starting at amino acid position 16, or a homologue, fragment, variant or derivative thereof.

2. A PDHK crystal according to claim 1, wherein the PDHK-2 protein consists of PDHK-2 amino acid sequence as shown in SEQ ID NO: 2, but starting at amino acid position 16, or a homologue, fragment, variant or derivative thereof.

3. A PDHK crystal according to claim 1 or claim 2, wherein the PDHK-2 amino acid sequence is preceded by an amino acid sequence that does not correspond to the amino acid sequence at positions 1 to 15 of SEQ ID NO: 2.

4. A PDHK crystal according to any one of the preceding claims, wherein the PDHK-2 amino acid sequence is preceded by amino acid sequence that is an affinity purification tag.

5. A PDHK sequence according to any one of the preceding claims, wherein the PDHK-2 amino acid sequence is preceded by an amino acid sequence that is a specific protein cleavage site.

6. A PDHK crystal according to any one of the preceding claims, wherein the PDHK-2 amino acid sequence is preceded by an amino acid sequence that is an affinity purification tag followed by a specific protein cleavage site.

7. A PDHK crystal according to claim 4 or claim 6, wherein the affinity purification tag is a multiple Histidine residue tag.

8. A PDHK crystal according to claim 7, wherein the multiple Histidine residue tag is a Hexa-His tag.

9. A PDHK crystal according to claim 5 or claim 6, wherein the specific protein cleavage site is a thrombin cleavage site.

10. A PDHK crystal according to claim 6, wherein the affinity purification tag is a Hexa-His tag and the specific protein cleavage site is a thrombin cleavage site.

11. A PDHK crystal according to claim 10, wherein the PDHK protein consists of PDHK amino acid sequence preceded by a Hexa-His tag followed by a thrombin cleavage site.

12. A PDHK crystal according to claim 11, wherein the PDHK protein additionally has an N-terminal methionine amino acid residue.

13. A PDHK crystal according to claim 12, wherein the PDHK-2 amino acid sequence is as shown in SEQ ID NO: 4.

14. A PDHK crystal, wherein the PDHK protein includes a PDHK-2 amino acid sequence as shown in SEQ ID NO: 5, or a homologue, fragment, variant or derivative thereof.

15. A PDHK crystal according to claim 1, wherein the PDHK protein consists of a PDHK -2 amino acid sequence preceded by one or two amino acid residues.

16. A PDHK crystal according to claim 14 or claim 15, wherein the PDHK protein consists of a PDHK-2 amino acid sequence as shown in SEQ ID NO: 5 or a homologue, fragment, variant or derivative thereof.

17. A PDHK crystal according to claim 16, wherein the PDHK protein consists of a PDHK amino acid sequence as shown in SEQ ID No: 5.

18. A PDHK crystal according to any one of claims 15 to 17, wherein the PDHK-2 amino acid sequence is provided by thrombin cleavage of the PDHK-2 amino acid sequence as shown in SEQ NO: 4.

19. A crystal as claimed in any one of claims 14 to 18, which is grown in 100mM sodium acetate pH 5.2-5.8, 5-10% isopropanol, and 25-150mM MgCl₂.

20. A crystal as claimed in any one of claims 14 to 19, which is grown in 100mM sodium acetate pH 5.6-5.8, 6-9% isopropanol, and 75-125mM MgCl₂.

21. A crystal as claimed in any one of claims 14 to 20, wherein the crystal has a space group P6₄.

22. A crystal as claimed in any one of claims 14 to 21, wherein the crystal has unit cell dimensions of a = b = 109 Å +/- 3 Å, c = 85 Å +/- 3 Å.

23. A crystal as claimed in any one of claims 11 to 13, wherein the crystal is monoclinic.

24. A crystal as claimed in any one of claims 11 to 13, or claim 23, which is grown using 100mM citrate pH 5.5-5.7, 15% PEG 4K, and between 200-400mM ammonium acetate.

25. A crystal as claimed in any one of claims 11 to 13, 23 or 24, which has a space group C2.

26. A crystal as claimed in any one of claims 11 to 13, or 23 to 25 , which has unit cell dimensions of a = 90 Å +/- 3 Å, b = 54 Å +/- 3 Å, c = 83 Å +/- 3 Å, and β = 106° +/-2°.

27. A crystal as claimed in any one of the preceding claims, which has a heavy atom soaked in.

28. A crystal as claimed in claim 27, wherein the heavy atom is selected from mercury, iridium, and osmium.

29. A crystal as claimed in any one of claims 1 to 26, which has a PDHK ligand and/or ATP or ADP soaked in.

30. A PDHK-2 co-crystal with a PDHK ligand.

31. A crystal as claimed in claim 30, wherein the PDHK-2 amino acid sequence includes or consists of the sequence of SEQ ID NO: 5.

32. A crystal of claim 31, wherein the crystal is grown in 100mM MES pH6, 10% isopropanol and 200 mM calcium acetate in the presence of a PDHK ligand.

33. A crystal as claimed in any one of claims 30 to 32, wherein the ligand is 4-{(2,5)-dimethyl-4-[3,3,3-trifluoro-2-hydroxy-2-methylpropanoyl]piperazinyl} carbonyl) benzonitrile.

34. A crystal as claimed in any one of claims 30 to 33, wherein ATP is soaked in.

35. A crystal as claimed in claim29, wherein the ligand is N-{4-[(ethylanilino)sulfonyl]-2-methylphenyl}-3,3,3-trifluoro-2-hydroxy-2-methylpropanamide.

36. A crystal as claimed in claim 29, wherein the ligand is N-(2-aminoethyl)-2-{3-chloro-4-[(4-isopropylbenzyl)oxy]phenyl} acetamide.

37. A crystal as claimed in claim 29, wherein the PDHK ligand is DCA and ADP is soaked in.

38. A crystal as claimed in any preceding claim which diffracts X-rays to 3.8 Å or better resolution.

39. A crystal as claimed in claim 38, which diffracts X-rays to 3.2 Å or better resolution.

40. A crystal as claimed in claim 38, which diffracts X-rays to 2.8 Å or better resolution.

41. A crystal as claimed in claim 38, which diffracts X-rays to 2.5 Å or better resolution.

42. A crystal as claimed in claim 38, which diffracts X-rays to 2.4 Å or better resolution.

43. A crystal as claimed in claim 38, which diffracts X-rays to 2.2 Å or better resolution.

44. A crystal as claimed in any one of claims 14 to 22, having the atomic coordinates set out in Table 3, or a derivative set as expressed in any reference frame.

45. A crystal as claimed in any one of claims 30 to 33, having the atomic coordinates set out in Table 4, or a derivative set as expressed in any reference frame.

46. A crystal as claimed in claim 34, having the atomic coordinates set out in Table 5, or a derivative set as expressed in any reference frame.

47. A crystal as claimed in claim 35, having the atomic coordinates set out in Table 6, or a derivative set as expressed in any reference frame.

48. A crystal as claimed in claim 36, having the atomic coordinates set out in Table 7, or a derivative set as expressed in any reference frame.

49. A crystal as claimed in claim 37, having the atomic coordinates set out in Table 8, or a derivative set as expressed in any reference frame.

50. The use of atomic coordinates of a crystal as claimed in any one of the preceding claims for deriving the three-dimensional structure of PDHK-2.

51. The use of the three-dimensional structure of PDHK-2 as derived in claim 50 to computationally or otherwise evaluate the binding interactions of a compound with PDHK-2.

52. The use of the three-dimensional structure of PDHK-2 as derived in claim 50 to computationally or otherwise evaluate the binding interactions of a compound with a binding site on PDHK-2.

53. The use of the three-dimensional structure of PDHK-2 as derived in claim 50 to computationally or otherwise evaluate the binding interactions of a compound with the ATP binding site on PDHK-2 which includes, but is not limited to, residues selected from Glu251, Leu252, Lys254, Asn255, Ala256, Arg258, Ala259, Met288, Ser289, Asp290, Gly292, Gly293, Gly294, Val295, Ile300, Leu303, Leu323, Ala324, Gly325, Phe326, Gly327, Tyr328, Gly329, Leu330, Pro331, Leu346, Ser348, Thr354, Asp355, Ala356.

54. The use of the three-dimensional structure of PDHK-2 as derived in claim 50 to computationally or otherwise evaluate the binding interactions of a compound with the putative E₂L₂ binding site on PDHK-2 which includes, but is not limited to, residues selected from Leu31, Gln35, Phe36, Asp38, Phe39, Thr48, Ser49, Phe52, Leu53, Met167, Leu168, Gln171, His172, Ile175, Phe176.

55. The use of the three-dimensional structure of PDHK-2 as derived in claim 50 to computationally or otherwise evaluate the binding interactions of a compound with a binding site on PDHK-2 which includes, but is not limited to, residues selected from Leu71, Pro72, Arg74, Val75, Thr78, Ser80, Val81, Met130, Gly133, Val134, Glu136, Tyr137, Asp144, Val146, Ser147, Asn150, Ile151, Phe154, Leu155, Tyr382.

56. The use of the three-dimensional structure of PDHK-2 as derived in claim 50 to computationally or otherwise evaluate the binding interactions of a compound with a binding site on PDHK-2 which includes, but is not limited to, residues selected from Leu57, Leu61, Tyr88, Ser91, Ile95, Ile119, Arg120, His123, Ser161, Arg162, Ile165, Arg166, Ile169.

57. The use of the three-dimensional structure of PDHK-2 as derived in claim 50 to design a compound capable of associating with PDHK-2.

58. The use of the three-dimensional structure of PDHK-2 as derived in claim 50 to design a compound capable of associating with a binding site of PDHK-2.

59. The use of the three-dimensional structure of PDHK-2 as derived in claim 50 to design a compound capable of associating with a binding site of PDHK-2 as defined in any one of claims 53 to 56.

60. The use according to any one of claims 50 to 59, wherein the compound is an inhibitor of PDHK-2.

61. A compound evaluated for its binding to PDHK-2 or designed as in any one of claims 51 to 60.

62. A method of selecting a PDHK-2 inhibitor compound from a group of potential PDHK-2 inhibitor compounds, comprising the following steps:
a) creating a three-dimensional representation of the structure of PDHK-2 as derived in claim 50;
b) displaying and superimposing a model of the potential PDHK-2 inhibitor compound on the model of the PDHK-2 structure;
c) assessing whether the model of the potential PDHK-2 inhibitor compound fits the model of the PDHK-2 structure.

63. A method of selecting a PDHK-2 inhibitor compound from a group of potential PDHK-2 inhibitor compounds, comprising the following steps:
a) creating a three-dimensional representation of any one of the binding sites of PDHK-2 as derived in claim 50, in a suitable computer program;
b) displaying and superimposing a model of the potential PDHK-2 inhibitor compound on the model of said binding site;
c) assessing whether the model of the potential PDHK-2 inhibitor compound fits the binding site model.

64. The method as claimed in claim 62 or claim 63, further comprising the following steps:
d) incorporating the potential PDHK-2 inhibitor compound in a biological PDHK-2 activity assay;
e) determining whether the potential PDHK-2 inhibitor compound inhibits PDHK-2 activity in this assay.

65. A compound selected by the method as claimed in claim 62, claim 63 or claim 64.

66. A compound evaluated or designed as claimed in claims 51 to 60, or selected by the methods as claimed 62 to 64, to bind at the dimerisation interface or otherwise interfere with dimerisation of PDHK-2.

67. A pharmaceutical composition comprising the compound of claim 61 or claim 65 or claim 66.

68. Use of a compound as claimed in claim 61 or claim 65 or claim 66 in the manufacture of a medicament for the treatment of diseases, wherein the inhibition of PDHK is beneficial.

69. The use as claimed in claim 68, wherein the diseases are ischemia-related diseases such as congestive heart failure, peripheral vascular disease, chronic obstructive pulmonary disease, or coronary artery disease.

70. Use as claimed in claim 68 wherein the disease is diabetes, ischemia, endotoxic shock, hemorrhagic shock, lactic acidosis, or cardiac insufficiency.

71. The use of the atomic coordinates of PDHK-2 as defined in claims 44 to 49, or portions thereof, to solve a crystal form of a mutant, homologue or co-complex of PDHK-2, for example by Molecular Replacement or Difference Fourier analysis.

72. The use of the atomic coordinates of PDHK-2 as defined in claims 44 to 49, or portions thereof, to produce a model of the three-dimensional structure of related proteins.

73. The use of the three-dimensional structure of PDHK-2 as derived in claim 50 to design site-directed mutants that mimic other PDHK isozymes or variants thereof.

74. A nucleotide sequence comprising (a) the sequence shown in SEQ ID NO: 3, or allelic variants, or mutants thereof; or (b) a sequence encoding the peptide sequence as shown in SEQ ID NO: 4 or allelic variants, or mutants thereof; or (c) the sequence as shown in SEQ ID NO: 9, or allelic variants, or mutants thereof; or (d) a sequence encoding PDHK-2 amino acid sequence as shown in SEQ ID NO: 2, but starting at amino acid position 16, or allelic variants or mutants thereof; or (e) the sequence as shown in SEQ ID NO: 8 or allelic variants, or mutants thereof; or (f) a sequence encoding the amino acid sequence shown in SEQ ID No: 5, or allelic variants or mutants thereof.

75. An expression vector, wherein a nucleotide sequence as claimed in claim 74 is linked with a regulatory sequence suitable for driving expression of the PDHK protein in a host cell.

76. A recombinant baculovirus, comprising a nucleotide sequence as claimed in claim 74.
